# EUROPEAN PATENT APPLICATION

(11) **EP 3 892 640 A1**
(43) Date of publication of application: **13.10.2021**
(21) Application number: 20217556.8
(22) Date of filing: 21.08.2012
(51) Int. Cl.: C07K 16/30, A61K 39/395, A61P 35/00

(54) **BISPECIFIC T CELL ACTIVATING ANTIGEN BINDING MOLECULES**

(30) Priority: 23.08.2011 EP 11178370; 16.05.2012 EP 12168192
(62) Divisional of application: 17206703.5
(71) Applicant: Roche Glycart AG, 8952 Schlieren (CH)
(72) Inventor: AST, Oliver, 8952 Schlieren (CH); BRUENKER, Peter, 8952 Schlieren (CH); FAUTI, Tanja, 8952 Schlieren (CH); FREIMOSER-GRUNDSCHOBER, Anne, 8952 Schlieren (CH); JAEGER, Christiane, 8952 Schlieren (CH); KLEIN, Christian, 8952 Schlieren (CH); MOESSNER, Ekkehard, 8952 Schlieren (CH); UMAÑA, Pablo, 8952 Schlieren (CH)
(74) Representative: Cueni, Leah Noëmi

(57) **Abstract**

The present invention generally relates to novel bispecific antigen binding molecules for T cell activation and re-direction to specific target cells. In addition, the present invention relates to polynucleotides encoding such bispecific antigen binding molecules, and vectors and host cells comprising such polynucleotides. The invention further relates to methods for producing the bispecific antigen binding molecules of the invention, and to methods of using these bispecific antigen binding molecules in the treatment of disease.

SEQUENCE LISTING

<110>  Roche Glycart AG

<120>  Bispecific T cell activating antigen binding molecules

<130>  30598

<150>  EP 11178370.0
<151>  2011-08-23

<150>  EP 12168192.8
<151>  2012-05-16

<160>  266

<170>  PatentIn version 3.5

<210>  1
<211>  700
<212>  PRT
<213>  Artificial Sequence

<220>
<223>  V9 (scFab)-Fc(hole) P329G LALA

<400>  1

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Arg Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Arg Leu Glu Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Gly Asn Thr Leu Pro Trp
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys Ser Gly Gly Gly Ser Gly Gly Gly Ser Glu
    210                 215                 220

Gly Gly Gly Ser Glu Gly Gly Gly Ser Glu Gly Gly Gly Ser Glu Gly
225                 230                 235                 240

Gly Gly Ser Gly Gly Gly Ser Gly Glu Val Gln Leu Val Glu Ser Gly
                245                 250                 255

Gly Gly Leu Val Gln Pro Gly Gly Ser Leu Arg Leu Ser Cys Ala Ala
            260                 265                 270

Ser Gly Tyr Ser Phe Thr Gly Tyr Thr Met Asn Trp Val Arg Gln Ala
        275                 280                 285

Pro Gly Lys Gly Leu Glu Trp Val Ala Leu Ile Asn Pro Tyr Lys Gly
    290                 295                 300

Val Ser Thr Tyr Asn Gln Lys Phe Lys Asp Arg Phe Thr Ile Ser Val
305                 310                 315                 320

Asp Lys Ser Lys Asn Thr Ala Tyr Leu Gln Met Asn Ser Leu Arg Ala
                325                 330                 335

Glu Asp Thr Ala Val Tyr Tyr Cys Ala Arg Ser Gly Tyr Tyr Gly Asp
            340                 345                 350

Ser Asp Trp Tyr Phe Asp Val Trp Gly Gln Gly Thr Leu Val Thr Val
        355                 360                 365

Ser Ser Ala Ser Thr Lys Gly Pro Ser Val Phe Pro Leu Ala Pro Ser
    370                 375                 380

Ser Lys Ser Thr Ser Gly Gly Thr Ala Ala Leu Gly Cys Leu Val Lys
385                 390                 395                 400

Asp Tyr Phe Pro Glu Pro Val Thr Val Ser Trp Asn Ser Gly Ala Leu
                405                 410                 415

Thr Ser Gly Val His Thr Phe Pro Ala Val Leu Gln Ser Ser Gly Leu
            420                 425                 430

Tyr Ser Leu Ser Ser Val Val Thr Val Pro Ser Ser Ser Leu Gly Thr
        435                 440                 445

Gln Thr Tyr Ile Cys Asn Val Asn His Lys Pro Ser Asn Thr Lys Val
    450                 455                 460

Asp Lys Lys Val Glu Pro Lys Ser Cys Asp Lys Thr His Thr Cys Pro
465                 470                 475                 480

Pro Cys Pro Ala Pro Glu Ala Ala Gly Gly Pro Ser Val Phe Leu Phe
                485                 490                 495

Pro Pro Lys Pro Lys Asp Thr Leu Met Ile Ser Arg Thr Pro Glu Val
            500                 505                 510

Thr Cys Val Val Val Asp Val Ser His Glu Asp Pro Glu Val Lys Phe
        515                 520                 525

Asn Trp Tyr Val Asp Gly Val Glu Val His Asn Ala Lys Thr Lys Pro
    530                 535                 540

Arg Glu Glu Gln Tyr Asn Ser Thr Tyr Arg Val Val Ser Val Leu Thr
545                 550                 555                 560

Val Leu His Gln Asp Trp Leu Asn Gly Lys Glu Tyr Lys Cys Lys Val
                565                 570                 575

Ser Asn Lys Ala Leu Gly Ala Pro Ile Glu Lys Thr Ile Ser Lys Ala
            580                 585                 590

Lys Gly Gln Pro Arg Glu Pro Gln Val Cys Thr Leu Pro Pro Ser Arg
        595                 600                 605

Asp Glu Leu Thr Lys Asn Gln Val Ser Leu Ser Cys Ala Val Lys Gly
    610                 615                 620

Phe Tyr Pro Ser Asp Ile Ala Val Glu Trp Glu Ser Asn Gly Gln Pro
625                 630                 635                 640

Glu Asn Asn Tyr Lys Thr Thr Pro Pro Val Leu Asp Ser Asp Gly Ser

645                 650                 655

Phe Phe Leu Val Ser Lys Leu Thr Val Asp Lys Ser Arg Trp Gln Gln
            660                 665                 670

Gly Asn Val Phe Ser Cys Ser Val Met His Glu Ala Leu His Asn His
        675                 680                 685

Tyr Thr Gln Lys Ser Leu Ser Leu Ser Pro Gly Lys
    690                 695                 700

<210>  2
<211>  2103
<212>  DNA
<213>  Artificial Sequence

<220>
<223>  V9 (scFab)-Fc(hole) P329G LALA

<400>  2
gacatccaga tgacccagag cccctctagc ctgagcgcca gcgtgggcga cagagtgacc      60

atcacctgtc gggccagcca ggacatcaga aactacctga actggtatca gcagaagccc     120

ggcaaggccc ccaagctgct gatctactac acctctagac tggaaagcgg cgtgcccagc     180

cggtttagcg gcagcggctc cggcaccgac tacaccctga ccatcagcag cctgcagccc     240

gaggacttcg ccacctacta ctgccagcag ggcaacacac tcccctggac cttcggccag     300

ggcaccaagg tggagatcaa gcgtacggtg gccgctccca gcgtgttcat cttccccccc     360

agcgacgagc agctgaagtc cggcaccgcc agcgtcgtgt gcctgctgaa caacttctac     420

ccccgggagg ccaaggtgca gtggaaggtg gacaacgccc tgcagagcgg caacagccag     480

gaaagcgtca ccgagcagga cagcaaggac tccacctaca gcctgagcag caccctgacc     540

ctgagcaagg ccgactacga gaagcacaag gtgtacgcct gcgaagtgac ccaccagggc     600

ctgtccagcc ccgtgaccaa gagcttcaac cggggcgagt gcagcggcgg aggctctgga     660

ggcggctctg aaggcggagg aagtgagggc ggaggctcag aaggcggcgg aagcgaaggt     720

ggcggctctg gcggcggatc cggcgaggtg cagctggtcg agtccggcgg aggcctggtg     780

cagcctggcg gcagcctgag actgagctgc gccgccagcg gctacagctt caccggctac     840

accatgaact gggtccggca ggctcctggc aagggcctcg aatgggtggc cctgatcaac     900

ccctacaagg gcgtgagcac ctacaaccag aagttcaagg accggttcac catcagcgtg     960

gacaagagca agaacaccgc ctatctgcag atgaacagcc tgcgggccga ggacaccgcc    1020

gtgtactact gcgccagaag cggctactac ggcgacagcg actggtactt cgacgtgtgg    1080

ggccagggca cactggtcac cgtgtccagc gctagcacca agggcccatc ggtcttcccc    1140

ctggcaccct cctccaagag cacctctggg ggcacagcgg ccctgggctg cctggtcaag    1200

gactacttcc ccgaaccggt gacggtgtcg tggaactcag gcgccctgac cagcggcgtg 1260

cacaccttcc cggctgtcct acagtcctca ggactctact ccctcagcag cgtggtgacc 1320

gtgccctcca gcagcttggg cacccagacc tacatctgca acgtgaatca caagcccagc 1380

aacaccaagg tggacaagaa agttgagccc aaatcttgtg acaaaactca cacatgccca 1440

ccgtgcccag cacctgaagc tgcaggggga ccgtcagtct tcctcttccc cccaaaaccc 1500

aaggacaccc tcatgatctc ccggacccct gaggtcacat gcgtggtggt ggacgtgagc 1560

cacgaagacc ctgaggtcaa gttcaactgg tacgtggacg gcgtggaggt gcataatgcc 1620

aagacaaagc cgcgggagga gcagtacaac agcacgtacc gtgtggtcag cgtcctcacc 1680

gtcctgcacc aggactggct gaatggcaag gagtacaagt gcaaggtctc caacaaagcc 1740

ctcggcgccc ccatcgagaa aaccatctcc aaagccaaag ggcagccccg agaaccacag 1800

gtgtgcaccc tgcccccatc ccgggatgag ctgaccaaga accaggtcag cctctcgtgc 1860

gcagtcaaag gcttctatcc cagcgacatc gccgtggagt gggagagcaa tgggcagccg 1920

gagaacaact acaagaccac gcctcccgtg ctggactccg acggctcctt cttcctcgtg 1980

agcaagctca ccgtggacaa gagcaggtgg cagcagggga acgtcttctc atgctccgtg 2040

atgcatgagg ctctgcacaa ccactacacg cagaagagcc tctccctgtc tccgggtaaa 2100

tga 2103

<210> 3
<211> 442
<212> PRT
<213> Artificial Sequence

<220>
<223> LC007 (VH-CH1)-Fc(knob) P329G LALA

<400> 3

Glu Val Gln Leu Gln Glu Ser Gly Pro Gly Leu Val Lys Pro Ser Gln
1               5                   10                  15

Ser Leu Ser Leu Thr Cys Ser Val Thr Gly Tyr Ser Ile Thr Ser Gly
            20                  25                  30

Tyr Tyr Trp Asn Trp Ile Arg Gln Phe Pro Gly Asn Lys Leu Glu Trp
        35                  40                  45

Met Gly Tyr Ile Thr Tyr Asp Gly Ser Asn Asn Tyr Asn Pro Ser Leu
    50                  55                  60

Lys Asn Arg Ile Ser Ile Thr Arg Asp Thr Ser Lys Asn Gln Phe Phe
65                  70                  75                  80

Leu Lys Leu Asn Ser Val Thr Thr Glu Asp Thr Ala Thr Tyr Tyr Cys
                85                  90                  95

Ala Asp Phe Asp Tyr Trp Gly Gln Gly Thr Thr Leu Thr Val Ser Ser
            100                 105                 110

Ala Ser Thr Lys Gly Pro Ser Val Phe Pro Leu Ala Pro Ser Ser Lys
        115                 120                 125

Ser Thr Ser Gly Gly Thr Ala Ala Leu Gly Cys Leu Val Lys Asp Tyr
    130                 135                 140

Phe Pro Glu Pro Val Thr Val Ser Trp Asn Ser Gly Ala Leu Thr Ser
145                 150                 155                 160

Gly Val His Thr Phe Pro Ala Val Leu Gln Ser Ser Gly Leu Tyr Ser
                165                 170                 175

Leu Ser Ser Val Val Thr Val Pro Ser Ser Ser Leu Gly Thr Gln Thr
            180                 185                 190

Tyr Ile Cys Asn Val Asn His Lys Pro Ser Asn Thr Lys Val Asp Lys
        195                 200                 205

Lys Val Glu Pro Lys Ser Cys Asp Lys Thr His Thr Cys Pro Pro Cys
    210                 215                 220

Pro Ala Pro Glu Ala Ala Gly Gly Pro Ser Val Phe Leu Phe Pro Pro
225                 230                 235                 240

Lys Pro Lys Asp Thr Leu Met Ile Ser Arg Thr Pro Glu Val Thr Cys
                245                 250                 255

Val Val Val Asp Val Ser His Glu Asp Pro Glu Val Lys Phe Asn Trp
            260                 265                 270

Tyr Val Asp Gly Val Glu Val His Asn Ala Lys Thr Lys Pro Arg Glu
        275                 280                 285

Glu Gln Tyr Asn Ser Thr Tyr Arg Val Val Ser Val Leu Thr Val Leu
    290                 295                 300

His Gln Asp Trp Leu Asn Gly Lys Glu Tyr Lys Cys Lys Val Ser Asn
305                 310                 315                 320

Lys Ala Leu Gly Ala Pro Ile Glu Lys Thr Ile Ser Lys Ala Lys Gly
                325                 330                 335

Gln Pro Arg Glu Pro Gln Val Tyr Thr Leu Pro Pro Cys Arg Asp Glu
            340                 345                 350

Leu Thr Lys Asn Gln Val Ser Leu Trp Cys Leu Val Lys Gly Phe Tyr
        355                 360                 365

Pro Ser Asp Ile Ala Val Glu Trp Glu Ser Asn Gly Gln Pro Glu Asn
    370                 375                 380

Asn Tyr Lys Thr Thr Pro Pro Val Leu Asp Ser Asp Gly Ser Phe Phe
385                 390                 395                 400

Leu Tyr Ser Lys Leu Thr Val Asp Lys Ser Arg Trp Gln Gln Gly Asn
                405                 410                 415

Val Phe Ser Cys Ser Val Met His Glu Ala Leu His Asn His Tyr Thr
            420                 425                 430

Gln Lys Ser Leu Ser Leu Ser Pro Gly Lys
        435                 440

<210>  4
<211>  1329
<212>  DNA
<213>  Artificial Sequence

<220>
<223>  LC007 (VH-CH1)-Fc(knob) P329G LALA

<400>  4
gaggtccagc tgcaggagtc aggacctggc ctcgtgaaac cttctcagtc tctgtctctc      60

acctgctctg tcactggcta ctccatcacc agtggttatt actggaactg gatccggcag     120

tttccaggaa acaagctgga atggatgggc tacataacct acgacggtag caataactac     180

aacccatctc tcaaaaatcg aatctccatc actcgtgaca catctaagaa ccagtttttc     240

ctgaagttga attctgtgac tactgaggac acagctacat attactgtgc ggactttgac     300

tactggggcc aaggcaccac tctcacagtc tcctcagcta gcaccaaggg cccatcggtc     360

ttccccctgg caccctcctc caagagcacc tctgggggca cagcggccct gggctgcctg     420

gtcaaggact acttccccga accggtgacg gtgtcgtgga actcaggcgc cctgaccagc     480

ggcgtgcaca ccttcccggc tgtcctacag tcctcaggac tctactccct cagcagcgtg     540

gtgaccgtgc cctccagcag cttgggcacc cagacctaca tctgcaacgt gaatcacaag     600

cccagcaaca ccaaggtgga caagaaagtt gagcccaaat cttgtgacaa gacccacacc     660

tgtccccctt gccctgcccc tgaagctgct ggtggccctt ccgtgttcct gttcccccca     720

aagcccaagg acaccctgat gatcagccgg acccccgaag tgacctgcgt ggtggtcgat 780

gtgtcccacg aggaccctga agtgaagttc aattggtacg tggacggcgt ggaagtgcac 840

aatgccaaga ccaagccgcg ggaggagcag tacaacagca cgtaccgtgt ggtcagcgtc 900

ctcaccgtcc tgcaccagga ctggctgaat ggcaaggagt acaagtgcaa ggtctccaac 960

aaagccctcg gcgcccccat cgagaaaacc atctccaaag ccaaagggca gccccgagaa 1020

ccacaggtgt acaccctgcc cccatgccgg gatgagctga ccaagaacca ggtcagcctg 1080

tggtgcctgg tcaaaggctt ctatcccagc gacatcgccg tggagtggga gagcaatggg 1140

cagccggaga acaactacaa gaccacgcct cccgtgctgg actccgacgg ctccttcttc 1200

ctctacagca agctcaccgt ggacaagagc aggtggcagc aggggaacgt cttctcatgc 1260

tccgtgatgc atgaggctct gcacaaccac tacacgcaga agagcctctc cctgtctccg 1320

ggtaaataa 1329

<210> 5
<211> 214
<212> PRT
<213> Artificial Sequence

<220>
<223> LC007 (VL-CL)

<400> 5

Asp Ile Val Leu Thr Gln Ser Pro Ser Ser Leu Ser Ala Ser Leu Gly
1               5                   10                  15

Asp Arg Val Thr Ile Ser Cys Ser Ala Ser Gln Gly Ile Arg Asn Tyr
            20                  25                  30

Leu Asn Trp Tyr Gln Gln Arg Pro Asp Gly Thr Val Lys Leu Leu Ile
        35                  40                  45

Tyr Tyr Thr Ser Ser Leu His Ser Gly Val Pro Ser Arg Phe Ser Gly
    50                  55                  60

Ser Gly Ser Gly Thr Asp Tyr Ser Leu Thr Ile Ser Asn Leu Glu Pro
65                  70                  75                  80

Glu Asp Ile Ala Thr Tyr Tyr Cys Gln Gln Tyr Ser Lys Leu Pro Trp
                85                  90                  95

Thr Phe Gly Gly Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  6
<211>  645
<212>  DNA
<213>  Artificial Sequence

<220>
<223>  LC007 (VL-CL)

<400>  6
gatattgtgc tcacacagtc tccatcctcc ctgtctgcct ctctgggaga cagagtcacc      60

atcagttgca gtgcaagtca gggcattaga aattatttaa actggtatca gcagagacca     120

gatggaactg ttaaactcct gatctattac acatcaagtt tacactcagg agtcccatca     180

aggttcagtg gcagtgggtc tgggacagat tattctctca ccatcagcaa cctggaacct     240

gaagatattg ccacttacta ttgtcagcag tatagtaagc ttccttggac gttcggtgga     300

ggcaccaagc tggaaatcaa acgtacggtg gctgcaccat ctgtcttcat cttcccgcca     360

tctgatgagc agttgaaatc tggaactgcc tctgttgtgt gcctgctgaa taacttctat     420

cccagagagg ccaaagtaca gtggaaggtg gataacgccc tccaatcggg taactcccag     480

gagagtgtca cagagcagga cagcaaggac agcacctaca gcctcagcag caccctgacg     540

ctgagcaaag cagactacga gaaacacaaa gtctacgcct gcgaagtcac ccatcagggc     600

ctgagctcgc ccgtcacaaa gagcttcaac aggggagagt gttag                     645

<210>  7
<211>  704
<212>  PRT
<213>  Artificial Sequence

<220>
<223>  LC007 (scFab)-Fc(hole) P329G LALA

<400>  7

Asp Ile Val Leu Thr Gln Ser Pro Ser Ser Leu Ser Ala Ser Leu Gly
1               5                   10                  15

Asp Arg Val Thr Ile Ser Cys Ser Ala Ser Gln Gly Ile Arg Asn Tyr
            20                  25                  30

Leu Asn Trp Tyr Gln Gln Arg Pro Asp Gly Thr Val Lys Leu Leu Ile
        35                  40                  45

Tyr Tyr Thr Ser Ser Leu His Ser Gly Val Pro Ser Arg Phe Ser Gly
    50                  55                  60

Ser Gly Ser Gly Thr Asp Tyr Ser Leu Thr Ile Ser Asn Leu Glu Pro
65                  70                  75                  80

Glu Asp Ile Ala Thr Tyr Tyr Cys Gln Gln Tyr Ser Lys Leu Pro Trp
                85                  90                  95

Thr Phe Gly Gly Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys Ser Gly Gly Gly Ser Gly Gly Gly Ser Glu
    210                 215                 220

Gly Gly Gly Ser Glu Gly Gly Gly Ser Glu Gly Gly Gly Ser Glu Gly
225                 230                 235                 240

Gly Gly Ser Gly Gly Gly Ser Gly Glu Val Gln Leu Gln Glu Ser Gly
                245                 250                 255

Pro Gly Leu Val Lys Pro Ser Gln Ser Leu Ser Leu Thr Cys Ser Val
            260                 265                 270

Thr Gly Tyr Ser Ile Thr Ser Gly Tyr Tyr Trp Asn Trp Ile Arg Gln
        275                 280                 285

Phe Pro Gly Asn Lys Leu Glu Trp Met Gly Tyr Ile Thr Tyr Asp Gly
    290                 295                 300

Ser Asn Asn Tyr Asn Pro Ser Leu Lys Asn Arg Ile Ser Ile Thr Arg
305                 310                 315                 320

Asp Thr Ser Lys Asn Gln Phe Phe Leu Lys Leu Asn Ser Val Thr Thr
                325                 330                 335

Glu Asp Thr Ala Thr Tyr Tyr Cys Ala Asp Phe Asp Tyr Trp Gly Gln
            340                 345                 350

Gly Thr Thr Leu Thr Val Ser Ser Ala Ser Thr Lys Gly Pro Ser Val
        355                 360                 365

Phe Pro Leu Ala Pro Ser Ser Lys Ser Thr Ser Gly Gly Thr Ala Ala
    370                 375                 380

Leu Gly Cys Leu Val Lys Asp Tyr Phe Pro Glu Pro Val Thr Val Ser
385                 390                 395                 400

Trp Asn Ser Gly Ala Leu Thr Ser Gly Val His Thr Phe Pro Ala Val
                405                 410                 415

Leu Gln Ser Ser Gly Leu Tyr Ser Leu Ser Ser Val Val Thr Val Pro
            420                 425                 430

Ser Ser Ser Leu Gly Thr Gln Thr Tyr Ile Cys Asn Val Asn His Lys
        435                 440                 445

Pro Ser Asn Thr Lys Val Asp Lys Lys Val Glu Pro Lys Ser Cys Asp
    450                 455                 460

Gly Gly Gly Gly Ser Gly Gly Gly Gly Ser Gly Ala Gln Asp Lys Thr
465                 470                 475                 480

His Thr Cys Pro Pro Cys Pro Ala Pro Glu Ala Ala Gly Gly Pro Ser

485                 490                 495

Val Phe Leu Phe Pro Pro Lys Pro Lys Asp Thr Leu Met Ile Ser Arg
            500                 505                 510

Thr Pro Glu Val Thr Cys Val Val Val Asp Val Ser His Glu Asp Pro
        515                 520                 525

Glu Val Lys Phe Asn Trp Tyr Val Asp Gly Val Glu Val His Asn Ala
    530                 535                 540

Lys Thr Lys Pro Arg Glu Glu Gln Tyr Asn Ser Thr Tyr Arg Val Val
545                 550                 555                 560

Ser Val Leu Thr Val Leu His Gln Asp Trp Leu Asn Gly Lys Glu Tyr
                565                 570                 575

Lys Cys Lys Val Ser Asn Lys Ala Leu Pro Ala Pro Ile Glu Lys Thr
            580                 585                 590

Ile Ser Lys Ala Lys Gly Gln Pro Arg Glu Pro Gln Val Cys Thr Leu
        595                 600                 605

Pro Pro Ser Arg Asp Glu Leu Thr Lys Asn Gln Val Ser Leu Ser Cys
    610                 615                 620

Ala Val Lys Gly Phe Tyr Pro Ser Asp Ile Ala Val Glu Trp Glu Ser
625                 630                 635                 640

Asn Gly Gln Pro Glu Asn Asn Tyr Lys Thr Thr Pro Pro Val Leu Asp
                645                 650                 655

Ser Asp Gly Ser Phe Phe Leu Val Ser Lys Leu Thr Val Asp Lys Ser
            660                 665                 670

Arg Trp Gln Gln Gly Asn Val Phe Ser Cys Ser Val Met His Glu Ala
        675                 680                 685

Leu His Asn His Tyr Thr Gln Lys Ser Leu Ser Leu Ser Pro Gly Lys
    690                 695                 700

<210>  8
<211>  2115
<212>  DNA
<213>  Artificial Sequence

<220>
<223>  LC007 (scFab)-Fc(hole) P329G LALA

<400>  8
gacatcgtgc tgacccagag ccctagcagc ctgagcgcca gcctgggcga cagagtgacc      60

atcagctgta gcgcctccca gggcatcaga aactacctga actggtatca gcagagaccc     120

gacggcacag tgaagctgct gatctactac accagcagcc tgcacagcgg cgtgccaagc     180

agattcagcg gcagcggctc cggcacagac tacagcctga ccatctccaa cctggaaccc     240

gaggatatcg ccacctacta ctgccagcag tacagcaagc tgccctggac cttcggcgga     300

ggcaccaagc tggaaatcaa gcggaccgtg gccgctccca gcgtgttcat cttcccaccc     360

agcgacgagc agctgaagtc cggcacagcc agcgtcgtgt gcctgctgaa caacttctac     420

ccccgggagg ccaaggtgca gtggaaggtg gacaacgccc tgcagagcgg caacagccag     480

gaaagcgtca ccgagcagga cagcaaggac tccacctaca gcctgtccag caccctgacc     540

ctgagcaagg ccgactacga gaagcacaag gtgtacgcct gcgaagtgac ccaccagggc     600

ctgagcagcc ccgtgaccaa gagcttcaac cggggcgagt gtagtggcgg aggctctggc     660

ggaggaagcg agggcggagg atctgaaggc ggcggatctg agggggaggg cagtgaaggg     720

ggaggctcag ggggaggatc cggcgaggtg cagctgcagg aatctggccc tggcctggtc     780

aagccaagcc agagtctgag cctgacctgc agcgtgaccg gctacagcat taccagcggc     840

tactactgga actggattcg gcagttcccc ggcaataagc tggaatggat gggctacatc     900

acctacgacg gcagcaacaa ctacaacccc agcctgaaga accggatcag catcacccgg     960

gacaccagca agaaccagtt cttcctgaag ctgaacagcg tgaccaccga ggacaccgcc    1020

acatactatt gcgccgactt cgactactgg ggccagggca ccaccctgac cgtgtccagc    1080

gccagcacaa agggccctag cgtgttccct ctggccccca gcagcaagag cacaagcggc    1140

ggaacagccg ccctgggctg cctcgtgaag gactacttcc ccgagcccgt gacagtgtct    1200

tggaacagcg gagccctgac aagcggcgtg cacaccttcc ctgccgtgct gcagagcagc    1260

ggcctgtact ccctgagcag cgtggtcacc gtgcctagca gcagcctggg cacccagacc    1320

tacatctgca acgtgaacca caagcccagc aacaccaaag tggacaagaa ggtggagccc    1380

aagagctgtg atggcggagg agggtccgga ggcggtggat ccggagctca ggacaaaact    1440

cacacatgcc caccgtgccc agcacctgaa gctgcagggg gaccgtcagt cttcctcttc    1500

cccccaaaac ccaaggacac cctcatgatc tcccggaccc ctgaggtcac atgcgtggtg    1560

gtggacgtga gccacgaaga ccctgaggtc aagttcaact ggtacgtgga cggcgtggag    1620

gtgcataatg ccaagacaaa gccgcgggag gagcagtaca acagcacgta ccgtgtggtc    1680

agcgtcctca ccgtcctgca ccaggactgg ctgaatggca aggagtacaa gtgcaaggtc    1740

tccaacaaag ccctcccagc ccccatcgag aaaaccatct ccaaagccaa agggcagccc    1800

cgagaaccac aggtgtgcac cctgccccca tcccgggatg agctgaccaa gaaccaggtc    1860

agcctctcgt gcgcagtcaa aggcttctat cccagcgaca tcgccgtgga gtgggagagc 1920

aatgggcagc cggagaacaa ctacaagacc acgcctcccg tgctggactc cgacggctcc 1980

ttcttcctcg tgagcaagct caccgtggac aagagcaggt ggcagcaggg gaacgtcttc 2040

tcatgctccg tgatgcatga ggctctgcac aaccactaca cgcagaagag cctctccctg 2100

tctccgggta aatga 2115

<210> 9
<211> 466
<212> PRT
<213> Artificial Sequence

<220>
<223> V9 (VH-CH1) -Fc(knob) LALA

<400> 9

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Ser Phe Thr Gly Tyr
            20                  25                  30

Thr Met Asn Trp Val Arg Gln Ala Pro Gly Lys Gly Leu Glu Trp Val
        35                  40                  45

Ala Leu Ile Asn Pro Tyr Lys Gly Val Ser Thr Tyr Asn Gln Lys Phe
    50                  55                  60

Lys Asp Arg Phe Thr Ile Ser Val Asp Lys Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg Ser Gly Tyr Tyr Gly Asp Ser Asp Trp Tyr Phe Asp Val Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Gly Gly Gly Gly Ser Gly Gly Gly Gly Ser Gly Ala Gln Asp
225                 230                 235                 240

Lys Thr His Thr Cys Pro Pro Cys Pro Ala Pro Glu Ala Ala Gly Gly
                245                 250                 255

Pro Ser Val Phe Leu Phe Pro Pro Lys Pro Lys Asp Thr Leu Met Ile
            260                 265                 270

Ser Arg Thr Pro Glu Val Thr Cys Val Val Val Asp Val Ser His Glu
        275                 280                 285

Asp Pro Glu Val Lys Phe Asn Trp Tyr Val Asp Gly Val Glu Val His
    290                 295                 300

Asn Ala Lys Thr Lys Pro Arg Glu Glu Gln Tyr Asn Ser Thr Tyr Arg
305                 310                 315                 320

Val Val Ser Val Leu Thr Val Leu His Gln Asp Trp Leu Asn Gly Lys
                325                 330                 335

Glu Tyr Lys Cys Lys Val Ser Asn Lys Ala Leu Pro Ala Pro Ile Glu
            340                 345                 350

Lys Thr Ile Ser Lys Ala Lys Gly Gln Pro Arg Glu Pro Gln Val Tyr
        355                 360                 365

Thr Leu Pro Pro Cys Arg Asp Glu Leu Thr Lys Asn Gln Val Ser Leu
    370                 375                 380

Trp Cys Leu Val Lys Gly Phe Tyr Pro Ser Asp Ile Ala Val Glu Trp
385                 390                 395                 400

Glu Ser Asn Gly Gln Pro Glu Asn Asn Tyr Lys Thr Thr Pro Pro Val
                405                 410                 415

Leu Asp Ser Asp Gly Ser Phe Phe Leu Tyr Ser Lys Leu Thr Val Asp

420                 425                 430

Lys Ser Arg Trp Gln Gln Gly Asn Val Phe Ser Cys Ser Val Met His
        435                 440                 445

Glu Ala Leu His Asn His Tyr Thr Gln Lys Ser Leu Ser Leu Ser Pro
    450                 455                 460

Gly Lys
465

<210>  10
<211>  1401
<212>  DNA
<213>  Artificial Sequence

<220>
<223>  V9 (VH-CH1) -Fc(knob) LALA

<400>  10
gaggtgcagc tggtcgagag cggaggcggc ctggtgcagc ctggcggcag cctgagactg      60

agctgcgccg ccagcggcta cagcttcacc ggctacacca tgaactgggt ccggcaggca     120

cctggcaagg gactggaatg ggtggccctg atcaacccct acaagggcgt gagcacctac     180

aaccagaagt tcaaggaccg gttcaccatc agcgtggaca agagcaagaa caccgcctat     240

ctgcagatga acagcctgcg ggccgaggac accgccgtgt actactgcgc cagaagcggc     300

tactacggcg acagcgactg gtacttcgac gtgtggggcc agggcaccct cgtgaccgtg     360

tctagcgcta gcaccaaggg cccctccgtg ttccccctgg cccccagcag caagagcacc     420

agcggcggca cagccgctct gggctgcctg gtcaaggact acttccccga gcccgtgacc     480

gtgtcctgga acagcggagc cctgacctcc ggcgtgcaca ccttccccgc cgtgctgcag     540

agttctggcc tgtatagcct gagcagcgtg gtcaccgtgc cttctagcag cctgggcacc     600

cagacctaca tctgcaacgt gaaccacaag cccagcaaca ccaaggtgga caagaaggtg     660

gagcccaaga gctgcgacgg cggtggtggc tccggaggcg gtggatccgg agctcaggac     720

aaaactcaca catgcccacc gtgcccagca cctgaagctg cagggggacc gtcagtcttc     780

ctcttccccc caaaacccaa ggacaccctc atgatctccc ggacccctga ggtcacatgc     840

gtggtggtgg acgtgagcca cgaagaccct gaggtcaagt tcaactggta cgtggacggc     900

gtggaggtgc ataatgccaa gacaaagccg cgggaggagc agtacaacag cacgtaccgt     960

gtggtcagcg tcctcaccgt cctgcaccag gactggctga atggcaagga gtacaagtgc    1020

aaggtctcca acaaagccct cccagccccc atcgagaaaa ccatctccaa agccaaaggg    1080

cagccccgag aaccacaggt gtacaccctg cccccatgcc gggatgagct gaccaagaac    1140

caggtcagcc tgtggtgcct ggtcaaaggc ttctatccca gcgacatcgc cgtggagtgg    1200

gagagcaatg ggcagccgga gaacaactac aagaccacgc ctcccgtgct ggactccgac 1260

ggctccttct tcctctacag caagctcacc gtggacaaga gcaggtggca gcaggggaac 1320

gtcttctcat gctccgtgat gcatgaggct ctgcacaacc actacacgca gaagagcctc 1380

tccctgtctc cgggtaaatg a 1401

<210> 11
<211> 214
<212> PRT
<213> Artificial Sequence

<220>
<223> V9 (VL-CL)

<400> 11

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Arg Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Arg Leu Glu Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Gly Asn Thr Leu Pro Trp
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  12
<211>  645
<212>  DNA
<213>  Artificial Sequence

<220>
<223>  V9 (VL-CL)

<400>  12
gacatccaga tgacccagag ccccagcagc ctgagcgcca gcgtgggcga cagagtgacc      60

atcacctgtc gggccagcca ggacatcaga aactacctga actggtatca gcagaagccc     120

ggcaaggccc ccaagctgct gatctactac acctctagac tggaaagcgg cgtgcccagc     180

cggtttagcg gcagcggctc cggcaccgac tacaccctga ccatcagcag cctgcagccc     240

gaggacttcg ccacctacta ctgccagcag ggcaacacac tgccctggac cttcggccag     300

ggcacaaagg tggagatcaa gcgtacggtg gctgcaccat ctgtcttcat cttcccgcca     360

tctgatgagc agttgaaatc tggaactgcc tctgttgtgt gcctgctgaa taacttctat     420

cccagagagg ccaaagtaca gtggaaggtg gataacgccc tccaatcggg taactcccag     480

gagagtgtca cagagcagga cagcaaggac agcacctaca gcctcagcag caccctgacg     540

ctgagcaaag cagactacga gaaacacaaa gtctacgcct gcgaagtcac ccatcagggc     600

ctgagctcgc ccgtcacaaa gagcttcaac aggggagagt gttag                     645

<210>  13
<211>  926
<212>  PRT
<213>  Artificial Sequence

<220>
<223>  V9 (scFab)-LC007 (VH-CH1)-Fc(knob) wt

<400>  13

<210>  14
<211>  2781
<212>  DNA
<213>  Artificial Sequence

<220>
<223>  V9 (scFab)-LC007 (VH-CH1)-Fc(knob) wt

<400>  14
gacatccaga tgacccagag cccctctagc ctgagcgcca gcgtgggcga cagagtgacc      60

atcacctgtc gggccagcca ggacatcaga aactacctga actggtatca gcagaagccc     120

ggcaaggccc ccaagctgct gatctactac acctctagac tggaaagcgg cgtgcccagc     180

cggtttagcg gcagcggctc cggcaccgac tacaccctga ccatcagcag cctgcagccc     240

gaggacttcg ccacctacta ctgccagcag ggcaacacac tcccctggac cttcggccag     300

ggcaccaagg tggagatcaa gcgtacggtg gccgctccca gcgtgttcat cttccccccc     360

agcgacgagc agctgaagtc cggcaccgcc agcgtcgtgt gcctgctgaa caacttctac     420

ccccgggagg ccaaggtgca gtggaaggtg gacaacgccc tgcagagcgg caacagccag     480

gaaagcgtca ccgagcagga cagcaaggac tccacctaca gcctgagcag caccctgacc     540

ctgagcaagg ccgactacga gaagcacaag gtgtacgcct gcgaagtgac ccaccagggc     600

ctgtccagcc ccgtgaccaa gagcttcaac cggggcgagt gcagcggcgg aggctctgga 660

ggcggctctg aaggcggagg aagtgagggc ggaggctcag aaggcggcgg aagcgaaggt 720

ggcggctctg gcggcggatc cggcgaggtg cagctggtcg agtccggcgg aggcctggtg 780

cagcctggcg gcagcctgag actgagctgc gccgccagcg gctacagctt caccggctac 840

accatgaact gggtccggca ggctcctggc aagggcctcg aatgggtggc cctgatcaac 900

ccctacaagg gcgtgagcac ctacaaccag aagttcaagg accggttcac catcagcgtg 960

gacaagagca agaacaccgc ctatctgcag atgaacagcc tgcgggccga ggacaccgcc 1020

gtgtactact gcgccagaag cggctactac ggcgacagcg actggtactt cgacgtgtgg 1080

ggccagggca cactggtcac cgtgtccagc gctagcacca agggcccctc cgtgttcccc 1140

ctggccccca gcagcaagag caccagcggc ggcacagccg ccctcggctg cctggtcaag 1200

gactacttcc ccgagcccgt gaccgtgtcc tggaacagcg gagccctgac ctccggcgtg 1260

cacaccttcc ccgccgtgct gcagagcagc ggcctgtaca gcctgtccag cgtggtcacc 1320

gtgccctcca gcagcctggg cacccagacc tacatctgca acgtgaacca caagcccagc 1380

aataccaagg tggacaagaa ggtggagccc aagagctgcg acggcggtgg tggctccgga 1440

ggcggtggat ctgaagtgca gctgcaggaa agcggccctg gcctggtcaa gcccagccag 1500

agcctgagcc tgacctgtag cgtgaccggc tactccatca cctccggcta ctactggaat 1560

tggattcggc agttccccgg caacaagctg gaatggatgg gctacatcac ctacgacggc 1620

agcaacaact acaaccccag cctgaagaac cggatcagca tcacccggga caccagcaag 1680

aaccagttct tcctgaagtt gaattctgtg actactgagg acacagctac atattactgt 1740

gcggactttg actactgggg ccaaggcacc actctcacag tctcctcagc tagcaccaag 1800

ggcccatcgg tcttccccct ggcaccctcc tccaagagca cctctggggg cacagcggcc 1860

ctgggctgcc tggtcaagga ctacttcccc gaaccggtga cggtgtcgtg gaactcaggc 1920

gccctgacca gcggcgtgca caccttcccg gctgtcctac agtcctcagg actctactcc 1980

ctcagcagcg tggtgaccgt gccctccagc agcttgggca cccagaccta catctgcaac 2040

gtgaatcaca agcccagcaa caccaaggtg gacaagaaag ttgagcccaa atcttgtgac 2100

aaaactcaca catgcccacc gtgcccagca cctgaactcc tggggggacc gtcagtcttc 2160

ctcttccccc caaaacccaa ggacaccctc atgatctccc ggacccctga ggtcacatgc 2220

gtggtggtgg acgtgagcca cgaagaccct gaggtcaagt tcaactggta cgtggacggc 2280

gtggaggtgc ataatgccaa gacaaagccg cgggaggagc agtacaacag cacgtaccgt 2340

gtggtcagcg tcctcaccgt cctgcaccag gactggctga atggcaagga gtacaagtgc 2400

aaggtctcca acaaagccct cccagccccc atcgagaaaa ccatctccaa agccaaaggg 2460

cagccccgag aaccacaggt gtacaccctg cccccatgcc gggatgagct gaccaagaac 2520

caggtcagcc tgtggtgcct ggtcaaaggc ttctatccca gcgacatcgc cgtggagtgg 2580

gagagcaatg ggcagccgga gaacaactac aagaccacgc ctcccgtgct ggactccgac 2640

ggctccttct tcctctacag caagctcacc gtggacaaga gcaggtggca gcaggggaac 2700

gtcttctcat gctccgtgat gcatgaggct ctgcacaacc actacacgca gaagagcctc 2760

tccctgtctc cgggtaaatg a 2781

<210> 15
<211> 442
<212> PRT
<213> Artificial Sequence

<220>
<223> LC007 (VH-CH1)-Fc(hole) wt

<400> 15

Glu Val Gln Leu Gln Glu Ser Gly Pro Gly Leu Val Lys Pro Ser Gln
1               5                   10                  15

Ser Leu Ser Leu Thr Cys Ser Val Thr Gly Tyr Ser Ile Thr Ser Gly
            20                  25                  30

Tyr Tyr Trp Asn Trp Ile Arg Gln Phe Pro Gly Asn Lys Leu Glu Trp
        35                  40                  45

Met Gly Tyr Ile Thr Tyr Asp Gly Ser Asn Asn Tyr Asn Pro Ser Leu
    50                  55                  60

Lys Asn Arg Ile Ser Ile Thr Arg Asp Thr Ser Lys Asn Gln Phe Phe
65                  70                  75                  80

Leu Lys Leu Asn Ser Val Thr Thr Glu Asp Thr Ala Thr Tyr Tyr Cys
                85                  90                  95

Ala Asp Phe Asp Tyr Trp Gly Gln Gly Thr Thr Leu Thr Val Ser Ser
            100                 105                 110

Ala Ser Thr Lys Gly Pro Ser Val Phe Pro Leu Ala Pro Ser Ser Lys
        115                 120                 125

Ser Thr Ser Gly Gly Thr Ala Ala Leu Gly Cys Leu Val Lys Asp Tyr
    130                 135                 140

Phe Pro Glu Pro Val Thr Val Ser Trp Asn Ser Gly Ala Leu Thr Ser
145                 150                 155                 160

Gly Val His Thr Phe Pro Ala Val Leu Gln Ser Ser Gly Leu Tyr Ser
                165                 170                 175

Leu Ser Ser Val Val Thr Val Pro Ser Ser Ser Leu Gly Thr Gln Thr
            180                 185                 190

Tyr Ile Cys Asn Val Asn His Lys Pro Ser Asn Thr Lys Val Asp Lys
        195                 200                 205

Lys Val Glu Pro Lys Ser Cys Asp Lys Thr His Thr Cys Pro Pro Cys
    210                 215                 220

Pro Ala Pro Glu Leu Leu Gly Gly Pro Ser Val Phe Leu Phe Pro Pro
225                 230                 235                 240

Lys Pro Lys Asp Thr Leu Met Ile Ser Arg Thr Pro Glu Val Thr Cys
                245                 250                 255

Val Val Val Asp Val Ser His Glu Asp Pro Glu Val Lys Phe Asn Trp
            260                 265                 270

Tyr Val Asp Gly Val Glu Val His Asn Ala Lys Thr Lys Pro Arg Glu
        275                 280                 285

Glu Gln Tyr Asn Ser Thr Tyr Arg Val Val Ser Val Leu Thr Val Leu
    290                 295                 300

His Gln Asp Trp Leu Asn Gly Lys Glu Tyr Lys Cys Lys Val Ser Asn
305                 310                 315                 320

Lys Ala Leu Pro Ala Pro Ile Glu Lys Thr Ile Ser Lys Ala Lys Gly
                325                 330                 335

Gln Pro Arg Glu Pro Gln Val Cys Thr Leu Pro Pro Ser Arg Asp Glu
            340                 345                 350

Leu Thr Lys Asn Gln Val Ser Leu Ser Cys Ala Val Lys Gly Phe Tyr
        355                 360                 365

Pro Ser Asp Ile Ala Val Glu Trp Glu Ser Asn Gly Gln Pro Glu Asn
    370                 375                 380

Asn Tyr Lys Thr Thr Pro Pro Val Leu Asp Ser Asp Gly Ser Phe Phe
385                 390                 395                 400

Leu Val Ser Lys Leu Thr Val Asp Lys Ser Arg Trp Gln Gln Gly Asn
                405                 410                 415

Val Phe Ser Cys Ser Val Met His Glu Ala Leu His Asn His Tyr Thr
            420                 425                 430

Gln Lys Ser Leu Ser Leu Ser Pro Gly Lys
        435                 440

<210>  16
<211>  1329
<212>  DNA
<213>  Artificial Sequence

<220>
<223>  LC007 (VH-CH1)-Fc(hole) wt

<400>  16
gaggtccagc tgcaggagtc aggacctggc ctcgtgaaac cttctcagtc tctgtctctc      60

acctgctctg tcactggcta ctccatcacc agtggttatt actggaactg gatccggcag     120

tttccaggaa acaagctgga atggatgggc tacataacct acgacggtag caataactac     180

aacccatctc tcaaaaatcg aatctccatc actcgtgaca catctaagaa ccagtttttc     240

ctgaagttga attctgtgac tactgaggac acagctacat attactgtgc ggactttgac     300

tactggggcc aaggcaccac tctcacagtc tcctcagcta gcaccaaggg cccaagcgtg     360

ttccctctgg cccccagcag caagagcaca agcggcggaa cagccgccct gggctgcctg     420

gtcaaggact acttccccga gcccgtgaca gtgtcctgga acagcggagc cctgaccagc     480

ggcgtgcaca cctttccagc cgtgctgcag agcagcggcc tgtacagcct gagcagcgtg     540

gtcacagtgc ctagcagcag cctgggcacc cagacctaca tctgcaacgt gaaccacaag     600

cccagcaaca ccaaggtgga caagaaggtg gagcccaaga gctgcgacaa gacccacacc     660

tgtccccctt gtcctgcccc tgagctgctg ggcggaccca gcgtgttcct gttcccccca     720

aagcccaagg acaccctgat gatcagccgg acccccgaag tgacctgcgt ggtggtggac     780

gtgtcccacg aggaccctga agtgaagttc aattggtacg tggacggcgt ggaggtgcac     840

aatgccaaga ccaagccccg ggaggaacag tacaacagca cctaccgggt ggtgtccgtg     900

ctgaccgtgc tgcaccagga ctggctgaac ggcaaagagt acaagtgcaa ggtctccaac     960

aaggccctgc ctgcccccat cgagaaaacc atcagcaagg ccaagggcca gcccagagaa    1020

ccccaggtgt gcaccctgcc ccccagcaga gatgagctga ccaagaacca ggtgtccctg    1080

agctgtgccg tcaagggctt ctaccccagc gatatcgccg tggagtggga gagcaacggc    1140

cagcctgaga acaactacaa gaccaccccc cctgtgctgg acagcgacgg cagcttcttc    1200

ctggtgtcca aactgaccgt ggacaagagc cggtggcagc agggcaacgt gttcagctgc    1260

agcgtgatgc acgaggccct gcacaaccac tacacccaga agtccctgag cctgagcccc    1320

ggcaagtga                                                            1329

<210>  17
<211>  926
<212>  PRT
<213>  Artificial Sequence

<220>
<223>  V9 (scFab)-LC007 (VH-CH1)-Fc(knob) LALA

<400>  17

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Arg Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Arg Leu Glu Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Gly Asn Thr Leu Pro Trp
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys Ser Gly Gly Gly Ser Gly Gly Gly Ser Glu
    210                 215                 220

Gly Gly Gly Ser Glu Gly Gly Gly Ser Glu Gly Gly Gly Ser Glu Gly
225                 230                 235                 240

Gly Gly Ser Gly Gly Gly Ser Gly Glu Val Gln Leu Val Glu Ser Gly
                245                 250                 255

Gly Gly Leu Val Gln Pro Gly Gly Ser Leu Arg Leu Ser Cys Ala Ala
            260                 265                 270

Ser Gly Tyr Ser Phe Thr Gly Tyr Thr Met Asn Trp Val Arg Gln Ala
        275                 280                 285

Pro Gly Lys Gly Leu Glu Trp Val Ala Leu Ile Asn Pro Tyr Lys Gly
    290                 295                 300

Val Ser Thr Tyr Asn Gln Lys Phe Lys Asp Arg Phe Thr Ile Ser Val
305                 310                 315                 320

Asp Lys Ser Lys Asn Thr Ala Tyr Leu Gln Met Asn Ser Leu Arg Ala
                325                 330                 335

Glu Asp Thr Ala Val Tyr Tyr Cys Ala Arg Ser Gly Tyr Tyr Gly Asp
            340                 345                 350

Ser Asp Trp Tyr Phe Asp Val Trp Gly Gln Gly Thr Leu Val Thr Val
        355                 360                 365

Ser Ser Ala Ser Thr Lys Gly Pro Ser Val Phe Pro Leu Ala Pro Ser
    370                 375                 380

Ser Lys Ser Thr Ser Gly Gly Thr Ala Ala Leu Gly Cys Leu Val Lys
385                 390                 395                 400

Asp Tyr Phe Pro Glu Pro Val Thr Val Ser Trp Asn Ser Gly Ala Leu
                405                 410                 415

Thr Ser Gly Val His Thr Phe Pro Ala Val Leu Gln Ser Ser Gly Leu
            420                 425                 430

Tyr Ser Leu Ser Ser Val Val Thr Val Pro Ser Ser Ser Leu Gly Thr
        435                 440                 445

Gln Thr Tyr Ile Cys Asn Val Asn His Lys Pro Ser Asn Thr Lys Val
    450                 455                 460

Asp Lys Lys Val Glu Pro Lys Ser Cys Asp Gly Gly Gly Gly Ser Gly
465                 470                 475                 480

Gly Gly Gly Ser Glu Val Gln Leu Gln Glu Ser Gly Pro Gly Leu Val
                485                 490                 495

Lys Pro Ser Gln Ser Leu Ser Leu Thr Cys Ser Val Thr Gly Tyr Ser
            500                 505                 510

Ile Thr Ser Gly Tyr Tyr Trp Asn Trp Ile Arg Gln Phe Pro Gly Asn
        515                 520                 525

Lys Leu Glu Trp Met Gly Tyr Ile Thr Tyr Asp Gly Ser Asn Asn Tyr
    530                 535                 540

Asn Pro Ser Leu Lys Asn Arg Ile Ser Ile Thr Arg Asp Thr Ser Lys
545                 550                 555                 560

Asn Gln Phe Phe Leu Lys Leu Asn Ser Val Thr Thr Glu Asp Thr Ala
                565                 570                 575

Thr Tyr Tyr Cys Ala Asp Phe Asp Tyr Trp Gly Gln Gly Thr Thr Leu
            580                 585                 590

Thr Val Ser Ser Ala Ser Thr Lys Gly Pro Ser Val Phe Pro Leu Ala
        595                 600                 605

Pro Ser Ser Lys Ser Thr Ser Gly Gly Thr Ala Ala Leu Gly Cys Leu
    610                 615                 620

Val Lys Asp Tyr Phe Pro Glu Pro Val Thr Val Ser Trp Asn Ser Gly
625                 630                 635                 640

Ala Leu Thr Ser Gly Val His Thr Phe Pro Ala Val Leu Gln Ser Ser
                645                 650                 655

Gly Leu Tyr Ser Leu Ser Ser Val Val Thr Val Pro Ser Ser Ser Leu
            660                 665                 670

Gly Thr Gln Thr Tyr Ile Cys Asn Val Asn His Lys Pro Ser Asn Thr
        675                 680                 685

Lys Val Asp Lys Lys Val Glu Pro Lys Ser Cys Asp Lys Thr His Thr
    690                 695                 700

Cys Pro Pro Cys Pro Ala Pro Glu Ala Ala Gly Gly Pro Ser Val Phe

705                 710                 715                 720

Leu Phe Pro Pro Lys Pro Lys Asp Thr Leu Met Ile Ser Arg Thr Pro
                725                 730                 735

Glu Val Thr Cys Val Val Val Asp Val Ser His Glu Asp Pro Glu Val
            740                 745                 750

Lys Phe Asn Trp Tyr Val Asp Gly Val Glu Val His Asn Ala Lys Thr
        755                 760                 765

Lys Pro Arg Glu Glu Gln Tyr Asn Ser Thr Tyr Arg Val Val Ser Val
    770                 775                 780

Leu Thr Val Leu His Gln Asp Trp Leu Asn Gly Lys Glu Tyr Lys Cys
785                 790                 795                 800

Lys Val Ser Asn Lys Ala Leu Pro Ala Pro Ile Glu Lys Thr Ile Ser
                805                 810                 815

Lys Ala Lys Gly Gln Pro Arg Glu Pro Gln Val Tyr Thr Leu Pro Pro
            820                 825                 830

Cys Arg Asp Glu Leu Thr Lys Asn Gln Val Ser Leu Trp Cys Leu Val
        835                 840                 845

Lys Gly Phe Tyr Pro Ser Asp Ile Ala Val Glu Trp Glu Ser Asn Gly
    850                 855                 860

Gln Pro Glu Asn Asn Tyr Lys Thr Thr Pro Pro Val Leu Asp Ser Asp
865                 870                 875                 880

Gly Ser Phe Phe Leu Tyr Ser Lys Leu Thr Val Asp Lys Ser Arg Trp
                885                 890                 895

Gln Gln Gly Asn Val Phe Ser Cys Ser Val Met His Glu Ala Leu His
            900                 905                 910

Asn His Tyr Thr Gln Lys Ser Leu Ser Leu Ser Pro Gly Lys
        915                 920                 925

<210>  18
<211>  2781
<212>  DNA
<213>  Artificial Sequence

<220>
<223>  V9 (scFab)-LC007 (VH-CH1)-Fc(knob) LALA

<400>  18
gacatccaga tgacccagag cccctctagc ctgagcgcca gcgtgggcga cagagtgacc      60

atcacctgtc gggccagcca ggacatcaga aactacctga actggtatca gcagaagccc     120

ggcaaggccc ccaagctgct gatctactac acctctagac tggaaagcgg cgtgcccagc     180

cggtttagcg gcagcggctc cggcaccgac tacaccctga ccatcagcag cctgcagccc     240

gaggacttcg ccacctacta ctgccagcag ggcaacacac tcccctggac cttcggccag     300

ggcaccaagg tggagatcaa gcgtacggtg gccgctccca gcgtgttcat cttccccccc     360

agcgacgagc agctgaagtc cggcaccgcc agcgtcgtgt gcctgctgaa caacttctac     420

ccccgggagg ccaaggtgca gtggaaggtg gacaacgccc tgcagagcgg caacagccag     480

gaaagcgtca ccgagcagga cagcaaggac tccacctaca gcctgagcag caccctgacc     540

ctgagcaagg ccgactacga gaagcacaag gtgtacgcct gcgaagtgac ccaccagggc     600

ctgtccagcc ccgtgaccaa gagcttcaac cggggcgagt gcagcggcgg aggctctgga     660

ggcggctctg aaggcggagg aagtgagggc ggaggctcag aaggcggcgg aagcgaaggt     720

ggcggctctg gcggcggatc cggcgaggtg cagctggtcg agtccggcgg aggcctggtg     780

cagcctggcg gcagcctgag actgagctgc gccgccagcg gctacagctt caccggctac     840

accatgaact gggtccggca ggctcctggc aagggcctcg aatgggtggc cctgatcaac     900

ccctacaagg gcgtgagcac ctacaaccag aagttcaagg accggttcac catcagcgtg     960

gacaagagca agaacaccgc ctatctgcag atgaacagcc tgcgggccga ggacaccgcc    1020

gtgtactact gcgccagaag cggctactac ggcgacagcg actggtactt cgacgtgtgg    1080

ggccagggca cactggtcac cgtgtccagc gctagcacca agggcccctc cgtgttcccc    1140

ctggccccca gcagcaagag caccagcggc ggcacagccg ccctcggctg cctggtcaag    1200

gactacttcc ccgagcccgt gaccgtgtcc tggaacagcg gagccctgac ctccggcgtg    1260

cacaccttcc ccgccgtgct gcagagcagc ggcctgtaca gcctgtccag cgtggtcacc    1320

gtgccctcca gcagcctggg cacccagacc tacatctgca acgtgaacca caagcccagc    1380

aataccaagg tggacaagaa ggtggagccc aagagctgcg acggcggtgg tggctccgga    1440

ggcggtggat ctgaagtgca gctgcaggaa agcggccctg gcctggtcaa gcccagccag    1500

agcctgagcc tgacctgtag cgtgaccggc tactccatca cctccggcta ctactggaat    1560

tggattcggc agttccccgg caacaagctg gaatggatgg gctacatcac ctacgacggc    1620

agcaacaact acaaccccag cctgaagaac cggatcagca tcacccggga caccagcaag    1680

aaccagttct tcctgaagtt gaattctgtg actactgagg acacagctac atattactgt    1740

gcggactttg actactgggg ccaaggcacc actctcacag tctcctcagc tagcaccaag    1800

ggcccatcgg tcttccccct ggcaccctcc tccaagagca cctctggggg cacagcggcc    1860

ctgggctgcc tggtcaagga ctacttcccc gaaccggtga cggtgtcgtg gaactcaggc 1920

gccctgacca gcggcgtgca caccttcccg gctgtcctac agtcctcagg actctactcc 1980

ctcagcagcg tggtgaccgt gccctccagc agcttgggca cccagaccta catctgcaac 2040

gtgaatcaca agcccagcaa caccaaggtg gacaagaaag ttgagcccaa atcttgtgac 2100

aaaactcaca catgcccacc gtgcccagca cctgaagctg cagggggacc gtcagtcttc 2160

ctcttccccc caaaacccaa ggacaccctc atgatctccc ggacccctga ggtcacatgc 2220

gtggtggtgg acgtgagcca cgaagaccct gaggtcaagt tcaactggta cgtggacggc 2280

gtggaggtgc ataatgccaa gacaaagccg cgggaggagc agtacaacag cacgtaccgt 2340

gtggtcagcg tcctcaccgt cctgcaccag gactggctga atggcaagga gtacaagtgc 2400

aaggtctcca acaaagccct cccagccccc atcgagaaaa ccatctccaa agccaaaggg 2460

cagccccgag aaccacaggt gtacaccctg cccccatgcc gggatgagct gaccaagaac 2520

caggtcagcc tgtggtgcct ggtcaaaggc ttctatccca gcgacatcgc cgtggagtgg 2580

gagagcaatg ggcagccgga gaacaactac aagaccacgc ctcccgtgct ggactccgac 2640

ggctccttct tcctctacag caagctcacc gtggacaaga gcaggtggca gcaggggaac 2700

gtcttctcat gctccgtgat gcatgaggct ctgcacaacc actacacgca gaagagcctc 2760

tccctgtctc cgggtaaatg a 2781

<210> 19
<211> 442
<212> PRT
<213> Artificial Sequence

<220>
<223> LC007 (VH-CH1)-Fc(hole) LALA

<400> 19

Glu Val Gln Leu Gln Glu Ser Gly Pro Gly Leu Val Lys Pro Ser Gln
1               5                   10                  15

Ser Leu Ser Leu Thr Cys Ser Val Thr Gly Tyr Ser Ile Thr Ser Gly
            20                  25                  30

Tyr Tyr Trp Asn Trp Ile Arg Gln Phe Pro Gly Asn Lys Leu Glu Trp
        35                  40                  45

Met Gly Tyr Ile Thr Tyr Asp Gly Ser Asn Asn Tyr Asn Pro Ser Leu
    50                  55                  60

Lys Asn Arg Ile Ser Ile Thr Arg Asp Thr Ser Lys Asn Gln Phe Phe
65                  70                  75                  80

Leu Lys Leu Asn Ser Val Thr Thr Glu Asp Thr Ala Thr Tyr Tyr Cys
                85                  90                  95

Ala Asp Phe Asp Tyr Trp Gly Gln Gly Thr Thr Leu Thr Val Ser Ser
            100                 105                 110

Ala Ser Thr Lys Gly Pro Ser Val Phe Pro Leu Ala Pro Ser Ser Lys
        115                 120                 125

Ser Thr Ser Gly Gly Thr Ala Ala Leu Gly Cys Leu Val Lys Asp Tyr
    130                 135                 140

Phe Pro Glu Pro Val Thr Val Ser Trp Asn Ser Gly Ala Leu Thr Ser
145                 150                 155                 160

Gly Val His Thr Phe Pro Ala Val Leu Gln Ser Ser Gly Leu Tyr Ser
                165                 170                 175

Leu Ser Ser Val Val Thr Val Pro Ser Ser Ser Leu Gly Thr Gln Thr
            180                 185                 190

Tyr Ile Cys Asn Val Asn His Lys Pro Ser Asn Thr Lys Val Asp Lys
        195                 200                 205

Lys Val Glu Pro Lys Ser Cys Asp Lys Thr His Thr Cys Pro Pro Cys
    210                 215                 220

Pro Ala Pro Glu Ala Ala Gly Gly Pro Ser Val Phe Leu Phe Pro Pro
225                 230                 235                 240

Lys Pro Lys Asp Thr Leu Met Ile Ser Arg Thr Pro Glu Val Thr Cys
                245                 250                 255

Val Val Val Asp Val Ser His Glu Asp Pro Glu Val Lys Phe Asn Trp
            260                 265                 270

Tyr Val Asp Gly Val Glu Val His Asn Ala Lys Thr Lys Pro Arg Glu
        275                 280                 285

Glu Gln Tyr Asn Ser Thr Tyr Arg Val Val Ser Val Leu Thr Val Leu
    290                 295                 300

His Gln Asp Trp Leu Asn Gly Lys Glu Tyr Lys Cys Lys Val Ser Asn
305                 310                 315                 320

Lys Ala Leu Pro Ala Pro Ile Glu Lys Thr Ile Ser Lys Ala Lys Gly
                325                 330                 335

Gln Pro Arg Glu Pro Gln Val Cys Thr Leu Pro Pro Ser Arg Asp Glu
            340                 345                 350

Leu Thr Lys Asn Gln Val Ser Leu Ser Cys Ala Val Lys Gly Phe Tyr
        355                 360                 365

Pro Ser Asp Ile Ala Val Glu Trp Glu Ser Asn Gly Gln Pro Glu Asn
    370                 375                 380

Asn Tyr Lys Thr Thr Pro Pro Val Leu Asp Ser Asp Gly Ser Phe Phe
385                 390                 395                 400

Leu Val Ser Lys Leu Thr Val Asp Lys Ser Arg Trp Gln Gln Gly Asn
                405                 410                 415

Val Phe Ser Cys Ser Val Met His Glu Ala Leu His Asn His Tyr Thr
            420                 425                 430

Gln Lys Ser Leu Ser Leu Ser Pro Gly Lys
        435                 440

<210>  20
<211>  1329
<212>  DNA
<213>  Artificial Sequence

<220>
<223>  LC007 (VH-CH1)-Fc(hole) LALA

<400>  20
gaggtccagc tgcaggagtc aggacctggc ctcgtgaaac cttctcagtc tctgtctctc      60

acctgctctg tcactggcta ctccatcacc agtggttatt actggaactg gatccggcag     120

tttccaggaa acaagctgga atggatgggc tacataacct acgacggtag caataactac     180

aacccatctc tcaaaaatcg aatctccatc actcgtgaca catctaagaa ccagtttttc     240

ctgaagttga attctgtgac tactgaggac acagctacat attactgtgc ggactttgac     300

tactggggcc aaggcaccac tctcacagtc tcctcagcta gcaccaaggg cccatcggtc     360

ttccccctgg caccctcctc caagagcacc tctgggggca cagcggccct gggctgcctg     420

gtcaaggact acttccccga accggtgacg gtgtcgtgga actcaggcgc cctgaccagc     480

ggcgtgcaca ccttcccggc tgtcctacag tcctcaggac tctactccct cagcagcgtg     540

gtgaccgtgc cctccagcag cttgggcacc cagacctaca tctgcaacgt gaatcacaag     600

cccagcaaca ccaaggtgga caagaaagtt gagcccaaat cttgtgacaa aactcacaca     660

tgcccaccgt gcccagcacc tgaagctgca gggggaccgt cagtcttcct cttcccccca     720

aaacccaagg acaccctcat gatctcccgg acccctgagg tcacatgcgt ggtggtggac     780

gtgagccacg aagaccctga ggtcaagttc aactggtacg tggacggcgt ggaggtgcat 840

aatgccaaga caaagccgcg ggaggagcag tacaacagca cgtaccgtgt ggtcagcgtc 900

ctcaccgtcc tgcaccagga ctggctgaat ggcaaggagt acaagtgcaa ggtctccaac 960

aaagccctcc cagcccccat cgagaaaacc atctccaaag ccaaagggca gccccgagaa 1020

ccacaggtgt gcaccctgcc cccatcccgg gatgagctga ccaagaacca ggtcagcctc 1080

tcgtgcgcag tcaaaggctt ctatcccagc gacatcgccg tggagtggga gagcaatggg 1140

cagccggaga acaactacaa gaccacgcct cccgtgctgg actccgacgg ctccttcttc 1200

ctcgtgagca agctcaccgt ggacaagagc aggtggcagc aggggaacgt cttctcatgc 1260

tccgtgatgc atgaggctct gcacaaccac tacacgcaga agagcctctc cctgtctccg 1320

ggtaaatga 1329

<210> 21
<211> 926
<212> PRT
<213> Artificial Sequence

<220>
<223> V9 (scFab)-LC007 (VH-CH1)-Fc(knob) P329G LALA

<400> 21

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Arg Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Arg Leu Glu Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Gly Asn Thr Leu Pro Trp
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys Ser Gly Gly Gly Ser Gly Gly Gly Ser Glu
    210                 215                 220

Gly Gly Gly Ser Glu Gly Gly Gly Ser Glu Gly Gly Gly Ser Glu Gly
225                 230                 235                 240

Gly Gly Ser Gly Gly Gly Ser Gly Glu Val Gln Leu Val Glu Ser Gly
                245                 250                 255

Gly Gly Leu Val Gln Pro Gly Gly Ser Leu Arg Leu Ser Cys Ala Ala
            260                 265                 270

Ser Gly Tyr Ser Phe Thr Gly Tyr Thr Met Asn Trp Val Arg Gln Ala
        275                 280                 285

Pro Gly Lys Gly Leu Glu Trp Val Ala Leu Ile Asn Pro Tyr Lys Gly
    290                 295                 300

Val Ser Thr Tyr Asn Gln Lys Phe Lys Asp Arg Phe Thr Ile Ser Val
305                 310                 315                 320

Asp Lys Ser Lys Asn Thr Ala Tyr Leu Gln Met Asn Ser Leu Arg Ala
                325                 330                 335

Glu Asp Thr Ala Val Tyr Tyr Cys Ala Arg Ser Gly Tyr Tyr Gly Asp
            340                 345                 350

Ser Asp Trp Tyr Phe Asp Val Trp Gly Gln Gly Thr Leu Val Thr Val
        355                 360                 365

Ser Ser Ala Ser Thr Lys Gly Pro Ser Val Phe Pro Leu Ala Pro Ser
    370                 375                 380

Ser Lys Ser Thr Ser Gly Gly Thr Ala Ala Leu Gly Cys Leu Val Lys
385                 390                 395                 400

Asp Tyr Phe Pro Glu Pro Val Thr Val Ser Trp Asn Ser Gly Ala Leu
                405                 410                 415

Thr Ser Gly Val His Thr Phe Pro Ala Val Leu Gln Ser Ser Gly Leu
            420                 425                 430

Tyr Ser Leu Ser Ser Val Val Thr Val Pro Ser Ser Ser Leu Gly Thr
        435                 440                 445

Gln Thr Tyr Ile Cys Asn Val Asn His Lys Pro Ser Asn Thr Lys Val
    450                 455                 460

Asp Lys Lys Val Glu Pro Lys Ser Cys Asp Gly Gly Gly Gly Ser Gly
465                 470                 475                 480

Gly Gly Gly Ser Glu Val Gln Leu Gln Glu Ser Gly Pro Gly Leu Val
                485                 490                 495

Lys Pro Ser Gln Ser Leu Ser Leu Thr Cys Ser Val Thr Gly Tyr Ser
            500                 505                 510

Ile Thr Ser Gly Tyr Tyr Trp Asn Trp Ile Arg Gln Phe Pro Gly Asn
        515                 520                 525

Lys Leu Glu Trp Met Gly Tyr Ile Thr Tyr Asp Gly Ser Asn Asn Tyr
    530                 535                 540

Asn Pro Ser Leu Lys Asn Arg Ile Ser Ile Thr Arg Asp Thr Ser Lys
545                 550                 555                 560

Asn Gln Phe Phe Leu Lys Leu Asn Ser Val Thr Thr Glu Asp Thr Ala
                565                 570                 575

Thr Tyr Tyr Cys Ala Asp Phe Asp Tyr Trp Gly Gln Gly Thr Thr Leu
            580                 585                 590

Thr Val Ser Ser Ala Ser Thr Lys Gly Pro Ser Val Phe Pro Leu Ala
        595                 600                 605

Pro Ser Ser Lys Ser Thr Ser Gly Gly Thr Ala Ala Leu Gly Cys Leu
    610                 615                 620

Val Lys Asp Tyr Phe Pro Glu Pro Val Thr Val Ser Trp Asn Ser Gly

625                 630                 635                 640

Ala Leu Thr Ser Gly Val His Thr Phe Pro Ala Val Leu Gln Ser Ser
                645                 650                 655

Gly Leu Tyr Ser Leu Ser Ser Val Val Thr Val Pro Ser Ser Ser Leu
            660                 665                 670

Gly Thr Gln Thr Tyr Ile Cys Asn Val Asn His Lys Pro Ser Asn Thr
        675                 680                 685

Lys Val Asp Lys Lys Val Glu Pro Lys Ser Cys Asp Lys Thr His Thr
    690                 695                 700

Cys Pro Pro Cys Pro Ala Pro Glu Ala Ala Gly Gly Pro Ser Val Phe
705                 710                 715                 720

Leu Phe Pro Pro Lys Pro Lys Asp Thr Leu Met Ile Ser Arg Thr Pro
                725                 730                 735

Glu Val Thr Cys Val Val Val Asp Val Ser His Glu Asp Pro Glu Val
            740                 745                 750

Lys Phe Asn Trp Tyr Val Asp Gly Val Glu Val His Asn Ala Lys Thr
        755                 760                 765

Lys Pro Arg Glu Glu Gln Tyr Asn Ser Thr Tyr Arg Val Val Ser Val
    770                 775                 780

Leu Thr Val Leu His Gln Asp Trp Leu Asn Gly Lys Glu Tyr Lys Cys
785                 790                 795                 800

Lys Val Ser Asn Lys Ala Leu Gly Ala Pro Ile Glu Lys Thr Ile Ser
                805                 810                 815

Lys Ala Lys Gly Gln Pro Arg Glu Pro Gln Val Tyr Thr Leu Pro Pro
            820                 825                 830

Cys Arg Asp Glu Leu Thr Lys Asn Gln Val Ser Leu Trp Cys Leu Val
        835                 840                 845

Lys Gly Phe Tyr Pro Ser Asp Ile Ala Val Glu Trp Glu Ser Asn Gly
    850                 855                 860

Gln Pro Glu Asn Asn Tyr Lys Thr Thr Pro Pro Val Leu Asp Ser Asp
865                 870                 875                 880

Gly Ser Phe Phe Leu Tyr Ser Lys Leu Thr Val Asp Lys Ser Arg Trp
                885                 890                 895

Gln Gln Gly Asn Val Phe Ser Cys Ser Val Met His Glu Ala Leu His
            900                 905                 910

Asn His Tyr Thr Gln Lys Ser Leu Ser Leu Ser Pro Gly Lys
        915                 920                 925

<210>  22
<211>  2781
<212>  DNA
<213>  Artificial Sequence

<220>
<223>  V9 (scFab)-LC007 (VH-CH1)-Fc(knob) P329G LALA

<400>  22
gacatccaga tgacccagag cccctctagc ctgagcgcca gcgtgggcga cagagtgacc      60

atcacctgtc gggccagcca ggacatcaga aactacctga actggtatca gcagaagccc     120

ggcaaggccc ccaagctgct gatctactac acctctagac tggaaagcgg cgtgcccagc     180

cggtttagcg gcagcggctc cggcaccgac tacaccctga ccatcagcag cctgcagccc     240

gaggacttcg ccacctacta ctgccagcag ggcaacacac tcccctggac cttcggccag     300

ggcaccaagg tggagatcaa gcgtacggtg gccgctccca gcgtgttcat cttccccccc     360

agcgacgagc agctgaagtc cggcaccgcc agcgtcgtgt gcctgctgaa caacttctac     420

ccccgggagg ccaaggtgca gtggaaggtg gacaacgccc tgcagagcgg caacagccag     480

gaaagcgtca ccgagcagga cagcaaggac tccacctaca gcctgagcag caccctgacc     540

ctgagcaagg ccgactacga gaagcacaag gtgtacgcct gcgaagtgac ccaccagggc     600

ctgtccagcc ccgtgaccaa gagcttcaac cggggcgagt gcagcggcgg aggctctgga     660

ggcggctctg aaggcggagg aagtgagggc ggaggctcag aaggcggcgg aagcgaaggt     720

ggcggctctg gcggcggatc cggcgaggtg cagctggtcg agtccggcgg aggcctggtg     780

cagcctggcg gcagcctgag actgagctgc gccgccagcg gctacagctt caccggctac     840

accatgaact gggtccggca ggctcctggc aagggcctcg aatgggtggc cctgatcaac     900

ccctacaagg gcgtgagcac ctacaaccag aagttcaagg accggttcac catcagcgtg     960

gacaagagca agaacaccgc ctatctgcag atgaacagcc tgcgggccga ggacaccgcc    1020

gtgtactact gcgccagaag cggctactac ggcgacagcg actggtactt cgacgtgtgg    1080

ggccagggca cactggtcac cgtgtccagc gctagcacca agggcccctc cgtgttcccc    1140

ctggccccca gcagcaagag caccagcggc ggcacagccg ccctcggctg cctggtcaag    1200

gactacttcc ccgagcccgt gaccgtgtcc tggaacagcg gagccctgac ctccggcgtg    1260

cacaccttcc ccgccgtgct gcagagcagc ggcctgtaca gcctgtccag cgtggtcacc 1320

gtgccctcca gcagcctggg cacccagacc tacatctgca acgtgaacca caagcccagc 1380

aataccaagg tggacaagaa ggtggagccc aagagctgcg acggcggtgg tggctccgga 1440

ggcggtggat ctgaagtgca gctgcaggaa agcggccctg gcctggtcaa gcccagccag 1500

agcctgagcc tgacctgtag cgtgaccggc tactccatca cctccggcta ctactggaat 1560

tggattcggc agttccccgg caacaagctg gaatggatgg gctacatcac ctacgacggc 1620

agcaacaact acaaccccag cctgaagaac cggatcagca tcacccggga caccagcaag 1680

aaccagttct tcctgaagtt gaattctgtg actactgagg acacagctac atattactgt 1740

gcggactttg actactgggg ccaaggcacc actctcacag tctcctcagc tagcaccaag 1800

ggcccatcgg tcttccccct ggcaccctcc tccaagagca cctctggggg cacagcggcc 1860

ctgggctgcc tggtcaagga ctacttcccc gaaccggtga cggtgtcgtg gaactcaggc 1920

gccctgacca gcggcgtgca caccttcccg gctgtcctac agtcctcagg actctactcc 1980

ctcagcagcg tggtgaccgt gccctccagc agcttgggca cccagaccta catctgcaac 2040

gtgaatcaca agcccagcaa caccaaggtg gacaagaaag ttgagcccaa atcttgtgac 2100

aaaactcaca catgcccacc gtgcccagca cctgaagctg cagggggacc gtcagtcttc 2160

ctcttccccc caaaacccaa ggacaccctc atgatctccc ggacccctga ggtcacatgc 2220

gtggtggtgg acgtgagcca cgaagaccct gaggtcaagt tcaactggta cgtggacggc 2280

gtggaggtgc ataatgccaa gacaaagccg cgggaggagc agtacaacag cacgtaccgt 2340

gtggtcagcg tcctcaccgt cctgcaccag gactggctga atggcaagga gtacaagtgc 2400

aaggtctcca acaaagccct cggcgccccc atcgagaaaa ccatctccaa agccaaaggg 2460

cagccccgag aaccacaggt gtacaccctg cccccatgcc gggatgagct gaccaagaac 2520

caggtcagcc tgtggtgcct ggtcaaaggc ttctatccca gcgacatcgc cgtggagtgg 2580

gagagcaatg ggcagccgga gaacaactac aagaccacgc ctcccgtgct ggactccgac 2640

ggctccttct tcctctacag caagctcacc gtggacaaga gcaggtggca gcaggggaac 2700

gtcttctcat gctccgtgat gcatgaggct ctgcacaacc actacacgca gaagagcctc 2760

tccctgtctc cgggtaaatg a 2781

<210> 23
<211> 442
<212> PRT
<213> Artificial Sequence

<220>
<223> LC007 (VH-CH1)-Fc(hole) P329G LALA

<400> 23

Glu Val Gln Leu Gln Glu Ser Gly Pro Gly Leu Val Lys Pro Ser Gln
1               5                   10                  15

Ser Leu Ser Leu Thr Cys Ser Val Thr Gly Tyr Ser Ile Thr Ser Gly
            20                  25                  30

Tyr Tyr Trp Asn Trp Ile Arg Gln Phe Pro Gly Asn Lys Leu Glu Trp
        35                  40                  45

Met Gly Tyr Ile Thr Tyr Asp Gly Ser Asn Asn Tyr Asn Pro Ser Leu
    50                  55                  60

Lys Asn Arg Ile Ser Ile Thr Arg Asp Thr Ser Lys Asn Gln Phe Phe
65                  70                  75                  80

Leu Lys Leu Asn Ser Val Thr Thr Glu Asp Thr Ala Thr Tyr Tyr Cys
                85                  90                  95

Ala Asp Phe Asp Tyr Trp Gly Gln Gly Thr Thr Leu Thr Val Ser Ser
            100                 105                 110

Ala Ser Thr Lys Gly Pro Ser Val Phe Pro Leu Ala Pro Ser Ser Lys
        115                 120                 125

Ser Thr Ser Gly Gly Thr Ala Ala Leu Gly Cys Leu Val Lys Asp Tyr
    130                 135                 140

Phe Pro Glu Pro Val Thr Val Ser Trp Asn Ser Gly Ala Leu Thr Ser
145                 150                 155                 160

Gly Val His Thr Phe Pro Ala Val Leu Gln Ser Ser Gly Leu Tyr Ser
                165                 170                 175

Leu Ser Ser Val Val Thr Val Pro Ser Ser Ser Leu Gly Thr Gln Thr
            180                 185                 190

Tyr Ile Cys Asn Val Asn His Lys Pro Ser Asn Thr Lys Val Asp Lys
        195                 200                 205

Lys Val Glu Pro Lys Ser Cys Asp Lys Thr His Thr Cys Pro Pro Cys
    210                 215                 220

Pro Ala Pro Glu Ala Ala Gly Gly Pro Ser Val Phe Leu Phe Pro Pro
225                 230                 235                 240

Lys Pro Lys Asp Thr Leu Met Ile Ser Arg Thr Pro Glu Val Thr Cys
                245                 250                 255

Val Val Val Asp Val Ser His Glu Asp Pro Glu Val Lys Phe Asn Trp
            260                 265                 270

Tyr Val Asp Gly Val Glu Val His Asn Ala Lys Thr Lys Pro Arg Glu
        275                 280                 285

Glu Gln Tyr Asn Ser Thr Tyr Arg Val Val Ser Val Leu Thr Val Leu
    290                 295                 300

His Gln Asp Trp Leu Asn Gly Lys Glu Tyr Lys Cys Lys Val Ser Asn
305                 310                 315                 320

Lys Ala Leu Gly Ala Pro Ile Glu Lys Thr Ile Ser Lys Ala Lys Gly
                325                 330                 335

Gln Pro Arg Glu Pro Gln Val Cys Thr Leu Pro Pro Ser Arg Asp Glu
            340                 345                 350

Leu Thr Lys Asn Gln Val Ser Leu Ser Cys Ala Val Lys Gly Phe Tyr
        355                 360                 365

Pro Ser Asp Ile Ala Val Glu Trp Glu Ser Asn Gly Gln Pro Glu Asn
    370                 375                 380

Asn Tyr Lys Thr Thr Pro Pro Val Leu Asp Ser Asp Gly Ser Phe Phe
385                 390                 395                 400

Leu Val Ser Lys Leu Thr Val Asp Lys Ser Arg Trp Gln Gln Gly Asn
                405                 410                 415

Val Phe Ser Cys Ser Val Met His Glu Ala Leu His Asn His Tyr Thr
            420                 425                 430

Gln Lys Ser Leu Ser Leu Ser Pro Gly Lys
        435                 440

<210>  24
<211>  1329
<212>  DNA
<213>  Artificial Sequence

<220>
<223>  LC007 (VH-CH1)-Fc(hole) P329G LALA

<400>  24
gaggtccagc tgcaggagtc aggacctggc ctcgtgaaac cttctcagtc tctgtctctc      60

acctgctctg tcactggcta ctccatcacc agtggttatt actggaactg gatccggcag     120

tttccaggaa acaagctgga atggatgggc tacataacct acgacggtag caataactac     180

aacccatctc tcaaaaatcg aatctccatc actcgtgaca catctaagaa ccagtttttc 240

ctgaagttga attctgtgac tactgaggac acagctacat attactgtgc ggactttgac 300

tactggggcc aaggcaccac tctcacagtc tcctcagcta gcaccaaggg cccatcggtc 360

ttccccctgg caccctcctc caagagcacc tctgggggca cagcggccct gggctgcctg 420

gtcaaggact acttccccga accggtgacg gtgtcgtgga actcaggcgc cctgaccagc 480

ggcgtgcaca ccttcccggc tgtcctacag tcctcaggac tctactccct cagcagcgtg 540

gtgaccgtgc cctccagcag cttgggcacc cagacctaca tctgcaacgt gaatcacaag 600

cccagcaaca ccaaggtgga caagaaagtt gagcccaaat cttgtgacaa aactcacaca 660

tgcccaccgt gcccagcacc tgaagctgca gggggaccgt cagtcttcct cttcccccca 720

aaacccaagg acaccctcat gatctcccgg acccctgagg tcacatgcgt ggtggtggac 780

gtgagccacg aagaccctga ggtcaagttc aactggtacg tggacggcgt ggaggtgcat 840

aatgccaaga caaagccgcg ggaggagcag tacaacagca cgtaccgtgt ggtcagcgtc 900

ctcaccgtcc tgcaccagga ctggctgaat ggcaaggagt acaagtgcaa ggtctccaac 960

aaagccctcg gcgcccccat cgagaaaacc atctccaaag ccaaagggca gccccgagaa 1020

ccacaggtgt gcaccctgcc cccatcccgg gatgagctga ccaagaacca ggtcagcctc 1080

tcgtgcgcag tcaaaggctt ctatcccagc gacatcgccg tggagtggga gagcaatggg 1140

cagccggaga acaactacaa gaccacgcct cccgtgctgg actccgacgg ctccttcttc 1200

ctcgtgagca agctcaccgt ggacaagagc aggtggcagc aggggaacgt cttctcatgc 1260

tccgtgatgc atgaggctct gcacaaccac tacacgcaga agagcctctc cctgtctccg 1320

ggtaaatga 1329

<210> 25
<211> 926
<212> PRT
<213> Artificial Sequence

<220>
<223> V9 (scFab)-LC007 (VH-CH1)-Fc(knob) P329G LALA N297D

<400> 25

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Arg Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Arg Leu Glu Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Gly Asn Thr Leu Pro Trp
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys Ser Gly Gly Gly Ser Gly Gly Gly Ser Glu
    210                 215                 220

Gly Gly Gly Ser Glu Gly Gly Gly Ser Glu Gly Gly Gly Ser Glu Gly
225                 230                 235                 240

Gly Gly Ser Gly Gly Gly Ser Gly Glu Val Gln Leu Val Glu Ser Gly
                245                 250                 255

Gly Gly Leu Val Gln Pro Gly Gly Ser Leu Arg Leu Ser Cys Ala Ala
            260                 265                 270

Ser Gly Tyr Ser Phe Thr Gly Tyr Thr Met Asn Trp Val Arg Gln Ala
        275                 280                 285

Pro Gly Lys Gly Leu Glu Trp Val Ala Leu Ile Asn Pro Tyr Lys Gly
    290                 295                 300

Val Ser Thr Tyr Asn Gln Lys Phe Lys Asp Arg Phe Thr Ile Ser Val
305                 310                 315                 320

Asp Lys Ser Lys Asn Thr Ala Tyr Leu Gln Met Asn Ser Leu Arg Ala
                325                 330                 335

Glu Asp Thr Ala Val Tyr Tyr Cys Ala Arg Ser Gly Tyr Tyr Gly Asp
            340                 345                 350

Ser Asp Trp Tyr Phe Asp Val Trp Gly Gln Gly Thr Leu Val Thr Val
        355                 360                 365

Ser Ser Ala Ser Thr Lys Gly Pro Ser Val Phe Pro Leu Ala Pro Ser
    370                 375                 380

Ser Lys Ser Thr Ser Gly Gly Thr Ala Ala Leu Gly Cys Leu Val Lys
385                 390                 395                 400

Asp Tyr Phe Pro Glu Pro Val Thr Val Ser Trp Asn Ser Gly Ala Leu
                405                 410                 415

Thr Ser Gly Val His Thr Phe Pro Ala Val Leu Gln Ser Ser Gly Leu
            420                 425                 430

Tyr Ser Leu Ser Ser Val Val Thr Val Pro Ser Ser Ser Leu Gly Thr
        435                 440                 445

Gln Thr Tyr Ile Cys Asn Val Asn His Lys Pro Ser Asn Thr Lys Val
    450                 455                 460

Asp Lys Lys Val Glu Pro Lys Ser Cys Asp Gly Gly Gly Gly Ser Gly
465                 470                 475                 480

Gly Gly Gly Ser Glu Val Gln Leu Gln Glu Ser Gly Pro Gly Leu Val
                485                 490                 495

Lys Pro Ser Gln Ser Leu Ser Leu Thr Cys Ser Val Thr Gly Tyr Ser
            500                 505                 510

Ile Thr Ser Gly Tyr Tyr Trp Asn Trp Ile Arg Gln Phe Pro Gly Asn
        515                 520                 525

Lys Leu Glu Trp Met Gly Tyr Ile Thr Tyr Asp Gly Ser Asn Asn Tyr
    530                 535                 540

Asn Pro Ser Leu Lys Asn Arg Ile Ser Ile Thr Arg Asp Thr Ser Lys

545                 550                 555                 560

Asn Gln Phe Phe Leu Lys Leu Asn Ser Val Thr Thr Glu Asp Thr Ala
                565                 570                 575

Thr Tyr Tyr Cys Ala Asp Phe Asp Tyr Trp Gly Gln Gly Thr Thr Leu
            580                 585                 590

Thr Val Ser Ser Ala Ser Thr Lys Gly Pro Ser Val Phe Pro Leu Ala
        595                 600                 605

Pro Ser Ser Lys Ser Thr Ser Gly Gly Thr Ala Ala Leu Gly Cys Leu
    610                 615                 620

Val Lys Asp Tyr Phe Pro Glu Pro Val Thr Val Ser Trp Asn Ser Gly
625                 630                 635                 640

Ala Leu Thr Ser Gly Val His Thr Phe Pro Ala Val Leu Gln Ser Ser
                645                 650                 655

Gly Leu Tyr Ser Leu Ser Ser Val Val Thr Val Pro Ser Ser Ser Leu
            660                 665                 670

Gly Thr Gln Thr Tyr Ile Cys Asn Val Asn His Lys Pro Ser Asn Thr
        675                 680                 685

Lys Val Asp Lys Lys Val Glu Pro Lys Ser Cys Asp Lys Thr His Thr
    690                 695                 700

Cys Pro Pro Cys Pro Ala Pro Glu Ala Ala Gly Gly Pro Ser Val Phe
705                 710                 715                 720

Leu Phe Pro Pro Lys Pro Lys Asp Thr Leu Met Ile Ser Arg Thr Pro
                725                 730                 735

Glu Val Thr Cys Val Val Val Asp Val Ser His Glu Asp Pro Glu Val
            740                 745                 750

Lys Phe Asn Trp Tyr Val Asp Gly Val Glu Val His Asn Ala Lys Thr
        755                 760                 765

Lys Pro Arg Glu Glu Gln Tyr Asp Ser Thr Tyr Arg Val Val Ser Val
    770                 775                 780

Leu Thr Val Leu His Gln Asp Trp Leu Asn Gly Lys Glu Tyr Lys Cys
785                 790                 795                 800

Lys Val Ser Asn Lys Ala Leu Gly Ala Pro Ile Glu Lys Thr Ile Ser
                805                 810                 815

Lys Ala Lys Gly Gln Pro Arg Glu Pro Gln Val Tyr Thr Leu Pro Pro
            820                 825                 830

Cys Arg Asp Glu Leu Thr Lys Asn Gln Val Ser Leu Trp Cys Leu Val
        835                 840                 845

Lys Gly Phe Tyr Pro Ser Asp Ile Ala Val Glu Trp Glu Ser Asn Gly
    850                 855                 860

Gln Pro Glu Asn Asn Tyr Lys Thr Thr Pro Pro Val Leu Asp Ser Asp
865                 870                 875                 880

Gly Ser Phe Phe Leu Tyr Ser Lys Leu Thr Val Asp Lys Ser Arg Trp
                885                 890                 895

Gln Gln Gly Asn Val Phe Ser Cys Ser Val Met His Glu Ala Leu His
            900                 905                 910

Asn His Tyr Thr Gln Lys Ser Leu Ser Leu Ser Pro Gly Lys
        915                 920                 925

<210>  26
<211>  2781
<212>  DNA
<213>  Artificial Sequence

<220>
<223>  V9 (scFab)-LC007 (VH-CH1)-Fc(knob) P329G LALA N297D

<400>  26
gacatccaga tgacccagag cccctctagc ctgagcgcca gcgtgggcga cagagtgacc      60

atcacctgtc gggccagcca ggacatcaga aactacctga actggtatca gcagaagccc     120

ggcaaggccc ccaagctgct gatctactac acctctagac tggaaagcgg cgtgcccagc     180

cggtttagcg gcagcggctc cggcaccgac tacaccctga ccatcagcag cctgcagccc     240

gaggacttcg ccacctacta ctgccagcag ggcaacacac tcccctggac cttcggccag     300

ggcaccaagg tggagatcaa gcgtacggtg gccgctccca gcgtgttcat cttccccccc     360

agcgacgagc agctgaagtc cggcaccgcc agcgtcgtgt gcctgctgaa caacttctac     420

ccccgggagg ccaaggtgca gtggaaggtg gacaacgccc tgcagagcgg caacagccag     480

gaaagcgtca ccgagcagga cagcaaggac tccacctaca gcctgagcag caccctgacc     540

ctgagcaagg ccgactacga gaagcacaag gtgtacgcct gcgaagtgac ccaccagggc     600

ctgtccagcc ccgtgaccaa gagcttcaac cggggcgagt gcagcggcgg aggctctgga     660

ggcggctctg aaggcggagg aagtgagggc ggaggctcag aaggcggcgg aagcgaaggt 720

ggcggctctg gcggcggatc cggcgaggtg cagctggtcg agtccggcgg aggcctggtg 780

cagcctggcg gcagcctgag actgagctgc gccgccagcg gctacagctt caccggctac 840

accatgaact gggtccggca ggctcctggc aagggcctcg aatgggtggc cctgatcaac 900

ccctacaagg gcgtgagcac ctacaaccag aagttcaagg accggttcac catcagcgtg 960

gacaagagca agaacaccgc ctatctgcag atgaacagcc tgcgggccga ggacaccgcc 1020

gtgtactact gcgccagaag cggctactac ggcgacagcg actggtactt cgacgtgtgg 1080

ggccagggca cactggtcac cgtgtccagc gctagcacca agggcccctc cgtgttcccc 1140

ctggccccca gcagcaagag caccagcggc ggcacagccg ccctcggctg cctggtcaag 1200

gactacttcc ccgagcccgt gaccgtgtcc tggaacagcg gagccctgac ctccggcgtg 1260

cacaccttcc ccgccgtgct gcagagcagc ggcctgtaca gcctgtccag cgtggtcacc 1320

gtgccctcca gcagcctggg cacccagacc tacatctgca acgtgaacca caagcccagc 1380

aataccaagg tggacaagaa ggtggagccc aagagctgcg acggcggtgg tggctccgga 1440

ggcggtggat ctgaagtgca gctgcaggaa agcggccctg gcctggtcaa gcccagccag 1500

agcctgagcc tgacctgtag cgtgaccggc tactccatca cctccggcta ctactggaat 1560

tggattcggc agttccccgg caacaagctg gaatggatgg gctacatcac ctacgacggc 1620

agcaacaact acaaccccag cctgaagaac cggatcagca tcacccggga caccagcaag 1680

aaccagttct tcctgaagtt gaattctgtg actactgagg acacagctac atattactgt 1740

gcggactttg actactgggg ccaaggcacc actctcacag tctcctcagc tagcaccaag 1800

ggcccatcgg tcttccccct ggcaccctcc tccaagagca cctctggggg cacagcggcc 1860

ctgggctgcc tggtcaagga ctacttcccc gaaccggtga cggtgtcgtg gaactcaggc 1920

gccctgacca gcggcgtgca caccttcccg gctgtcctac agtcctcagg actctactcc 1980

ctcagcagcg tggtgaccgt gccctccagc agcttgggca cccagaccta catctgcaac 2040

gtgaatcaca agcccagcaa caccaaggtg gacaagaaag ttgagcccaa atcttgtgac 2100

aaaactcaca catgcccacc gtgcccagca cctgaagctg cagggggacc gtcagtcttc 2160

ctcttccccc caaaacccaa ggacaccctc atgatctccc ggacccctga ggtcacatgc 2220

gtggtggtgg acgtgagcca cgaagaccct gaggtcaagt tcaactggta cgtggacggc 2280

gtggaggtgc ataatgccaa gacaaagccg cgggaggagc agtacgacag cacgtaccgt 2340

gtggtcagcg tcctcaccgt cctgcaccag gactggctga atggcaagga gtacaagtgc 2400

aaggtctcca acaaagccct cggcgccccc atcgagaaaa ccatctccaa agccaaaggg 2460

cagccccgag aaccacaggt gtacaccctg cccccatgcc gggatgagct gaccaagaac 2520

caggtcagcc tgtggtgcct ggtcaaaggc ttctatccca gcgacatcgc cgtggagtgg 2580

gagagcaatg ggcagccgga gaacaactac aagaccacgc ctcccgtgct ggactccgac 2640

ggctccttct tcctctacag caagctcacc gtggacaaga gcaggtggca gcaggggaac 2700

gtcttctcat gctccgtgat gcatgaggct ctgcacaacc actacacgca gaagagcctc 2760

tccctgtctc cgggtaaatg a 2781

<210> 27
<211> 442
<212> PRT
<213> Artificial Sequence

<220>
<223> LC007 (VH-CH1)-Fc(hole) P329G LALA N297D

<400> 27

Glu Val Gln Leu Gln Glu Ser Gly Pro Gly Leu Val Lys Pro Ser Gln
1               5                   10                  15

Ser Leu Ser Leu Thr Cys Ser Val Thr Gly Tyr Ser Ile Thr Ser Gly
            20                  25                  30

Tyr Tyr Trp Asn Trp Ile Arg Gln Phe Pro Gly Asn Lys Leu Glu Trp
        35                  40                  45

Met Gly Tyr Ile Thr Tyr Asp Gly Ser Asn Asn Tyr Asn Pro Ser Leu
    50                  55                  60

Lys Asn Arg Ile Ser Ile Thr Arg Asp Thr Ser Lys Asn Gln Phe Phe
65                  70                  75                  80

Leu Lys Leu Asn Ser Val Thr Thr Glu Asp Thr Ala Thr Tyr Tyr Cys
                85                  90                  95

Ala Asp Phe Asp Tyr Trp Gly Gln Gly Thr Thr Leu Thr Val Ser Ser
            100                 105                 110

Ala Ser Thr Lys Gly Pro Ser Val Phe Pro Leu Ala Pro Ser Ser Lys
        115                 120                 125

Ser Thr Ser Gly Gly Thr Ala Ala Leu Gly Cys Leu Val Lys Asp Tyr
    130                 135                 140

Phe Pro Glu Pro Val Thr Val Ser Trp Asn Ser Gly Ala Leu Thr Ser
145                 150                 155                 160

Gly Val His Thr Phe Pro Ala Val Leu Gln Ser Ser Gly Leu Tyr Ser
                165                 170                 175

Leu Ser Ser Val Val Thr Val Pro Ser Ser Ser Leu Gly Thr Gln Thr
        180                 185                 190

Tyr Ile Cys Asn Val Asn His Lys Pro Ser Asn Thr Lys Val Asp Lys
    195                 200                 205

Lys Val Glu Pro Lys Ser Cys Asp Lys Thr His Thr Cys Pro Pro Cys
    210                 215                 220

Pro Ala Pro Glu Ala Ala Gly Gly Pro Ser Val Phe Leu Phe Pro Pro
225                 230                 235                 240

Lys Pro Lys Asp Thr Leu Met Ile Ser Arg Thr Pro Glu Val Thr Cys
                245                 250                 255

Val Val Val Asp Val Ser His Glu Asp Pro Glu Val Lys Phe Asn Trp
            260                 265                 270

Tyr Val Asp Gly Val Glu Val His Asn Ala Lys Thr Lys Pro Arg Glu
        275                 280                 285

Glu Gln Tyr Asp Ser Thr Tyr Arg Val Val Ser Val Leu Thr Val Leu
    290                 295                 300

His Gln Asp Trp Leu Asn Gly Lys Glu Tyr Lys Cys Lys Val Ser Asn
305                 310                 315                 320

Lys Ala Leu Gly Ala Pro Ile Glu Lys Thr Ile Ser Lys Ala Lys Gly
                325                 330                 335

Gln Pro Arg Glu Pro Gln Val Cys Thr Leu Pro Pro Ser Arg Asp Glu
            340                 345                 350

Leu Thr Lys Asn Gln Val Ser Leu Ser Cys Ala Val Lys Gly Phe Tyr
        355                 360                 365

Pro Ser Asp Ile Ala Val Glu Trp Glu Ser Asn Gly Gln Pro Glu Asn
    370                 375                 380

Asn Tyr Lys Thr Thr Pro Pro Val Leu Asp Ser Asp Gly Ser Phe Phe
385                 390                 395                 400

Leu Val Ser Lys Leu Thr Val Asp Lys Ser Arg Trp Gln Gln Gly Asn
                405                 410                 415

Val Phe Ser Cys Ser Val Met His Glu Ala Leu His Asn His Tyr Thr
            420                 425                 430

Gln Lys Ser Leu Ser Leu Ser Pro Gly Lys
        435                 440

<210>  28
<211>  1329
<212>  DNA
<213>  Artificial Sequence

<220>
<223>  LC007 (VH-CH1)-Fc(hole) P329G LALA N297D

<400>  28
gaggtccagc tgcaggagtc aggacctggc ctcgtgaaac cttctcagtc tctgtctctc      60

acctgctctg tcactggcta ctccatcacc agtggttatt actggaactg gatccggcag     120

tttccaggaa acaagctgga atggatgggc tacataacct acgacggtag caataactac     180

aacccatctc tcaaaaatcg aatctccatc actcgtgaca catctaagaa ccagtttttc     240

ctgaagttga attctgtgac tactgaggac acagctacat attactgtgc ggactttgac     300

tactggggcc aaggcaccac tctcacagtc tcctcagcta gcaccaaggg cccatcggtc     360

ttccccctgg caccctcctc caagagcacc tctgggggca cagcggccct gggctgcctg     420

gtcaaggact acttccccga accggtgacg gtgtcgtgga actcaggcgc cctgaccagc     480

ggcgtgcaca ccttcccggc tgtcctacag tcctcaggac tctactccct cagcagcgtg     540

gtgaccgtgc cctccagcag cttgggcacc cagacctaca tctgcaacgt gaatcacaag     600

cccagcaaca ccaaggtgga caagaaagtt gagcccaaat cttgtgacaa aactcacaca     660

tgcccaccgt gcccagcacc tgaagctgca gggggaccgt cagtcttcct cttcccccca     720

aaacccaagg acaccctcat gatctcccgg acccctgagg tcacatgcgt ggtggtggac     780

gtgagccacg aagaccctga ggtcaagttc aactggtacg tggacggcgt ggaggtgcat     840

aatgccaaga caaagccgcg ggaggagcag tacgacagca cgtaccgtgt ggtcagcgtc     900

ctcaccgtcc tgcaccagga ctggctgaat ggcaaggagt acaagtgcaa ggtctccaac     960

aaagccctcg gcgcccccat cgagaaaacc atctccaaag ccaaagggca gccccgagaa    1020

ccacaggtgt gcaccctgcc cccatcccgg gatgagctga ccaagaacca ggtcagcctc    1080

tcgtgcgcag tcaaaggctt ctatcccagc gacatcgccg tggagtggga gagcaatggg    1140

cagccggaga acaactacaa gaccacgcct cccgtgctgg actccgacgg ctccttcttc    1200

ctcgtgagca agctcaccgt ggacaagagc aggtggcagc aggggaacgt cttctcatgc    1260

tccgtgatgc atgaggctct gcacaaccac tacacgcaga agagcctctc cctgtctccg    1320

ggtaaatga                                                            1329

<210>  29

<211>  664
<212>  PRT
<213>  Artificial Sequence

<220>
<223>  V9 (VL-CH1)-LC007 (VH-CH1)-Fc(hole) P329G LALA

<400>  29

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Arg Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Arg Leu Glu Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Gly Asn Thr Leu Pro Trp
                85                  90                  95

Thr Phe Gly Gln Gly Thr Lys Val Glu Ile Lys Ser Ser Ala Ser Thr
            100                 105                 110

Lys Gly Pro Ser Val Phe Pro Leu Ala Pro Ser Ser Lys Ser Thr Ser
        115                 120                 125

Gly Gly Thr Ala Ala Leu Gly Cys Leu Val Lys Asp Tyr Phe Pro Glu
    130                 135                 140

Pro Val Thr Val Ser Trp Asn Ser Gly Ala Leu Thr Ser Gly Val His
145                 150                 155                 160

Thr Phe Pro Ala Val Leu Gln Ser Ser Gly Leu Tyr Ser Leu Ser Ser
                165                 170                 175

Val Val Thr Val Pro Ser Ser Ser Leu Gly Thr Gln Thr Tyr Ile Cys
            180                 185                 190

Asn Val Asn His Lys Pro Ser Asn Thr Lys Val Asp Lys Lys Val Glu
        195                 200                 205

Pro Lys Ser Cys Gly Gly Gly Gly Ser Gly Gly Gly Gly Ser Glu Val
    210                 215                 220

Gln Leu Gln Glu Ser Gly Pro Gly Leu Val Lys Pro Ser Gln Ser Leu
225                 230                 235                 240

Ser Leu Thr Cys Ser Val Thr Gly Tyr Ser Ile Thr Ser Gly Tyr Tyr
                245                 250                 255

Trp Asn Trp Ile Arg Gln Phe Pro Gly Asn Lys Leu Glu Trp Met Gly
            260                 265                 270

Tyr Ile Thr Tyr Asp Gly Ser Asn Asn Tyr Asn Pro Ser Leu Lys Asn
        275                 280                 285

Arg Ile Ser Ile Thr Arg Asp Thr Ser Lys Asn Gln Phe Phe Leu Lys
    290                 295                 300

Leu Asn Ser Val Thr Thr Glu Asp Thr Ala Thr Tyr Tyr Cys Ala Asp
305                 310                 315                 320

Phe Asp Tyr Trp Gly Gln Gly Thr Thr Leu Thr Val Ser Ser Ala Ser
                325                 330                 335

Thr Lys Gly Pro Ser Val Phe Pro Leu Ala Pro Ser Ser Lys Ser Thr
            340                 345                 350

Ser Gly Gly Thr Ala Ala Leu Gly Cys Leu Val Lys Asp Tyr Phe Pro
        355                 360                 365

Glu Pro Val Thr Val Ser Trp Asn Ser Gly Ala Leu Thr Ser Gly Val
    370                 375                 380

His Thr Phe Pro Ala Val Leu Gln Ser Ser Gly Leu Tyr Ser Leu Ser
385                 390                 395                 400

Ser Val Val Thr Val Pro Ser Ser Ser Leu Gly Thr Gln Thr Tyr Ile
                405                 410                 415

Cys Asn Val Asn His Lys Pro Ser Asn Thr Lys Val Asp Lys Lys Val
            420                 425                 430

Glu Pro Lys Ser Cys Asp Lys Thr His Thr Cys Pro Pro Cys Pro Ala
        435                 440                 445

Pro Glu Ala Ala Gly Gly Pro Ser Val Phe Leu Phe Pro Pro Lys Pro
    450                 455                 460

Lys Asp Thr Leu Met Ile Ser Arg Thr Pro Glu Val Thr Cys Val Val

465                 470                 475                 480

Val Asp Val Ser His Glu Asp Pro Glu Val Lys Phe Asn Trp Tyr Val
                485                 490                 495

Asp Gly Val Glu Val His Asn Ala Lys Thr Lys Pro Arg Glu Glu Gln
            500                 505                 510

Tyr Asn Ser Thr Tyr Arg Val Val Ser Val Leu Thr Val Leu His Gln
        515                 520                 525

Asp Trp Leu Asn Gly Lys Glu Tyr Lys Cys Lys Val Ser Asn Lys Ala
    530                 535                 540

Leu Gly Ala Pro Ile Glu Lys Thr Ile Ser Lys Ala Lys Gly Gln Pro
545                 550                 555                 560

Arg Glu Pro Gln Val Cys Thr Leu Pro Pro Ser Arg Asp Glu Leu Thr
                565                 570                 575

Lys Asn Gln Val Ser Leu Ser Cys Ala Val Lys Gly Phe Tyr Pro Ser
            580                 585                 590

Asp Ile Ala Val Glu Trp Glu Ser Asn Gly Gln Pro Glu Asn Asn Tyr
        595                 600                 605

Lys Thr Thr Pro Pro Val Leu Asp Ser Asp Gly Ser Phe Phe Leu Val
    610                 615                 620

Ser Lys Leu Thr Val Asp Lys Ser Arg Trp Gln Gln Gly Asn Val Phe
625                 630                 635                 640

Ser Cys Ser Val Met His Glu Ala Leu His Asn His Tyr Thr Gln Lys
                645                 650                 655

Ser Leu Ser Leu Ser Pro Gly Lys
            660

<210>  30
<211>  1995
<212>  DNA
<213>  Artificial Sequence

<220>
<223>  V9 (VL-CH1)-LC007 (VH-CH1)-Fc(hole) P329G LALA

<400>  30
gatatccaga tgacccagag ccccagctct ctgagcgcca gcgtgggcga cagagtgacc      60

atcacctgtc gggccagcca ggacatcaga aactacctga actggtatca gcagaagccc     120

ggcaaggccc ccaagctgct gatctactac accagcagac tggaaagcgg cgtgccctcc 180

agattttccg gcagcggctc cggcaccgac tacaccctga ccatcagcag cctgcagccc 240

gaggatttcg ccacatatta ctgccagcag ggcaataccc tgccctggac cttcggacag 300

ggcacaaaag tggaaatcaa gagcagcgct tccaccaaag gcccttccgt gtttcctctg 360

gctcctagct ccaagtccac ctctggaggc accgctgctc tcggatgcct cgtgaaggat 420

tattttcctg agcctgtgac agtgtcctgg aatagcggag cactgacctc tggagtgcat 480

actttccccg ctgtgctgca gtcctctgga ctgtacagcc tgagcagcgt ggtgacagtg 540

cccagcagca gcctgggcac ccagacctac atctgcaacg tgaaccacaa gcccagcaac 600

accaaggtgg acaagaaggt ggaacccaag tcttgtggcg gaggcggatc cggcggaggg 660

ggatctgagg tgcagctgca ggaaagcggc cctggcctgg tgaaacccag ccagagcctg 720

agcctgacct gcagcgtgac cggctacagc atcaccagcg gctactactg gaactggatc 780

agacagttcc ccggcaacaa gctggaatgg atgggctaca tcacctacga cggcagcaac 840

aactacaacc ccagcctgaa gaacagaatc agcatcaccc gggacaccag caagaaccag 900

ttcttcctga agctgaacag cgtgaccacc gaggacaccg ccacctacta ctgcgccgac 960

ttcgactact ggggccaggg caccaccctg accgtgtcct ccgcctctac caagggcccc 1020

agcgtgttcc ccctggcacc cagcagcaag agcacatctg gcggaacagc cgctctgggc 1080

tgtctggtga aagactactt ccccgagccc gtgaccgtgt cttggaactc tggcgccctg 1140

accagcggcg tgcacacctt tccagccgtg ctgcagagca gcggcctgta ctccctgtcc 1200

tccgtggtca ccgtgccctc tagctccctg ggaacacaga catatatctg taatgtcaat 1260

cacaagcctt ccaacaccaa agtcgataag aaagtcgagc ccaagagctg cgacaaaact 1320

cacacatgcc caccgtgccc agcacctgaa gctgcagggg gaccgtcagt cttcctcttc 1380

cccccaaaac ccaaggacac cctcatgatc tcccggaccc ctgaggtcac atgcgtggtg 1440

gtggacgtga gccacgaaga ccctgaggtc aagttcaact ggtacgtgga cggcgtggag 1500

gtgcataatg ccaagacaaa gccgcgggag gagcagtaca acagcacgta ccgtgtggtc 1560

agcgtcctca ccgtcctgca ccaggactgg ctgaatggca aggagtacaa gtgcaaggtc 1620

tccaacaaag ccctcggcgc ccccatcgag aaaaccatct ccaaagccaa agggcagccc 1680

cgagaaccac aggtgtgcac cctgccccca tcccgggatg agctgaccaa gaaccaggtc 1740

agcctctcgt gcgcagtcaa aggcttctat cccagcgaca tcgccgtgga gtgggagagc 1800

aatgggcagc cggagaacaa ctacaagacc acgcctcccg tgctggactc cgacggctcc 1860

ttcttcctcg tgagcaagct caccgtggac aagagcaggt ggcagcaggg gaacgtcttc 1920

tcatgctccg tgatgcatga ggctctgcac aaccactaca cgcagaagag cctctccctg 1980

tctccgggta aatga                                                    1995

<210>  31
<211>  229
<212>  PRT
<213>  Artificial Sequence

<220>
<223>  Fc(knob) wt

<400>  31

Asp Lys Thr His Thr Cys Pro Pro Cys Pro Ala Pro Glu Leu Leu Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Cys Arg Asp Glu Leu Thr Lys Asn Gln Val Ser
    130                 135                 140

Leu Trp Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly Lys Ser Gly
225

<210>  32
<211>  690
<212>  DNA
<213>  Artificial Sequence

<220>
<223>  Fc(knob) wt

<400>  32
gacaaaactc acacatgccc accgtgccca gcacctgaac tcctgggggg accgtcagtc      60

ttcctcttcc ccccaaaacc caaggacacc ctcatgatct cccggacccc tgaggtcaca     120

tgcgtggtgg tggacgtgag ccacgaagac cctgaggtca agttcaactg gtacgtggac     180

ggcgtggagg tgcataatgc caagacaaag ccgcgggagg agcagtacaa cagcacgtac     240

cgtgtggtca gcgtcctcac cgtcctgcac caggactggc tgaatggcaa ggagtacaag     300

tgcaaggtct ccaacaaagc cctcccagcc cccatcgaga aaaccatctc caaagccaaa     360

gggcagcccc gagaaccaca ggtgtacacc ctgcccccat gccgggatga gctgaccaag     420

aaccaggtca gcctgtggtg cctggtcaaa ggcttctatc ccagcgacat cgccgtggag     480

tgggagagca atgggcagcc ggagaacaac tacaagacca cgcctcccgt gctggactcc     540

gacggctcct tcttcctcta cagcaagctc accgtggaca agagcaggtg gcagcagggg     600

aacgtcttct catgctccgt gatgcatgag gctctgcaca accactacac gcagaagagc     660

ctctccctgt ctccgggtaa atccggatga                                      690

<210>  33
<211>  229
<212>  PRT
<213>  Artificial Sequence

<220>
<223>  V9 (VH-CL)

<400>  33

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Ser Phe Thr Gly Tyr
            20                  25                  30

Thr Met Asn Trp Val Arg Gln Ala Pro Gly Lys Gly Leu Glu Trp Val

35                  40                  45

Ala Leu Ile Asn Pro Tyr Lys Gly Val Ser Thr Tyr Asn Gln Lys Phe
    50                  55                  60

Lys Asp Arg Phe Thr Ile Ser Val Asp Lys Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg Ser Gly Tyr Tyr Gly Asp Ser Asp Trp Tyr Phe Asp Val Trp
            100                 105                 110

Gly Gln Gly Thr Leu Val Thr Val Ser Ser Ala Ser Val Ala Ala Pro
        115                 120                 125

Ser Val Phe Ile Phe Pro Pro Ser Asp Glu Gln Leu Lys Ser Gly Thr
    130                 135                 140

Ala Ser Val Val Cys Leu Leu Asn Asn Phe Tyr Pro Arg Glu Ala Lys
145                 150                 155                 160

Val Gln Trp Lys Val Asp Asn Ala Leu Gln Ser Gly Asn Ser Gln Glu
                165                 170                 175

Ser Val Thr Glu Gln Asp Ser Lys Asp Ser Thr Tyr Ser Leu Ser Ser
            180                 185                 190

Thr Leu Thr Leu Ser Lys Ala Asp Tyr Glu Lys His Lys Val Tyr Ala
        195                 200                 205

Cys Glu Val Thr His Gln Gly Leu Ser Ser Pro Val Thr Lys Ser Phe
    210                 215                 220

Asn Arg Gly Glu Cys
225

<210>  34
<211>  690
<212>  DNA
<213>  Artificial Sequence

<220>
<223>  V9 (VH-CL)

<400>  34
gaggtgcagc tggtcgagag cggaggcggc ctggtgcagc ctggcggcag cctgagactg        60

agctgcgccg ccagcggcta cagcttcacc ggctacacca tgaactgggt ccggcaggca       120

cctggcaagg gactggaatg ggtggccctg atcaacccct acaagggcgt gagcacctac 180

aaccagaagt tcaaggaccg gttcaccatc agcgtggaca agagcaagaa caccgcctat 240

ctgcagatga acagcctgcg ggccgaggac accgccgtgt actactgcgc cagaagcggc 300

tactacggcg acagcgactg gtacttcgac gtgtggggcc agggcaccct cgtgaccgtg 360

tctagcgcta gcgtggctgc accatctgtc ttcatcttcc cgccatctga tgagcagttg 420

aaatctggaa ctgcctctgt tgtgtgcctg ctgaataact tctatcccag agaggccaaa 480

gtacagtgga aggtggataa cgccctccaa tcgggtaact cccaggagag tgtcacagag 540

caggacagca aggacagcac ctacagcctc agcagcaccc tgacgctgag caaagcagac 600

tacgagaaac acaaagtcta cgcctgcgaa gtcacccatc agggcctgag ctcgcccgtc 660

acaaagagct tcaacagggg agagtgttga 690

<210> 35
<211> 670
<212> PRT
<213> Artificial Sequence

<220>
<223> FN18 (VL-CH1)-LC007 (VH-CH1)-Fc(hole) P329G LALA

<400> 35

Asp Ile Val Met Ser Gln Ser Pro Ser Ser Leu Ala Val Ser Val Gly
1               5                   10                  15

Glu Lys Val Thr Met Ser Cys Lys Ser Ser Gln Ser Leu Leu Tyr Ser
            20                  25                  30

Ser Asn Gln Lys Asn Tyr Leu Ala Trp Tyr Gln Gln Lys Pro Gly Gln
        35                  40                  45

Ser Pro Lys Leu Leu Ile Asn Trp Ala Ser Thr Arg Glu Ser Gly Val
    50                  55                  60

Pro Asp Arg Phe Thr Gly Ser Gly Ser Arg Thr Asp Phe Thr Leu Thr
65                  70                  75                  80

Ile Ser Ser Val Lys Ala Glu Asp Leu Ala Val Tyr Phe Cys Gln Gln
                85                  90                  95

Phe Tyr Ser Tyr Pro Pro Thr Phe Gly Gly Gly Thr Lys Leu Glu Ile
            100                 105                 110

Lys Ser Ser Ala Ser Thr Lys Gly Pro Ser Val Phe Pro Leu Ala Pro
        115                 120                 125

Ser Ser Lys Ser Thr Ser Gly Gly Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190

Thr Gln Thr Tyr Ile Cys Asn Val Asn His Lys Pro Ser Asn Thr Lys
        195                 200                 205

Val Asp Lys Lys Val Glu Pro Lys Ser Cys Gly Gly Gly Gly Ser Gly
    210                 215                 220

Gly Gly Gly Ser Glu Val Gln Leu Gln Glu Ser Gly Pro Gly Leu Val
225                 230                 235                 240

Lys Pro Ser Gln Ser Leu Ser Leu Thr Cys Ser Val Thr Gly Tyr Ser
                245                 250                 255

Ile Thr Ser Gly Tyr Tyr Trp Asn Trp Ile Arg Gln Phe Pro Gly Asn
            260                 265                 270

Lys Leu Glu Trp Met Gly Tyr Ile Thr Tyr Asp Gly Ser Asn Asn Tyr
        275                 280                 285

Asn Pro Ser Leu Lys Asn Arg Ile Ser Ile Thr Arg Asp Thr Ser Lys
    290                 295                 300

Asn Gln Phe Phe Leu Lys Leu Asn Ser Val Thr Thr Glu Asp Thr Ala
305                 310                 315                 320

Thr Tyr Tyr Cys Ala Asp Phe Asp Tyr Trp Gly Gln Gly Thr Thr Leu
                325                 330                 335

Thr Val Ser Ser Ala Ser Thr Lys Gly Pro Ser Val Phe Pro Leu Ala
            340                 345                 350

Pro Ser Ser Lys Ser Thr Ser Gly Gly Thr Ala Ala Leu Gly Cys Leu
        355                 360                 365

Val Lys Asp Tyr Phe Pro Glu Pro Val Thr Val Ser Trp Asn Ser Gly
    370                 375                 380

Ala Leu Thr Ser Gly Val His Thr Phe Pro Ala Val Leu Gln Ser Ser
385                 390                 395                 400

Gly Leu Tyr Ser Leu Ser Ser Val Val Thr Val Pro Ser Ser Ser Leu
                405                 410                 415

Gly Thr Gln Thr Tyr Ile Cys Asn Val Asn His Lys Pro Ser Asn Thr
            420                 425                 430

Lys Val Asp Lys Lys Val Glu Pro Lys Ser Cys Asp Lys Thr His Thr
        435                 440                 445

Cys Pro Pro Cys Pro Ala Pro Glu Ala Ala Gly Gly Pro Ser Val Phe
    450                 455                 460

Leu Phe Pro Pro Lys Pro Lys Asp Thr Leu Met Ile Ser Arg Thr Pro
465                 470                 475                 480

Glu Val Thr Cys Val Val Val Asp Val Ser His Glu Asp Pro Glu Val
                485                 490                 495

Lys Phe Asn Trp Tyr Val Asp Gly Val Glu Val His Asn Ala Lys Thr
            500                 505                 510

Lys Pro Arg Glu Glu Gln Tyr Asn Ser Thr Tyr Arg Val Val Ser Val
        515                 520                 525

Leu Thr Val Leu His Gln Asp Trp Leu Asn Gly Lys Glu Tyr Lys Cys
    530                 535                 540

Lys Val Ser Asn Lys Ala Leu Gly Ala Pro Ile Glu Lys Thr Ile Ser
545                 550                 555                 560

Lys Ala Lys Gly Gln Pro Arg Glu Pro Gln Val Cys Thr Leu Pro Pro
                565                 570                 575

Ser Arg Asp Glu Leu Thr Lys Asn Gln Val Ser Leu Ser Cys Ala Val
            580                 585                 590

Lys Gly Phe Tyr Pro Ser Asp Ile Ala Val Glu Trp Glu Ser Asn Gly
        595                 600                 605

Gln Pro Glu Asn Asn Tyr Lys Thr Thr Pro Pro Val Leu Asp Ser Asp
    610                 615                 620

Gly Ser Phe Phe Leu Val Ser Lys Leu Thr Val Asp Lys Ser Arg Trp

625                 630                 635                 640

Gln Gln Gly Asn Val Phe Ser Cys Ser Val Met His Glu Ala Leu His
                645                 650                 655

Asn His Tyr Thr Gln Lys Ser Leu Ser Leu Ser Pro Gly Lys
            660                 665                 670

<210>  36
<211>  2013
<212>  DNA
<213>  Artificial Sequence

<220>
<223>  FN18 (VL-CH1)-LC007 (VH-CH1)-Fc(hole) P329G LALA

<400>  36
gacatcgtga tgagccagag ccccagcagc ctggccgtgt ccgtgggcga gaaagtgacc      60

atgagctgca agagcagcca gagcctgctg tactcctcta accagaagaa ctacctggcc     120

tggtatcagc agaagcccgg ccagtccccc aagctgctga tcaactgggc cagcacccgc     180

gagagcggcg tgcccgatag attcacaggc agcggcagcc ggaccgactt caccctgacc     240

atcagcagcg tgaaggccga ggatctggcc gtgtacttct gccagcagtt ctacagctac     300

ccccccacct tcggcggagg cacgaagctg gaaatcaaga gcagcgcttc caccaaaggc     360

ccttccgtgt ttcctctggc tcctagctcc aagtccacct ctggaggcac cgctgctctc     420

ggatgcctcg tgaaggatta ttttcctgag cctgtgacag tgtcctggaa tagcggagca     480

ctgacctctg gagtgcatac tttccccgct gtgctgcagt cctctggact gtacagcctg     540

agcagcgtgg tgacagtgcc cagcagcagc ctgggcaccc agacctacat ctgcaacgtg     600

aaccacaagc ccagcaacac caaggtggac aagaaggtgg aacccaagtc ttgtggcgga     660

ggcggatccg gcggaggggg atctgaggtg cagctgcagg aaagcggccc tggcctggtg     720

aaacccagcc agagcctgag cctgacctgc agcgtgaccg gctacagcat caccagcggc     780

tactactgga actggatcag acagttcccc ggcaacaagc tggaatggat gggctacatc     840

acctacgacg gcagcaacaa ctacaacccc agcctgaaga acagaatcag catcacccgg     900

gacaccagca agaaccagtt cttcctgaag ctgaacagcg tgaccaccga ggacaccgcc     960

acctactact gcgccgactt cgactactgg ggccagggca ccaccctgac cgtgtcctcc    1020

gcctctacca agggccccag cgtgttcccc ctggcaccca gcagcaagag cacatctggc    1080

ggaacagccg ctctgggctg tctggtgaaa gactacttcc ccgagcccgt gaccgtgtct    1140

tggaactctg gcgccctgac cagcggcgtg cacacctttc cagccgtgct gcagagcagc    1200

ggcctgtact ccctgtcctc cgtggtcacc gtgccctcta gctccctggg aacacagaca    1260

tatatctgta atgtcaatca caagccttcc aacaccaaag tcgataagaa agtcgagccc    1320

aagagctgcg acaaaactca cacatgccca ccgtgcccag cacctgaagc tgcaggggga 1380

ccgtcagtct tcctcttccc cccaaaaccc aaggacaccc tcatgatctc ccggacccct 1440

gaggtcacat gcgtggtggt ggacgtgagc cacgaagacc ctgaggtcaa gttcaactgg 1500

tacgtggacg gcgtggaggt gcataatgcc aagacaaagc cgcgggagga gcagtacaac 1560

agcacgtacc gtgtggtcag cgtcctcacc gtcctgcacc aggactggct gaatggcaag 1620

gagtacaagt gcaaggtctc caacaaagcc ctcggcgccc ccatcgagaa aaccatctcc 1680

aaagccaaag ggcagccccg agaaccacag gtgtgcaccc tgcccccatc ccgggatgag 1740

ctgaccaaga accaggtcag cctctcgtgc gcagtcaaag gcttctatcc cagcgacatc 1800

gccgtggagt gggagagcaa tgggcagccg gagaacaact acaagaccac gcctcccgtg 1860

ctggactccg acggctcctt cttcctcgtg agcaagctca ccgtggacaa gagcaggtgg 1920

cagcagggga acgtcttctc atgctccgtg atgcatgagg ctctgcacaa ccactacacg 1980

cagaagagcc tctccctgtc tccgggtaaa tga 2013

<210> 37
<211> 231
<212> PRT
<213> Artificial Sequence

<220>
<223> FN18 (VH-CL)

<400> 37

Gln Val Gln Leu Gln Gln Ser Glu Ala Glu Leu Ala Arg Pro Gly Ala
1               5                   10                  15

Ser Val Lys Met Ser Cys Lys Ala Ser Gly Tyr Thr Phe Thr Asp Tyr
            20                  25                  30

Thr Ile His Trp Leu Lys Gln Arg Pro Gly Gln Gly Leu Asp Trp Ile
        35                  40                  45

Gly Tyr Phe Asn Pro Ser Ser Glu Ser Thr Glu Tyr Asn Arg Lys Phe
    50                  55                  60

Lys Asp Arg Thr Ile Leu Thr Ala Asp Arg Ser Ser Thr Thr Ala Tyr
65                  70                  75                  80

Met Gln Leu Ser Ser Leu Thr Ser Glu Asp Ser Ala Val Tyr Tyr Cys
                85                  90                  95

Ser Arg Lys Gly Glu Lys Leu Leu Gly Asn Arg Tyr Trp Tyr Phe Asp
            100                 105                 110

Val Trp Gly Ala Gly Thr Ser Val Thr Val Ser Ser Ala Ser Val Ala
        115                 120                 125

Ala Pro Ser Val Phe Ile Phe Pro Pro Ser Asp Glu Gln Leu Lys Ser
    130                 135                 140

Gly Thr Ala Ser Val Val Cys Leu Leu Asn Asn Phe Tyr Pro Arg Glu
145                 150                 155                 160

Ala Lys Val Gln Trp Lys Val Asp Asn Ala Leu Gln Ser Gly Asn Ser
                165                 170                 175

Gln Glu Ser Val Thr Glu Gln Asp Ser Lys Asp Ser Thr Tyr Ser Leu
            180                 185                 190

Ser Ser Thr Leu Thr Leu Ser Lys Ala Asp Tyr Glu Lys His Lys Val
        195                 200                 205

Tyr Ala Cys Glu Val Thr His Gln Gly Leu Ser Ser Pro Val Thr Lys
    210                 215                 220

Ser Phe Asn Arg Gly Glu Cys
225                 230

<210> 38
<211> 696
<212> DNA
<213> Artificial Sequence

<220>
<223> FN18 (VH-CL)

<400> 38
caggtgcagc tgcagcagag cgaggccgag ctggctagac ctggagccag cgtgaagatg 60

agctgcaagg ccagcggcta caccttcacc gactacacca tccactggct gaagcagcgg 120

cctggacagg gcctggactg gatcggctac ttcaacccca gcagcgagag caccgagtac 180

aaccggaagt tcaaggaccg gaccatcctg accgccgaca gaagcagcac caccgcctac 240

atgcagctga gcagcctgac cagcgaggac agcgccgtgt actactgcag ccggaagggc 300

gagaagctgc tgggcaacag atactggtac ttcgacgtgt ggggagccgg caccagcgtg 360

accgtgtcta gcgctagcgt ggctgcacca tctgtcttca tcttcccgcc atctgatgag 420

cagttgaaat ctggaactgc ctctgttgtg tgcctgctga ataacttcta tcccagagag 480

gccaaagtac agtggaaggt ggataacgcc ctccaatcgg gtaactccca ggagagtgtc 540

acagagcagg acagcaagga cagcacctac agcctcagca gcaccctgac gctgagcaaa 600

gcagactacg agaaacacaa agtctacgcc tgcgaagtca cccatcaggg cctgagctcg 660

cccgtcacaa agagcttcaa caggggagag tgttga                                696

<210>  39
<211>  664
<212>  PRT
<213>  Artificial Sequence

<220>
<223>  2C11 (VL-CH1)-LC007 (VH-CH1)-Fc(hole) P329G LALA

<400>  39

Asp Ile Gln Met Thr Gln Ser Pro Ser Ser Leu Pro Ala Ser Leu Gly
1               5                   10                  15

Asp Arg Val Thr Ile Asn Cys Gln Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Tyr Thr Asn Lys Leu Ala Asp Gly Val Pro Ser Arg Phe Ser Gly
    50                  55                  60

Ser Gly Ser Gly Arg Asp Ser Ser Phe Thr Ile Ser Ser Leu Glu Ser
65                  70                  75                  80

Glu Asp Ile Gly Ser Tyr Tyr Cys Gln Gln Tyr Tyr Asn Tyr Pro Trp
                85                  90                  95

Thr Phe Gly Pro Gly Thr Lys Leu Glu Ile Lys Ser Ser Ala Ser Thr
            100                 105                 110

Lys Gly Pro Ser Val Phe Pro Leu Ala Pro Ser Ser Lys Ser Thr Ser
        115                 120                 125

Gly Gly Thr Ala Ala Leu Gly Cys Leu Val Lys Asp Tyr Phe Pro Glu
    130                 135                 140

Pro Val Thr Val Ser Trp Asn Ser Gly Ala Leu Thr Ser Gly Val His
145                 150                 155                 160

Thr Phe Pro Ala Val Leu Gln Ser Ser Gly Leu Tyr Ser Leu Ser Ser
                165                 170                 175

Val Val Thr Val Pro Ser Ser Ser Leu Gly Thr Gln Thr Tyr Ile Cys
            180                 185                 190

Asn Val Asn His Lys Pro Ser Asn Thr Lys Val Asp Lys Lys Val Glu

195                 200                 205

Pro Lys Ser Cys Gly Gly Gly Gly Ser Gly Gly Gly Gly Ser Glu Val
    210                 215                 220

Gln Leu Gln Glu Ser Gly Pro Gly Leu Val Lys Pro Ser Gln Ser Leu
225                 230                 235                 240

Ser Leu Thr Cys Ser Val Thr Gly Tyr Ser Ile Thr Ser Gly Tyr Tyr
                245                 250                 255

Trp Asn Trp Ile Arg Gln Phe Pro Gly Asn Lys Leu Glu Trp Met Gly
            260                 265                 270

Tyr Ile Thr Tyr Asp Gly Ser Asn Asn Tyr Asn Pro Ser Leu Lys Asn
        275                 280                 285

Arg Ile Ser Ile Thr Arg Asp Thr Ser Lys Asn Gln Phe Phe Leu Lys
    290                 295                 300

Leu Asn Ser Val Thr Thr Glu Asp Thr Ala Thr Tyr Tyr Cys Ala Asp
305                 310                 315                 320

Phe Asp Tyr Trp Gly Gln Gly Thr Thr Leu Thr Val Ser Ser Ala Ser
                325                 330                 335

Thr Lys Gly Pro Ser Val Phe Pro Leu Ala Pro Ser Ser Lys Ser Thr
            340                 345                 350

Ser Gly Gly Thr Ala Ala Leu Gly Cys Leu Val Lys Asp Tyr Phe Pro
        355                 360                 365

Glu Pro Val Thr Val Ser Trp Asn Ser Gly Ala Leu Thr Ser Gly Val
    370                 375                 380

His Thr Phe Pro Ala Val Leu Gln Ser Ser Gly Leu Tyr Ser Leu Ser
385                 390                 395                 400

Ser Val Val Thr Val Pro Ser Ser Ser Leu Gly Thr Gln Thr Tyr Ile
                405                 410                 415

Cys Asn Val Asn His Lys Pro Ser Asn Thr Lys Val Asp Lys Lys Val
            420                 425                 430

Glu Pro Lys Ser Cys Asp Lys Thr His Thr Cys Pro Pro Cys Pro Ala
        435                 440                 445

Pro Glu Ala Ala Gly Gly Pro Ser Val Phe Leu Phe Pro Pro Lys Pro
    450                 455                 460

Lys Asp Thr Leu Met Ile Ser Arg Thr Pro Glu Val Thr Cys Val Val
465                 470                 475                 480

Val Asp Val Ser His Glu Asp Pro Glu Val Lys Phe Asn Trp Tyr Val
                485                 490                 495

Asp Gly Val Glu Val His Asn Ala Lys Thr Lys Pro Arg Glu Glu Gln
            500                 505                 510

Tyr Asn Ser Thr Tyr Arg Val Val Ser Val Leu Thr Val Leu His Gln
        515                 520                 525

Asp Trp Leu Asn Gly Lys Glu Tyr Lys Cys Lys Val Ser Asn Lys Ala
    530                 535                 540

Leu Gly Ala Pro Ile Glu Lys Thr Ile Ser Lys Ala Lys Gly Gln Pro
545                 550                 555                 560

Arg Glu Pro Gln Val Cys Thr Leu Pro Pro Ser Arg Asp Glu Leu Thr
                565                 570                 575

Lys Asn Gln Val Ser Leu Ser Cys Ala Val Lys Gly Phe Tyr Pro Ser
            580                 585                 590

Asp Ile Ala Val Glu Trp Glu Ser Asn Gly Gln Pro Glu Asn Asn Tyr
        595                 600                 605

Lys Thr Thr Pro Pro Val Leu Asp Ser Asp Gly Ser Phe Phe Leu Val
    610                 615                 620

Ser Lys Leu Thr Val Asp Lys Ser Arg Trp Gln Gln Gly Asn Val Phe
625                 630                 635                 640

Ser Cys Ser Val Met His Glu Ala Leu His Asn His Tyr Thr Gln Lys
                645                 650                 655

Ser Leu Ser Leu Ser Pro Gly Lys
            660

<210>  40
<211>  1995
<212>  DNA
<213>  Artificial Sequence

<220>
<223>  2C11 (VL-CH1)-LC007 (VH-CH1)-Fc(hole) P329G LALA

<400> 40
gacatccaga tgacccagag ccccagcagc ctgcctgcca gcctgggcga cagagtgacc 60

atcaactgcc aggccagcca ggacatcagc aactacctga actggtatca gcagaagcct 120

ggcaaggccc ccaagctgct gatctactac accaacaagc tggccgacgg cgtgcccagc 180

agattcagcg gcagcggctc cggcagagac agcagcttca ccatctccag cctggaaagc 240

gaggacatcg gcagctacta ctgccagcag tactacaact acccctggac cttcggccct 300

ggcaccaagc tggaaatcaa gagcagcgct tccaccaaag gcccttccgt gtttcctctg 360

gctcctagct ccaagtccac ctctggaggc accgctgctc tcggatgcct cgtgaaggat 420

tattttcctg agcctgtgac agtgtcctgg aatagcggag cactgacctc tggagtgcat 480

actttccccg ctgtgctgca gtcctctgga ctgtacagcc tgagcagcgt ggtgacagtg 540

cccagcagca gcctgggcac ccagacctac atctgcaacg tgaaccacaa gcccagcaac 600

accaaggtgg acaagaaggt ggaacccaag tcttgtggcg gaggcggatc cggcggaggg 660

ggatctgagg tgcagctgca ggaaagcggc cctggcctgg tgaaacccag ccagagcctg 720

agcctgacct gcagcgtgac cggctacagc atcaccagcg gctactactg gaactggatc 780

agacagttcc ccggcaacaa gctggaatgg atgggctaca tcacctacga cggcagcaac 840

aactacaacc ccagcctgaa gaacagaatc agcatcaccc gggacaccag caagaaccag 900

ttcttcctga agctgaacag cgtgaccacc gaggacaccg ccacctacta ctgcgccgac 960

ttcgactact ggggccaggg caccaccctg accgtgtcct ccgcctctac caagggcccc 1020

agcgtgttcc ccctggcacc cagcagcaag agcacatctg gcggaacagc cgctctgggc 1080

tgtctggtga aagactactt ccccgagccc gtgaccgtgt cttggaactc tggcgccctg 1140

accagcggcg tgcacacctt tccagccgtg ctgcagagca gcggcctgta ctccctgtcc 1200

tccgtggtca ccgtgccctc tagctccctg ggaacacaga catatatctg taatgtcaat 1260

cacaagcctt ccaacaccaa agtcgataag aaagtcgagc ccaagagctg cgacaaaact 1320

cacacatgcc caccgtgccc agcacctgaa gctgcagggg gaccgtcagt cttcctcttc 1380

cccccaaaac ccaaggacac cctcatgatc tcccggaccc ctgaggtcac atgcgtggtg 1440

gtggacgtga gccacgaaga ccctgaggtc aagttcaact ggtacgtgga cggcgtggag 1500

gtgcataatg ccaagacaaa gccgcgggag gagcagtaca acagcacgta ccgtgtggtc 1560

agcgtcctca ccgtcctgca ccaggactgg ctgaatggca aggagtacaa gtgcaaggtc 1620

tccaacaaag ccctcggcgc ccccatcgag aaaaccatct ccaaagccaa agggcagccc 1680

cgagaaccac aggtgtgcac cctgccccca tcccgggatg agctgaccaa gaaccaggtc 1740

agcctctcgt gcgcagtcaa aggcttctat cccagcgaca tcgccgtgga gtgggagagc 1800

aatgggcagc cggagaacaa ctacaagacc acgcctcccg tgctggactc cgacggctcc 1860

ttcttcctcg tgagcaagct caccgtggac aagagcaggt ggcagcaggg gaacgtcttc 1920

tcatgctccg tgatgcatga ggctctgcac aaccactaca cgcagaagag cctctccctg 1980

tctccgggta aatga 1995

<210> 41
<211> 223
<212> PRT
<213> Artificial Sequence

<220>
<223> 2C11 (VH-CL)

<400> 41

Glu Val Gln Leu Val Glu Ser Gly Gly Gly Leu Val Gln Pro Gly Lys
1               5                   10                  15

Ser Leu Lys Leu Ser Cys Glu Ala Ser Gly Phe Thr Phe Ser Gly Tyr
            20                  25                  30

Gly Met His Trp Val Arg Gln Ala Pro Gly Arg Gly Leu Glu Ser Val
        35                  40                  45

Ala Tyr Ile Thr Ser Ser Ser Ile Asn Ile Lys Tyr Ala Asp Ala Val
    50                  55                  60

Lys Gly Arg Phe Thr Val Ser Arg Asp Asn Ala Lys Asn Leu Leu Phe
65                  70                  75                  80

Leu Gln Met Asn Ile Leu Lys Ser Glu Asp Thr Ala Met Tyr Tyr Cys
                85                  90                  95

Ala Arg Phe Asp Trp Asp Lys Asn Tyr Trp Gly Gln Gly Thr Met Val
            100                 105                 110

Thr Val Ser Ser Ala Ser Val Ala Ala Pro Ser Val Phe Ile Phe Pro
        115                 120                 125

Pro Ser Asp Glu Gln Leu Lys Ser Gly Thr Ala Ser Val Val Cys Leu
    130                 135                 140

Leu Asn Asn Phe Tyr Pro Arg Glu Ala Lys Val Gln Trp Lys Val Asp
145                 150                 155                 160

Asn Ala Leu Gln Ser Gly Asn Ser Gln Glu Ser Val Thr Glu Gln Asp
                165                 170                 175

Ser Lys Asp Ser Thr Tyr Ser Leu Ser Ser Thr Leu Thr Leu Ser Lys

180                 185                 190

Ala Asp Tyr Glu Lys His Lys Val Tyr Ala Cys Glu Val Thr His Gln
        195                 200                 205

Gly Leu Ser Ser Pro Val Thr Lys Ser Phe Asn Arg Gly Glu Cys
    210                 215                 220

<210>  42
<211>  672
<212>  DNA
<213>  Artificial Sequence

<220>
<223>  2C11 (VH-CL)

<400>  42
gaggtgcagc tggtggaaag cggcggaggc ctggtgcagc ccggcaagag cctgaagctg      60

agctgcgagg ccagcggctt caccttcagc ggctacggca tgcactgggt gagacaggcc     120

cctggcagag gactggaaag cgtggcctac atcaccagca gcagcatcaa cattaagtac     180

gccgacgccg tgaagggccg gttcaccgtg tccagggata acgccaagaa cctgctgttc     240

ctgcagatga acatcctgaa gtccgaggac accgctatgt attactgcgc cagattcgac     300

tgggacaaga actactgggg ccagggcacc atggtcacag tgtctagcgc tagcgtggct     360

gcaccatctg tcttcatctt cccgccatct gatgagcagt tgaaatctgg aactgcctct     420

gttgtgtgcc tgctgaataa cttctatccc agagaggcca aagtacagtg gaaggtggat     480

aacgccctcc aatcgggtaa ctcccaggag agtgtcacag agcaggacag caaggacagc     540

acctacagcc tcagcagcac cctgacgctg agcaaagcag actacgagaa acacaaagtc     600

tacgcctgcg aagtcaccca tcagggcctg agctcgcccg tcacaaagag cttcaacagg     660

ggagagtgtt ga                                                         672

<210>  43
<211>  700
<212>  PRT
<213>  Artificial Sequence

<220>
<223>  V9 (scFab)-Fc(knob) P329G LALA

<400>  43

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Arg Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Arg Leu Glu Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Gly Asn Thr Leu Pro Trp
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys Ser Gly Gly Gly Ser Gly Gly Gly Ser Glu
    210                 215                 220

Gly Gly Gly Ser Glu Gly Gly Gly Ser Glu Gly Gly Gly Ser Glu Gly
225                 230                 235                 240

Gly Gly Ser Gly Gly Gly Ser Gly Glu Val Gln Leu Val Glu Ser Gly
                245                 250                 255

Gly Gly Leu Val Gln Pro Gly Gly Ser Leu Arg Leu Ser Cys Ala Ala
            260                 265                 270

Ser Gly Tyr Ser Phe Thr Gly Tyr Thr Met Asn Trp Val Arg Gln Ala
        275                 280                 285

Pro Gly Lys Gly Leu Glu Trp Val Ala Leu Ile Asn Pro Tyr Lys Gly
    290                 295                 300

Val Ser Thr Tyr Asn Gln Lys Phe Lys Asp Arg Phe Thr Ile Ser Val
305                 310                 315                 320

Asp Lys Ser Lys Asn Thr Ala Tyr Leu Gln Met Asn Ser Leu Arg Ala
                325                 330                 335

Glu Asp Thr Ala Val Tyr Tyr Cys Ala Arg Ser Gly Tyr Tyr Gly Asp
            340                 345                 350

Ser Asp Trp Tyr Phe Asp Val Trp Gly Gln Gly Thr Leu Val Thr Val
        355                 360                 365

Ser Ser Ala Ser Thr Lys Gly Pro Ser Val Phe Pro Leu Ala Pro Ser
    370                 375                 380

Ser Lys Ser Thr Ser Gly Gly Thr Ala Ala Leu Gly Cys Leu Val Lys
385                 390                 395                 400

Asp Tyr Phe Pro Glu Pro Val Thr Val Ser Trp Asn Ser Gly Ala Leu
                405                 410                 415

Thr Ser Gly Val His Thr Phe Pro Ala Val Leu Gln Ser Ser Gly Leu
            420                 425                 430

Tyr Ser Leu Ser Ser Val Val Thr Val Pro Ser Ser Ser Leu Gly Thr
        435                 440                 445

Gln Thr Tyr Ile Cys Asn Val Asn His Lys Pro Ser Asn Thr Lys Val
    450                 455                 460

Asp Lys Lys Val Glu Pro Lys Ser Cys Asp Lys Thr His Thr Cys Pro
465                 470                 475                 480

Pro Cys Pro Ala Pro Glu Ala Ala Gly Gly Pro Ser Val Phe Leu Phe
                485                 490                 495

Pro Pro Lys Pro Lys Asp Thr Leu Met Ile Ser Arg Thr Pro Glu Val
            500                 505                 510

Thr Cys Val Val Val Asp Val Ser His Glu Asp Pro Glu Val Lys Phe
        515                 520                 525

Asn Trp Tyr Val Asp Gly Val Glu Val His Asn Ala Lys Thr Lys Pro
    530                 535                 540

Arg Glu Glu Gln Tyr Asn Ser Thr Tyr Arg Val Val Ser Val Leu Thr
545                 550                 555                 560

Val Leu His Gln Asp Trp Leu Asn Gly Lys Glu Tyr Lys Cys Lys Val
                565                 570                 575

Ser Asn Lys Ala Leu Gly Ala Pro Ile Glu Lys Thr Ile Ser Lys Ala
            580                 585                 590

Lys Gly Gln Pro Arg Glu Pro Gln Val Tyr Thr Leu Pro Pro Cys Arg
        595                 600                 605

Asp Glu Leu Thr Lys Asn Gln Val Ser Leu Trp Cys Leu Val Lys Gly
    610                 615                 620

Phe Tyr Pro Ser Asp Ile Ala Val Glu Trp Glu Ser Asn Gly Gln Pro
625                 630                 635                 640

Glu Asn Asn Tyr Lys Thr Thr Pro Pro Val Leu Asp Ser Asp Gly Ser
                645                 650                 655

Phe Phe Leu Tyr Ser Lys Leu Thr Val Asp Lys Ser Arg Trp Gln Gln
            660                 665                 670

Gly Asn Val Phe Ser Cys Ser Val Met His Glu Ala Leu His Asn His
        675                 680                 685

Tyr Thr Gln Lys Ser Leu Ser Leu Ser Pro Gly Lys
    690                 695                 700

<210>  44
<211>  2103
<212>  DNA
<213>  Artificial Sequence

<220>
<223>  V9 (scFab)-Fc(knob) P329G LALA

<400>  44
gacatccaga tgacccagag cccctctagc ctgagcgcca gcgtgggcga cagagtgacc      60

atcacctgtc gggccagcca ggacatcaga aactacctga actggtatca gcagaagccc     120

ggcaaggccc ccaagctgct gatctactac acctctagac tggaaagcgg cgtgcccagc     180

cggtttagcg gcagcggctc cggcaccgac tacaccctga ccatcagcag cctgcagccc     240

gaggacttcg ccacctacta ctgccagcag ggcaacacac tcccctggac cttcggccag     300

ggcaccaagg tggagatcaa gcgtacggtg gccgctccca gcgtgttcat cttccccccc     360

agcgacgagc agctgaagtc cggcaccgcc agcgtcgtgt gcctgctgaa caacttctac 420

ccccgggagg ccaaggtgca gtggaaggtg gacaacgccc tgcagagcgg caacagccag 480

gaaagcgtca ccgagcagga cagcaaggac tccacctaca gcctgagcag caccctgacc 540

ctgagcaagg ccgactacga gaagcacaag gtgtacgcct gcgaagtgac ccaccagggc 600

ctgtccagcc ccgtgaccaa gagcttcaac cggggcgagt gcagcggcgg aggctctgga 660

ggcggctctg aaggcggagg aagtgagggc ggaggctcag aaggcggcgg aagcgaaggt 720

ggcggctctg gcggcggatc cggcgaggtg cagctggtcg agtccggcgg aggcctggtg 780

cagcctggcg gcagcctgag actgagctgc gccgccagcg gctacagctt caccggctac 840

accatgaact gggtccggca ggctcctggc aagggcctcg aatgggtggc cctgatcaac 900

ccctacaagg gcgtgagcac ctacaaccag aagttcaagg accggttcac catcagcgtg 960

gacaagagca agaacaccgc ctatctgcag atgaacagcc tgcgggccga ggacaccgcc 1020

gtgtactact gcgccagaag cggctactac ggcgacagcg actggtactt cgacgtgtgg 1080

ggccagggca cactggtcac cgtgtccagc gctagcacca agggccctag cgtgttccct 1140

ctggccccta gcagcaagag cacaagtgga ggaacagccg ccctgggctg cctggtcaag 1200

gactacttcc ccgagcccgt gaccgtgtcc tggaattctg gcgccctgac aagcggcgtg 1260

cacacatttc cagccgtgct gcagagcagc ggcctgtact ctctgagcag cgtcgtgacc 1320

gtgccctcta gctctctggg cacccagacc tacatctgca acgtgaacca caagcccagc 1380

aacaccaaag tggacaagaa ggtggaaccc aagagctgcg acaagaccca cacctgtccc 1440

ccttgccctg cccctgaagc tgctggtggc ccttccgtgt tcctgttccc cccaaagccc 1500

aaggacaccc tgatgatcag ccggaccccc gaagtgacct gcgtggtggt cgatgtgtcc 1560

cacgaggacc ctgaagtgaa gttcaattgg tacgtggacg gcgtggaagt gcacaatgcc 1620

aagaccaagc cgcgggagga gcagtacaac agcacgtacc gtgtggtcag cgtcctcacc 1680

gtcctgcacc aggactggct gaatggcaag gagtacaagt gcaaggtctc caacaaagcc 1740

ctcggcgccc ccatcgagaa aaccatctcc aaagccaaag ggcagccccg agaaccacag 1800

gtgtacaccc tgcccccatg ccgggatgag ctgaccaaga accaggtcag cctgtggtgc 1860

ctggtcaaag gcttctatcc cagcgacatc gccgtggagt gggagagcaa tgggcagccg 1920

gagaacaact acaagaccac gcctcccgtg ctggactccg acggctcctt cttcctctac 1980

agcaagctca ccgtggacaa gagcaggtgg cagcagggga acgtcttctc atgctccgtg 2040

atgcatgagg ctctgcacaa ccactacacg cagaagagcc tctccctgtc tccgggtaaa 2100

taa 2103

<210> 45
<211> 450

<212>  PRT
<213>  Artificial Sequence

<220>
<223>  GA201 (VH-CH1)-Fc(hole) P329G LALA

<400>  45

Gln Val Gln Leu Val Gln Ser Gly Ala Glu Val Lys Lys Pro Gly Ser
1               5                   10                  15

Ser Val Lys Val Ser Cys Lys Ala Ser Gly Phe Thr Phe Thr Asp Tyr
            20                  25                  30

Lys Ile His Trp Val Arg Gln Ala Pro Gly Gln Gly Leu Glu Trp Met
        35                  40                  45

Gly Tyr Phe Asn Pro Asn Ser Gly Tyr Ser Thr Tyr Ala Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Leu Ser Pro Gly Gly Tyr Tyr Val Met Asp Ala Trp Gly Gln
            100                 105                 110

Gly Thr Thr Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Ala Ala Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Gly Ala Pro Ile Glu
                325                 330                 335

Lys Thr Ile Ser Lys Ala Lys Gly Gln Pro Arg Glu Pro Gln Val Cys
            340                 345                 350

Thr Leu Pro Pro Ser Arg Asp Glu Leu Thr Lys Asn Gln Val Ser Leu
        355                 360                 365

Ser Cys Ala Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Val Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210>  46
<211>  1353

<212>  DNA
<213>  Artificial Sequence

<220>
<223>  GA201 (VH-CH1)-Fc(hole) P329G LALA

<400>  46
caggtgcagc tggtgcagtc tggggctgag gtgaagaagc ctgggtcctc ggtgaaggtc      60

tcctgcaagg cctctggttt cacattcact gactacaaga tacactgggt gcgacaggcc     120

cctggacaag ggctcgagtg gatgggatat ttcaacccta acagcggtta tagtacctac     180

gcacagaagt tccagggcag ggtcaccatt accgcggaca aatccacgag cacagcctac     240

atggagctga gcagcctgag atctgaggac acggccgtgt attactgtgc gagactatcc     300

ccaggcggtt actatgttat ggatgcctgg ggccaaggga ccaccgtgac cgtctcctca     360

gctagcacca agggcccatc ggtcttcccc ctggcaccct cctccaagag cacctctggg     420

ggcacagcgg ccctgggctg cctggtcaag gactacttcc ccgaaccggt gacggtgtcg     480

tggaactcag gcgccctgac cagcggcgtg cacaccttcc cggctgtcct acagtcctca     540

ggactctact ccctcagcag cgtggtgacc gtgccctcca gcagcttggg cacccagacc     600

tacatctgca acgtgaatca caagcccagc aacaccaagg tggacaagaa agttgagccc     660

aaatcttgtg acaaaactca cacatgccca ccgtgcccag cacctgaagc tgcaggggga     720

ccgtcagtct tcctcttccc cccaaaaccc aaggacaccc tcatgatctc ccggacccct     780

gaggtcacat gcgtggtggt ggacgtgagc cacgaagacc ctgaggtcaa gttcaactgg     840

tacgtggacg gcgtggaggt gcataatgcc aagacaaagc cgcgggagga gcagtacaac     900

agcacgtacc gtgtggtcag cgtcctcacc gtcctgcacc aggactggct gaatggcaag     960

gagtacaagt gcaaggtctc caacaaagcc ctcggcgccc ccatcgagaa aaccatctcc    1020

aaagccaaag ggcagccccg agaaccacag gtgtgcaccc tgcccccatc ccgggatgag    1080

ctgaccaaga accaggtcag cctctcgtgc gcagtcaaag gcttctatcc cagcgacatc    1140

gccgtggagt gggagagcaa tgggcagccg gagaacaact acaagaccac gcctcccgtg    1200

ctggactccg acggctcctt cttcctcgtg agcaagctca ccgtggacaa gagcaggtgg    1260

cagcagggga acgtcttctc atgctccgtg atgcatgagg ctctgcacaa ccactacacg    1320

cagaagagcc tctccctgtc tccgggtaaa tga                                 1353


<210>  47
<211>  213
<212>  PRT
<213>  Artificial Sequence

<220>
<223>  GA201 (VL-CL)

<400>  47

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Asn Asn Tyr
            20                  25                  30

Leu Asn Trp Tyr Gln Gln Lys Pro Gly Lys Ala Pro Lys Arg Leu Ile
        35                  40                  45

Tyr Asn Thr Asn Asn Leu Gln Thr Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Glu Asp Phe Ala Thr Tyr Tyr Cys Leu Gln His Asn Ser Phe Pro Thr
                85                  90                  95

Phe Gly Gln Gly Thr Lys Leu Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

<210>  48
<211>  642
<212>  DNA
<213>  Artificial Sequence

<220>

<223>  GA201 (VL-CL)

<400>  48
gatatccaga tgacccagtc tccatcctcc ctgtctgcat ctgtcggaga ccgggtcacc      60

atcacctgcc gggcaagtca gggcattaac aattacttaa attggtacca gcagaagcca     120

gggaaagccc ctaagcgcct gatctataat accaacaact tgcagacagg cgtcccatca     180

aggttcagcg gcagtggatc cgggacagaa ttcactctca ccatcagcag cctgcagcct     240

gaagattttg ccacctatta ctgcttgcag cataatagtt ttcccacgtt tggccagggc     300

accaagctcg agatcaagcg tacggtggct gcaccatctg tcttcatctt cccgccatct     360

gatgagcagt tgaaatctgg aactgcctct gttgtgtgcc tgctgaataa cttctatccc     420

agagaggcca aagtacagtg gaaggtggat aacgccctcc aatcgggtaa ctcccaggag     480

agtgtcacag agcaggacag caaggacagc acctacagcc tcagcagcac cctgacgctg     540

agcaaagcag actacgagaa acacaaagtc tacgcctgcg aagtcaccca tcagggcctg     600

agctcgcccg tcacaaagag cttcaacagg ggagagtgtt ag                        642


<210>  49
<211>  697
<212>  PRT
<213>  Artificial Sequence

<220>
<223>  GA201 (scFab)-Fc(knob) P329G LALA

<400>  49

Asp Ile Gln Met Thr Gln Ser Pro Ser Ser Leu Ser Ala Ser Val Gly
1               5                   10                  15


Asp Arg Val Thr Ile Thr Cys Arg Ala Ser Gln Gly Ile Asn Asn Tyr
            20                  25                  30


Leu Asn Trp Tyr Gln Gln Lys Pro Gly Lys Ala Pro Lys Arg Leu Ile
        35                  40                  45


Tyr Asn Thr Asn Asn Leu Gln Thr Gly Val Pro Ser Arg Phe Ser Gly
    50                  55                  60


Ser Gly Ser Gly Thr Glu Phe Thr Leu Thr Ile Ser Ser Leu Gln Pro
65                  70                  75                  80


Glu Asp Phe Ala Thr Tyr Tyr Cys Leu Gln His Asn Ser Phe Pro Thr
                85                  90                  95


Phe Gly Gln Gly Thr Lys Leu Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys Ser Gly Gly Gly Ser Gly Gly Gly Ser Glu Gly
    210                 215                 220

Gly Gly Ser Glu Gly Gly Gly Ser Glu Gly Gly Gly Ser Glu Gly Gly
225                 230                 235                 240

Gly Ser Gly Gly Gly Ser Gly Gln Val Gln Leu Val Gln Ser Gly Ala
                245                 250                 255

Glu Val Lys Lys Pro Gly Ser Ser Val Lys Val Ser Cys Lys Ala Ser
            260                 265                 270

Gly Phe Thr Phe Thr Asp Tyr Lys Ile His Trp Val Arg Gln Ala Pro
        275                 280                 285

Gly Gln Gly Leu Glu Trp Met Gly Tyr Phe Asn Pro Asn Ser Gly Tyr
    290                 295                 300

Ser Thr Tyr Ala Gln Lys Phe Gln Gly Arg Val Thr Ile Thr Ala Asp
305                 310                 315                 320

Lys Ser Thr Ser Thr Ala Tyr Met Glu Leu Ser Ser Leu Arg Ser Glu
                325                 330                 335

Asp Thr Ala Val Tyr Tyr Cys Ala Arg Leu Ser Pro Gly Gly Tyr Tyr
            340                 345                 350

Val Met Asp Ala Trp Gly Gln Gly Thr Thr Val Thr Val Ser Ser Ala
        355                 360                 365

Ser Thr Lys Gly Pro Ser Val Phe Pro Leu Ala Pro Ser Ser Lys Ser
    370                 375                 380

Thr Ser Gly Gly Thr Ala Ala Leu Gly Cys Leu Val Lys Asp Tyr Phe
385                 390                 395                 400

Pro Glu Pro Val Thr Val Ser Trp Asn Ser Gly Ala Leu Thr Ser Gly
                405                 410                 415

Val His Thr Phe Pro Ala Val Leu Gln Ser Ser Gly Leu Tyr Ser Leu
            420                 425                 430

Ser Ser Val Val Thr Val Pro Ser Ser Ser Leu Gly Thr Gln Thr Tyr
        435                 440                 445

Ile Cys Asn Val Asn His Lys Pro Ser Asn Thr Lys Val Asp Lys Lys
    450                 455                 460

Val Glu Pro Lys Ser Cys Asp Lys Thr His Thr Cys Pro Pro Cys Pro
465                 470                 475                 480

Ala Pro Glu Ala Ala Gly Gly Pro Ser Val Phe Leu Phe Pro Pro Lys
                485                 490                 495

Pro Lys Asp Thr Leu Met Ile Ser Arg Thr Pro Glu Val Thr Cys Val
            500                 505                 510

Val Val Asp Val Ser His Glu Asp Pro Glu Val Lys Phe Asn Trp Tyr
        515                 520                 525

Val Asp Gly Val Glu Val His Asn Ala Lys Thr Lys Pro Arg Glu Glu
    530                 535                 540

Gln Tyr Asn Ser Thr Tyr Arg Val Val Ser Val Leu Thr Val Leu His
545                 550                 555                 560

Gln Asp Trp Leu Asn Gly Lys Glu Tyr Lys Cys Lys Val Ser Asn Lys
                565                 570                 575

Ala Leu Gly Ala Pro Ile Glu Lys Thr Ile Ser Lys Ala Lys Gly Gln
            580                 585                 590

Pro Arg Glu Pro Gln Val Tyr Thr Leu Pro Pro Cys Arg Asp Glu Leu
        595                 600                 605

Thr Lys Asn Gln Val Ser Leu Trp Cys Leu Val Lys Gly Phe Tyr Pro

610                 615                 620

Ser Asp Ile Ala Val Glu Trp Glu Ser Asn Gly Gln Pro Glu Asn Asn
625                 630                 635                 640

Tyr Lys Thr Thr Pro Pro Val Leu Asp Ser Asp Gly Ser Phe Phe Leu
                645                 650                 655

Tyr Ser Lys Leu Thr Val Asp Lys Ser Arg Trp Gln Gln Gly Asn Val
            660                 665                 670

Phe Ser Cys Ser Val Met His Glu Ala Leu His Asn His Tyr Thr Gln
        675                 680                 685

Lys Ser Leu Ser Leu Ser Pro Gly Lys
    690                 695

<210>  50
<211>  2094
<212>  DNA
<213>  Artificial Sequence

<220>
<223>  GA201 (scFab)-Fc(knob) P329G LALA

<400>  50
gatatccaga tgacccagtc tccatcctcc ctgtctgcat ctgtcggaga ccgggtcacc      60

atcacctgcc gggcaagtca gggcattaac aattacttaa attggtacca gcagaagcca     120

gggaaagccc ctaagcgcct gatctataat accaacaact tgcagacagg cgtcccatca     180

aggttcagcg gcagtggatc cgggacagaa ttcactctca ccatcagcag cctgcagcct     240

gaagattttg ccacctatta ctgcttgcag cataatagtt ttcccacgtt tggccagggc     300

accaagctcg agatcaagcg tacggtggcc gctcccagcg tgttcatctt cccccccagc     360

gacgagcagc tgaaatctgg caccgccagc gtcgtgtgcc tgctgaacaa cttctacccc     420

cgggaggcca aggtgcagtg gaaggtggac aacgccctgc agagcggcaa cagccaggaa     480

agcgtcaccg agcaggacag caaggactcc acctatagcc tgtccagcac cctgaccctg     540

agcaaggccg actacgagaa gcacaaggtg tacgcctgcg aagtgaccca ccagggcctg     600

agcagccccg tgaccaagag cttcaaccgg ggcgagtgca gcggcggagg tagcggaggc     660

ggctctgagg gcggaggaag cgagggcgga ggctccgaag gcggcggaag cgaaggtggc     720

ggctctggcg gcggatccgg ccaggtgcag ctggtgcagt ctggggctga ggtgaagaag     780

cctgggtcct cggtgaaggt ctcctgcaag gcctctggtt tcacattcac tgactacaag     840

atacactggg tgcgacaggc ccctggacaa gggctcgagt ggatgggata tttcaaccct     900

aacagcggtt atagtaccta cgcacagaag ttccagggca gggtcaccat taccgcggac     960

aaatccacga gcacagccta catggagctg agcagcctga gatctgagga cacggccgtg 1020

tattactgtg cgagactatc cccaggcggt tactatgtta tggatgcctg gggccaaggg 1080

accaccgtga ccgtctcctc agctagcacc aagggcccta gcgtgttccc tctggcccct 1140

agcagcaaga gcacaagtgg aggaacagcc gccctgggct gcctggtcaa ggactacttc 1200

cccgagcccg tgaccgtgtc ctggaattct ggcgccctga caagcggcgt gcacacattt 1260

ccagccgtgc tgcagagcag cggcctgtac tctctgagca gcgtcgtgac cgtgccctct 1320

agctctctgg gcacccagac ctacatctgc aacgtgaacc acaagcccag caacaccaaa 1380

gtggacaaga aggtggaacc caagagctgc gacaagaccc acacctgtcc cccttgccct 1440

gcccctgaag ctgctggtgg cccttccgtg ttcctgttcc ccccaaagcc caaggacacc 1500

ctgatgatca gccggacccc cgaagtgacc tgcgtggtgg tcgatgtgtc ccacgaggac 1560

cctgaagtga agttcaattg gtacgtggac ggcgtggaag tgcacaatgc caagaccaag 1620

ccgcgggagg agcagtacaa cagcacgtac cgtgtggtca gcgtcctcac cgtcctgcac 1680

caggactggc tgaatggcaa ggagtacaag tgcaaggtct ccaacaaagc cctcggcgcc 1740

cccatcgaga aaaccatctc caaagccaaa gggcagcccc gagaaccaca ggtgtacacc 1800

ctgcccccat gccgggatga gctgaccaag aaccaggtca gcctgtggtg cctggtcaaa 1860

ggcttctatc ccagcgacat cgccgtggag tgggagagca atgggcagcc ggagaacaac 1920

tacaagacca cgcctcccgt gctggactcc gacggctcct tcttcctcta cagcaagctc 1980

accgtggaca agagcaggtg gcagcagggg aacgtcttct catgctccgt gatgcatgag 2040

gctctgcaca accactacac gcagaagagc ctctccctgt ctccgggtaa ataa 2094

<210> 51
<211> 452
<212> PRT
<213> Artificial Sequence

<220>
<223> V9 (VH-CH1)-Fc(hole) P329G LALA

<400> 51

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Ser Phe Thr Gly Tyr
            20                  25                  30

Thr Met Asn Trp Val Arg Gln Ala Pro Gly Lys Gly Leu Glu Trp Val
        35                  40                  45

Ala Leu Ile Asn Pro Tyr Lys Gly Val Ser Thr Tyr Asn Gln Lys Phe

50                  55                  60

Lys Asp Arg Phe Thr Ile Ser Val Asp Lys Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg Ser Gly Tyr Tyr Gly Asp Ser Asp Trp Tyr Phe Asp Val Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Ala Ala
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Gly Ala Pro
                325                 330                 335

Ile Glu Lys Thr Ile Ser Lys Ala Lys Gly Gln Pro Arg Glu Pro Gln
            340                 345                 350

Val Cys Thr Leu Pro Pro Ser Arg Asp Glu Leu Thr Lys Asn Gln Val
        355                 360                 365

Ser Leu Ser Cys Ala Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Val Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly Lys
    450

<210>  52
<211>  1359
<212>  DNA
<213>  Artificial Sequence

<220>
<223>  V9 (VH-CH1)-Fc(hole) P329G LALA

<400>  52
gaggtgcagc tggtcgagag cggaggcggc ctggtgcagc ctggcggcag cctgagactg      60

agctgcgccg ccagcggcta cagcttcacc ggctacacca tgaactgggt ccggcaggca     120

cctggcaagg gactggaatg ggtggccctg atcaacccct acaagggcgt gagcacctac     180

aaccagaagt tcaaggaccg gttcaccatc agcgtggaca agagcaagaa caccgcctat     240

ctgcagatga acagcctgcg ggccgaggac accgccgtgt actactgcgc cagaagcggc     300

tactacggcg acagcgactg gtacttcgac gtgtggggcc agggcaccct cgtgaccgtg     360

tctagcgcta gcaccaaggg cccctccgtg ttccccctgg cccccagcag caagagcacc     420

agcggcggca cagccgctct gggctgcctg gtcaaggact acttccccga gcccgtgacc 480

gtgtcctgga acagcggagc cctgacctcc ggcgtgcaca ccttccccgc cgtgctgcag 540

agttctggcc tgtatagcct gagcagcgtg gtcaccgtgc cttctagcag cctgggcacc 600

cagacctaca tctgcaacgt gaaccacaag cccagcaaca ccaaggtgga caagaaggtg 660

gagcccaaga gctgcgacaa aactcacaca tgcccaccgt gcccagcacc tgaagctgca 720

gggggaccgt cagtcttcct cttcccccca aaacccaagg acaccctcat gatctcccgg 780

acccctgagg tcacatgcgt ggtggtggac gtgagccacg aagaccctga ggtcaagttc 840

aactggtacg tggacggcgt ggaggtgcat aatgccaaga caaagccgcg ggaggagcag 900

tacaacagca cgtaccgtgt ggtcagcgtc ctcaccgtcc tgcaccagga ctggctgaat 960

ggcaaggagt acaagtgcaa ggtctccaac aaagccctcg gcgcccccat cgagaaaacc 1020

atctccaaag ccaaagggca gccccgagaa ccacaggtgt gcaccctgcc cccatcccgg 1080

gatgagctga ccaagaacca ggtcagcctc tcgtgcgcag tcaaaggctt ctatcccagc 1140

gacatcgccg tggagtggga gagcaatggg cagccggaga acaactacaa gaccacgcct 1200

cccgtgctgg actccgacgg ctccttcttc ctcgtgagca agctcaccgt ggacaagagc 1260

aggtggcagc aggggaacgt cttctcatgc tccgtgatgc atgaggctct gcacaaccac 1320

tacacgcaga agagcctctc cctgtctccg ggtaaatga 1359

<210> 53
<211> 934
<212> PRT
<213> Artificial Sequence

<220>
<223> V9 (scFab)-GA201 (VH-CH1)-Fc(knob) P329G LALA

<400> 53

<210>  54
<211>  2805
<212>  DNA
<213>  Artificial Sequence

<220>
<223>  V9 (scFab)-GA201 (VH-CH1)-Fc(knob) P329G LALA

<400>  54
gacatccaga tgacccagag cccctctagc ctgagcgcca gcgtgggcga cagagtgacc      60

atcacctgtc gggccagcca ggacatcaga aactacctga actggtatca gcagaagccc     120

ggcaaggccc ccaagctgct gatctactac acctctagac tggaaagcgg cgtgcccagc     180

cggtttagcg gcagcggctc cggcaccgac tacaccctga ccatcagcag cctgcagccc     240

gaggacttcg ccacctacta ctgccagcag ggcaacacac tcccctggac cttcggccag     300

ggcaccaagg tggagatcaa gcgtacggtg gccgctccca gcgtgttcat cttccccccc     360

agcgacgagc agctgaagtc cggcaccgcc agcgtcgtgt gcctgctgaa caacttctac     420

ccccgggagg ccaaggtgca gtggaaggtg gacaacgccc tgcagagcgg caacagccag     480

gaaagcgtca ccgagcagga cagcaaggac tccacctaca gcctgagcag caccctgacc     540

ctgagcaagg ccgactacga gaagcacaag gtgtacgcct gcgaagtgac ccaccagggc     600

ctgtccagcc ccgtgaccaa gagcttcaac cggggcgagt gcagcggcgg aggctctgga     660

ggcggctctg aaggcggagg aagtgagggc ggaggctcag aaggcggcgg aagcgaaggt     720

ggcggctctg gcggcggatc cggcgaggtg cagctggtcg agtccggcgg aggcctggtg     780

cagcctggcg gcagcctgag actgagctgc gccgccagcg gctacagctt caccggctac     840

accatgaact gggtccggca ggctcctggc aagggcctcg aatgggtggc cctgatcaac 900

ccctacaagg gcgtgagcac ctacaaccag aagttcaagg accggttcac catcagcgtg 960

gacaagagca agaacaccgc ctatctgcag atgaacagcc tgcgggccga ggacaccgcc 1020

gtgtactact gcgccagaag cggctactac ggcgacagcg actggtactt cgacgtgtgg 1080

ggccagggca cactggtcac cgtgtccagc gctagcacca agggcccctc cgtgttcccc 1140

ctggccccca gcagcaagag caccagcggc ggcacagccg ccctcggctg cctggtcaag 1200

gactacttcc ccgagcccgt gaccgtgtcc tggaacagcg gagccctgac ctccggcgtg 1260

cacaccttcc ccgccgtgct gcagagcagc ggcctgtaca gcctgtccag cgtggtcacc 1320

gtgccctcca gcagcctggg cacccagacc tacatctgca acgtgaacca caagcccagc 1380

aataccaagg tggacaagaa ggtggagccc aagagctgcg acggcggtgg tggctccgga 1440

ggcggtggat ctcaggtgca gctggtgcag tctggggctg aggtgaagaa gcctgggtcc 1500

tcggtgaagg tctcctgcaa ggcctctggt ttcacattca ctgactacaa gatacactgg 1560

gtgcgacagg cccctggaca agggctcgag tggatgggat atttcaaccc taacagcggt 1620

tatagtacct acgcacagaa gttccagggc agggtcacca ttaccgcgga caaatccacg 1680

agcacagcct acatggagct gagcagcctg agatctgagg acacggccgt gtattactgt 1740

gcgagactat ccccaggcgg ttactatgtt atggatgcct ggggccaagg gaccaccgtg 1800

accgtctcct cagctagcac caagggcccc tccgtgttcc ccctggcccc cagcagcaag 1860

agcaccagcg gcggcacagc cgctctgggc tgcctggtca aggactactt ccccgagccc 1920

gtgaccgtgt cctggaacag cggagccctg acctccggcg tgcacacctt ccccgccgtg 1980

ctgcagagtt ctggcctgta tagcctgagc agcgtggtca ccgtgccttc tagcagcctg 2040

ggcacccaga cctacatctg caacgtgaac cacaagccca gcaacaccaa ggtggacaag 2100

aaggtggagc ccaagagctg cgacaaaact cacacatgcc caccgtgccc agcacctgaa 2160

gctgcagggg gaccgtcagt cttcctcttc cccccaaaac ccaaggacac cctcatgatc 2220

tcccggaccc ctgaggtcac atgcgtggtg gtggacgtga gccacgaaga ccctgaggtc 2280

aagttcaact ggtacgtgga cggcgtggag gtgcataatg ccaagacaaa gccgcgggag 2340

gagcagtaca acagcacgta ccgtgtggtc agcgtcctca ccgtcctgca ccaggactgg 2400

ctgaatggca aggagtacaa gtgcaaggtc tccaacaaag ccctcggcgc ccccatcgag 2460

aaaaccatct ccaaagccaa agggcagccc cgagaaccac aggtgtacac cctgccccca 2520

tgccgggatg agctgaccaa gaaccaggtc agcctgtggt gcctggtcaa aggcttctat 2580

cccagcgaca tcgccgtgga gtgggagagc aatgggcagc cggagaacaa ctacaagacc 2640

acgcctcccg tgctggactc cgacggctcc ttcttcctct acagcaagct caccgtggac 2700

aagagcaggt ggcagcaggg gaacgtcttc tcatgctccg tgatgcatga ggctctgcac 2760

aaccactaca cgcagaagag cctctccctg tctccgggta aatga 2805

<210> 55
<211> 694
<212> PRT
<213> Artificial Sequence

<220>
<223> 3F2 (scFab)-Fc(knob) P329G LALA

<400> 55

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Thr Ser Ser
            20                  25                  30

Tyr Leu Ala Trp Tyr Gln Gln Lys Pro Gly Gln Ala Pro Arg Leu Leu
        35                  40                  45

Ile Asn Val Gly Ser Arg Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Gly Ile Met Leu Pro
                85                  90                  95

Pro Thr Phe Gly Gln Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys Gly Gly Gly Gly Ser Gly Gly Gly Gly
    210                 215                 220

Ser Gly Gly Gly Gly Ser Gly Gly Gly Gly Ser Gly Gly Gly Gly Ser
225                 230                 235                 240

Gly Gly Gly Gly Ser Gly Gly Glu Val Gln Leu Leu Glu Ser Gly Gly
                245                 250                 255

Gly Leu Val Gln Pro Gly Gly Ser Leu Arg Leu Ser Cys Ala Ala Ser
            260                 265                 270

Gly Phe Thr Phe Ser Ser Tyr Ala Met Ser Trp Val Arg Gln Ala Pro
        275                 280                 285

Gly Lys Gly Leu Glu Trp Val Ser Ala Ile Ser Gly Ser Gly Gly Ser
    290                 295                 300

Thr Tyr Tyr Ala Asp Ser Val Lys Gly Arg Phe Thr Ile Ser Arg Asp
305                 310                 315                 320

Asn Ser Lys Asn Thr Leu Tyr Leu Gln Met Asn Ser Leu Arg Ala Glu
                325                 330                 335

Asp Thr Ala Val Tyr Tyr Cys Ala Lys Gly Trp Phe Gly Gly Phe Asn
            340                 345                 350

Tyr Trp Gly Gln Gly Thr Leu Val Thr Val Ser Ser Ala Ser Thr Lys
        355                 360                 365

Gly Pro Ser Val Phe Pro Leu Ala Pro Ser Ser Lys Ser Thr Ser Gly
    370                 375                 380

Gly Thr Ala Ala Leu Gly Cys Leu Val Lys Asp Tyr Phe Pro Glu Pro
385                 390                 395                 400

Val Thr Val Ser Trp Asn Ser Gly Ala Leu Thr Ser Gly Val His Thr
                405                 410                 415

Phe Pro Ala Val Leu Gln Ser Ser Gly Leu Tyr Ser Leu Ser Ser Val
            420                 425                 430

Val Thr Val Pro Ser Ser Ser Leu Gly Thr Gln Thr Tyr Ile Cys Asn
        435                 440                 445

Val Asn His Lys Pro Ser Asn Thr Lys Val Asp Lys Lys Val Glu Pro
    450                 455                 460

Lys Ser Cys Asp Lys Thr His Thr Cys Pro Pro Cys Pro Ala Pro Glu
465                 470                 475                 480

Ala Ala Gly Gly Pro Ser Val Phe Leu Phe Pro Pro Lys Pro Lys Asp
                485                 490                 495

Thr Leu Met Ile Ser Arg Thr Pro Glu Val Thr Cys Val Val Val Asp
            500                 505                 510

Val Ser His Glu Asp Pro Glu Val Lys Phe Asn Trp Tyr Val Asp Gly
        515                 520                 525

Val Glu Val His Asn Ala Lys Thr Lys Pro Arg Glu Glu Gln Tyr Asn
    530                 535                 540

Ser Thr Tyr Arg Val Val Ser Val Leu Thr Val Leu His Gln Asp Trp
545                 550                 555                 560

Leu Asn Gly Lys Glu Tyr Lys Cys Lys Val Ser Asn Lys Ala Leu Gly
                565                 570                 575

Ala Pro Ile Glu Lys Thr Ile Ser Lys Ala Lys Gly Gln Pro Arg Glu
            580                 585                 590

Pro Gln Val Tyr Thr Leu Pro Pro Cys Arg Asp Glu Leu Thr Lys Asn
        595                 600                 605

Gln Val Ser Leu Trp Cys Leu Val Lys Gly Phe Tyr Pro Ser Asp Ile
    610                 615                 620

Ala Val Glu Trp Glu Ser Asn Gly Gln Pro Glu Asn Asn Tyr Lys Thr
625                 630                 635                 640

Thr Pro Pro Val Leu Asp Ser Asp Gly Ser Phe Phe Leu Tyr Ser Lys
                645                 650                 655

Leu Thr Val Asp Lys Ser Arg Trp Gln Gln Gly Asn Val Phe Ser Cys
            660                 665                 670

Ser Val Met His Glu Ala Leu His Asn His Tyr Thr Gln Lys Ser Leu
        675                 680                 685

Ser Leu Ser Pro Gly Lys
    690

<210>  56
<211>  2085
<212>  DNA
<213>  Artificial Sequence

<220>
<223>  3F2 (scFab)-Fc(knob) P329G LALA

<400>  56
gagatcgtgc tgacacagag ccccggaacc ctgtctctga gccctggcga aagagccacc      60

ctgagctgta gagccagcca gagcgtgacc agcagctacc tggcctggta tcagcagaag     120

cctggacagg cccccagact gctgatcaat gtgggcagca gacgggccac cggcatccct     180

gatagatttt ctggcagcgg cagcggcacc gacttcaccc tgaccatcag cagactggaa     240

cccgaggact tcgccgtgta ctactgccag cagggcatca tgctgccccc tacatttggc     300

cagggcacca aggtggaaat caagcgtacg gtggccgctc ccagcgtgtt catcttccca     360

cctagcgacg agcagctgaa gtctggcaca gccagcgtcg tgtgcctgct gaacaacttc     420

tacccccgcg aggccaaggt gcagtggaag gtggacaacg ccctgcagag cggcaacagc     480

caggaaagcg tcaccgagca ggacagcaag gactccacct acagcctgag cagcaccctg     540

accctgagca aggccgacta cgagaagcac aaggtgtacg cctgcgaagt gacccaccag     600

ggcctgtcta gccccgtgac caagagcttc aaccggggag aatgtggcgg cggaggatct     660

ggtggcggag gtagtggtgg tggtggatct ggcggaggcg gatccggcgg aggtggaagc     720

ggaggtggtg gaagtggggg agaagtgcag ctgctggaaa gtggcggagg cctggtgcag     780

cctggcggat ctctgagact gagctgtgcc gccagcggct tcacctttag cagctacgcc     840

atgagctggg tccgacaggc ccctggaaag ggactggaat gggtgtccgc catctctggc     900

tctggcggca gcacctacta cgccgatagc gtgaagggcc ggttcaccat cagccgggac     960

aacagcaaga acaccctgta cctgcagatg aacagcctgc gggccgagga taccgccgtg    1020

tattattgcg ccaagggatg gttcggcggc ttcaactatt ggggccaggg aaccctggtc    1080

accgtgtcta gtgctagcac caagggccct agcgtgttcc ctctggcccc tagcagcaag    1140

agcacaagtg gaggaacagc cgccctgggc tgcctggtca aggactactt ccccgagccc    1200

gtgaccgtgt cctggaattc tggcgccctg acaagcggcg tgcacacatt tccagccgtg    1260

ctgcagagca gcggcctgta ctctctgagc agcgtcgtga ccgtgccctc tagctctctg    1320

ggcacccaga cctacatctg caacgtgaac cacaagccca gcaacaccaa agtggacaag    1380

aaggtggaac ccaagagctg cgacaagacc cacacctgtc ccccttgccc tgcccctgaa    1440

gctgctggtg gcccttccgt gttcctgttc cccccaaagc ccaaggacac cctgatgatc    1500

agccggaccc ccgaagtgac ctgcgtggtg gtcgatgtgt cccacgagga ccctgaagtg    1560

aagttcaatt ggtacgtgga cggcgtggaa gtgcacaatg ccaagaccaa gccgcgggag 1620

gagcagtaca acagcacgta ccgtgtggtc agcgtcctca ccgtcctgca ccaggactgg 1680

ctgaatggca aggagtacaa gtgcaaggtc tccaacaaag ccctcggcgc ccccatcgag 1740

aaaaccatct ccaaagccaa agggcagccc cgagaaccac aggtgtacac cctgccccca 1800

tgccgggatg agctgaccaa gaaccaggtc agcctgtggt gcctggtcaa aggcttctat 1860

cccagcgaca tcgccgtgga gtgggagagc aatgggcagc cggagaacaa ctacaagacc 1920

acgcctcccg tgctggactc cgacggctcc ttcttcctct acagcaagct caccgtggac 1980

aagagcaggt ggcagcaggg gaacgtcttc tcatgctccg tgatgcatga ggctctgcac 2040

aaccactaca cgcagaagag cctctccctg tctccgggta aataa 2085

<210> 57
<211> 931
<212> PRT
<213> Artificial Sequence

<220>
<223> V9 (scFab)-3F2 (VH-CH1)-Fc(knob) P329G LALA

<400> 57

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Arg Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Arg Leu Glu Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Gly Asn Thr Leu Pro Trp
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys Ser Gly Gly Gly Ser Gly Gly Gly Ser Glu
    210                 215                 220

Gly Gly Gly Ser Glu Gly Gly Gly Ser Glu Gly Gly Gly Ser Glu Gly
225                 230                 235                 240

Gly Gly Ser Gly Gly Gly Ser Gly Glu Val Gln Leu Val Glu Ser Gly
                245                 250                 255

Gly Gly Leu Val Gln Pro Gly Gly Ser Leu Arg Leu Ser Cys Ala Ala
            260                 265                 270

Ser Gly Tyr Ser Phe Thr Gly Tyr Thr Met Asn Trp Val Arg Gln Ala
        275                 280                 285

Pro Gly Lys Gly Leu Glu Trp Val Ala Leu Ile Asn Pro Tyr Lys Gly
    290                 295                 300

Val Ser Thr Tyr Asn Gln Lys Phe Lys Asp Arg Phe Thr Ile Ser Val
305                 310                 315                 320

Asp Lys Ser Lys Asn Thr Ala Tyr Leu Gln Met Asn Ser Leu Arg Ala
                325                 330                 335

Glu Asp Thr Ala Val Tyr Tyr Cys Ala Arg Ser Gly Tyr Tyr Gly Asp
            340                 345                 350

Ser Asp Trp Tyr Phe Asp Val Trp Gly Gln Gly Thr Leu Val Thr Val
        355                 360                 365

Ser Ser Ala Ser Thr Lys Gly Pro Ser Val Phe Pro Leu Ala Pro Ser
    370                 375                 380

Ser Lys Ser Thr Ser Gly Gly Thr Ala Ala Leu Gly Cys Leu Val Lys

385                 390                 395                 400

Asp Tyr Phe Pro Glu Pro Val Thr Val Ser Trp Asn Ser Gly Ala Leu
                405                 410                 415

Thr Ser Gly Val His Thr Phe Pro Ala Val Leu Gln Ser Ser Gly Leu
            420                 425                 430

Tyr Ser Leu Ser Ser Val Val Thr Val Pro Ser Ser Ser Leu Gly Thr
        435                 440                 445

Gln Thr Tyr Ile Cys Asn Val Asn His Lys Pro Ser Asn Thr Lys Val
    450                 455                 460

Asp Lys Lys Val Glu Pro Lys Ser Cys Asp Gly Gly Gly Gly Ser Gly
465                 470                 475                 480

Gly Gly Gly Ser Glu Val Gln Leu Leu Glu Ser Gly Gly Gly Leu Val
                485                 490                 495

Gln Pro Gly Gly Ser Leu Arg Leu Ser Cys Ala Ala Ser Gly Phe Thr
            500                 505                 510

Phe Ser Ser Tyr Ala Met Ser Trp Val Arg Gln Ala Pro Gly Lys Gly
        515                 520                 525

Leu Glu Trp Val Ser Ala Ile Ser Gly Ser Gly Gly Ser Thr Tyr Tyr
    530                 535                 540

Ala Asp Ser Val Lys Gly Arg Phe Thr Ile Ser Arg Asp Asn Ser Lys
545                 550                 555                 560

Asn Thr Leu Tyr Leu Gln Met Asn Ser Leu Arg Ala Glu Asp Thr Ala
                565                 570                 575

Val Tyr Tyr Cys Ala Lys Gly Trp Phe Gly Gly Phe Asn Tyr Trp Gly
            580                 585                 590

Gln Gly Thr Leu Val Thr Val Ser Ser Ala Ser Thr Lys Gly Pro Ser
        595                 600                 605

Val Phe Pro Leu Ala Pro Ser Ser Lys Ser Thr Ser Gly Gly Thr Ala
    610                 615                 620

Ala Leu Gly Cys Leu Val Lys Asp Tyr Phe Pro Glu Pro Val Thr Val
625                 630                 635                 640

Ser Trp Asn Ser Gly Ala Leu Thr Ser Gly Val His Thr Phe Pro Ala
                645                 650                 655

Val Leu Gln Ser Ser Gly Leu Tyr Ser Leu Ser Ser Val Val Thr Val
            660                 665                 670

Pro Ser Ser Ser Leu Gly Thr Gln Thr Tyr Ile Cys Asn Val Asn His
        675                 680                 685

Lys Pro Ser Asn Thr Lys Val Asp Lys Lys Val Glu Pro Lys Ser Cys
    690                 695                 700

Asp Lys Thr His Thr Cys Pro Pro Cys Pro Ala Pro Glu Ala Ala Gly
705                 710                 715                 720

Gly Pro Ser Val Phe Leu Phe Pro Pro Lys Pro Lys Asp Thr Leu Met
                725                 730                 735

Ile Ser Arg Thr Pro Glu Val Thr Cys Val Val Val Asp Val Ser His
            740                 745                 750

Glu Asp Pro Glu Val Lys Phe Asn Trp Tyr Val Asp Gly Val Glu Val
        755                 760                 765

His Asn Ala Lys Thr Lys Pro Arg Glu Glu Gln Tyr Asn Ser Thr Tyr
    770                 775                 780

Arg Val Val Ser Val Leu Thr Val Leu His Gln Asp Trp Leu Asn Gly
785                 790                 795                 800

Lys Glu Tyr Lys Cys Lys Val Ser Asn Lys Ala Leu Gly Ala Pro Ile
                805                 810                 815

Glu Lys Thr Ile Ser Lys Ala Lys Gly Gln Pro Arg Glu Pro Gln Val
            820                 825                 830

Tyr Thr Leu Pro Pro Cys Arg Asp Glu Leu Thr Lys Asn Gln Val Ser
        835                 840                 845

Leu Trp Cys Leu Val Lys Gly Phe Tyr Pro Ser Asp Ile Ala Val Glu
    850                 855                 860

Trp Glu Ser Asn Gly Gln Pro Glu Asn Asn Tyr Lys Thr Thr Pro Pro
865                 870                 875                 880

Val Leu Asp Ser Asp Gly Ser Phe Phe Leu Tyr Ser Lys Leu Thr Val
                885                 890                 895

Asp Lys Ser Arg Trp Gln Gln Gly Asn Val Phe Ser Cys Ser Val Met
            900                 905                 910

His Glu Ala Leu His Asn His Tyr Thr Gln Lys Ser Leu Ser Leu Ser
        915                 920                 925

Pro Gly Lys
    930

<210>  58
<211>  2796
<212>  DNA
<213>  Artificial Sequence

<220>
<223>  V9 (scFab)-3F2 (VH-CH1)-Fc(knob) P329G LALA

<400>  58
gacatccaga tgacccagag cccctctagc ctgagcgcca gcgtgggcga cagagtgacc      60

atcacctgtc gggccagcca ggacatcaga aactacctga actggtatca gcagaagccc     120

ggcaaggccc ccaagctgct gatctactac acctctagac tggaaagcgg cgtgcccagc     180

cggtttagcg gcagcggctc cggcaccgac tacaccctga ccatcagcag cctgcagccc     240

gaggacttcg ccacctacta ctgccagcag ggcaacacac tcccctggac cttcggccag     300

ggcaccaagg tggagatcaa gcgtacggtg gccgctccca gcgtgttcat cttccccccc     360

agcgacgagc agctgaagtc cggcaccgcc agcgtcgtgt gcctgctgaa caacttctac     420

ccccgggagg ccaaggtgca gtggaaggtg gacaacgccc tgcagagcgg caacagccag     480

gaaagcgtca ccgagcagga cagcaaggac tccacctaca gcctgagcag caccctgacc     540

ctgagcaagg ccgactacga gaagcacaag gtgtacgcct gcgaagtgac ccaccagggc     600

ctgtccagcc ccgtgaccaa gagcttcaac cggggcgagt gcagcggcgg aggctctgga     660

ggcggctctg aaggcggagg aagtgagggc ggaggctcag aaggcggcgg aagcgaaggt     720

ggcggctctg gcggcggatc cggcgaggtg cagctggtcg agtccggcgg aggcctggtg     780

cagcctggcg gcagcctgag actgagctgc gccgccagcg gctacagctt caccggctac     840

accatgaact gggtccggca ggctcctggc aagggcctcg aatgggtggc cctgatcaac     900

ccctacaagg gcgtgagcac ctacaaccag aagttcaagg accggttcac catcagcgtg     960

gacaagagca agaacaccgc ctatctgcag atgaacagcc tgcgggccga ggacaccgcc    1020

gtgtactact gcgccagaag cggctactac ggcgacagcg actggtactt cgacgtgtgg    1080

ggccagggca cactggtcac cgtgtccagc gctagcacca agggcccctc cgtgttcccc    1140

ctggccccca gcagcaagag caccagcggc ggcacagccg ccctcggctg cctggtcaag    1200

gactacttcc ccgagcccgt gaccgtgtcc tggaacagcg gagccctgac ctccggcgtg    1260

cacaccttcc ccgccgtgct gcagagcagc ggcctgtaca gcctgtccag cgtggtcacc 1320

gtgccctcca gcagcctggg cacccagacc tacatctgca acgtgaacca caagcccagc 1380

aataccaagg tggacaagaa ggtggagccc aagagctgcg acggcggtgg tggctccgga 1440

ggaggaggca gcgaggtgca gctgctggaa tctggaggcg gcctggtgca gcctggcggc 1500

agcctgagac tgtcttgcgc cgccagcggc ttcaccttca gcagctacgc catgagctgg 1560

gtccgacagg ctcctggcaa gggactggaa tgggtgtccg ccatctccgg cagcggaggc 1620

agcacctact acgccgacag cgtgaagggc cggttcacca tcagcagaga caacagcaag 1680

aacaccctgt acctgcagat gaacagcctg cgggccgagg ataccgccgt gtattattgc 1740

gccaagggat ggttcggcgg cttcaactac tggggccagg gaaccctggt gacagtgtcc 1800

agcgccagca ccaagggccc ctccgtgttt cctctggccc ccagcagcaa gagcacctct 1860

ggcggaacag ccgccctggg ctgcctggtg aaagactact tccccgagcc cgtgaccgtg 1920

tcctggaact ctggcgccct gaccagcggc gtgcacacct ttccagccgt gctgcagagc 1980

agcggcctgt actccctgag cagcgtggtg acagtgccct ccagcagcct gggcacccag 2040

acctacatct gcaacgtgaa ccacaagccc agcaacacca aagtggacaa gaaggtggaa 2100

cccaagagct gcgacaaaac tcacacatgc ccaccgtgcc cagcacctga agctgcaggg 2160

ggaccgtcag tcttcctctt ccccccaaaa cccaaggaca ccctcatgat ctcccggacc 2220

cctgaggtca catgcgtggt ggtggacgtg agccacgaag accctgaggt caagttcaac 2280

tggtacgtgg acggcgtgga ggtgcataat gccaagacaa agccgcggga ggagcagtac 2340

aacagcacgt accgtgtggt cagcgtcctc accgtcctgc accaggactg gctgaatggc 2400

aaggagtaca agtgcaaggt ctccaacaaa gccctcggcg cccccatcga gaaaaccatc 2460

tccaaagcca aagggcagcc ccgagaacca caggtgtaca ccctgccccc atgccgggat 2520

gagctgacca agaaccaggt cagcctgtgg tgcctggtca aaggcttcta tcccagcgac 2580

atcgccgtgg agtgggagag caatgggcag ccggagaaca actacaagac cacgcctccc 2640

gtgctggact ccgacggctc cttcttcctc tacagcaagc tcaccgtgga caagagcagg 2700

tggcagcagg ggaacgtctt ctcatgctcc gtgatgcatg aggctctgca caaccactac 2760

acgcagaaga gcctctccct gtctccgggt aaatga 2796

<210> 59
<211> 447
<212> PRT
<213> Artificial Sequence

<220>
<223> 3F2 (VH-CH1)-Fc(hole) P329G LALA

<400> 59

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Trp Phe Gly Gly Phe Asn Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Ala Ala Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Gly Ala Pro Ile Glu Lys Thr Ile
                325                 330                 335

Ser Lys Ala Lys Gly Gln Pro Arg Glu Pro Gln Val Cys Thr Leu Pro
            340                 345                 350

Pro Ser Arg Asp Glu Leu Thr Lys Asn Gln Val Ser Leu Ser Cys Ala
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Val Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly Lys
        435                 440                 445

<210> 60
<211> 1344
<212> DNA
<213> Artificial Sequence

<220>
<223> 3F2 (VH-CH1)-Fc(hole) P329G LALA

<400> 60
gaggtgcagc tgctggaatc tggaggcggc ctggtgcagc ctggcggcag cctgagactg 60

tcttgcgccg ccagcggctt caccttcagc agctacgcca tgagctgggt ccgacaggct 120

cctggcaagg gactggaatg ggtgtccgcc atctccggca gcggaggcag cacctactac 180

gccgacagcg tgaagggccg gttcaccatc agcagagaca acagcaagaa caccctgtac 240

ctgcagatga acagcctgcg ggccgaggat accgccgtgt attattgcgc caagggatgg 300

ttcggcggct tcaactactg gggccaggga accctggtga cagtgtccag cgccagcacc 360

aagggcccct ccgtgtttcc tctggccccc agcagcaaga gcacctctgg cggaacagcc 420

gccctgggct gcctggtgaa agactacttc cccgagcccg tgaccgtgtc ctggaactct 480

ggcgccctga ccagcggcgt gcacaccttt ccagccgtgc tgcagagcag cggcctgtac 540

tccctgagca gcgtggtgac agtgccctcc agcagcctgg gcacccagac ctacatctgc 600

aacgtgaacc acaagcccag caacaccaaa gtggacaaga aggtggaacc caagagctgc 660

gacaaaactc acacatgccc accgtgccca gcacctgaag ctgcaggggg accgtcagtc 720

ttcctcttcc ccccaaaacc caaggacacc ctcatgatct cccggacccc tgaggtcaca 780

tgcgtggtgg tggacgtgag ccacgaagac cctgaggtca agttcaactg gtacgtggac 840

ggcgtggagg tgcataatgc caagacaaag ccgcgggagg agcagtacaa cagcacgtac 900

cgtgtggtca gcgtcctcac cgtcctgcac caggactggc tgaatggcaa ggagtacaag 960

tgcaaggtct ccaacaaagc cctcggcgcc cccatcgaga aaaccatctc caaagccaaa 1020

gggcagcccc gagaaccaca ggtgtgcacc ctgcccccat cccgggatga gctgaccaag 1080

aaccaggtca gcctctcgtg cgcagtcaaa ggcttctatc ccagcgacat cgccgtggag 1140

tgggagagca atgggcagcc ggagaacaac tacaagacca cgcctcccgt gctggactcc 1200

gacggctcct tcttcctcgt gagcaagctc accgtggaca agagcaggtg gcagcagggg 1260

aacgtcttct catgctccgt gatgcatgag gctctgcaca accactacac gcagaagagc 1320

ctctccctgt ctccgggtaa atga 1344

<210> 61
<211> 215
<212> PRT
<213> Artificial Sequence

<220>
<223> 3F2 (VL-CL)

<400> 61

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Thr Ser Ser
            20                  25                  30

Tyr Leu Ala Trp Tyr Gln Gln Lys Pro Gly Gln Ala Pro Arg Leu Leu
        35                  40                  45

Ile Asn Val Gly Ser Arg Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Gly Ile Met Leu Pro
                85                  90                  95

Pro Thr Phe Gly Gln Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  62
<211>  648
<212>  DNA
<213>  Artificial Sequence

<220>
<223>  3F2 (VL-CL)

<400>  62
gagatcgtgc tgacccagtc tcccggcacc ctgagcctga gccctggcga gagagccacc      60

ctgagctgca gagccagcca gagcgtgacc agcagctacc tggcctggta tcagcagaag     120

cccggccagg cccccagact gctgatcaac gtgggcagca gacgggccac cggcatcccc     180

gatagattca gcggcagcgg ctccggcacc gacttcaccc tgaccatcag ccggctggaa     240

cccgaggact tcgccgtgta ctactgccag cagggcatca tgctgccccc caccttcggc 300

cagggcacca aggtggaaat caagcggacc gtggccgctc ccagcgtgtt catcttccca 360

cccagcgacg agcagctgaa gtccggcaca gccagcgtgg tgtgcctgct gaacaacttc 420

tacccccgcg aggccaaggt gcagtggaag gtggacaacg ccctgcagag cggcaacagc 480

caggaatccg tgaccgagca ggacagcaag gactccacct acagcctgag cagcaccctg 540

accctgagca aggccgacta cgagaagcac aaggtgtacg cctgcgaagt gacccaccag 600

ggcctgtcca gccccgtgac caagagcttc aaccggggcg agtgctga 648

<210> 63
<211> 673
<212> PRT
<213> Artificial Sequence

<220>
<223> CH1A1A (VH-CH1)- V9 (VL-CH1)-Fc(knob) P329G LALA

<400> 63

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Glu Phe
            20                  25                  30

Gly Met Asn Trp Val Arg Gln Ala Pro Gly Gln Gly Leu Glu Trp Met
        35                  40                  45

Gly Trp Ile Asn Thr Lys Thr Gly Glu Ala Thr Tyr Val Glu Glu Phe
    50                  55                  60

Lys Gly Arg Val Thr Phe Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Trp Asp Phe Ala Tyr Tyr Val Glu Ala Met Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Gly Gly Gly Gly Ser Gly Gly Gly Gly Ser Asp Ile Gln Met Thr Gln
225                 230                 235                 240

Ser Pro Ser Ser Leu Ser Ala Ser Val Gly Asp Arg Val Thr Ile Thr
                245                 250                 255

Cys Arg Ala Ser Gln Asp Ile Arg Asn Tyr Leu Asn Trp Tyr Gln Gln
            260                 265                 270

Lys Pro Gly Lys Ala Pro Lys Leu Leu Ile Tyr Tyr Thr Ser Arg Leu
        275                 280                 285

Glu Ser Gly Val Pro Ser Arg Phe Ser Gly Ser Gly Ser Gly Thr Asp
    290                 295                 300

Tyr Thr Leu Thr Ile Ser Ser Leu Gln Pro Glu Asp Phe Ala Thr Tyr
305                 310                 315                 320

Tyr Cys Gln Gln Gly Asn Thr Leu Pro Trp Thr Phe Gly Gln Gly Thr
                325                 330                 335

Lys Val Glu Ile Lys Ser Ser Ala Ser Thr Lys Gly Pro Ser Val Phe
            340                 345                 350

Pro Leu Ala Pro Ser Ser Lys Ser Thr Ser Gly Gly Thr Ala Ala Leu
        355                 360                 365

Gly Cys Leu Val Lys Asp Tyr Phe Pro Glu Pro Val Thr Val Ser Trp
    370                 375                 380

Asn Ser Gly Ala Leu Thr Ser Gly Val His Thr Phe Pro Ala Val Leu
385                 390                 395                 400

Gln Ser Ser Gly Leu Tyr Ser Leu Ser Ser Val Val Thr Val Pro Ser

                405                 410                 415

Ser Ser Leu Gly Thr Gln Thr Tyr Ile Cys Asn Val Asn His Lys Pro
            420                 425                 430

Ser Asn Thr Lys Val Asp Lys Lys Val Glu Pro Lys Ser Cys Asp Lys
        435                 440                 445

Thr His Thr Cys Pro Pro Cys Pro Ala Pro Glu Ala Ala Gly Gly Pro
    450                 455                 460

Ser Val Phe Leu Phe Pro Pro Lys Pro Lys Asp Thr Leu Met Ile Ser
465                 470                 475                 480

Arg Thr Pro Glu Val Thr Cys Val Val Val Asp Val Ser His Glu Asp
                485                 490                 495

Pro Glu Val Lys Phe Asn Trp Tyr Val Asp Gly Val Glu Val His Asn
            500                 505                 510

Ala Lys Thr Lys Pro Arg Glu Glu Gln Tyr Asn Ser Thr Tyr Arg Val
        515                 520                 525

Val Ser Val Leu Thr Val Leu His Gln Asp Trp Leu Asn Gly Lys Glu
    530                 535                 540

Tyr Lys Cys Lys Val Ser Asn Lys Ala Leu Gly Ala Pro Ile Glu Lys
545                 550                 555                 560

Thr Ile Ser Lys Ala Lys Gly Gln Pro Arg Glu Pro Gln Val Tyr Thr
                565                 570                 575

Leu Pro Pro Cys Arg Asp Glu Leu Thr Lys Asn Gln Val Ser Leu Trp
            580                 585                 590

Cys Leu Val Lys Gly Phe Tyr Pro Ser Asp Ile Ala Val Glu Trp Glu
        595                 600                 605

Ser Asn Gly Gln Pro Glu Asn Asn Tyr Lys Thr Thr Pro Pro Val Leu
    610                 615                 620

Asp Ser Asp Gly Ser Phe Phe Leu Tyr Ser Lys Leu Thr Val Asp Lys
625                 630                 635                 640

Ser Arg Trp Gln Gln Gly Asn Val Phe Ser Cys Ser Val Met His Glu
                645                 650                 655

Ala Leu His Asn His Tyr Thr Gln Lys Ser Leu Ser Leu Ser Pro Gly
            660                 665                 670

Lys

<210>  64
<211>  2022
<212>  DNA
<213>  Artificial Sequence

<220>
<223>  CH1A1A (VH-CH1)- V9 (VL-CH1)-Fc(knob) P329G LALA

<400>  64
caggtgcagc tggtgcagtc tggcgccgaa gtgaagaaac ctggcgccag cgtgaaggtg      60

tcctgcaagg ccagcggcta caccttcacc gagttcggca tgaactgggt ccgacaggcc     120

cctggacagg gcctggaatg gatgggctgg atcaacacca agaccggcga ggccacctac     180

gtggaagagt tcaagggcag agtgaccttc accaccgaca ccagcaccag caccgcctac     240

atggaactgc ggagcctgag aagcgacgac accgccgtgt actactgcgc cagatgggac     300

ttcgcctact atgtggaagc catggactac tggggccagg gcaccaccgt gaccgtgtct     360

agtgctagca caaagggccc cagcgtgttc cctctggccc ctagcagcaa gagcacatct     420

ggcggaacag ccgccctggg ctgcctggtc aaggactact tcccgagccc cgtgacagtg     480

tcctggaact ctggcgccct gacaagcggc gtgcacacct ttccagccgt gctgcagagc     540

agcggcctgt actctctgag cagcgtggtc accgtgccta gctctagcct gggcacccag     600

acctacatct gcaacgtgaa ccacaagccc agcaacacca aggtggacaa gaaggtggaa     660

cccaagagct gcggcggagg cggatccgga ggcggaggat ctgatatcca gatgacccag     720

agccccagca gcctgtctgc cagcgtgggc gacagagtga ccattacctg cagagccagc     780

caggacatca gaaactacct gaactggtat cagcagaagc ccggcaaggc ccccaagctg     840

ctgatctact acaccagcag actggaatcc ggcgtgccca gcagattttc cggcagcggc     900

tctggcaccg actacaccct gacaatcagc agcctgcagc ccgaggactt cgccacctac     960

tactgccagc agggcaacac cctgccctgg acatttggac agggcacaaa ggtggaaatc    1020

aagagcagcg cctccaccaa gggcccttcc gtgtttccac tggcccccag ctctaagagc    1080

accagcggag gaacagctgc tctgggatgt ctcgtgaagg attacttccc cgaacctgtg    1140

accgtcagct ggaacagcgg cgctctgaca tctggggtgc acacattccc cgctgtcctg    1200

cagtcctccg gcctgtacag tctgtccagc gtcgtgacag tgcctagcag ctccctggga    1260

acacagacat atatctgtaa tgtcaatcac aagccctcta ataccaaggt cgacaaaaaa    1320

gtcgagccca agtcctgcga caagacccac acctgtcccc cttgtcctgc ccctgaagct    1380

gctggcggcc cttctgtgtt cctgttcccc ccaaagccca aggacaccct gatgatcagc 1440

cggacccccg aagtgacctg cgtggtggtg gatgtgtccc acgaggaccc tgaagtgaag 1500

ttcaattggt acgtggacgg cgtggaagtg cacaacgcca agacaaagcc gcgggaggag 1560

cagtacaaca gcacgtaccg tgtggtcagc gtcctcaccg tcctgcacca ggactggctg 1620

aatggcaagg agtacaagtg caaggtctcc aacaaagccc tcggcgcccc catcgagaaa 1680

accatctcca aagccaaagg gcagccccga gaaccacagg tgtacaccct gcccccatgc 1740

cgggatgagc tgaccaagaa ccaggtcagc ctgtggtgcc tggtcaaagg cttctatccc 1800

agcgacatcg ccgtggagtg ggagagcaat gggcagccgg agaacaacta caagaccacg 1860

cctcccgtgc tggactccga cggctccttc ttcctctaca gcaagctcac cgtggacaag 1920

agcaggtggc agcaggggaa cgtcttctca tgctccgtga tgcatgaggc tctgcacaac 1980

cactacacgc agaagagcct ctccctgtct ccgggtaaat ga 2022

<210> 65
<211> 451
<212> PRT
<213> Artificial Sequence

<220>
<223> CH1A1A (VH-CH1)-Fc(hole) P329G LALA

<400> 65

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Glu Phe
            20                  25                  30

Gly Met Asn Trp Val Arg Gln Ala Pro Gly Gln Gly Leu Glu Trp Met
        35                  40                  45

Gly Trp Ile Asn Thr Lys Thr Gly Glu Ala Thr Tyr Val Glu Glu Phe
    50                  55                  60

Lys Gly Arg Val Thr Phe Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Trp Asp Phe Ala Tyr Tyr Val Glu Ala Met Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Ala Ala Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Gly Ala Pro Ile
                325                 330                 335

Glu Lys Thr Ile Ser Lys Ala Lys Gly Gln Pro Arg Glu Pro Gln Val
            340                 345                 350

Cys Thr Leu Pro Pro Ser Arg Asp Glu Leu Thr Lys Asn Gln Val Ser
        355                 360                 365

Leu Ser Cys Ala Val Lys Gly Phe Tyr Pro Ser Asp Ile Ala Val Glu

370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Val Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450

<210>  66
<211>  1356
<212>  DNA
<213>  Artificial Sequence

<220>
<223>  CH1A1A (VH-CH1)-Fc(hole) P329G LALA

<400>  66
caggtgcagc tggtgcagtc tggcgccgaa gtgaagaaac ctggagctag tgtgaaggtg      60

tcctgcaagg ccagcggcta caccttcacc gagttcggca tgaactgggt ccgacaggct     120

ccaggccagg gcctcgaatg gatgggctgg atcaacacca agaccggcga ggccacctac     180

gtggaagagt tcaagggcag agtgaccttc accacggaca ccagcaccag caccgcctac     240

atggaactgc ggagcctgag aagcgacgac accgccgtgt actactgcgc cagatgggac     300

ttcgcctatt acgtggaagc catggactac tggggccagg gcaccaccgt gaccgtgtct     360

agcgctagca ccaagggccc ctccgtgttc cccctggccc ccagcagcaa gagcaccagc     420

ggcggcacag ccgctctggg ctgcctggtc aaggactact tccccgagcc cgtgaccgtg     480

tcctggaaca gcggagccct gacctccggc gtgcacacct tccccgccgt gctgcagagt     540

tctggcctgt atagcctgag cagcgtggtc accgtgcctt ctagcagcct gggcacccag     600

acctacatct gcaacgtgaa ccacaagccc agcaacacca aggtggacaa gaaggtggag     660

cccaagagct gcgacaaaac tcacacatgc ccaccgtgcc cagcacctga agctgcaggg     720

ggaccgtcag tcttcctctt ccccccaaaa cccaaggaca ccctcatgat ctcccggacc     780

cctgaggtca catgcgtggt ggtggacgtg agccacgaag accctgaggt caagttcaac     840

tggtacgtgg acggcgtgga ggtgcataat gccaagacaa agccgcggga ggagcagtac     900

aacagcacgt accgtgtggt cagcgtcctc accgtcctgc accaggactg gctgaatggc     960

aaggagtaca agtgcaaggt ctccaacaaa gccctcggcg cccccatcga gaaaaccatc 1020

tccaaagcca aagggcagcc ccgagaacca caggtgtgca ccctgccccc atcccgggat 1080

gagctgacca agaaccaggt cagcctctcg tgcgcagtca aaggcttcta tcccagcgac 1140

atcgccgtgg agtgggagag caatgggcag ccggagaaca actacaagac cacgcctccc 1200

gtgctggact ccgacggctc cttcttcctc gtgagcaagc tcaccgtgga caagagcagg 1260

tggcagcagg ggaacgtctt ctcatgctcc gtgatgcatg aggctctgca caaccactac 1320

acgcagaaga gcctctccct gtctccgggt aaatga 1356

<210> 67
<211> 215
<212> PRT
<213> Artificial Sequence

<220>
<223> CH1A1A (VL-CL)

<400> 67

Asp Ile Gln Met Thr Gln Ser Pro Ser Ser Leu Ser Ala Ser Val Gly
1               5                   10                  15

Asp Arg Val Thr Ile Thr Cys Lys Ala Ser Ala Ala Val Gly Thr Tyr
            20                  25                  30

Val Ala Trp Tyr Gln Gln Lys Pro Gly Lys Ala Pro Lys Leu Leu Ile
        35                  40                  45

Tyr Ser Ala Ser Tyr Arg Lys Arg Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys His Gln Tyr Tyr Thr Tyr Pro Leu
                85                  90                  95

Phe Thr Phe Gly Gln Gly Thr Lys Leu Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser

145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  68
<211>  648
<212>  DNA
<213>  Artificial Sequence

<220>
<223>  CH1A1A (VL-CL)

<400>  68
gatatccaga tgacccagtc tccatcctcc ctgtctgcat ctgtgggaga cagagtcacc      60

atcacttgca aggccagtgc ggctgtgggt acgtatgttg cgtggtatca gcagaaacca     120

gggaaagcac ctaagctcct gatctattcg gcatcctacc gcaaaagggg agtcccatca     180

aggttcagtg gcagtggatc tgggacagat ttcactctca ccatcagcag tctgcaacct     240

gaagatttcg caacttacta ctgtcaccaa tattacacct atcctctatt cacgtttggc     300

cagggcacca agctcgagat caagcgtacg gtggctgcac catctgtctt catcttcccg     360

ccatctgatg agcagttgaa atctggaact gcctctgttg tgtgcctgct gaataacttc     420

tatcccagag aggccaaagt acagtggaag gtggataacg ccctccaatc gggtaactcc     480

caggagagtg tcacagagca ggacagcaag gacagcacct acagcctcag cagcaccctg     540

acgctgagca aagcagacta cgagaaacac aaagtctacg cctgcgaagt cacccatcag     600

ggcctgagct cgcccgtcac aaagagcttc aacaggggag agtgttag                  648

<210>  69
<211>  11
<212>  PRT
<213>  Artificial Sequence

<220>
<223>  LC007 HCDR1

<400>  69

Gly Tyr Ser Ile Thr Ser Gly Tyr Tyr Trp Asn
1               5                   10

<210>  70
<211>  33
<212>  DNA
<213>  Artificial Sequence

<220>
<223>  LC007 HCDR1

<400>  70
ggctactcca tcaccagtgg ttattactgg aac                                    33


<210>  71
<211>  16
<212>  PRT
<213>  Artificial Sequence

<220>
<223>  LC007 HCDR2

<400>  71

Tyr Ile Thr Tyr Asp Gly Ser Asn Asn Tyr Asn Pro Ser Leu Lys Asn
1               5                   10                  15


<210>  72
<211>  48
<212>  DNA
<213>  Artificial Sequence

<220>
<223>  LC007 HCDR2

<400>  72
tacataacct acgacggtag caataactac aacccatctc tcaaaaat                    48


<210>  73
<211>  3
<212>  PRT
<213>  Artificial Sequence

<220>
<223>  LC007 HCDR3

<400>  73

Phe Asp Tyr
1


<210>  74
<211>  9
<212>  DNA
<213>  Artificial Sequence

<220>
<223>  LC007 HCDR3

<400>  74

tttgactac                                                              9

<210>  75
<211>  112
<212>  PRT
<213>  Artificial Sequence

<220>
<223>  LC007 VH

<400>  75

Glu Val Gln Leu Gln Glu Ser Gly Pro Gly Leu Val Lys Pro Ser Gln
1               5                   10                  15

Ser Leu Ser Leu Thr Cys Ser Val Thr Gly Tyr Ser Ile Thr Ser Gly
            20                  25                  30

Tyr Tyr Trp Asn Trp Ile Arg Gln Phe Pro Gly Asn Lys Leu Glu Trp
        35                  40                  45

Met Gly Tyr Ile Thr Tyr Asp Gly Ser Asn Asn Tyr Asn Pro Ser Leu
    50                  55                  60

Lys Asn Arg Ile Ser Ile Thr Arg Asp Thr Ser Lys Asn Gln Phe Phe
65                  70                  75                  80

Leu Lys Leu Asn Ser Val Thr Thr Glu Asp Thr Ala Thr Tyr Tyr Cys
                85                  90                  95

Ala Asp Phe Asp Tyr Trp Gly Gln Gly Thr Thr Leu Thr Val Ser Ser
            100                 105                 110

<210>  76
<211>  336
<212>  DNA
<213>  Artificial Sequence

<220>
<223>  LC007 VH

<400>  76
gaggtccagc tgcaggagtc aggacctggc ctcgtgaaac cttctcagtc tctgtctctc      60

acctgctctg tcactggcta ctccatcacc agtggttatt actggaactg gatccggcag     120

tttccaggaa acaagctgga atggatgggc tacataacct acgacggtag caataactac     180

aacccatctc tcaaaaatcg aatctccatc actcgtgaca catctaagaa ccagtttttc     240

ctgaagttga attctgtgac tactgaggac acagctacat attactgtgc ggactttgac     300

tactggggcc aaggcaccac tctcacagtc tcctca                               336

<210>  77
<211>  11
<212>  PRT
<213>  Artificial Sequence

<220>
<223>  LC007 LCDR1

<400>  77

Ser Ala Ser Gln Gly Ile Arg Asn Tyr Leu Asn
1               5                   10


<210>  78
<211>  33
<212>  DNA
<213>  Artificial Sequence

<220>
<223>  LC007 LCDR1

<400>  78
agtgcaagtc agggcattag aaattattta aac                                    33


<210>  79
<211>  7
<212>  PRT
<213>  Artificial Sequence

<220>
<223>  LC007 LCDR2

<400>  79

Tyr Thr Ser Ser Leu His Ser
1               5


<210>  80
<211>  21
<212>  DNA
<213>  Artificial Sequence

<220>
<223>  LC007 LCDR2

<400>  80
tacacatcaa gtttacactc a                                                 21


<210>  81
<211>  9
<212>  PRT
<213>  Artificial Sequence

<220>
<223>  LC007 LCDR3

<400>  81

Gln Gln Tyr Ser Lys Leu Pro Trp Thr

1               5


<210>  82
<211>  27
<212>  DNA
<213>  Artificial Sequence

<220>
<223>  LC007 LCDR3

<400>  82
cagcagtata gtaagcttcc ttggacg                                          27


<210>  83
<211>  107
<212>  PRT
<213>  Artificial Sequence

<220>
<223>  LC007 VL

<400>  83

Asp Ile Val Leu Thr Gln Ser Pro Ser Ser Leu Ser Ala Ser Leu Gly
1               5                   10                  15


Asp Arg Val Thr Ile Ser Cys Ser Ala Ser Gln Gly Ile Arg Asn Tyr
            20                  25                  30


Leu Asn Trp Tyr Gln Gln Arg Pro Asp Gly Thr Val Lys Leu Leu Ile
        35                  40                  45


Tyr Tyr Thr Ser Ser Leu His Ser Gly Val Pro Ser Arg Phe Ser Gly
    50                  55                  60


Ser Gly Ser Gly Thr Asp Tyr Ser Leu Thr Ile Ser Asn Leu Glu Pro
65                  70                  75                  80


Glu Asp Ile Ala Thr Tyr Tyr Cys Gln Gln Tyr Ser Lys Leu Pro Trp
                85                  90                  95


Thr Phe Gly Gly Gly Thr Lys Leu Glu Ile Lys
            100                 105


<210>  84
<211>  321
<212>  DNA
<213>  Artificial Sequence

<220>
<223>  LC007 VL

<400>  84
gatattgtgc tcacacagtc tccatcctcc ctgtctgcct ctctgggaga cagagtcacc      60

atcagttgca gtgcaagtca gggcattaga aattatttaa actggtatca gcagagacca 120

gatggaactg ttaaactcct gatctattac acatcaagtt tacactcagg agtcccatca 180

aggttcagtg gcagtgggtc tgggacagat tattctctca ccatcagcaa cctggaacct 240

gaagatattg ccacttacta ttgtcagcag tatagtaagc ttccttggac gttcggtgga 300

ggcaccaagc tggaaatcaa a 321

<210> 85
<211> 5
<212> PRT
<213> Artificial Sequence

<220>
<223> GA201 HCDR1

<400> 85

Asp Tyr Lys Ile His
1               5

<210> 86
<211> 15
<212> DNA
<213> Artificial Sequence

<220>
<223> GA201 HCDR1

<400> 86
gactacaaga tacac 15

<210> 87
<211> 17
<212> PRT
<213> Artificial Sequence

<220>
<223> GA201 HCDR2

<400> 87

Tyr Phe Asn Pro Asn Ser Gly Tyr Ser Thr Tyr Ala Gln Lys Phe Gln
1               5                   10                  15

Gly

<210> 88
<211> 51
<212> DNA
<213> Artificial Sequence

<220>
<223> GA201 HCDR2

<400>  88
tatttcaacc ctaacagcgg ttatagtacc tacgcacaga agttccaggg c                51

<210>  89
<211>  11
<212>  PRT
<213>  Artificial Sequence

<220>
<223>  GA201 HCDR3

<400>  89

Leu Ser Pro Gly Gly Tyr Tyr Val Met Asp Ala
1               5                   10

<210>  90
<211>  33
<212>  DNA
<213>  Artificial Sequence

<220>
<223>  GA201 HCDR3

<400>  90
ctatccccag gcggttacta tgttatggat gcc                                    33

<210>  91
<211>  120
<212>  PRT
<213>  Artificial Sequence

<220>
<223>  GA201 VH

<400>  91

<210>  92
<211>  360
<212>  DNA
<213>  Artificial Sequence

<220>
<223>  GA201 VH

<400>  92
caggtgcagc tggtgcagtc tggggctgag gtgaagaagc ctgggtcctc ggtgaaggtc      60

tcctgcaagg cctctggttt cacattcact gactacaaga tacactgggt gcgacaggcc     120

cctggacaag ggctcgagtg gatgggatat ttcaacccta acagcggtta tagtacctac     180

gcacagaagt tccagggcag ggtcaccatt accgcggaca aatccacgag cacagcctac     240

atggagctga gcagcctgag atctgaggac acggccgtgt attactgtgc gagactatcc     300

ccaggcggtt actatgttat ggatgcctgg ggccaaggga ccaccgtgac cgtctcctca     360

<210>  93
<211>  11
<212>  PRT
<213>  Artificial Sequence

<220>
<223>  GA201 LCDR1

<400>  93

Arg Ala Ser Gln Gly Ile Asn Asn Tyr Leu Asn
1               5                   10

<210>  94
<211>  33
<212>  DNA
<213>  Artificial Sequence

<220>
<223>  GA201 LCDR1

<400>  94
cgggcaagtc agggcattaa caattactta aat                                   33

<210>  95
<211>  7
<212>  PRT
<213>  Artificial Sequence

<220>
<223>  GA201 LCDR2

<400>  95

Asn Thr Asn Asn Leu Gln Thr
1               5


<210>  96
<211>  21
<212>  DNA
<213>  Artificial Sequence

<220>
<223>  GA201 LCDR2

<400>  96
aataccaaca acttgcagac a                                                21


<210>  97
<211>  8
<212>  PRT
<213>  Artificial Sequence

<220>
<223>  GA201 LCDR3

<400>  97

Leu Gln His Asn Ser Phe Pro Thr
1               5


<210>  98
<211>  24
<212>  DNA
<213>  Artificial Sequence

<220>
<223>  GA201 LCDR3

<400>  98
ttgcagcata atagttttcc cacg                                             24


<210>  99
<211>  106
<212>  PRT
<213>  Artificial Sequence

<220>
<223>  GA201 VL

<400>  99

<210>  100
<211>  318
<212>  DNA
<213>  Artificial Sequence

<220>
<223>  GA201 VL

<400>  100
gatatccaga tgacccagtc tccatcctcc ctgtctgcat ctgtcggaga ccgggtcacc      60

atcacctgcc gggcaagtca gggcattaac aattacttaa attggtacca gcagaagcca     120

gggaaagccc ctaagcgcct gatctataat accaacaact tgcagacagg cgtcccatca     180

aggttcagcg gcagtggatc cgggacagaa ttcactctca ccatcagcag cctgcagcct     240

gaagattttg ccacctatta ctgcttgcag cataatagtt ttcccacgtt tggccagggc     300

accaagctcg agatcaag                                                   318

<210>  101
<211>  5
<212>  PRT
<213>  Artificial Sequence

<220>
<223>  3F2 HCDR1

<400>  101

Ser Tyr Ala Met Ser
1               5

<210>  102
<211>  15
<212>  DNA
<213>  Artificial Sequence

<220>
<223>  3F2 HCDR1

<400>  102
agctacgcca tgagc                                                        15


<210>  103
<211>  16
<212>  PRT
<213>  Artificial Sequence

<220>
<223>  3F2 HCDR2

<400>  103

Ala Ile Ser Gly Ser Gly Gly Ser Thr Tyr Tyr Ala Asp Ser Val Lys
1               5                   10                  15


<210>  104
<211>  48
<212>  DNA
<213>  Artificial Sequence

<220>
<223>  3F2 HCDR2

<400>  104
gccatctccg gcagcggagg cagcacctac tacgccgaca gcgtgaag                    48


<210>  105
<211>  8
<212>  PRT
<213>  Artificial Sequence

<220>
<223>  3F2 HCDR3

<400>  105

Tyr Cys Ala Lys Gly Trp Phe Gly
1               5


<210>  106
<211>  24
<212>  DNA
<213>  Artificial Sequence

<220>
<223>  3F2 HCDR3

<400>  106
tattgcgcca agggatggtt cggc                                              24


<210>  107
<211>  117
<212>  PRT
<213>  Artificial Sequence

<220>
<223>  3F2 VH

<400>  107

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Trp Phe Gly Gly Phe Asn Tyr Trp Gly Gln Gly Thr Leu
            100                 105                 110

Val Thr Val Ser Ser
        115

<210>  108
<211>  351
<212>  DNA
<213>  Artificial Sequence

<220>
<223>  3F2 VH

<400>  108
gaggtgcagc tgctggaatc tggaggcggc ctggtgcagc ctggcggcag cctgagactg      60

tcttgcgccg ccagcggctt caccttcagc agctacgcca tgagctgggt ccgacaggct     120

cctggcaagg gactggaatg ggtgtccgcc atctccggca gcggaggcag cacctactac     180

gccgacagcg tgaagggccg gttcaccatc agcagagaca acagcaagaa caccctgtac     240

ctgcagatga acagcctgcg ggccgaggat accgccgtgt attattgcgc caagggatgg     300

ttcggcggct tcaactactg gggccaggga accctggtga cagtgtccag c              351

<210>  109
<211>  11
<212>  PRT

<213>  Artificial Sequence

<220>
<223>  3F2 LCDR1

<400>  109

Arg Ala Ser Gln Ser Val Thr Ser Ser Tyr Leu
1               5                   10


<210>  110
<211>  33
<212>  DNA
<213>  Artificial Sequence

<220>
<223>  3F2 LCDR1

<400>  110
agagccagcc agagcgtgac cagcagctac ctg                                    33


<210>  111
<211>  7
<212>  PRT
<213>  Artificial Sequence

<220>
<223>  3F2 LCDR2

<400>  111

Asn Val Gly Ser Arg Arg Ala
1               5


<210>  112
<211>  21
<212>  DNA
<213>  Artificial Sequence

<220>
<223>  3F2 LCDR2

<400>  112
aacgtgggca gcagacgggc c                                                 21


<210>  113
<211>  9
<212>  PRT
<213>  Artificial Sequence

<220>
<223>  3F2 LCDR3

<400>  113

Cys Gln Gln Gly Ile Met Leu Pro Pro
1               5

<210>  114
<211>  27
<212>  DNA
<213>  Artificial Sequence

<220>
<223>  3F2 LCDR3

<400>  114
tgccagcagg gcatcatgct gcccccc                                          27


<210>  115
<211>  108
<212>  PRT
<213>  Artificial Sequence

<220>
<223>  3F2 VL

<400>  115

Glu Ile Val Leu Thr Gln Ser Pro Gly Thr Leu Ser Leu Ser Pro Gly
1               5                   10                  15


Glu Arg Ala Thr Leu Ser Cys Arg Ala Ser Gln Ser Val Thr Ser Ser
            20                  25                  30


Tyr Leu Ala Trp Tyr Gln Gln Lys Pro Gly Gln Ala Pro Arg Leu Leu
        35                  40                  45


Ile Asn Val Gly Ser Arg Arg Ala Thr Gly Ile Pro Asp Arg Phe Ser
    50                  55                  60


Gly Ser Gly Ser Gly Thr Asp Phe Thr Leu Thr Ile Ser Arg Leu Glu
65                  70                  75                  80


Pro Glu Asp Phe Ala Val Tyr Tyr Cys Gln Gln Gly Ile Met Leu Pro
                85                  90                  95


Pro Thr Phe Gly Gln Gly Thr Lys Val Glu Ile Lys
            100                 105


<210>  116
<211>  324
<212>  DNA
<213>  Artificial Sequence

<220>
<223>  3F2 VL

<400>  116
gagatcgtgc tgacccagtc tcccggcacc ctgagcctga gccctggcga gagagccacc      60

ctgagctgca gagccagcca gagcgtgacc agcagctacc tggcctggta tcagcagaag     120

cccggccagg cccccagact gctgatcaac gtgggcagca gacgggccac cggcatcccc 180

gatagattca gcggcagcgg ctccggcacc gacttcaccc tgaccatcag ccggctggaa 240

cccgaggact tcgccgtgta ctactgccag cagggcatca tgctgccccc caccttcggc 300

cagggcacca aggtggaaat caag 324

<210> 117
<211> 5
<212> PRT
<213> Artificial Sequence

<220>
<223> CH1A1A HCDR1

<400> 117

Glu Phe Gly Met Asn
1               5

<210> 118
<211> 15
<212> DNA
<213> Artificial Sequence

<220>
<223> CH1A1A HCDR1

<400> 118
gagttcggca tgaac 15

<210> 119
<211> 17
<212> PRT
<213> Artificial Sequence

<220>
<223> CH1A1A HCDR2

<400> 119

Trp Ile Asn Thr Lys Thr Gly Glu Ala Thr Tyr Val Glu Glu Phe Lys
1               5                   10                  15

Gly

<210> 120
<211> 51
<212> DNA
<213> Artificial Sequence

<220>
<223> CH1A1A HCDR2

<400> 120
tggatcaaca ccaagaccgg cgaggccacc tacgtggaag agttcaaggg c 51

<210>  121
<211>  12
<212>  PRT
<213>  Artificial Sequence

<220>
<223>  CH1A1A HCDR3

<400>  121

Trp Asp Phe Ala Tyr Tyr Val Glu Ala Met Asp Tyr
1               5                   10


<210>  122
<211>  36
<212>  DNA
<213>  Artificial Sequence

<220>
<223>  CH1A1A HCDR3

<400>  122
tgggacttcg cctattacgt ggaagccatg gactac                                36


<210>  123
<211>  121
<212>  PRT
<213>  Artificial Sequence

<220>
<223>  CH1A1A VH

<400>  123

Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Glu Phe
            20                  25                  30


Gly Met Asn Trp Val Arg Gln Ala Pro Gly Gln Gly Leu Glu Trp Met
        35                  40                  45


Gly Trp Ile Asn Thr Lys Thr Gly Glu Ala Thr Tyr Val Glu Glu Phe
    50                  55                  60


Lys Gly Arg Val Thr Phe Thr Thr Asp Thr Ser Thr Ser Thr Ala Tyr
65                  70                  75                  80


Met Glu Leu Arg Ser Leu Arg Ser Asp Asp Thr Ala Val Tyr Tyr Cys
                85                  90                  95


Ala Arg Trp Asp Phe Ala Tyr Tyr Val Glu Ala Met Asp Tyr Trp Gly

100                 105                 110

Gln Gly Thr Thr Val Thr Val Ser Ser
        115                 120

<210>  124
<211>  363
<212>  DNA
<213>  Artificial Sequence

<220>
<223>  CH1A1A VH

<400>  124
caggtgcagc tggtgcagtc tggcgccgaa gtgaagaaac ctggagctag tgtgaaggtg        60

tcctgcaagg ccagcggcta caccttcacc gagttcggca tgaactgggt ccgacaggct       120

ccaggccagg gcctcgaatg gatgggctgg atcaacacca agaccggcga ggccacctac       180

gtggaagagt tcaagggcag agtgaccttc accacggaca ccagcaccag caccgcctac       240

atggaactgc ggagcctgag aagcgacgac accgccgtgt actactgcgc cagatgggac       300

ttcgcctatt acgtggaagc catggactac tggggccagg gcaccaccgt gaccgtgtct       360

agc                                                                     363

<210>  125
<211>  11
<212>  PRT
<213>  Artificial Sequence

<220>
<223>  CH1A1A LCDR1

<400>  125

Lys Ala Ser Ala Ala Val Gly Thr Tyr Val Ala
1               5                   10

<210>  126
<211>  33
<212>  DNA
<213>  Artificial Sequence

<220>
<223>  CH1A1A LCDR1

<400>  126
aaggccagtg cggctgtggg tacgtatgtt gcg                                     33

<210>  127
<211>  7
<212>  PRT
<213>  Artificial Sequence

<220>

<223>  CH1A1A LCDR2

<400>  127

Ser Ala Ser Tyr Arg Lys Arg
1               5


<210>  128
<211>  21
<212>  DNA
<213>  Artificial Sequence

<220>
<223>  CH1A1A LCDR2

<400>  128
tcggcatcct accgcaaaag g                                                 21


<210>  129
<211>  10
<212>  PRT
<213>  Artificial Sequence

<220>
<223>  CH1A1A LCDR3

<400>  129

His Gln Tyr Tyr Thr Tyr Pro Leu Phe Thr
1               5                   10


<210>  130
<211>  30
<212>  DNA
<213>  Artificial Sequence

<220>
<223>  CH1A1A LCDR3

<400>  130
caccaatatt acacctatcc tctattcacg                                        30


<210>  131
<211>  108
<212>  PRT
<213>  Artificial Sequence

<220>
<223>  CH1A1A VL

<400>  131

Asp Ile Gln Met Thr Gln Ser Pro Ser Ser Leu Ser Ala Ser Val Gly
1               5                   10                  15


Asp Arg Val Thr Ile Thr Cys Lys Ala Ser Ala Ala Val Gly Thr Tyr
            20                  25                  30

Val Ala Trp Tyr Gln Gln Lys Pro Gly Lys Ala Pro Lys Leu Leu Ile
        35                  40                  45

Tyr Ser Ala Ser Tyr Arg Lys Arg Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys His Gln Tyr Tyr Thr Tyr Pro Leu
                85                  90                  95

Phe Thr Phe Gly Gln Gly Thr Lys Leu Glu Ile Lys
            100                 105

<210>  132
<211>  324
<212>  DNA
<213>  Artificial Sequence

<220>
<223>  CH1A1A VL

<400>  132
gatatccaga tgacccagtc tccatcctcc ctgtctgcat ctgtgggaga cagagtcacc      60

atcacttgca aggccagtgc ggctgtgggt acgtatgttg cgtggtatca gcagaaacca     120

gggaaagcac ctaagctcct gatctattcg gcatcctacc gcaaaagggg agtcccatca     180

aggttcagtg gcagtggatc tgggacagat ttcactctca ccatcagcag tctgcaacct     240

gaagatttcg caacttacta ctgtcaccaa tattacacct atcctctatt cacgtttggc     300

cagggcacca agctcgagat caag                                            324

<210>  133
<211>  10
<212>  PRT
<213>  Artificial Sequence

<220>
<223>  Anti-CD33 HCDR1

<400>  133

Gly Tyr Thr Ile Thr Asp Ser Asn Ile His
1               5                   10

<210>  134
<211>  30
<212>  DNA
<213>  Artificial Sequence

<220>

<223>  Anti-CD33 HCDR1

<400>  134
ggctacacca tcaccgacag caacatccac                                        30


<210>  135
<211>  13
<212>  PRT
<213>  Artificial Sequence

<220>
<223>  Anti-CD33 HCDR2

<400>  135

Tyr Ile Tyr Pro Tyr Asn Gly Gly Thr Asp Tyr Asn Gln
1               5                   10


<210>  136
<211>  39
<212>  DNA
<213>  Artificial Sequence

<220>
<223>  Anti-CD33 HCDR2

<400>  136
tacatctacc cctacaacgg cggcaccgac tacaaccag                              39


<210>  137
<211>  7
<212>  PRT
<213>  Artificial Sequence

<220>
<223>  Anti-CD33 HCDR3

<400>  137

Gly Asn Pro Trp Leu Ala Tyr
1               5


<210>  138
<211>  21
<212>  DNA
<213>  Artificial Sequence

<220>
<223>  Anti-CD33 HCDR3

<400>  138
ggcaacccct ggctggccta t                                                 21


<210>  139
<211>  116
<212>  PRT
<213>  Artificial Sequence

<220>
<223>  Anti-CD33 VH

<400>  139


<210>  140
<211>  348
<212>  DNA
<213>  Artificial Sequence

<220>
<223>  Anti-CD33 VH

<400>  140
gaagtgcagc tggtgcagtc tggcgccgaa gtgaagaaac ccggcagcag cgtgaaggtg      60

tcctgcaagg ccagcggcta caccatcacc gacagcaaca tccactgggt ccgacaggcc     120

cctgggcaga gcctggaatg gatcggctac atctacccct acaacggcgg caccgactac     180

aaccagaagt tcaagaaccg ggccaccctg accgtggaca accccaccaa caccgcctac     240

atggaactga gcagcctgcg gagcgaggac accgccttct actactgcgt gaacggcaac     300

ccctggctgg cctattgggg ccagggaacc ctggtcaccg tgtctagc                  348


<210>  141
<211>  15
<212>  PRT
<213>  Artificial Sequence

<220>
<223>  Anti-CD33 LCDR1

<400>  141

Arg Ala Ser Glu Ser Leu Asp Asn Tyr Gly Ile Arg Phe Leu Thr
1               5                   10                  15


<210>  142
<211>  45
<212>  DNA
<213>  Artificial Sequence

<220>
<223>  Anti-CD33 LCDR1

<400>  142
cgggccagcg agagcctgga caactacggc atccggtttc tgacc                        45


<210>  143
<211>  7
<212>  PRT
<213>  Artificial Sequence

<220>
<223>  Anti-CD33 LCDR2

<400>  143

Ala Ala Ser Asn Gln Gly Ser
1               5


<210>  144
<211>  21
<212>  DNA
<213>  Artificial Sequence

<220>
<223>  Anti-CD33 LCDR2

<400>  144
gccgccagca accagggcag c                                                  21


<210>  145
<211>  9
<212>  PRT
<213>  Artificial Sequence

<220>
<223>  Anti-CD33 LCDR3

<400>  145

Gln Gln Thr Lys Glu Val Pro Trp Ser
1               5


<210>  146

<211>  27
<212>  DNA
<213>  Artificial Sequence

<220>
<223>  Anti-CD33 LCDR3

<400>  146
cagcagacca aagaggtgcc ctggtcc                                          27


<210>  147
<211>  111
<212>  PRT
<213>  Artificial Sequence

<220>
<223>  Anti-CD33 VL

<400>  147


<210>  148
<211>  333
<212>  DNA
<213>  Artificial Sequence

<220>
<223>  Anti-CD33 VL

<400>  148
gacatccagc tgacccagag ccccagcacc ctgtctgcca gcgtgggcga cagagtgacc      60

atcacctgtc gggccagcga gagcctggac aactacggca tccggtttct gacctggttc     120

cagcagaagc ccggcaaggc ccccaagctg ctgatgtacg ccgccagcaa ccagggcagc     180

ggcgtgccaa gcagattcag cggcagcggc tccggcaccg agttcaccct gaccatcagc 240

agcctgcagc ccgacgactt cgccacctac tactgccagc agaccaaaga ggtgccctgg 300

tccttcggcc agggcaccaa ggtggaagtg aag 333

<210> 149
<211> 227
<212> PRT
<213> Homo sapiens

<400> 149

Asp Lys Thr His Thr Cys Pro Pro Cys Pro Ala Pro Glu Leu Leu Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly Lys
225

<210>  150
<211>  15
<212>  PRT
<213>  Artificial Sequence

<220>
<223>  Linker

<400>  150

Glu Pro Lys Ser Cys Gly Gly Gly Gly Ser Gly Gly Gly Gly Ser
1               5                   10                  15

<210>  151
<211>  16
<212>  PRT
<213>  Artificial Sequence

<220>
<223>  Linker

<400>  151

Glu Pro Lys Ser Cys Asp Gly Gly Gly Gly Ser Gly Gly Gly Gly Ser
1               5                   10                  15

<210>  152
<211>  32
<212>  PRT
<213>  Artificial Sequence

<220>
<223>  Linker

<400>  152

Gly Gly Gly Gly Ser Gly Gly Gly Gly Ser Gly Gly Gly Gly Ser Gly
1               5                   10                  15

Gly Gly Gly Ser Gly Gly Gly Gly Ser Gly Gly Gly Gly Ser Gly Gly
            20                  25                  30

<210>  153
<211>  34
<212>  PRT
<213>  Artificial Sequence

<220>
<223>  Linker

<400>  153

Ser Gly Gly Gly Ser Gly Gly Gly Ser Glu Gly Gly Gly Ser Glu Gly
1               5                   10                  15

Gly Gly Ser Glu Gly Gly Gly Ser Glu Gly Gly Gly Ser Gly Gly Gly
            20                  25                  30

Ser Gly

<210>  154
<211>  19
<212>  PRT
<213>  Artificial Sequence

<220>
<223>  Leader 1

<400>  154

Met Asp Trp Thr Trp Arg Ile Leu Phe Leu Val Ala Ala Ala Thr Gly
1               5                   10                  15

Ala His Ser

<210>  155
<211>  57
<212>  DNA
<213>  Artificial Sequence

<220>
<223>  Leader 1

<400>  155
atggactgga cctggagaat cctcttcttg gtggcagcag ccacaggagc ccactcc          57

<210>  156
<211>  57
<212>  DNA
<213>  Artificial Sequence

<220>
<223>  Leader 1

<400>  156
atggactgga cctggaggat cctcttcttg gtggcagcag ccacaggagc ccactcc          57

<210>  157
<211>  22
<212>  PRT
<213>  Artificial Sequence

<220>
<223>  Leader 2

<400>  157

Met Asp Met Arg Val Pro Ala Gln Leu Leu Gly Leu Leu Leu Leu Trp
1               5                   10                  15

Phe Pro Gly Ala Arg Cys
            20

<210>  158
<211>  66
<212>  DNA
<213>  Artificial Sequence

<220>
<223>  Leader 2

<400>  158
atggacatga gggtccccgc tcagctcctg ggcctcctgc tgctctggtt cccaggtgcc      60

aggtgt                                                                  66

<210>  159
<211>  19
<212>  PRT
<213>  Artificial Sequence

<220>
<223>  Leader 3

<400>  159

Met Gly Trp Ser Cys Ile Ile Leu Phe Leu Val Ala Thr Ala Thr Gly
1               5                   10                  15

Val His Ser

<210>  160
<211>  57
<212>  DNA
<213>  Artificial Sequence

<220>
<223>  Leader 3

<400>  160
atgggatgga gctgtatcat cctcttcttg gtagcaacag ctaccggtgt gcattcc         57

<210>  161
<211>  57
<212>  DNA
<213>  Artificial Sequence

<220>
<223>  Leader 3

<400>  161
atgggctggt cctgcatcat cctgtttctg gtggctaccg ccactggagt gcattcc          57


<210>  162
<211>  57
<212>  DNA
<213>  Artificial Sequence

<220>
<223>  Leader 3

<400>  162
atgggctggt cctgcatcat cctgtttctg gtcgccacag ccaccggcgt gcactct          57


<210>  163
<211>  5
<212>  PRT
<213>  Artificial Sequence

<220>
<223>  V9 HCDR1

<400>  163

Gly Tyr Thr Met Asn
1               5


<210>  164
<211>  15
<212>  DNA
<213>  Artificial Sequence

<220>
<223>  V9 HCDR1

<400>  164
ggctacacca tgaac                                                        15


<210>  165
<211>  17
<212>  PRT
<213>  Artificial Sequence

<220>
<223>  V9 HCDR2

<400>  165

Leu Ile Asn Pro Tyr Lys Gly Val Ser Thr Tyr Asn Gln Lys Phe Lys
1               5                   10                  15


Asp

<210>  166
<211>  51
<212>  DNA
<213>  Artificial Sequence

<220>
<223>  V9 HCDR2

<400>  166
ctgatcaacc cctacaaggg cgtgagcacc tacaaccaga agttcaagga c                51


<210>  167
<211>  13
<212>  PRT
<213>  Artificial Sequence

<220>
<223>  V9 HCDR3

<400>  167

Ser Gly Tyr Tyr Gly Asp Ser Asp Trp Tyr Phe Asp Val
1               5                   10


<210>  168
<211>  39
<212>  DNA
<213>  Artificial Sequence

<220>
<223>  V9 HCDR3

<400>  168
agcggctact acggcgacag cgactggtac ttcgacgtg                              39


<210>  169
<211>  122
<212>  PRT
<213>  Artificial Sequence

<220>
<223>  V9 VH

<400>  169

<210>  170
<211>  366
<212>  DNA
<213>  Artificial Sequence

<220>
<223>  V9 VH

<400>  170
gaggtgcagc tggtcgagtc cggcggaggc ctggtgcagc ctggcggcag cctgagactg      60

agctgcgccg ccagcggcta cagcttcacc ggctacacca tgaactgggt ccggcaggct     120

cctggcaagg gcctcgaatg ggtggccctg atcaacccct acaagggcgt gagcacctac     180

aaccagaagt tcaaggaccg gttcaccatc agcgtggaca agagcaagaa caccgcctat     240

ctgcagatga acagcctgcg ggccgaggac accgccgtgt actactgcgc cagaagcggc     300

tactacggcg acagcgactg gtacttcgac gtgtggggcc agggcacact ggtcaccgtg     360

tccagc                                                                366

<210>  171
<211>  11
<212>  PRT
<213>  Artificial Sequence

<220>
<223>  V9 LCDR1

<400>  171

Arg Ala Ser Gln Asp Ile Arg Asn Tyr Leu Asn
1               5                   10

<210>  172
<211>  33
<212>  DNA
<213>  Artificial Sequence

<220>
<223>  V9 LCDR1

<400>  172
cgggccagcc aggacatcag aaactacctg aac                                    33


<210>  173
<211>  7
<212>  PRT
<213>  Artificial Sequence

<220>
<223>  V9 LCDR2

<400>  173

Tyr Thr Ser Arg Leu Glu Ser
1               5


<210>  174
<211>  21
<212>  DNA
<213>  Artificial Sequence

<220>
<223>  V9 LCDR2

<400>  174
tacacctcta gactggaaag c                                                 21


<210>  175
<211>  9
<212>  PRT
<213>  Artificial Sequence

<220>
<223>  V9 LCDR3

<400>  175

Gln Gln Gly Asn Thr Leu Pro Trp Thr
1               5


<210>  176
<211>  27
<212>  DNA
<213>  Artificial Sequence

<220>
<223>  V9 LCDR3

<400>  176
cagcagggca acacactccc ctggacc                                           27


<210>  177
<211>  107
<212>  PRT
<213>  Artificial Sequence

<220>
<223>  V9 VL

<400>  177

<210>  178
<211>  321
<212>  DNA
<213>  Artificial Sequence

<220>
<223>  V9 VL

<400>  178
gacatccaga tgacccagag cccctctagc ctgagcgcca gcgtgggcga cagagtgacc      60

atcacctgtc gggccagcca ggacatcaga aactacctga actggtatca gcagaagccc     120

ggcaaggccc ccaagctgct gatctactac acctctagac tggaaagcgg cgtgcccagc     180

cggtttagcg gcagcggctc cggcaccgac tacaccctga ccatcagcag cctgcagccc     240

gaggacttcg ccacctacta ctgccagcag ggcaacacac tcccctggac cttcggccag     300

ggcaccaagg tggagatcaa g                                               321

<210>  179
<211>  454
<212>  PRT
<213>  Artificial Sequence

<220>
<223>  V9(VH-CL)-LC007(VL-CL)

<400>  179

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Ser Phe Thr Gly Tyr
            20                  25                  30

Thr Met Asn Trp Val Arg Gln Ala Pro Gly Lys Gly Leu Glu Trp Val
        35                  40                  45

Ala Leu Ile Asn Pro Tyr Lys Gly Val Ser Thr Tyr Asn Gln Lys Phe
    50                  55                  60

Lys Asp Arg Phe Thr Ile Ser Val Asp Lys Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg Ser Gly Tyr Tyr Gly Asp Ser Asp Trp Tyr Phe Asp Val Trp
            100                 105                 110

Gly Gln Gly Thr Leu Val Thr Val Ser Ser Ala Ser Val Ala Ala Pro
        115                 120                 125

Ser Val Phe Ile Phe Pro Pro Ser Asp Glu Gln Leu Lys Ser Gly Thr
    130                 135                 140

Ala Ser Val Val Cys Leu Leu Asn Asn Phe Tyr Pro Arg Glu Ala Lys
145                 150                 155                 160

Val Gln Trp Lys Val Asp Asn Ala Leu Gln Ser Gly Asn Ser Gln Glu
                165                 170                 175

Ser Val Thr Glu Gln Asp Ser Lys Asp Ser Thr Tyr Ser Leu Ser Ser
            180                 185                 190

Thr Leu Thr Leu Ser Lys Ala Asp Tyr Glu Lys His Lys Val Tyr Ala
        195                 200                 205

Cys Glu Val Thr His Gln Gly Leu Ser Ser Pro Val Thr Lys Ser Phe
    210                 215                 220

Asn Arg Gly Glu Cys Asp Gly Gly Gly Gly Ser Gly Gly Gly Gly Ser
225                 230                 235                 240

Asp Ile Val Leu Thr Gln Ser Pro Ser Ser Leu Ser Ala Ser Leu Gly
                245                 250                 255

Asp Arg Val Thr Ile Ser Cys Ser Ala Ser Gln Gly Ile Arg Asn Tyr
            260                 265                 270

Leu Asn Trp Tyr Gln Gln Arg Pro Asp Gly Thr Val Lys Leu Leu Ile
        275                 280                 285

Tyr Tyr Thr Ser Ser Leu His Ser Gly Val Pro Ser Arg Phe Ser Gly
    290                 295                 300

Ser Gly Ser Gly Thr Asp Tyr Ser Leu Thr Ile Ser Asn Leu Glu Pro
305                 310                 315                 320

Glu Asp Ile Ala Thr Tyr Tyr Cys Gln Gln Tyr Ser Lys Leu Pro Trp
                325                 330                 335

Thr Phe Gly Gly Gly Thr Lys Leu Glu Ile Lys Arg Thr Val Ala Ala
            340                 345                 350

Pro Ser Val Phe Ile Phe Pro Pro Ser Asp Glu Gln Leu Lys Ser Gly
        355                 360                 365

Thr Ala Ser Val Val Cys Leu Leu Asn Asn Phe Tyr Pro Arg Glu Ala
    370                 375                 380

Lys Val Gln Trp Lys Val Asp Asn Ala Leu Gln Ser Gly Asn Ser Gln
385                 390                 395                 400

Glu Ser Val Thr Glu Gln Asp Ser Lys Asp Ser Thr Tyr Ser Leu Ser
                405                 410                 415

Ser Thr Leu Thr Leu Ser Lys Ala Asp Tyr Glu Lys His Lys Val Tyr
            420                 425                 430

Ala Cys Glu Val Thr His Gln Gly Leu Ser Ser Pro Val Thr Lys Ser
        435                 440                 445

Phe Asn Arg Gly Glu Cys
    450

<210>  180
<211>  1362
<212>  DNA
<213>  Artificial Sequence

<220>
<223>  V9(VH-CL)-LC007(VL-CL)

<400>  180
gaggtgcagc tggtggaatc tggcggcgga ctggtgcagc ctggcggatc tctgagactg        60

agctgtgccg ccagcggcta cagcttcacc ggctacacca tgaactgggt gcgccaggcc 120

cctggcaagg gactggaatg ggtggccctg atcaacccct acaagggcgt gtccacctac 180

aaccagaagt tcaaggaccg gttcaccatc agcgtggaca agagcaagaa caccgcctac 240

ctgcagatga acagcctgcg ggccgaggac accgccgtgt actattgtgc cagaagcggc 300

tactacggcg acagcgactg gtacttcgac gtgtggggcc agggcacact cgtgaccgtg 360

tcaagcgcta gcgtggccgc tcccagcgtg ttcatcttcc cacctagcga cgagcagctg 420

aagtccggca cagcctctgt cgtgtgcctg ctgaacaact tctacccccg cgaggccaag 480

gtgcagtgga aggtggacaa tgccctgcag agcggcaaca gccaggaaag cgtgaccgag 540

caggacagca aggatagcac ctacagcctg agcagcaccc tgaccctgag caaggccgac 600

tacgagaagc acaaggtgta cgcctgcgaa gtgacccacc agggcctgtc tagccccgtg 660

accaagagct tcaaccgggg cgagtgtgat ggcggaggcg gatccggggg aggcggctct 720

gatattgtgc tgacccagag ccccagcagc ctgtctgcct ctctgggcga cagagtgacc 780

atcagctgta gcgcctctca gggcatccgg aactacctga actggtatca gcagcggccc 840

gacggcaccg tgaagctgct gatctactac accagctccc tgcactccgg cgtgcccagc 900

agattttctg gcagcggctc cggcaccgac tactccctga ccatctccaa cctggaaccc 960

gaggatatcg ccacctacta ctgccagcag tactccaagc tgccctggac ctttggaggc 1020

ggcaccaagc tggaaatcaa gcgtacggtg gctgccccct ccgtgtttat ctttccccca 1080

tccgatgaac agctgaaaag cggcaccgcc agcgtcgtgt gtctgctgaa caatttttac 1140

cctagggaag ctaaagtgca gtggaaagtg gataacgcac tgcagtccgg caactcccag 1200

gaatctgtga cagaacagga ctctaaggac agcacatact ccctgtcctc caccctgaca 1260

ctgtctaagg ctgattatga gaaacacaaa gtgtatgctt gtgaagtgac acatcaggga 1320

ctgagcagcc ctgtgacaaa gtccttcaac agaggcgagt gc 1362

<210> 181
<211> 227
<212> PRT
<213> Artificial Sequence

<220>
<223> Fc(knob) P329G LALA

<400> 181

Asp Lys Thr His Thr Cys Pro Pro Cys Pro Ala Pro Glu Ala Ala Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Gly Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Cys Arg Asp Glu Leu Thr Lys Asn Gln Val Ser
    130                 135                 140

Leu Trp Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly Lys
225

<210>  182
<211>  681
<212>  DNA
<213>  Artificial Sequence

<220>
<223>  Fc(knob) P329G LALA

<400>  182
gacaaaactc acacatgccc accgtgccca gcacctgaag ctgcaggggg accgtcagtc        60

ttcctcttcc ccccaaaacc caaggacacc ctcatgatct cccggacccc tgaggtcaca 120

tgcgtggtgg tggacgtgag ccacgaagac cctgaggtca agttcaactg gtacgtggac 180

ggcgtggagg tgcataatgc caagacaaag ccgcgggagg agcagtacaa cagcacgtac 240

cgtgtggtca gcgtcctcac cgtcctgcac caggactggc tgaatggcaa ggagtacaag 300

tgcaaggtct ccaacaaagc cctcggcgcc cccatcgaga aaaccatctc caaagccaaa 360

gggcagcccc gagaaccaca ggtgtacacc ctgcccccat gccgggatga gctgaccaag 420

aaccaggtca gcctgtggtg cctggtcaaa ggcttctatc ccagcgacat cgccgtggag 480

tgggagagca atgggcagcc ggagaacaac tacaagacca cgcctcccgt gctggactcc 540

gacggctcct tcttcctcta cagcaagctc accgtggaca agagcaggtg gcagcagggg 600

aacgtcttct catgctccgt gatgcatgag gctctgcaca accactacac gcagaagagc 660

ctctccctgt ctccgggtaa a 681

<210> 183
<211> 212
<212> PRT
<213> Artificial Sequence

<220>
<223> V9(VL-CH1)

<400> 183

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Arg Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Arg Leu Glu Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Gly Asn Thr Leu Pro Trp
                85                  90                  95

Thr Phe Gly Gln Gly Thr Lys Val Glu Ile Lys Ser Ser Ala Ser Thr
            100                 105                 110

Lys Gly Pro Ser Val Phe Pro Leu Ala Pro Ser Ser Lys Ser Thr Ser

115                 120                 125

Gly Gly Thr Ala Ala Leu Gly Cys Leu Val Lys Asp Tyr Phe Pro Glu
    130                 135                 140

Pro Val Thr Val Ser Trp Asn Ser Gly Ala Leu Thr Ser Gly Val His
145                 150                 155                 160

Thr Phe Pro Ala Val Leu Gln Ser Ser Gly Leu Tyr Ser Leu Ser Ser
                165                 170                 175

Val Val Thr Val Pro Ser Ser Ser Leu Gly Thr Gln Thr Tyr Ile Cys
            180                 185                 190

Asn Val Asn His Lys Pro Ser Asn Thr Lys Val Asp Lys Lys Val Glu
        195                 200                 205

Pro Lys Ser Cys
    210

<210>  184
<211>  636
<212>  DNA
<213>  Artificial Sequence

<220>
<223>  V9(VL-CH1)

<400>  184
gatattcaga tgacccagag ccccagctct ctgagcgcca gcgtgggcga cagagtgacc      60

atcacctgtc gggccagcca ggacatcaga aactacctga actggtatca gcagaagccc     120

ggcaaggccc ccaagctgct gatctactac accagcagac tggaaagcgg cgtgccctcc     180

agattttccg gcagcggctc cggcaccgac tacaccctga ccatcagcag cctgcagccc     240

gaggatttcg ccacatatta ctgccagcag ggcaataccc tgccctggac cttcggacag     300

ggcacaaaag tggaaatcaa gagcagcgct tccaccaaag gcccttccgt gtttcctctg     360

gctcctagct ccaagtccac ctctggaggc accgctgctc tcggatgcct cgtgaaggat     420

tattttcctg agcctgtgac agtgtcctgg aatagcggag cactgacctc tggagtgcat     480

actttccccg ctgtgctgca gtcctctgga ctgtacagcc tgagcagcgt ggtgacagtg     540

cccagcagca gcctgggcac ccagacctac atctgcaacg tgaaccacaa gcccagcaac     600

accaaggtgg acaagaaggt ggaacccaag tcttgt                               636

<210>  185
<211>  456
<212>  PRT
<213>  Artificial Sequence

<220>
<223>  V9(VH-CL)-Fc(knob) P329G LALA

<400>  185

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Ser Phe Thr Gly Tyr
            20                  25                  30

Thr Met Asn Trp Val Arg Gln Ala Pro Gly Lys Gly Leu Glu Trp Val
        35                  40                  45

Ala Leu Ile Asn Pro Tyr Lys Gly Val Ser Thr Tyr Asn Gln Lys Phe
    50                  55                  60

Lys Asp Arg Phe Thr Ile Ser Val Asp Lys Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg Ser Gly Tyr Tyr Gly Asp Ser Asp Trp Tyr Phe Asp Val Trp
            100                 105                 110

Gly Gln Gly Thr Leu Val Thr Val Ser Ser Ala Ser Val Ala Ala Pro
        115                 120                 125

Ser Val Phe Ile Phe Pro Pro Ser Asp Glu Gln Leu Lys Ser Gly Thr
    130                 135                 140

Ala Ser Val Val Cys Leu Leu Asn Asn Phe Tyr Pro Arg Glu Ala Lys
145                 150                 155                 160

Val Gln Trp Lys Val Asp Asn Ala Leu Gln Ser Gly Asn Ser Gln Glu
                165                 170                 175

Ser Val Thr Glu Gln Asp Ser Lys Asp Ser Thr Tyr Ser Leu Ser Ser
            180                 185                 190

Thr Leu Thr Leu Ser Lys Ala Asp Tyr Glu Lys His Lys Val Tyr Ala
        195                 200                 205

Cys Glu Val Thr His Gln Gly Leu Ser Ser Pro Val Thr Lys Ser Phe
    210                 215                 220

Asn Arg Gly Glu Cys Asp Lys Thr His Thr Cys Pro Pro Cys Pro Ala

225                 230                 235                 240

Pro Glu Ala Ala Gly Gly Pro Ser Val Phe Leu Phe Pro Pro Lys Pro
                245                 250                 255

Lys Asp Thr Leu Met Ile Ser Arg Thr Pro Glu Val Thr Cys Val Val
            260                 265                 270

Val Asp Val Ser His Glu Asp Pro Glu Val Lys Phe Asn Trp Tyr Val
        275                 280                 285

Asp Gly Val Glu Val His Asn Ala Lys Thr Lys Pro Arg Glu Glu Gln
    290                 295                 300

Tyr Asn Ser Thr Tyr Arg Val Val Ser Val Leu Thr Val Leu His Gln
305                 310                 315                 320

Asp Trp Leu Asn Gly Lys Glu Tyr Lys Cys Lys Val Ser Asn Lys Ala
                325                 330                 335

Leu Gly Ala Pro Ile Glu Lys Thr Ile Ser Lys Ala Lys Gly Gln Pro
            340                 345                 350

Arg Glu Pro Gln Val Tyr Thr Leu Pro Pro Cys Arg Asp Glu Leu Thr
        355                 360                 365

Lys Asn Gln Val Ser Leu Trp Cys Leu Val Lys Gly Phe Tyr Pro Ser
    370                 375                 380

Asp Ile Ala Val Glu Trp Glu Ser Asn Gly Gln Pro Glu Asn Asn Tyr
385                 390                 395                 400

Lys Thr Thr Pro Pro Val Leu Asp Ser Asp Gly Ser Phe Phe Leu Tyr
                405                 410                 415

Ser Lys Leu Thr Val Asp Lys Ser Arg Trp Gln Gln Gly Asn Val Phe
            420                 425                 430

Ser Cys Ser Val Met His Glu Ala Leu His Asn His Tyr Thr Gln Lys
        435                 440                 445

Ser Leu Ser Leu Ser Pro Gly Lys
    450                 455

<210>  186
<211>  1368
<212>  DNA
<213>  Artificial Sequence

<220>
<223>  V9(VH-CL)-Fc(knob) P329G LALA

<400>  186
gaggtgcagc tggtcgagag cggaggcggc ctggtgcagc ctggcggcag cctgagactg      60

agctgcgccg ccagcggcta cagcttcacc ggctacacca tgaactgggt ccggcaggca     120

cctggcaagg gactggaatg ggtggccctg atcaacccct acaagggcgt gagcacctac     180

aaccagaagt tcaaggaccg gttcaccatc agcgtggaca agagcaagaa caccgcctat     240

ctgcagatga acagcctgcg ggccgaggac accgccgtgt actactgcgc cagaagcggc     300

tactacggcg acagcgactg gtacttcgac gtgtggggcc agggcaccct cgtgaccgtg     360

tctagcgcta gcgtggccgc tccctccgtg tttatctttc ccccatccga tgaacagctg     420

aaaagcggca ccgcctccgt cgtgtgtctg ctgaacaatt tttaccctag ggaagctaaa     480

gtgcagtgga aagtggataa cgcactgcag tccggcaact cccaggaatc tgtgacagaa     540

caggactcca aggacagcac ctactccctg tcctccaccc tgacactgtc taaggctgat     600

tatgagaaac acaaagtcta cgcctgcgaa gtcacccatc agggcctgag ctcgcccgtc     660

acaaagagct tcaacagggg agagtgtgac aagacccaca cctgtccccc ttgtcctgcc     720

cctgaagctg ctggcggccc ttctgtgttc ctgttccccc caaagcccaa ggacaccctg     780

atgatcagcc ggacccccga agtgacctgc gtggtggtgg atgtgtccca cgaggaccct     840

gaagtgaagt tcaattggta cgtggacggc gtggaagtgc acaacgccaa gacaaagccg     900

cgggaggagc agtacaacag cacgtaccgt gtggtcagcg tcctcaccgt cctgcaccag     960

gactggctga atggcaagga gtacaagtgc aaggtctcca acaaagccct cggcgccccc    1020

atcgagaaaa ccatctccaa agccaaaggg cagccccgag aaccacaggt gtacaccctg    1080

cccccatgcc gggatgagct gaccaagaac caggtcagcc tgtggtgcct ggtcaaaggc    1140

ttctatccca gcgacatcgc cgtggagtgg gagagcaatg ggcagccgga gaacaactac    1200

aagaccacgc ctcccgtgct ggactccgac ggctccttct tcctctacag caagctcacc    1260

gtggacaaga gcaggtggca gcaggggaac gtcttctcat gctccgtgat gcatgaggct    1320

ctgcacaacc actacacgca gaagagcctc tccctgtctc cgggtaaa                 1368

<210>  187
<211>  682
<212>  PRT
<213>  Artificial Sequence

<220>
<223>  LC007(VH-CH1)-V9(VH-CL)-Fc(knob) P329G LALA

<400>  187

Glu Val Gln Leu Gln Glu Ser Gly Pro Gly Leu Val Lys Pro Ser Gln

1                   5                   10                  15

Ser Leu Ser Leu Thr Cys Ser Val Thr Gly Tyr Ser Ile Thr Ser Gly
            20                  25                  30

Tyr Tyr Trp Asn Trp Ile Arg Gln Phe Pro Gly Asn Lys Leu Glu Trp
        35                  40                  45

Met Gly Tyr Ile Thr Tyr Asp Gly Ser Asn Asn Tyr Asn Pro Ser Leu
    50                  55                  60

Lys Asn Arg Ile Ser Ile Thr Arg Asp Thr Ser Lys Asn Gln Phe Phe
65                  70                  75                  80

Leu Lys Leu Asn Ser Val Thr Thr Glu Asp Thr Ala Thr Tyr Tyr Cys
                85                  90                  95

Ala Asp Phe Asp Tyr Trp Gly Gln Gly Thr Thr Leu Thr Val Ser Ser
            100                 105                 110

Ala Ser Thr Lys Gly Pro Ser Val Phe Pro Leu Ala Pro Ser Ser Lys
        115                 120                 125

Ser Thr Ser Gly Gly Thr Ala Ala Leu Gly Cys Leu Val Lys Asp Tyr
    130                 135                 140

Phe Pro Glu Pro Val Thr Val Ser Trp Asn Ser Gly Ala Leu Thr Ser
145                 150                 155                 160

Gly Val His Thr Phe Pro Ala Val Leu Gln Ser Ser Gly Leu Tyr Ser
                165                 170                 175

Leu Ser Ser Val Val Thr Val Pro Ser Ser Ser Leu Gly Thr Gln Thr
            180                 185                 190

Tyr Ile Cys Asn Val Asn His Lys Pro Ser Asn Thr Lys Val Asp Lys
        195                 200                 205

Lys Val Glu Pro Lys Ser Cys Asp Gly Gly Gly Gly Ser Gly Gly Gly
    210                 215                 220

Gly Ser Glu Val Gln Leu Val Glu Ser Gly Gly Gly Leu Val Gln Pro
225                 230                 235                 240

Gly Gly Ser Leu Arg Leu Ser Cys Ala Ala Ser Gly Tyr Ser Phe Thr
                245                 250                 255

Gly Tyr Thr Met Asn Trp Val Arg Gln Ala Pro Gly Lys Gly Leu Glu
            260                 265                 270

Trp Val Ala Leu Ile Asn Pro Tyr Lys Gly Val Ser Thr Tyr Asn Gln
        275                 280                 285

Lys Phe Lys Asp Arg Phe Thr Ile Ser Val Asp Lys Ser Lys Asn Thr
    290                 295                 300

Ala Tyr Leu Gln Met Asn Ser Leu Arg Ala Glu Asp Thr Ala Val Tyr
305                 310                 315                 320

Tyr Cys Ala Arg Ser Gly Tyr Tyr Gly Asp Ser Asp Trp Tyr Phe Asp
                325                 330                 335

Val Trp Gly Gln Gly Thr Leu Val Thr Val Ser Ser Ala Ser Val Ala
            340                 345                 350

Ala Pro Ser Val Phe Ile Phe Pro Pro Ser Asp Glu Gln Leu Lys Ser
        355                 360                 365

Gly Thr Ala Ser Val Val Cys Leu Leu Asn Asn Phe Tyr Pro Arg Glu
    370                 375                 380

Ala Lys Val Gln Trp Lys Val Asp Asn Ala Leu Gln Ser Gly Asn Ser
385                 390                 395                 400

Gln Glu Ser Val Thr Glu Gln Asp Ser Lys Asp Ser Thr Tyr Ser Leu
                405                 410                 415

Ser Ser Thr Leu Thr Leu Ser Lys Ala Asp Tyr Glu Lys His Lys Val
            420                 425                 430

Tyr Ala Cys Glu Val Thr His Gln Gly Leu Ser Ser Pro Val Thr Lys
        435                 440                 445

Ser Phe Asn Arg Gly Glu Cys Asp Lys Thr His Thr Cys Pro Pro Cys
    450                 455                 460

Pro Ala Pro Glu Ala Ala Gly Gly Pro Ser Val Phe Leu Phe Pro Pro
465                 470                 475                 480

Lys Pro Lys Asp Thr Leu Met Ile Ser Arg Thr Pro Glu Val Thr Cys
                485                 490                 495

Val Val Val Asp Val Ser His Glu Asp Pro Glu Val Lys Phe Asn Trp
            500                 505                 510

Tyr Val Asp Gly Val Glu Val His Asn Ala Lys Thr Lys Pro Arg Glu
        515                 520                 525

Glu Gln Tyr Asn Ser Thr Tyr Arg Val Val Ser Val Leu Thr Val Leu
    530                 535                 540

His Gln Asp Trp Leu Asn Gly Lys Glu Tyr Lys Cys Lys Val Ser Asn
545                 550                 555                 560

Lys Ala Leu Gly Ala Pro Ile Glu Lys Thr Ile Ser Lys Ala Lys Gly
                565                 570                 575

Gln Pro Arg Glu Pro Gln Val Tyr Thr Leu Pro Pro Cys Arg Asp Glu
            580                 585                 590

Leu Thr Lys Asn Gln Val Ser Leu Trp Cys Leu Val Lys Gly Phe Tyr
        595                 600                 605

Pro Ser Asp Ile Ala Val Glu Trp Glu Ser Asn Gly Gln Pro Glu Asn
    610                 615                 620

Asn Tyr Lys Thr Thr Pro Pro Val Leu Asp Ser Asp Gly Ser Phe Phe
625                 630                 635                 640

Leu Tyr Ser Lys Leu Thr Val Asp Lys Ser Arg Trp Gln Gln Gly Asn
                645                 650                 655

Val Phe Ser Cys Ser Val Met His Glu Ala Leu His Asn His Tyr Thr
            660                 665                 670

Gln Lys Ser Leu Ser Leu Ser Pro Gly Lys
        675                 680

<210>  188
<211>  2046
<212>  DNA
<213>  Artificial Sequence

<220>
<223>  LC007(VH-CH1)- V9(VH-CL)-Fc(knob) P329G LALA

<400>  188
gaggtgcagc tgcaggaatc tggccctggc ctggtcaagc caagccagag tctgagcctg      60

acctgcagcg tgaccggcta cagcattacc agcggctact actggaactg gattcggcag     120

ttccccggca ataagctgga atggatgggc tacatcacct acgacggcag caacaactac     180

aaccccagcc tgaagaaccg gatcagcatc acccgggaca ccagcaagaa ccagttcttc     240

ctgaagctga acagcgtgac caccgaggac accgccacat actattgcgc cgacttcgac     300

tactggggcc agggcaccac cctgaccgtg tccagcgcca gcacaaaggg ccctagcgtg 360

ttccctctgg cccccagcag caagagcaca agcggcggaa cagccgccct gggctgcctc 420

gtgaaggact acttccccga gcccgtgaca gtgtcttgga acagcggagc cctgacaagc 480

ggcgtgcaca ccttccctgc cgtgctgcag agcagcggcc tgtactccct gagcagcgtg 540

gtcaccgtgc ctagcagcag cctgggcacc cagacctaca tctgcaacgt gaaccacaag 600

cccagcaaca ccaaagtgga caagaaggtg gagcccaaga gctgtgatgg cggaggaggg 660

tccggaggcg gaggatccga agtgcagctg gtggaatctg gcggaggcct ggtgcagcct 720

ggcggatctc tgagactgag ctgtgccgcc agcggctaca gcttcaccgg ctacaccatg 780

aactgggtgc gccaggcccc tggcaaggga ctggaatggg tggccctgat caacccctac 840

aagggcgtgt ccacatacaa ccagaagttc aaggaccggt tcaccatcag cgtggacaag 900

agcaagaaca ccgcctacct gcagatgaac agcctgcggg ccgaggacac cgccgtgtac 960

tattgtgcca gaagcggcta ctacggcgac agcgactggt acttcgacgt gtggggccag 1020

ggcacactcg tgaccgtgtc aagcgctagc gtggccgctc cctccgtgtt tatctttccc 1080

ccatccgatg aacagctgaa aagcggcacc gcctccgtcg tgtgtctgct gaacaatttt 1140

taccctaggg aagctaaagt gcagtggaaa gtggataacg cactgcagtc cggcaactcc 1200

caggaatctg tgacagaaca ggactccaag gacagcacct actccctgtc ctccaccctg 1260

acactgtcta aggctgatta tgagaaacac aaagtctacg cctgcgaagt cacccatcag 1320

ggcctgagct cgcccgtcac aaagagcttc aacaggggag agtgtgacaa gacccacacc 1380

tgtccccctt gtcctgcccc tgaagctgct ggcggccctt ctgtgttcct gttcccccca 1440

aagcccaagg acaccctgat gatcagccgg acccccgaag tgacctgcgt ggtggtggat 1500

gtgtcccacg aggaccctga agtgaagttc aattggtacg tggacggcgt ggaagtgcac 1560

aacgccaaga caaagccgcg ggaggagcag tacaacagca cgtaccgtgt ggtcagcgtc 1620

ctcaccgtcc tgcaccagga ctggctgaat ggcaaggagt acaagtgcaa ggtctccaac 1680

aaagccctcg gcgcccccat cgagaaaacc atctccaaag ccaaagggca gccccgagaa 1740

ccacaggtgt acaccctgcc cccatgccgg gatgagctga ccaagaacca ggtcagcctg 1800

tggtgcctgg tcaaaggctt ctatcccagc gacatcgccg tggagtggga gagcaatggg 1860

cagccggaga acaactacaa gaccacgcct cccgtgctgg actccgacgg ctccttcttc 1920

ctctacagca agctcaccgt ggacaagagc aggtggcagc aggggaacgt cttctcatgc 1980

tccgtgatgc atgaggctct gcacaaccac tacacgcaga agagcctctc cctgtctccg 2040

ggtaaa 2046

<210> 189

<211>  214
<212>  PRT
<213>  Artificial Sequence

<220>
<223>  M4-3 ML2(VL-CL)

<400>  189

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Arg Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Ser Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Lys Leu Pro Trp
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  190
<211>  642
<212>  DNA
<213>  Artificial Sequence

<220>
<223>  M4-3 ML2(VL-CL)

<400>  190
gacatccaga tgacccagag ccccagcagc ctgagcgcca gcgtgggcga cagagtgacc      60

atcacctgcc gggccagcca gggcatccgg aactacctga actggtatca gcagaagccc     120

ggcaaggccc ccaagctgct gatctactac accagcagcc tgcacagcgg cgtgcctagc     180

cggtttagcg gcagcggctc cggcaccgac ttcaccctga ccattagctc cctgcagccc     240

gaggacttcg ccacctacta ctgccagcag tacagcaagc tgccctggac cttcggccag     300

ggaacaaagg tggagatcaa gcgtacggtg gctgcaccat ctgtcttcat cttcccgcca     360

tctgatgagc agttgaaatc tggaactgcc tctgttgtgt gcctgctgaa taacttctat     420

cccagagagg ccaaagtaca gtggaaggtg gataacgccc tccaatcggg taactcccag     480

gagagtgtca cagagcagga cagcaaggac agcacctaca gcctcagcag caccctgacg     540

ctgagcaaag cagactacga gaaacacaaa gtctacgcct gcgaagtcac ccatcagggc     600

ctgagctcgc ccgtcacaaa gagcttcaac aggggagagt gt                        642


<210>  191
<211>  664
<212>  PRT
<213>  Artificial Sequence

<220>
<223>  V9(VL-CH1)-M4-3 ML2(VH-CH1)-Fc(knob) P329G LALA

<400>  191

Asp Ile Gln Met Thr Gln Ser Pro Ser Ser Leu Ser Ala Ser Val Gly
1               5                   10                  15


Asp Arg Val Thr Ile Thr Cys Arg Ala Ser Gln Asp Ile Arg Asn Tyr
            20                  25                  30


Leu Asn Trp Tyr Gln Gln Lys Pro Gly Lys Ala Pro Lys Leu Leu Ile
        35                  40                  45


Tyr Tyr Thr Ser Arg Leu Glu Ser Gly Val Pro Ser Arg Phe Ser Gly
    50                  55                  60


Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Gly Asn Thr Leu Pro Trp
                85                  90                  95

Thr Phe Gly Gln Gly Thr Lys Val Glu Ile Lys Ser Ser Ala Ser Thr
            100                 105                 110

Lys Gly Pro Ser Val Phe Pro Leu Ala Pro Ser Ser Lys Ser Thr Ser
        115                 120                 125

Gly Gly Thr Ala Ala Leu Gly Cys Leu Val Lys Asp Tyr Phe Pro Glu
    130                 135                 140

Pro Val Thr Val Ser Trp Asn Ser Gly Ala Leu Thr Ser Gly Val His
145                 150                 155                 160

Thr Phe Pro Ala Val Leu Gln Ser Ser Gly Leu Tyr Ser Leu Ser Ser
                165                 170                 175

Val Val Thr Val Pro Ser Ser Ser Leu Gly Thr Gln Thr Tyr Ile Cys
            180                 185                 190

Asn Val Asn His Lys Pro Ser Asn Thr Lys Val Asp Lys Lys Val Glu
        195                 200                 205

Pro Lys Ser Cys Gly Gly Gly Gly Ser Gly Gly Gly Gly Ser Gln Val
    210                 215                 220

Gln Leu Gln Glu Ser Gly Pro Gly Leu Val Lys Pro Ser Gln Thr Leu
225                 230                 235                 240

Ser Leu Thr Cys Thr Val Ser Gly Gly Ser Ile Thr Ser Gly Tyr Tyr
                245                 250                 255

Trp Asn Trp Ile Arg Gln His Pro Gly Lys Gly Leu Glu Trp Ile Gly
            260                 265                 270

Tyr Ile Thr Tyr Asp Gly Ser Asn Asn Tyr Asn Pro Ser Leu Lys Ser
        275                 280                 285

Arg Val Thr Ile Ser Arg Asp Thr Ser Lys Asn Gln Phe Ser Leu Lys
    290                 295                 300

Leu Ser Ser Val Thr Ala Ala Asp Thr Ala Val Tyr Tyr Cys Ala Asp
305                 310                 315                 320

Phe Asp Tyr Trp Gly Gln Gly Thr Leu Val Thr Val Ser Ser Ala Ser
                325                 330                 335

Thr Lys Gly Pro Ser Val Phe Pro Leu Ala Pro Ser Ser Lys Ser Thr
        340                 345                 350

Ser Gly Gly Thr Ala Ala Leu Gly Cys Leu Val Lys Asp Tyr Phe Pro
        355                 360                 365

Glu Pro Val Thr Val Ser Trp Asn Ser Gly Ala Leu Thr Ser Gly Val
    370                 375                 380

His Thr Phe Pro Ala Val Leu Gln Ser Ser Gly Leu Tyr Ser Leu Ser
385                 390                 395                 400

Ser Val Val Thr Val Pro Ser Ser Ser Leu Gly Thr Gln Thr Tyr Ile
                405                 410                 415

Cys Asn Val Asn His Lys Pro Ser Asn Thr Lys Val Asp Lys Lys Val
            420                 425                 430

Glu Pro Lys Ser Cys Asp Lys Thr His Thr Cys Pro Pro Cys Pro Ala
        435                 440                 445

Pro Glu Ala Ala Gly Gly Pro Ser Val Phe Leu Phe Pro Pro Lys Pro
    450                 455                 460

Lys Asp Thr Leu Met Ile Ser Arg Thr Pro Glu Val Thr Cys Val Val
465                 470                 475                 480

Val Asp Val Ser His Glu Asp Pro Glu Val Lys Phe Asn Trp Tyr Val
                485                 490                 495

Asp Gly Val Glu Val His Asn Ala Lys Thr Lys Pro Arg Glu Glu Gln
            500                 505                 510

Tyr Asn Ser Thr Tyr Arg Val Val Ser Val Leu Thr Val Leu His Gln
        515                 520                 525

Asp Trp Leu Asn Gly Lys Glu Tyr Lys Cys Lys Val Ser Asn Lys Ala
    530                 535                 540

Leu Gly Ala Pro Ile Glu Lys Thr Ile Ser Lys Ala Lys Gly Gln Pro
545                 550                 555                 560

Arg Glu Pro Gln Val Tyr Thr Leu Pro Pro Cys Arg Asp Glu Leu Thr
                565                 570                 575

Lys Asn Gln Val Ser Leu Trp Cys Leu Val Lys Gly Phe Tyr Pro Ser

580                 585                 590

Asp Ile Ala Val Glu Trp Glu Ser Asn Gly Gln Pro Glu Asn Asn Tyr
        595                 600                 605

Lys Thr Thr Pro Pro Val Leu Asp Ser Asp Gly Ser Phe Phe Leu Tyr
    610                 615                 620

Ser Lys Leu Thr Val Asp Lys Ser Arg Trp Gln Gln Gly Asn Val Phe
625                 630                 635                 640

Ser Cys Ser Val Met His Glu Ala Leu His Asn His Tyr Thr Gln Lys
                645                 650                 655

Ser Leu Ser Leu Ser Pro Gly Lys
            660

<210>  192
<211>  1992
<212>  DNA
<213>  Artificial Sequence

<220>
<223>  V9(VL-CH1)-M4-3 ML2(VH-CH1)-Fc(knob) P329G LALA (DNA)

<400>  192
gatatccaga tgacccagag ccccagctct ctgagcgcca gcgtgggcga cagagtgacc      60

atcacctgtc gggccagcca ggacatcaga aactacctga actggtatca gcagaagccc     120

ggcaaggccc ccaagctgct gatctactac accagcagac tggaaagcgg cgtgccctcc     180

agattttccg gcagcggctc cggcaccgac tacaccctga ccatcagcag cctgcagccc     240

gaggatttcg ccacatatta ctgccagcag ggcaataccc tgccctggac cttcggacag     300

ggcacaaaag tggaaatcaa gagcagcgct tccaccaaag gcccttccgt gtttcctctg     360

gctcctagct ccaagtccac ctctggaggc accgctgctc tcggatgcct cgtgaaggat     420

tattttcctg agcctgtgac agtgtcctgg aatagcggag cactgacctc tggagtgcat     480

actttccccg ctgtgctgca gtcctctgga ctgtacagcc tgagcagcgt ggtgacagtg     540

cccagcagca gcctgggcac ccagacctac atctgcaacg tgaaccacaa gcccagcaac     600

accaaggtgg acaagaaggt ggaacccaag tcttgtggcg gaggcggatc cggcggaggg     660

ggatctcagg tgcagctgca ggaaagcggc cctggcctgg tcaagcccag ccagaccctg     720

agcctgacct gcaccgtgtc cggcggcagc atcaccagcg gctactactg gaactggatt     780

cggcagcacc ccggcaaggg cctggaatgg atcggctaca tcacctacga cggcagcaac     840

aactacaacc ccagcctgaa gtccagagtg accatcagcc gggacaccag caagaaccag     900

ttcagcctga agctgtccag cgtgacagcc gccgacaccg ccgtgtacta ctgcgccgac     960

ttcgactact ggggccaggg caccctggtc accgtgtcca gcgctagcac caagggcccc 1020

agcgtgttcc ccctggcacc cagcagcaag agcacatctg gcggaacagc cgctctgggc 1080

tgtctggtga aagactactt ccccgagccc gtgaccgtgt cttggaactc tggcgccctg 1140

accagcggcg tgcacacctt tccagccgtg ctgcagagca gcggcctgta ctccctgtcc 1200

tccgtggtca ccgtgccctc tagctccctg ggaacacaga catatatctg taatgtcaat 1260

cacaagcctt ccaacaccaa agtcgataag aaagtcgagc ccaagagctg cgacaaaact 1320

cacacatgcc caccgtgccc agcacctgaa gctgcagggg gaccgtcagt cttcctcttc 1380

cccccaaaac ccaaggacac cctcatgatc tcccggaccc ctgaggtcac atgcgtggtg 1440

gtggacgtga gccacgaaga ccctgaggtc aagttcaact ggtacgtgga cggcgtggag 1500

gtgcataatg ccaagacaaa gccgcgggag gagcagtaca acagcacgta ccgtgtggtc 1560

agcgtcctca ccgtcctgca ccaggactgg ctgaatggca aggagtacaa gtgcaaggtc 1620

tccaacaaag ccctcggcgc ccccatcgag aaaaccatct ccaaagccaa agggcagccc 1680

cgagaaccac aggtgtacac cctgccccca tgccgggatg agctgaccaa gaaccaggtc 1740

agcctgtggt gcctggtcaa aggcttctat cccagcgaca tcgccgtgga gtgggagagc 1800

aatgggcagc cggagaacaa ctacaagacc acgcctcccg tgctggactc cgacggctcc 1860

ttcttcctct acagcaagct caccgtggac aagagcaggt ggcagcaggg gaacgtcttc 1920

tcatgctccg tgatgcatga ggctctgcac aaccactaca cgcagaagag cctctccctg 1980

tctccgggta aa 1992

<210> 193
<211> 442
<212> PRT
<213> Artificial Sequence

<220>
<223> M4-3 ML2(VH-CH1)-Fc(hole) P329G LALA

<400> 193

Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Thr Ser Gly
            20                  25                  30

Tyr Tyr Trp Asn Trp Ile Arg Gln His Pro Gly Lys Gly Leu Glu Trp
        35                  40                  45

Ile Gly Tyr Ile Thr Tyr Asp Gly Ser Asn Asn Tyr Asn Pro Ser Leu
    50                  55                  60

Lys Ser Arg Val Thr Ile Ser Arg Asp Thr Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Asp Phe Asp Tyr Trp Gly Gln Gly Thr Leu Val Thr Val Ser Ser
            100                 105                 110

Ala Ser Thr Lys Gly Pro Ser Val Phe Pro Leu Ala Pro Ser Ser Lys
        115                 120                 125

Ser Thr Ser Gly Gly Thr Ala Ala Leu Gly Cys Leu Val Lys Asp Tyr
    130                 135                 140

Phe Pro Glu Pro Val Thr Val Ser Trp Asn Ser Gly Ala Leu Thr Ser
145                 150                 155                 160

Gly Val His Thr Phe Pro Ala Val Leu Gln Ser Ser Gly Leu Tyr Ser
                165                 170                 175

Leu Ser Ser Val Val Thr Val Pro Ser Ser Ser Leu Gly Thr Gln Thr
            180                 185                 190

Tyr Ile Cys Asn Val Asn His Lys Pro Ser Asn Thr Lys Val Asp Lys
        195                 200                 205

Lys Val Glu Pro Lys Ser Cys Asp Lys Thr His Thr Cys Pro Pro Cys
    210                 215                 220

Pro Ala Pro Glu Ala Ala Gly Gly Pro Ser Val Phe Leu Phe Pro Pro
225                 230                 235                 240

Lys Pro Lys Asp Thr Leu Met Ile Ser Arg Thr Pro Glu Val Thr Cys
                245                 250                 255

Val Val Val Asp Val Ser His Glu Asp Pro Glu Val Lys Phe Asn Trp
            260                 265                 270

Tyr Val Asp Gly Val Glu Val His Asn Ala Lys Thr Lys Pro Arg Glu
        275                 280                 285

Glu Gln Tyr Asn Ser Thr Tyr Arg Val Val Ser Val Leu Thr Val Leu
    290                 295                 300

His Gln Asp Trp Leu Asn Gly Lys Glu Tyr Lys Cys Lys Val Ser Asn
305                 310                 315                 320

Lys Ala Leu Gly Ala Pro Ile Glu Lys Thr Ile Ser Lys Ala Lys Gly
                325                 330                 335

Gln Pro Arg Glu Pro Gln Val Cys Thr Leu Pro Pro Ser Arg Asp Glu
            340                 345                 350

Leu Thr Lys Asn Gln Val Ser Leu Ser Cys Ala Val Lys Gly Phe Tyr
        355                 360                 365

Pro Ser Asp Ile Ala Val Glu Trp Glu Ser Asn Gly Gln Pro Glu Asn
    370                 375                 380

Asn Tyr Lys Thr Thr Pro Pro Val Leu Asp Ser Asp Gly Ser Phe Phe
385                 390                 395                 400

Leu Val Ser Lys Leu Thr Val Asp Lys Ser Arg Trp Gln Gln Gly Asn
                405                 410                 415

Val Phe Ser Cys Ser Val Met His Glu Ala Leu His Asn His Tyr Thr
            420                 425                 430

Gln Lys Ser Leu Ser Leu Ser Pro Gly Lys
        435                 440

<210> 194
<211> 1326
<212> DNA
<213> Artificial Sequence

<220>
<223> M4-3 ML2(VH-CH1)-Fc(hole) P329G LALA

<400> 194
caggtgcagc tgcaggaaag cggccctggc ctggtcaagc ccagccagac cctgagcctg 60

acctgcaccg tgtccggcgg cagcatcacc agcggctact actggaactg gatccggcag 120

caccccggca agggcctgga atggatcggc tacatcacct acgacggcag caacaactac 180

aaccccagcc tgaagtccag agtgaccatc agccgggaca ccagcaagaa ccagttcagc 240

ctgaagctgt ccagcgtgac agccgccgac accgccgtgt actactgcgc cgacttcgac 300

tactggggcc agggcaccct ggtcaccgtg tccagcgcta gcaccaaggg cccctccgtg 360

ttccccctgg cccccagcag caagagcacc agcggcggca cagccgctct gggctgcctg 420

gtcaaggact acttccccga gcccgtgacc gtgtcctgga acagcggagc cctgacctcc 480

ggcgtgcaca ccttccccgc cgtgctgcag agttctggcc tgtatagcct gagcagcgtg 540

gtcaccgtgc cttctagcag cctgggcacc cagacctaca tctgcaacgt gaaccacaag 600

cccagcaaca ccaaggtgga caagaaggtg gagcccaaga gctgcgacaa aactcacaca 660

tgcccaccgt gcccagcacc tgaagctgca gggggaccgt cagtcttcct cttcccccca 720

aaacccaagg acaccctcat gatctcccgg acccctgagg tcacatgcgt ggtggtggac 780

gtgagccacg aagaccctga ggtcaagttc aactggtacg tggacggcgt ggaggtgcat 840

aatgccaaga caaagccgcg ggaggagcag tacaacagca cgtaccgtgt ggtcagcgtc 900

ctcaccgtcc tgcaccagga ctggctgaat ggcaaggagt acaagtgcaa ggtctccaac 960

aaagccctcg gcgcccccat cgagaaaacc atctccaaag ccaaagggca gccccgagaa 1020

ccacaggtgt gcaccctgcc cccatcccgg gatgagctga ccaagaacca ggtcagcctc 1080

tcgtgcgcag tcaaaggctt ctatcccagc gacatcgccg tggagtggga gagcaatggg 1140

cagccggaga acaactacaa gaccacgcct cccgtgctgg actccgacgg ctccttcttc 1200

ctcgtgagca agctcaccgt ggacaagagc aggtggcagc aggggaacgt cttctcatgc 1260

tccgtgatgc atgaggctct gcacaaccac tacacgcaga agagcctctc cctgtctccg 1320

ggtaaa 1326

<210> 195
<211> 681
<212> PRT
<213> Artificial Sequence

<220>
<223> V9(VH-CL)-M4-3 ML2(VH-CH1)-Fc(knob) P329G LALA

<400> 195

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Ser Phe Thr Gly Tyr
            20                  25                  30

Thr Met Asn Trp Val Arg Gln Ala Pro Gly Lys Gly Leu Glu Trp Val
        35                  40                  45

Ala Leu Ile Asn Pro Tyr Lys Gly Val Ser Thr Tyr Asn Gln Lys Phe
    50                  55                  60

Lys Asp Arg Phe Thr Ile Ser Val Asp Lys Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg Ser Gly Tyr Tyr Gly Asp Ser Asp Trp Tyr Phe Asp Val Trp
            100                 105                 110

Gly Gln Gly Thr Leu Val Thr Val Ser Ser Ala Ser Val Ala Ala Pro
        115                 120                 125

Ser Val Phe Ile Phe Pro Pro Ser Asp Glu Gln Leu Lys Ser Gly Thr
    130                 135                 140

Ala Ser Val Val Cys Leu Leu Asn Asn Phe Tyr Pro Arg Glu Ala Lys
145                 150                 155                 160

Val Gln Trp Lys Val Asp Asn Ala Leu Gln Ser Gly Asn Ser Gln Glu
                165                 170                 175

Ser Val Thr Glu Gln Asp Ser Lys Asp Ser Thr Tyr Ser Leu Ser Ser
            180                 185                 190

Thr Leu Thr Leu Ser Lys Ala Asp Tyr Glu Lys His Lys Val Tyr Ala
        195                 200                 205

Cys Glu Val Thr His Gln Gly Leu Ser Ser Pro Val Thr Lys Ser Phe
    210                 215                 220

Asn Arg Gly Glu Cys Gly Gly Gly Gly Ser Gly Gly Gly Gly Ser Gln
225                 230                 235                 240

Val Gln Leu Gln Glu Ser Gly Pro Gly Leu Val Lys Pro Ser Gln Thr
                245                 250                 255

Leu Ser Leu Thr Cys Thr Val Ser Gly Gly Ser Ile Thr Ser Gly Tyr
            260                 265                 270

Tyr Trp Asn Trp Ile Arg Gln His Pro Gly Lys Gly Leu Glu Trp Ile
        275                 280                 285

Gly Tyr Ile Thr Tyr Asp Gly Ser Asn Asn Tyr Asn Pro Ser Leu Lys
    290                 295                 300

Ser Arg Val Thr Ile Ser Arg Asp Thr Ser Lys Asn Gln Phe Ser Leu
305                 310                 315                 320

Lys Leu Ser Ser Val Thr Ala Ala Asp Thr Ala Val Tyr Tyr Cys Ala
                325                 330                 335

Asp Phe Asp Tyr Trp Gly Gln Gly Thr Leu Val Thr Val Ser Ser Ala
            340                 345                 350

Ser Thr Lys Gly Pro Ser Val Phe Pro Leu Ala Pro Ser Ser Lys Ser

355                 360                 365

Thr Ser Gly Gly Thr Ala Ala Leu Gly Cys Leu Val Lys Asp Tyr Phe
    370                 375                 380

Pro Glu Pro Val Thr Val Ser Trp Asn Ser Gly Ala Leu Thr Ser Gly
385                 390                 395                 400

Val His Thr Phe Pro Ala Val Leu Gln Ser Ser Gly Leu Tyr Ser Leu
                405                 410                 415

Ser Ser Val Val Thr Val Pro Ser Ser Ser Leu Gly Thr Gln Thr Tyr
            420                 425                 430

Ile Cys Asn Val Asn His Lys Pro Ser Asn Thr Lys Val Asp Lys Lys
        435                 440                 445

Val Glu Pro Lys Ser Cys Asp Lys Thr His Thr Cys Pro Pro Cys Pro
    450                 455                 460

Ala Pro Glu Ala Ala Gly Gly Pro Ser Val Phe Leu Phe Pro Pro Lys
465                 470                 475                 480

Pro Lys Asp Thr Leu Met Ile Ser Arg Thr Pro Glu Val Thr Cys Val
                485                 490                 495

Val Val Asp Val Ser His Glu Asp Pro Glu Val Lys Phe Asn Trp Tyr
            500                 505                 510

Val Asp Gly Val Glu Val His Asn Ala Lys Thr Lys Pro Arg Glu Glu
        515                 520                 525

Gln Tyr Asn Ser Thr Tyr Arg Val Val Ser Val Leu Thr Val Leu His
    530                 535                 540

Gln Asp Trp Leu Asn Gly Lys Glu Tyr Lys Cys Lys Val Ser Asn Lys
545                 550                 555                 560

Ala Leu Gly Ala Pro Ile Glu Lys Thr Ile Ser Lys Ala Lys Gly Gln
                565                 570                 575

Pro Arg Glu Pro Gln Val Tyr Thr Leu Pro Pro Cys Arg Asp Glu Leu
            580                 585                 590

Thr Lys Asn Gln Val Ser Leu Trp Cys Leu Val Lys Gly Phe Tyr Pro
        595                 600                 605

Ser Asp Ile Ala Val Glu Trp Glu Ser Asn Gly Gln Pro Glu Asn Asn
    610                 615                 620

Tyr Lys Thr Thr Pro Pro Val Leu Asp Ser Asp Gly Ser Phe Phe Leu
625                 630                 635                 640

Tyr Ser Lys Leu Thr Val Asp Lys Ser Arg Trp Gln Gln Gly Asn Val
                645                 650                 655

Phe Ser Cys Ser Val Met His Glu Ala Leu His Asn His Tyr Thr Gln
            660                 665                 670

Lys Ser Leu Ser Leu Ser Pro Gly Lys
        675                 680

<210>  196
<211>  2043
<212>  DNA
<213>  Artificial Sequence

<220>
<223>  V9(VH-CL)-M4-3 ML2(VH-CH1)-Fc(knob) P329G LALA (DNA)

<400>  196
gaggtgcagc tggtcgagag cggaggcggc ctggtgcagc ctggcggcag cctgagactg      60

agctgcgccg ccagcggcta cagcttcacc ggctacacca tgaactgggt ccggcaggca     120

cctggcaagg gactggaatg ggtggccctg atcaacccct acaagggcgt gagcacctac     180

aaccagaagt tcaaggaccg gttcaccatc agcgtggaca agagcaagaa caccgcctat     240

ctgcagatga acagcctgcg ggccgaggac accgccgtgt actactgcgc cagaagcggc     300

tactacggcg acagcgactg gtacttcgac gtgtggggcc agggcaccct cgtgaccgtg     360

tctagcgcta gcgtggctgc accatctgtc ttcatcttcc cgccatctga tgagcagttg     420

aaatctggaa ctgcctctgt tgtgtgcctg ctgaataact tctatcccag agaggccaaa     480

gtacagtgga aggtggataa cgccctccaa tcgggtaact cccaggagag tgtcacagag     540

caggacagca aggacagcac ctacagcctc agcagcaccc tgacgctgag caaagcagac     600

tacgagaaac acaaagtcta cgcctgcgaa gtcacccatc agggcctgag ctcgcccgtc     660

acaaagagct tcaacagggg agagtgtggc ggaggcggat ccggcggagg gggatctcag     720

gtgcagctgc aggaaagcgg ccctggcctg gtcaagccca gccagaccct gagcctgacc     780

tgcaccgtgt ccggcggcag catcaccagc ggctactact ggaactggat tcggcagcac     840

cccggcaagg gcctggaatg gatcggctac atcacctacg acggcagcaa caactacaac     900

cccagcctga agtccagagt gaccatcagc cgggacacca gcaagaacca gttcagcctg     960

aagctgtcca gcgtgacagc cgccgacacc gccgtgtact actgcgccga cttcgactac    1020

tggggccagg gcaccctggt caccgtgtcc agcgctagca ccaagggccc cagcgtgttc 1080

cccctggcac ccagcagcaa gagcacatct ggcggaacag ccgctctggg ctgtctggtg 1140

aaagactact tccccgagcc cgtgaccgtg tcttggaact ctggcgccct gaccagcggc 1200

gtgcacacct ttccagccgt gctgcagagc agcggcctgt actccctgtc ctccgtggtc 1260

accgtgccct ctagctccct gggaacacag acatatatct gtaatgtcaa tcacaagcct 1320

tccaacacca aagtcgataa gaaagtcgag cccaagagct gcgacaaaac tcacacatgc 1380

ccaccgtgcc cagcacctga agctgcaggg ggaccgtcag tcttcctctt ccccccaaaa 1440

cccaaggaca ccctcatgat ctcccggacc cctgaggtca catgcgtggt ggtggacgtg 1500

agccacgaag accctgaggt caagttcaac tggtacgtgg acggcgtgga ggtgcataat 1560

gccaagacaa agccgcggga ggagcagtac aacagcacgt accgtgtggt cagcgtcctc 1620

accgtcctgc accaggactg gctgaatggc aaggagtaca agtgcaaggt ctccaacaaa 1680

gccctcggcg cccccatcga gaaaaccatc tccaaagcca aagggcagcc ccgagaacca 1740

caggtgtaca ccctgccccc atgccgggat gagctgacca agaaccaggt cagcctgtgg 1800

tgcctggtca aaggcttcta tcccagcgac atcgccgtgg agtgggagag caatgggcag 1860

ccggagaaca actacaagac cacgcctccc gtgctggact ccgacggctc cttcttcctc 1920

tacagcaagc tcaccgtgga caagagcagg tggcagcagg ggaacgtctt ctcatgctcc 1980

gtgatgcatg aggctctgca caaccactac acgcagaaga gcctctccct gtctccgggt 2040

aaa 2043

<210> 197
<211> 690
<212> PRT
<213> Artificial Sequence

<220>
<223> CH1A1A(VH-CH1)- V9(VH-CL)-Fc(knob) P329G LALA

<400> 197

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Glu Phe
            20                  25                  30

Gly Met Asn Trp Val Arg Gln Ala Pro Gly Gln Gly Leu Glu Trp Met
        35                  40                  45

Gly Trp Ile Asn Thr Lys Thr Gly Glu Ala Thr Tyr Val Glu Glu Phe
    50                  55                  60

Lys Gly Arg Val Thr Phe Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Trp Asp Phe Ala Tyr Tyr Val Glu Ala Met Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Gly Gly Gly Gly Ser Gly Gly Gly Gly Ser Glu Val Gln Leu Val Glu
225                 230                 235                 240

Ser Gly Gly Gly Leu Val Gln Pro Gly Gly Ser Leu Arg Leu Ser Cys
                245                 250                 255

Ala Ala Ser Gly Tyr Ser Phe Thr Gly Tyr Thr Met Asn Trp Val Arg
            260                 265                 270

Gln Ala Pro Gly Lys Gly Leu Glu Trp Val Ala Leu Ile Asn Pro Tyr
        275                 280                 285

Lys Gly Val Ser Thr Tyr Asn Gln Lys Phe Lys Asp Arg Phe Thr Ile
    290                 295                 300

Ser Val Asp Lys Ser Lys Asn Thr Ala Tyr Leu Gln Met Asn Ser Leu
305                 310                 315                 320

Arg Ala Glu Asp Thr Ala Val Tyr Tyr Cys Ala Arg Ser Gly Tyr Tyr
                325                 330                 335

Gly Asp Ser Asp Trp Tyr Phe Asp Val Trp Gly Gln Gly Thr Leu Val
            340                 345                 350

Thr Val Ser Ser Ala Ser Val Ala Ala Pro Ser Val Phe Ile Phe Pro
        355                 360                 365

Pro Ser Asp Glu Gln Leu Lys Ser Gly Thr Ala Ser Val Val Cys Leu
    370                 375                 380

Leu Asn Asn Phe Tyr Pro Arg Glu Ala Lys Val Gln Trp Lys Val Asp
385                 390                 395                 400

Asn Ala Leu Gln Ser Gly Asn Ser Gln Glu Ser Val Thr Glu Gln Asp
                405                 410                 415

Ser Lys Asp Ser Thr Tyr Ser Leu Ser Ser Thr Leu Thr Leu Ser Lys
            420                 425                 430

Ala Asp Tyr Glu Lys His Lys Val Tyr Ala Cys Glu Val Thr His Gln
        435                 440                 445

Gly Leu Ser Ser Pro Val Thr Lys Ser Phe Asn Arg Gly Glu Cys Asp
    450                 455                 460

Lys Thr His Thr Cys Pro Pro Cys Pro Ala Pro Glu Ala Ala Gly Gly
465                 470                 475                 480

Pro Ser Val Phe Leu Phe Pro Pro Lys Pro Lys Asp Thr Leu Met Ile
                485                 490                 495

Ser Arg Thr Pro Glu Val Thr Cys Val Val Val Asp Val Ser His Glu
            500                 505                 510

Asp Pro Glu Val Lys Phe Asn Trp Tyr Val Asp Gly Val Glu Val His
        515                 520                 525

Asn Ala Lys Thr Lys Pro Arg Glu Glu Gln Tyr Asn Ser Thr Tyr Arg
    530                 535                 540

Val Val Ser Val Leu Thr Val Leu His Gln Asp Trp Leu Asn Gly Lys
545                 550                 555                 560

Glu Tyr Lys Cys Lys Val Ser Asn Lys Ala Leu Gly Ala Pro Ile Glu
                565                 570                 575

Lys Thr Ile Ser Lys Ala Lys Gly Gln Pro Arg Glu Pro Gln Val Tyr
            580                 585                 590

Thr Leu Pro Pro Cys Arg Asp Glu Leu Thr Lys Asn Gln Val Ser Leu
        595                 600                 605

Trp Cys Leu Val Lys Gly Phe Tyr Pro Ser Asp Ile Ala Val Glu Trp
    610                 615                 620

Glu Ser Asn Gly Gln Pro Glu Asn Asn Tyr Lys Thr Thr Pro Pro Val
625                 630                 635                 640

Leu Asp Ser Asp Gly Ser Phe Phe Leu Tyr Ser Lys Leu Thr Val Asp
                645                 650                 655

Lys Ser Arg Trp Gln Gln Gly Asn Val Phe Ser Cys Ser Val Met His
            660                 665                 670

Glu Ala Leu His Asn His Tyr Thr Gln Lys Ser Leu Ser Leu Ser Pro
        675                 680                 685

Gly Lys
    690

<210>  198
<211>  2070
<212>  DNA
<213>  Artificial Sequence

<220>
<223>  CH1A1A(VH-CH1)-V9(VH-CL)-Fc(knob) P329G LALA

<400>  198
caggtgcagc tggtgcagtc tggcgccgaa gtgaagaaac ctggcgccag cgtgaaggtg      60

tcctgcaagg ccagcggcta caccttcacc gagttcggca tgaactgggt ccgacaggcc     120

cctggacagg gcctggaatg gatgggctgg atcaacacca agaccggcga ggccacctac     180

gtggaagagt tcaagggcag agtgaccttc accaccgaca ccagcaccag caccgcctac     240

atggaactgc ggagcctgag aagcgacgac accgccgtgt actactgcgc cagatgggac     300

ttcgcctact atgtggaagc catggactac tggggccagg gcaccaccgt gaccgtgtct     360

agtgctagca caaagggccc cagcgtgttc cctctggccc ctagcagcaa gagcacatct     420

ggcggaacag ccgccctggg ctgcctggtc aaggactact ttcccgagcc cgtgacagtg     480

tcctggaact ctggcgccct gacaagcggc gtgcacacct ttccagccgt gctgcagagc     540

agcggcctgt actctctgag cagcgtggtc accgtgccta gctctagcct gggcacccag     600

acctacatct gcaacgtgaa ccacaagccc agcaacacca aggtggacaa gaaggtggaa 660

cccaagagct gcggcggagg cggatccgga ggcggaggat ccgaagtgca gctggtggaa 720

tctggcggag gcctggtgca gcctggcgga tctctgagac tgagctgtgc cgccagcggc 780

tacagcttca ccggctacac catgaactgg gtgcgccagg cccctggcaa gggactggaa 840

tgggtggccc tgatcaaccc ctacaagggc gtgtccacat acaaccagaa gttcaaggac 900

cggttcacca tcagcgtgga caagagcaag aacaccgcct acctgcagat gaacagcctg 960

cgggccgagg acaccgccgt gtactattgt gccagaagcg gctactacgg cgacagcgac 1020

tggtacttcg acgtgtgggg ccagggcaca ctcgtgaccg tgtcaagcgc tagcgtggcc 1080

gctccctccg tgtttatctt tcccccatcc gatgaacagc tgaaaagcgg caccgcctcc 1140

gtcgtgtgtc tgctgaacaa tttttaccct agggaagcta aagtgcagtg gaaagtggat 1200

aacgcactgc agtccggcaa ctcccaggaa tctgtgacag aacaggactc caaggacagc 1260

acctactccc tgtcctccac cctgacactg tctaaggctg attatgagaa acacaaagtc 1320

tacgcctgcg aagtcaccca tcagggcctg agctcgcccg tcacaaagag cttcaacagg 1380

ggagagtgtg acaagaccca cacctgtccc ccttgtcctg cccctgaagc tgctggcggc 1440

ccttctgtgt tcctgttccc cccaaagccc aaggacaccc tgatgatcag ccggaccccc 1500

gaagtgacct gcgtggtggt ggatgtgtcc cacgaggacc ctgaagtgaa gttcaattgg 1560

tacgtggacg gcgtggaagt gcacaacgcc aagacaaagc cgcgggagga gcagtacaac 1620

agcacgtacc gtgtggtcag cgtcctcacc gtcctgcacc aggactggct gaatggcaag 1680

gagtacaagt gcaaggtctc caacaaagcc ctcggcgccc ccatcgagaa aaccatctcc 1740

aaagccaaag ggcagccccg agaaccacag gtgtacaccc tgcccccatg ccgggatgag 1800

ctgaccaaga accaggtcag cctgtggtgc ctggtcaaag gcttctatcc cagcgacatc 1860

gccgtggagt gggagagcaa tgggcagccg gagaacaact acaagaccac gcctcccgtg 1920

ctggactccg acggctcctt cttcctctac agcaagctca ccgtggacaa gagcaggtgg 1980

cagcagggga acgtcttctc atgctccgtg atgcatgagg ctctgcacaa ccactacacg 2040

cagaagagcc tctccctgtc tccgggtaaa 2070

<210> 199
<211> 214
<212> PRT
<213> Artificial Sequence

<220>
<223> H2C(VL-CH1)

<400> 199

Gln Thr Val Val Thr Gln Glu Pro Ser Leu Thr Val Ser Pro Gly Gly
1               5                   10                  15

Thr Val Thr Leu Thr Cys Gly Ser Ser Thr Gly Ala Val Thr Ser Gly
            20                  25                  30

Tyr Tyr Pro Asn Trp Val Gln Gln Lys Pro Gly Gln Ala Pro Arg Gly
        35                  40                  45

Leu Ile Gly Gly Thr Lys Phe Leu Ala Pro Gly Thr Pro Ala Arg Phe
    50                  55                  60

Ser Gly Ser Leu Leu Gly Gly Lys Ala Ala Leu Thr Leu Ser Gly Val
65                  70                  75                  80

Gln Pro Glu Asp Glu Ala Glu Tyr Tyr Cys Ala Leu Trp Tyr Ser Asn
                85                  90                  95

Arg Trp Val Phe Gly Gly Gly Thr Lys Leu Thr Val Leu Ser Ser Ala
            100                 105                 110

Ser Thr Lys Gly Pro Ser Val Phe Pro Leu Ala Pro Ser Ser Lys Ser
        115                 120                 125

Thr Ser Gly Gly Thr Ala Ala Leu Gly Cys Leu Val Lys Asp Tyr Phe
    130                 135                 140

Pro Glu Pro Val Thr Val Ser Trp Asn Ser Gly Ala Leu Thr Ser Gly
145                 150                 155                 160

Val His Thr Phe Pro Ala Val Leu Gln Ser Ser Gly Leu Tyr Ser Leu
                165                 170                 175

Ser Ser Val Val Thr Val Pro Ser Ser Ser Leu Gly Thr Gln Thr Tyr
            180                 185                 190

Ile Cys Asn Val Asn His Lys Pro Ser Asn Thr Lys Val Asp Lys Lys
        195                 200                 205

Val Glu Pro Lys Ser Cys
    210

<210>  200
<211>  642
<212>  DNA
<213>  Artificial Sequence

<220>
<223>  H2C(VL-CH1)

<400>  200

cagaccgtgg tgacacagga acccagcctg accgtctccc ctggcggcac cgtgaccctg 60

acctgtggaa gcagcacagg cgccgtgacc agcggctact accccaactg ggtgcagcag 120

aagcccggcc aggcccctag aggactgatc ggcggcacca agtttctggc ccctggcacc 180

cccgccagat tctctggctc tctgctgggc ggcaaggccg ccctgacact gtctggcgtg 240

cagcctgagg acgaggccga gtactactgc gccctgtggt acagcaacag atgggtgttc 300

ggcggaggca ccaagctgac cgtgctgagc agcgcttcca ccaaaggccc ttccgtgttt 360

cctctggctc ctagctccaa gtccacctct ggaggcaccg ctgctctcgg atgcctcgtg 420

aaggattatt ttcctgagcc tgtgacagtg tcctggaata gcggagcact gacctctgga 480

gtgcatactt tccccgctgt gctgcagtcc tctggactgt acagcctgag cagcgtggtg 540

acagtgccca gcagcagcct gggcacccag acctacatct gcaacgtgaa ccacaagccc 600

agcaacacca aggtggacaa gaaggtggaa cccaagtctt gt 642

<210> 201
<211> 684
<212> PRT
<213> Artificial Sequence

<220>
<223> H2C(VH-CL)-M4-3 ML2(VH-CH1)-Fc(knob) P329G LALA

<400> 201

Glu Val Gln Leu Val Glu Ser Gly Gly Gly Leu Val Gln Pro Gly Gly
1               5                   10                  15

Ser Leu Lys Leu Ser Cys Ala Ala Ser Gly Phe Thr Phe Asn Lys Tyr
            20                  25                  30

Ala Met Asn Trp Val Arg Gln Ala Pro Gly Lys Gly Leu Glu Trp Val
        35                  40                  45

Ala Arg Ile Arg Ser Lys Tyr Asn Asn Tyr Ala Thr Tyr Tyr Ala Asp
    50                  55                  60

Ser Val Lys Asp Arg Phe Thr Ile Ser Arg Asp Asp Ser Lys Asn Thr
65                  70                  75                  80

Ala Tyr Leu Gln Met Asn Asn Leu Lys Thr Glu Asp Thr Ala Val Tyr
                85                  90                  95

Tyr Cys Val Arg His Gly Asn Phe Gly Asn Ser Tyr Ile Ser Tyr Trp
            100                 105                 110

Ala Tyr Trp Gly Gln Gly Thr Leu Val Thr Val Ser Ser Ala Ser Val
        115                 120                 125

Ala Ala Pro Ser Val Phe Ile Phe Pro Pro Ser Asp Glu Gln Leu Lys
    130                 135                 140

Ser Gly Thr Ala Ser Val Val Cys Leu Leu Asn Asn Phe Tyr Pro Arg
145                 150                 155                 160

Glu Ala Lys Val Gln Trp Lys Val Asp Asn Ala Leu Gln Ser Gly Asn
                165                 170                 175

Ser Gln Glu Ser Val Thr Glu Gln Asp Ser Lys Asp Ser Thr Tyr Ser
            180                 185                 190

Leu Ser Ser Thr Leu Thr Leu Ser Lys Ala Asp Tyr Glu Lys His Lys
        195                 200                 205

Val Tyr Ala Cys Glu Val Thr His Gln Gly Leu Ser Ser Pro Val Thr
    210                 215                 220

Lys Ser Phe Asn Arg Gly Glu Cys Gly Gly Gly Gly Ser Gly Gly Gly
225                 230                 235                 240

Gly Ser Gln Val Gln Leu Gln Glu Ser Gly Pro Gly Leu Val Lys Pro
                245                 250                 255

Ser Gln Thr Leu Ser Leu Thr Cys Thr Val Ser Gly Gly Ser Ile Thr
            260                 265                 270

Ser Gly Tyr Tyr Trp Asn Trp Ile Arg Gln His Pro Gly Lys Gly Leu
        275                 280                 285

Glu Trp Ile Gly Tyr Ile Thr Tyr Asp Gly Ser Asn Asn Tyr Asn Pro
    290                 295                 300

Ser Leu Lys Ser Arg Val Thr Ile Ser Arg Asp Thr Ser Lys Asn Gln
305                 310                 315                 320

Phe Ser Leu Lys Leu Ser Ser Val Thr Ala Ala Asp Thr Ala Val Tyr
                325                 330                 335

Tyr Cys Ala Asp Phe Asp Tyr Trp Gly Gln Gly Thr Leu Val Thr Val
            340                 345                 350

Ser Ser Ala Ser Thr Lys Gly Pro Ser Val Phe Pro Leu Ala Pro Ser
        355                 360                 365

Ser Lys Ser Thr Ser Gly Gly Thr Ala Ala Leu Gly Cys Leu Val Lys

370                 375                 380

Asp Tyr Phe Pro Glu Pro Val Thr Val Ser Trp Asn Ser Gly Ala Leu
385                 390                 395                 400

Thr Ser Gly Val His Thr Phe Pro Ala Val Leu Gln Ser Ser Gly Leu
                405                 410                 415

Tyr Ser Leu Ser Ser Val Val Thr Val Pro Ser Ser Ser Leu Gly Thr
            420                 425                 430

Gln Thr Tyr Ile Cys Asn Val Asn His Lys Pro Ser Asn Thr Lys Val
        435                 440                 445

Asp Lys Lys Val Glu Pro Lys Ser Cys Asp Lys Thr His Thr Cys Pro
    450                 455                 460

Pro Cys Pro Ala Pro Glu Ala Ala Gly Gly Pro Ser Val Phe Leu Phe
465                 470                 475                 480

Pro Pro Lys Pro Lys Asp Thr Leu Met Ile Ser Arg Thr Pro Glu Val
                485                 490                 495

Thr Cys Val Val Val Asp Val Ser His Glu Asp Pro Glu Val Lys Phe
            500                 505                 510

Asn Trp Tyr Val Asp Gly Val Glu Val His Asn Ala Lys Thr Lys Pro
        515                 520                 525

Arg Glu Glu Gln Tyr Asn Ser Thr Tyr Arg Val Val Ser Val Leu Thr
    530                 535                 540

Val Leu His Gln Asp Trp Leu Asn Gly Lys Glu Tyr Lys Cys Lys Val
545                 550                 555                 560

Ser Asn Lys Ala Leu Gly Ala Pro Ile Glu Lys Thr Ile Ser Lys Ala
                565                 570                 575

Lys Gly Gln Pro Arg Glu Pro Gln Val Tyr Thr Leu Pro Pro Cys Arg
            580                 585                 590

Asp Glu Leu Thr Lys Asn Gln Val Ser Leu Trp Cys Leu Val Lys Gly
        595                 600                 605

Phe Tyr Pro Ser Asp Ile Ala Val Glu Trp Glu Ser Asn Gly Gln Pro
    610                 615                 620

Glu Asn Asn Tyr Lys Thr Thr Pro Pro Val Leu Asp Ser Asp Gly Ser
625                 630                 635                 640

Phe Phe Leu Tyr Ser Lys Leu Thr Val Asp Lys Ser Arg Trp Gln Gln
                645                 650                 655

Gly Asn Val Phe Ser Cys Ser Val Met His Glu Ala Leu His Asn His
            660                 665                 670

Tyr Thr Gln Lys Ser Leu Ser Leu Ser Pro Gly Lys
        675                 680

<210>  202
<211>  2052
<212>  DNA
<213>  Artificial Sequence

<220>
<223>  H2C(VH-CL)-M4-3 ML2(VH-CH1)-Fc(knob) P329G LALA

<400>  202
gaggtgcagc tggtggaaag cggcggagga ctggtgcagc ctggcggaag cctgaagctg      60

tcttgcgccg ccagcggctt caccttcaac aaatacgcca tgaactgggt gcgccaggcc     120

cctggcaagg gactggaatg ggtggcccgg atcagaagca agtacaacaa ctacgccacc     180

tactacgccg acagcgtgaa ggaccggttc accatcagcc gggacgacag caagaacacc     240

gcctacctgc agatgaacaa cctgaaaacc gaggacaccg ccgtgtacta ctgcgtgcgg     300

cacggcaact tcggcaacag ctacatcagc tactgggcct actggggaca gggcaccctg     360

gtgacagtgt ccagcgctag cgtggctgca ccatctgtct tcatcttccc gccatctgat     420

gagcagttga aatctggaac tgcctctgtt gtgtgcctgc tgaataactt ctatcccaga     480

gaggccaaag tacagtggaa ggtggataac gccctccaat cgggtaactc ccaggagagt     540

gtcacagagc aggacagcaa ggacagcacc tacagcctca gcagcaccct gacgctgagc     600

aaagcagact acgagaaaca caaagtctac gcctgcgaag tcacccatca gggcctgagc     660

tcgcccgtca caaagagctt caacagggga gagtgtggcg gaggcggatc cggcggaggg     720

ggatctcagg tgcagctgca ggaaagcggc cctggcctgg tcaagcccag ccagaccctg     780

agcctgacct gcaccgtgtc cggcggcagc atcaccagcg gctactactg gaactggatt     840

cggcagcacc ccggcaaggg cctggaatgg atcggctaca tcacctacga cggcagcaac     900

aactacaacc ccagcctgaa gtccagagtg accatcagcc gggacaccag caagaaccag     960

ttcagcctga agctgtccag cgtgacagcc gccgacaccg ccgtgtacta ctgcgccgac    1020

ttcgactact ggggccaggg caccctggtc accgtgtcca gcgctagcac caagggcccc    1080

agcgtgttcc ccctggcacc cagcagcaag agcacatctg gcggaacagc cgctctgggc    1140

tgtctggtga aagactactt ccccgagccc gtgaccgtgt cttggaactc tggcgccctg 1200

accagcggcg tgcacacctt tccagccgtg ctgcagagca gcggcctgta ctccctgtcc 1260

tccgtggtca ccgtgccctc tagctccctg ggaacacaga catatatctg taatgtcaat 1320

cacaagcctt ccaacaccaa agtcgataag aaagtcgagc ccaagagctg cgacaaaact 1380

cacacatgcc caccgtgccc agcacctgaa gctgcagggg gaccgtcagt cttcctcttc 1440

cccccaaaac ccaaggacac cctcatgatc tcccggaccc ctgaggtcac atgcgtggtg 1500

gtggacgtga gccacgaaga ccctgaggtc aagttcaact ggtacgtgga cggcgtggag 1560

gtgcataatg ccaagacaaa gccgcgggag gagcagtaca acagcacgta ccgtgtggtc 1620

agcgtcctca ccgtcctgca ccaggactgg ctgaatggca aggagtacaa gtgcaaggtc 1680

tccaacaaag ccctcggcgc ccccatcgag aaaaccatct ccaaagccaa agggcagccc 1740

cgagaaccac aggtgtacac cctgccccca tgccgggatg agctgaccaa gaaccaggtc 1800

agcctgtggt gcctggtcaa aggcttctat cccagcgaca tcgccgtgga gtgggagagc 1860

aatgggcagc cggagaacaa ctacaagacc acgcctcccg tgctggactc cgacggctcc 1920

ttcttcctct acagcaagct caccgtggac aagagcaggt ggcagcaggg gaacgtcttc 1980

tcatgctccg tgatgcatga ggctctgcac aaccactaca cgcagaagag cctctccctg 2040

tctccgggta aa 2052

<210> 203
<211> 212
<212> PRT
<213> Artificial Sequence

<220>
<223> 431/26(VL-CL)

<400> 203

Asp Ile Gln Met Thr Gln Ser Pro Ser Ser Leu Ser Ala Ser Val Gly
1               5                   10                  15

Asp Arg Val Thr Ile Thr Cys Ser Thr Ser Ser Ser Val Ser Tyr Met
            20                  25                  30

His Trp Tyr Gln Gln Lys Pro Gly Lys Ala Pro Lys Leu Leu Ile Tyr
        35                  40                  45

Ser Thr Ser Asn Leu Ala Ser Gly Val Pro Ser Arg Phe Ser Gly Ser
    50                  55                  60

Gly Ser Gly Thr Asp Phe Thr Phe Thr Ile Ser Ser Leu Gln Pro Glu
65                  70                  75                  80

Asp Ile Ala Thr Tyr Tyr Cys His Gln Trp Ser Ser Tyr Pro Thr Phe
                85                  90                  95

Gly Gln Gly Thr Lys Val Glu Ile Lys Arg Thr Val Ala Ala Pro Ser
            100                 105                 110

Val Phe Ile Phe Pro Pro Ser Asp Glu Gln Leu Lys Ser Gly Thr Ala
        115                 120                 125

Ser Val Val Cys Leu Leu Asn Asn Phe Tyr Pro Arg Glu Ala Lys Val
    130                 135                 140

Gln Trp Lys Val Asp Asn Ala Leu Gln Ser Gly Asn Ser Gln Glu Ser
145                 150                 155                 160

Val Thr Glu Gln Asp Ser Lys Asp Ser Thr Tyr Ser Leu Ser Ser Thr
                165                 170                 175

Leu Thr Leu Ser Lys Ala Asp Tyr Glu Lys His Lys Val Tyr Ala Cys
            180                 185                 190

Glu Val Thr His Gln Gly Leu Ser Ser Pro Val Thr Lys Ser Phe Asn
        195                 200                 205

Arg Gly Glu Cys
    210

<210>  204
<211>  636
<212>  DNA
<213>  Artificial Sequence

<220>
<223>  431/26(VL-CL)

<400>  204
gacatccaga tgacccagag ccccagcagc ctgtctgcca gcgtgggcga cagagtgacc      60

atcacctgta gcaccagcag cagcgtgtcc tacatgcact ggtatcagca gaagcccggc     120

aaggccccca agctgctgat ctacagcacc tccaatctgg ccagcggcgt gcccagcaga     180

ttttctggca gcggctccgg caccgacttc accttcacca tcagctccct gcagcccgag     240

gatatcgcca cctactactg ccaccagtgg tccagctacc ccacctttgg ccagggcacc     300

aaggtggaaa tcaagcgtac ggtggctgca ccatctgtct tcatcttccc gccatctgat     360

gagcagttga aatctggaac tgcctctgtt gtgtgcctgc tgaataactt ctatcccaga     420

gaggccaaag tacagtggaa ggtggataac gccctccaat cgggtaactc ccaggagagt     480

gtcacagagc aggacagcaa ggacagcacc tacagcctca gcagcaccct gacgctgagc     540

aaagcagact acgagaaaca caaagtctac gcctgcgaag tcacccatca gggcctgagc 600

tcgcccgtca caaagagctt caacagggga gagtgt 636

<210> 205
<211> 689
<212> PRT
<213> Artificial Sequence

<220>
<223> 431/26(VH-CH1)-V9(VH-CL)-Fc(knob) P329G LALA

<400> 205

Gln Val Gln Leu Gln Glu Ser Gly Pro Gly Leu Val Arg Pro Ser Gln
1               5                   10                  15

Thr Leu Ser Leu Thr Cys Thr Val Ser Gly Phe Thr Ile Ser Ser Gly
            20                  25                  30

Tyr Ser Trp His Trp Val Arg Gln Pro Pro Gly Arg Gly Leu Glu Trp
        35                  40                  45

Ile Gly Tyr Ile Gln Tyr Ser Gly Ile Thr Asn Tyr Asn Pro Ser Leu
    50                  55                  60

Lys Ser Arg Val Thr Met Leu Val Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Glu Asp Tyr Asp Tyr His Trp Tyr Phe Asp Val Trp Gly Gln
            100                 105                 110

Gly Ser Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Gly
    210                 215                 220

Gly Gly Gly Ser Gly Gly Gly Gly Ser Glu Val Gln Leu Val Glu Ser
225                 230                 235                 240

Gly Gly Gly Leu Val Gln Pro Gly Gly Ser Leu Arg Leu Ser Cys Ala
                245                 250                 255

Ala Ser Gly Tyr Ser Phe Thr Gly Tyr Thr Met Asn Trp Val Arg Gln
            260                 265                 270

Ala Pro Gly Lys Gly Leu Glu Trp Val Ala Leu Ile Asn Pro Tyr Lys
        275                 280                 285

Gly Val Ser Thr Tyr Asn Gln Lys Phe Lys Asp Arg Phe Thr Ile Ser
    290                 295                 300

Val Asp Lys Ser Lys Asn Thr Ala Tyr Leu Gln Met Asn Ser Leu Arg
305                 310                 315                 320

Ala Glu Asp Thr Ala Val Tyr Tyr Cys Ala Arg Ser Gly Tyr Tyr Gly
                325                 330                 335

Asp Ser Asp Trp Tyr Phe Asp Val Trp Gly Gln Gly Thr Leu Val Thr
            340                 345                 350

Val Ser Ser Ala Ser Val Ala Ala Pro Ser Val Phe Ile Phe Pro Pro
        355                 360                 365

Ser Asp Glu Gln Leu Lys Ser Gly Thr Ala Ser Val Val Cys Leu Leu
    370                 375                 380

Asn Asn Phe Tyr Pro Arg Glu Ala Lys Val Gln Trp Lys Val Asp Asn
385                 390                 395                 400

Ala Leu Gln Ser Gly Asn Ser Gln Glu Ser Val Thr Glu Gln Asp Ser
                405                 410                 415

Lys Asp Ser Thr Tyr Ser Leu Ser Ser Thr Leu Thr Leu Ser Lys Ala
            420                 425                 430

Asp Tyr Glu Lys His Lys Val Tyr Ala Cys Glu Val Thr His Gln Gly
        435                 440                 445

Leu Ser Ser Pro Val Thr Lys Ser Phe Asn Arg Gly Glu Cys Asp Lys
    450                 455                 460

Thr His Thr Cys Pro Pro Cys Pro Ala Pro Glu Ala Ala Gly Gly Pro
465                 470                 475                 480

Ser Val Phe Leu Phe Pro Pro Lys Pro Lys Asp Thr Leu Met Ile Ser
                485                 490                 495

Arg Thr Pro Glu Val Thr Cys Val Val Val Asp Val Ser His Glu Asp
            500                 505                 510

Pro Glu Val Lys Phe Asn Trp Tyr Val Asp Gly Val Glu Val His Asn
        515                 520                 525

Ala Lys Thr Lys Pro Arg Glu Glu Gln Tyr Asn Ser Thr Tyr Arg Val
    530                 535                 540

Val Ser Val Leu Thr Val Leu His Gln Asp Trp Leu Asn Gly Lys Glu
545                 550                 555                 560

Tyr Lys Cys Lys Val Ser Asn Lys Ala Leu Gly Ala Pro Ile Glu Lys
                565                 570                 575

Thr Ile Ser Lys Ala Lys Gly Gln Pro Arg Glu Pro Gln Val Tyr Thr
            580                 585                 590

Leu Pro Pro Cys Arg Asp Glu Leu Thr Lys Asn Gln Val Ser Leu Trp
        595                 600                 605

Cys Leu Val Lys Gly Phe Tyr Pro Ser Asp Ile Ala Val Glu Trp Glu
    610                 615                 620

Ser Asn Gly Gln Pro Glu Asn Asn Tyr Lys Thr Thr Pro Pro Val Leu
625                 630                 635                 640

Asp Ser Asp Gly Ser Phe Phe Leu Tyr Ser Lys Leu Thr Val Asp Lys
                645                 650                 655

Ser Arg Trp Gln Gln Gly Asn Val Phe Ser Cys Ser Val Met His Glu
            660                 665                 670

Ala Leu His Asn His Tyr Thr Gln Lys Ser Leu Ser Leu Ser Pro Gly
        675                 680                 685

Lys

<210>  206
<211>  2067
<212>  DNA
<213>  Artificial Sequence

<220>
<223>  431/26(VH-CH1)-V9(VH-CL)-Fc(knob) P329G LALA

<400>  206
caggtgcagc tgcaggaatc tggccctgga ctcgtgcggc ctagccagac actgagcctg      60

acctgtaccg tgtccggctt caccatcagc agcggctaca gctggcattg ggtgcgccag     120

ccacctggca gaggcctgga atggatcggc tacatccagt acagcggcat caccaactac     180

aaccccagcc tgaagtccag agtgaccatg ctggtggaca cctccaagaa ccagttcagc     240

ctgcggctga gcagcgtgac agccgccgat acagccgtgt actactgcgc cagagaggac     300

tacgactacc actggtactt cgacgtgtgg ggccagggct ctctcgtgac cgtgtcaagc     360

gctagcacaa agggccccag cgtgttccct ctggccccta gcagcaagag cacatctggc     420

ggaacagccg ccctgggctg cctggtcaag gactactttc ccgagcccgt gacagtgtcc     480

tggaactctg gcgccctgac aagcggcgtg cacacctttc cagccgtgct gcagagcagc     540

ggcctgtact ctctgagcag cgtggtcacc gtgcctagct ctagcctggg cacccagacc     600

tacatctgca acgtgaacca caagcccagc aacaccaagg tggacaagaa ggtggaaccc     660

aagagctgcg gcggaggcgg atccggaggc ggaggatccg aagtgcagct ggtggaatct     720

ggcggaggcc tggtgcagcc tggcggatct ctgagactga gctgtgccgc cagcggctac     780

agcttcaccg gctacaccat gaactgggtg cgccaggccc ctggcaaggg actggaatgg     840

gtggccctga tcaaccccta caagggcgtg tccacataca accagaagtt caaggaccgg     900

ttcaccatca gcgtggacaa gagcaagaac accgcctacc tgcagatgaa cagcctgcgg     960

gccgaggaca ccgccgtgta ctattgtgcc agaagcggct actacggcga cagcgactgg    1020

tacttcgacg tgtggggcca gggcacactc gtgaccgtgt caagcgctag cgtggccgct    1080

ccctccgtgt ttatctttcc cccatccgat gaacagctga aaagcggcac cgcctccgtc    1140

gtgtgtctgc tgaacaattt ttaccctagg gaagctaaag tgcagtggaa agtggataac    1200

gcactgcagt ccggcaactc ccaggaatct gtgacagaac aggactccaa ggacagcacc    1260

tactccctgt cctccaccct gacactgtct aaggctgatt atgagaaaca caaagtctac    1320

gcctgcgaag tcacccatca gggcctgagc tcgcccgtca caaagagctt caacagggga    1380

gagtgtgaca agacccacac ctgtccccct tgtcctgccc ctgaagctgc tggcggccct    1440

tctgtgttcc tgttcccccc aaagcccaag gacaccctga tgatcagccg gacccccgaa    1500

gtgacctgcg tggtggtgga tgtgtcccac gaggaccctg aagtgaagtt caattggtac    1560

gtggacggcg tggaagtgca caacgccaag acaaagccgc gggaggagca gtacaacagc 1620

acgtaccgtg tggtcagcgt cctcaccgtc ctgcaccagg actggctgaa tggcaaggag 1680

tacaagtgca aggtctccaa caaagccctc ggcgccccca tcgagaaaac catctccaaa 1740

gccaaagggc agccccgaga accacaggtg tacaccctgc ccccatgccg ggatgagctg 1800

accaagaacc aggtcagcct gtggtgcctg gtcaaaggct tctatcccag cgacatcgcc 1860

gtggagtggg agagcaatgg gcagccggag aacaactaca agaccacgcc tcccgtgctg 1920

gactccgacg gctccttctt cctctacagc aagctcaccg tggacaagag caggtggcag 1980

caggggaacg tcttctcatg ctccgtgatg catgaggctc tgcacaacca ctacacgcag 2040

aagagcctct ccctgtctcc gggtaaa 2067

<210> 207
<211> 450
<212> PRT
<213> Artificial Sequence

<220>
<223> 431/26(VH-CH1)-Fc(hole) P329G LALA

<400> 207

Gln Val Gln Leu Gln Glu Ser Gly Pro Gly Leu Val Arg Pro Ser Gln
1               5                   10                  15

Thr Leu Ser Leu Thr Cys Thr Val Ser Gly Phe Thr Ile Ser Ser Gly
            20                  25                  30

Tyr Ser Trp His Trp Val Arg Gln Pro Pro Gly Arg Gly Leu Glu Trp
        35                  40                  45

Ile Gly Tyr Ile Gln Tyr Ser Gly Ile Thr Asn Tyr Asn Pro Ser Leu
    50                  55                  60

Lys Ser Arg Val Thr Met Leu Val Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Glu Asp Tyr Asp Tyr His Trp Tyr Phe Asp Val Trp Gly Gln
            100                 105                 110

Gly Ser Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Ala Ala Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Gly Ala Pro Ile Glu
                325                 330                 335

Lys Thr Ile Ser Lys Ala Lys Gly Gln Pro Arg Glu Pro Gln Val Cys
            340                 345                 350

Thr Leu Pro Pro Ser Arg Asp Glu Leu Thr Lys Asn Gln Val Ser Leu
        355                 360                 365

Ser Cys Ala Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val

385                 390                 395                 400

Leu Asp Ser Asp Gly Ser Phe Phe Leu Val Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210>  208
<211>  1350
<212>  DNA
<213>  Artificial Sequence

<220>
<223>  431/26(VH-CH1)-Fc(hole) P329G LALA

<400>  208
caggtgcagc tgcaggaatc tggccctgga ctcgtgcggc ctagccagac actgagcctg      60

acctgtaccg tgtccggctt caccatcagc agcggctaca gctggcattg ggtgcgccag     120

ccacctggca gaggcctgga atggatcggc tacatccagt acagcggcat caccaactac     180

aaccccagcc tgaagtccag agtgaccatg ctggtggaca cctccaagaa ccagttcagc     240

ctgcggctga gcagcgtgac agccgccgat acagccgtgt actactgcgc cagagaggac     300

tacgactacc actggtactt cgacgtgtgg ggccagggct ctctcgtgac cgtgtcaagc     360

gctagcacca agggcccctc cgtgttcccc ctggccccca gcagcaagag caccagcggc     420

ggcacagccg ctctgggctg cctggtcaag gactacttcc ccgagcccgt gaccgtgtcc     480

tggaacagcg gagccctgac ctccggcgtg cacaccttcc ccgccgtgct gcagagttct     540

ggcctgtata gcctgagcag cgtggtcacc gtgccttcta gcagcctggg cacccagacc     600

tacatctgca acgtgaacca caagcccagc aacaccaagg tggacaagaa ggtggagccc     660

aagagctgcg acaaaactca cacatgccca ccgtgcccag cacctgaagc tgcaggggga     720

ccgtcagtct tcctcttccc cccaaaaccc aaggacaccc tcatgatctc ccggacccct     780

gaggtcacat gcgtggtggt ggacgtgagc cacgaagacc ctgaggtcaa gttcaactgg     840

tacgtggacg gcgtggaggt gcataatgcc aagacaaagc cgcgggagga gcagtacaac     900

agcacgtacc gtgtggtcag cgtcctcacc gtcctgcacc aggactggct gaatggcaag     960

gagtacaagt gcaaggtctc caacaaagcc ctcggcgccc ccatcgagaa aaccatctcc    1020

aaagccaaag ggcagccccg agaaccacag gtgtgcaccc tgcccccatc ccgggatgag    1080

ctgaccaaga accaggtcag cctctcgtgc gcagtcaaag gcttctatcc cagcgacatc 1140

gccgtggagt gggagagcaa tgggcagccg gagaacaact acaagaccac gcctcccgtg 1200

ctggactccg acggctcctt cttcctcgtg agcaagctca ccgtggacaa gagcaggtgg 1260

cagcagggga acgtcttctc atgctccgtg atgcatgagg ctctgcacaa ccactacacg 1320

cagaagagcc tctccctgtc tccgggtaaa 1350

<210> 209
<211> 464
<212> PRT
<213> Artificial Sequence

<220>
<223> CH1A1A(VL-CL)-V9 (VH-CL)

<400> 209

Asp Ile Gln Met Thr Gln Ser Pro Ser Ser Leu Ser Ala Ser Val Gly
1               5                   10                  15

Asp Arg Val Thr Ile Thr Cys Lys Ala Ser Ala Ala Val Gly Thr Tyr
            20                  25                  30

Val Ala Trp Tyr Gln Gln Lys Pro Gly Lys Ala Pro Lys Leu Leu Ile
        35                  40                  45

Tyr Ser Ala Ser Tyr Arg Lys Arg Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys His Gln Tyr Tyr Thr Tyr Pro Leu
                85                  90                  95

Phe Thr Phe Gly Gln Gly Thr Lys Leu Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu

                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys Gly Gly Gly Gly Ser Gly Gly Gly Gly
    210                 215                 220

Ser Gly Gly Gly Gly Ser Gly Gly Gly Gly Ser Glu Val Gln Leu Val
225                 230                 235                 240

Glu Ser Gly Gly Gly Leu Val Gln Pro Gly Gly Ser Leu Arg Leu Ser
                245                 250                 255

Cys Ala Ala Ser Gly Tyr Ser Phe Thr Gly Tyr Thr Met Asn Trp Val
            260                 265                 270

Arg Gln Ala Pro Gly Lys Gly Leu Glu Trp Val Ala Leu Ile Asn Pro
        275                 280                 285

Tyr Lys Gly Val Ser Thr Tyr Asn Gln Lys Phe Lys Asp Arg Phe Thr
    290                 295                 300

Ile Ser Val Asp Lys Ser Lys Asn Thr Ala Tyr Leu Gln Met Asn Ser
305                 310                 315                 320

Leu Arg Ala Glu Asp Thr Ala Val Tyr Tyr Cys Ala Arg Ser Gly Tyr
                325                 330                 335

Tyr Gly Asp Ser Asp Trp Tyr Phe Asp Val Trp Gly Gln Gly Thr Leu
            340                 345                 350

Val Thr Val Ser Ser Ala Ser Val Ala Ala Pro Ser Val Phe Ile Phe
        355                 360                 365

Pro Pro Ser Asp Glu Gln Leu Lys Ser Gly Thr Ala Ser Val Val Cys
    370                 375                 380

Leu Leu Asn Asn Phe Tyr Pro Arg Glu Ala Lys Val Gln Trp Lys Val
385                 390                 395                 400

Asp Asn Ala Leu Gln Ser Gly Asn Ser Gln Glu Ser Val Thr Glu Gln
                405                 410                 415

Asp Ser Lys Asp Ser Thr Tyr Ser Leu Ser Ser Thr Leu Thr Leu Ser
            420                 425                 430

Lys Ala Asp Tyr Glu Lys His Lys Val Tyr Ala Cys Glu Val Thr His
        435                 440                 445

Gln Gly Leu Ser Ser Pro Val Thr Lys Ser Phe Asn Arg Gly Glu Cys
    450                 455                 460

<210>  210
<211>  1392
<212>  DNA
<213>  Artificial Sequence

<220>
<223>  CH1A1A(VL-CL)-V9 (VH-CL)

<400>  210
gatatccaga tgacccagag ccccagcagc ctgtctgcca gcgtgggcga cagagtgacc      60

atcacatgca aggcctctgc cgccgtgggc acatacgtgg cctggtatca gcagaagccc     120

ggcaaggccc ccaagctgct gatctacagc gccagctacc ggaagagagg cgtgcccagc     180

agattttccg gcagcggctc tggcaccgac ttcaccctga ccatcagctc cctgcagccc     240

gaggacttcg ccacctacta ctgccaccag tactacacct accccctgtt caccttcggc     300

cagggcacca agctcgagat caagcgtacg gtggccgctc ccagcgtgtt catcttccca     360

cctagcgacg agcagctgaa gtccggcaca gcctctgtcg tgtgcctgct gaacaacttc     420

tacccccgcg aggccaaggt gcagtggaag gtggacaatg ccctgcagag cggcaacagc     480

caggaaagcg tgaccgagca ggacagcaag gactccacct acagcctgag cagcaccctg     540

acactgagca aggccgacta cgagaagcac aaggtgtacg cctgcgaagt gacccaccag     600

ggcctgtcta gccccgtgac caagagcttc aaccggggcg aatgtggcgg cggaggatcc     660

ggcggaggcg gctccggagg cggaggaagt ggcggagggg gatctgaagt gcagctggtg     720

gaatctggcg gaggcctggt gcagcctggc ggatctctga gactgagctg tgccgccagc     780

ggctacagct tcaccggcta caccatgaac tgggtgcgcc aggcccctgg caagggactg     840

gaatgggtgg ccctgatcaa cccctacaag ggcgtgtcca catacaacca gaagttcaag     900

gaccggttca ccatcagcgt ggacaagagc aagaacaccg cctacctgca gatgaacagc     960

ctgcgggccg aggacaccgc cgtgtactac tgtgccagaa gcggctacta cggcgacagc    1020

gactggtact tcgacgtgtg gggccaggga accctcgtga ccgtgtcaag cgctagcgtg    1080

gccgcaccct ctgtgtttat ctttccaccc tctgacgaac agctgaaaag cggcaccgcc    1140

agcgtcgtgt gtctgctgaa caatttttac cctagggaag ctaaagtgca gtggaaagtg    1200

gataacgcac tgcagtccgg caactcccag gaatctgtga cagaacagga ctccaaggac    1260

agcacatact ccctgtccag cacactgacc ctgtctaagg ccgattatga gaaacacaaa 1320

gtgtatgctt gtgaagtgac acatcaggga ctgagcagcc ctgtgacaaa gtccttcaac 1380

agaggcgagt gt 1392

<210> 211
<211> 451
<212> PRT
<213> Artificial Sequence

<220>
<223> CH1A1A(VH-CH1)-Fc(knob) P329G LALA

<400> 211

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Glu Phe
            20                  25                  30

Gly Met Asn Trp Val Arg Gln Ala Pro Gly Gln Gly Leu Glu Trp Met
        35                  40                  45

Gly Trp Ile Asn Thr Lys Thr Gly Glu Ala Thr Tyr Val Glu Glu Phe
    50                  55                  60

Lys Gly Arg Val Thr Phe Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Trp Asp Phe Ala Tyr Tyr Val Glu Ala Met Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Ala Ala Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Gly Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Cys Arg Asp Glu Leu Thr Lys Asn Gln Val Ser
        355                 360                 365

Leu Trp Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser

435                 440                 445

Pro Gly Lys
    450

<210>  212
<211>  1353
<212>  DNA
<213>  Artificial Sequence

<220>
<223>  CH1A1A(VH-CH1)-Fc(knob) P329G LALA

<400>  212
caggtgcagc tggtgcagtc tggcgccgaa gtgaagaaac ctggagctag tgtgaaggtg      60

tcctgcaagg ccagcggcta caccttcacc gagttcggca tgaactgggt ccgacaggct     120

ccaggccagg gcctcgaatg gatgggctgg atcaacacca agaccggcga ggccacctac     180

gtggaagagt tcaagggcag agtgaccttc accacggaca ccagcaccag caccgcctac     240

atggaactgc ggagcctgag aagcgacgac accgccgtgt actactgcgc cagatgggac     300

ttcgcctatt acgtggaagc catggactac tggggccagg gcaccaccgt gaccgtgtct     360

agcgctagca ccaagggccc atcggtcttc cccctggcac cctcctccaa gagcacctct     420

gggggcacag cggccctggg ctgcctggtc aaggactact tccccgaacc ggtgacggtg     480

tcgtggaact caggcgccct gaccagcggc gtgcacacct tcccggctgt cctacagtcc     540

tcaggactct actccctcag cagcgtggtg accgtgccct ccagcagctt gggcacccag     600

acctacatct gcaacgtgaa tcacaagccc agcaacacca aggtggacaa gaaagttgag     660

cccaaatctt gtgacaaaac tcacacatgc ccaccgtgcc cagcacctga agctgcaggg     720

ggaccgtcag tcttcctctt ccccccaaaa cccaaggaca ccctcatgat ctcccggacc     780

cctgaggtca catgcgtggt ggtggacgtg agccacgaag accctgaggt caagttcaac     840

tggtacgtgg acggcgtgga ggtgcataat gccaagacaa agccgcggga ggagcagtac     900

aacagcacgt accgtgtggt cagcgtcctc accgtcctgc accaggactg gctgaatggc     960

aaggagtaca agtgcaaggt ctccaacaaa gccctcggcg cccccatcga gaaaaccatc    1020

tccaaagcca aagggcagcc ccgagaacca caggtgtaca ccctgccccc atgccgggat    1080

gagctgacca agaaccaggt cagcctgtgg tgcctggtca aaggcttcta tcccagcgac    1140

atcgccgtgg agtgggagag caatgggcag ccggagaaca actacaagac cacgcctccc    1200

gtgctggact ccgacggctc cttcttcctc tacagcaagc tcaccgtgga caagagcagg    1260

tggcagcagg ggaacgtctt ctcatgctcc gtgatgcatg aggctctgca caaccactac    1320

acgcagaaga gcctctccct gtctccgggt aaa                                 1353

<210>  213
<211>  227
<212>  PRT
<213>  Artificial Sequence

<220>
<223>  Fc(hole) P329G LALA

<400>  213

Asp Lys Thr His Thr Cys Pro Pro Cys Pro Ala Pro Glu Ala Ala Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Gly Ala Pro Ile
            100                 105                 110

Glu Lys Thr Ile Ser Lys Ala Lys Gly Gln Pro Arg Glu Pro Gln Val
        115                 120                 125

Cys Thr Leu Pro Pro Ser Arg Asp Glu Leu Thr Lys Asn Gln Val Ser
    130                 135                 140

Leu Ser Cys Ala Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Val Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser

210                 215                 220

Pro Gly Lys
225

<210>  214
<211>  681
<212>  DNA
<213>  Artificial Sequence

<220>
<223>  Fc(hole) P329G LALA

<400>  214
gacaaaactc acacatgccc accgtgccca gcacctgaag ctgcaggggg accgtcagtc      60

ttcctcttcc ccccaaaacc caaggacacc ctcatgatct cccggacccc tgaggtcaca     120

tgcgtggtgg tggacgtgag ccacgaagac cctgaggtca agttcaactg gtacgtggac     180

ggcgtggagg tgcataatgc caagacaaag ccgcgggagg agcagtacaa cagcacgtac     240

cgtgtggtca gcgtcctcac cgtcctgcac caggactggc tgaatggcaa ggagtacaag     300

tgcaaggtct ccaacaaagc cctcggcgcc cccatcgaga aaaccatctc caaagccaaa     360

gggcagcccc gagaaccaca ggtgtgcacc ctgcccccat cccgggatga gctgaccaag     420

aaccaggtca gcctctcgtg cgcagtcaaa ggcttctatc ccagcgacat cgccgtggag     480

tgggagagca atgggcagcc ggagaacaac tacaagacca cgcctcccgt gctggactcc     540

gacggctcct tcttcctcgt gagcaagctc accgtggaca agagcaggtg gcagcagggg     600

aacgtcttct catgctccgt gatgcatgag gctctgcaca accactacac gcagaagagc     660

ctctccctgt ctccgggtaa a                                               681

<210>  215
<211>  214
<212>  PRT
<213>  Artificial Sequence

<220>
<223>  CH2527(VL-CH1)

<400>  215

Gln Ala Val Val Thr Gln Glu Ser Ala Leu Thr Thr Ser Pro Gly Glu
1               5                   10                  15

Thr Val Thr Leu Thr Cys Arg Ser Ser Thr Gly Ala Val Thr Thr Ser
            20                  25                  30

Asn Tyr Ala Asn Trp Val Gln Glu Lys Pro Asp His Leu Phe Thr Gly
        35                  40                  45

Leu Ile Gly Gly Thr Asn Lys Arg Ala Pro Gly Val Pro Ala Arg Phe
    50                  55                  60

Ser Gly Ser Leu Ile Gly Asp Lys Ala Ala Leu Thr Ile Thr Gly Ala
65                  70                  75                  80

Gln Thr Glu Asp Glu Ala Ile Tyr Phe Cys Ala Leu Trp Tyr Ser Asn
                85                  90                  95

Leu Trp Val Phe Gly Gly Gly Thr Lys Leu Thr Val Leu Ser Ser Ala
            100                 105                 110

Ser Thr Lys Gly Pro Ser Val Phe Pro Leu Ala Pro Ser Ser Lys Ser
        115                 120                 125

Thr Ser Gly Gly Thr Ala Ala Leu Gly Cys Leu Val Lys Asp Tyr Phe
    130                 135                 140

Pro Glu Pro Val Thr Val Ser Trp Asn Ser Gly Ala Leu Thr Ser Gly
145                 150                 155                 160

Val His Thr Phe Pro Ala Val Leu Gln Ser Ser Gly Leu Tyr Ser Leu
                165                 170                 175

Ser Ser Val Val Thr Val Pro Ser Ser Ser Leu Gly Thr Gln Thr Tyr
            180                 185                 190

Ile Cys Asn Val Asn His Lys Pro Ser Asn Thr Lys Val Asp Lys Lys
        195                 200                 205

Val Glu Pro Lys Ser Cys
    210

<210>  216
<211>  642
<212>  DNA
<213>  Artificial Sequence

<220>
<223>  CH2527(VL-CH1)

<400>  216
caggccgtcg tgacccagga aagcgccctg acaacaagcc ctggcgagac agtgaccctg      60

acctgcagat ctagcacagg cgccgtgacc accagcaact acgccaactg ggtgcaggaa     120

aagcccgacc acctgttcac cggcctgatc ggcggcacca acaaaagggc tccaggcgtg     180

ccagccagat tcagcggcag cctgattggc gataaggccg ccctgaccat cactggcgcc     240

cagacagagg acgaggccat ctacttttgc gccctgtggt acagcaacct gtgggtgttc     300

ggcggaggca ccaagctgac agtgctgagc agcgcttcca ccaaaggccc ttccgtgttt 360

cctctggctc ctagctccaa gtccacctct ggaggcaccg ctgctctcgg atgcctcgtg 420

aaggattatt ttcctgagcc tgtgacagtg tcctggaata gcggagcact gacctctgga 480

gtgcatactt tccccgctgt gctgcagtcc tctggactgt acagcctgag cagcgtggtg 540

acagtgccca gcagcagcct gggcacccag acctacatct gcaacgtgaa ccacaagccc 600

agcaacacca aggtggacaa gaaggtggaa cccaagtctt gt 642

<210> 217
<211> 684
<212> PRT
<213> Artificial Sequence

<220>
<223> CH2527(VH-CL)-LC007(VH-CH1)-Fc(knob) P329G LALA

<400> 217

Glu Val Gln Leu Val Glu Ser Gly Gly Gly Leu Val Gln Pro Lys Gly
1               5                   10                  15

Ser Leu Lys Leu Ser Cys Ala Ala Ser Gly Phe Thr Phe Asn Thr Tyr
            20                  25                  30

Ala Met Asn Trp Val Arg Gln Ala Pro Gly Lys Gly Leu Glu Trp Val
        35                  40                  45

Ala Arg Ile Arg Ser Lys Tyr Asn Asn Tyr Ala Thr Tyr Tyr Ala Asp
    50                  55                  60

Ser Val Lys Asp Arg Phe Thr Ile Ser Arg Asp Asp Ser Gln Ser Ile
65                  70                  75                  80

Leu Tyr Leu Gln Met Asn Asn Leu Lys Thr Glu Asp Thr Ala Met Tyr
                85                  90                  95

Tyr Cys Val Arg His Gly Asn Phe Gly Asn Ser Tyr Val Ser Trp Phe
            100                 105                 110

Ala Tyr Trp Gly Gln Gly Thr Leu Val Thr Val Ser Ala Ala Ser Val
        115                 120                 125

Ala Ala Pro Ser Val Phe Ile Phe Pro Pro Ser Asp Glu Gln Leu Lys
    130                 135                 140

Ser Gly Thr Ala Ser Val Val Cys Leu Leu Asn Asn Phe Tyr Pro Arg
145                 150                 155                 160

Glu Ala Lys Val Gln Trp Lys Val Asp Asn Ala Leu Gln Ser Gly Asn
                165                 170                 175

Ser Gln Glu Ser Val Thr Glu Gln Asp Ser Lys Asp Ser Thr Tyr Ser
            180                 185                 190

Leu Ser Ser Thr Leu Thr Leu Ser Lys Ala Asp Tyr Glu Lys His Lys
        195                 200                 205

Val Tyr Ala Cys Glu Val Thr His Gln Gly Leu Ser Ser Pro Val Thr
    210                 215                 220

Lys Ser Phe Asn Arg Gly Glu Cys Gly Gly Gly Gly Ser Gly Gly Gly
225                 230                 235                 240

Gly Ser Glu Val Gln Leu Gln Glu Ser Gly Pro Gly Leu Val Lys Pro
                245                 250                 255

Ser Gln Ser Leu Ser Leu Thr Cys Ser Val Thr Gly Tyr Ser Ile Thr
            260                 265                 270

Ser Gly Tyr Tyr Trp Asn Trp Ile Arg Gln Phe Pro Gly Asn Lys Leu
        275                 280                 285

Glu Trp Met Gly Tyr Ile Thr Tyr Asp Gly Ser Asn Asn Tyr Asn Pro
    290                 295                 300

Ser Leu Lys Asn Arg Ile Ser Ile Thr Arg Asp Thr Ser Lys Asn Gln
305                 310                 315                 320

Phe Phe Leu Lys Leu Asn Ser Val Thr Thr Glu Asp Thr Ala Thr Tyr
                325                 330                 335

Tyr Cys Ala Asp Phe Asp Tyr Trp Gly Gln Gly Thr Thr Leu Thr Val
            340                 345                 350

Ser Ser Ala Ser Thr Lys Gly Pro Ser Val Phe Pro Leu Ala Pro Ser
        355                 360                 365

Ser Lys Ser Thr Ser Gly Gly Thr Ala Ala Leu Gly Cys Leu Val Lys
    370                 375                 380

Asp Tyr Phe Pro Glu Pro Val Thr Val Ser Trp Asn Ser Gly Ala Leu
385                 390                 395                 400

Thr Ser Gly Val His Thr Phe Pro Ala Val Leu Gln Ser Ser Gly Leu
                405                 410                 415

Tyr Ser Leu Ser Ser Val Val Thr Val Pro Ser Ser Ser Leu Gly Thr
            420                 425                 430

Gln Thr Tyr Ile Cys Asn Val Asn His Lys Pro Ser Asn Thr Lys Val
        435                 440                 445

Asp Lys Lys Val Glu Pro Lys Ser Cys Asp Lys Thr His Thr Cys Pro
    450                 455                 460

Pro Cys Pro Ala Pro Glu Ala Ala Gly Gly Pro Ser Val Phe Leu Phe
465                 470                 475                 480

Pro Pro Lys Pro Lys Asp Thr Leu Met Ile Ser Arg Thr Pro Glu Val
                485                 490                 495

Thr Cys Val Val Val Asp Val Ser His Glu Asp Pro Glu Val Lys Phe
            500                 505                 510

Asn Trp Tyr Val Asp Gly Val Glu Val His Asn Ala Lys Thr Lys Pro
        515                 520                 525

Arg Glu Glu Gln Tyr Asn Ser Thr Tyr Arg Val Val Ser Val Leu Thr
    530                 535                 540

Val Leu His Gln Asp Trp Leu Asn Gly Lys Glu Tyr Lys Cys Lys Val
545                 550                 555                 560

Ser Asn Lys Ala Leu Gly Ala Pro Ile Glu Lys Thr Ile Ser Lys Ala
                565                 570                 575

Lys Gly Gln Pro Arg Glu Pro Gln Val Tyr Thr Leu Pro Pro Cys Arg
            580                 585                 590

Asp Glu Leu Thr Lys Asn Gln Val Ser Leu Trp Cys Leu Val Lys Gly
        595                 600                 605

Phe Tyr Pro Ser Asp Ile Ala Val Glu Trp Glu Ser Asn Gly Gln Pro
    610                 615                 620

Glu Asn Asn Tyr Lys Thr Thr Pro Pro Val Leu Asp Ser Asp Gly Ser
625                 630                 635                 640

Phe Phe Leu Tyr Ser Lys Leu Thr Val Asp Lys Ser Arg Trp Gln Gln
                645                 650                 655

Gly Asn Val Phe Ser Cys Ser Val Met His Glu Ala Leu His Asn His
            660                 665                 670

Tyr Thr Gln Lys Ser Leu Ser Leu Ser Pro Gly Lys
        675                 680

<210>  218
<211>  2052
<212>  DNA
<213>  Artificial Sequence

<220>
<223>  CH2527(VH-CL)-LC007(VH-CH1)-Fc(knob) P329G LALA

<400>  218
gaagtgcagc tggtggaaag cggcggaggc ctggtgcagc ctaagggctc tctgaagctg      60

agctgtgccg ccagcggctt caccttcaac acctacgcca tgaactgggt gcgccaggcc     120

cctggcaaag gcctggaatg ggtggcccgg atcagaagca agtacaacaa ttacgccacc     180

tactacgccg acagcgtgaa ggaccggttc accatcagcc gggacgacag ccagagcatc     240

ctgtacctgc agatgaacaa cctgaaaacc gaggacaccg ccatgtacta ctgcgtgcgg     300

cacggcaact tcggcaacag ctatgtgtct tggtttgcct actggggcca gggcaccctc     360

gtgacagtgt ctgctgctag cgtggctgca ccatctgtct tcatcttccc gccatctgat     420

gagcagttga aatctggaac tgcctctgtt gtgtgcctgc tgaataactt ctatcccaga     480

gaggccaaag tacagtggaa ggtggataac gccctccaat cgggtaactc ccaggagagt     540

gtcacagagc aggacagcaa ggacagcacc tacagcctca gcagcaccct gacgctgagc     600

aaagcagact acgagaaaca caaagtctac gcctgcgaag tcacccatca gggcctgagc     660

tcgcccgtca caaagagctt caacagggga gagtgtggcg gaggcggatc cggcggaggg     720

ggatctgagg tccagctgca ggagtcagga cctggcctcg tgaaaccttc tcagtctctg     780

tctctcacct gctctgtcac tggctactcc atcaccagtg gttattactg gaactggatt     840

cggcagtttc caggaaacaa gctggaatgg atgggctaca taacctacga cggtagcaat     900

aactacaacc catctctcaa aaatcgaatc tccattactc gtgacacatc taagaaccag     960

tttttcctga agttgaattc tgtgactact gaggacacag ctacatatta ctgtgcggac    1020

tttgactact ggggccaagg caccactctc acagtctcca gcgctagcac caagggcccc    1080

agcgtgttcc ccctggcacc cagcagcaag agcacatctg gcggaacagc cgctctgggc    1140

tgtctggtga aagactactt ccccgagccc gtgaccgtgt cttggaactc tggcgccctg    1200

accagcggcg tgcacacctt tccagccgtg ctgcagagca gcggcctgta ctccctgtcc    1260

tccgtggtca ccgtgccctc tagctccctg ggaacacaga catatatctg taatgtcaat    1320

cacaagcctt ccaacaccaa agtcgataag aaagtcgagc ccaagagctg cgacaaaact    1380

cacacatgcc caccgtgccc agcacctgaa gctgcagggg gaccgtcagt cttcctcttc    1440

cccccaaaac ccaaggacac cctcatgatc tcccggaccc ctgaggtcac atgcgtggtg 1500

gtggacgtga gccacgaaga ccctgaggtc aagttcaact ggtacgtgga cggcgtggag 1560

gtgcataatg ccaagacaaa gccgcgggag gagcagtaca acagcacgta ccgtgtggtc 1620

agcgtcctca ccgtcctgca ccaggactgg ctgaatggca aggagtacaa gtgcaaggtc 1680

tccaacaaag ccctcggcgc ccccatcgag aaaaccatct ccaaagccaa agggcagccc 1740

cgagaaccac aggtgtacac cctgccccca tgccgggatg agctgaccaa gaaccaggtc 1800

agcctgtggt gcctggtcaa aggcttctat cccagcgaca tcgccgtgga gtgggagagc 1860

aatgggcagc cggagaacaa ctacaagacc acgcctcccg tgctggactc cgacggctcc 1920

ttcttcctct acagcaagct caccgtggac aagagcaggt ggcagcaggg gaacgtcttc 1980

tcatgctccg tgatgcatga ggctctgcac aaccactaca cgcagaagag cctctccctg 2040

tctccgggta aa 2052

<210> 219
<211> 685
<212> PRT
<213> Artificial Sequence

<220>
<223> LC007(VH-CH1)-CH2527(VH-CL)-Fc(knob) P329G LALA

<400> 219

Glu Val Gln Leu Gln Glu Ser Gly Pro Gly Leu Val Lys Pro Ser Gln
1               5                   10                  15

Ser Leu Ser Leu Thr Cys Ser Val Thr Gly Tyr Ser Ile Thr Ser Gly
            20                  25                  30

Tyr Tyr Trp Asn Trp Ile Arg Gln Phe Pro Gly Asn Lys Leu Glu Trp
        35                  40                  45

Met Gly Tyr Ile Thr Tyr Asp Gly Ser Asn Asn Tyr Asn Pro Ser Leu
    50                  55                  60

Lys Asn Arg Ile Ser Ile Thr Arg Asp Thr Ser Lys Asn Gln Phe Phe
65                  70                  75                  80

Leu Lys Leu Asn Ser Val Thr Thr Glu Asp Thr Ala Thr Tyr Tyr Cys
                85                  90                  95

Ala Asp Phe Asp Tyr Trp Gly Gln Gly Thr Thr Leu Thr Val Ser Ser
            100                 105                 110

Ala Ser Thr Lys Gly Pro Ser Val Phe Pro Leu Ala Pro Ser Ser Lys
        115                 120                 125

Ser Thr Ser Gly Gly Thr Ala Ala Leu Gly Cys Leu Val Lys Asp Tyr
    130                 135                 140

Phe Pro Glu Pro Val Thr Val Ser Trp Asn Ser Gly Ala Leu Thr Ser
145                 150                 155                 160

Gly Val His Thr Phe Pro Ala Val Leu Gln Ser Ser Gly Leu Tyr Ser
                165                 170                 175

Leu Ser Ser Val Val Thr Val Pro Ser Ser Ser Leu Gly Thr Gln Thr
            180                 185                 190

Tyr Ile Cys Asn Val Asn His Lys Pro Ser Asn Thr Lys Val Asp Lys
        195                 200                 205

Lys Val Glu Pro Lys Ser Cys Asp Gly Gly Gly Gly Ser Gly Gly Gly
    210                 215                 220

Gly Ser Glu Val Gln Leu Val Glu Ser Gly Gly Gly Leu Val Gln Pro
225                 230                 235                 240

Lys Gly Ser Leu Lys Leu Ser Cys Ala Ala Ser Gly Phe Thr Phe Asn
                245                 250                 255

Thr Tyr Ala Met Asn Trp Val Arg Gln Ala Pro Gly Lys Gly Leu Glu
            260                 265                 270

Trp Val Ala Arg Ile Arg Ser Lys Tyr Asn Asn Tyr Ala Thr Tyr Tyr
        275                 280                 285

Ala Asp Ser Val Lys Asp Arg Phe Thr Ile Ser Arg Asp Asp Ser Gln
    290                 295                 300

Ser Ile Leu Tyr Leu Gln Met Asn Asn Leu Lys Thr Glu Asp Thr Ala
305                 310                 315                 320

Met Tyr Tyr Cys Val Arg His Gly Asn Phe Gly Asn Ser Tyr Val Ser
                325                 330                 335

Trp Phe Ala Tyr Trp Gly Gln Gly Thr Leu Val Thr Val Ser Ala Ala
            340                 345                 350

Ser Val Ala Ala Pro Ser Val Phe Ile Phe Pro Pro Ser Asp Glu Gln
        355                 360                 365

Leu Lys Ser Gly Thr Ala Ser Val Val Cys Leu Leu Asn Asn Phe Tyr

370                 375                 380

Pro Arg Glu Ala Lys Val Gln Trp Lys Val Asp Asn Ala Leu Gln Ser
385                 390                 395                 400

Gly Asn Ser Gln Glu Ser Val Thr Glu Gln Asp Ser Lys Asp Ser Thr
                405                 410                 415

Tyr Ser Leu Ser Ser Thr Leu Thr Leu Ser Lys Ala Asp Tyr Glu Lys
            420                 425                 430

His Lys Val Tyr Ala Cys Glu Val Thr His Gln Gly Leu Ser Ser Pro
        435                 440                 445

Val Thr Lys Ser Phe Asn Arg Gly Glu Cys Asp Lys Thr His Thr Cys
    450                 455                 460

Pro Pro Cys Pro Ala Pro Glu Ala Ala Gly Gly Pro Ser Val Phe Leu
465                 470                 475                 480

Phe Pro Pro Lys Pro Lys Asp Thr Leu Met Ile Ser Arg Thr Pro Glu
                485                 490                 495

Val Thr Cys Val Val Val Asp Val Ser His Glu Asp Pro Glu Val Lys
            500                 505                 510

Phe Asn Trp Tyr Val Asp Gly Val Glu Val His Asn Ala Lys Thr Lys
        515                 520                 525

Pro Arg Glu Glu Gln Tyr Asn Ser Thr Tyr Arg Val Val Ser Val Leu
    530                 535                 540

Thr Val Leu His Gln Asp Trp Leu Asn Gly Lys Glu Tyr Lys Cys Lys
545                 550                 555                 560

Val Ser Asn Lys Ala Leu Gly Ala Pro Ile Glu Lys Thr Ile Ser Lys
                565                 570                 575

Ala Lys Gly Gln Pro Arg Glu Pro Gln Val Tyr Thr Leu Pro Pro Cys
            580                 585                 590

Arg Asp Glu Leu Thr Lys Asn Gln Val Ser Leu Trp Cys Leu Val Lys
        595                 600                 605

Gly Phe Tyr Pro Ser Asp Ile Ala Val Glu Trp Glu Ser Asn Gly Gln
    610                 615                 620

Pro Glu Asn Asn Tyr Lys Thr Thr Pro Pro Val Leu Asp Ser Asp Gly
625                 630                 635                 640

Ser Phe Phe Leu Tyr Ser Lys Leu Thr Val Asp Lys Ser Arg Trp Gln
                645                 650                 655

Gln Gly Asn Val Phe Ser Cys Ser Val Met His Glu Ala Leu His Asn
            660                 665                 670

His Tyr Thr Gln Lys Ser Leu Ser Leu Ser Pro Gly Lys
        675                 680                 685

<210>  220
<211>  2055
<212>  DNA
<213>  Artificial Sequence

<220>
<223>  LC007(VH-CH1)-CH2527(VH-CL)-Fc(knob) P329G LALA

<400>  220
gaggtgcagc tgcaggaatc tggccctggc ctggtcaagc caagccagag tctgagcctg      60

acctgcagcg tgaccggcta cagcattacc agcggctact actggaactg gattcggcag     120

ttccccggca ataagctgga atggatgggc tacatcacct acgacggcag caacaactac     180

aaccccagcc tgaagaaccg gatcagcatc acccgggaca ccagcaagaa ccagttcttc     240

ctgaagctga acagcgtgac caccgaggac accgccacat actattgcgc cgacttcgac     300

tactggggcc agggcaccac cctgaccgtg tccagcgcca gcacaaaggg ccctagcgtg     360

ttccctctgg cccccagcag caagagcaca agcggcggaa cagccgccct gggctgcctc     420

gtgaaggact acttccccga gcccgtgaca gtgtcttgga acagcggagc cctgacaagc     480

ggcgtgcaca ccttccctgc cgtgctgcag agcagcggcc tgtactccct gagcagcgtg     540

gtcaccgtgc ctagcagcag cctgggcacc cagacctaca tctgcaacgt gaaccacaag     600

cccagcaaca ccaaagtgga caagaaggtg gagcccaaga gctgtgatgg cggaggaggg     660

tccggaggcg gaggatccga agtgcagctg gtggaaagcg gcggaggcct ggtgcagcct     720

aagggctctc tgaagctgag ctgtgccgcc agcggcttca ccttcaacac ctacgccatg     780

aactgggtgc gccaggcccc tggcaaaggc ctggaatggg tggcccggat cagaagcaag     840

tacaacaatt acgccaccta ctacgccgac agcgtgaagg accggttcac catcagccgg     900

gacgacagcc agagcatcct gtacctgcag atgaacaacc tgaaaaccga ggacaccgcc     960

atgtactact gcgtgcggca cggcaacttc ggcaacagct atgtgtcttg gtttgcctac    1020

tggggccagg gcaccctcgt gacagtgtct gctgctagcg tggccgctcc ctccgtgttt    1080

atctttcccc catccgatga acagctgaaa agcggcaccg cctccgtcgt gtgtctgctg    1140

aacaattttt accctaggga agctaaagtg cagtggaaag tggataacgc actgcagtcc 1200

ggcaactccc aggaatctgt gacagaacag gactccaagg acagcaccta ctccctgtcc 1260

tccaccctga cactgtctaa ggctgattat gagaaacaca aagtctacgc ctgcgaagtc 1320

acccatcagg gcctgagctc gcccgtcaca aagagcttca acaggggaga gtgtgacaag 1380

acccacacct gtcccccttg tcctgcccct gaagctgctg gcggcccttc tgtgttcctg 1440

ttccccccaa agcccaagga caccctgatg atcagccgga cccccgaagt gacctgcgtg 1500

gtggtggatg tgtcccacga ggaccctgaa gtgaagttca attggtacgt ggacggcgtg 1560

gaagtgcaca acgccaagac aaagccgcgg gaggagcagt acaacagcac gtaccgtgtg 1620

gtcagcgtcc tcaccgtcct gcaccaggac tggctgaatg gcaaggagta caagtgcaag 1680

gtctccaaca aagccctcgg cgcccccatc gagaaaacca tctccaaagc caaagggcag 1740

ccccgagaac cacaggtgta caccctgccc ccatgccggg atgagctgac caagaaccag 1800

gtcagcctgt ggtgcctggt caaaggcttc tatcccagcg acatcgccgt ggagtgggag 1860

agcaatgggc agccggagaa caactacaag accacgcctc ccgtgctgga ctccgacggc 1920

tccttcttcc tctacagcaa gctcaccgtg gacaagagca ggtggcagca ggggaacgtc 1980

ttctcatgct ccgtgatgca tgaggctctg cacaaccact acacgcagaa gagcctctcc 2040

ctgtctccgg gtaaa 2055

<210> 221
<211> 218
<212> PRT
<213> Artificial Sequence

<220>
<223> anti-CD33(VL-CL)

<400> 221

Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Ser Val Asp Asn Tyr
            20                  25                  30

Gly Ile Ser Phe Met Asn Trp Phe Gln Gln Lys Pro Gly Lys Ala Pro
        35                  40                  45

Lys Leu Leu Ile Tyr Ala Ala Ser Asn Gln Gly Ser Gly Val Pro Ser
    50                  55                  60

Arg Phe Ser Gly Ser Gly Ser Gly Thr Asp Phe Thr Leu Thr Ile Ser
65                  70                  75                  80

Ser Leu Gln Pro Asp Asp Phe Ala Thr Tyr Tyr Cys Gln Gln Ser Lys
                85                  90                  95

Glu Val Pro Trp Thr Phe Gly Gln Gly Thr Lys Val Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210>  222
<211>  654
<212>  DNA
<213>  Artificial Sequence

<220>
<223>  anti-CD33(VL-CL)

<400>  222
gacatccaga tgacccagag ccccagcagc ctgagcgcca gcgtgggcga cagagtgacc      60

atcacctgtc gggccagcga gagcgtggac aactacggca tcagcttcat gaactggttc     120

cagcagaagc ccggcaaggc ccccaagctg ctgatctacg ccgccagcaa tcagggcagc     180

ggcgtgccca gcagattcag cggctctggc agcggcaccg acttcaccct gaccatcagc     240

agcctgcagc ccgacgactt cgccacctac tactgccagc agagcaaaga ggtgccctgg     300

accttcggcc agggcaccaa ggtggaaatc aagcgtacgg tggctgcacc atctgtcttc     360

atcttcccgc catctgatga gcagttgaaa tctggaactg cctctgttgt gtgcctgctg     420

aataacttct atcccagaga ggccaaagta cagtggaagg tggataacgc cctccaatcg     480

ggtaactccc aggagagtgt cacagagcag gacagcaagg acagcaccta cagcctcagc     540

agcaccctga cgctgagcaa agcagactac gagaaacaca aagtctacgc ctgcgaagtc 600

acccatcagg gcctgagctc gcccgtcaca aagagcttca acaggggaga gtgt 654

<210> 223
<211> 668
<212> PRT
<213> Artificial Sequence

<220>
<223> V9(VL-CH1)-anti-CD33(VH-CH1)-Fc(knob) P329G LALA

<400> 223

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Arg Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Arg Leu Glu Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Gly Asn Thr Leu Pro Trp
                85                  90                  95

Thr Phe Gly Gln Gly Thr Lys Val Glu Ile Lys Ser Ser Ala Ser Thr
            100                 105                 110

Lys Gly Pro Ser Val Phe Pro Leu Ala Pro Ser Ser Lys Ser Thr Ser
        115                 120                 125

Gly Gly Thr Ala Ala Leu Gly Cys Leu Val Lys Asp Tyr Phe Pro Glu
    130                 135                 140

Pro Val Thr Val Ser Trp Asn Ser Gly Ala Leu Thr Ser Gly Val His
145                 150                 155                 160

Thr Phe Pro Ala Val Leu Gln Ser Ser Gly Leu Tyr Ser Leu Ser Ser
                165                 170                 175

Val Val Thr Val Pro Ser Ser Ser Leu Gly Thr Gln Thr Tyr Ile Cys
            180                 185                 190

Asn Val Asn His Lys Pro Ser Asn Thr Lys Val Asp Lys Lys Val Glu
        195                 200                 205

Pro Lys Ser Cys Gly Gly Gly Gly Ser Gly Gly Gly Gly Ser Gln Val
    210                 215                 220

Gln Leu Val Gln Ser Gly Ala Glu Val Lys Lys Pro Gly Ser Ser Val
225                 230                 235                 240

Lys Val Ser Cys Lys Ala Ser Gly Tyr Thr Phe Thr Asp Tyr Asn Met
                245                 250                 255

His Trp Val Arg Gln Ala Pro Gly Gln Gly Leu Glu Trp Ile Gly Tyr
            260                 265                 270

Ile Tyr Pro Tyr Asn Gly Gly Thr Gly Tyr Asn Gln Lys Phe Lys Ser
        275                 280                 285

Lys Ala Thr Ile Thr Ala Asp Glu Ser Thr Asn Thr Ala Tyr Met Glu
    290                 295                 300

Leu Ser Ser Leu Arg Ser Glu Asp Thr Ala Val Tyr Tyr Cys Ala Arg
305                 310                 315                 320

Gly Arg Pro Ala Met Asp Tyr Trp Gly Gln Gly Thr Leu Val Thr Val
                325                 330                 335

Ser Ser Ala Ser Thr Lys Gly Pro Ser Val Phe Pro Leu Ala Pro Ser
            340                 345                 350

Ser Lys Ser Thr Ser Gly Gly Thr Ala Ala Leu Gly Cys Leu Val Lys
        355                 360                 365

Asp Tyr Phe Pro Glu Pro Val Thr Val Ser Trp Asn Ser Gly Ala Leu
    370                 375                 380

Thr Ser Gly Val His Thr Phe Pro Ala Val Leu Gln Ser Ser Gly Leu
385                 390                 395                 400

Tyr Ser Leu Ser Ser Val Val Thr Val Pro Ser Ser Ser Leu Gly Thr
                405                 410                 415

Gln Thr Tyr Ile Cys Asn Val Asn His Lys Pro Ser Asn Thr Lys Val
            420                 425                 430

Asp Lys Lys Val Glu Pro Lys Ser Cys Asp Lys Thr His Thr Cys Pro
        435                 440                 445

Pro Cys Pro Ala Pro Glu Ala Ala Gly Gly Pro Ser Val Phe Leu Phe
    450                 455                 460

Pro Pro Lys Pro Lys Asp Thr Leu Met Ile Ser Arg Thr Pro Glu Val
465                 470                 475                 480

Thr Cys Val Val Val Asp Val Ser His Glu Asp Pro Glu Val Lys Phe
                485                 490                 495

Asn Trp Tyr Val Asp Gly Val Glu Val His Asn Ala Lys Thr Lys Pro
            500                 505                 510

Arg Glu Glu Gln Tyr Asn Ser Thr Tyr Arg Val Val Ser Val Leu Thr
        515                 520                 525

Val Leu His Gln Asp Trp Leu Asn Gly Lys Glu Tyr Lys Cys Lys Val
    530                 535                 540

Ser Asn Lys Ala Leu Gly Ala Pro Ile Glu Lys Thr Ile Ser Lys Ala
545                 550                 555                 560

Lys Gly Gln Pro Arg Glu Pro Gln Val Tyr Thr Leu Pro Pro Cys Arg
                565                 570                 575

Asp Glu Leu Thr Lys Asn Gln Val Ser Leu Trp Cys Leu Val Lys Gly
            580                 585                 590

Phe Tyr Pro Ser Asp Ile Ala Val Glu Trp Glu Ser Asn Gly Gln Pro
        595                 600                 605

Glu Asn Asn Tyr Lys Thr Thr Pro Pro Val Leu Asp Ser Asp Gly Ser
    610                 615                 620

Phe Phe Leu Tyr Ser Lys Leu Thr Val Asp Lys Ser Arg Trp Gln Gln
625                 630                 635                 640

Gly Asn Val Phe Ser Cys Ser Val Met His Glu Ala Leu His Asn His
                645                 650                 655

Tyr Thr Gln Lys Ser Leu Ser Leu Ser Pro Gly Lys
            660                 665

<210>  224
<211>  2004
<212>  DNA
<213>  Artificial Sequence

<220>

<223>  V9(VL-CH1)-anti-CD33(VH-CH1)-Fc(knob) P329G LALA

<400>  224
gatatccaga tgacccagag ccccagctct ctgagcgcca gcgtgggcga cagagtgacc      60

atcacctgtc gggccagcca ggacatcaga aactacctga actggtatca gcagaagccc     120

ggcaaggccc ccaagctgct gatctactac accagcagac tggaaagcgg cgtgccctcc     180

agattttccg gcagcggctc cggcaccgac tacaccctga ccatcagcag cctgcagccc     240

gaggatttcg ccacatatta ctgccagcag ggcaataccc tgccctggac cttcggacag     300

ggcacaaaag tggaaatcaa gagcagcgct tccaccaaag gcccttccgt gtttcctctg     360

gctcctagct ccaagtccac ctctggaggc accgctgctc tcggatgcct cgtgaaggat     420

tattttcctg agcctgtgac agtgtcctgg aatagcggag cactgacctc tggagtgcat     480

actttccccg ctgtgctgca gtcctctgga ctgtacagcc tgagcagcgt ggtgacagtg     540

cccagcagca gcctgggcac ccagacctac atctgcaacg tgaaccacaa gcccagcaac     600

accaaggtgg acaagaaggt ggaacccaag tcttgtggcg gaggcggatc cggcggaggc     660

ggatctcagg tgcagctggt gcagtctggc gccgaagtga agaaacccgg cagcagcgtg     720

aaggtgtcct gcaaggccag cggctacacc ttcaccgact acaacatgca ctgggtccgc     780

caggccccag gccagggact ggaatggatc ggctacatct acccctacaa cggcggcacc     840

ggctacaacc agaagttcaa gagcaaggcc accatcaccg ccgacgagag caccaacacc     900

gcctacatgg aactgagcag cctgcggagc gaggacaccg ccgtgtacta ctgcgccaga     960

ggcagacccg ccatggacta ctggggccag ggcaccctgg tgacagtgtc cagcgccagc    1020

acaaagggcc ccagcgtgtt ccccctggca cccagcagca agagcacatc tggcggaaca    1080

gccgctctgg gctgtctggt gaaagactac ttccccgagc ccgtgaccgt gtcttggaac    1140

tctggcgccc tgaccagcgg cgtgcacacc tttccagccg tgctgcagag cagcggcctg    1200

tactccctgt cctccgtggt caccgtgccc tctagctccc tgggaacaca gacatatatc    1260

tgtaatgtca atcacaagcc ttccaacacc aaagtcgata agaaagtcga gcccaagagc    1320

tgcgacaaaa ctcacacatg cccaccgtgc ccagcacctg aagctgcagg gggaccgtca    1380

gtcttcctct tccccccaaa acccaaggac accctcatga tctcccggac ccctgaggtc    1440

acatgcgtgg tggtggacgt gagccacgaa gaccctgagg tcaagttcaa ctggtacgtg    1500

gacggcgtgg aggtgcataa tgccaagaca aagccgcggg aggagcagta caacagcacg    1560

taccgtgtgg tcagcgtcct caccgtcctg caccaggact ggctgaatgg caaggagtac    1620

aagtgcaagg tctccaacaa agccctcggc gcccccatcg agaaaaccat ctccaaagcc    1680

aaagggcagc cccgagaacc acaggtgtac accctgcccc catgccggga tgagctgacc    1740

aagaaccagg tcagcctgtg gtgcctggtc aaaggcttct atcccagcga catcgccgtg    1800

gagtgggaga gcaatgggca gccggagaac aactacaaga ccacgcctcc cgtgctggac 1860

tccgacggct ccttcttcct ctacagcaag ctcaccgtgg acaagagcag gtggcagcag 1920

gggaacgtct tctcatgctc cgtgatgcat gaggctctgc acaaccacta cacgcagaag 1980

agcctctccc tgtctccggg taaa 2004

<210> 225
<211> 446
<212> PRT
<213> Artificial Sequence

<220>
<223> anti-CD33(VH-CH1)-Fc(hole) P329G LALA

<400> 225

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asp Tyr
            20                  25                  30

Asn Met His Trp Val Arg Gln Ala Pro Gly Gln Gly Leu Glu Trp Ile
        35                  40                  45

Gly Tyr Ile Tyr Pro Tyr Asn Gly Gly Thr Gly Tyr Asn Gln Lys Phe
    50                  55                  60

Lys Ser Lys Ala Thr Ile Thr Ala Asp Glu Ser Thr Asn Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Arg Pro Ala Met Asp Tyr Trp Gly Gln Gly Thr Leu Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Asp Lys Thr His Thr
    210                 215                 220

Cys Pro Pro Cys Pro Ala Pro Glu Ala Ala Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Gly Ala Pro Ile Glu Lys Thr Ile Ser
                325                 330                 335

Lys Ala Lys Gly Gln Pro Arg Glu Pro Gln Val Cys Thr Leu Pro Pro
            340                 345                 350

Ser Arg Asp Glu Leu Thr Lys Asn Gln Val Ser Leu Ser Cys Ala Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Val Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Lys
        435                 440                 445

<210>  226
<211>  1338
<212>  DNA
<213>  Artificial Sequence

<220>
<223>  anti-CD33(VH-CH1)-Fc(hole) P329G LALA

<400>  226
caggtgcagc tggtgcagtc tggcgccgaa gtgaagaaac ccggcagcag cgtgaaggtg      60

tcctgcaagg ccagcggcta caccttcacc gactacaaca tgcactgggt ccgccaggcc     120

ccaggccagg gactggaatg gatcggctac atctacccct acaacggcgg caccggctac     180

aaccagaagt tcaagagcaa ggccaccatc accgccgacg agagcaccaa caccgcctac     240

atggaactga gcagcctgcg gagcgaggac accgccgtgt actactgcgc cagaggcaga     300

cccgccatgg actactgggg ccagggcacc ctggtgacag tgtccagcgc tagcaccaag     360

ggcccctccg tgttccccct ggcccccagc agcaagagca ccagcggcgg cacagccgct     420

ctgggctgcc tggtcaagga ctacttcccc gagcccgtga ccgtgtcctg gaacagcgga     480

gccctgacct ccggcgtgca caccttcccc gccgtgctgc agagttctgg cctgtatagc     540

ctgagcagcg tggtcaccgt gccttctagc agcctgggca cccagaccta catctgcaac     600

gtgaaccaca agcccagcaa caccaaggtg gacaagaagg tggagcccaa gagctgcgac     660

aaaactcaca catgcccacc gtgcccagca cctgaagctg cagggggacc gtcagtcttc     720

ctcttccccc caaaacccaa ggacaccctc atgatctccc ggacccctga ggtcacatgc     780

gtggtggtgg acgtgagcca cgaagaccct gaggtcaagt tcaactggta cgtggacggc     840

gtggaggtgc ataatgccaa gacaaagccg cgggaggagc agtacaacag cacgtaccgt     900

gtggtcagcg tcctcaccgt cctgcaccag gactggctga atggcaagga gtacaagtgc     960

aaggtctcca acaaagccct cggcgccccc atcgagaaaa ccatctccaa agccaaaggg    1020

cagccccgag aaccacaggt gtgcaccctg cccccatccc gggatgagct gaccaagaac    1080

caggtcagcc tctcgtgcgc agtcaaaggc ttctatccca gcgacatcgc cgtggagtgg    1140

gagagcaatg ggcagccgga gaacaactac aagaccacgc ctcccgtgct ggactccgac    1200

ggctccttct tcctcgtgag caagctcacc gtggacaaga gcaggtggca gcaggggaac    1260

gtcttctcat gctccgtgat gcatgaggct ctgcacaacc actacacgca gaagagcctc    1320

tccctgtctc cgggtaaa                                                  1338

<210>  227
<211>  219

<212>  PRT
<213>  Artificial Sequence

<220>
<223>  anti-CD20(VL-CL)

<400>  227

Asp Ile Val Met Thr Gln Thr Pro Leu Ser Leu Pro Val Thr Pro Gly
1               5                   10                  15

Glu Pro Ala Ser Ile Ser Cys Arg Ser Ser Lys Ser Leu Leu His Ser
            20                  25                  30

Asn Gly Ile Thr Tyr Leu Tyr Trp Tyr Leu Gln Lys Pro Gly Gln Ser
        35                  40                  45

Pro Gln Leu Leu Ile Tyr Gln Met Ser Asn Leu Val Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ala Gln Asn
                85                  90                  95

Leu Glu Leu Pro Tyr Thr Phe Gly Gly Gly Thr Lys Val Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>  228
<211>  657
<212>  DNA
<213>  Artificial Sequence

<220>
<223>  anti-CD20(VL-CL)

<400>  228
gatatcgtga tgacccagac tccactctcc ctgcccgtca cccctggaga gcccgccagc      60

attagctgca ggtctagcaa gagcctcttg cacagcaatg gcatcactta tttgtattgg     120

tacctgcaaa agccagggca gtctccacag ctcctgattt atcaaatgtc caaccttgtc     180

tctggcgtcc ctgaccggtt ctccggatcc gggtcaggca ctgatttcac actgaaaatc     240

agcagggtgg aggctgagga tgttggagtt tattactgcg ctcagaatct agaacttcct     300

tacaccttcg gcggagggac caaggtggag atcaaacgta cggtggctgc accatctgtc     360

ttcatcttcc cgccatctga tgagcagttg aaatctggaa ctgcctctgt tgtgtgcctg     420

ctgaataact tctatcccag agaggccaaa gtacagtgga aggtggataa cgccctccaa     480

tcgggtaact cccaggagag tgtcacagag caggacagca aggacagcac ctacagcctc     540

agcagcaccc tgacgctgag caaagcagac tacgagaaac acaaagtcta cgcctgcgaa     600

gtcacccatc agggcctgag ctcgcccgtc acaaagagct tcaacagggg agagtgt        657


<210>  229
<211>  671
<212>  PRT
<213>  Artificial Sequence

<220>
<223>  V9(VL-CH1)-anti-CD20(VH-CH1)-Fc(knob) P329G LALA

<400>  229

Asp Ile Gln Met Thr Gln Ser Pro Ser Ser Leu Ser Ala Ser Val Gly
1               5                   10                  15


Asp Arg Val Thr Ile Thr Cys Arg Ala Ser Gln Asp Ile Arg Asn Tyr
            20                  25                  30


Leu Asn Trp Tyr Gln Gln Lys Pro Gly Lys Ala Pro Lys Leu Leu Ile
        35                  40                  45


Tyr Tyr Thr Ser Arg Leu Glu Ser Gly Val Pro Ser Arg Phe Ser Gly
    50                  55                  60


Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Gly Asn Thr Leu Pro Trp
                85                  90                  95

Thr Phe Gly Gln Gly Thr Lys Val Glu Ile Lys Ser Ser Ala Ser Thr
            100                 105                 110

Lys Gly Pro Ser Val Phe Pro Leu Ala Pro Ser Ser Lys Ser Thr Ser
        115                 120                 125

Gly Gly Thr Ala Ala Leu Gly Cys Leu Val Lys Asp Tyr Phe Pro Glu
    130                 135                 140

Pro Val Thr Val Ser Trp Asn Ser Gly Ala Leu Thr Ser Gly Val His
145                 150                 155                 160

Thr Phe Pro Ala Val Leu Gln Ser Ser Gly Leu Tyr Ser Leu Ser Ser
                165                 170                 175

Val Val Thr Val Pro Ser Ser Ser Leu Gly Thr Gln Thr Tyr Ile Cys
            180                 185                 190

Asn Val Asn His Lys Pro Ser Asn Thr Lys Val Asp Lys Lys Val Glu
        195                 200                 205

Pro Lys Ser Cys Gly Gly Gly Gly Ser Gly Gly Gly Gly Ser Gln Val
    210                 215                 220

Gln Leu Val Gln Ser Gly Ala Glu Val Lys Lys Pro Gly Ser Ser Val
225                 230                 235                 240

Lys Val Ser Cys Lys Ala Ser Gly Tyr Ala Phe Ser Tyr Ser Trp Ile
                245                 250                 255

Asn Trp Val Arg Gln Ala Pro Gly Gln Gly Leu Glu Trp Met Gly Arg
            260                 265                 270

Ile Phe Pro Gly Asp Gly Asp Thr Asp Tyr Asn Gly Lys Phe Lys Gly
        275                 280                 285

Arg Val Thr Ile Thr Ala Asp Lys Ser Thr Ser Thr Ala Tyr Met Glu
    290                 295                 300

Leu Ser Ser Leu Arg Ser Glu Asp Thr Ala Val Tyr Tyr Cys Ala Arg
305                 310                 315                 320

Asn Val Phe Asp Gly Tyr Trp Leu Val Tyr Trp Gly Gln Gly Thr Leu
                325                 330                 335

Val Thr Val Ser Ser Ala Ser Thr Lys Gly Pro Ser Val Phe Pro Leu
            340                 345                 350

Ala Pro Ser Ser Lys Ser Thr Ser Gly Gly Thr Ala Ala Leu Gly Cys
        355                 360                 365

Leu Val Lys Asp Tyr Phe Pro Glu Pro Val Thr Val Ser Trp Asn Ser
    370                 375                 380

Gly Ala Leu Thr Ser Gly Val His Thr Phe Pro Ala Val Leu Gln Ser
385                 390                 395                 400

Ser Gly Leu Tyr Ser Leu Ser Ser Val Val Thr Val Pro Ser Ser Ser
                405                 410                 415

Leu Gly Thr Gln Thr Tyr Ile Cys Asn Val Asn His Lys Pro Ser Asn
            420                 425                 430

Thr Lys Val Asp Lys Lys Val Glu Pro Lys Ser Cys Asp Lys Thr His
        435                 440                 445

Thr Cys Pro Pro Cys Pro Ala Pro Glu Ala Ala Gly Gly Pro Ser Val
    450                 455                 460

Phe Leu Phe Pro Pro Lys Pro Lys Asp Thr Leu Met Ile Ser Arg Thr
465                 470                 475                 480

Pro Glu Val Thr Cys Val Val Val Asp Val Ser His Glu Asp Pro Glu
                485                 490                 495

Val Lys Phe Asn Trp Tyr Val Asp Gly Val Glu Val His Asn Ala Lys
            500                 505                 510

Thr Lys Pro Arg Glu Glu Gln Tyr Asn Ser Thr Tyr Arg Val Val Ser
        515                 520                 525

Val Leu Thr Val Leu His Gln Asp Trp Leu Asn Gly Lys Glu Tyr Lys
    530                 535                 540

Cys Lys Val Ser Asn Lys Ala Leu Gly Ala Pro Ile Glu Lys Thr Ile
545                 550                 555                 560

Ser Lys Ala Lys Gly Gln Pro Arg Glu Pro Gln Val Tyr Thr Leu Pro
                565                 570                 575

Pro Cys Arg Asp Glu Leu Thr Lys Asn Gln Val Ser Leu Trp Cys Leu
            580                 585                 590

Val Lys Gly Phe Tyr Pro Ser Asp Ile Ala Val Glu Trp Glu Ser Asn
        595                 600                 605

Gly Gln Pro Glu Asn Asn Tyr Lys Thr Thr Pro Pro Val Leu Asp Ser
    610                 615                 620

Asp Gly Ser Phe Phe Leu Tyr Ser Lys Leu Thr Val Asp Lys Ser Arg
625                 630                 635                 640

Trp Gln Gln Gly Asn Val Phe Ser Cys Ser Val Met His Glu Ala Leu
                645                 650                 655

His Asn His Tyr Thr Gln Lys Ser Leu Ser Leu Ser Pro Gly Lys
            660                 665                 670

<210>  230
<211>  2013
<212>  DNA
<213>  Artificial Sequence

<220>
<223>  V9(VL-CH1)-anti-CD20(VH-CH1)-Fc(knob) P329G LALA

<400>  230
gatatccaga tgacccagag ccccagctct ctgagcgcca gcgtgggcga cagagtgacc      60

atcacctgtc gggccagcca ggacatcaga aactacctga actggtatca gcagaagccc     120

ggcaaggccc ccaagctgct gatctactac accagcagac tggaaagcgg cgtgccctcc     180

agattttccg gcagcggctc cggcaccgac tacaccctga ccatcagcag cctgcagccc     240

gaggatttcg ccacatatta ctgccagcag ggcaataccc tgccctggac cttcggacag     300

ggcacaaaag tggaaatcaa gagcagcgct tccaccaaag gcccttccgt gtttcctctg     360

gctcctagct ccaagtccac ctctggaggc accgctgctc tcggatgcct cgtgaaggat     420

tattttcctg agcctgtgac agtgtcctgg aatagcggag cactgacctc tggagtgcat     480

actttccccg ctgtgctgca gtcctctgga ctgtacagcc tgagcagcgt ggtgacagtg     540

cccagcagca gcctgggcac ccagacctac atctgcaacg tgaaccacaa gcccagcaac     600

accaaggtgg acaagaaggt ggaacccaag tcttgtggcg gaggcggatc cggcggaggg     660

ggatctcagg tgcaattggt gcagtctggc gctgaagtta agaagcctgg gagttcagtg     720

aaggtctcct gcaaggcttc cggatacgcc ttcagctatt cttggatcaa ttgggtgcgg     780

caggcgcctg gacaagggct cgagtggatg ggacggatct ttcccggcga tggggatact     840

gactacaatg ggaaattcaa gggcagagtc acaattaccg ccgacaaatc cactagcaca     900

gcctatatgg agctgagcag cctgagatct gaggacacgg ccgtgtatta ctgtgcaaga     960

aatgtctttg atggttactg gcttgtttac tggggccagg gaaccctggt caccgtctcc 1020

tcagctagca ccaagggccc cagcgtgttc cccctggcac ccagcagcaa gagcacatct 1080

ggcggaacag ccgctctggg ctgtctggtg aaagactact tccccgagcc cgtgaccgtg 1140

tcttggaact ctggcgccct gaccagcggc gtgcacacct ttccagccgt gctgcagagc 1200

agcggcctgt actccctgtc ctccgtggtc accgtgccct ctagctccct gggaacacag 1260

acatatatct gtaatgtcaa tcacaagcct tccaacacca aagtcgataa gaaagtcgag 1320

cccaagagct gcgacaaaac tcacacatgc ccaccgtgcc cagcacctga agctgcaggg 1380

ggaccgtcag tcttcctctt ccccccaaaa cccaaggaca ccctcatgat ctcccggacc 1440

cctgaggtca catgcgtggt ggtggacgtg agccacgaag accctgaggt caagttcaac 1500

tggtacgtgg acggcgtgga ggtgcataat gccaagacaa agccgcggga ggagcagtac 1560

aacagcacgt accgtgtggt cagcgtcctc accgtcctgc accaggactg gctgaatggc 1620

aaggagtaca agtgcaaggt ctccaacaaa gccctcggcg cccccatcga gaaaaccatc 1680

tccaaagcca aagggcagcc ccgagaacca caggtgtaca ccctgccccc atgccgggat 1740

gagctgacca agaaccaggt cagcctgtgg tgcctggtca aaggcttcta tcccagcgac 1800

atcgccgtgg agtgggagag caatgggcag ccggagaaca actacaagac cacgcctccc 1860

gtgctggact ccgacggctc cttcttcctc tacagcaagc tcaccgtgga caagagcagg 1920

tggcagcagg ggaacgtctt ctcatgctcc gtgatgcatg aggctctgca caaccactac 1980

acgcagaaga gcctctccct gtctccgggt aaa 2013

<210> 231
<211> 449
<212> PRT
<213> Artificial Sequence

<220>
<223> anti-CD20(VH-CH1)-Fc(hole) P329G LALA

<400> 231

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Ala Phe Ser Tyr Ser
            20                  25                  30

Trp Ile Asn Trp Val Arg Gln Ala Pro Gly Gln Gly Leu Glu Trp Met
        35                  40                  45

Gly Arg Ile Phe Pro Gly Asp Gly Asp Thr Asp Tyr Asn Gly Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asn Val Phe Asp Gly Tyr Trp Leu Val Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Ala Ala Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Gly Ala Pro Ile Glu Lys
                325                 330                 335

Thr Ile Ser Lys Ala Lys Gly Gln Pro Arg Glu Pro Gln Val Cys Thr
            340                 345                 350

Leu Pro Pro Ser Arg Asp Glu Leu Thr Lys Asn Gln Val Ser Leu Ser
        355                 360                 365

Cys Ala Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Val Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Lys

<210>  232
<211>  1347
<212>  DNA
<213>  Artificial Sequence

<220>
<223>  anti-CD20(VH-CH1)-Fc(hole) P329G LALA

<400>  232
caggtgcaat tggtgcagtc tggcgctgaa gttaagaagc ctgggagttc agtgaaggtc      60

tcctgcaagg cttccggata cgccttcagc tattcttgga tcaattgggt gcggcaggcg     120

cctggacaag ggctcgagtg gatgggacgg atctttcccg gcgatgggga tactgactac     180

aatgggaaat tcaagggcag agtcacaatt accgccgaca aatccactag cacagcctat     240

atggagctga gcagcctgag atctgaggac acggccgtgt attactgtgc aagaaatgtc     300

tttgatggtt actggcttgt ttactggggc cagggaaccc tggtcaccgt ctcctcagct     360

agcaccaagg gcccatcggt cttccccctg gcaccctcct ccaagagcac ctctgggggc     420

acagcggccc tgggctgcct ggtcaaggac tacttccccg aaccggtgac ggtgtcgtgg     480

aactcaggcg ccctgaccag cggcgtgcac accttcccgg ctgtcctaca gtcctcagga     540

ctctactccc tcagcagcgt ggtgaccgtg ccctccagca gcttgggcac ccagacctac 600

atctgcaacg tgaatcacaa gcccagcaac accaaggtgg acaagaaagt tgagcccaaa 660

tcttgtgaca aaactcacac atgcccaccg tgcccagcac ctgaagctgc agggggaccg 720

tcagtcttcc tcttcccccc aaaacccaag gacaccctca tgatctcccg gacccctgag 780

gtcacatgcg tggtggtgga cgtgagccac gaagaccctg aggtcaagtt caactggtac 840

gtggacggcg tggaggtgca taatgccaag acaaagccgc gggaggagca gtacaacagc 900

acgtaccgtg tggtcagcgt cctcaccgtc ctgcaccagg actggctgaa tggcaaggag 960

tacaagtgca aggtctccaa caaagccctc ggcgccccca tcgagaaaac catctccaaa 1020

gccaaagggc agccccgaga accacaggtg tgcaccctgc ccccatcccg ggatgagctg 1080

accaagaacc aggtcagcct ctcgtgcgca gtcaaaggct tctatcccag cgacatcgcc 1140

gtggagtggg agagcaatgg gcagccggag aacaactaca agaccacgcc tcccgtgctg 1200

gactccgacg gctccttctt cctcgtgagc aagctcaccg tggacaagag caggtggcag 1260

caggggaacg tcttctcatg ctccgtgatg catgaggctc tgcacaacca ctacacgcag 1320

aagagcctct ccctgtctcc gggtaaa 1347

<210> 233
<211> 11
<212> PRT
<213> Artificial Sequence

<220>
<223> M4-3 ML2 HCDR1

<400> 233

Gly Gly Ser Ile Thr Ser Gly Tyr Tyr Trp Asn
1               5                   10

<210> 234
<211> 33
<212> DNA
<213> Artificial Sequence

<220>
<223> M4-3 ML2 HCDR1

<400> 234
ggcggcagca tcaccagcgg ctactactgg aac 33

<210> 235
<211> 16
<212> PRT
<213> Artificial Sequence

<220>
<223> M4-3 ML2 HCDR2

<400>  235

Tyr Ile Thr Tyr Asp Gly Ser Asn Asn Tyr Asn Pro Ser Leu Lys Ser
1               5                   10                  15


<210>  236
<211>  48
<212>  DNA
<213>  Artificial Sequence

<220>
<223>  M4-3 ML2 HCDR2

<400>  236
tacatcacct acgacggcag caacaactac aaccccagcc tgaagtcc                48


<210>  237
<211>  3
<212>  PRT
<213>  Artificial Sequence

<220>
<223>  M4-3 ML2 HCDR3

<400>  237

Phe Asp Tyr
1


<210>  238
<211>  9
<212>  DNA
<213>  Artificial Sequence

<220>
<223>  M4-3 ML2 HCDR3

<400>  238
ttcgactac                                                            9


<210>  239
<211>  112
<212>  PRT
<213>  Artificial Sequence

<220>
<223>  M4-3 ML2 VH

<400>  239

Gln Val Gln Leu Gln Glu Ser Gly Pro Gly Leu Val Lys Pro Ser Gln
1               5                   10                  15


Thr Leu Ser Leu Thr Cys Thr Val Ser Gly Gly Ser Ile Thr Ser Gly
            20                  25                  30

Tyr Tyr Trp Asn Trp Ile Arg Gln His Pro Gly Lys Gly Leu Glu Trp
        35                  40                  45

Ile Gly Tyr Ile Thr Tyr Asp Gly Ser Asn Asn Tyr Asn Pro Ser Leu
    50                  55                  60

Lys Ser Arg Val Thr Ile Ser Arg Asp Thr Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Asp Phe Asp Tyr Trp Gly Gln Gly Thr Leu Val Thr Val Ser Ser
            100                 105                 110

<210>  240
<211>  336
<212>  DNA
<213>  Artificial Sequence

<220>
<223>  M4-3 ML2 VH

<400>  240
caggtgcagc tgcaggaaag cggccctggc ctggtcaagc ccagccagac cctgagcctg      60

acctgcaccg tgtccggcgg cagcatcacc agcggctact actggaactg gatccggcag     120

caccccggca agggcctgga atggatcggc tacatcacct acgacggcag caacaactac     180

aaccccagcc tgaagtccag agtgaccatc agccgggaca ccagcaagaa ccagttcagc     240

ctgaagctgt ccagcgtgac agccgccgac accgccgtgt actactgcgc cgacttcgac     300

tactggggcc agggcaccct ggtcaccgtg tccagc                               336

<210>  241
<211>  11
<212>  PRT
<213>  Artificial Sequence

<220>
<223>  M4-3 ML2 LCDR1

<400>  241

Arg Ala Ser Gln Gly Ile Arg Asn Tyr Leu Asn
1               5                   10

<210>  242
<211>  33
<212>  DNA
<213>  Artificial Sequence

<220>
<223>  M4-3 ML2 LCDR1

<400>  242
cgggccagcc agggcatccg gaactacctg aac                                    33


<210>  243
<211>  7
<212>  PRT
<213>  Artificial Sequence

<220>
<223>  M4-3 ML2 LCDR2

<400>  243

Tyr Thr Ser Ser Leu His Ser
1               5


<210>  244
<211>  21
<212>  DNA
<213>  Artificial Sequence

<220>
<223>  M4-3 ML2 LCDR2

<400>  244
tacaccagca gcctgcacag c                                                 21


<210>  245
<211>  9
<212>  PRT
<213>  Artificial Sequence

<220>
<223>  M4-3 ML2 LCDR3

<400>  245

Gln Gln Tyr Ser Lys Leu Pro Trp Thr
1               5


<210>  246
<211>  27
<212>  DNA
<213>  Artificial Sequence

<220>
<223>  M4-3 ML2 LCDR3

<400>  246
cagcagtaca gcaagctgcc ctggacc                                           27


<210>  247
<211>  107
<212>  PRT
<213>  Artificial Sequence

<220>

<223>  M4-3 ML2 VL

<400>  247

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Arg Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Ser Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Lys Leu Pro Trp
                85                  90                  95

Thr Phe Gly Gln Gly Thr Lys Val Glu Ile Lys
            100                 105

<210>  248
<211>  321
<212>  DNA
<213>  Artificial Sequence

<220>
<223>  M4-3 ML2 VL

<400>  248
gacatccaga tgacccagag ccccagcagc ctgagcgcca gcgtgggcga cagagtgacc      60

atcacctgcc gggccagcca gggcatccgg aactacctga actggtatca gcagaagccc     120

ggcaaggccc ccaagctgct gatctactac accagcagcc tgcacagcgg cgtgcctagc     180

cggtttagcg gcagcggctc cggcaccgac ttcaccctga ccattagctc cctgcagccc     240

gaggacttcg ccacctacta ctgccagcag tacagcaagc tgccctggac cttcggccag     300

ggaacaaagg tggagatcaa g                                               321

<210>  249
<211>  5
<212>  PRT
<213>  Artificial Sequence

<220>
<223>  anti-CD3 HCDR1

<400>  249

Thr Tyr Ala Met Asn
1               5

<210>  250
<211>  15
<212>  DNA
<213>  Artificial Sequence

<220>
<223>  anti-CD3 HCDR1

<400>  250
acctacgcca tgaac                                                        15

<210>  251
<211>  19
<212>  PRT
<213>  Artificial Sequence

<220>
<223>  anti-CD3 HCDR2

<400>  251

Arg Ile Arg Ser Lys Tyr Asn Asn Tyr Ala Thr Tyr Tyr Ala Asp Ser
1               5                   10                  15

Val Lys Asp

<210>  252
<211>  57
<212>  DNA
<213>  Artificial Sequence

<220>
<223>  anti-CD3 HCDR2

<400>  252
cggatcagaa gcaagtacaa caattacgcc acctactacg ccgacagcgt gaaggac          57

<210>  253
<211>  14
<212>  PRT
<213>  Artificial Sequence

<220>
<223>  anti-CD3 HCDR3

<400>  253

His Gly Asn Phe Gly Asn Ser Tyr Val Ser Trp Phe Ala Tyr
1               5                   10

<210>  254
<211>  42

<212>  DNA
<213>  Artificial Sequence

<220>
<223>  anti-CD3 HCDR3

<400>  254
cacggcaact tcggcaacag ctatgtgtct tggtttgcct ac                          42


<210>  255
<211>  125
<212>  PRT
<213>  Artificial Sequence

<220>
<223>  anti-CD3 VH

<400>  255

Glu Val Lys Leu Leu Glu Ser Gly Gly Gly Leu Val Gln Pro Lys Gly
1               5                   10                  15

Ser Leu Lys Leu Ser Cys Ala Ala Ser Gly Phe Thr Phe Asn Thr Tyr
            20                  25                  30

Ala Met Asn Trp Val Arg Gln Ala Pro Gly Lys Gly Leu Glu Trp Val
        35                  40                  45

Ala Arg Ile Arg Ser Lys Tyr Asn Asn Tyr Ala Thr Tyr Tyr Ala Asp
    50                  55                  60

Ser Val Lys Asp Arg Phe Thr Ile Ser Arg Asp Asp Ser Gln Ser Ile
65                  70                  75                  80

Leu Tyr Leu Gln Met Asn Asn Leu Lys Thr Glu Asp Thr Ala Met Tyr
                85                  90                  95

Tyr Cys Val Arg His Gly Asn Phe Gly Asn Ser Tyr Val Ser Trp Phe
            100                 105                 110

Ala Tyr Trp Gly Gln Gly Thr Leu Val Thr Val Ser Ala
        115                 120                 125


<210>  256
<211>  375
<212>  DNA
<213>  Artificial Sequence

<220>
<223>  anti-CD3 VH

<400>  256
gaagtgcagc tggtggaaag cggcggaggc ctggtgcagc ctaagggctc tctgaagctg       60

agctgtgccg ccagcggctt caccttcaac acctacgcca tgaactgggt gcgccaggcc 120

cctggcaaag gcctggaatg ggtggcccgg atcagaagca agtacaacaa ttacgccacc 180

tactacgccg acagcgtgaa ggaccggttc accatcagcc gggacgacag ccagagcatc 240

ctgtacctgc agatgaacaa cctgaaaacc gaggacaccg ccatgtacta ctgcgtgcgg 300

cacggcaact tcggcaacag ctatgtgtct tggtttgcct actggggcca gggcaccctc 360

gtgacagtgt ctgct 375

<210> 257
<211> 14
<212> PRT
<213> Artificial Sequence

<220>
<223> anti-CD3 LCDR1

<400> 257

Arg Ser Ser Thr Gly Ala Val Thr Thr Ser Asn Tyr Ala Asn
1               5                   10

<210> 258
<211> 42
<212> DNA
<213> Artificial Sequence

<220>
<223> anti-CD3 LCDR1

<400> 258
agatctagca caggcgccgt gaccaccagc aactacgcca ac 42

<210> 259
<211> 7
<212> PRT
<213> Artificial Sequence

<220>
<223> anti-CD3 LCDR2

<400> 259

Gly Thr Asn Lys Arg Ala Pro
1               5

<210> 260
<211> 21
<212> DNA
<213> Artificial Sequence

<220>
<223> anti-CD3 LCDR2

<400> 260
ggcaccaaca aaagggctcc a 21

<210>  261
<211>  9
<212>  PRT
<213>  Artificial Sequence

<220>
<223>  anti-CD3 LCDR3

<400>  261

Ala Leu Trp Tyr Ser Asn Leu Trp Val
1               5


<210>  262
<211>  27
<212>  DNA
<213>  Artificial Sequence

<220>
<223>  anti-CD3 LCDR3

<400>  262
gccctgtggt acagcaacct gtgggtg                                          27


<210>  263
<211>  109
<212>  PRT
<213>  Artificial Sequence

<220>
<223>  anti-CD3 VL

<400>  263

Gln Ala Val Val Thr Gln Glu Ser Ala Leu Thr Thr Ser Pro Gly Glu
1               5                   10                  15

Thr Val Thr Leu Thr Cys Arg Ser Ser Thr Gly Ala Val Thr Thr Ser
            20                  25                  30

Asn Tyr Ala Asn Trp Val Gln Glu Lys Pro Asp His Leu Phe Thr Gly
        35                  40                  45

Leu Ile Gly Gly Thr Asn Lys Arg Ala Pro Gly Val Pro Ala Arg Phe
    50                  55                  60

Ser Gly Ser Leu Ile Gly Asp Lys Ala Ala Leu Thr Ile Thr Gly Ala
65                  70                  75                  80

Gln Thr Glu Asp Glu Ala Ile Tyr Phe Cys Ala Leu Trp Tyr Ser Asn
                85                  90                  95

Leu Trp Val Phe Gly Gly Gly Thr Lys Leu Thr Val Leu

            100                 105

<210> 264
<211> 327
<212> DNA
<213> Artificial Sequence

<220>
<223> anti-CD3 VL

<400> 264
caggccgtcg tgacccagga aagcgccctg acaacaagcc ctggcgagac agtgaccctg 60

acctgcagat ctagcacagg cgccgtgacc accagcaact acgccaactg ggtgcaggaa 120

aagcccgacc acctgttcac cggcctgatc ggcggcacca acaaaagggc tccaggcgtg 180

ccagccagat tcagcggcag cctgattggc gataaggccg ccctgaccat cactggcgcc 240

cagacagagg acgaggccat ctacttttgc gccctgtggt acagcaacct gtgggtgttc 300

ggcggaggca ccaagctgac agtgctg 327

<210> 265
<211> 207
<212> PRT
<213> Homo sapiens

<400> 265

Met Gln Ser Gly Thr His Trp Arg Val Leu Gly Leu Cys Leu Leu Ser
1               5                   10                  15

Val Gly Val Trp Gly Gln Asp Gly Asn Glu Glu Met Gly Gly Ile Thr
            20                  25                  30

Gln Thr Pro Tyr Lys Val Ser Ile Ser Gly Thr Thr Val Ile Leu Thr
        35                  40                  45

Cys Pro Gln Tyr Pro Gly Ser Glu Ile Leu Trp Gln His Asn Asp Lys
    50                  55                  60

Asn Ile Gly Gly Asp Glu Asp Asp Lys Asn Ile Gly Ser Asp Glu Asp
65                  70                  75                  80

His Leu Ser Leu Lys Glu Phe Ser Glu Leu Glu Gln Ser Gly Tyr Tyr
                85                  90                  95

Val Cys Tyr Pro Arg Gly Ser Lys Pro Glu Asp Ala Asn Phe Tyr Leu
            100                 105                 110

Tyr Leu Arg Ala Arg Val Cys Glu Asn Cys Met Glu Met Asp Val Met
        115                 120                 125

Ser Val Ala Thr Ile Val Ile Val Asp Ile Cys Ile Thr Gly Gly Leu
    130                 135                 140

Leu Leu Leu Val Tyr Tyr Trp Ser Lys Asn Arg Lys Ala Lys Ala Lys
145                 150                 155                 160

Pro Val Thr Arg Gly Ala Gly Ala Gly Gly Arg Gln Arg Gly Gln Asn
                165                 170                 175

Lys Glu Arg Pro Pro Pro Val Pro Asn Pro Asp Tyr Glu Pro Ile Arg
            180                 185                 190

Lys Gly Gln Arg Asp Leu Tyr Ser Gly Leu Asn Gln Arg Arg Ile
        195                 200                 205

<210>  266
<211>  198
<212>  PRT
<213>  Macaca fascicularis

<400>  266

Met Gln Ser Gly Thr Arg Trp Arg Val Leu Gly Leu Cys Leu Leu Ser
1               5                   10                  15

Ile Gly Val Trp Gly Gln Asp Gly Asn Glu Glu Met Gly Ser Ile Thr
            20                  25                  30

Gln Thr Pro Tyr Gln Val Ser Ile Ser Gly Thr Thr Val Ile Leu Thr
        35                  40                  45

Cys Ser Gln His Leu Gly Ser Glu Ala Gln Trp Gln His Asn Gly Lys
    50                  55                  60

Asn Lys Glu Asp Ser Gly Asp Arg Leu Phe Leu Pro Glu Phe Ser Glu
65                  70                  75                  80

Met Glu Gln Ser Gly Tyr Tyr Val Cys Tyr Pro Arg Gly Ser Asn Pro
                85                  90                  95

Glu Asp Ala Ser His His Leu Tyr Leu Lys Ala Arg Val Cys Glu Asn
            100                 105                 110

Cys Met Glu Met Asp Val Met Ala Val Ala Thr Ile Val Ile Val Asp
        115                 120                 125

Ile Cys Ile Thr Leu Gly Leu Leu Leu Leu Val Tyr Tyr Trp Ser Lys
    130                 135                 140

Asn Arg Lys Ala Lys Ala Lys Pro Val Thr Arg Gly Ala Gly Ala Gly
145                 150                 155                 160

Gly Arg Gln Arg Gly Gln Asn Lys Glu Arg Pro Pro Pro Val Pro Asn
                165                 170                 175

Pro Asp Tyr Glu Pro Ile Arg Lys Gly Gln Gln Asp Leu Tyr Ser Gly
            180                 185                 190

Leu Asn Gln Arg Arg Ile
        195

## Description

### Field of the Invention

The present invention generally relates to bispecific antigen binding molecules for activating T cells. In addition, the present invention relates to polynucleotides encoding such bispecific antigen binding molecules, and vectors and host cells comprising such polynucleotides. The invention further relates to methods for producing the bispecific antigen binding molecules of the invention, and to methods of using these bispecific antigen binding molecules in the treatment of disease.

### Background

The selective destruction of an individual cell or a specific cell type is often desirable in a variety of clinical settings. For example, it is a primary goal of cancer therapy to specifically destroy tumor cells, while leaving healthy cells and tissues intact and undamaged.

An attractive way of achieving this is by inducing an immune response against the tumor, to make immune effector cells such as natural killer (NK) cells or cytotoxic T lymphocytes (CTLs) attack and destroy tumor cells. CTLs constitute the most potent effector cells of the immune system, however they cannot be activated by the effector mechanism mediated by the Fc domain of conventional therapeutic antibodies.

In this regard, bispecific antibodies designed to bind with one "arm" to a surface antigen on target cells, and with the second "arm" to an activating, invariant component of the T cell receptor (TCR) complex, have become of interest in recent years. The simultaneous binding of such an antibody to both of its targets will force a temporary interaction between target cell and T cell, causing activation of any cytotoxic T cell and subsequent lysis of the target cell. Hence, the immune response is re-directed to the target cells and is independent of peptide antigen presentation by the target cell or the specificity of the T cell as would be relevant for normal MHC-restricted activation of CTLs. In this context it is crucial that CTLs are only activated when a target cell is presenting the bispecific antibody to them, i.e. the immunological synapse is mimicked. Particularly desirable are bispecific antibodies that do not require lymphocyte preconditioning or co-stimulation in order to elicit efficient lysis of target cells.

Several bispecific antibody formats have been developed and their suitability for T cell mediated immunotherapy investigated. Out of these, the so-called BiTE (bispecific T cell engager) molecules have been very well characterized and already shown some promise in the clinic (reviewed in Nagorsen and Bäuerle, Exp Cell Res 317, 1255-1260 (2011)). BiTEs are tandem scFv molecules wherein two scFv molecules are fused by a flexible linker. Further bispecific formats being evaluated for T cell engagement include diabodies (Holliger et al., Prot Eng 9, 299-305 (1996)) and derivatives thereof, such as tandem diabodies (Kipriyanov et al., J Mol Biol 293, 41-66 (1999)). A more recent development are the so-called DART (dual affinity retargeting) molecules, which are based on the diabody format but feature a C-terminal disulfide bridge for additional stabilization (Moore et al., Blood 117, 4542-51 (2011)). The so-called triomabs, which are whole hybrid mouse/rat IgG molecules and also currently being evaluated in clinical trials, represent a larger sized format (reviewed in Seimetz et al., Cancer Treat Rev 36, 458-467 (2010)).

The variety of formats that are being developed shows the great potential attributed to T cell re-direction and activation in immunotherapy. The task of generating bispecific antibodies suitable therefor is, however, by no means trivial, but involves a number of challenges that have to be met related to efficacy, toxicity, applicability and produceability of the antibodies.

Small constructs such as, for example, BiTE molecules - while being able to efficiently crosslink effector and target cells - have a very short serum half life requiring them to be administered to patients by continuous infusion. IgG-like formats on the other hand - while having the great benefit of a long half life - suffer from toxicity associated with the native effector functions inherent to IgG molecules. Their immunogenic potential constitutes another unfavorable feature of IgG-like bispecific antibodies, especially non-human formats, for successful therapeutic development. Finally, a major challenge in the general development of bispecific antibodies has been the production of bispecific antibody constructs at a clinically sufficient quantity and purity, due to the mispairing of antibody heavy and light chains of different specificities upon co-expression, which decreases the yield of the correctly assembled construct and results in a number of non-functional side products from which the desired bispecific antibody may be difficult to separate.

Given the difficulties and disadvantages associated with currently available bispecific antibodies for T cell mediated immunotherapy, there remains a need for novel, improved formats of such molecules. The present invention provides bispecific antigen binding molecules designed for T cell activation and re-direction that combine good efficacy and produceability with low toxicity and favorable pharmacokinetic properties.

### Summary of the Invention

In a first aspect the present invention provides a T cell activating bispecific antigen binding molecule comprising a first and a second antigen binding moiety, one of which is a Fab molecule capable of specific binding to an activating T cell antigen and the other one of which is a Fab molecule capable of specific binding to a target cell antigen, and an Fc domain composed of a first and a second subunit capable of stable association; wherein the first antigen binding moiety is (a) a single chain Fab molecule wherein the Fab light chain and the Fab heavy chain are connected by a peptide linker, or (b) a crossover Fab molecule wherein either the variable or the constant regions of the Fab light chain and the Fab heavy chain are exchanged.

In a particular embodiment, not more than one antigen binding moiety capable of specific binding to an activating T cell antigen is present in the T cell activating bispecific antigen binding molecule (i.e. the T cell activating bispecific antigen binding molecule provides monovalent binding to the activating T cell antigen). In particular embodiments, the first antigen binding moiety is a crossover Fab molecule. In even more particular embodiments, the first antigen binding moiety is a crossover Fab molecule wherein the constant regions of the Fab light chain and the Fab heavy chain are exchanged.

In some embodiments, the first and the second antigen binding moiety of the T cell activating bispecific antigen binding molecule are fused to each other, optionally via a peptide linker. In one such embodiment, the second antigen binding moiety is fused at the C-terminus of the Fab heavy chain to the N-terminus of the Fab heavy chain of the first antigen binding moiety. In another such embodiment, the first antigen binding moiety is fused at the C-terminus of the Fab heavy chain to the N-terminus of the Fab heavy chain of the second antigen binding moiety. In yet another such embodiment, the second antigen binding moiety is fused at the C-terminus of the Fab light chain to the N-terminus of the Fab light chain of the first antigen binding moiety. In embodiments wherein the first antigen binding moiety is a crossover Fab molecule and wherein either (i) the second antigen binding moiety is fused at the C-terminus of the Fab heavy chain to the N-terminus of the Fab heavy chain of the first antigen binding moiety or (ii) the first antigen binding moiety is fused at the C-terminus of the Fab heavy chain to the N-terminus of the Fab heavy chain of the second antigen binding moiety, additionally the Fab light chain of the first antigen binding moiety and the Fab light chain of the second antigen binding moiety may be fused to each other, optionally via a peptide linker.

In one embodiment, the second antigen binding moiety of the T cell activating bispecific antigen binding molecule is fused at the C-terminus of the Fab heavy chain to the N-terminus of the first or the second subunit of the Fc domain. In another embodiment, the first antigen binding moiety is fused at the C-terminus of the Fab heavy chain to the N-terminus of the first or second subunit of the Fc domain.

In one embodiment, the first and the second antigen binding moiety of the T cell activating bispecific antigen binding molecule are each fused at the C-terminus of the Fab heavy chain to the N-terminus of one of the subunits of the Fc domain.

In certain embodiments, the T cell activating bispecific antigen binding molecule comprises a third antigen binding moiety which is a Fab molecule capable of specific binding to a target cell antigen. In one such embodiment, the third antigen binding moiety is fused at the C-terminus of the Fab heavy chain to the N-terminus of the first or second subunit of the Fc domain. In a particular embodiment, the second and the third antigen binding moiety of the T cell activating antigen binding molecule are each fused at the C-terminus of the Fab heavy chain to the N-terminus of one of the subunits of the Fc domain, and the first antigen binding moiety is fused at the C-terminus of the Fab heavy chain to the N-terminus of the Fab heavy chain of the second antigen binding moiety. In another particular embodiment, the first and the third antigen binding moiety of the T cell activating antigen binding molecule are each fused at the C-terminus of the Fab heavy chain to the N-terminus of one of the subunits of the Fc domain, and the second antigen binding moiety is fused at the C-terminus of the Fab heavy chain to the N-terminus of the Fab heavy chain of the first antigen binding moiety. The components of the T cell activating bispecific antigen binding molecule may be fused directly or through suitable peptide linkers. In one embodiment the second and the third antigen binding moiety and the Fc domain are part of an immunoglobulin molecule. In a particular embodiment the immunoglobulin molecule is an IgG class immunoglobulin. In an even more particular embodiment the immunoglobulin is an IgG₁ subclass immunoglobulin. In another embodiment, the immunoglobulin is an IgG₄ subclass immunoglobulin.

In a particular embodiment, the Fc domain is an IgG Fc domain. In a specific embodiment, the Fc domain is an IgG₁ Fc domain. In another specific embodiment, the Fc domain is an IgG₄ Fc domain. In an even more specific embodiment, the Fc domain is an IgG₄ Fc domain comprising the amino acid substitution S228P (EU numbering). In particular embodiments the Fc domain is a human Fc domain.

In particular embodiments the Fc domain comprises a modification promoting the association of the first and the second Fc domain subunit. In a specific such embodiment, an amino acid residue in the CH3 domain of the first subunit of the Fc domain is replaced with an amino acid residue having a larger side chain volume, thereby generating a protuberance within the CH3 domain of the first subunit which is positionable in a cavity within the CH3 domain of the second subunit, and an amino acid residue in the CH3 domain of the second subunit of the Fc domain is replaced with an amino acid residue having a smaller side chain volume, thereby generating a cavity within the CH3 domain of the second subunit within which the protuberance within the CH3 domain of the first subunit is positionable.

In a particular embodiment the Fc domain exhibits reduced binding affinity to an Fc receptor and/or reduced effector function, as compared to a native IgG₁ Fc domain. In certain embodiments the Fc domain is engineered to have reduced binding affinity to an Fc receptor and/or reduced effector function, as compared to a non-engineered Fc domain. In one embodiment, the Fc domain comprises one or more amino acid substitution that reduces binding to an Fc receptor and/or effector function. In one embodiment, the one or more amino acid substitution in the Fc domain that reduces binding to an Fc receptor and/or effector function is at one or more position selected from the group of L234, L235, and P329 (EU numbering). In particular embodiments, each subunit of the Fc domain comprises three amino acid substitutions that reduce binding to an Fc receptor and/or effector function wherein said amino acid substitutions are L234A, L235A and P329G. In one such embodiment, the Fc domain is an IgG₁ Fc domain, particularly a human IgG₁ Fc domain. In other embodiments, each subunit of the Fc domain comprises two amino acid substitutions that reduce binding to an Fc receptor and/or effector function wherein said amino acid substitutions are L235E and P329G. In one such embodiment, the Fc domain is an IgG₄ Fc domain, particularly a human IgG₄ Fc domain.

In one embodiment the Fc receptor is an Fcγ receptor. In one embodiment the Fc receptor is a human Fc receptor. In one embodiment, the Fc receptor is an activating Fc receptor. In a specific embodiment, the Fc receptor is human FcyRIIa, FcyRI, and/or FcyRIIIa. In one embodiment, the effector function is antibody-dependent cell-mediated cytotoxicity (ADCC).

In a particular embodiment, the activating T cell antigen that the bispecific antigen binding molecule is capable of binding is CD3. In other embodiments, the target cell antigen that the bispecific antigen binding molecule is capable of binding is a tumor cell antigen. In one embodiment, the target cell antigen is selected from the group consisting of: Melanoma-associated Chondroitin Sulfate Proteoglycan (MCSP), Epidermal Growth Factor Receptor (EGFR), Carcinoembryonic Antigen (CEA), Fibroblast Activation Protein (FAP), CD19, CD20 and CD33.

According to another aspect of the invention there is provided an isolated polynucleotide encoding a T cell activating bispecific antigen binding molecule of the invention or a fragment thereof. The invention also encompasses polypeptides encoded by the polynucleotides of the invention. The invention further provides an expression vector comprising the isolated polynucleotide of the invention, and a host cell comprising the isolated polynucleotide or the expression vector of the invention. In some embodiments the host cell is a eukaryotic cell, particularly a mammalian cell.

In another aspect is provided a method of producing the T cell activating bispecific antigen binding molecule of the invention, comprising the steps of a) culturing the host cell of the invention under conditions suitable for the expression of the T cell activating bispecific antigen binding molecule and b) recovering the T cell activating bispecific antigen binding molecule. The invention also encompasses a T cell activating bispecific antigen binding molecule produced by the method of the invention.

The invention further provides a pharmaceutical composition comprising the T cell activating bispecific antigen binding molecule of the invention and a pharmaceutically acceptable carrier. Also encompassed by the invention are methods of using the T cell activating bispecific antigen binding molecule and pharmaceutical composition of the invention. In one aspect the invention provides a T cell activating bispecific antigen binding molecule or a pharmaceutical composition of the invention for use as a medicament. In one aspect is provided a T cell activating bispecific antigen binding molecule or a pharmaceutical composition according to the invention for use in the treatment of a disease in an individual in need thereof. In a specific embodiment the disease is cancer.

Also provided is the use of a T cell activating bispecific antigen binding molecule of the invention for the manufacture of a medicament for the treatment of a disease in an individual in need thereof; as well as a method of treating a disease in an individual, comprising administering to said individual a therapeutically effective amount of a composition comprising the T cell activating bispecific antigen binding molecule according to the invention in a pharmaceutically acceptable form. In a specific embodiment the disease is cancer. In any of the above embodiments the individual preferably is a mammal, particularly a human.

The invention also provides a method for inducing lysis of a target cell, particularly a tumor cell, comprising contacting a target cell with a T cell activating bispecific antigen binding molecule of the invention in the presence of a T cell, particularly a cytotoxic T cell.

### Brief Description of the Drawings

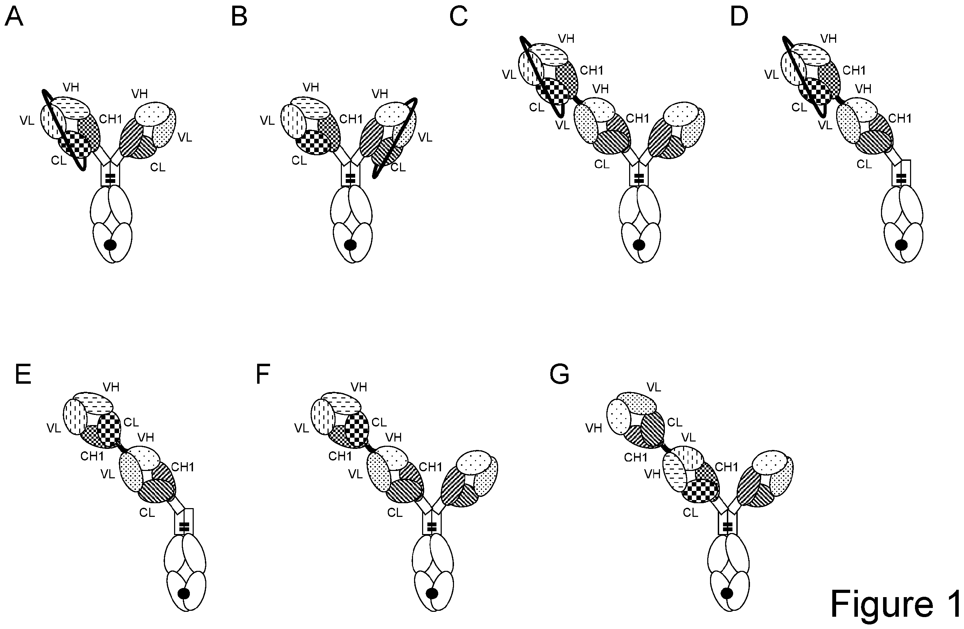

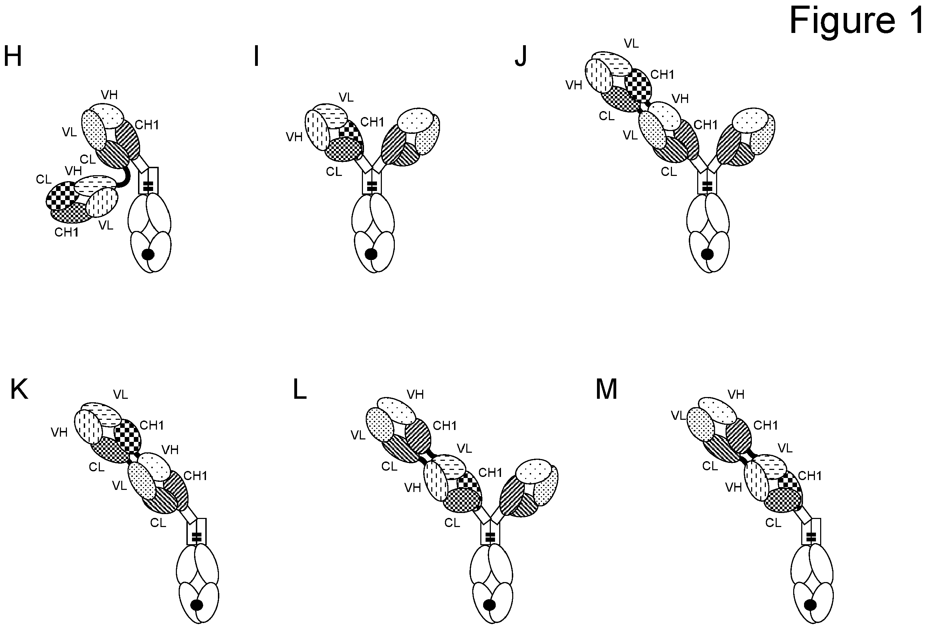

FIGURE 1. Exemplary configurations of the T cell activating bispecific antigen binding molecules of the invention. Illustration of (A) the "1+1 IgG scFab, one armed", and (B) the "1+1 IgG scFab, one armed inverted" molecule. In the "1+1 IgG scFab, one armed" molecule the light chain of the T cell targeting Fab is fused to the heavy chain by a linker, while the "1+1 IgG scFab, one armed inverted" molecule has the linker in the tumor targeting Fab. (C) Illustration of the "2+1 IgG scFab" molecule. (D) Illustration of the "1+1 IgG scFab" molecule. (E) Illustration of the "1+1 IgG Crossfab" molecule. (F) Illustration of the "2+1 IgG Crossfab" molecule. (G) Illustration of the "2+1 IgG Crossfab" molecule with alternative order of Crossfab and Fab components ("inverted"). (H) Illustration of the "1+1 IgG Crossfab light chain (LC) fusion" molecule. (I) Illustration of the "1+1 CrossMab" molecule. (J) Illustration of the "2+1 IgG Crossfab, linked light chain" molecule. (K) Illustration of the "1+1 IgG Crossfab, linked light chain" molecule. (L) Illustration of the "2+1 IgG Crossfab, inverted, linked light chain" molecule. (M) Illustration of the "1+1 IgG Crossfab, inverted, linked light chain" molecule. Black dot: optional modification in the Fc domain promoting heterodimerization.

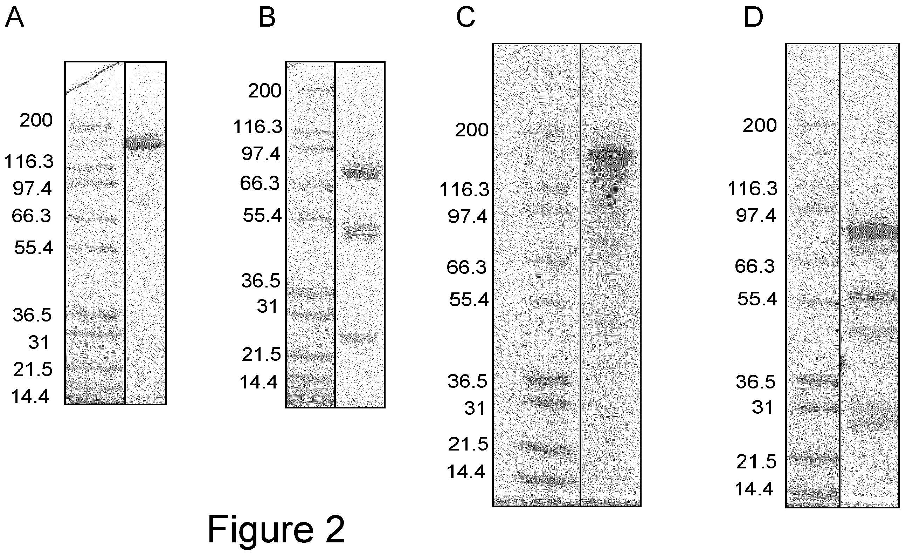

FIGURE 2. SDS PAGE (4-12% Bis/Tris, NuPage Invitrogen, Coomassie-stained) of "1+1 IgG scFab, one armed" (anti-MCSP/anti-huCD3) (see SEQ ID NOs 1, 3, 5), non reduced (A) and reduced (B), and of "1+1 IgG scFab, one armed inverted" (anti-MCSP/anti-huCD3) (see SEQ ID NOs 7, 9, 11), non reduced (C) and reduced (D).

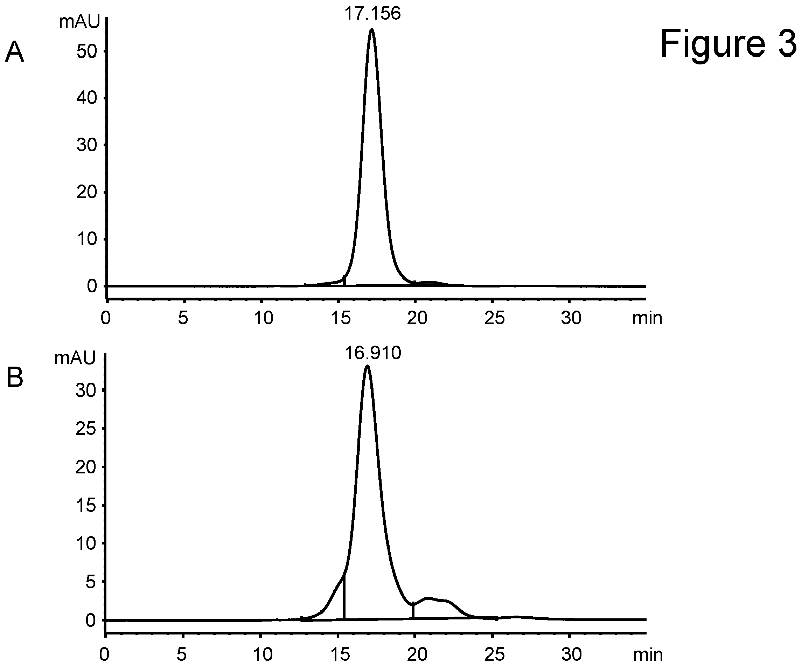

FIGURE 3. Analytical size exclusion chromatography (Superdex 200 10/300 GL GE Healthcare; 2 mM MOPS pH 7.3, 150 mM NaCl, 0.02% (w/v) NaCl; 50 µg sample injected) of "1+1 IgG scFab, one armed" (anti-MCSP/anti-huCD3) (see SEQ ID NOs 1, 3, 5) (A) and "1+1 IgG scFab, one armed inverted" (anti-MCSP/anti-huCD3) (see SEQ ID NOs 7, 9, 11) (B).

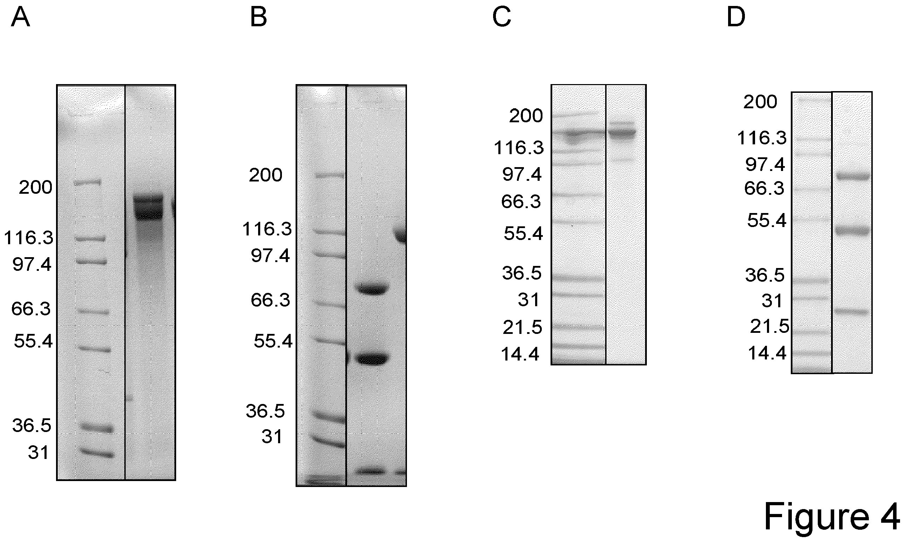

FIGURE 4. SDS PAGE (4-12% Bis/Tris, NuPage Invitrogen, Coomassie-stained) of "1+1 IgG scFab, one armed" (anti-EGFR/anti-huCD3) (see SEQ ID NOs 43, 45, 57), non reduced (A) and reduced (B), and of "1+1 IgG scFab, one armed inverted" (anti-EGFR/anti-huCD3) (see SEQ ID NOs 11, 49, 51), non reduced (C) and reduced (D).

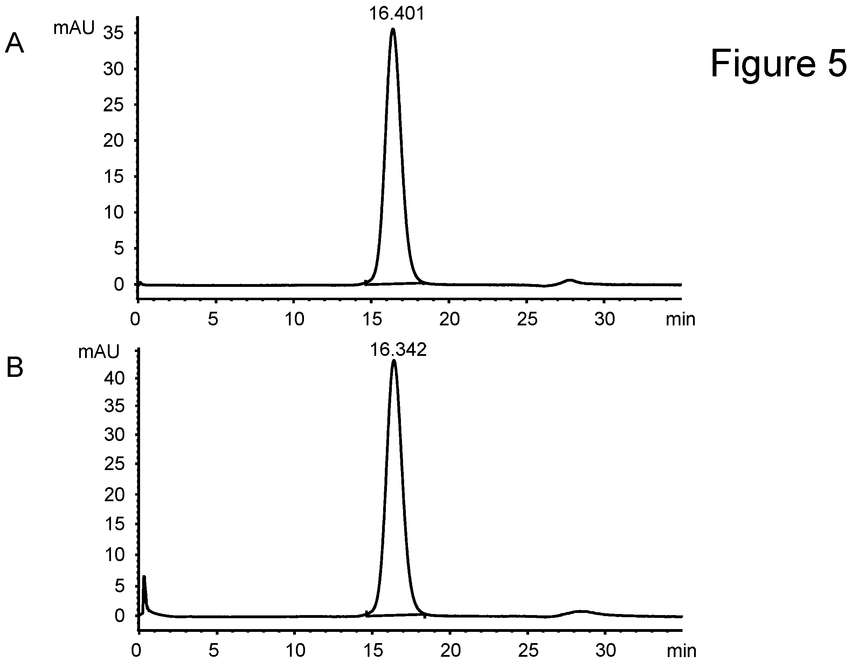

FIGURE 5. Analytical size exclusion chromatography (Superdex 200 10/300 GL GE Healthcare; 2 mM MOPS pH 7.3, 150 mM NaCl, 0.02% (w/v) NaCl; 50 µg sample injected) of "1+1 IgG scFab, one armed" (anti-EGFR/anti-huCD3) (see SEQ ID NOs 43, 45, 47) (A) and "1+1 IgG scFab, one armed inverted" (anti-EGFR/anti-huCD3) (see SEQ ID NOs 11, 49, 51) (B).

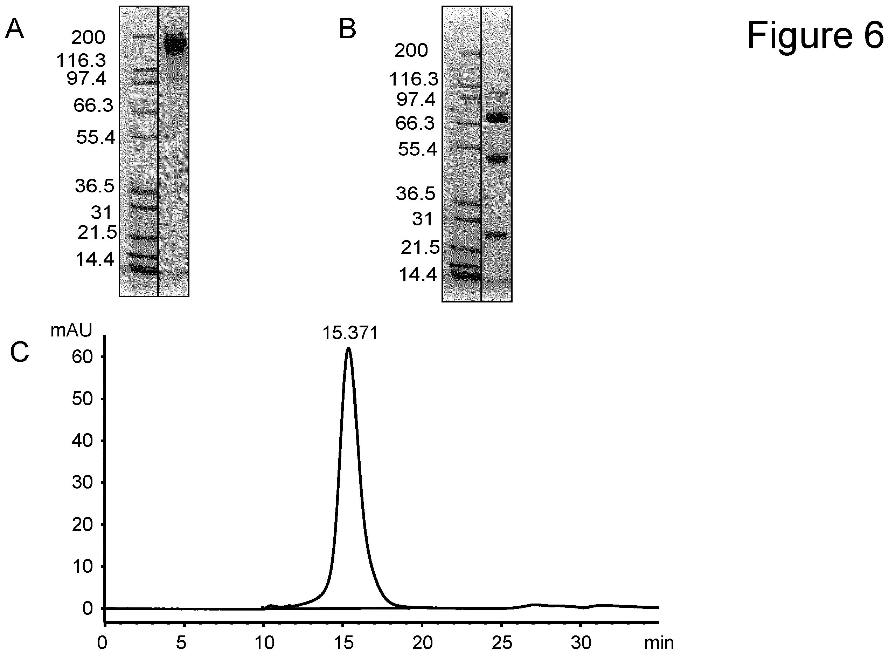

FIGURE 6. (A, B) SDS PAGE (4-12% Bis/Tris, NuPage Invitrogen, Coomassie-stained) of "1+1 IgG scFab, one armed inverted" (anti-FAP/anti-huCD3) (see SEQ ID NOs 11, 51, 55), non reduced (A) and reduced (B). (C) Analytical size exclusion chromatography (Superdex 200 10/300 GL GE Healthcare; 2 mM MOPS pH 7.3, 150 mM NaCl, 0.02% (w/v) NaCl; 50 µg sample injected) of "1+1 IgG scFab, one armed inverted" (anti-FAP/anti-huCD3).

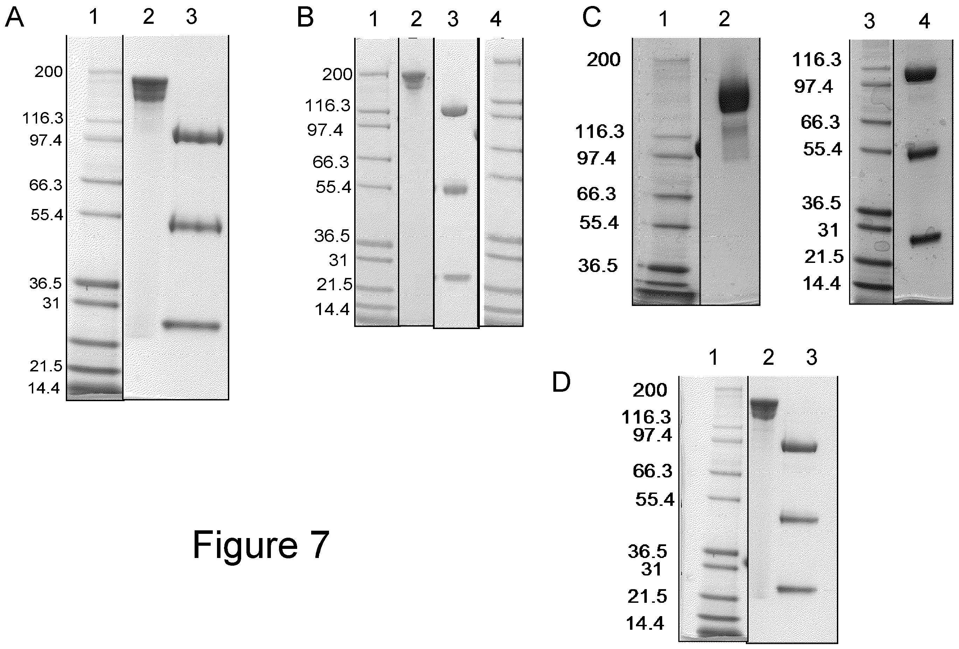

FIGURE 7. SDS PAGE (4-12% Bis/Tris, NuPage Invitrogen, Coomassie-stained) of (A) "2+1 IgG scFab, P329G LALA" (anti-MCSP/anti-huCD3) (see SEQ ID NOs 5, 21, 23), non reduced (lane 2) and reduced (lane 3); of (B) "2+1 IgG scFab, LALA" (anti-MCSP/anti-huCD3) (see SEQ ID NOs 5, 17, 19), non reduced (lane 2) and reduced (lane 3); of (C) "2+1 IgG scFab, wt" (anti-MCSP/anti-huCD3) (see SEQ ID NOs 5, 13, 15), non reduced (lane 2) and reduced (lane 3); and of (D) "2+1 IgG scFab, P329G LALA N297D" (anti-MCSP/anti-huCD3) (see SEQ ID NOs 5, 25, 27), non reduced (lane 2) and reduced (lane 3).

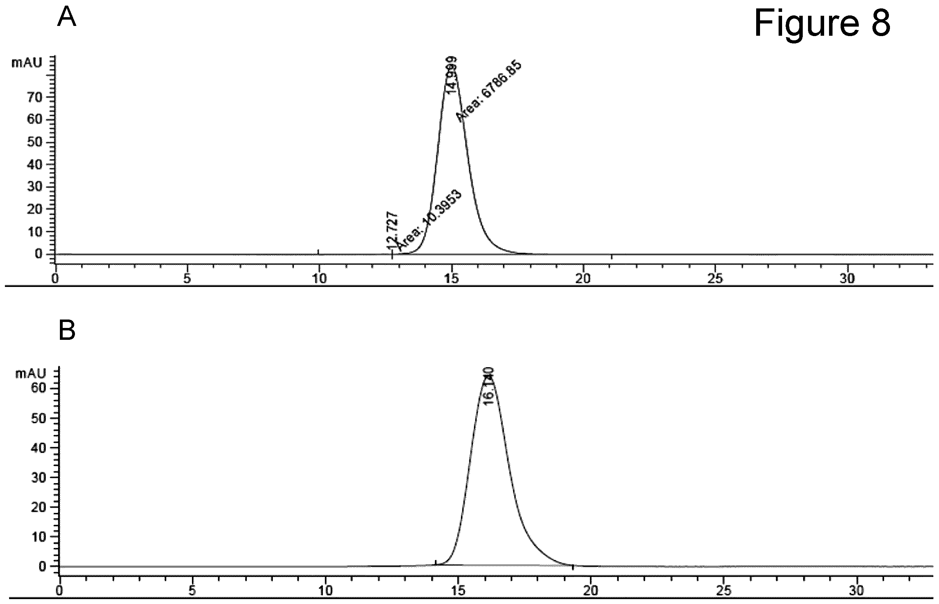

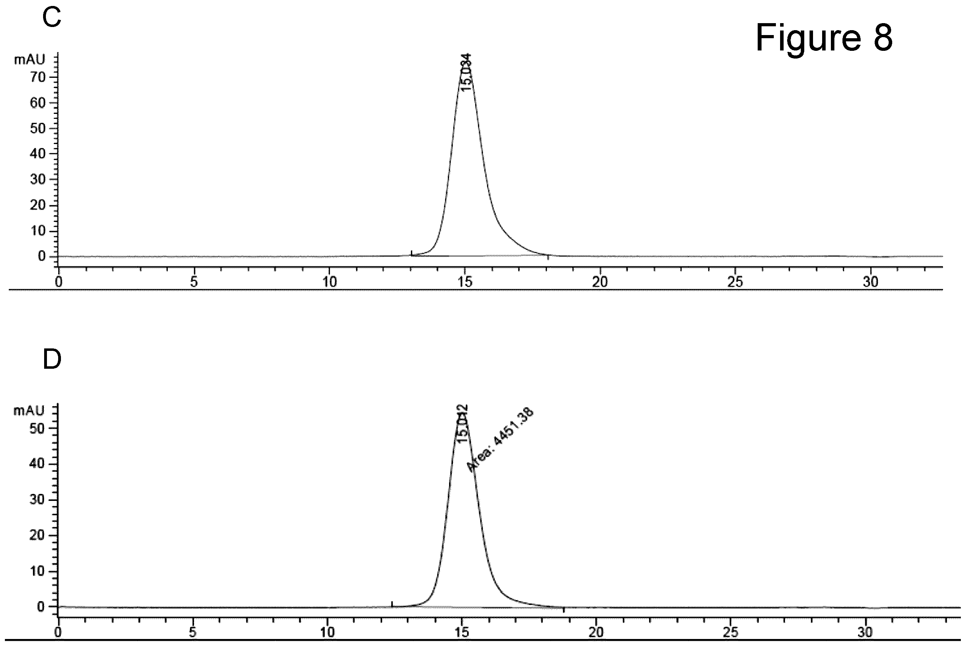

FIGURE 8. Analytical size exclusion chromatography (Superdex 200 10/300 GL GE Healthcare; 2 mM MOPS pH 7.3, 150 mM NaCl, 0.02% (w/v) NaCl; 50 µg sample injected) of (A) "2+1 IgG scFab, P329G LALA" (anti-MCSP/anti-huCD3) (see SEQ ID NOs 5, 21, 23); of (B) "2+1 IgG scFab, LALA" (anti-MCSP/anti-huCD3) (see SEQ ID NOs 5, 17, 19); of (C) "2+1 IgG scFab, wt" (anti-MCSP/anti-huCD3) (see SEQ ID NOs 5, 13, 15); and of (D) "2+1 IgG scFab, P329G LALA N297D" (anti-MCSP/anti-huCD3) (see SEQ ID NOs 5, 25, 27).

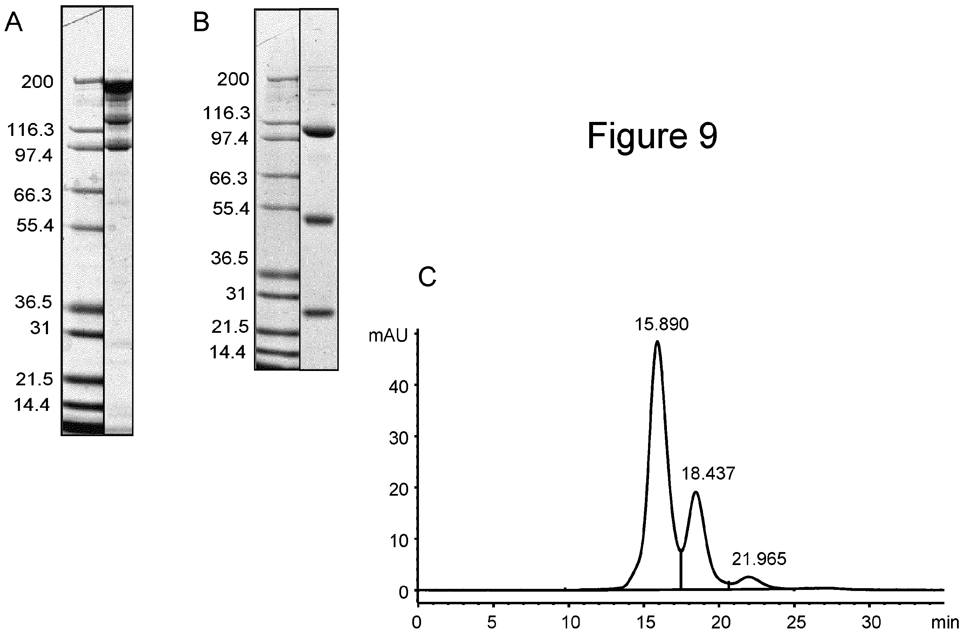

FIGURE 9. (A, B) SDS PAGE (4-12% Bis/Tris, NuPage Invitrogen, Coomassie-stained) of "2+1 IgG scFab, P329G LALA" (anti-EGFR/anti-huCD3) (see SEQ ID NOs 45, 47, 53), non reduced (A) and reduced (B). (C) Analytical size exclusion chromatography (Superdex 200 10/300 GL GE Healthcare; 2 mM MOPS pH 7.3, 150 mM NaCl, 0.02% (w/v) NaCl; 50 µg sample injected) of "2+1 IgG scFab, P329G LALA" (anti-EGFR/anti-huCD3).

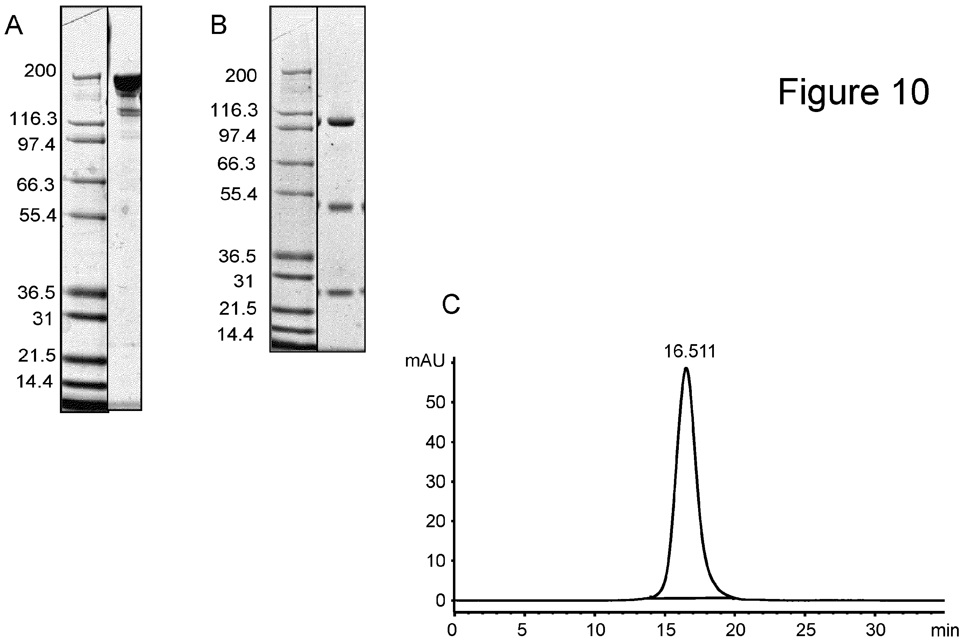

FIGURE 10. (A, B) SDS PAGE (4-12% Bis/Tris, NuPage Invitrogen, Coomassie-stained) of "2+1 IgG scFab, P329G LALA" (anti-FAP/anti-huCD3) (see SEQ ID NOs 57, 59, 61), non reduced (A) and reduced (B). (C) Analytical size exclusion chromatography (Superdex 200 10/300 GL GE Healthcare; 2 mM MOPS pH 7.3, 150 mM NaCl, 0.02% (w/v) NaCl; 50 µg sample injected) of "2+1 IgG scFab, P329G LALA" (anti-FAP/anti-huCD3).

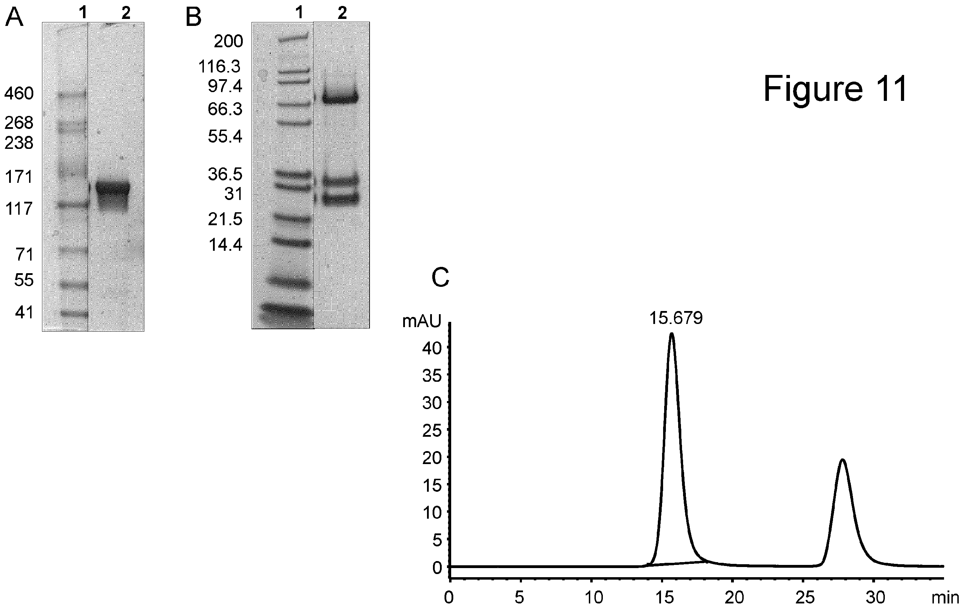

FIGURE 11. (A, B) SDS PAGE (4-12% Tris-Acetate (A) or 4-12% Bis/Tris (B), NuPage Invitrogen, Coomassie-stained) of "1+1 IgG Crossfab, Fc(hole) P329G LALA / Fc(knob) wt" (anti-MCSP/anti-huCD3) (see SEQ ID NOs 5, 29, 31, 33), non reduced (A) and reduced (B). (C) Analytical size exclusion chromatography (Superdex 200 10/300 GL GE Healthcare; 2 mM MOPS pH 7.3, 150 mM NaCl, 0.02% (w/v) NaCl; 50 µg sample injected) of "1+1 IgG Crossfab, Fc(hole) P329G LALA / Fc(knob) wt" (anti-MCSP/anti-huCD3).

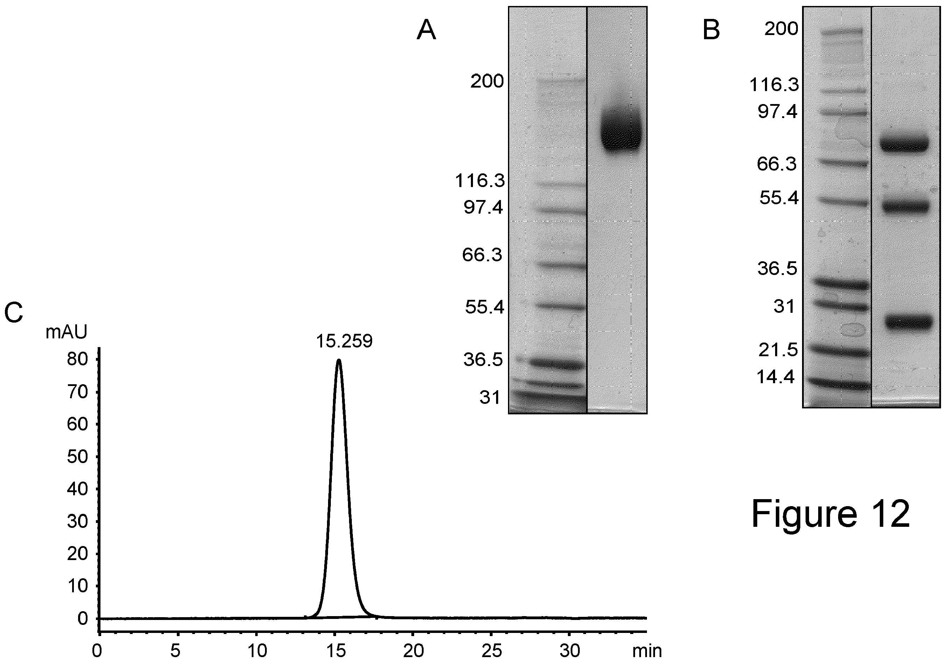

FIGURE 12. (A, B) SDS PAGE (4-12% Bis/Tris, NuPage Invitrogen, Coomassie-stained) of "2+1 IgG Crossfab" (anti-MCSP/anti-huCD3) (see SEQ ID NOs 3, 5, 29, 33), non reduced (A) and reduced (B). (C) Analytical size exclusion chromatography (Superdex 200 10/300 GL GE Healthcare; 2 mM MOPS pH 7.3, 150 mM NaCl, 0.02% (w/v) NaCl; 50 µg sample injected) of "2+1 IgG Crossfab" (anti-MCSP/anti-huCD3).

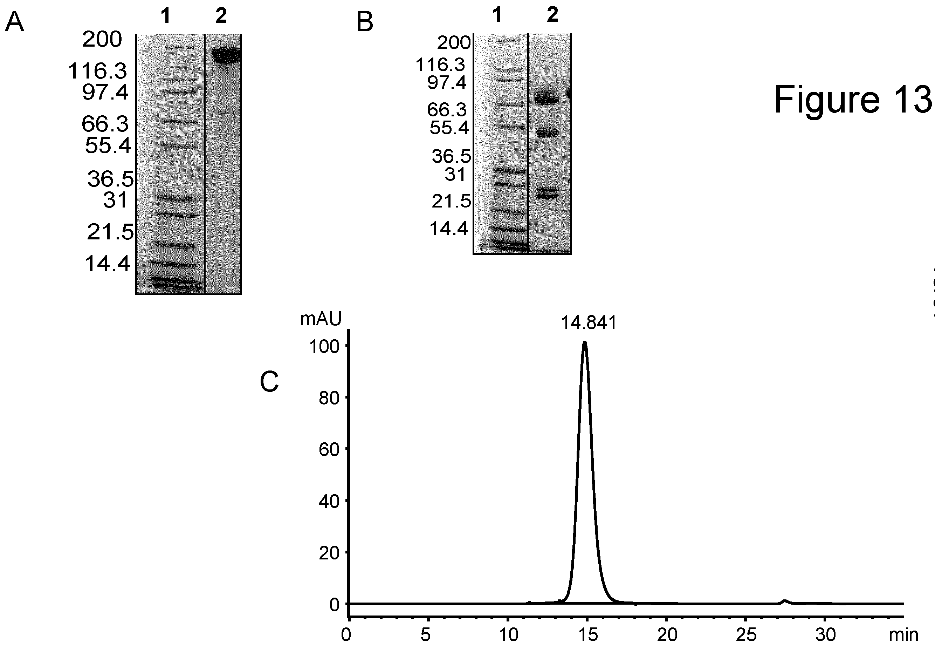

FIGURE 13. (A, B) SDS PAGE (4-12% Bis/Tris, NuPage Invitrogen, Coomassie-stained) of "2+1 IgG Crossfab" (anti-MCSP/anti-cyCD3) (see SEQ ID NOs 3, 5, 35, 37), non reduced (A) and reduced (B). (C) Analytical size exclusion chromatography (Superdex 200 10/300 GL GE Healthcare; 2 mM MOPS pH 7.3, 150 mM NaCl, 0.02% (w/v) NaCl; 50 µg sample injected) of "2+1 IgG Crossfab" (anti-MCSP/anti-cyCD3).

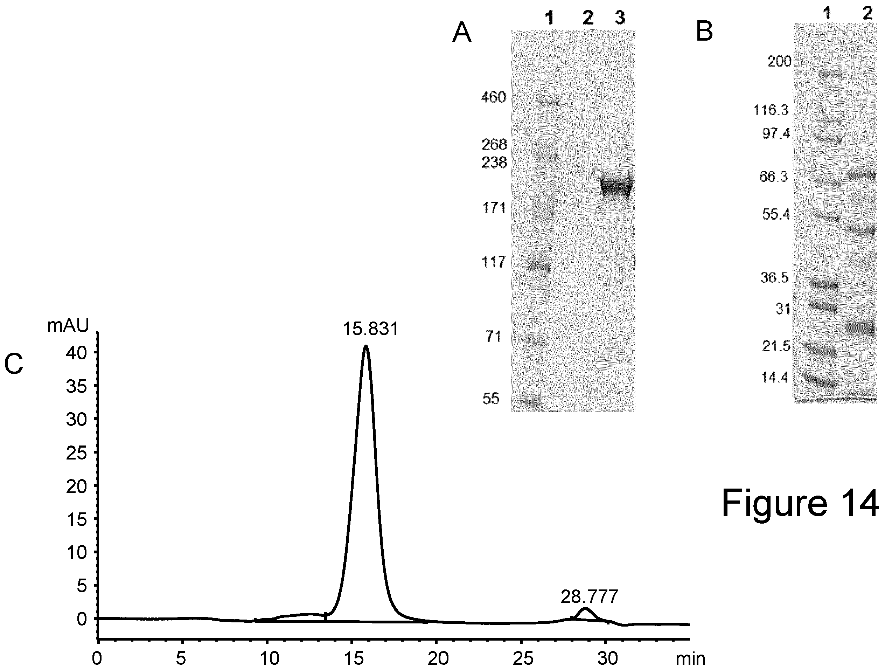

FIGURE 14. (A, B) SDS PAGE (4-12% Bis/Tris, NuPage Invitrogen, Coomassie-stained) of "2+1 IgG Crossfab, inverted" (anti-CEA/anti-huCD3) (see SEQ ID NOs 33, 63, 65, 67), non reduced (A) and reduced (B). (C) Analytical size exclusion chromatography (Superdex 200 10/300 GL GE Healthcare; 2 mM MOPS pH 7.3, 150 mM NaCl, 0.02% (w/v) NaCl; 50 µg sample injected) of "2+1 IgG Crossfab, inverted" (anti-CEA/anti-huCD3).

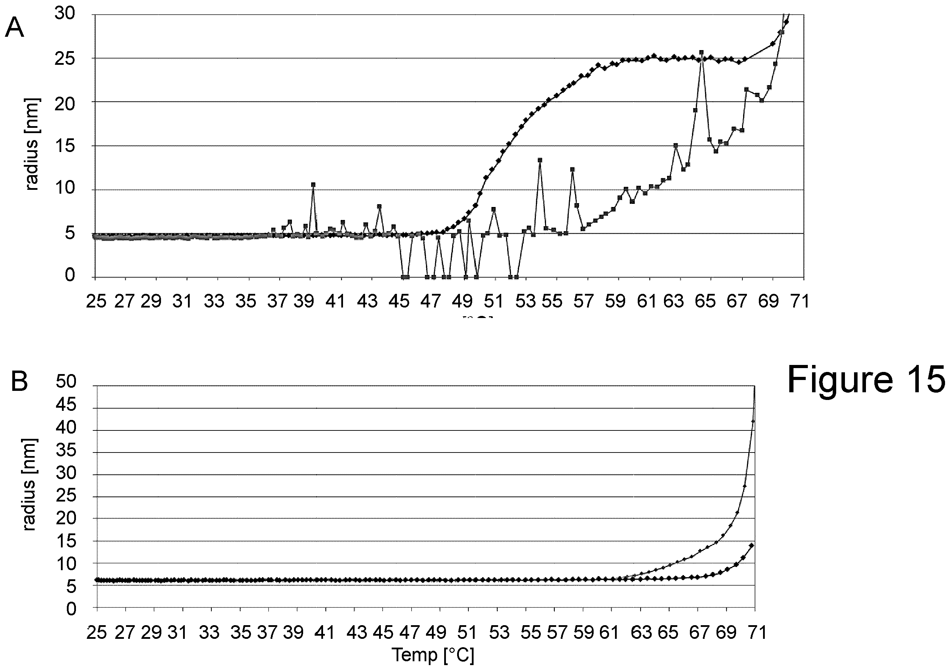

FIGURE 15. (A) Thermal stability of "(scFv)₂-Fc" and "(dsscFv)₂-Fc" (anti-MCSP (LC007)/anti-huCD3 (V9)). Dynamic Light Scattering, measured in a temperature ramp from 25-75°C at 0.05°C/min. Black curve: "(scFv)2-Fc"; grey curve: "(dsscFv)2-Fc". (B) Thermal stability of "2+1 IgG scFab" (see SEQ ID NOs 5, 21, 23) and "2+1 IgG Crossfab" (anti-MCSP/anti-huCD3) (see SEQ ID NOs 3, 5, 29, 33). Dynamic Light Scattering, measured in a temperature ramp from 25-75°C at 0.05°C/min. Black curve: "2+1 IgG scFab"; grey curve: "2+1 IgG Crossfab".

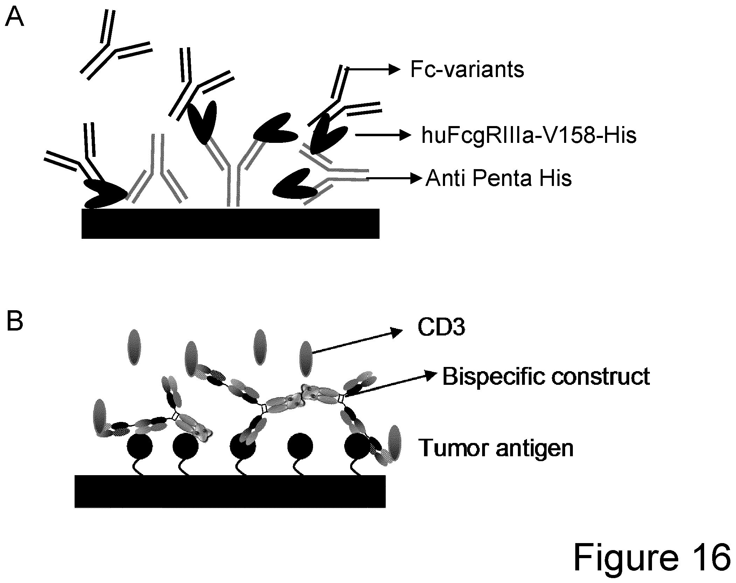

FIGURE 16. Biacore assay setup for (A) determination of interaction of various Fc-mutants with human FcγRIIIa, and for (B) simultaneous binding of T cell bespecific constructs with tumor target and human CD3γ(G₄S)₅CD3ε-AcTev-Fc(knob)-Avi/Fc(hole).
FIGURE 17. Simultaneous binding of T-cell bispecific constructs to the D3 domain of human MCSP and human CD3γ(G₄S)₅CD3ε-AcTev-Fc(knob)-Avi/Fc(hole). (A) "2+1 IgG Crossfab" (see SEQ ID NOs 3, 5, 29, 33), (B) "2+1 IgG scFab" (see SEQ ID NOs 5, 21, 23).

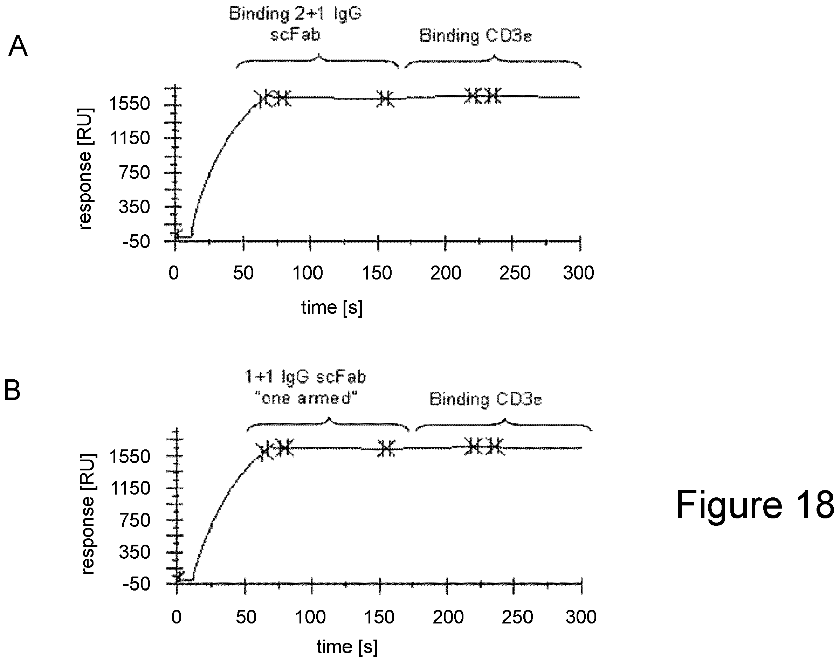

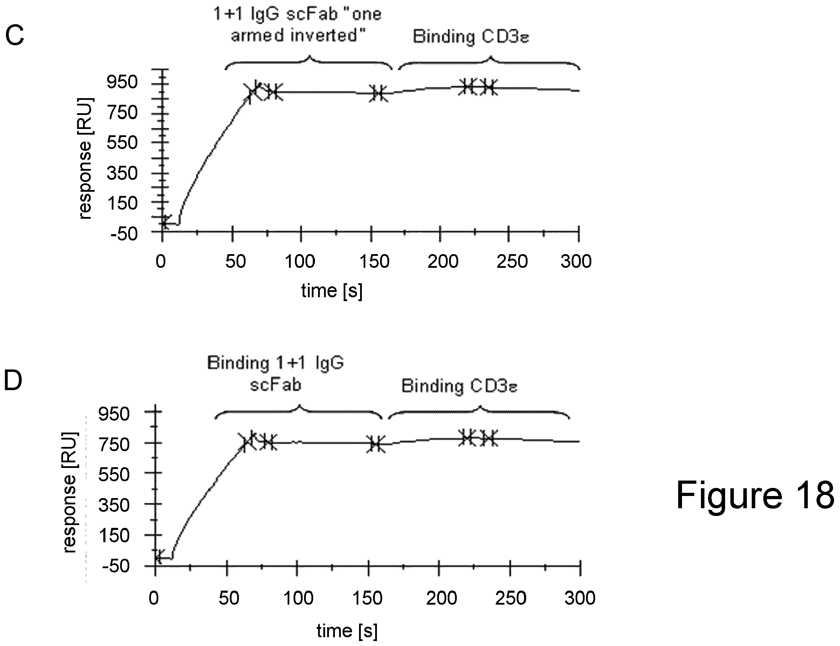

FIGURE 18. Simultaneous binding of T-cell bispecific constructs to human EGFR and human CD3γ(G₄S)₅CD3ε-AcTev-Fc(knob)-Avi/Fc(hole). (A) "2+1 IgG scFab" (see SEQ ID NOs 45, 47, 53), (B) "1+1 IgG scFab, one armed" (see SEQ ID NOs 43, 45, 47), (C) "1+1 IgG scFab, one armed inverted" (see SEQ ID NOs 11, 49, 51), and (D) "1+1 IgG scFab" (see SEQ ID NOs 47, 53,213).

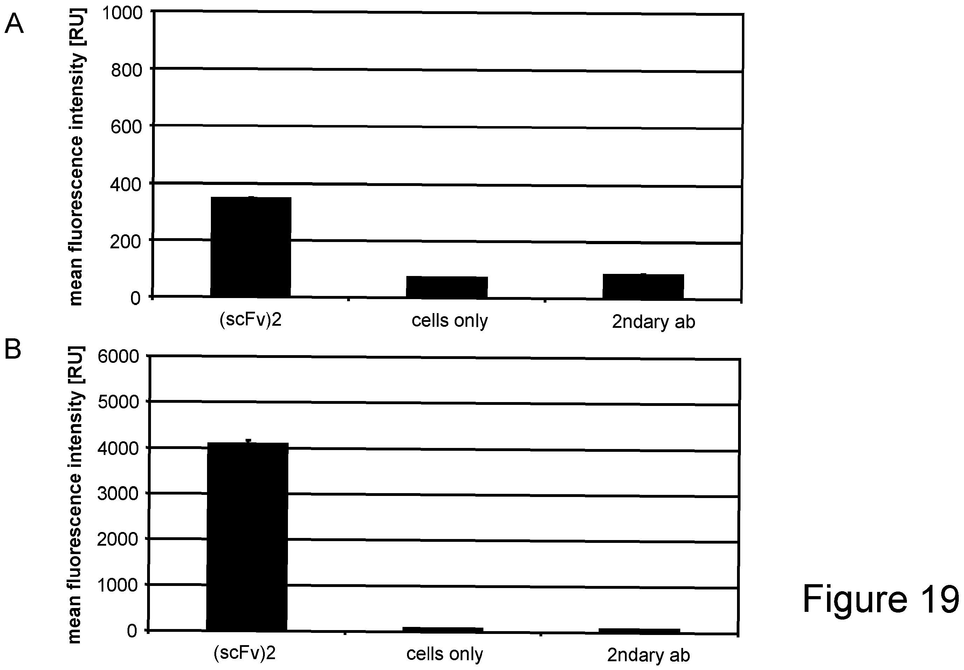

FIGURE 19. Binding of the "(scFv)2" molecule (50 nM) to CD3 expressed on Jurkat cells (A), or to MCSP on Colo-38 cells (B) measured by FACS. Mean fluorescence intensity compared to untreated cells and cells stained with the secondary antibody only is depicted.

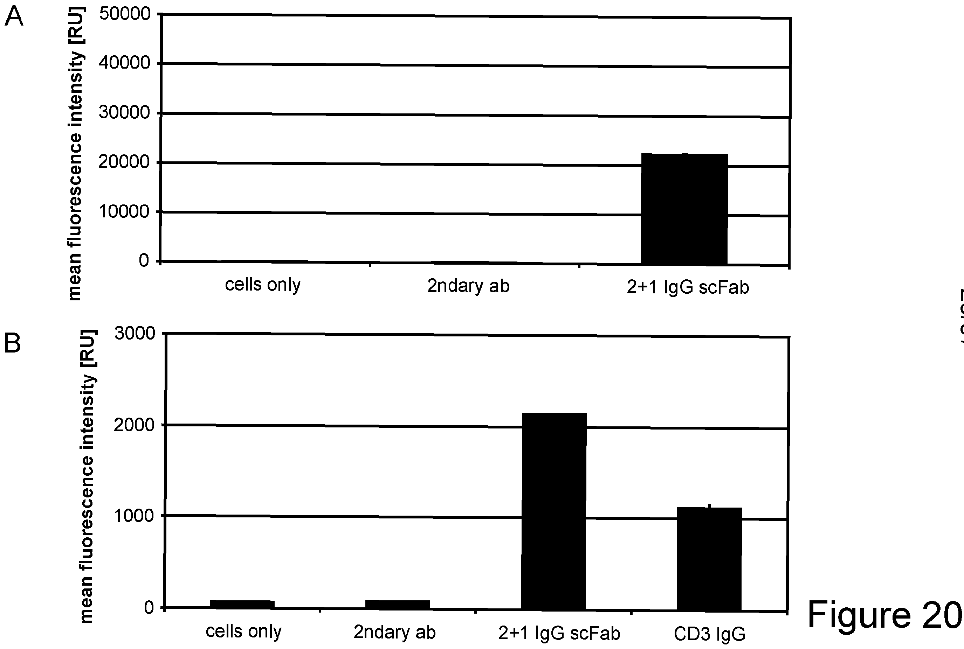

FIGURE 20. Binding of the "2+1 IgG scFab, LALA" (see SEQ ID NOs 5, 17, 19) construct (50 nM) to CD3 expressed on Jurkat cells (A), or to MCSP on Colo-38 cells (B) measured by FACS. Mean fluorescence intensity compared to cells treated with the reference anti-CD3 IgG (as indicated), untreated cells, and cells stained with the secondary antibody only is depicted.

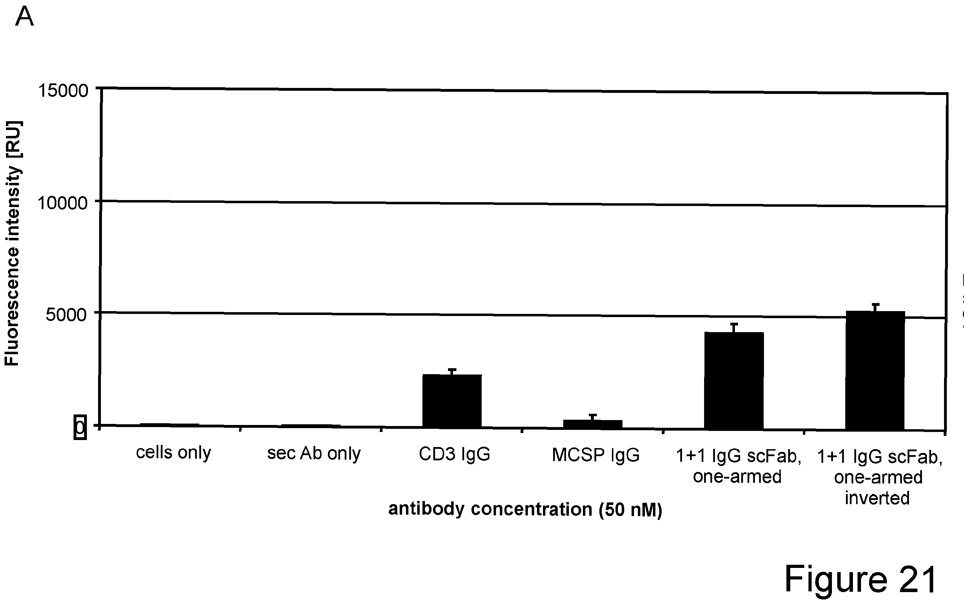

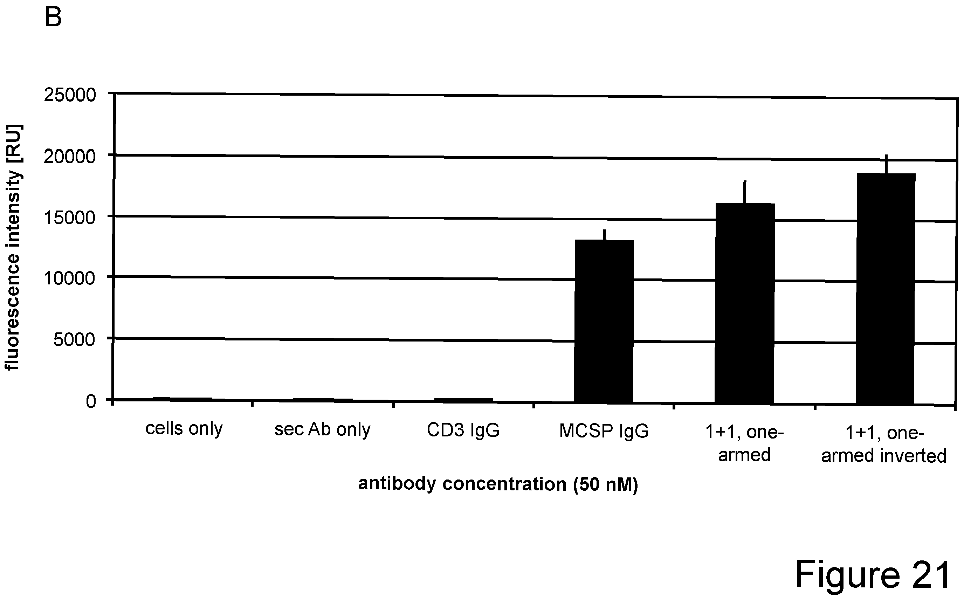

FIGURE 21. Binding of the "1+1 IgG scFab, one armed" (see SEQ ID NOs 1, 3, 5) and "1+1 IgG scFab, one armed inverted" (see SEQ ID NOs 7, 9, 11) constructs (50 nM) to CD3 expressed on Jurkat cells (A), or to MCSP on Colo-38 cells (B) measured by FACS. Mean fluorescence intensity compared to cells treated with the reference anti-CD3 or anti-MCSP IgG (as indicated), untreated cells, and cells stained with the secondary antibody only is depicted.

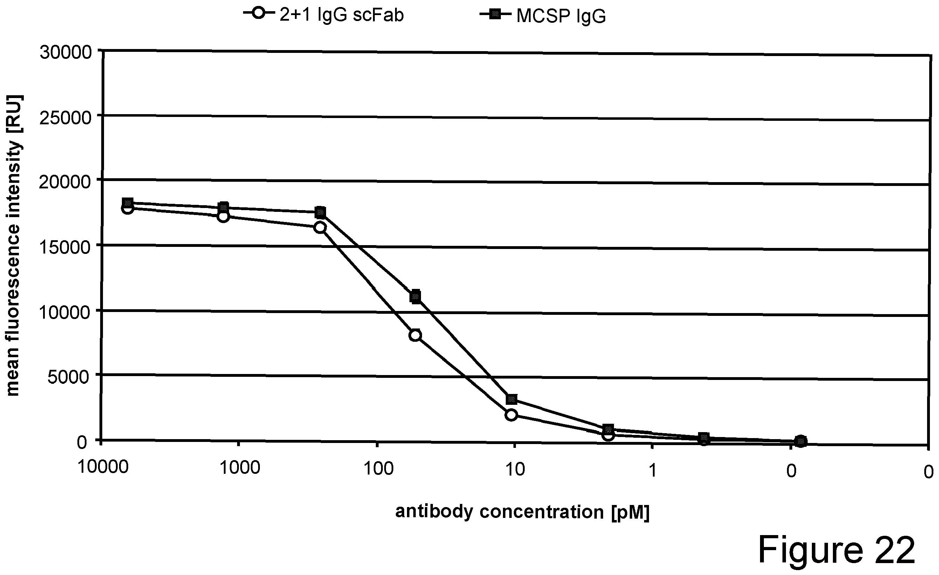

FIGURE 22. Dose dependent binding of the "2+1 IgG scFab, LALA" (see SEQ ID NOs 5, 17, 19) bispecific construct and the corresponding anti-MCSP IgG to MCSP on Colo-38 cells as measured by FACS.

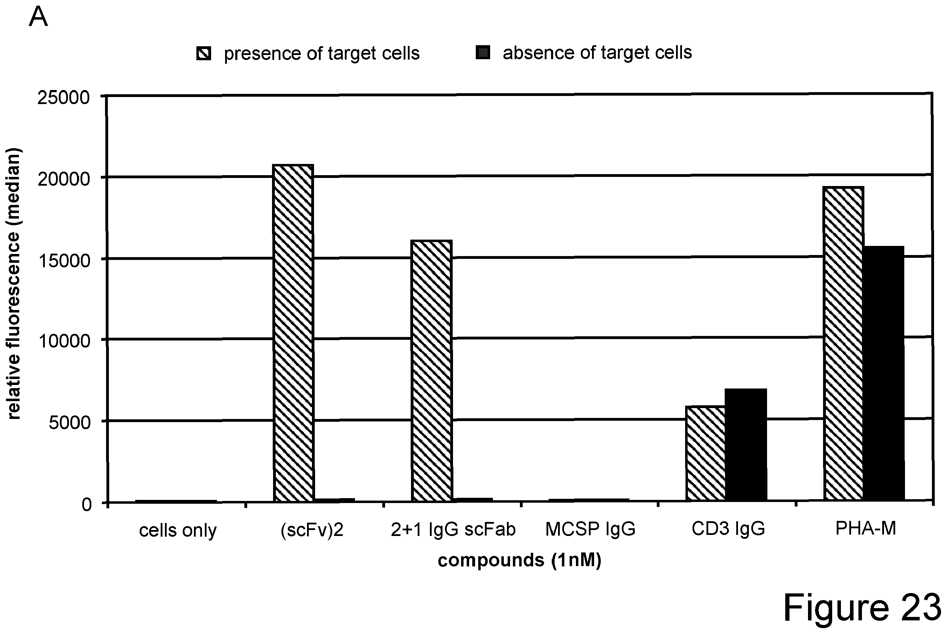

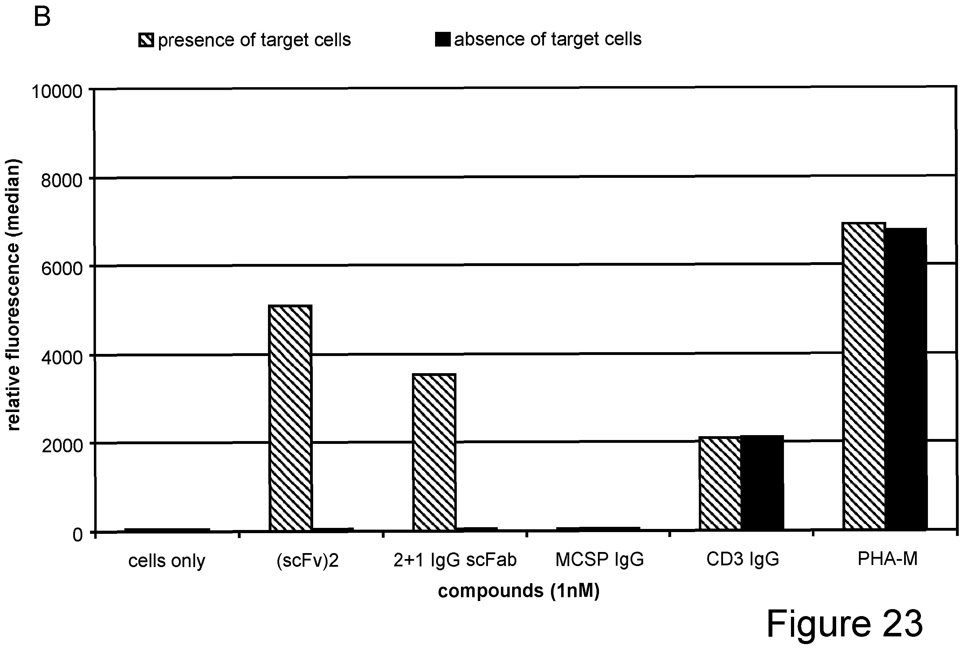

FIGURE 23. Surface expression level of different activation markers on human T cells after incubation with 1 nM of "2+1 IgG scFab, LALA" (see SEQ ID NOs 5, 17, 19) or "(scFv)2" CD3-MCSP bispecific constructs in the presence or absence of Colo-38 tumor target cells, as indicated (E:T ratio of PBMCs to tumor cells = 10:1). Depicted is the expression level of the early activation marker CD69 (A), or the late activation marker CD25 (B) on CD8⁺ T cells after 15 or 24 hours incubation, respectively.

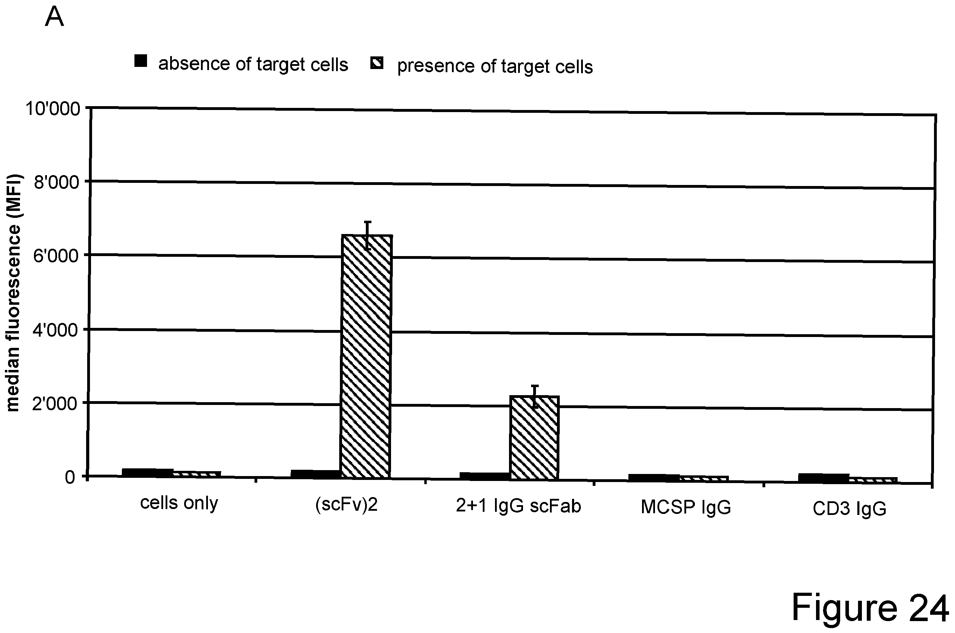

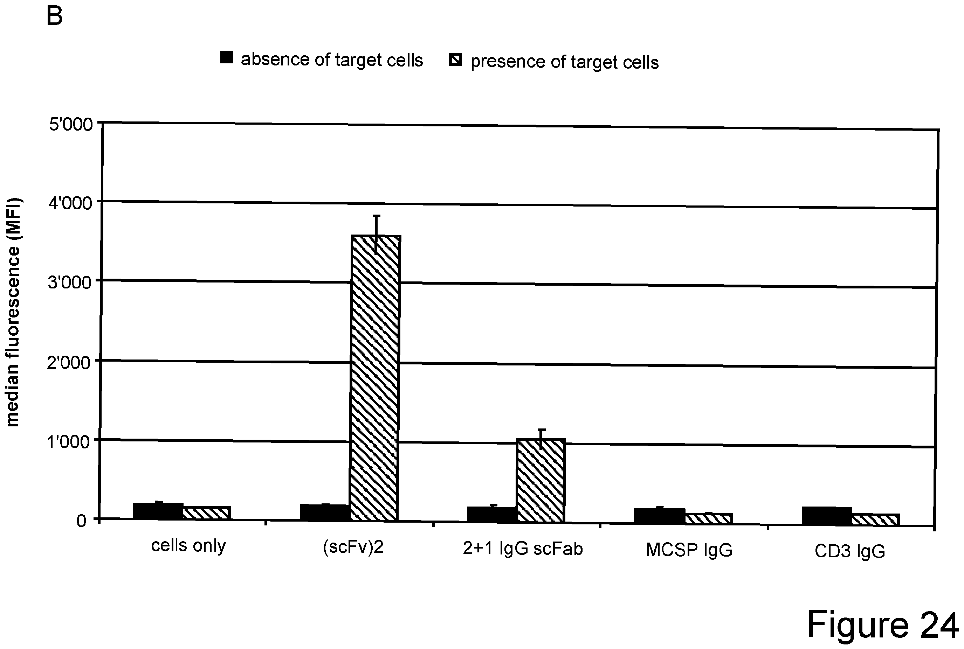

FIGURE 24. Surface expression level of the late activation marker CD25 on human T cells after incubation with 1 nM of "2+1 IgG scFab, LALA" (see SEQ ID NOs 5, 17, 19) or "(scFv)2" CD3-MCSP bispecific constructs in the presence or absence of Colo-38 tumor target cells, as indicated (E:T ratio = 5:1). Depicted is the expression level of the late activation marker CD25 on CD8⁺ T cells (A) or on CD4⁺ T cells (B) after 5 days incubation.

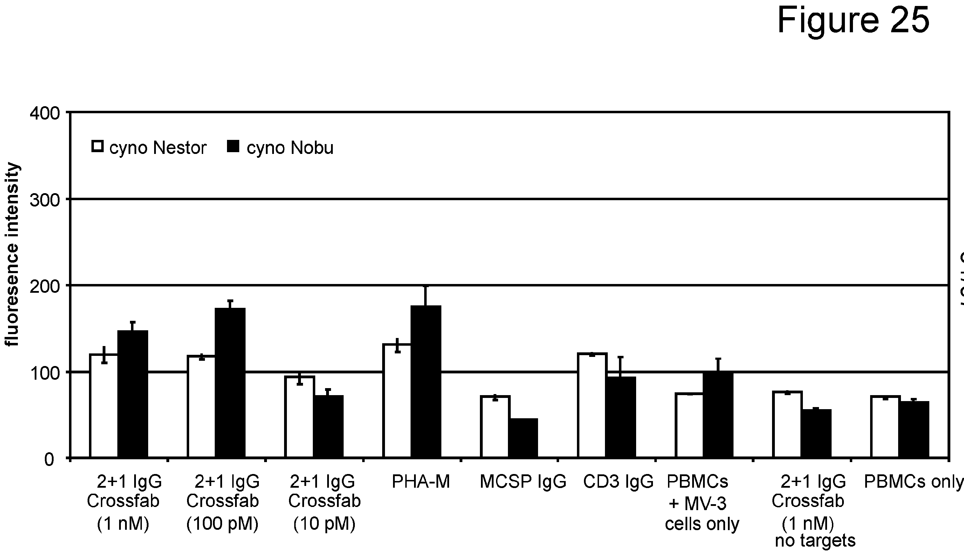

FIGURE 25. Surface expression level of the late activation marker CD25 on cynomolgus CD8⁺ T cells from two different animals (cyno Nestor, cyno Nobu) after 43 hours incubation with the indicated concentrations of the "2+1 IgG Crossfab" bispecific construct (targeting cynomolgus CD3 and human MCSP; see SEQ ID NOs 3, 5, 35, 37), in the presence or absence of human MCSP-expressing MV-3 tumor target cells (E:T ratio = 3:1). As controls, the reference IgGs (anti-cynomolgus CD3 IgG, anti-human MCSP IgG) or the unphysiologic stimulus PHA-M were used.

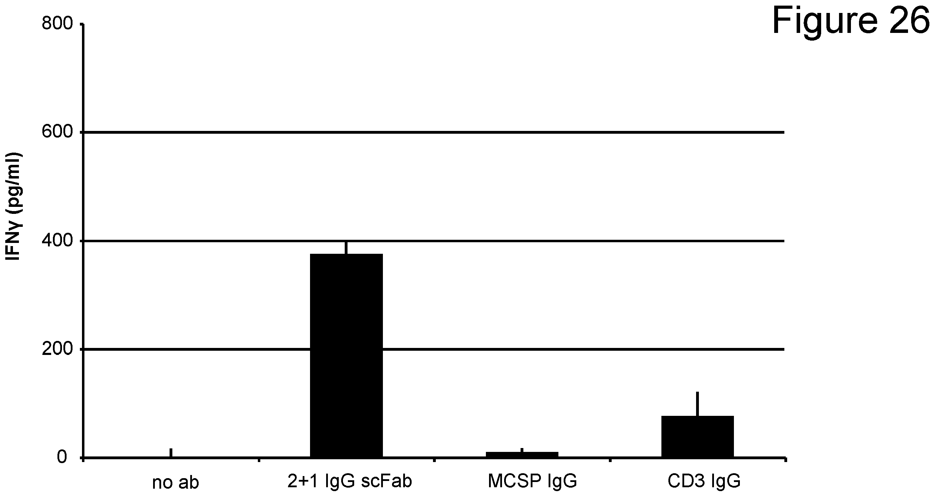

FIGURE 26. IFN-γ levels, secreted by human pan T cells that were activated for 18.5 hours by the "2+1 IgG scFab, LALA" CD3-MCSP bispecific construct (see SEQ ID NOs 5, 17, 19) in the presence of U87MG tumor cells (E:T ratio = 5:1). As controls, the corresponding anti-CD3 and anti-MCSP IgGs were administered.

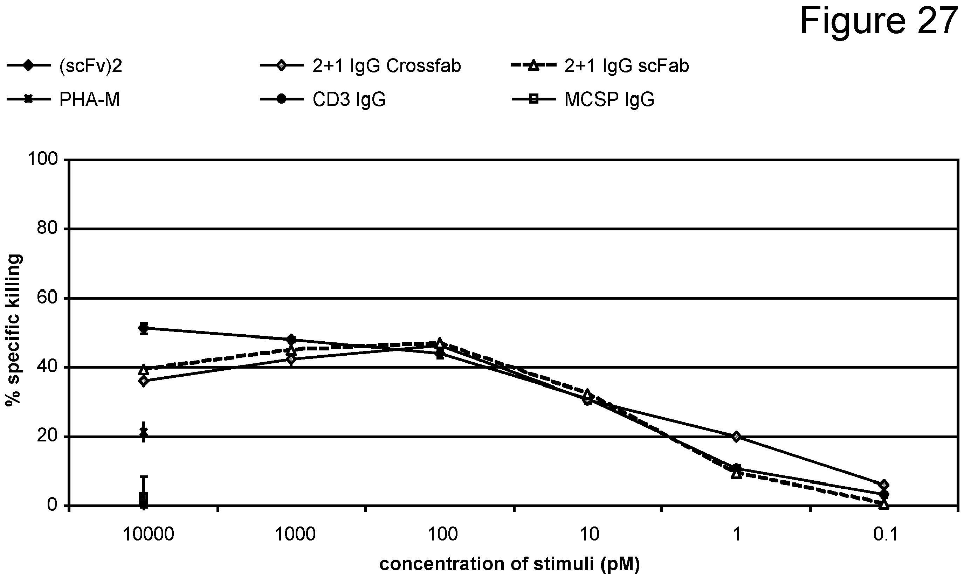

FIGURE 27. Killing (as measured by LDH release) of MDA-MB-435 tumor cells upon co-culture with human pan T cells (E:T ratio = 5:1) and activation for 20 hours by different concentrations of the "2+1 IgG scFab" (see SEQ ID NOs 5, 21, 23), "2+1 IgG Crossfab" (see SEQ ID NOs 3, 5, 29, 33) and "(scFv)2" bispecific molecules and corresponding IgGs.

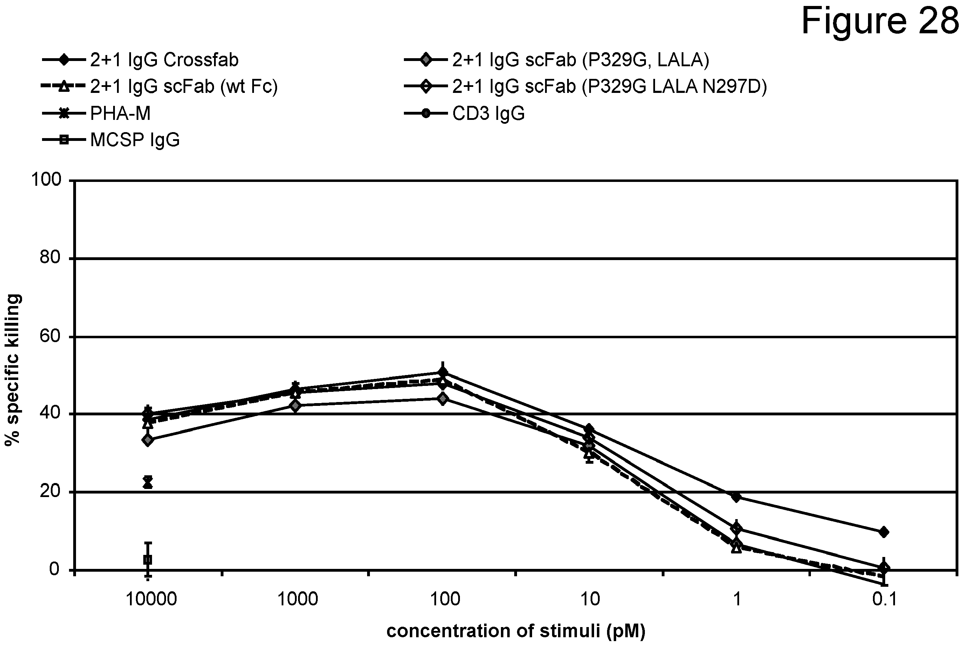

FIGURE 28. Killing (as measured by LDH release) of MDA-MB-435 tumor cells upon co-culture with human pan T cells (E:T ratio = 5:1), and activation for 20 hours by different concentrations of the bispecific constructs and corresponding IgGs. "2+1 IgG scFab" constructs differing in their Fc-domain (having either a wild-type Fc domain (see SEQ ID NOs 5, 13, 15), or a Fc-domain mutated to abolish (NK) effector cell function: P329G LALA (see SEQ ID NOs 5, 21, 23), P329G LALA N297D (see SEQ ID NOs 5, 25, 27)) and the "2+1 IgG Crossfab" (see SEQ ID NOs 3, 5, 29, 33) construct were compared.

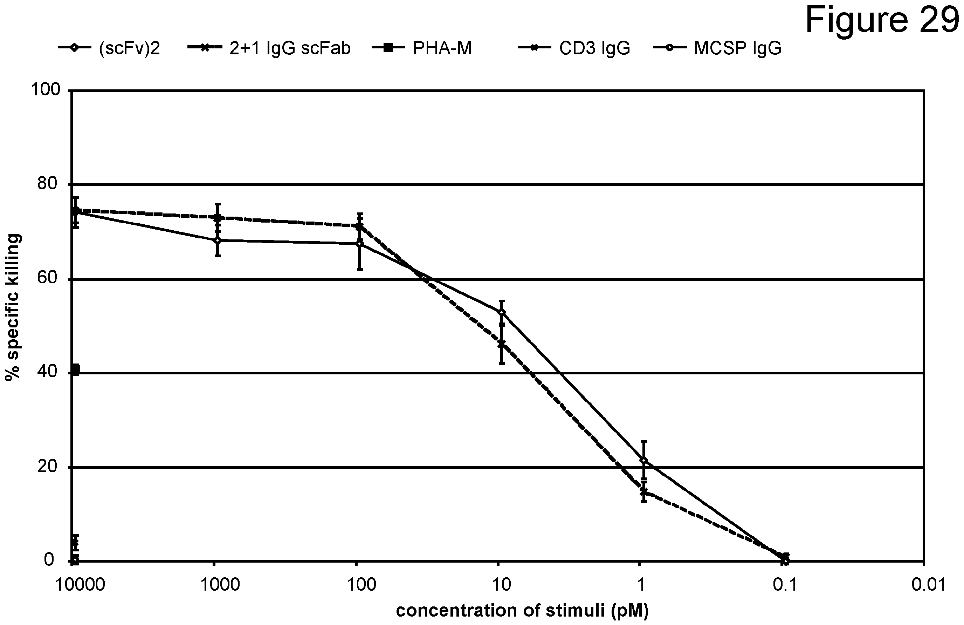

FIGURE 29. Killing (as measured by LDH release) of Colo-38 tumor cells upon co-culture with human pan T cells (E:T ratio = 5:1), treated with CD3-MCSP bispecific "2+1 IgG scFab, LALA" (see SEQ ID NOs 5, 17, 19) construct, "(scFv)2" molecule or corresponding IgGs for 18.5 hours.

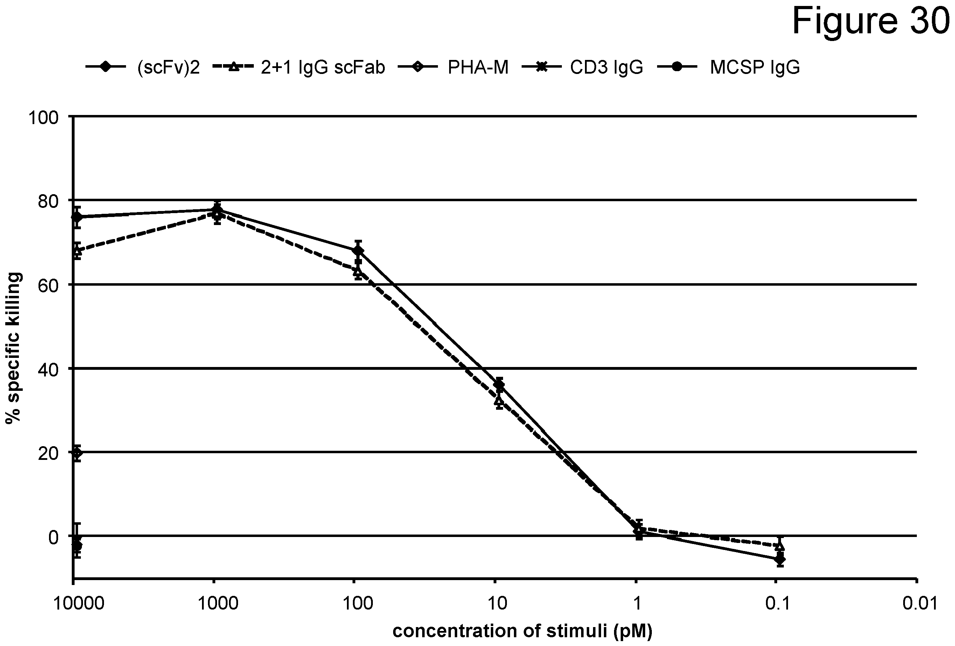

FIGURE 30. Killing (as measured by LDH release) of Colo-38 tumor cells upon co-culture with human pan T cells (E:T ratio = 5:1), treated with CD3-MCSP bispecific "2+1 IgG scFab, LALA" (see SEQ ID NOs 5, 17, 19) construct, the "(scFv)2" molecule or corresponding IgGs for 18 hours.

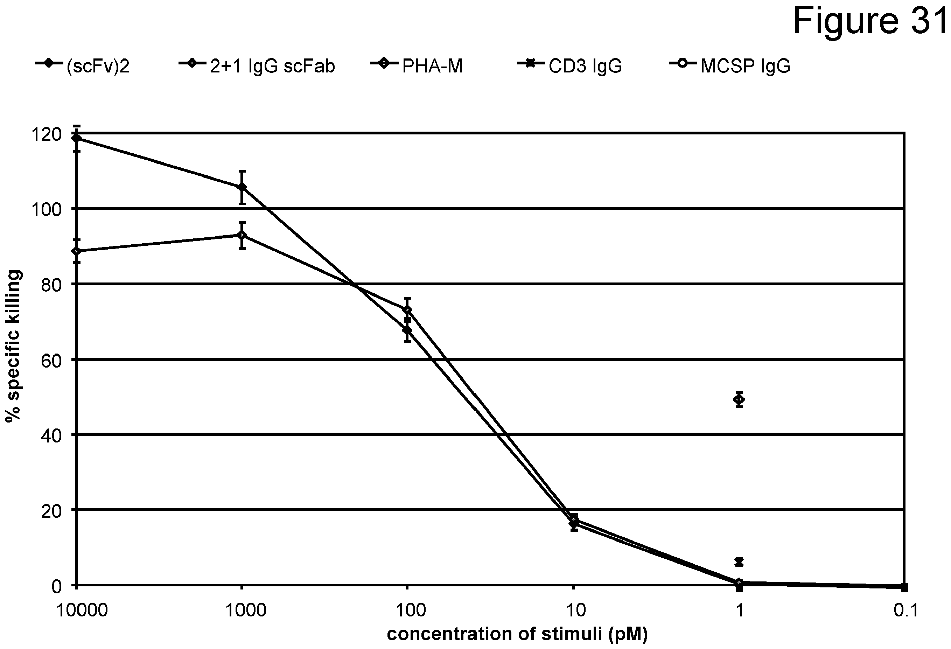

FIGURE 31. Killing (as measured by LDH release) of MDA-MB-435 tumor cells upon co-culture with human pan T cells (E:T ratio = 5:1), and activation for 23.5 hours by different concentrations of the CD3-MCSP bispecific "2+1 IgG scFab, LALA" (see SEQ ID NOs 5, 17, 19) construct, "(scFv)2" molecule or corresponding IgGs.

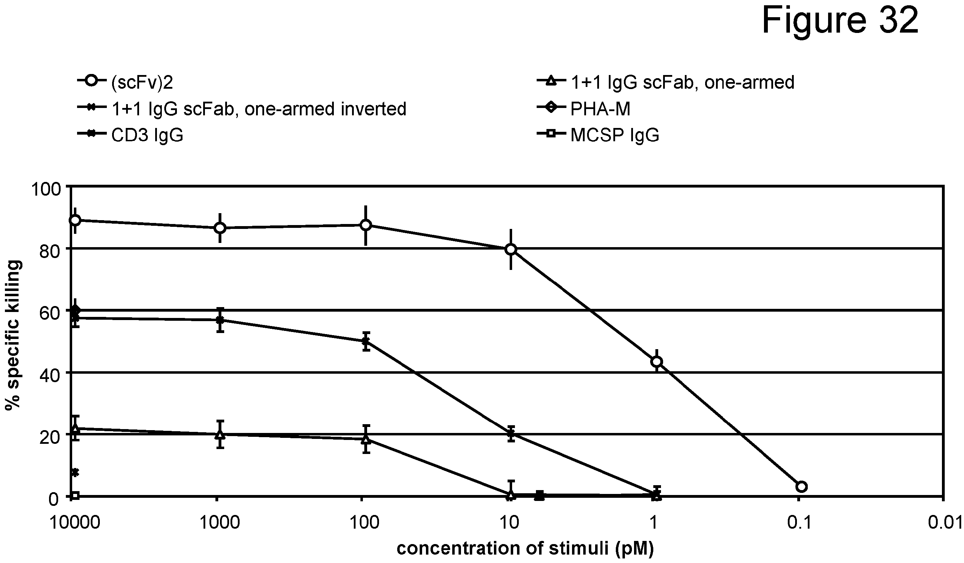

FIGURE 32. Killing (as measured by LDH release) of Colo-38 tumor cells upon co-culture with human pan T cells (E:T ratio = 5:1) and activation for 19 hours by different concentrations of the CD3-MCSP bispecific "1+1 IgG scFab, one armed" (see SEQ ID NOs 1, 3, 5), "1+1 IgG scFab, one armed inverted" (see SEQ ID NOs 7, 9, 11) or "(scFv)2" constructs, or corresponding IgGs.

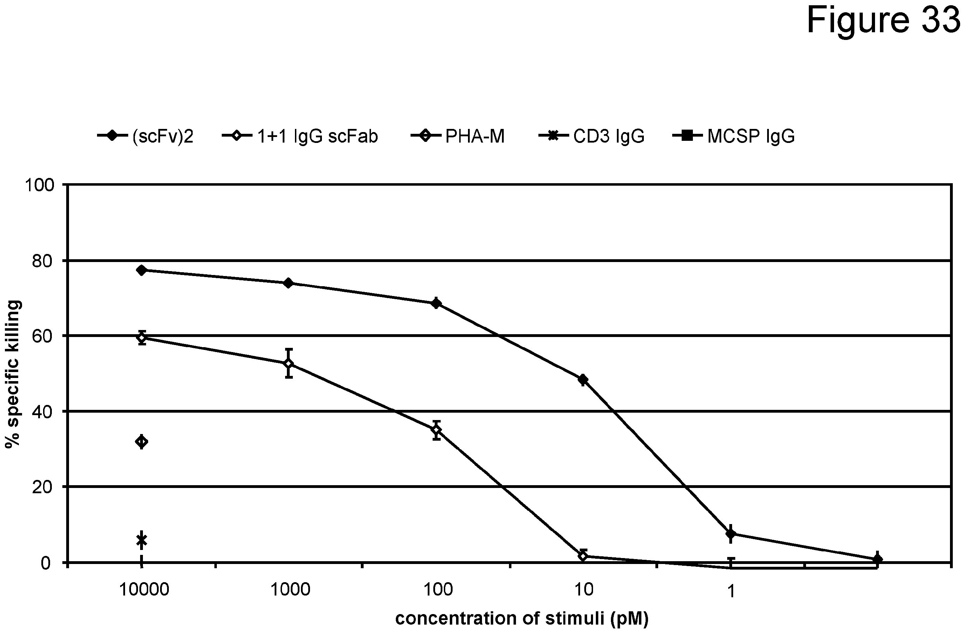

FIGURE 33. Killing (as measured by LDH release) of Colo-38 tumor cells upon co-culture with human pan T cells (E:T ratio = 5:1), treated with "1+1 IgG scFab" CD3-MCSP bispecific construct (see SEQ ID NOs 5, 21, 213) or "(scFv)2" molecule for 20 hours.

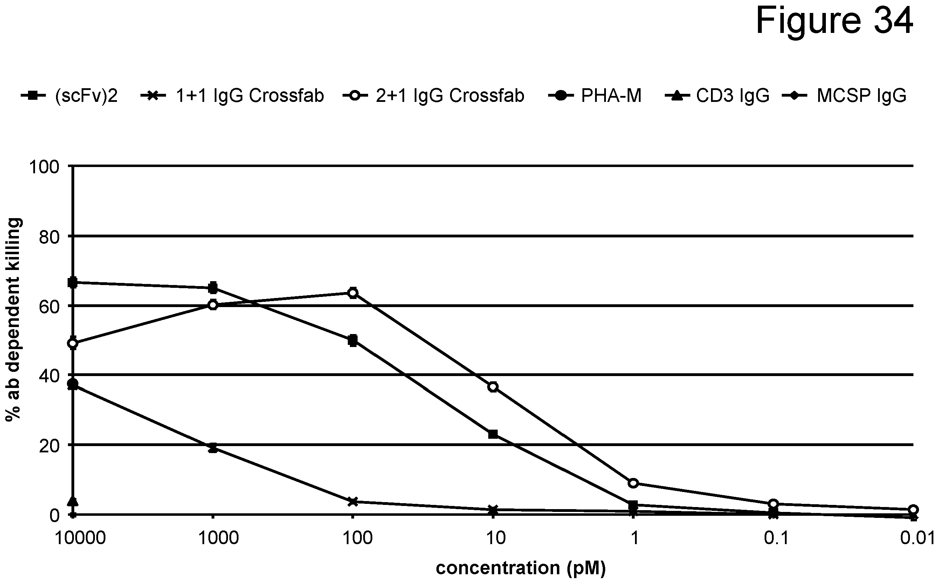

FIGURE 34. Killing (as measured by LDH release) of MDA-MB-435 tumor cells upon co-culture with human pan T cells (E:T ratio = 5:1), and activation for 21 hours by different concentrations of the bispecific constructs and corresponding IgGs. The CD3-MCSP bispecific "2+1 IgG Crossfab" (see SEQ ID NOs 3, 5, 29, 33) and "1+1 IgG Crossfab" (see SEQ ID NOs 5, 29, 31, 33) constructs, the "(scFv)2" molecule and corresponding IgGs were compared.

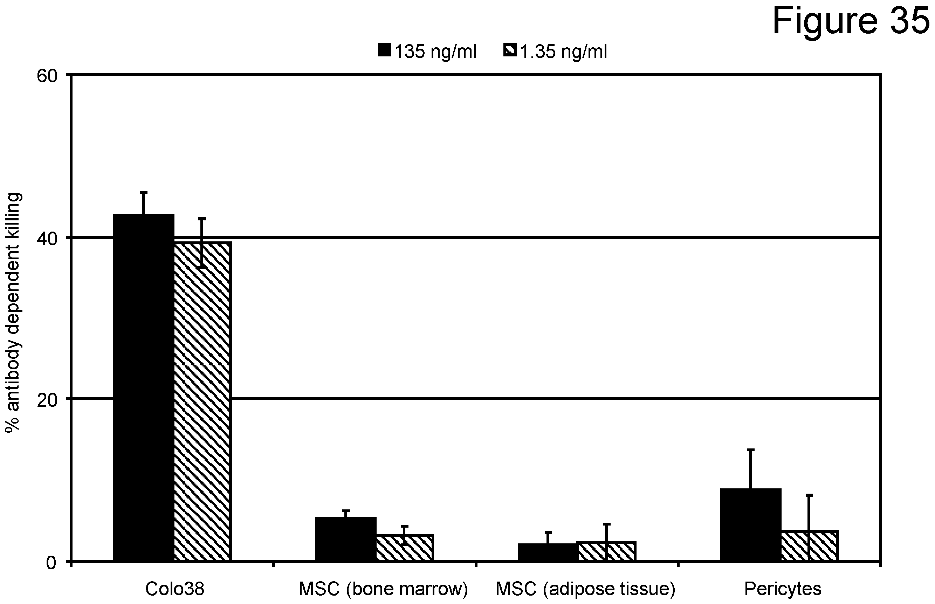

FIGURE 35. Killing (as measured by LDH release) of different target cells (MCSP-positive Colo-38 tumor target cells, mesenchymal stem cells derived from bone marrow or adipose tissue, or pericytes from placenta; as indicated) induced by the activation of human T cells by 135 ng/ml or 1.35 ng/ml of the "2+1 IgG Crossfab" CD3-MCSP bispecific construct (see SEQ ID NOs 3, 5, 29, 33) (E:T ratio = 25:1).

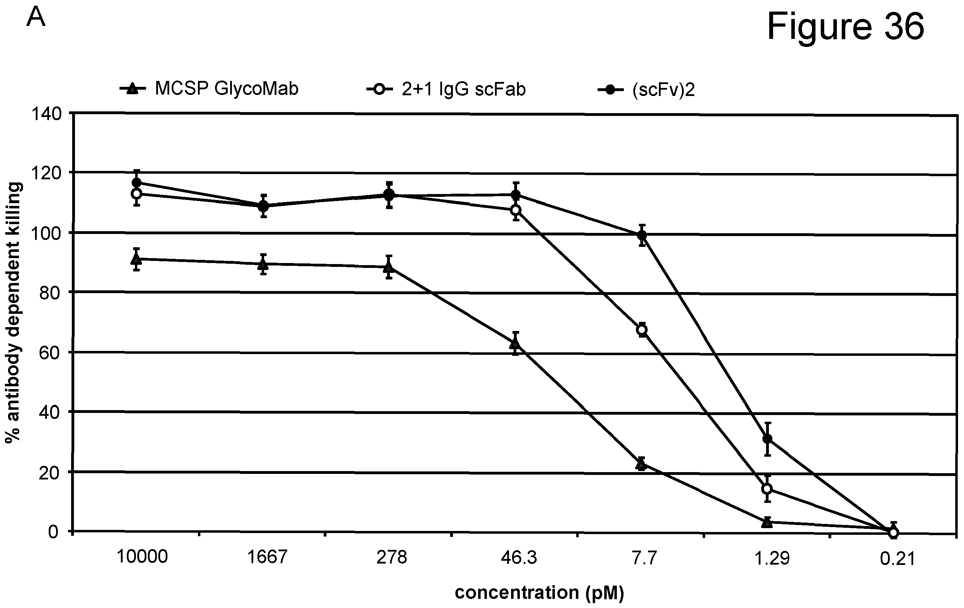

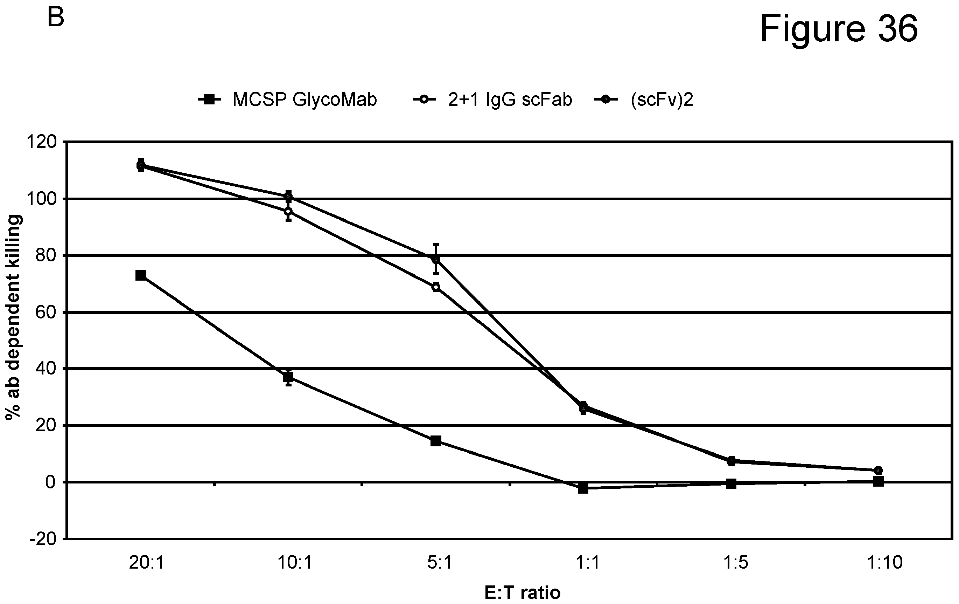

FIGURE 36. Killing (as measured by LDH release) of Colo-38 tumor target cells, measured after an overnight incubation of 21h, upon co-culture with human PBMCs and different CD3-MCSP bispecific constructs ("2+1 IgG scFab, LALA" (see SEQ ID NOs 5, 17, 19) and "(scFv)₂") or a glycoengineered anti-MCSP IgG (GlycoMab). The effector to target cell ratio was fixed at 25:1 (A), or varied as depicted (B). PBMCs were isolated from fresh blood (A) or from a Buffy Coat (B).

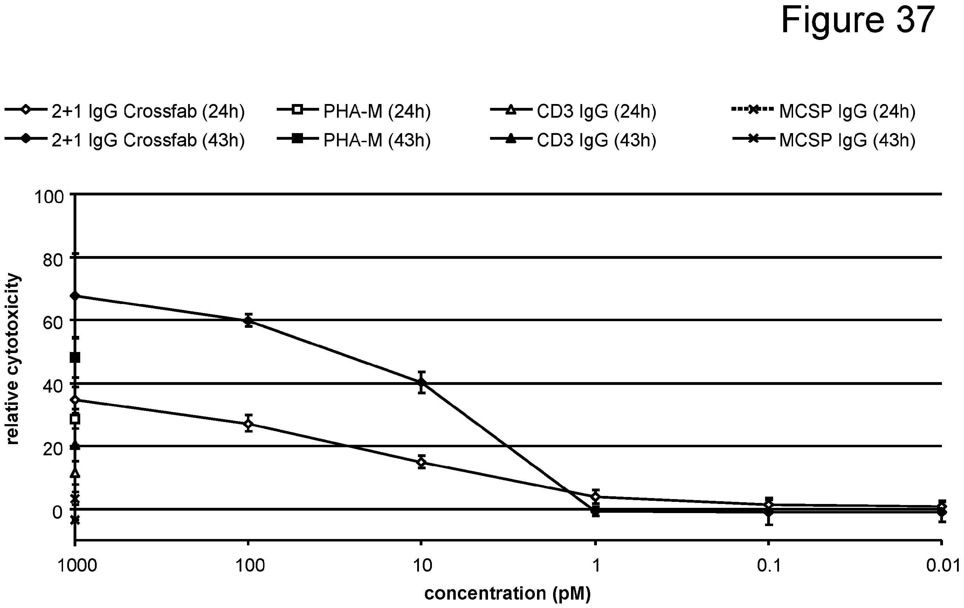

FIGURE 37. Time-dependent cytotoxic effect of the "2+1 IgG Crossfab" construct, targeting cynomolgus CD3 and human MCSP (see SEQ ID NOs 3, 5, 35, 37). Depicted is the LDH release from human MCSP-expressing MV-3 cells upon co-culture with primary cynomolgus PBMCs (E:T ratio = 3:1) for 24 h or 43 h. As controls, the reference IgGs (anti-cyno CD3 IgG and anti-human MCSP IgG) were used at the same molarity. PHA-M served as a control for (unphysiologic) T cell activation.

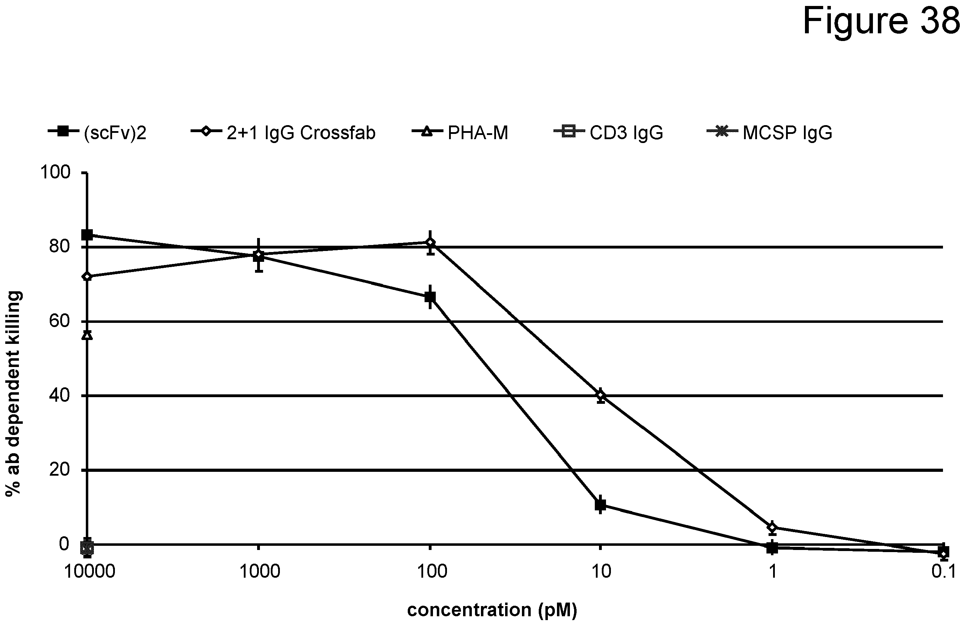

FIGURE 38. Killing (as measured by LDH release) of huMCSP-positive MV-3 melanoma cells upon co-culture with human PBMCs (E:T ratio = 10:1), treated with different CD3-MCSP bispecific constructs ("2+1 IgG Crossfab" (see SEQ ID NOs 3, 5, 29, 33) and "(scFv)₂") for -26 hours.

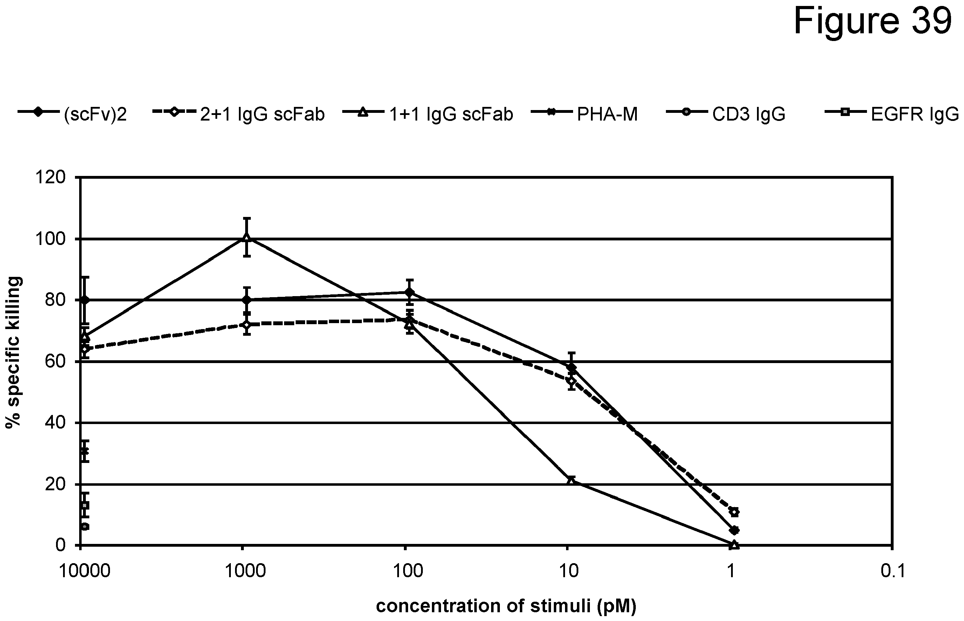

FIGURE 39. Killing (as measured by LDH release) of EGFR-positive LS-174T tumor cells upon co-culture with human pan T cells (E:T ratio = 5:1), treated with different CD3-EGFR bispecific constructs ("2+1 IgG scFab" (see SEQ ID NOs 45, 47, 53), "1+1 IgG scFab" (see SEQ ID NOs 47, 53, 213) and "(scFv)₂") or reference IgGs for 18 hours.

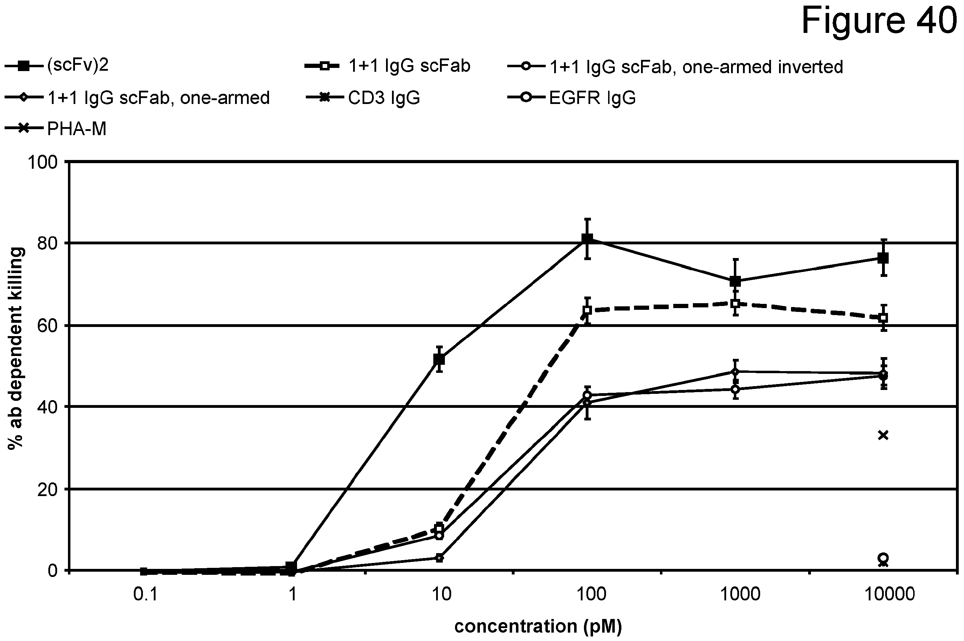

FIGURE 40. Killing (as measured by LDH release) of EGFR-positive LS-174T tumor cells upon co-culture with human pan T cells (E:T ratio = 5:1), treated with different CD3-EGFR bispecific constructs ("1+1 IgG scFab, one armed" (see SEQ ID NOs 43, 45, 47), "1+1 IgG scFab, one armed inverted" (see SEQ ID NOs 11, 49, 51), "1+1 IgG scFab" (see SEQ ID NOs 47, 53, 213) and "(scFv)₂") or reference IgGs for 21 hours.

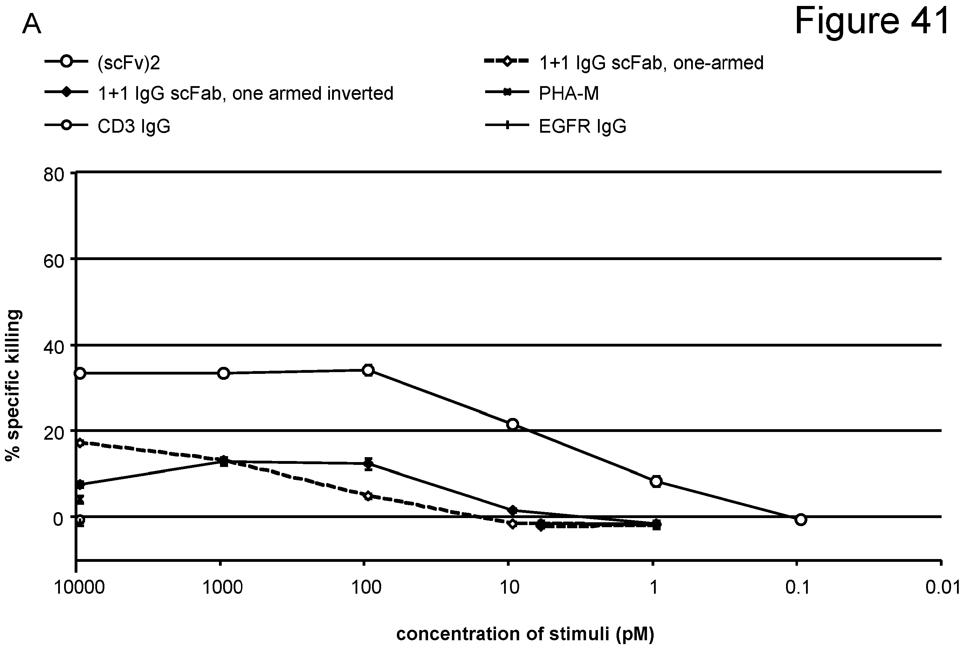

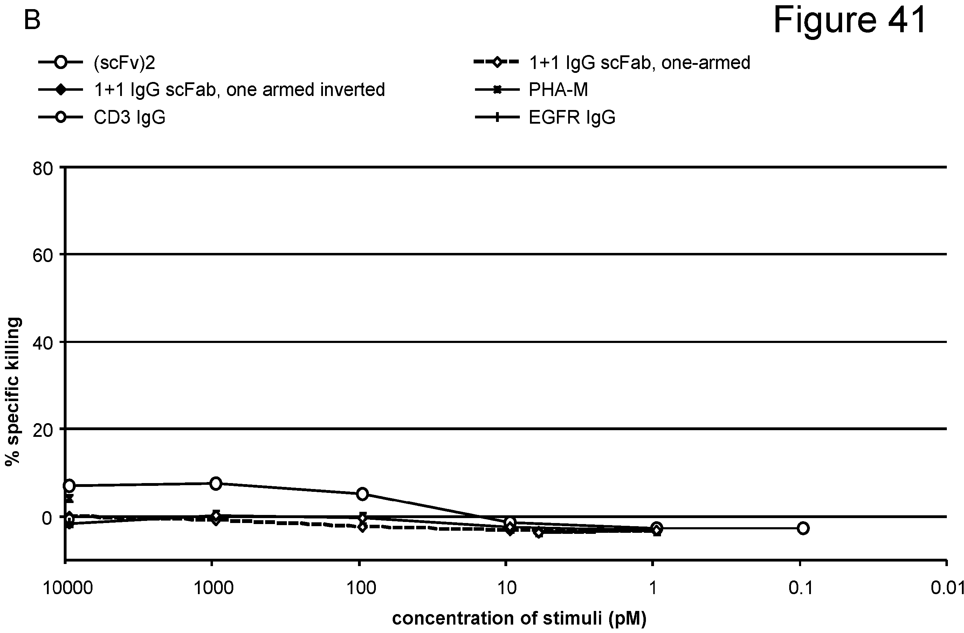

FIGURE 41. Killing (as measured by LDH release) of EGFR-positive LS-174T tumor cells upon co-culture with either human pan T cells (A) or human naive T cells (B), treated with different CD3-EGFR bispecific constructs ("1+1 IgG scFab, one armed" (see SEQ ID NOs 43, 45, 47), "1+1 IgG scFab, one armed inverted" (see SEQ ID NOs 11, 49, 51) and "(scFv)₂") or reference IgGs for 16 hours. The effector to target cell ratio was 5:1.

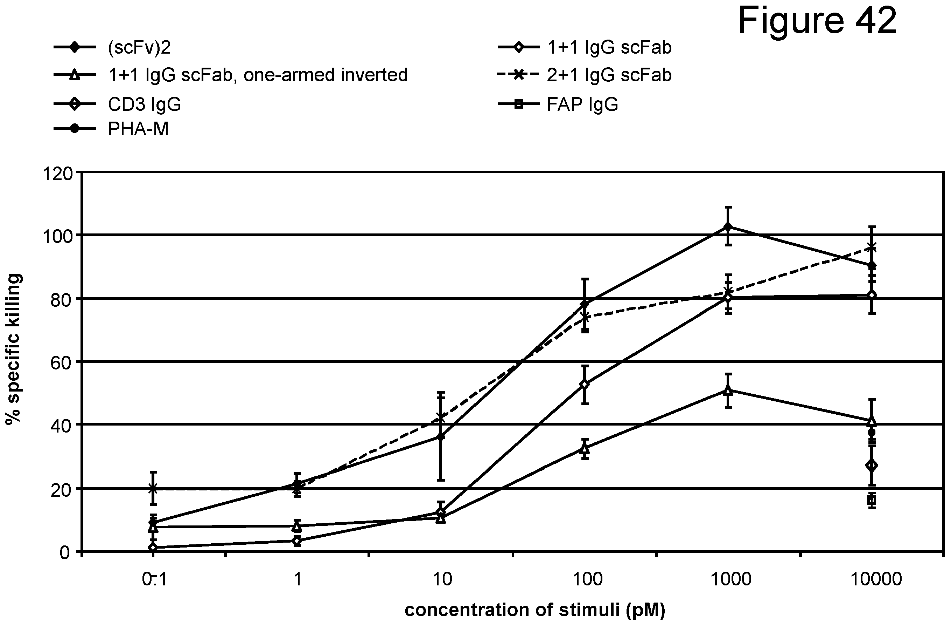

FIGURE 42. Killing (as measured by LDH release) of FAP-positive GM05389 fibroblasts upon co-culture with human pan T cells (E:T ratio = 5:1), treated with different CD3-FAP bispecific constructs ("1+1 IgG scFab, one armed inverted" (see SEQ ID NOs 11, 51, 55), "1+1 IgG scFab" (see SEQ ID NOs 57, 61, 213), "2+1 IgG scFab" (see SEQ ID NOs 57, 59, 61) and "(scFv)₂") for ∼18 hours.

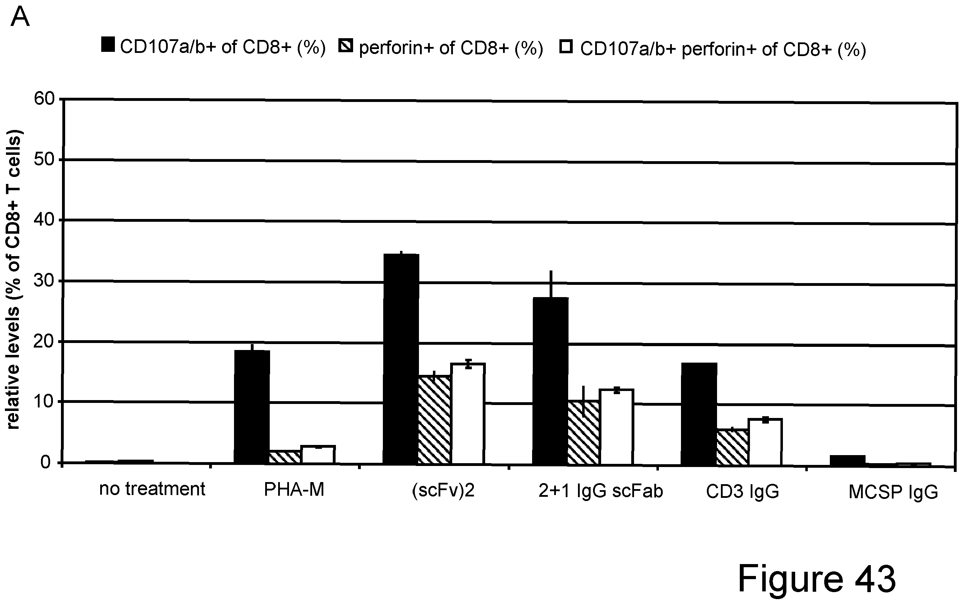

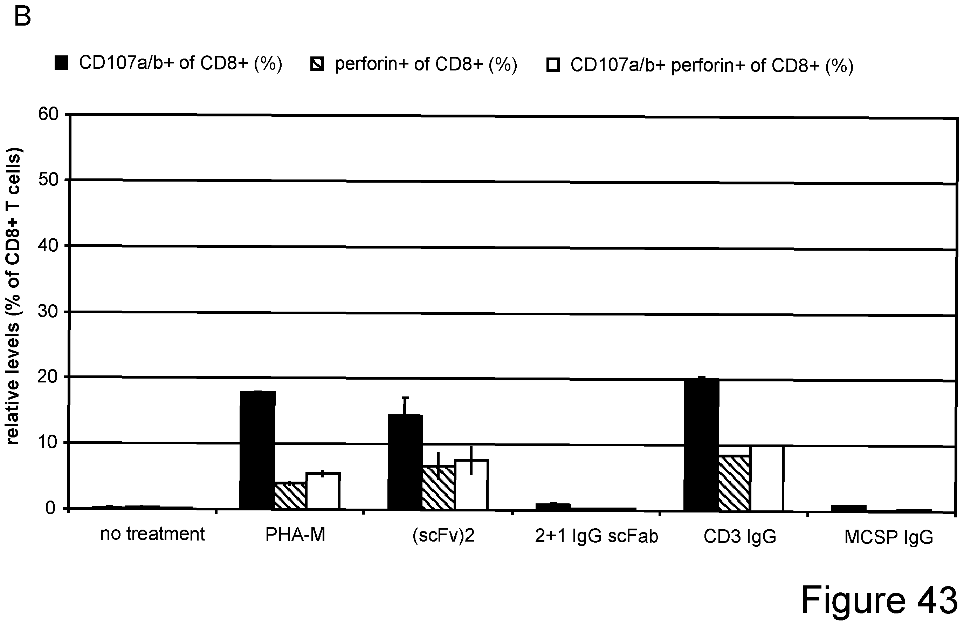

FIGURE 43. Flow cytrometric analysis of expression levels of CD107a/b, as well as perforin levels in CD8⁺ T cells that have been treated with different CD3-MCSP bispecific constructs ("2+1 IgG scFab, LALA" (see SEQ ID NOs 5, 17, 19) and "(scFv)₂") or corresponding control IgGs in the presence (A) or absence (B) of target cells for 6h. Human pan T cells were incubated with 9.43 nM of the different molecules in the presence or absence of Colo-38 tumor target cells at an effector to target ratio of 5:1. Monensin was added after the first hour of incubation to increase intracellular protein levels by preventing protein transport. Gates were set either on all CD107a/b positive, perforin-positive or double-positive cells, as depicted.

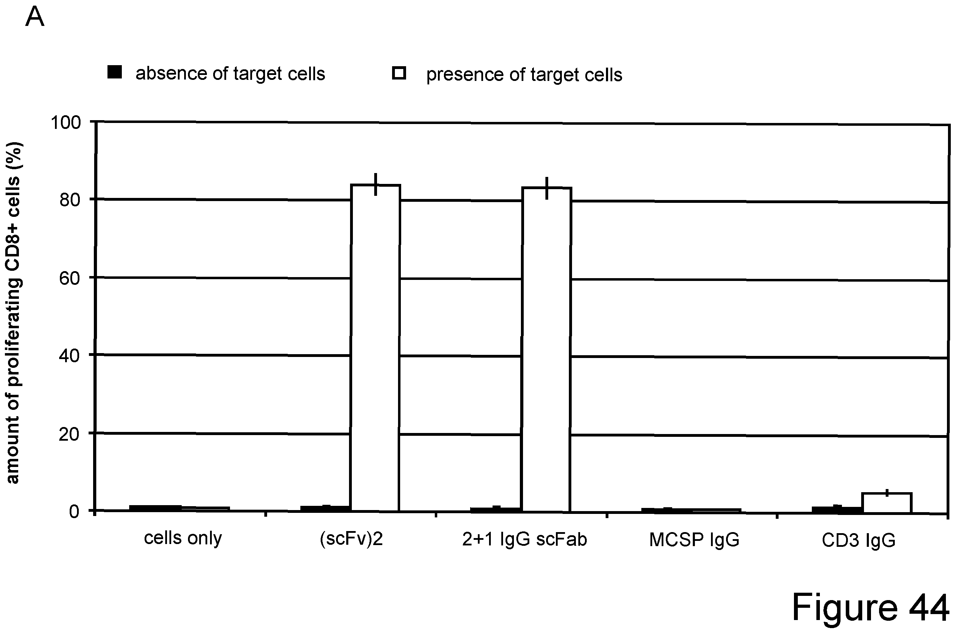

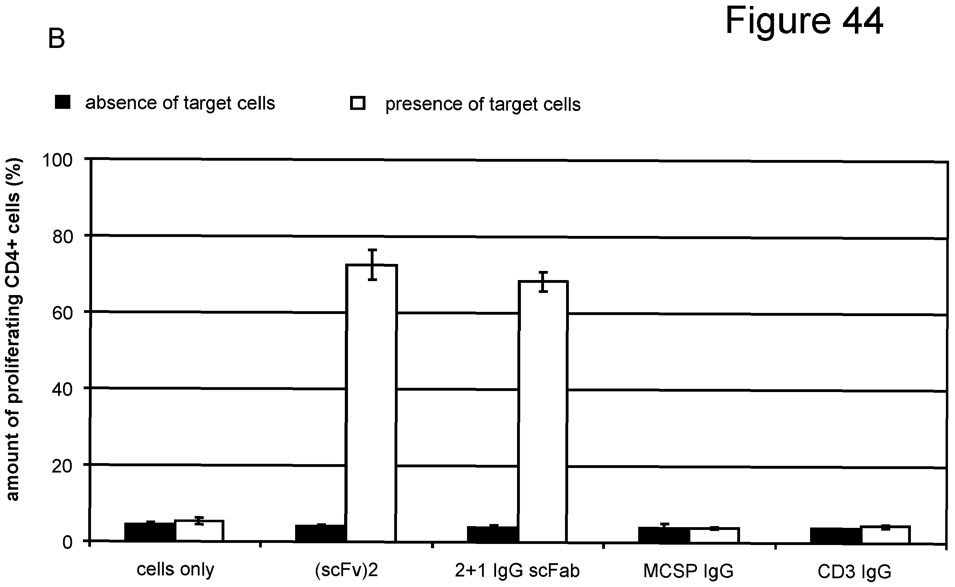

FIGURE 44. Relative proliferation of either CD8⁺ (A) or CD4⁺ (B) human T cells upon incubation with 1 nM of different CD3-MCSP bispecific constructs ("2+1 IgG scFab, LALA" (see SEQ ID NOs 5, 17, 19) or "(scFv)₂") or corresponding control IgGs in the presence or absence of Colo-38 tumor target cells at an effector to target cell ratio of 5:1. CFSE-labeled human pan T cells were characterized by FACS. The relative proliferation level was determined by setting a gate around the non-proliferating cells and using the cell number of this gate relative to the overall measured cell number as the reference.

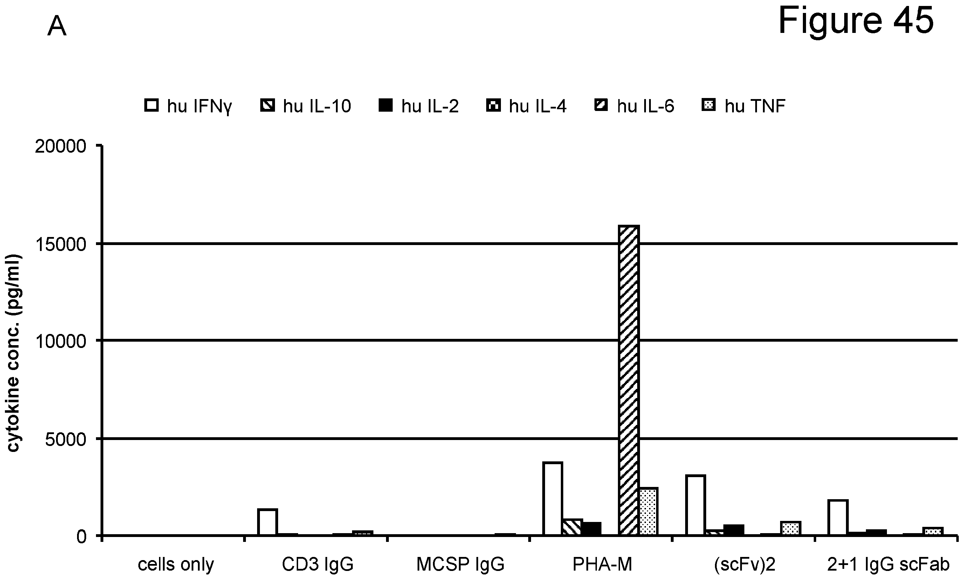

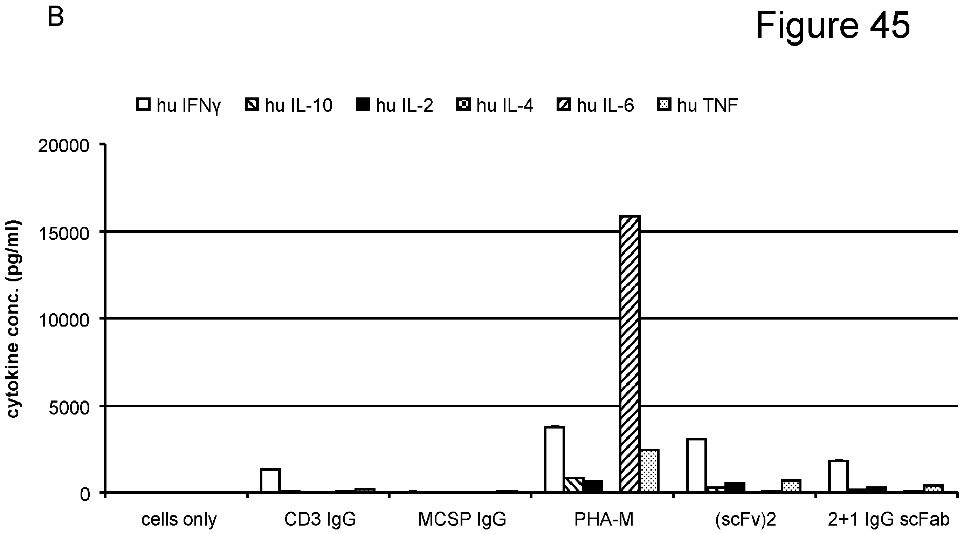

FIGURE 45. Levels of different cytokines measured in the supernatant of human PBMCs after treatment with 1 nM of different CD3-MCSP bispecific constructs ("2+1 IgG scFab, LALA" (see SEQ ID NOs 5, 17, 19) or "(scFv)₂") or corresponding control IgGs in the presence (A) or absence (B) of Colo-38 tumor cells for 24 hours. The effector to target cell ratio was 10:1.

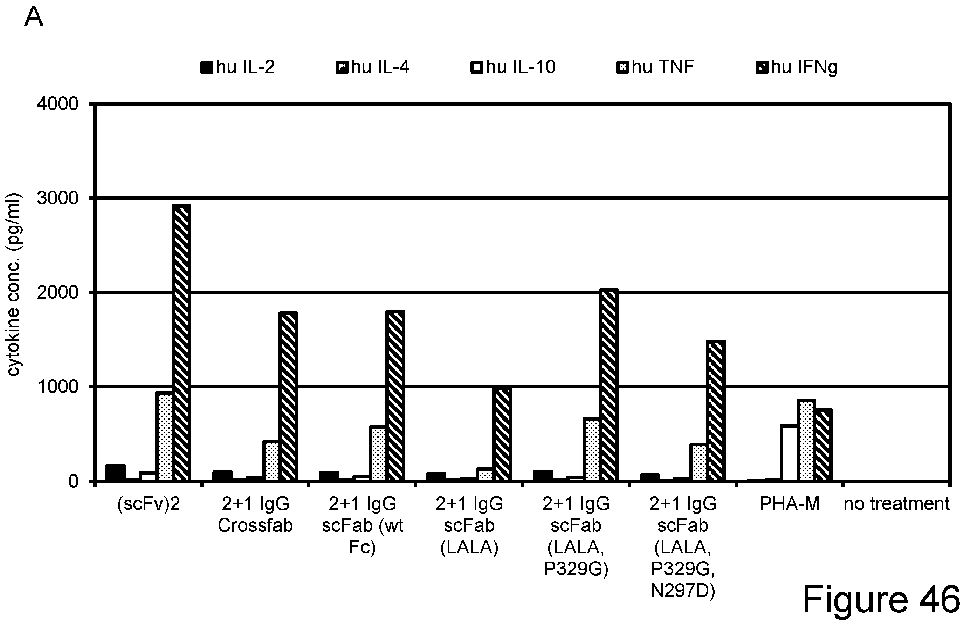

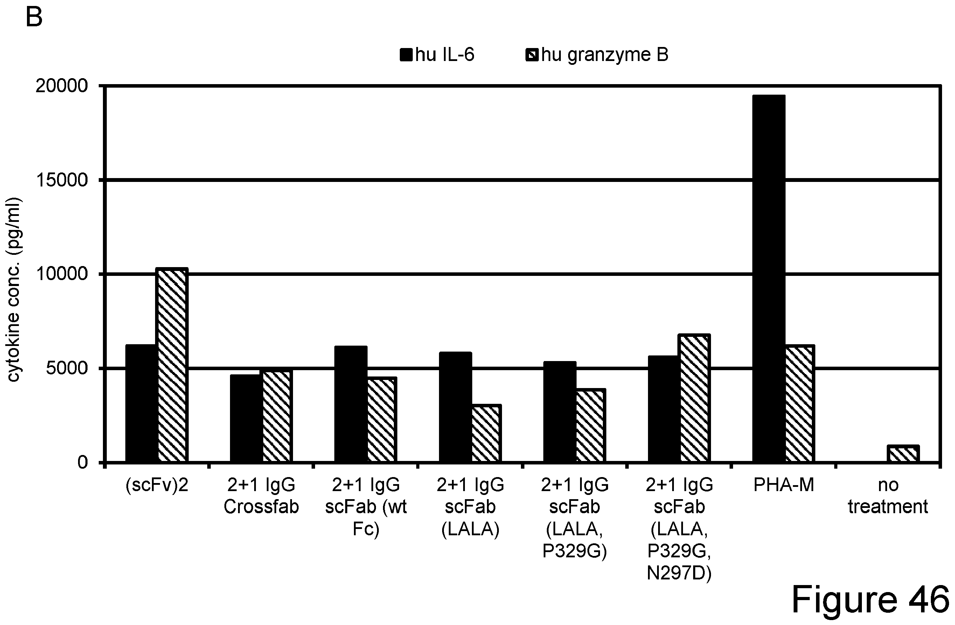

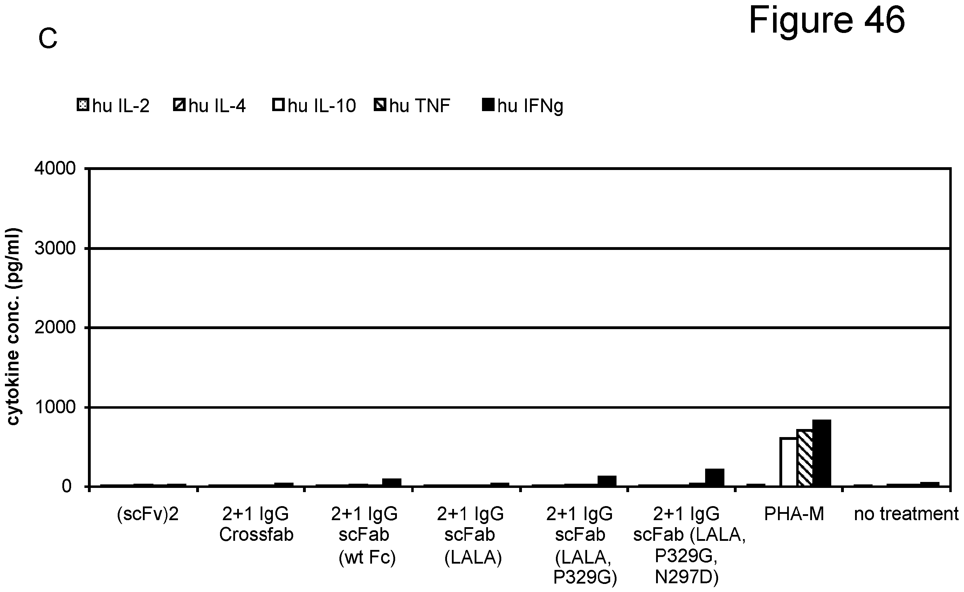

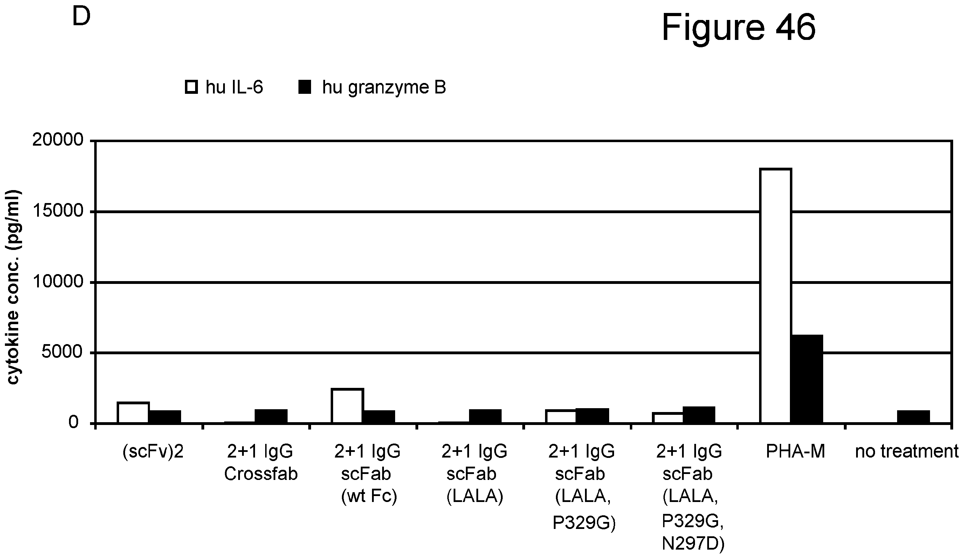

FIGURE 46. Levels of different cytokines measured in the supernatant of whole blood after treatment with 1 nM of different CD3-MCSP bispecific constructs ("2+1 IgG scFab", "2+1 IgG Crossfab" (see SEQ ID NOs 3, 5, 29, 33) or "(scFv)₂") or corresponding control IgGs in the presence (A, B) or absence (C, D) of Colo-38 tumor cells for 24 hours. Among the bispecific constructs were different "2+1 IgG scFab" constructs having either a wild-type Fc domain (see SEQ ID NOs 5, 13, 15), or an Fc domain mutated to abolish (NK) effector cell function (LALA (see SEQ ID NOs 5, 17, 19), P329G LALA (see SEQ ID NOs 5, 2, 23) and P329G LALA N297D (see SEQ ID NOs 5, 25, 27)).

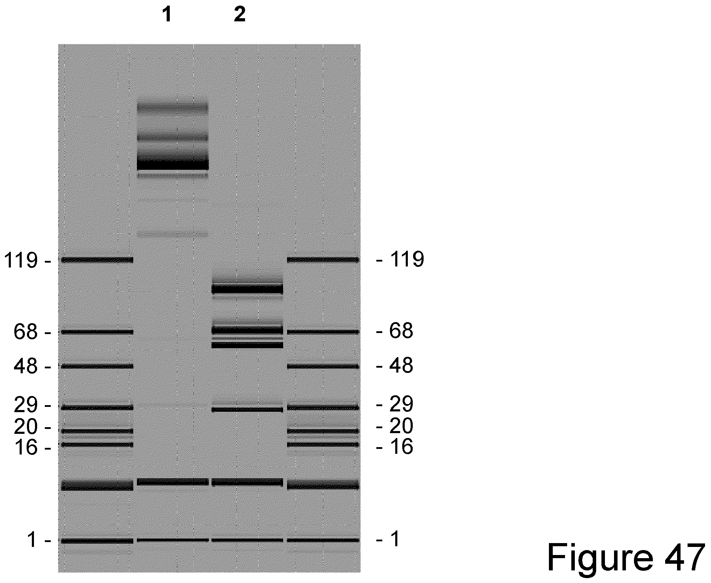

FIGURE 47. CE-SDS analyses. Electropherogram shown as SDS PAGE of 2+1 IgG Crossfab, linked light chain (see SEQ ID NOs 3, 5, 29, 179). (lane 1: reduced, lane 2: non-reduced).

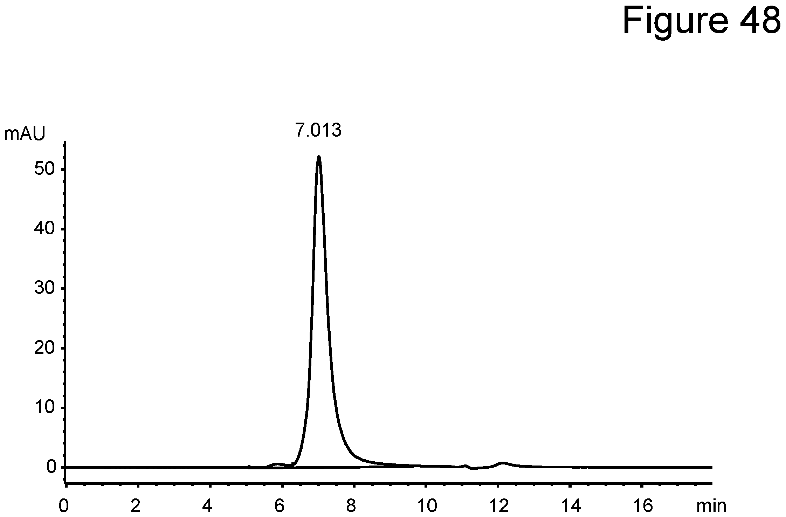

FIGURE 48. Analytical size exclusion chromatography of 2+1 IgG Crossfab, linked light chain (see SEQ ID NOs 3, 5, 29, 179) (final product). 20 µg sample were injected.

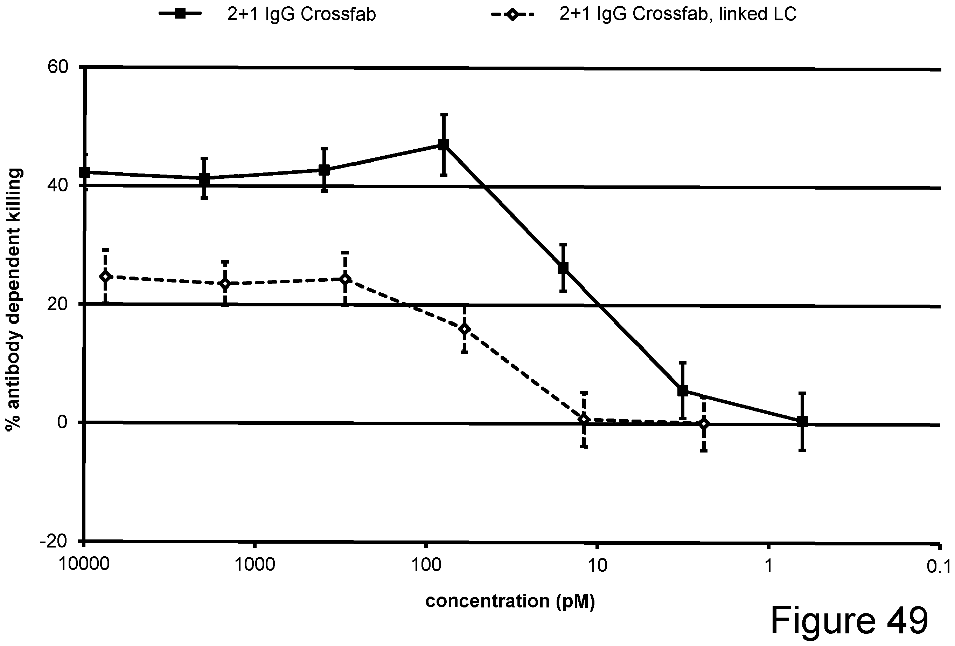

FIGURE 49. Killing (as measured by LDH release) of MCSP-positive MV-3 tumor cells upon co-culture by human PBMCs (E:T ratio = 10:1), treated with different CD3-MCSP bispecific constructs for ∼ 44 hours ("2+1 IgG Crossfab" (see SEQ ID NOs 3, 5, 29, 33) and "2+1 IgG Crossfab, linked LC" (see SEQ ID NOs 3, 5, 29, 179)). Human PBMCs were isolated from fresh blood of healthy volunteers.

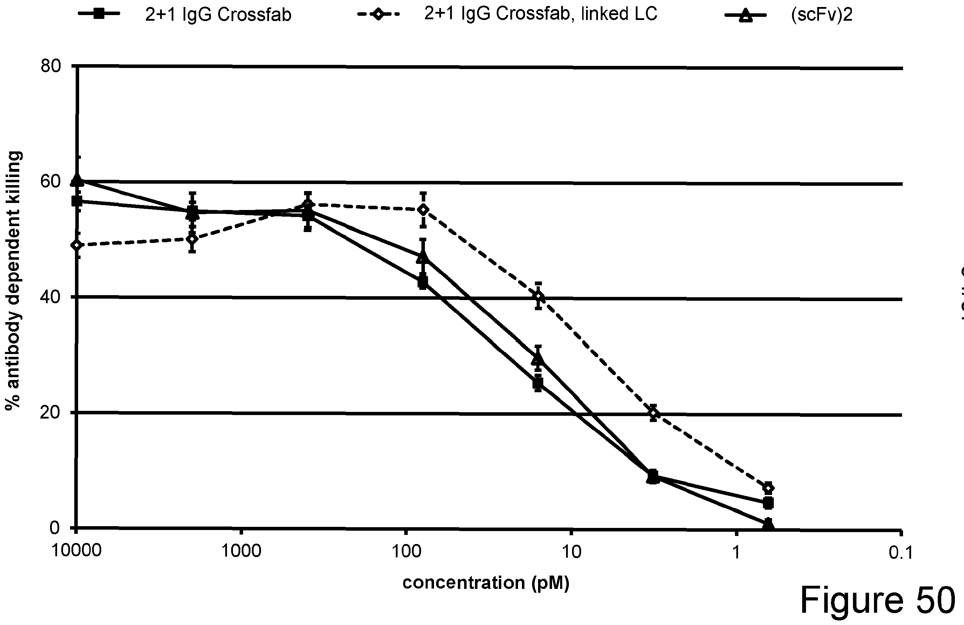

FIGURE 50. Killing (as measured by LDH release) of MCSP-positive Colo-38 tumor cells upon co-culture by human PBMCs (E:T ratio = 10:1), treated with different CD3-MCSP bispecific constructs for -22 hours ("2+1 IgG Crossfab" (see SEQ ID NOs 3, 5, 29, 33) and "2+1 IgG Crossfab, linked LC" (see SEQ ID NOs 3, 5, 29, 179)). Human PBMCs were isolated from fresh blood of healthy volunteers.

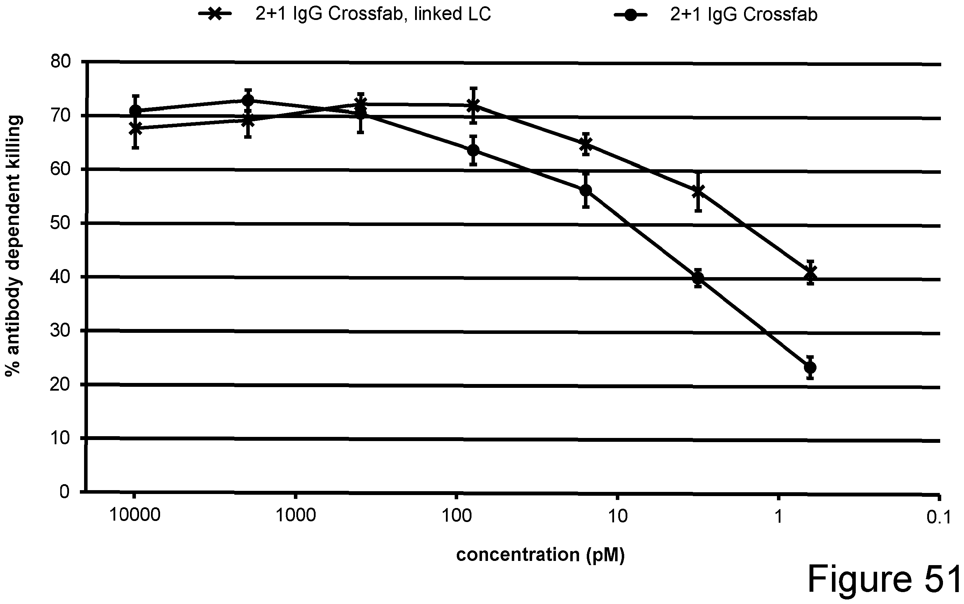

FIGURE 51. Killing (as measured by LDH release) of MCSP-positive Colo-38 tumor cells upon co-culture by human PBMCs (E:T ratio = 10:1), treated with different CD3-MCSP bispecific constructs for -22 hours ("2+1 IgG Crossfab" (see SEQ ID NOs 3, 5, 29, 33) and "2+1 IgG Crossfab, linked LC" (see SEQ ID NOs 3, 5, 29, 179)). Human PBMCs were isolated from fresh blood of healthy volunteers.

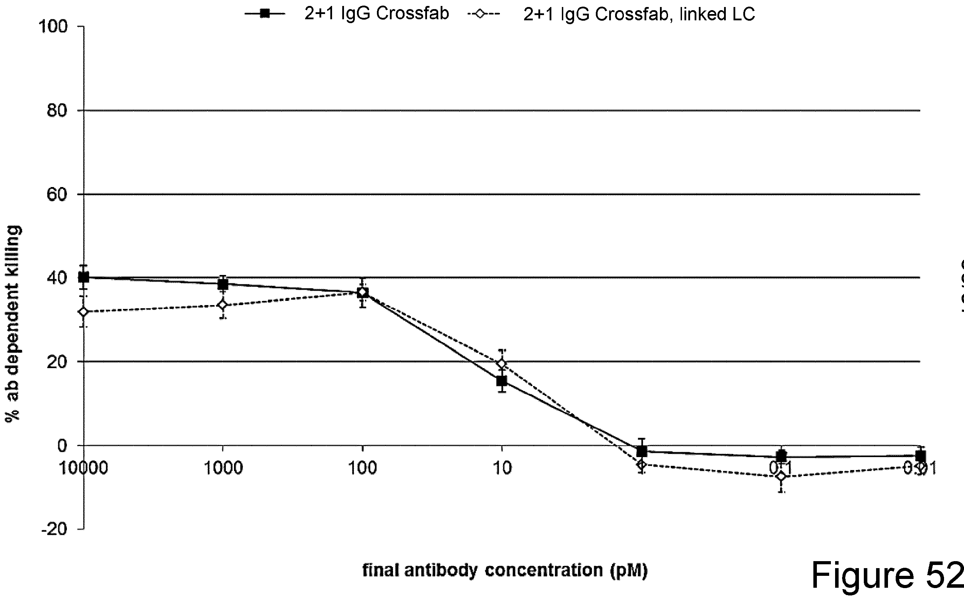

FIGURE 52. Killing (as measured by LDH release) of MCSP-positive WM266-4 cells upon co-culture by human PBMCs (E:T ratio = 10:1), treated with different CD3-MCSP bispecific constructs for -22 hours ("2+1 IgG Crossfab" (see SEQ ID NOs 3, 5, 29, 33) and "2+1 IgG Crossfab, linked LC" (see SEQ ID NOs 3, 5, 29, 179)). Human PBMCs were isolated from fresh blood of healthy volunteers.

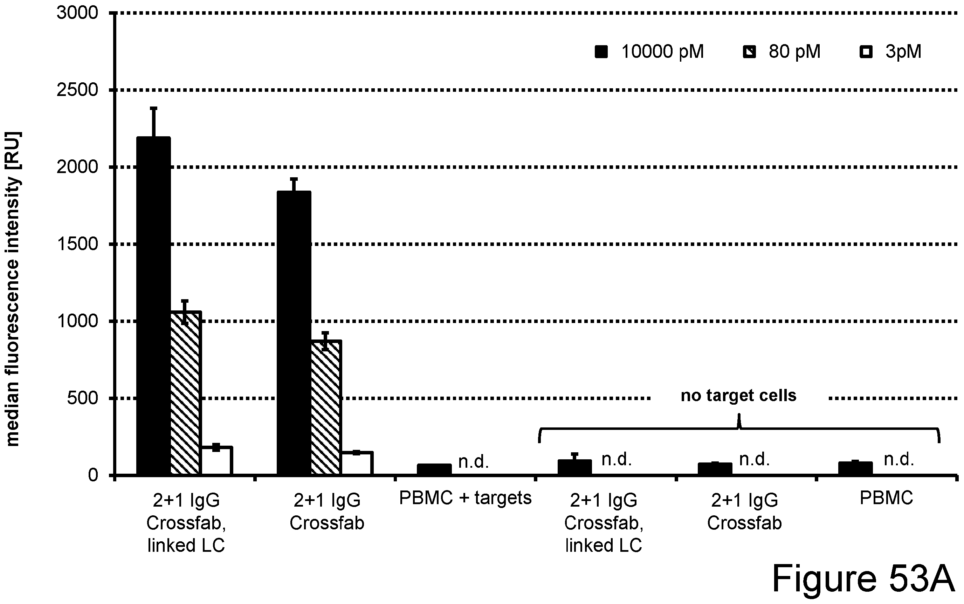

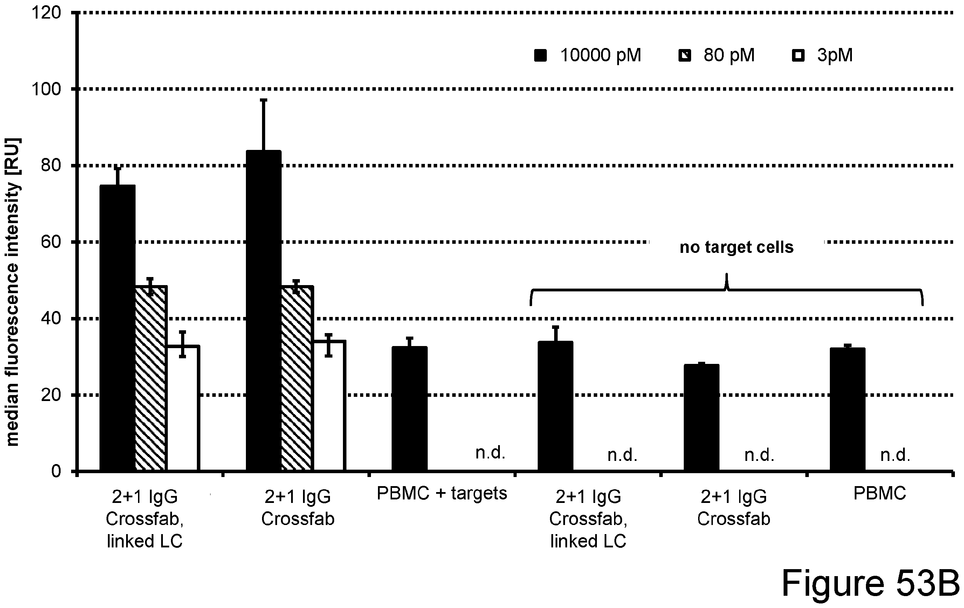

FIGURE 53. Surface expression level of the early activation marker CD69 (A) and the late activation marker CD25 (B) on human CD8⁺ T cells after 22 hours incubation with 10 nM, 80 pM or 3 pM of different CD3-MCSP bispecific constructs ("2+1 IgG Crossfab" (see SEQ ID NOs 3, 5, 29, 33) and "2+1 IgG Crossfab, linked LC" (see SEQ ID NOs 3, 5, 29, 179)) in the presence or absence of human MCSP-expressing Colo-38 tumor target cells (E:T ratio = 10:1).

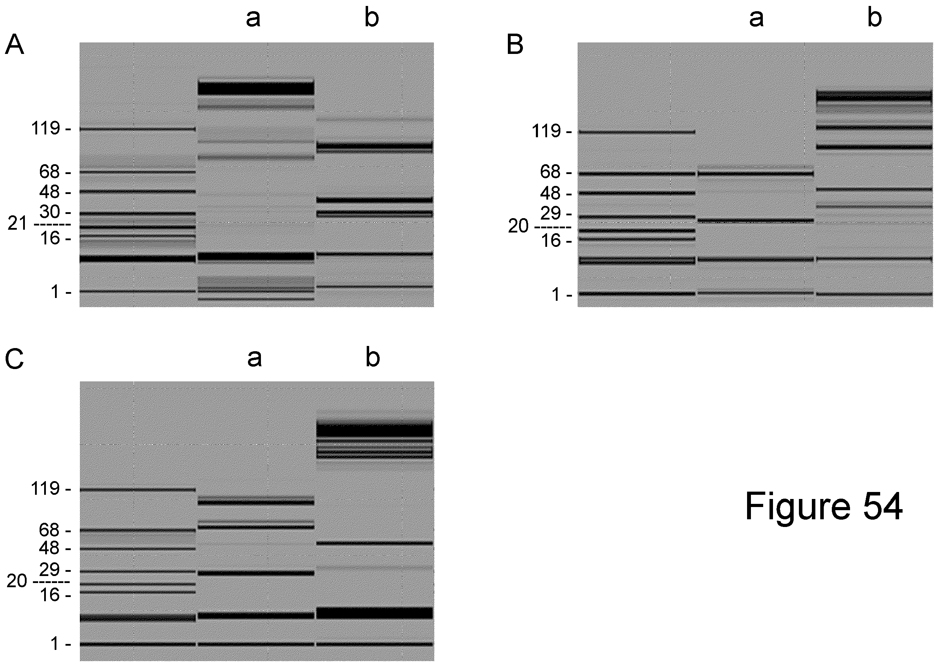

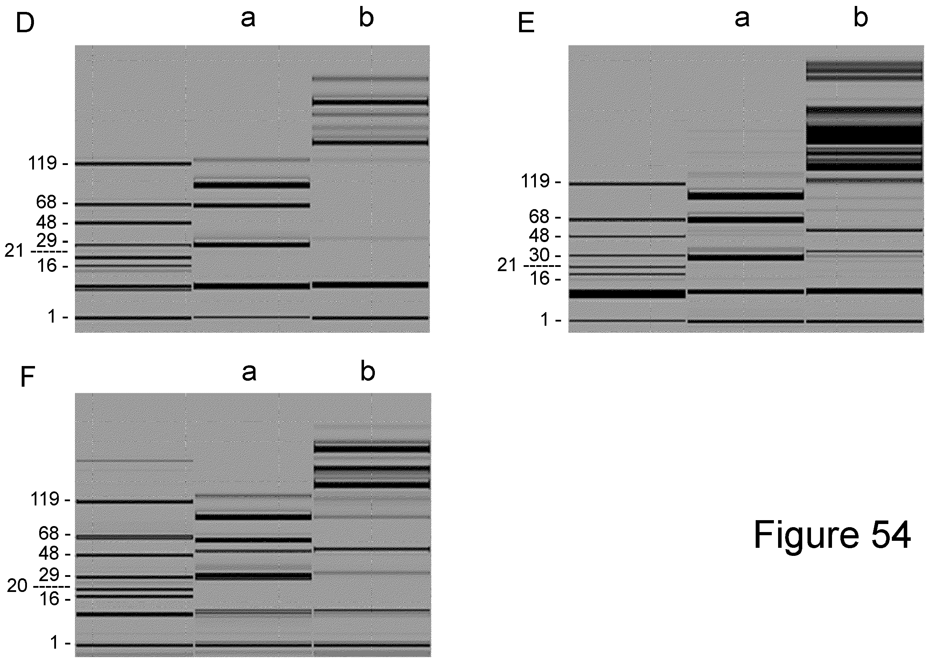

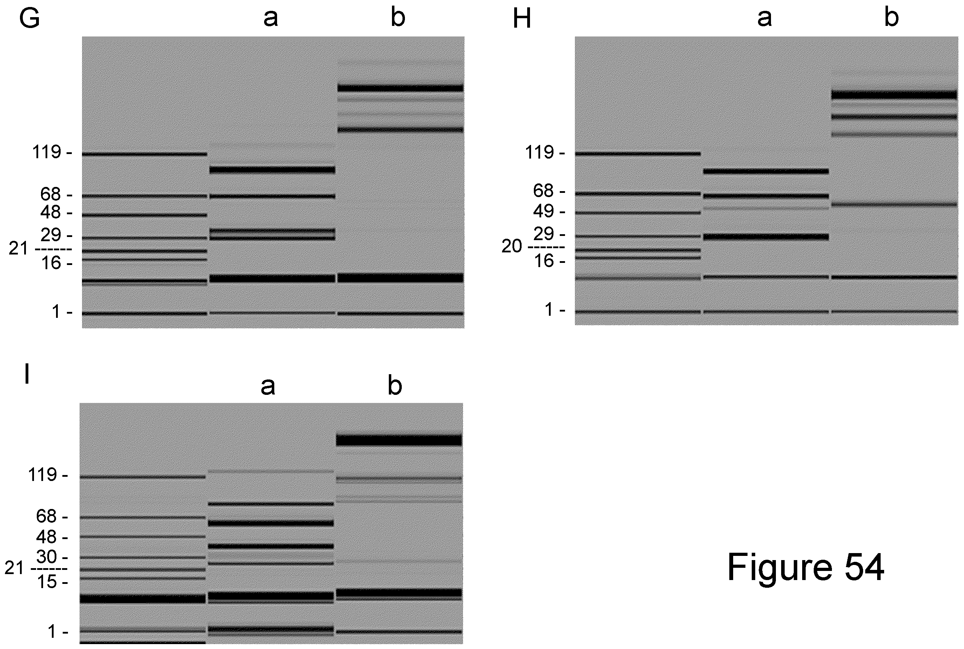

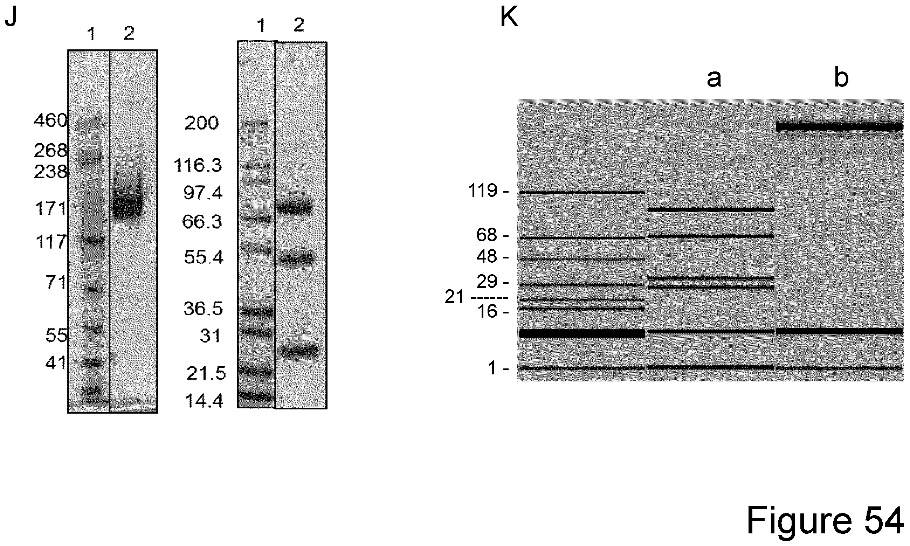

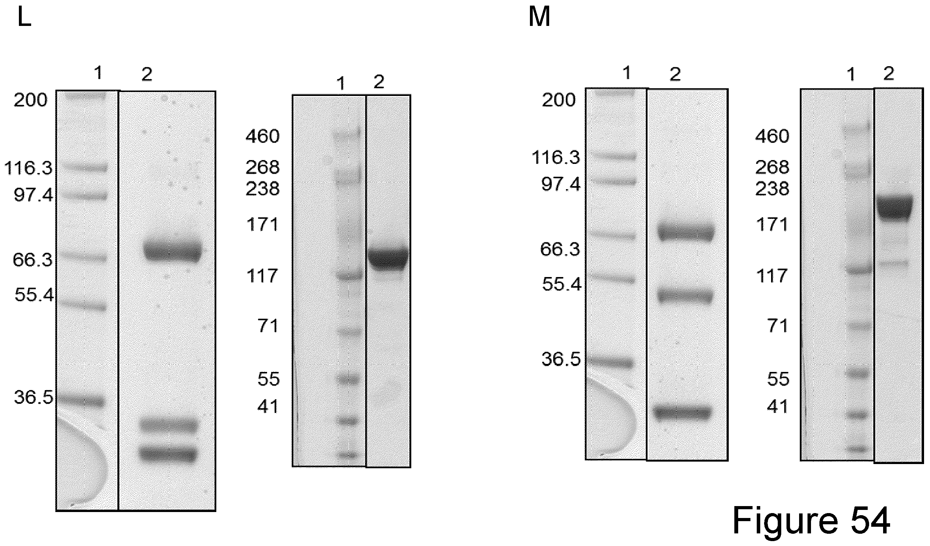

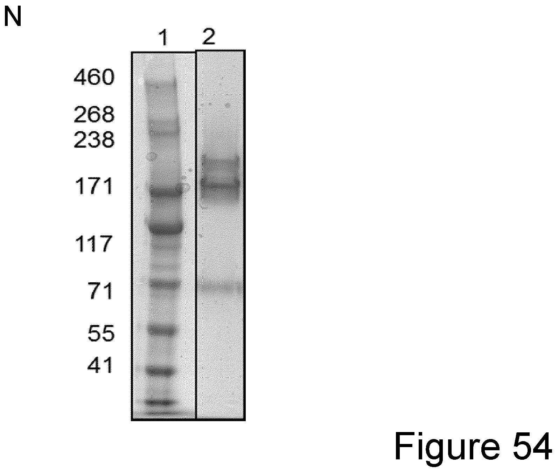

FIGURE 54. CE-SDS analyses. (A) Electropherogram shown as SDS-PAGE of 1+1 IgG Crossfab; VL/VH exchange (LC007/V9) (see SEQ ID NOs 5, 29, 33, 181): a) non-reduced, b) reduced. (B) Electropherogram shown as SDS-PAGE of 1+1 CrossMab; CL/CH1 exchange (LC007/V9) (see SEQ ID NOs 5, 23, 183, 185): a) reduced, b) non-reduced. (C) Electropherogram shown as SDS-PAGE of 2+1 IgG Crossfab, inverted; CL/CH1 exchange (LC007/V9) (see SEQ ID NOs 5, 23, 183, 187): a) reduced, b) non-reduced. (D) Electropherogram shown as SDS-PAGE of 2+1 IgG Crossfab; VL/VH exchange (M4-3 ML2/V9) (see SEQ ID NOs 33, 189, 191, 193): a) reduced, b) non-reduced. (E) Electropherogram shown as SDS-PAGE of 2+1 IgG Crossfab; CL/CH1 exchange (M4-3 ML2/V9) (see SEQ ID NOs 183, 189, 193, 195): a) reduced, b) non-reduced. (F) Electropherogram shown as SDS-PAGE of 2+1 IgG Crossfab, inverted; CL/CH1 exchange (CH1A1A/V9) (see SEQ ID NOs 65, 67, 183, 197): a) reduced, b) non-reduced. (G) Electropherogram shown as SDS-PAGE of 2+1 IgG Crossfab; CL/CH1 exchange (M4-3 ML2/H2C) (see SEQ ID NOs 189, 193, 199, 201): a) reduced, b) non-reduced. (H) Electropherogram shown as SDS-PAGE of 2+1 IgG Crossfab, inverted; CL/CH1 exchange (431/26/V9) (see SEQ ID NOs 183, 203, 205, 207): a) reduced, b) non-reduced. (I) Electropherogram shown as SDS-PAGE of "2+1 IgG Crossfab light chain fusion" (CH1A1A/V9) (see SEQ ID NOs 183, 209, 211, 213): a) reduced, b) non-reduced. (J) SDS PAGE (4-12% Bis/Tris, NuPage Invitrogen, Coomassie-stained) of "2+1 IgG Crossfab" (anti-MCSP/anti-huCD3) (see SEQ ID NOs 5, 23, 215, 217), non-reduced (left) and reduced (right). (K) Electropherogram shown as SDS-PAGE of "2+1 IgG Crossfab, inverted" (anti-MCSP/anti-huCD3) (see SEQ ID NOs 5, 23, 215, 219): a) reduced, b) non-reduced. (L) SDS PAGE (4-12% Bis/Tris, NuPage Invitrogen, Coomassie-stained) of "1+1 IgG Crossfab" (anti-CD33/anti-huCD3) (see SEQ ID NOs 33, 213, 221, 223), reduced (left) and non-reduced (right). (M) SDS PAGE (4-12% Bis/Tris, NuPage Invitrogen, Coomassie-stained) of "2+1 IgG Crossfab" (anti-CD33/anti-huCD3) (see SEQ ID NOs 33, 221, 223, 225), reduced (left) and non-reduced (right). (N) SDS PAGE (4-12% Bis/Tris, NuPage Invitrogen, Coomassie-stained) of "2+1 IgG Crossfab" (anti-CD20/anti-huCD3) (see SEQ ID NOs 33, 227, 229, 231), non-reduced.

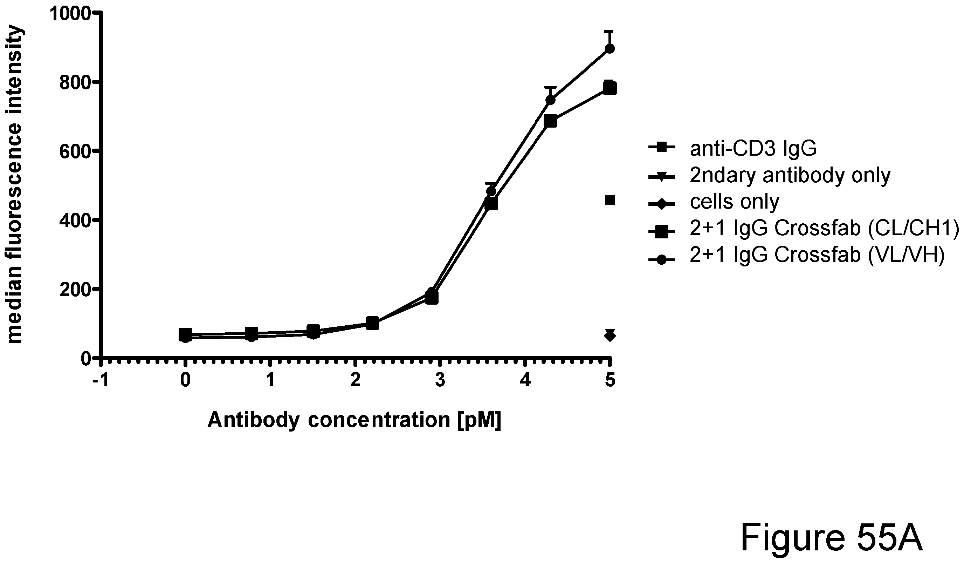

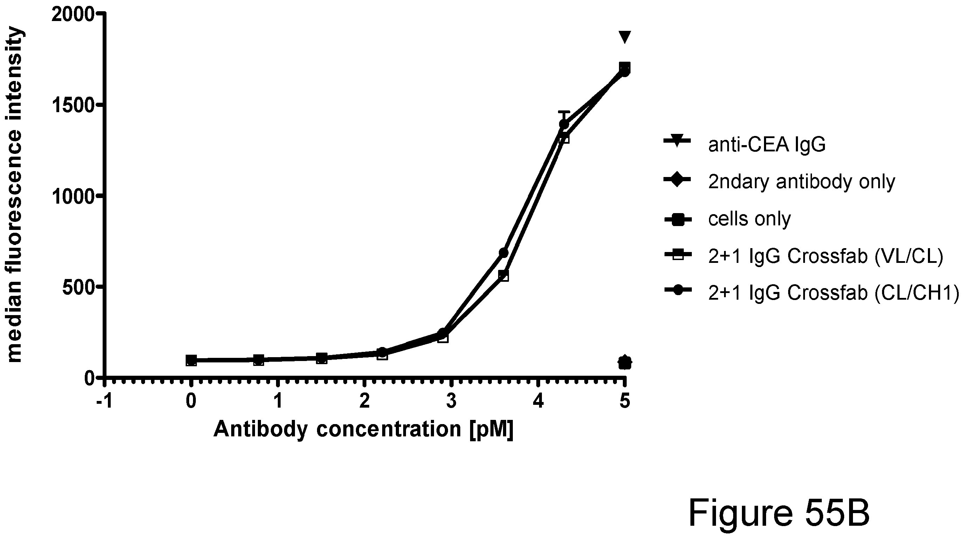

FIGURE 55. Binding of bispecific constructs (CEA/CD3 "2+1 IgG Crossfab, inverted (VL/VH)" (see SEQ ID NOs 33, 63, 65, 67) and "2+1 IgG Crossfab, inverted (CL/CH1) 2 (see SEQ ID NOs 65, 67, 183, 197)) to human CD3, expressed by Jurkat cells (A), or to human CEA, expressed by LS-174T cells (B) as determined by FACS. As a control, the equivalent maximum concentration of the reference IgGs and the background staining due to the labeled 2ndary antibody (goat anti-human FITC-conjugated AffiniPure F(ab')2 Fragment, Fcγ Fragment-specific, Jackson Immuno Research Lab # 109-096-098) were assessed as well.

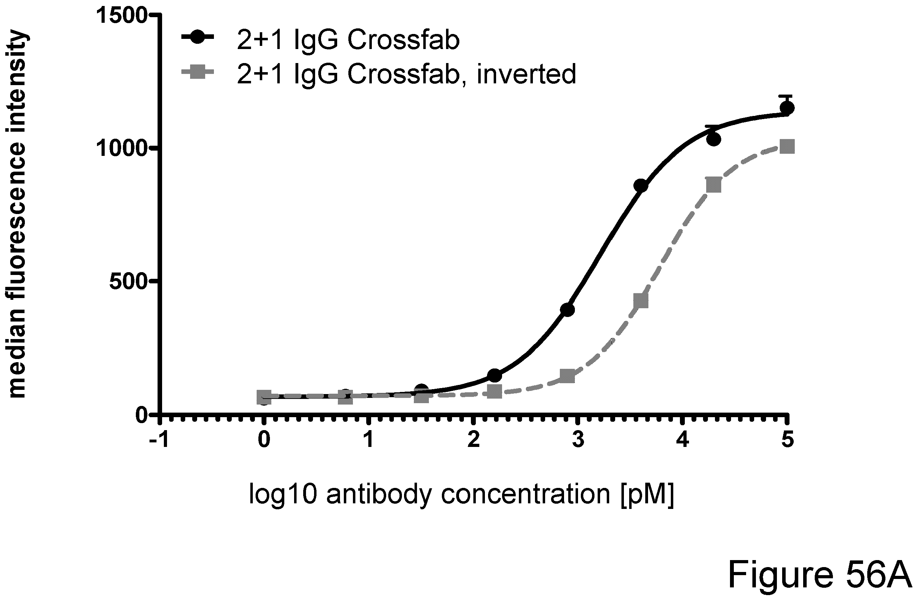

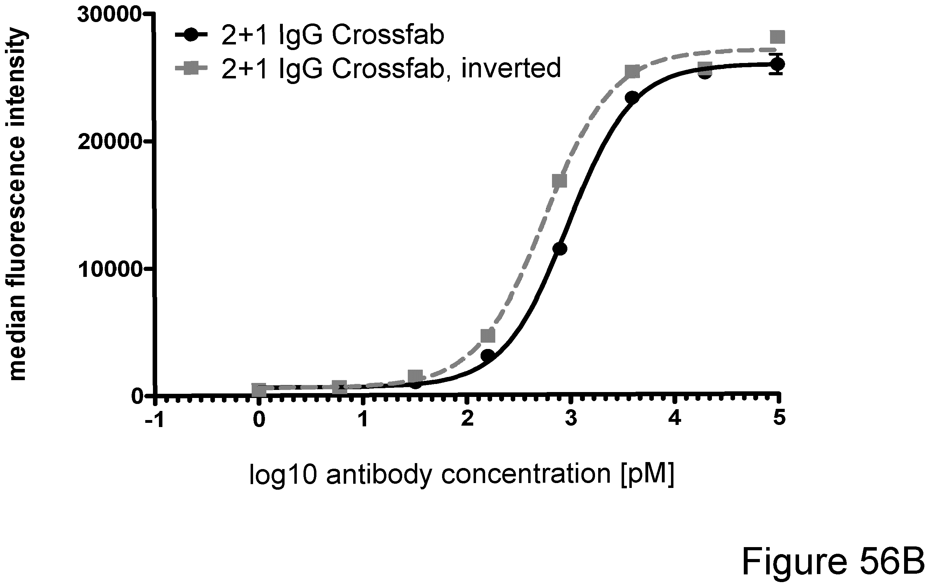

FIGURE 56. Binding of bispecific constructs constructs (MCSP/CD3 "2+1 IgG Crossfab" (see SEQ ID NOs 3, 5, 29, 33) and "2+1 IgG Crossfab, inverted" (see SEQ ID NOs 5, 23, 183, 187)) to human CD3, expressed by Jurkat cells (A), or to human MCSP, expressed by WM266-4 tumor cells (B) as determined by FACS.

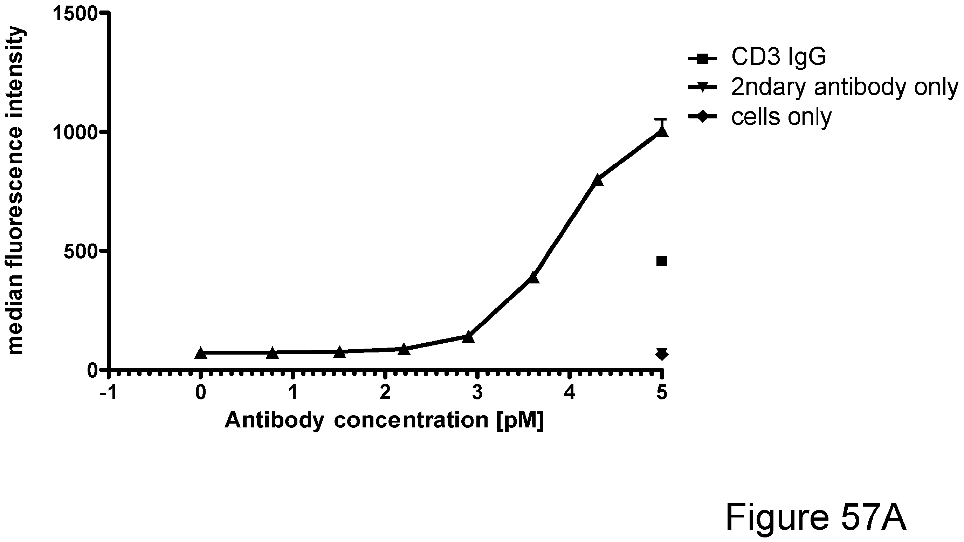

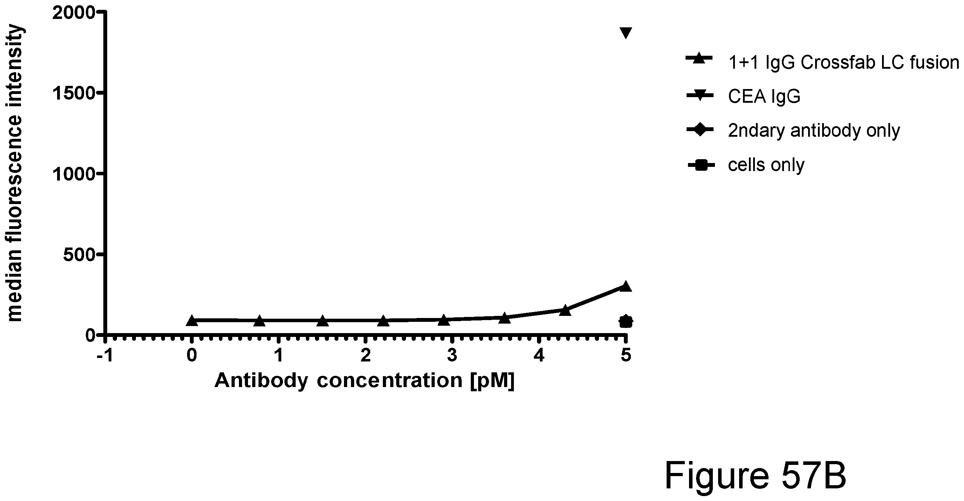

FIGURE 57. Binding of the "1+1 IgG Crossfab light chain fusion" (see SEQ ID NOs 183, 209, 211, 213) to human CD3, expressed by Jurkat cells (A), or to human CEA, expressed by LS-174T cells (B) as determined by FACS.

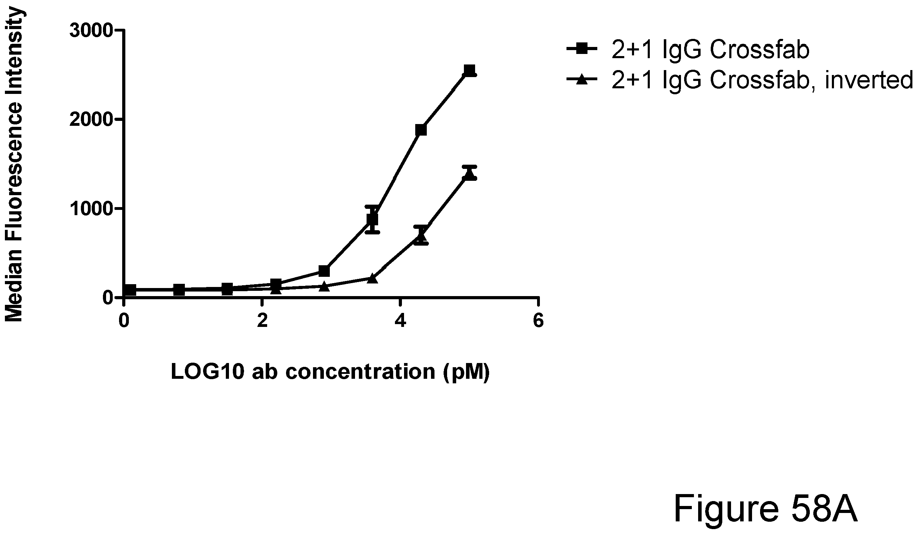

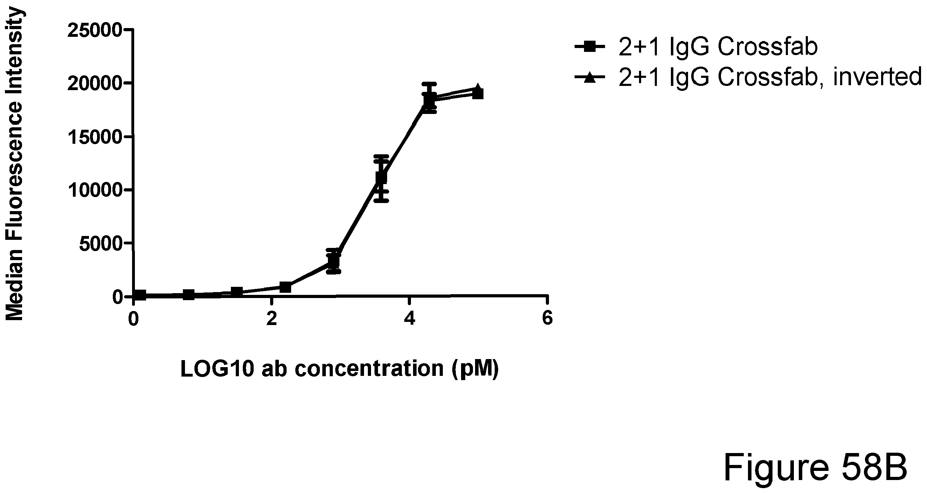

FIGURE 58. Binding of the "2+1 IgG Crossfab" (see SEQ ID NOs 5, 23, 215, 217) and the "2+1 IgG Crossfab, inverted" (see SEQ ID NOs 5, 23, 215, 219) constructs to human CD3, expressed by Jurkat cells (A), or human MCSP, expressed by WM266-4 tumor cells (B) as determined by FACS.

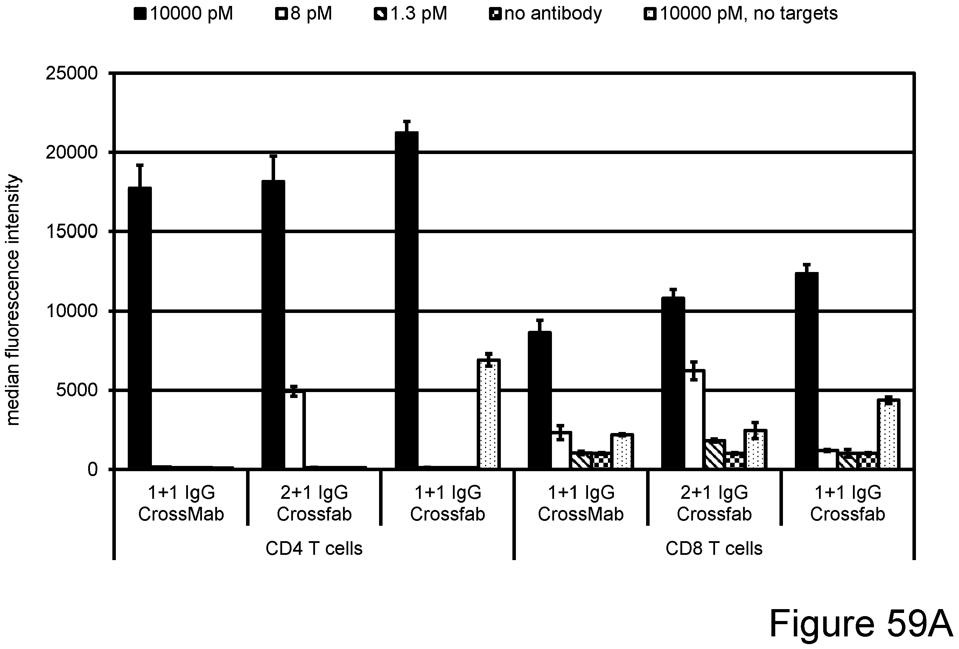

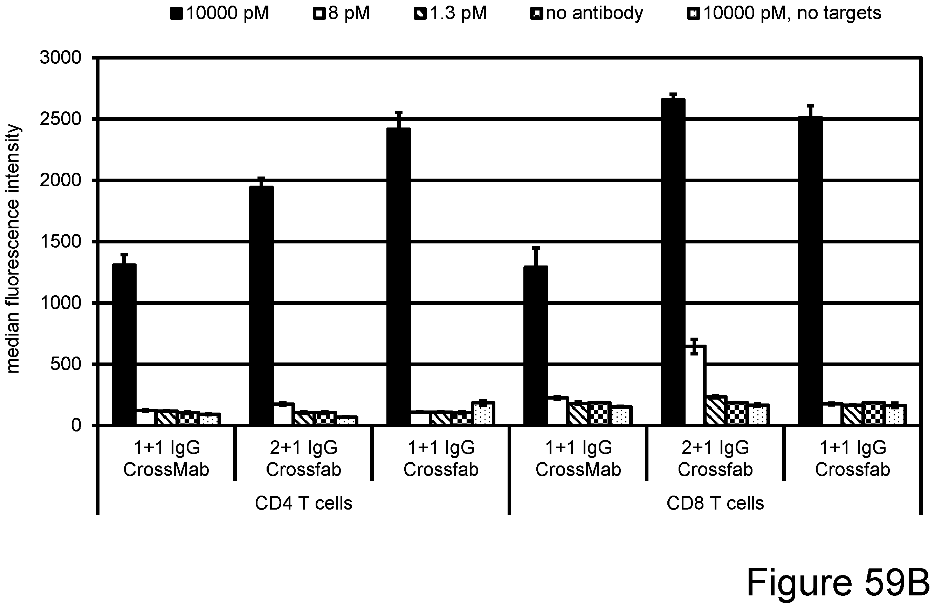

FIGURE 59. Surface expression level of the early activation marker CD69 (A) or the late activation marker CD25 (B) on human CD4⁺ or CD8⁺ T cells after 24 hours incubation with the indicated concentrations of the CD3/MCSP "1+1 CrossMab" (see SEQ ID NOs 5, 23, 183, 185), "1+1 IgG Crossfab" (see SEQ ID NOs 5, 29, 33, 181) and "2+1 IgG Crossfab" (see SEQ ID NOs 3, 5, 29, 33) constructs. The assay was performed in the presence or absence of MV-3 target cells, as indicated.

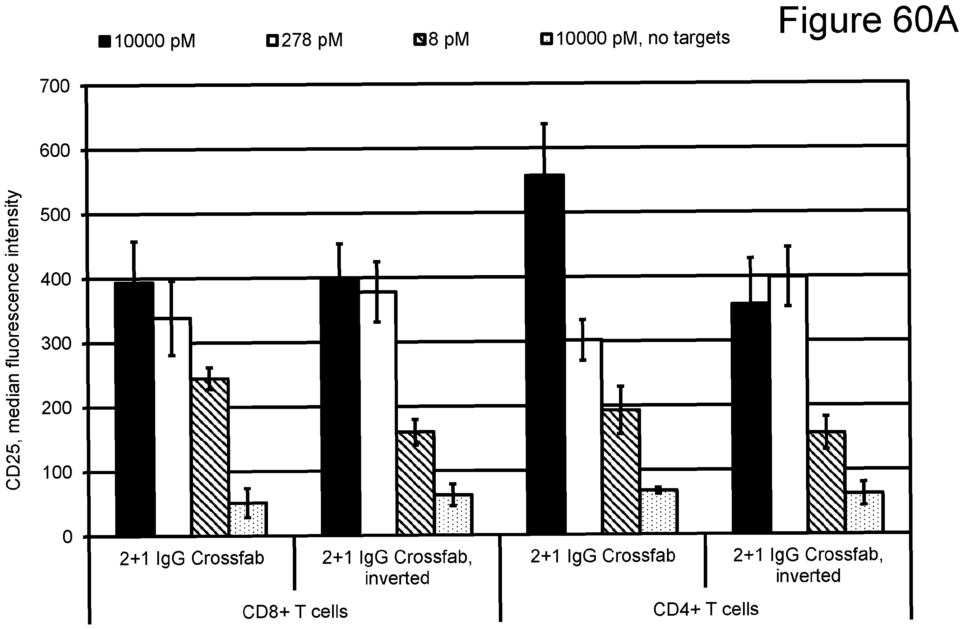

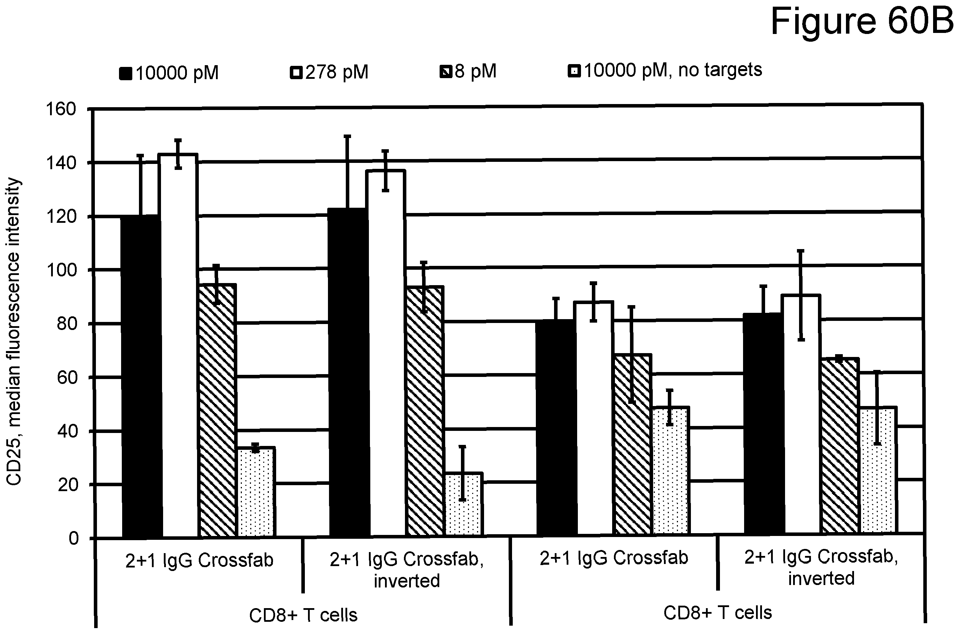

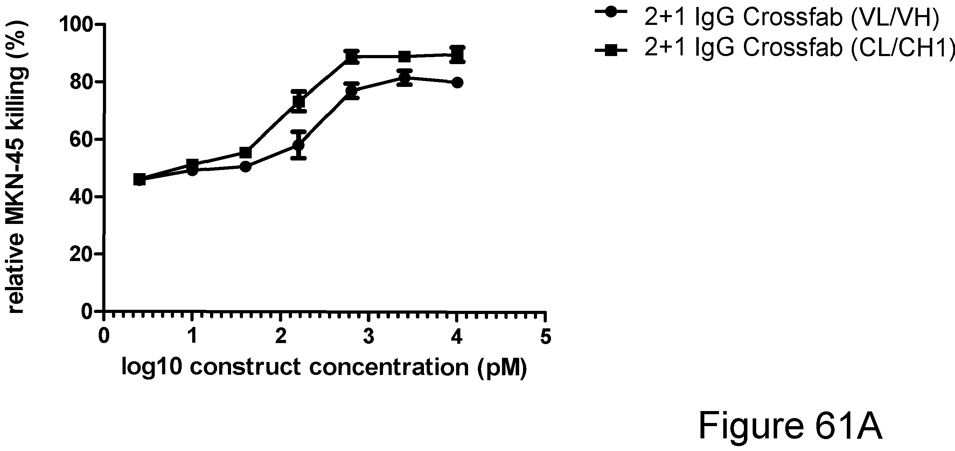

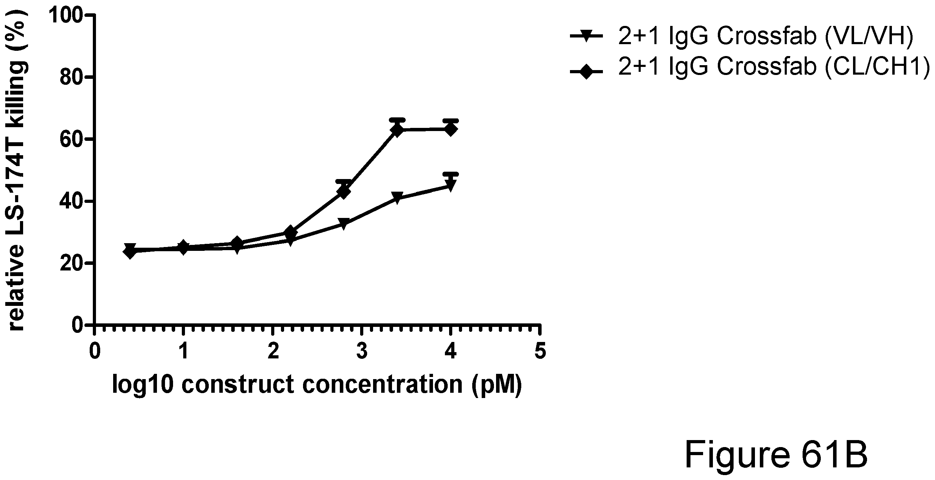

FIGURE 60. Surface expression level of the early activation marker CD25 on CD4⁺ or CD8⁺ T cells from two different cynomolgus monkeys (A and B) in the presence or absence of huMCSP-positive MV-3 tumor cells upon co-culture with cynomolgus PBMCs (E:T ratio = 3:1, normalized to CD3⁺ numbers), treated with the "2+1 IgG Crossfab" (see SEQ ID NOs 5, 23, 215, 217) and the "2+1 IgG Crossfab, inverted" (see SEQ ID NOs 5, 23, 215, 219) for ∼41 hours. FIGURE 61. Killing (as measured by LDH release) of MKN-45 (A) or LS-174T (B) tumor cells upon co-culture with human PBMCs (E:T ratio = 10:1) and activation for 28 hours by different concentrations of the "2+1 IgG Crossfab, inverted (VL/VH)" (see SEQ ID NOs 33, 63, 65, 67) versus the "2+1 IgG Crossfab, inverted (CL/CH1)" (see SEQ ID NOs 65, 67, 183, 197) construct.

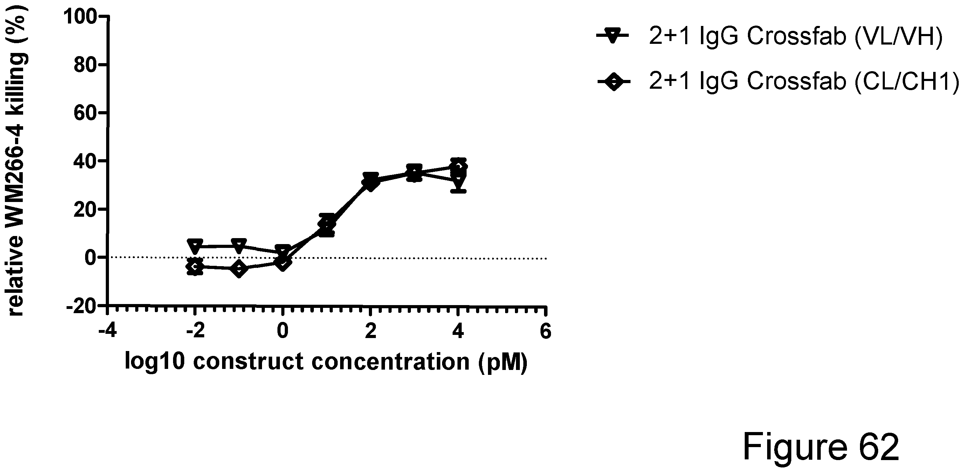

FIGURE 62. Killing (as measured by LDH release) of WM266-4 tumor cells upon co-culture with human PBMCs (E:T ratio = 10:1) and activation for 26 hours by different concentrations of the "2+1 IgG Crossfab (VL/VH)" (see SEQ ID NOs 33, 189, 191, 193) versus the "2+1 IgG Crossfab (CL/CH1)" (see SEQ ID NOs 183, 189, 193, 195) construct.

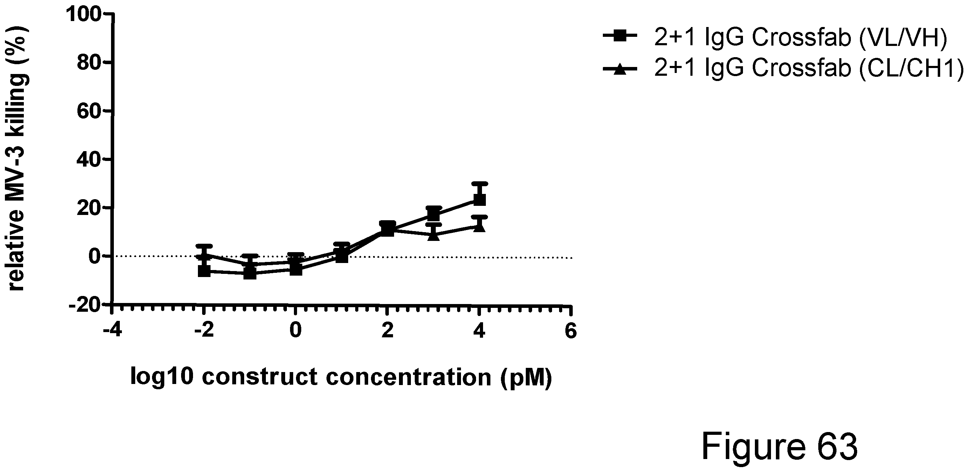

FIGURE 63. Killing (as measured by LDH release) of MV-3 tumor cells upon co-culture with human PBMCs (E:T ratio = 10:1) and activation for 27 hours by different concentrations of the "2+1 IgG Crossfab (VH/VL)" (see SEQ ID NOs 33, 189, 191, 193) versus the "2+1 IgG Crossfab (CL/CH1)" (see SEQ ID NOs 183, 189, 193, 195) constructs.

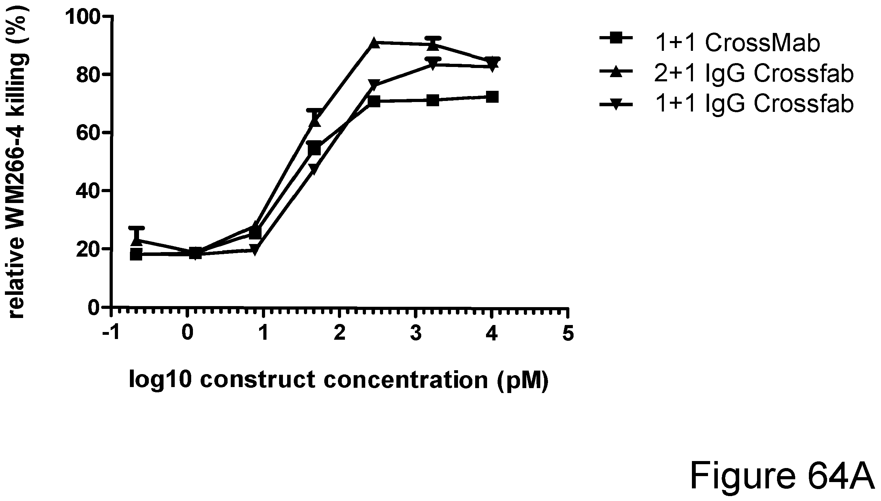

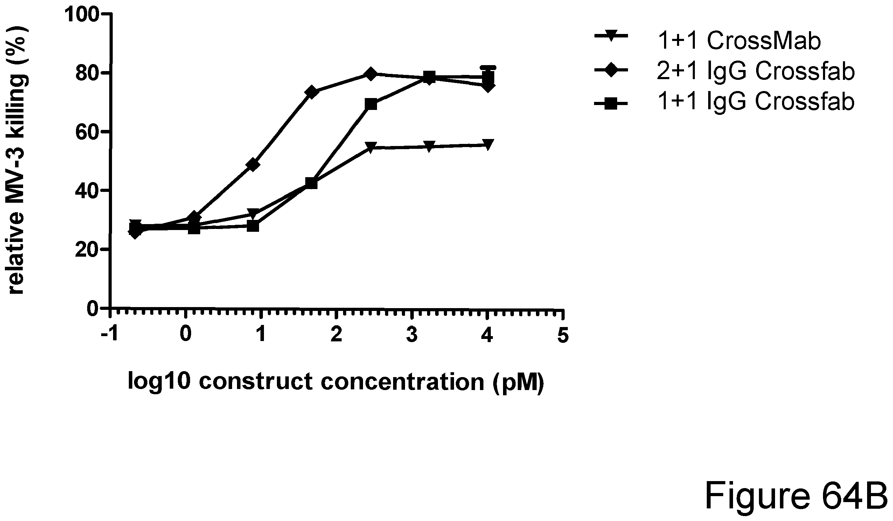

FIGURE 64. Killing (as measured by LDH release) of human MCSP-positive WM266-4 (A) or MV-3 (B) tumor cells upon co-culture with human PBMCs (E:T ratio = 10:1) and activation for 21 hours by different concentrations of the "2+1 IgG Crossfab" (see SEQ ID NOs 3, 5, 29, 33), the "1+1 CrossMab" (see SEQ ID NOs 5, 23, 183, 185), and the "1+1 IgG Crossfab" (see SEQ ID NOs 5, 29, 33, 181), as indicated.

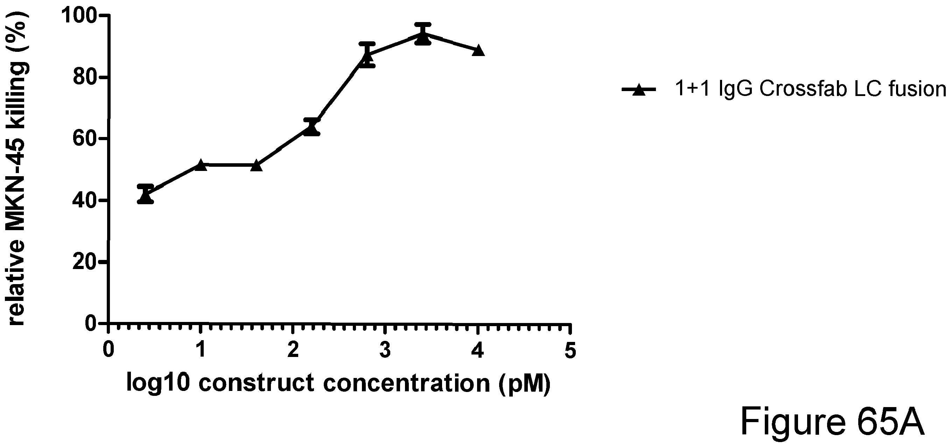

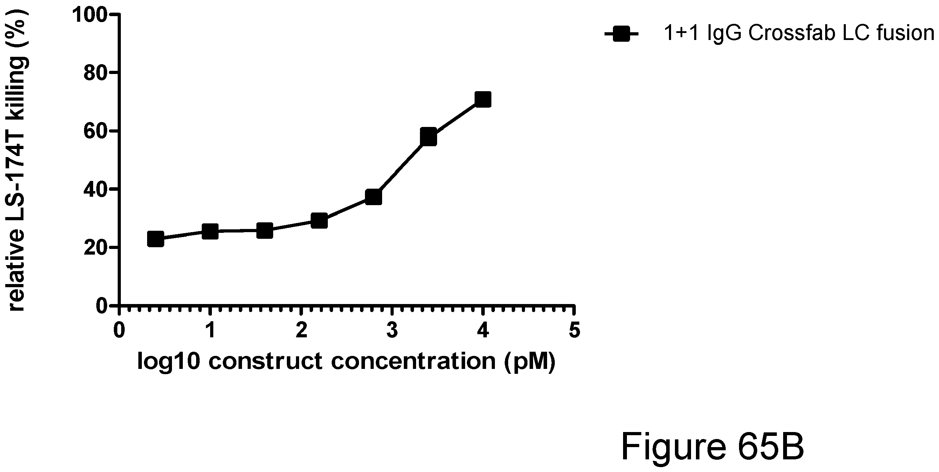

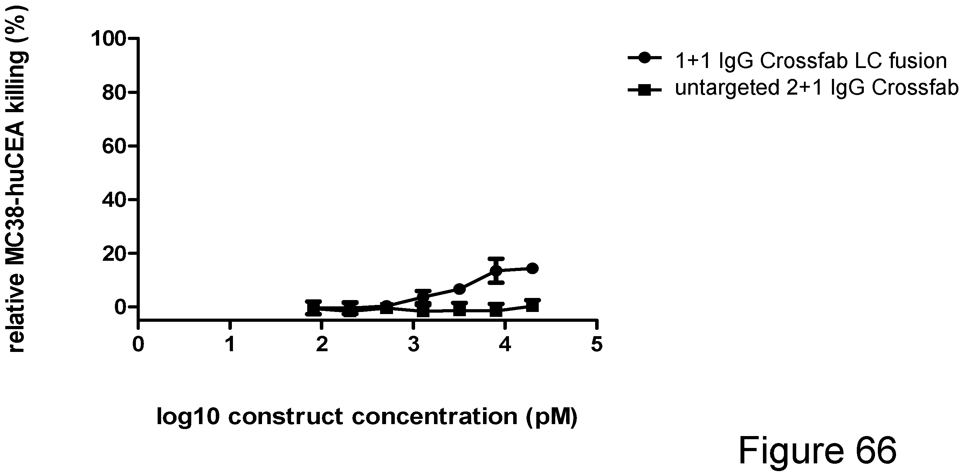

FIGURE 65. Killing (as measured by LDH release) of MKN-45 (A) or LS-174T (B) tumor cells upon co-culture with human PBMCs (E:T ratio = 10:1) and activation for 28 hours by different concentrations of the "1+1 IgG Crossfab LC fusion" (see SEQ ID NOs 183, 209, 211, 213). FIGURE 66. Killing (as measured by LDH release) of MC38-huCEA tumor cells upon co-culture with human PBMCs (E:T ratio = 10:1) and activation for 24 hours by different concentrations of the "1+1 IgG Crossfab LC fusion" (see SEQ ID NOs 183, 209, 211, 213) versus an untargeted "2+1 IgG Crossfab" reference.

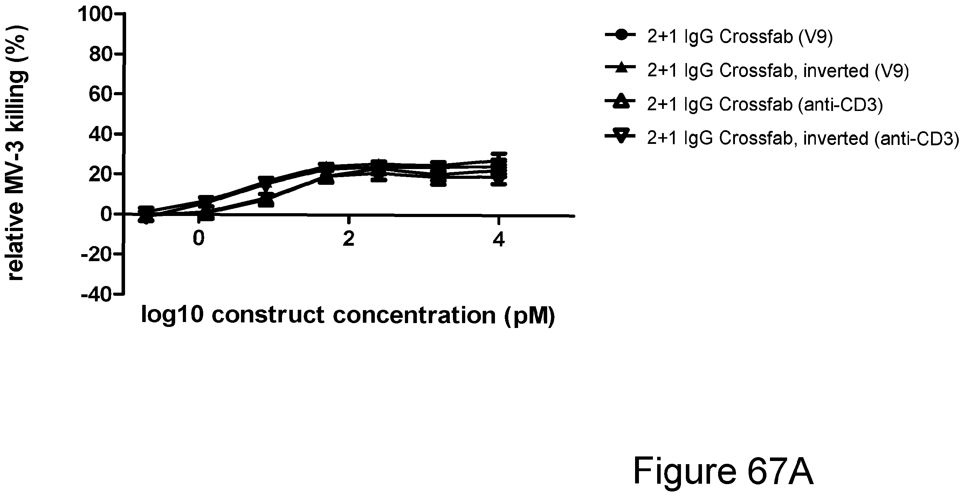

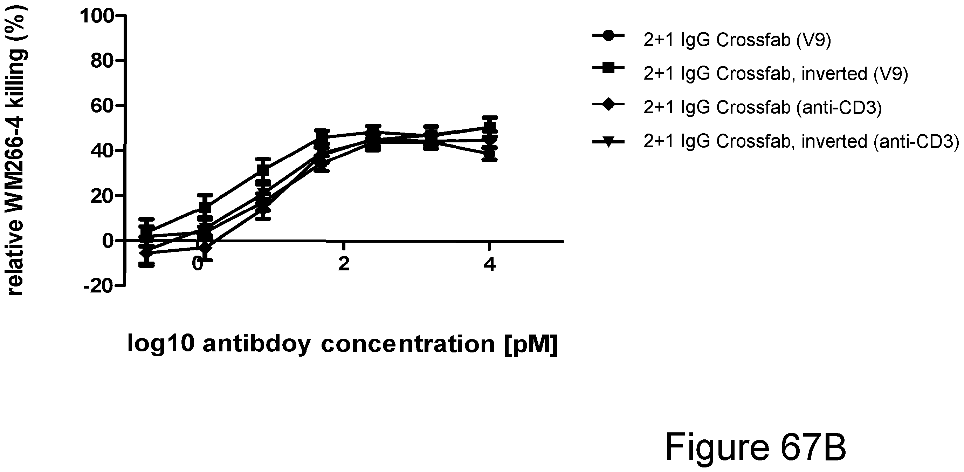

FIGURE 67. Killing (as measured by LDH release) of human MCSP-positive MV-3 (A) or WM266-4 (B) tumor cells upon co-culture with human PBMCs (E:T ratio = 10:1), treated with the "2+1 IgG Crossfab (V9)" (see SEQ ID NOs 3, 5, 29, 33) and the "2+1 IgG Crossfab, inverted (V9)" (see SEQ ID NOs 5, 23, 183, 187), the "2+1 IgG Crossfab (anti-CD3)" (see SEQ ID NOs 5, 23, 215, 217) and the "2+1 IgG Crossfab, inverted (anti-CD3)" (see SEQ ID NOs 5, 23, 215, 219) constructs.

### Detailed Description of the Invention

### Definitions

Terms are used herein as generally used in the art, unless otherwise defined in the following. As used herein, the term "antigen binding molecule" refers in its broadest sense to a molecule that specifically binds an antigenic determinant. Examples of antigen binding molecules are immunoglobulins and derivatives, e.g. fragments, thereof.

The term "bispecific" means that the antigen binding molecule is able to specifically bind to at least two distinct antigenic determinants. Typically, a bispecific antigen binding molecule comprises two antigen binding sites, each of which is specific for a different antigenic determinant. In certain embodiments the bispecific antigen binding molecule is capable of simultaneously binding two antigenic determinants, particularly two antigenic determinants expressed on two distinct cells.

The term "valent" as used herein denotes the presence of a specified number of antigen binding sites in an antigen binding molecule. As such, the term "monovalent binding to an antigen" denotes the presence of one (and not more than one) antigen binding site specific for the antigen in the antigen binding molecule.

An "antigen binding site" refers to the site, i.e. one or more amino acid residues, of an antigen binding molecule which provides interaction with the antigen. For example, the antigen binding site of an antibody comprises amino acid residues from the complementarity determining regions (CDRs). A native immunoglobulin molecule typically has two antigen binding sites, a Fab molecule typically has a single antigen binding site.

As used herein, the term "antigen binding moiety" refers to a polypeptide molecule that specifically binds to an antigenic determinant. In one embodiment, an antigen binding moiety is able to direct the entity to which it is attached (e.g. a second antigen binding moiety) to a target site, for example to a specific type of tumor cell or tumor stroma bearing the antigenic determinant. In another embodiment an antigen binding moiety is able to activate signaling through its target antigen, for example a T cell receptor complex antigen. Antigen binding moieties include antibodies and fragments thereof as further defined herein. Particular antigen binding moieties include an antigen binding domain of an antibody, comprising an antibody heavy chain variable region and an antibody light chain variable region. In certain embodiments, the antigen binding moieties may comprise antibody constant regions as further defined herein and known in the art. Useful heavy chain constant regions include any of the five isotypes: α, δ, ε, γ, or µ. Useful light chain constant regions include any of the two isotypes: κ and λ.

As used herein, the term "antigenic determinant" is synonymous with "antigen" and "epitope," and refers to a site (e.g. a contiguous stretch of amino acids or a conformational configuration made up of different regions of non-contiguous amino acids) on a polypeptide macromolecule to which an antigen binding moiety binds, forming an antigen binding moiety-antigen complex. Useful antigenic determinants can be found, for example, on the surfaces of tumor cells, on the surfaces of virus-infected cells, on the surfaces of other diseased cells, on the surface of immune cells, free in blood serum, and/or in the extracellular matrix (ECM). The proteins referred to as antigens herein (e.g. MCSP, FAP, CEA, EGFR, CD33, CD3) can be any native form the proteins from any vertebrate source, including mammals such as primates (e.g. humans) and rodents (e.g. mice and rats), unless otherwise indicated. In a particular embodiment the antigen is a human protein. Where reference is made to a specific protein herein, the term encompasses the "full-length", unprocessed protein as well as any form of the protein that results from processing in the cell. The term also encompasses naturally occurring variants of the protein, e.g. splice variants or allelic variants. Exemplary human proteins useful as antigens include, but are not limited to: Melanoma-associated Chondroitin Sulfate Proteoglycan (MCSP), also known as Chondroitin Sulfate Proteoglycan 4 (UniProt no. Q6UVK1 (version 70), NCBI RefSeq no. NP_001888.2); Fibroblast Activation Protein (FAP), also known as Seprase (Uni Prot nos. Q12884, Q86Z29, Q99998, NCBI Accession no. NP_004451); Carcinoembroynic antigen (CEA), also known as Carcinoembryonic antigen-related cell adhesion molecule 5 (UniProt no. P06731 (version 119), NCBI RefSeq no. NP_004354.2); CD33, also known as gp67 or Siglec-3 (UniProt no. P20138, NCBI Accession nos. NP_001076087, NP_001171079); Epidermal Growth Factor Receptor (EGFR), also known as ErbB-1 or Her1 (UniProt no. P0053, NCBI Accession nos. NP_958439, NP_958440), and CD3, particularly the epsilon subunit of CD3 (see UniProt no. P07766 (version 130), NCBI RefSeq no. NP_000724.1, SEQ ID NO: 265 for the human sequence; or UniProt no. Q95LI5 (version 49), NCBI GenBank no. BAB71849.1, SEQ ID NO: 266 for the cynomolgus [Macaca fascicularis] sequence). In certain embodiments the T cell activating bispecific antigen binding molecule of the invention binds to an epitope of an activating T cell antigen or a target cell antigen that is conserved among the activating T cell antigen or target antigen from different species.

By "specific binding" is meant that the binding is selective for the antigen and can be discriminated from unwanted or non-specific interactions. The ability of an antigen binding moiety to bind to a specific antigenic determinant can be measured either through an enzyme-linked immunosorbent assay (ELISA) or other techniques familiar to one of skill in the art, e.g. surface plasmon resonance (SPR) technique (analyzed on a BIAcore instrument) (Liljeblad et al., Glyco J 17, 323-329 (2000)), and traditional binding assays (Heeley, Endocr Res 28, 217-229 (2002)). In one embodiment, the extent of binding of an antigen binding moiety to an unrelated protein is less than about 10% of the binding of the antigen binding moiety to the antigen as measured, e.g., by SPR. In certain embodiments, an antigen binding moiety that binds to the antigen, or an antigen binding molecule comprising that antigen binding moiety, has a dissociation constant (K_{D}) of ≤ 1 µM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M).

"Affinity" refers to the strength of the sum total of non-covalent interactions between a single binding site of a molecule (e.g., a receptor) and its binding partner (e.g., a ligand). Unless indicated otherwise, as used herein, "binding affinity" refers to intrinsic binding affinity which reflects a 1:1 interaction between members of a binding pair (e.g., an antigen binding moiety and an antigen, or a receptor and its ligand). The affinity of a molecule X for its partner Y can generally be represented by the dissociation constant (K_{D}), which is the ratio of dissociation and association rate constants (k_{off} and kₒₙ, respectively). Thus, equivalent affinities may comprise different rate constants, as long as the ratio of the rate constants remains the same. Affinity can be measured by well established methods known in the art, including those described herein. A particular method for measuring affinity is Surface Plasmon Resonance (SPR).

"Reduced binding", for example reduced binding to an Fc receptor, refers to a decrease in affinity for the respective interaction, as measured for example by SPR. For clarity the term includes also reduction of the affinity to zero (or below the detection limit of the analytic method), i.e. complete abolishment of the interaction. Conversely, "increased binding" refers to an increase in binding affinity for the respective interaction.

An "activating T cell antigen" as used herein refers to an antigenic determinant expressed on the surface of a T lymphocyte, particularly a cytotoxic T lymphocyte, which is capable of inducing T cell activation upon interaction with an antigen binding molecule. Specifically, interaction of an antigen binding molecule with an activating T cell antigen may induce T cell activation by triggering the signaling cascade of the T cell receptor complex. In a particular embodiment the activating T cell antigen is CD3.

"T cell activation" as used herein refers to one or more cellular response of a T lymphocyte, particularly a cytotoxic T lymphocyte, selected from: proliferation, differentiation, cytokine secretion, cytotoxic effector molecule release, cytotoxic activity, and expression of activation markers. The T cell activating bispecific antigen binding molecules of the invention are capable of inducing T cell activation. Suitable assays to measure T cell activation are known in the art described herein.

A "target cell antigen" as used herein refers to an antigenic determinant presented on the surface of a target cell, for example a cell in a tumor such as a cancer cell or a cell of the tumor stroma. As used herein, the terms "first" and "second" with respect to antigen binding moieties etc., are used for convenience of distinguishing when there is more than one of each type of moiety. Use of these terms is not intended to confer a specific order or orientation of the T cell activating bispecific antigen binding molecule unless explicitly so stated.

A "Fab molecule" refers to a protein consisting of the VH and CH1 domain of the heavy chain (the "Fab heavy chain") and the VL and CL domain of the light chain (the "Fab light chain") of an immunoglobulin.

By "fused" is meant that the components (e.g. a Fab molecule and an Fc domain subunit) are linked by peptide bonds, either directly or via one or more peptide linkers.

As used herein, the term "single-chain" refers to a molecule comprising amino acid monomers linearly linked by peptide bonds. In certain embodiments, one of the antigen binding moieties is a single-chain Fab molecule, i.e. a Fab molecule wherein the Fab light chain and the Fab heavy chain are connected by a peptide linker to form a single peptide chain. In a particular such embodiment, the C-terminus of the Fab light chain is connected to the N-terminus of the Fab heavy chain in the single-chain Fab molecule.

By a "crossover" Fab molecule (also termed "Crossfab") is meant a Fab molecule wherein either the variable regions or the constant regions of the Fab heavy and light chain are exchanged, i.e. the crossover Fab molecule comprises a peptide chain composed of the light chain variable region and the heavy chain constant region, and a peptide chain composed of the heavy chain variable region and the light chain constant region. For clarity, in a crossover Fab molecule wherein the variable regions of the Fab light chain and the Fab heavy chain are exchanged, the peptide chain comprising the heavy chain constant region is referred to herein as the "heavy chain" of the crossover Fab molecule. Conversely, in a crossover Fab molecule wherein the constant regions of the Fab light chain and the Fab heavy chain are exchanged, the peptide chain comprising the heavy chain variable region is referred to herein as the "heavy chain" of the crossover Fab molecule.

The term "immunoglobulin molecule" refers to a protein having the structure of a naturally occurring antibody. For example, immunoglobulins of the IgG class are heterotetrameric glycoproteins of about 150,000 daltons, composed of two light chains and two heavy chains that are disulfide-bonded. From N- to C-terminus, each heavy chain has a variable region (VH), also called a variable heavy domain or a heavy chain variable domain, followed by three constant domains (CH1, CH2, and CH3), also called a heavy chain constant region. Similarly, from N- to C-terminus, each light chain has a variable region (VL), also called a variable light domain or a light chain variable domain, followed by a constant light (CL) domain, also called a light chain constant region. The heavy chain of an immunoglobulin may be assigned to one of five types, called α (IgA), δ (IgD), ε (IgE), γ (IgG), or µ (IgM), some of which may be further divided into subtypes, e.g. γ₁ (IgG₁), γ₂ (IgG₂), γ₃ (IgG₃), γ₄ (IgG₄), α₁ (IgA₁) and α₂ (IgA₂). The light chain of an immunoglobulin may be assigned to one of two types, called kappa (κ) and lambda (λ), based on the amino acid sequence of its constant domain. An immunoglobulin essentially consists of two Fab molecules and an Fc domain, linked via the immunoglobulin hinge region.

The term "antibody" herein is used in the broadest sense and encompasses various antibody structures, including but not limited to monoclonal antibodies, polyclonal antibodies, and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂, diabodies, linear antibodies, single-chain antibody molecules (e.g. scFv), and single-domain antibodies. For a review of certain antibody fragments, see Hudson et al., Nat Med 9, 129-134 (2003). For a review of scFv fragments, see e.g. Plückthun, in The Pharmacology of Monoclonal Antibodies, vol. 113, Rosenburg and Moore eds., Springer-Verlag, New York, pp. 269-315 (1994); see also WO 93/16185; and U.S. Patent Nos. 5,571,894 and 5,587,458. For discussion of Fab and F(ab')₂ fragments comprising salvage receptor binding epitope residues and having increased in vivo half-life, see U.S. Patent No. 5,869,046. Diabodies are antibody fragments with two antigen-binding sites that may be bivalent or bispecific. See, for example, EP 404,097; WO 1993/01161; Hudson et al., Nat Med 9, 129-134 (2003); and Hollinger et al., Proc Natl Acad Sci USA 90, 6444-6448 (1993). Triabodies and tetrabodies are also described in Hudson et al., Nat Med 9, 129-134 (2003). Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see e.g. U.S. Patent No. 6,248,516 B1). Antibody fragments can be made by various techniques, including but not limited to proteolytic digestion of an intact antibody as well as production by recombinant host cells (e.g. E. coli or phage), as described herein.

The term "antigen binding domain" refers to the part of an antibody that comprises the area which specifically binds to and is complementary to part or all of an antigen. An antigen binding domain may be provided by, for example, one or more antibody variable domains (also called antibody variable regions). Particularly, an antigen binding domain comprises an antibody light chain variable region (VL) and an antibody heavy chain variable region (VH).

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). See, e.g., Kindt et al., Kuby Immunology, 6th ed., W.H. Freeman and Co., page 91 (2007). A single VH or VL domain may be sufficient to confer antigen-binding specificity.

The term "hypervariable region" or "HVR", as used herein, refers to each of the regions of an antibody variable domain which are hypervariable in sequence and/or form structurally defined loops ("hypervariable loops"). Generally, native four-chain antibodies comprise six HVRs; three in the VH (H1, H2, H3), and three in the VL (L1, L2, L3). HVRs generally comprise amino acid residues from the hypervariable loops and/or from the complementarity determining regions (CDRs), the latter being of highest sequence variability and/or involved in antigen recognition. With the exception of CDR1 in VH, CDRs generally comprise the amino acid residues that form the hypervariable loops. Hypervariable regions (HVRs) are also referred to as "complementarity determining regions" (CDRs), and these terms are used herein interchangeably in reference to portions of the variable region that form the antigen binding regions. This particular region has been described by Kabat et al., U.S. Dept. of Health and Human Services, Sequences of Proteins of Immunological Interest (1983) and by Chothia et al., J Mol Biol 196:901-917 (1987), where the definitions include overlapping or subsets of amino acid residues when compared against each other. Nevertheless, application of either definition to refer to a CDR of an antibody or variants thereof is intended to be within the scope of the term as defined and used herein. The appropriate amino acid residues which encompass the CDRs as defined by each of the above cited references are set forth below in Table 1 as a comparison. The exact residue numbers which encompass a particular CDR will vary depending on the sequence and size of the CDR. Those skilled in the art can routinely determine which residues comprise a particular CDR given the variable region amino acid sequence of the antibody.

**TABLE 1. CDR Definitions¹**

| **CDR** | **Kabat** | **Chothia** | **AbM²** |
|---|---|---|---|
| V_{H} CDR1 | 31-35 | 26-32 | 26-35 |
| V_{H} CDR2 | 50-65 | 52-58 | 50-58 |
| V_{H} CDR3 | 95-102 | 95-102 | 95-102 |
| V_{L} CDR1 | 24-34 | 26-32 | 24-34 |
| V_{L} CDR2 | 50-56 | 50-52 | 50-56 |
| V_{L} CDR3 | 89-97 | 91-96 | 89-97 |

| | | | |
|---|---|---|---|
| ¹ Numbering of all CDR definitions in Table 1 is according to the numbering conventions set forth by Kabat et al. (see below). ² "AbM" with a lowercase "b" as used in Table 1 refers to the CDRs as defined by Oxford Molecular's "AbM" antibody modeling software. | | | |

Kabat et al. also defined a numbering system for variable region sequences that is applicable to any antibody. One of ordinary skill in the art can unambiguously assign this system of "Kabat numbering" to any variable region sequence, without reliance on any experimental data beyond the sequence itself. As used herein, "Kabat numbering" refers to the numbering system set forth by Kabat et al., U.S. Dept. of Health and Human Services, "Sequence of Proteins of Immunological Interest" (1983). Unless otherwise specified, references to the numbering of specific amino acid residue positions in an antibody variable region are according to the Kabat numbering system.

The polypeptide sequences of the sequence listing (i.e., SEQ ID NOs 1, 3, 5, 7, 9, 11, 13, 15 etc.) are not numbered according to the Kabat numbering system. However, it is well within the ordinary skill of one in the art to convert the numbering of the sequences of the Sequence Listing to Kabat numbering.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The "class" of an antibody or immunoglobulin refers to the type of constant domain or constant region possessed by its heavy chain. There are five major classes of antibodies: IgA, IgD, IgE, IgG, and IgM, and several of these may be further divided into subclasses (isotypes), e.g., IgG₁, IgG₂, IgG₃, IgG₄, IgA₁, and IgA₂. The heavy chain constant domains that correspond to the different classes of immunoglobulins are called α, δ, ε, γ, and µ, respectively.

The term "Fc domain" or "Fc region" herein is used to define a C-terminal region of an immunoglobulin heavy chain that contains at least a portion of the constant region. The term includes native sequence Fc regions and variant Fc regions. Although the boundaries of the Fc region of an IgG heavy chain might vary slightly, the human IgG heavy chain Fc region is usually defined to extend from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991. A "subunit" of an Fc domain as used herein refers to one of the two polypeptides forming the dimeric Fc domain, i.e. a polypeptide comprising C-terminal constant regions of an immunoglobulin heavy chain, capable of stable self-association. For example, a subunit of an IgG Fc domain comprises an IgG CH2 and an IgG CH3 constant domain.

A "modification promoting the association of the first and the second subunit of the Fc domain" is a manipulation of the peptide backbone or the post-translational modifications of an Fc domain subunit that reduces or prevents the association of a polypeptide comprising the Fc domain subunit with an identical polypeptide to form a homodimer. A modification promoting association as used herein particularly includes separate modifications made to each of the two Fc domain subunits desired to associate (i.e. the first and the second subunit of the Fc domain), wherein the modifications are complementary to each other so as to promote association of the two Fc domain subunits. For example, a modification promoting association may alter the structure or charge of one or both of the Fc domain subunits so as to make their association sterically or electrostatically favorable, respectively. Thus, (hetero)dimerization occurs between a polypeptide comprising the first Fc domain subunit and a polypeptide comprising the second Fc domain subunit, which might be non-identical in the sense that further components fused to each of the subunits (e.g. antigen binding moieties) are not the same. In some embodiments the modification promoting association comprises an amino acid mutation in the Fc domain, specifically an amino acid substitution. In a particular embodiment, the modification promoting association comprises a separate amino acid mutation, specifically an amino acid substitution, in each of the two subunits of the Fc domain.

The term "effector functions" refers to those biological activities attributable to the Fc region of an antibody, which vary with the antibody isotype. Examples of antibody effector functions include: C1q binding and complement dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake by antigen presenting cells, down regulation of cell surface receptors (e.g. B cell receptor), and B cell activation.

As used herein, the terms "engineer, engineered, engineering", are considered to include any manipulation of the peptide backbone or the post-translational modifications of a naturally occurring or recombinant polypeptide or fragment thereof. Engineering includes modifications of the amino acid sequence, of the glycosylation pattern, or of the side chain group of individual amino acids, as well as combinations of these approaches.

The term "amino acid mutation" as used herein is meant to encompass amino acid substitutions, deletions, insertions, and modifications. Any combination of substitution, deletion, insertion, and modification can be made to arrive at the final construct, provided that the final construct possesses the desired characteristics, e.g., reduced binding to an Fc receptor, or increased association with another peptide. Amino acid sequence deletions and insertions include amino- and/or carboxy-terminal deletions and insertions of amino acids. Particular amino acid mutations are amino acid substitutions. For the purpose of altering e.g. the binding characteristics of an Fc region, non-conservative amino acid substitutions, i.e. replacing one amino acid with another amino acid having different structural and/or chemical properties, are particularly preferred. Amino acid substitutions include replacement by non-naturally occurring amino acids or by naturally occurring amino acid derivatives of the twenty standard amino acids (e.g. 4-hydroxyproline, 3-methylhistidine, ornithine, homoserine, 5-hydroxylysine). Amino acid mutations can be generated using genetic or chemical methods well known in the art. Genetic methods may include site-directed mutagenesis, PCR, gene synthesis and the like. It is contemplated that methods of altering the side chain group of an amino acid by methods other than genetic engineering, such as chemical modification, may also be useful. Various designations may be used herein to indicate the same amino acid mutation. For example, a substitution from proline at position 329 of the Fc domain to glycine can be indicated as 329G, G329, G₃₂₉, P329G, or Pro329Gly.

As used herein, term "polypeptide" refers to a molecule composed of monomers (amino acids) linearly linked by amide bonds (also known as peptide bonds). The term "polypeptide" refers to any chain of two or more amino acids, and does not refer to a specific length of the product. Thus, peptides, dipeptides, tripeptides, oligopeptides, "protein," "amino acid chain," or any other term used to refer to a chain of two or more amino acids, are included within the definition of "polypeptide," and the term "polypeptide" may be used instead of, or interchangeably with any of these terms. The term "polypeptide" is also intended to refer to the products of post-expression modifications of the polypeptide, including without limitation glycosylation, acetylation, phosphorylation, amidation, derivatization by known protecting/blocking groups, proteolytic cleavage, or modification by non-naturally occurring amino acids. A polypeptide may be derived from a natural biological source or produced by recombinant technology, but is not necessarily translated from a designated nucleic acid sequence. It may be generated in any manner, including by chemical synthesis. A polypeptide of the invention may be of a size of about 3 or more, 5 or more, 10 or more, 20 or more, 25 or more, 50 or more, 75 or more, 100 or more, 200 or more, 500 or more, 1,000 or more, or 2,000 or more amino acids. Polypeptides may have a defined three-dimensional structure, although they do not necessarily have such structure. Polypeptides with a defined three-dimensional structure are referred to as folded, and polypeptides which do not possess a defined three-dimensional structure, but rather can adopt a large number of different conformations, and are referred to as unfolded.

By an "isolated" polypeptide or a variant, or derivative thereof is intended a polypeptide that is not in its natural milieu. No particular level of purification is required. For example, an isolated polypeptide can be removed from its native or natural environment. Recombinantly produced polypeptides and proteins expressed in host cells are considered isolated for the purpose of the invention, as are native or recombinant polypeptides which have been separated, fractionated, or partially or substantially purified by any suitable technique.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can

determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "polynucleotide" refers to an isolated nucleic acid molecule or construct, e.g. messenger RNA (mRNA), virally-derived RNA, or plasmid DNA (pDNA). A polynucleotide may comprise a conventional phosphodiester bond or a non-conventional bond (e.g. an amide bond, such as found in peptide nucleic acids (PNA). The term "nucleic acid molecule" refers to any one or more nucleic acid segments, e.g. DNA or RNA fragments, present in a polynucleotide.

By "isolated" nucleic acid molecule or polynucleotide is intended a nucleic acid molecule, DNA or RNA, which has been removed from its native environment. For example, a recombinant polynucleotide encoding a polypeptide contained in a vector is considered isolated for the purposes of the present invention. Further examples of an isolated polynucleotide include recombinant polynucleotides maintained in heterologous host cells or purified (partially or substantially) polynucleotides in solution. An isolated polynucleotide includes a polynucleotide molecule contained in cells that ordinarily contain the polynucleotide molecule, but the polynucleotide molecule is present extrachromosomally or at a chromosomal location that is different from its natural chromosomal location. Isolated RNA molecules include *in vivo* or *in vitro* RNA transcripts of the present invention, as well as positive and negative strand forms, and double-stranded forms. Isolated polynucleotides or nucleic acids according to the present invention further include such molecules produced synthetically. In addition, a polynucleotide or a nucleic acid may be or may include a regulatory element such as a promoter, ribosome binding site, or a transcription terminator.

By a nucleic acid or polynucleotide having a nucleotide sequence at least, for example, 95% "identical" to a reference nucleotide sequence of the present invention, it is intended that the nucleotide sequence of the polynucleotide is identical to the reference sequence except that the polynucleotide sequence may include up to five point mutations per each 100 nucleotides of the reference nucleotide sequence. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These alterations of the reference sequence may occur at the 5' or 3' terminal positions of the reference nucleotide sequence or anywhere between those terminal positions, interspersed either individually among residues in the reference sequence or in one or more contiguous groups within the reference sequence. As a practical matter, whether any particular polynucleotide sequence is at least 80%, 85%, 90%, 95%, 96%, 97%, 98% or 99% identical to a nucleotide sequence of the present invention can be determined conventionally using known computer programs, such as the ones discussed above for polypeptides (e.g. ALIGN-2).

The term "expression cassette" refers to a polynucleotide generated recombinantly or synthetically, with a series of specified nucleic acid elements that permit transcription of a particular nucleic acid in a target cell. The recombinant expression cassette can be incorporated into a plasmid, chromosome, mitochondrial DNA, plastid DNA, virus, or nucleic acid fragment. Typically, the recombinant expression cassette portion of an expression vector includes, among other sequences, a nucleic acid sequence to be transcribed and a promoter. In certain embodiments, the expression cassette of the invention comprises polynucleotide sequences that encode bispecific antigen binding molecules of the invention or fragments thereof.

The term "vector" or "expression vector" is synonymous with "expression construct" and refers to a DNA molecule that is used to introduce and direct the expression of a specific gene to which it is operably associated in a target cell. The term includes the vector as a self-replicating nucleic acid structure as well as the vector incorporated into the genome of a host cell into which it has been introduced. The expression vector of the present invention comprises an expression cassette. Expression vectors allow transcription of large amounts of stable mRNA. Once the expression vector is inside the target cell, the ribonucleic acid molecule or protein that is encoded by the gene is produced by the cellular transcription and/or translation machinery. In one embodiment, the expression vector of the invention comprises an expression cassette that comprises polynucleotide sequences that encode bispecific antigen binding molecules of the invention or fragments thereof.

The terms "host cell", "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein. A host cell is any type of cellular system that can be used to generate the bispecific antigen binding molecules of the present invention. Host cells include cultured cells, e.g. mammalian cultured cells, such as CHO cells, BHK cells, NS0 cells, SP2/0 cells, YO myeloma cells, P3X63 mouse myeloma cells, PER cells, PER.C6 cells or hybridoma cells, yeast cells, insect cells, and plant cells, to name only a few, but also cells comprised within a transgenic animal, transgenic plant or cultured plant or animal tissue.

An "activating Fc receptor" is an Fc receptor that following engagement by an Fc domain of an antibody elicits signaling events that stimulate the receptor-bearing cell to perform effector functions. Human activating Fc receptors include FcγRIIIa (CD16a), FcyRI (CD64), FcyRIIa (CD32), and FcaRI (CD89).

Antibody-dependent cell-mediated cytotoxicity (ADCC) is an immune mechanism leading to the lysis of antibody-coated target cells by immune effector cells. The target cells are cells to which antibodies or derivatives thereof comprising an Fc region specifically bind, generally via the protein part that is N-terminal to the Fc region. As used herein, the term "reduced ADCC" is defined as either a reduction in the number of target cells that are lysed in a given time, at a given concentration of antibody in the medium surrounding the target cells, by the mechanism of ADCC defined above, and/or an increase in the concentration of antibody in the medium surrounding the target cells, required to achieve the lysis of a given number of target cells in a given time, by the mechanism of ADCC. The reduction in ADCC is relative to the ADCC mediated by the same antibody produced by the same type of host cells, using the same standard production, purification, formulation and storage methods (which are known to those skilled in the art), but that has not been engineered. For example the reduction in ADCC mediated by an antibody comprising in its Fc domain an amino acid substitution that reduces ADCC, is relative to the ADCC mediated by the same antibody without this amino acid substitution in the Fc domain. Suitable assays to measure ADCC are well known in the art (see e.g. PCT publication no. WO 2006/082515 or PCT patent application no. PCT/EP2012/055393).

An "effective amount" of an agent refers to the amount that is necessary to result in a physiological change in the cell or tissue to which it is administered.

A "therapeutically effective amount" of an agent, e.g. a pharmaceutical composition, refers to an amount effective, at dosages and for periods of time necessary, to achieve the desired therapeutic or prophylactic result. A therapeutically effective amount of an agent for example eliminates, decreases, delays, minimizes or prevents adverse effects of a disease.

An "individual" or "subject" is a mammal. Mammals include, but are not limited to, domesticated animals (e.g. cows, sheep, cats, dogs, and horses), primates (e.g. humans and non-human primates such as monkeys), rabbits, and rodents (e.g. mice and rats). Particularly, the individual or subject is a human.

The term "pharmaceutical composition" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical composition, other than an active ingredient, which is nontoxic to a subject. A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of a disease in the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, T cell activating bispecific antigen binding molecules of the invention are used to delay development of a disease or to slow the progression of a disease. The term "package insert" is used to refer to instructions customarily included in commercial packages of therapeutic products, that contain information about the indications, usage, dosage, administration, combination therapy, contraindications and/or warnings concerning the use of such therapeutic products.

### Detailed Description of the Embodiments

In a first aspect the invention provides a T cell activating bispecific antigen binding molecule comprising a first and a second antigen binding moiety, one of which is a Fab molecule capable of specific binding to an activating T cell antigen and the other one of which is a Fab molecule capable of specific binding to a target cell antigen, and an Fc domain composed of a first and a second subunit capable of stable association;
wherein the first antigen binding moiety is
(a) a single chain Fab molecule wherein the Fab light chain and the Fab heavy chain are connected by a peptide linker, or
(b) a crossover Fab molecule wherein either the variable or the constant regions of the Fab light chain and the Fab heavy chain are exchanged.

### T cell activating bispecific antigen binding molecule formats

The components of the T cell activating bispecific antigen binding molecule can be fused to each other in a variety of configurations. Exemplary configurations are depicted in Figure 1.

In some embodiments, the second antigen binding moiety is fused at the C-terminus of the Fab heavy chain to the N-terminus of the first or the second subunit of the Fc domain.

In a particular such embodiment, the first antigen binding moiety is fused at the C-terminus of the Fab heavy chain to the N-terminus of the Fab heavy chain of the second antigen binding moiety. In a specific such embodiment, the T cell activating bispecific antigen binding molecule essentially consists of a first and a second antigen binding moiety, an Fc domain composed of a first and a second subunit, and optionally one or more peptide linkers, wherein the first antigen binding moiety is fused at the C-terminus of the Fab heavy chain to the N-terminus of the Fab heavy chain of the second antigen binding moiety, and the second antigen binding moiety is fused at the C-terminus of the Fab heavy chain to the N-terminus of the first or the second subunit of the Fc domain. In an even more specific embodiment, the first antigen binding moiety is a single chain Fab molecule. Alternatively, in a particular embodiment, the first antigen binding moiety is a crossover Fab molecule. Optionally, if the first antigen binding moiety is a crossover Fab molecule, the Fab light chain of the first antigen binding moiety and the Fab light chain of the second antigen binding moiety may additionally be fused to each other.

In an alternative such embodiment, the first antigen binding moiety is fused at the C-terminus of the Fab heavy chain to the N-terminus of the first or second subunit of the Fc domain. In a specific such embodiment, the T cell activating bispecific antigen binding molecule essentially consists of a first and a second antigen binding moiety, an Fc domain composed of a first and a second subunit, and optionally one or more peptide linkers, wherein the first and the second antigen binding moiety are each fused at the C-terminus of the Fab heavy chain to the N-terminus of one of the subunits of the Fc domain. In an even more specific embodiment, the first antigen binding moiety is a single chain Fab molecule. Alternatively, in a particular embodiment, the first antigen binding moiety is a crossover Fab molecule.

In yet another such embodiment, the second antigen binding moiety is fused at the C-terminus of the Fab light chain to the N-terminus of the Fab light chain of the first antigen binding moiety. In a specific such embodiment, the T cell activating bispecific antigen binding molecule essentially consists of a first and a second antigen binding moiety, an Fc domain composed of a first and a second subunit, and optionally one or more peptide linkers, wherein the first antigen binding moiety is fused at the N-terminus of the Fab light chain to the C-terminus of the Fab light chain of the second antigen binding moiety, and the second antigen binding moiety is fused at the C-terminus of the Fab heavy chain to the N-terminus of the first or the second subunit of the Fc domain. In an even more specific embodiment, the first antigen binding moiety is a crossover Fab molecule.

In other embodiments, the first antigen binding moiety is fused at the C-terminus of the Fab heavy chain to the N-terminus of the first or second subunit of the Fc domain.

In a particular such embodiment, the second antigen binding moiety is fused at the C-terminus of the Fab heavy chain to the N-terminus of the Fab heavy chain of the first antigen binding moiety. In a specific such embodiment, the T cell activating bispecific antigen binding molecule essentially consists of a first and a second antigen binding moiety, an Fc domain composed of a first and a second subunit, and optionally one or more peptide linkers, wherein the second antigen binding moiety is fused at the C-terminus of the Fab heavy chain to the N-terminus of the Fab heavy chain of the first antigen binding moiety, and the first antigen binding moiety is fused at the C-terminus of the Fab heavy chain to the N-terminus of the first or the second subunit of the Fc domain. In an even more specific embodiment, the first antigen binding moiety is a crossover Fab molecule. Optionally, the Fab light chain of the first antigen binding moiety and the Fab light chain of the second antigen binding moiety may additionally be fused to each other.

In particular of these embodiments, the first antigen binding moiety is capable of specific binding to an activating T cell antigen. In other embodiments, the first antigen binding moiety is capable of specific binding to a target cell antigen.

The antigen binding moieties may be fused to the Fc domain or to each other directly or through a peptide linker, comprising one or more amino acids, typically about 2-20 amino acids. Peptide linkers are known in the art and are described herein. Suitable, non-immunogenic peptide linkers include, for example, (G4S)n, (SG4)n, (G₄S)ₙ or G4(SG4)n peptide linkers, "n" is generally a number between 1 and 10, typically between 2 and 4. A particularly suitable peptide linker for fusing the Fab light chains of the first and the second antigen binding moiety to each other is (G4S)2. An exemplary peptide linker suitable for connecting the Fab heavy chains of the first and the second antigen binding moiety is EPKSC(D)-(G₄S)₂ (SEQ ID NOs 150 and 151). Additionally, linkers may comprise (a portion of) an immunoglobulin hinge region. Particularly where an antigen binding moiety is fused to the N-terminus of an Fc domain subunit, it may be fused via an immunoglobulin hinge region or a portion thereof, with or without an additional peptide linker.

A T cell activating bispecific antigen binding molecule with a single antigen binding moiety capable of specific binding to a target cell antigen (for example as shown in Figure 1A, 1B, 1D, 1E, 1H, 1I, 1K or 1M) is useful, particularly in cases where internalization of the target cell antigen is to be expected following binding of a high affinity antigen binding moiety. In such cases, the presence of more than one antigen binding moiety specific for the target cell antigen may enhance internalization of the target cell antigen, thereby reducing its availablity.

In many other cases, however, it will be advantageous to have a T cell activating bispecific antigen binding molecule comprising two or more antigen binding moieties specific for a target cell antigen (see examples in shown in Figure 1C, 1F, 1G, 1J or 1L), for example to optimize targeting to the target site or to allow crosslinking of target cell antigens.

Accordingly, in certain embodiments, the T cell activating bispecific antigen binding molecule of the invention further comprises a third antigen binding moiety which is a Fab molecule capable of specific binding to a target cell antigen. In one embodiment, the third antigen binding moiety is capable of specific binding to the same target cell antigen as the first or second antigen binding moiety. In a particular embodiment, the first antigen binding moiety is capable of specific binding to an activating T cell antigen, and the second and third antigen binding moieties are capable of specific binding to a target cell antigen.

In one embodiment, the third antigen binding moiety is fused at the C-terminus of the Fab heavy chain to the N-terminus of the first or second subunit of the Fc domain. In a particular embodiment, the second and the third antigen binding moiety are each fused at the C-terminus of the Fab heavy chain to the N-terminus of one of the subunits of the Fc domain, and the first antigen binding moiety is fused at the C-terminus of the Fab heavy chain to the N-terminus of the Fab heavy chain of the second antigen binding moiety. In one such embodiment the first antigen binding moiety is a single chain Fab molecule. In a particular such embodiment the first antigen binding moiety is a crossover Fab molecule. Optionally, if the first antigen binding moiety is a crossover Fab molecule, the Fab light chain of the first antigen binding moiety and the Fab light chain of the second antigen binding moiety may additionally be fused to each other. The second and the third antigen binding moiety may be fused to the Fc domain directly or through a peptide linker. In a particular embodiment the second and the third antigen binding moiety are each fused to the Fc domain through an immunoglobulin hinge region. In a specific embodiment, the immunoglobulin hinge region is a human IgG₁ hinge region. In one embodiment the second and the third antigen binding moiety and the Fc domain are part of an immunoglobulin molecule. In a particular embodiment the immunoglobulin molecule is an IgG class immunoglobulin. In an even more particular embodiment the immunoglobulin is an IgG₁ subclass immunoglobulin. In another embodiment the immunoglobulin is an IgG₄ subclass immunoglobulin. In a further particular embodiment the immunoglobulin is a human immunoglobulin. In other embodiments the immunoglobulin is a chimeric immunoglobulin or a humanized immunoglobulin. In one embodiment, the T cell activating bispecific antigen binding molecule essentially consists of an immunoglobulin molecule capable of specific binding to a target cell antigen, and an antigen binding moiety capable of specific binding to an activating T cell antigen wherein the antigen binding moiety is a single chain Fab molecule or a crossover Fab molecule, particularly a crossover Fab molecule, fused to the N-terminus of one of the immunoglobulin heavy chains, optionally via a peptide linker.

In an alternative embodiment, the first and the third antigen binding moiety are each fused at the C-terminus of the Fab heavy chain to the N-terminus of one of the subunits of the Fc domain, and the second antigen binding moiety is fused at the C-terminus of the Fab heavy chain to the N-terminus of the Fab heavy chain of the first antigen binding moiety. In a specific such embodiment, the T cell activating bispecific antigen binding molecule essentially consists of a first, a second and a third antigen binding moiety, an Fc domain composed of a first and a second subunit, and optionally one or more peptide linkers, wherein the second antigen binding moiety is fused at the C-terminus of the Fab heavy chain to the N-terminus of the Fab heavy chain of the first antigen binding moiety, and the first antigen binding moiety is fused at the C-terminus of the Fab heavy chain to the N-terminus of the first subunit of the Fc domain, and wherein the third antigen binding moiety is fused at the C-terminus of the Fab heavy chain to the N-terminus of the second subunit of the Fc domain. In a particular such embodiment the first antigen binding moiety is a crossover Fab molecule. Optionally, the Fab light chain of the first antigen binding moiety and the Fab light chain of the second antigen binding moiety may additionally be fused to each other.

In some of the T cell activating bispecific antigen binding molecule of the invention, the Fab light chain of the first antigen binding moiety and the Fab light chain of the second antigen binding moiety are fused to each other, optionally via a linker peptide. Depending on the configuration of the first and the second antigen binding moiety, the Fab light chain of the first antigen binding moiety may be fused at its C-terminus to the N-terminus of the Fab light chain of the second antigen binding moiety, or the Fab light chain of the second antigen binding moiety may be fused at its C-terminus to the N-terminus of the Fab light chain of the first antigen binding moiety. Fusion of the Fab light chains of the first and the second antigen binding moiety further reduces mispairing of unmatched Fab heavy and light chains, and also reduces the number of plasmids needed for expression of some of the T cell activating bispecific antigen binding molecules of the invention.

In certain embodiments the T cell activating bispecific antigen binding molecule comprises a polypeptide wherein a first Fab light chain shares a carboxy-terminal peptide bond with a peptide linker, which in turn shares a carboxy-terminal peptide bond with a first Fab heavy chain, which in turn shares a carboxy-terminal peptide bond with an Fc domain subunit (VL-CL-linker-VH-CH1-CH2-CH2(-CH4)), and a polypeptide wherein a second Fab heavy chain shares a carboxy-terminal peptide bond with an Fc domain subunit (VH-CH1-CH2-CH3(-CH4)). In some embodiments the T cell activating bispecific antigen binding molecule further comprises a second Fab light chain polypeptide (VL-CL). In certain embodiments the polypeptides are covalently linked, e.g., by a disulfide bond.

In some embodiments, the T cell activating bispecific antigen binding molecule comprises a polypeptide wherein a first Fab light chain shares a carboxy-terminal peptide bond with a peptide linker, which in turn shares a carboxy-terminal peptide bond with a first Fab heavy chain, which in turn shares a carboxy-terminal peptide bond with a second Fab heavy chain, which in turn shares a carboxy-terminal peptide bond with an Fc domain subunit (VL-CL-linker-VH-CH1-VH-CH1-CH2-CH3(-CH4)). In one of these embodiments that T cell activating bispecific antigen binding molecule further comprises a second Fab light chain polypeptide (VL-CL). The T cell activating bispecific antigen binding molecule according to these embodiments may further comprise (i) an Fc domain subunit polypeptide (CH2-CH3(-CH4)), or (ii) a polypeptide wherein a third Fab heavy chain shares a carboxy-terminal peptide bond with an Fc domain subunit (VH-CH1-CH2-CH3(-CH4)) and a third Fab light chain polypeptide (VL-CL). In certain embodiments the polypeptides are covalently linked, e.g., by a disulfide bond.

In certain embodiments the T cell activating bispecific antigen binding molecule comprises a polypeptide wherein a first Fab light chain variable region shares a carboxy-terminal peptide bond with a first Fab heavy chain constant region (i.e. a crossover Fab heavy chain, wherein the heavy chain variable region is replaced by a light chain variable region), which in turn shares a carboxy-terminal peptide bond with an Fc domain subunit (VL-CH1-CH2-CH2(-CH4)), and a polypeptide wherein a second Fab heavy chain shares a carboxy-terminal peptide bond with an Fc domain subunit (VH-CH1-CH2-CH3(-CH4)). In some embodiments the T cell activating bispecific antigen binding molecule further comprises a polypeptide wherein a Fab heavy chain variable region shares a carboxy-terminal peptide bond with a Fab light chain constant region (VH-CL) and a Fab light chain polypeptide (VL-CL). In certain embodiments the polypeptides are covalently linked, e.g., by a disulfide bond.

In alternative embodiments the T cell activating bispecific antigen binding molecule comprises a polypeptide wherein a first Fab heavy chain variable region shares a carboxy-terminal peptide bond with a first Fab light chain constant region (i.e. a crossover Fab heavy chain, wherein the heavy chain constant region is replaced by a light chain constant region), which in turn shares a carboxy-terminal peptide bond with an Fc domain subunit (VH-CL-CH2-CH2(-CH4)), and a polypeptide wherein a second Fab heavy chain shares a carboxy-terminal peptide bond with an Fc domain subunit (VH-CH1-CH2-CH3(-CH4)). In some embodiments the T cell activating bispecific antigen binding molecule further comprises a polypeptide wherein a Fab light chain variable region shares a carboxy-terminal peptide bond with a Fab heavy chain constant region (VL-CH1) and a Fab light chain polypeptide (VL-CL). In certain embodiments the polypeptides are covalently linked, e.g., by a disulfide bond.

In some embodiments, the T cell activating bispecific antigen binding molecule comprises a polypeptide wherein a first Fab light chain variable region shares a carboxy-terminal peptide bond with a first Fab heavy chain constant region (i.e. a crossover Fab heavy chain, wherein the heavy chain variable region is replaced by a light chain variable region), which in turn shares a carboxy-terminal peptide bond with a second Fab heavy chain, which in turn shares a carboxy-terminal peptide bond with an Fc domain subunit (VL-CH1-VH-CH1-CH2-CH3(-CH4)). In other embodiments, the T cell activating bispecific antigen binding molecule comprises a polypeptide wherein a first Fab heavy chain variable region shares a carboxy-terminal peptide bond with a first Fab light chain constant region (i.e. a crossover Fab heavy chain, wherein the heavy chain constant region is replaced by a light chain constant region), which in turn shares a carboxy-terminal peptide bond with a second Fab heavy chain, which in turn shares a carboxy-terminal peptide bond with an Fc domain subunit (VH-CL-VH-CH1-CH2-CH3(-CH4)). In still other embodiments, the T cell activating bispecific antigen binding molecule comprises a polypeptide wherein a second Fab heavy chain shares a carboxy-terminal peptide bond with a first Fab light chain variable region which in turn shares a carboxy-terminal peptide bond with a first Fab heavy chain constant region (i.e. a crossover Fab heavy chain, wherein the heavy chain variable region is replaced by a light chain variable region), which in turn shares a carboxy-terminal peptide bond with an Fc domain subunit (VH-CH1-VL-CH1-CH2-CH3(-CH4)). In other embodiments, the T cell activating bispecific antigen binding molecule comprises a polypeptide wherein a second Fab heavy chain shares a carboxy-terminal peptide bond with a first Fab heavy chain variable region which in turn shares a carboxy-terminal peptide bond with a first Fab light chain constant region (i.e. a crossover Fab heavy chain, wherein the heavy chain constant region is replaced by a light chain constant region), which in turn shares a carboxy-terminal peptide bond with an Fc domain subunit (VH-CH1-VH-CL-CH2-CH3(-CH4)).

In some of these embodiments the T cell activating bispecific antigen binding molecule further comprises a crossover Fab light chain polypeptide, wherein a Fab heavy chain variable region shares a carboxy-terminal peptide bond with a Fab light chain constant region (VH-CL), and a Fab light chain polypeptide (VL-CL). In others of these embodiments the T cell activating bispecific antigen binding molecule further comprises a crossover Fab light chain polypeptide, wherein a Fab light chain variable region shares a carboxy-terminal peptide bond with a Fab heavy chain constant region (VL-CH1), and a Fab light chain polypeptide (VL-CL). In still others of these embodiments the T cell activating bispecific antigen binding molecule further comprises a polypeptide wherein a Fab light chain variable region shares a carboxy-terminal peptide bond with a Fab heavy chain constant region which in turn shares a carboxy-terminal peptide bond with a Fab light chain polypeptide (VL-CH1-VL-CL), a polypeptide wherein a Fab heavy chain variable region shares a carboxy-terminal peptide bond with a Fab light chain constant region which in turn shares a carboxy-terminal peptide bond with a Fab light chain polypeptide (VH-CL-VL-CL), a polypeptide wherein a Fab light chain polypeptide shares a carboxy-terminal peptide bond with a Fab light chain variable region which in turn shares a carboxy-terminal peptide bond with a Fab heavy chain constant region (VL-CL-VL-CH1), or a polypeptide wherein a Fab light chain polypeptide shares a carboxy-terminal peptide bond with a Fab heavy chain variable region which in turn shares a carboxy-terminal peptide bond with a Fab light chain constant region (VL-CL-VH-CL).

The T cell activating bispecific antigen binding molecule according to these embodiments may further comprise (i) an Fc domain subunit polypeptide (CH2-CH3(-CH4)), or (ii) a polypeptide wherein a third Fab heavy chain shares a carboxy-terminal peptide bond with an Fc domain subunit (VH-CH1-CH2-CH3(-CH4)) and a third Fab light chain polypeptide (VL-CL). In certain embodiments the polypeptides are covalently linked, e.g., by a disulfide bond.

In one embodiment, the T cell activating bispecific antigen binding molecule comprises a polypeptide wherein a second Fab light chain shares a carboxy-terminal peptide bond with a first Fab light chain variable region which in turn shares a carboxy-terminal peptide bond with a first Fab heavy chain constant region (i.e. a crossover Fab light chain, wherein the light chain constant region is replaced by a heavy chain constant region) (VL-CL-VL-CH1), a polypeptide wherein a second Fab heavy chain shares a carboxy-terminal peptide bond with an Fc domain subunit (VH-CH1-CH2-CH3(-CH4)), and a polypeptide wherein a first Fab heavy chain variable region shares a carboxy-terminal peptide bond with a first Fab light chain constant region (VH-CL). In another embodiment, the T cell activating bispecific antigen binding molecule comprises a polypeptide wherein a second Fab light chain shares a carboxy-terminal peptide bond with a first Fab heavy chain variable region which in turn shares a carboxy-terminal peptide bond with a first Fab light chain constant region (i.e. a crossover Fab light chain, wherein the light chain variable region is replaced by a heavy chain variable region) (VL-CL-VH-CL), a polypeptide wherein a second Fab heavy chain shares a carboxy-terminal peptide bond with an Fc domain subunit (VH-CH1-CH2-CH3(-CH4)), and a polypeptide wherein a first Fab light chain variable region shares a carboxy-terminal peptide bond with a first Fab heavy chain constant region (VL-CH1). The T cell activating bispecific antigen binding molecule according to these embodiments may further comprise (i) an Fc domain subunit polypeptide (CH2-CH3(-CH4)), or (ii) a polypeptide wherein a third Fab heavy chain shares a carboxy-terminal peptide bond with an Fc domain subunit (VH-CH1-CH2-CH3(-CH4)) and a third Fab light chain polypeptide (VL-CL). In certain embodiments the polypeptides are covalently linked, e.g., by a disulfide bond.

According to any of the above embodiments, components of the T cell activating bispecific antigen binding molecule (e.g. antigen binding moiety, Fc domain) may be fused directly or through various linkers, particularly peptide linkers comprising one or more amino acids, typically about 2-20 amino acids, that are described herein or are known in the art. Suitable, non-immunogenic peptide linkers include, for example, (G4S)n, (SG4)n, (G₄S)ₙ or G4(SG4)n peptide linkers, wherein n is generally a number between 1 and 10, typically between 2 and 4.

### Fc domain

The Fc domain of the T cell activating bispecific antigen binding molecule consists of a pair of polypeptide chains comprising heavy chain domains of an immunoglobulin molecule. For example, the Fc domain of an immunoglobulin G (IgG) molecule is a dimer, each subunit of which comprises the CH2 and CH3 IgG heavy chain constant domains. The two subunits of the Fc domain are capable of stable association with each other. In one embodiment the T cell activating bispecific antigen binding molecule of the invention comprises not more than one Fc domain.

In one embodiment according the invention the Fc domain of the T cell activating bispecific antigen binding molecule is an IgG Fc domain. In a particular embodiment the Fc domain is an IgG₁ Fc domain. In another embodiment the Fc domain is an IgG₄ Fc domain. In a more specific embodiment, the Fc domain is an IgG4 Fc domain comprising an amino acid substitution at position S228 (EU numbering), particularly the amino acid substitution S228P. This amino acid substitution reduces in vivo Fab arm exchange of IgG4 antibodies (see Stubenrauch et al., Drug Metabolism and Disposition 38, 84-91 (2010)). In a further particular embodiment the Fc domain is human. An exemplary sequence of a human IgG₁ Fc region is given in SEQ ID NO: 149.

### Fc domain modifications promoting heterodimerization

T cell activating bispecific antigen binding molecules according to the invention comprise different antigen binding moieties, fused to one or the other of the two subunits of the Fc domain, thus the two subunits of the Fc domain are typically comprised in two non-identical polypeptide chains. Recombinant co-expression of these polypeptides and subsequent dimerization leads to several possible combinations of the two polypeptides. To improve the yield and purity of T cell activating bispecific antigen binding molecules in recombinant production, it will thus be advantageous to introduce in the Fc domain of the T cell activating bispecific antigen binding molecule a modification promoting the association of the desired polypeptides.

Accordingly, in particular embodiments the Fc domain of the T cell activating bispecific antigen binding molecule according to the invention comprises a modification promoting the association of the first and the second subunit of the Fc domain. The site of most extensive protein-protein interaction between the two subunits of a human IgG Fc domain is in the CH3 domain of the Fc domain. Thus, in one embodiment said modification is in the CH3 domain of the Fc domain.

In a specific embodiment said modification is a so-called "knob-into-hole" modification, comprising a "knob" modification in one of the two subunits of the Fc domain and a "hole" modification in the other one of the two subunits of the Fc domain.

The knob-into-hole technology is described e.g. in US 5,731,168; US 7,695,936; Ridgway et al., Prot Eng 9, 617-621 (1996) and Carter, J Immunol Meth 248, 7-15 (2001). Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (e.g. tyrosine or tryptophan). Compensatory cavities of identical or similar size to the protuberances are created in the interface of the second polypeptide by replacing large amino acid side chains with smaller ones (e.g. alanine or threonine).

Accordingly, in a particular embodiment, in the CH3 domain of the first subunit of the Fc domain of the T cell activating bispecific antigen binding molecule an amino acid residue is replaced with an amino acid residue having a larger side chain volume, thereby generating a protuberance within the CH3 domain of the first subunit which is positionable in a cavity within the CH3 domain of the second subunit, and in the CH3 domain of the second subunit of the Fc domain an amino acid residue is replaced with an amino acid residue having a smaller side chain volume, thereby generating a cavity within the CH3 domain of the second subunit within which the protuberance within the CH3 domain of the first subunit is positionable.

The protuberance and cavity can be made by altering the nucleic acid encoding the polypeptides, e.g. by site-specific mutagenesis, or by peptide synthesis.

In a specific embodiment, in the CH3 domain of the first subunit of the Fc domain the threonine residue at position 366 is replaced with a tryptophan residue (T366W), and in the CH3 domain of the second subunit of the Fc domain the tyrosine residue at position 407 is replaced with a valine residue (Y407V). In one embodiment, in the second subunit of the Fc domain additionally the threonine residue at position 366 is replaced with a serine residue (T366S) and the leucine residue at position 368 is replaced with an alanine residue (L368A).

In yet a further embodiment, in the first subunit of the Fc domain additionally the serine residue at position 354 is replaced with a cysteine residue (S354C), and in the second subunit of the Fc domain additionally the tyrosine residue at position 349 is replaced by a cysteine residue (Y349C). Introduction of these two cysteine residues results in formation of a disulfide bridge between the two subunits of the Fc domain, further stabilizing the dimer (Carter, J Immunol Methods 248, 7-15 (2001)).

In a particular embodiment the antigen binding moiety capable of binding to an activating T cell antigen is fused (optionally via the antigen binding moiety capable of binding to a target cell antigen) to the first subunit of the Fc domain (comprising the "knob" modification). Without wishing to be bound by theory, fusion of the antigen binding moiety capable of binding to an activating T cell antigen to the knob-containing subunit of the Fc domain will (further) minimize the generation of antigen binding molecules comprising two antigen binding moieties capable of binding to an activating T cell antigen (steric clash of two knob-containing polypeptides).

In an alternative embodiment a modification promoting association of the first and the second subunit of the Fc domain comprises a modification mediating electrostatic steering effects, e.g. as described in PCT publication WO 2009/089004. Generally, this method involves replacement of one or more amino acid residues at the interface of the two Fc domain subunits by charged amino acid residues so that homodimer formation becomes electrostatically unfavorable but heterodimerization electrostatically favorable.

### Fc domain modifications reducing Fc receptor binding and/or effector function

The Fc domain confers to the T cell activating bispecific antigen binding molecule favorable pharmacokinetic properties, including a long serum half-life which contributes to good accumulation in the target tissue and a favorable tissue-blood distribution ratio. At the same time it may, however, lead to undesirable targeting of the T cell activating bispecific antigen binding molecule to cells expressing Fc receptors rather than to the preferred antigen-bearing cells. Moreover, the co-activation of Fc receptor signaling pathways may lead to cytokine release which, in combination with the T cell activating properties and the long half-life of the antigen binding molecule, results in excessive activation of cytokine receptors and severe side effects upon systemic administration. Activation of (Fc receptor-bearing) immune cells other than T cells may even reduce efficacy of the T cell activating bispecific antigen binding molecule due to the potential destruction of T cells e.g. by NK cells.

Accordingly, in particular embodiments the Fc domain of the T cell activating bispecific antigen binding molecules according to the invention exhibits reduced binding affinity to an Fc receptor and/or reduced effector function, as compared to a native IgG₁ Fc domain. In one such embodiment the Fc domain (or the T cell activating bispecific antigen binding molecule comprising said Fc domain) exhibits less than 50%, preferably less than 20%, more preferably less than 10% and most preferably less than 5% of the binding affinity to an Fc receptor, as compared to a native IgG₁ Fc domain (or a T cell activating bispecific antigen binding molecule comprising a native IgG₁ Fc domain), and/or less than 50%, preferably less than 20%, more preferably less than 10% and most preferably less than 5% of the effector function, as compared to a native IgG₁ Fc domain domain (or a T cell activating bispecific antigen binding molecule comprising a native IgG₁ Fc domain). In one embodiment, the Fc domain domain (or the T cell activating bispecific antigen binding molecule comprising said Fc domain) does not substantially bind to an Fc receptor and/or induce effector function. In a particular embodiment the Fc receptor is an Fcγ receptor. In one embodiment the Fc receptor is a human Fc receptor. In one embodiment the Fc receptor is an activating Fc receptor. In a specific embodiment the Fc receptor is an activating human Fcγ receptor, more specifically human FcyRIIIa, FcyRI or FcyRIIa, most specifically human FcyRIIIa. In one embodiment the effector function is one or more selected from the group of CDC, ADCC, ADCP, and cytokine secretion. In a particular embodiment the effector function is ADCC. In one embodiment the Fc domain domain exhibits substantially similar binding affinity to neonatal Fc receptor (FcRn), as compared to a native IgG₁ Fc domain domain. Substantially similar binding to FcRn is achieved when the Fc domain (or the T cell activating bispecific antigen binding molecule comprising said Fc domain) exhibits greater than about 70%, particularly greater than about 80%, more particularly greater than about 90% of the binding affinity of a native IgG₁ Fc domain (or the T cell activating bispecific antigen binding molecule comprising a native IgG₁ Fc domain) to FcRn.

In certain embodiments the Fc domain is engineered to have reduced binding affinity to an Fc receptor and/or reduced effector function, as compared to a non-engineered Fc domain. In particular embodiments, the Fc domain of the T cell activating bispecific antigen binding molecule comprises one or more amino acid mutation that reduces the binding affinity of the Fc domain to an Fc receptor and/or effector function. Typically, the same one or more amino acid mutation is present in each of the two subunits of the Fc domain. In one embodiment the amino acid mutation reduces the binding affinity of the Fc domain to an Fc receptor. In one embodiment the amino acid mutation reduces the binding affinity of the Fc domain to an Fc receptor by at least 2-fold, at least 5-fold, or at least 10-fold. In embodiments where there is more than one amino acid mutation that reduces the binding affinity of the Fc domain to the Fc receptor, the combination of these amino acid mutations may reduce the binding affinity of the Fc domain to an Fc receptor by at least 10-fold, at least 20-fold, or even at least 50-fold. In one embodiment the T cell activating bispecific antigen binding molecule comprising an engineered Fc domain exhibits less than 20%, particularly less than 10%, more particularly less than 5% of the binding affinity to an Fc receptor as compared to a T cell activating bispecific antigen binding molecule comprising a non-engineered Fc domain. In a particular embodiment the Fc receptor is an Fcγ receptor. In some embodiments the Fc receptor is a human Fc receptor. In some embodiments the Fc receptor is an activating Fc receptor. In a specific embodiment the Fc receptor is an activating human Fcγ receptor, more specifically human FcyRIIIa, FcyRI or FcyRIIa, most specifically human FcyRIIIa. Preferably, binding to each of these receptors is reduced. In some embodiments binding affinity to a complement component, specifically binding affinity to C1q, is also reduced. In one embodiment binding affinity to neonatal Fc receptor (FcRn) is not reduced. Substantially similar binding to FcRn, i.e. preservation of the binding affinity of the Fc domain to said receptor, is achieved when the Fc domain (or the T cell activating bispecific antigen binding molecule comprising said Fc domain) exhibits greater than about 70% of the binding affinity of a non-engineered form of the Fc domain (or the T cell activating bispecific antigen binding molecule comprising said non-engineered form of the Fc domain) to FcRn. The Fc domain, or T cell activating bispecific antigen binding molecules of the invention comprising said Fc domain, may exhibit greater than about 80% and even greater than about 90% of such affinity. In certain embodiments the Fc domain of the T cell activating bispecific antigen binding molecule is engineered to have reduced effector function, as compared to a non-engineered Fc domain. The reduced effector function can include, but is not limited to, one or more of the following: reduced complement dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen-presenting cells, reduced binding to NK cells, reduced binding to macrophages, reduced binding to monocytes, reduced binding to polymorphonuclear cells, reduced direct signaling inducing apoptosis, reduced crosslinking of target-bound antibodies, reduced dendritic cell maturation, or reduced T cell priming. In one embodiment the reduced effector function is one or more selected from the group of reduced CDC, reduced ADCC, reduced ADCP, and reduced cytokine secretion. In a particular embodiment the reduced effector function is reduced ADCC. In one embodiment the reduced ADCC is less than 20% of the ADCC induced by a non-engineered Fc domain (or a T cell activating bispecific antigen binding molecule comprising a non-engineered Fc domain).

In one embodiment the amino acid mutation that reduces the binding affinity of the Fc domain to an Fc receptor and/or effector function is an amino acid substitution. In one embodiment the Fc domain comprises an amino acid substitution at a position selected from the group of E233, L234, L235, N297, P331 and P329. In a more specific embodiment the Fc domain comprises an amino acid substitution at a position selected from the group of L234, L235 and P329. In some embodiments the Fc domain comprises the amino acid substitutions L234A and L235A. In one such embodiment, the Fc domain is an IgG₁ Fc domain, particularly a human IgG₁ Fc domain. In one embodiment the Fc domain comprises an amino acid substitution at position P329. In a more specific embodiment the amino acid substitution is P329A or P329G, particularly P329G. In one embodiment the Fc domain comprises an amino acid substitution at position P329 and a further amino acid substitution at a position selected from E233, L234, L235, N297 and P331. In a more specific embodiment the further amino acid substitution is E233P, L234A, L235A, L235E, N297A, N297D or P331S. In particular embodiments the Fc domain comprises amino acid substitutions at positions P329, L234 and L235. In more particular embodiments the Fc domain comprises the amino acid mutations L234A, L235A and P329G ("P329G LALA"). In one such embodiment, the Fc domain is an IgG₁ Fc domain, particularly a human IgG₁ Fc domain. The "P329G LALA" combination of amino acid substitutions almost completely abolishes Fcγ receptor binding of a human IgG₁ Fc domain, as described in PCT patent application no. PCT/EP2012/055393, incorporated herein by reference in its entirety. PCT/EP2012/055393 also describes methods of preparing such mutant Fc domains and methods for determining its properties such as Fc receptor binding or effector functions.

IgG4 antibodies exhibit reduced binding affinity to Fc receptors and reduced effector functions as compared to IgG₁ antibodies. Hence, in some embodiments the Fc domain of the T cell activating bispecific antigen binding molecules of the invention is an IgG4 Fc domain, particularly a human IgG₄ Fc domain. In one embodiment the IgG₄ Fc domain comprises amino acid substitutions at position S228, specifically the amino acid substitution S228P. To further reduce its binding affinity to an Fc receptor and/or its effector function, in one embodiment the IgG₄ Fc domain comprises an amino acid substitution at position L235, specifically the amino acid substitution L235E. In another embodiment, the IgG₄ Fc domain comprises an amino acid substitution at position P329, specifically the amino acid substitution P329G. In a particular embodiment, the IgG₄ Fc domain comprises amino acid substitutions at positions S228, L235 and P329, specifically amino acid substitutions S228P, L235E and P329G. Such IgG₄ Fc domain mutants and their Fcγ receptor binding properties are described in PCT patent application no. PCT/EP2012/055393, incorporated herein by reference in its entirety.

In a particular embodiment the Fc domain exhibiting reduced binding affinity to an Fc receptor and/or reduced effector function, as compared to a native IgG₁ Fc domain, is a human IgG₁ Fc domain comprising the amino acid substitutions L234A, L235A and optionally P329G, or a human IgG₄ Fc domain comprising the amino acid substitutions S228P, L235E and optionally P329G.

In certain embodiments N-glycosylation of the Fc domain has been eliminated. In one such embodiment the Fc domain comprises an amino acid mutation at position N297, particularly an amino acid substitution replacing asparagine by alanine (N297A) or aspartic acid (N297D).

In addition to the Fc domains described hereinabove and in PCT patent application no. PCT/EP2012/055393, Fc domains with reduced Fc receptor binding and/or effector function also include those with substitution of one or more of Fc domain residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Mutant Fc domains can be prepared by amino acid deletion, substitution, insertion or modification using genetic or chemical methods well known in the art. Genetic methods may include site-specific mutagenesis of the encoding DNA sequence, PCR, gene synthesis, and the like. The correct nucleotide changes can be verified for example by sequencing.

Binding to Fc receptors can be easily determined e.g. by ELISA, or by Surface Plasmon Resonance (SPR) using standard instrumentation such as a BIAcore instrument (GE Healthcare), and Fc receptors such as may be obtained by recombinant expression. A suitable such binding assay is described herein. Alternatively, binding affinity of Fc domains or cell activating bispecific antigen binding molecules comprising an Fc domain for Fc receptors may be evaluated using cell lines known to express particular Fc receptors, such as human NK cells expressing FcγIIIa receptor.

Effector function of an Fc domain, or a T cell activating bispecific antigen binding molecule comprising an Fc domain, can be measured by methods known in the art. A suitable assay for measuring ADCC is described herein. Other examples of *in vitro* assays to assess ADCC activity of a molecule of interest are described in U.S. Patent No. 5,500,362; Hellstrom et al. Proc Natl Acad Sci USA 83, 7059-7063 (1986) and Hellstrom et al., Proc Natl Acad Sci USA 82, 1499-1502 (1985); U.S. Patent No. 5,821,337; Bruggemann et al., J Exp Med 166, 1351-1361 (1987). Alternatively, non-radioactive assays methods may be employed (see, for example, ACTI™ non-radioactive cytotoxicity assay for flow cytometry (CellTechnology, Inc. Mountain View, CA); and CytoTox 96® non-radioactive cytotoxicity assay (Promega, Madison, WI)). Useful effector cells for such assays include peripheral blood mononuclear cells (PBMC) and Natural Killer (NK) cells. Alternatively, or additionally, ADCC activity of the molecule of interest may be assessed *in vivo,* e.g. in a animal model such as that disclosed in Clynes et al., Proc Natl Acad Sci USA 95, 652-656 (1998).

In some embodiments, binding of the Fc domain to a complement component, specifically to C1q, is reduced. Accordingly, in some embodiments wherein the Fc domain is engineered to have reduced effector function, said reduced effector function includes reduced CDC. C1q binding assays may be carried out to determine whether the T cell activating bispecific antigen binding molecule is able to bind C1q and hence has CDC activity. See e.g., C1q and C3c binding ELISA in WO 2006/029879 and WO 2005/100402. To assess complement activation, a CDC assay may be performed (see, for example, Gazzano-Santoro et al., J Immunol Methods 202, 163 (1996); Cragg et al., Blood 101, 1045-1052 (2003); and Cragg and Glennie, Blood 103, 2738-2743 (2004)).

### Antigen Binding Moieties

The antigen binding molecule of the invention is bispecific, i.e. it comprises at least two antigen binding moieties capable of specific binding to two distinct antigenic determinants. According to the invention, the antigen binding moieties are Fab molecules (i.e. antigen binding domains composed of a heavy and a light chain, each comprising a variable and a constant region). In one embodiment said Fab molecules are human. In another embodiment said Fab molecules are humanized. In yet another embodiment said Fab molecules comprise human heavy and light chain constant regions.

At least one of the antigen binding moieties is a single chain Fab molecule or a crossover Fab molecule. Such modifications prevent mispairing of heavy and light chains from different Fab molecules, thereby improving the yield and purity of the T cell activating bispecific antigen binding molecule of the invention in recombinant production. In a particular single chain Fab molecule useful for the T cell activating bispecific antigen binding molecule of the invention, the C-terminus of the Fab light chain is connected to the N-terminus of the Fab heavy chain by a peptide linker. The peptide linker allows arrangement of the Fab heavy and light chain to form a functional antigen binding moiety. Peptide linkers suitable for connecting the Fab heavy and light chain include, for example, (G4S)6-GG (SEQ ID NO: 152) or (SG3)2-(SEG3)4-(SG3)-SG (SEQ ID NO: 153). In a particular crossover Fab molecule useful for the T cell activating bispecific antigen binding molecule of the invention, the constant regions of the Fab light chain and the Fab heavy chain are exchanged. In another crossover Fab molecule useful for the T cell activating bispecific antigen binding molecule of the invention, the variable regions of the Fab light chain and the Fab heavy chain are exchanged.

In a particular embodiment according to the invention, the T cell activating bispecific antigen binding molecule is capable of simultaneous binding to a target cell antigen, particularly a tumor cell antigen, and an activating T cell antigen. In one embodiment, the T cell activating bispecific antigen binding molecule is capable of crosslinking a T cell and a target cell by simultaneous binding to a target cell antigen and an activating T cell antigen. In an even more particular embodiment, such simultaneous binding results in lysis of the target cell, particularly a tumor cell. In one embodiment, such simultaneous binding results in activation of the T cell. In other embodiments, such simultaneous binding results in a cellular response of a T lymphocyte, particularly a cytotoxic T lymphocyte, selected from the group of: proliferation, differentiation, cytokine secretion, cytotoxic effector molecule release, cytotoxic activity, and expression of activation markers. In one embodiment, binding of the T cell activating bispecific antigen binding molecule to the activating T cell antigen without simultaneous binding to the target cell antigen does not result in T cell activation.

In one embodiment, the T cell activating bispecific antigen binding molecule is capable of redirecting cytotoxic activity of a T cell to a target cell. In a particular embodiment, said re-direction is independent of MHC-mediated peptide antigen presentation by the target cell and and/or specificity of the T cell.

Particularly, a T cell according to any of the embodiments of the invention is a cytotoxic T cell. In some embodiments the T cell is a CD4⁺ or a CD8⁺ T cell, particularly a CD8⁺ T cell.

### Activating T cell antigen binding moiety

The T cell activating bispecific antigen binding molecule of the invention comprises at least one antigen binding moiety capable of binding to an activating T cell antigen (also referred to herein as an "activating T cell antigen binding moiety"). In a particular embodiment, the T cell activating bispecific antigen binding molecule comprises not more than one antigen binding moiety capable of specific binding to an activating T cell antigen. In one embodiment the T cell activating bispecific antigen binding molecule provides monovalent binding to the activating T cell antigen. The activating T cell antigen binding moiety can either be a conventional Fab molecule or a modified Fab molecule, i.e. a single chain or crossover Fab molecule. In embodiments where there is more than one antigen binding moiety capable of specific binding to a target cell antigen comprised in the T cell activating bispecific antigen binding molecule, the antigen binding moiety capable of specific binding to an activating T cell antigen preferably is a modified Fab molecule.

In a particular embodiment the activating T cell antigen is CD3, particularly human CD3 (SEQ ID NO: 265) or cynomolgus CD3 (SEQ ID NO: 266), most particularly human CD3. In a particular embodiment the activating T cell antigen binding moiety is cross-reactive for (i.e. specifically binds to) human and cynomolgus CD3. In some embodiments, the activating T cell antigen is the epsilon subunit of CD3.

In one embodiment, the activating T cell antigen binding moiety can compete with monoclonal antibody H2C (described in PCT publication no. WO2008/119567) for binding an epitope of CD3. In another embodiment, the activating T cell antigen binding moiety can compete with monoclonal antibody V9 (described in Rodrigues et al., Int J Cancer Suppl 7, 45-50 (1992) and US patent no. 6,054,297) for binding an epitope of CD3. In yet another embodiment, the activating T cell antigen binding moiety can compete with monoclonal antibody FN18 (described in Nooij et al., Eur J Immunol 19, 981-984 (1986)) for binding an epitope of CD3. In a particular embodiment, the activating T cell antigen binding moiety can compete with monoclonal antibody SP34 (described in Pessano et al., EMBO J 4, 337-340 (1985)) for binding an epitope of CD3. In one embodiment, the activating T cell antigen binding moiety binds to the same epitope of CD3 as monoclonal antibody SP34. In one embodiment, the activating T cell antigen binding moiety comprises the heavy chain CDR1 of SEQ ID NO: 163, the heavy chain CDR2 of SEQ ID NO: 165, the heavy chain CDR3 of SEQ ID NO: 167, the light chain CDR1 of SEQ ID NO: 171, the light chain CDR2 of SEQ ID NO: 173, and the light chain CDR3 of SEQ ID NO: 175. In a further embodiment, the activating T cell antigen binding moiety comprises a heavy chain variable region sequence that is at least about 80%, 85%, 90%, 95%, 96%, 97%, 98%, 99% or 100% identical to SEQ ID NO: 169 and a light chain variable region sequence that is at least about 80%, 85%, 90%, 95%, 96%, 97%, 98%, 99% or 100% identical to SEQ ID NO: 177, or variants thereof that retain functionality.

In a particular embodiment, the activating T cell antigen binding moiety comprises the heavy chain CDR1 of SEQ ID NO: 249, the heavy chain CDR2 of SEQ ID NO: 251, the heavy chain CDR3 of SEQ ID NO: 253, the light chain CDR1 of SEQ ID NO: 257, the light chain CDR2 of SEQ ID NO: 259, and the light chain CDR3 of SEQ ID NO: 261. In one embodiment, the activating T cell antigen binding moiety can compete for binding an epitope of CD3 with an antigen binding moiety comprising the heavy chain CDR1 of SEQ ID NO: 249, the heavy chain CDR2 of SEQ ID NO: 251, the heavy chain CDR3 of SEQ ID NO: 253, the light chain CDR1 of SEQ ID NO: 257, the light chain CDR2 of SEQ ID NO: 259, and the light chain CDR3 of SEQ ID NO: 261. In one embodiment, the activating T cell antigen binding moiety binds to the same epitope of CD3 as an antigen binding moiety comprising the heavy chain CDR1 of SEQ ID NO: 249, the heavy chain CDR2 of SEQ ID NO: 251, the heavy chain CDR3 of SEQ ID NO: 253, the light chain CDR1 of SEQ ID NO: 257, the light chain CDR2 of SEQ ID NO: 259, and the light chain CDR3 of SEQ ID NO: 261. In a further embodiment, the activating T cell antigen binding moiety comprises a heavy chain variable region sequence that is at least about 80%, 85%, 90%, 95%, 96%, 97%, 98%, 99% or 100% identical to SEQ ID NO: 255 and a light chain variable region sequence that is at least about 80%, 85%, 90%, 95%, 96%, 97%, 98%, 99% or 100% identical to SEQ ID NO: 263, or variants thereof that retain functionality. In one embodiment, the activating T cell antigen binding moiety can compete for binding an epitope of CD3 with an antigen binding moiety comprising the heavy chain variable region sequence of SEQ ID NO: 255 and the light chain variable region sequence of SEQ ID NO: 263. In one embodiment, the activating T cell antigen binding moiety binds to the same epitope of CD3 as an antigen binding moiety comprising the heavy chain variable region sequence of SEQ ID NO: 255 and the light chain variable region sequence of SEQ ID NO: 263. In another embodiment, the activating T cell antigen binding moiety comprises a humanized version of the heavy chain variable region sequence of SEQ ID NO: 255 and a humanized version of the light chain variable region sequence of SEQ ID NO: 263. In one embodiment, the activating T cell antigen binding moiety comprises the heavy chain CDR1 of SEQ ID NO: 249, the heavy chain CDR2 of SEQ ID NO: 251, the heavy chain CDR3 of SEQ ID NO: 253, the light chain CDR1 of SEQ ID NO: 257, the light chain CDR2 of SEQ ID NO: 259, the light chain CDR3 of SEQ ID NO: 261, and human heavy and light chain variable region framework sequences.

### Target cell antigen binding moiety

The T cell activating bispecific antigen binding molecule of the invention comprises at least one antigen binding moiety capable of binding to a target cell antigen (also referred to herein as an "target cell antigen binding moiety"). In certain embodiments, the T cell activating bispecific antigen binding molecule comprises two antigen binding moieties capable of binding to a target cell antigen. In a particular such embodiment, each of these antigen binding moieties specifically binds to the same antigenic determinant. In one embodiment, the T cell activating bispecific antigen binding molecule comprises an immunoglobulin molecule capable of specific binding to a target cell antigen. In one embodiment the T cell activating bispecific antigen binding molecule comprises not more than two antigen binding moieties capable of binding to a target cell antigen. The target cell antigen binding moiety is generally a Fab molecule that binds to a specific antigenic determinant and is able to direct the T cell activating bispecific antigen binding molecule to a target site, for example to a specific type of tumor cell that bears the antigenic determinant.

In certain embodiments the target cell antigen binding moiety is directed to an antigen associated with a pathological condition, such as an antigen presented on a tumor cell or on a virus-infected cell. Suitable antigens are cell surface antigens, for example, but not limited to, cell surface receptors. In particular embodiments the antigen is a human antigen. In a specific embodiment the target cell antigen is selected from the group of Fibroblast Activation Protein (FAP), Melanoma-associated Chondroitin Sulfate Proteoglycan (MCSP), Epidermal Growth Factor Receptor (EGFR), Carcinoembryonic Antigen (CEA),CD19, CD20 and CD33.

In particular embodiments the T cell activating bispecific antigen binding molecule comprises at least one antigen binding moiety that is specific for Melanoma-associated Chondroitin Sulfate Proteoglycan (MCSP). In one embodiment the T cell activating bispecific antigen binding molecule comprises at least one, typically two or more antigen binding moieties that can compete with monoclonal antibody LC007 (see SEQ ID NOs 75 and 83, and European patent application no. EP 11178393.2, incorporated herein by reference in its entirety) for binding to an epitope of MCSP. In one embodiment, the antigen binding moiety that is specific for MCSP comprises the heavy chain CDR1 of SEQ ID NO: 69, the heavy chain CDR2 of SEQ ID NO: 71, the heavy chain CDR3 of SEQ ID NO: 73, the light chain CDR1 of SEQ ID NO: 77, the light chain CDR2 of SEQ ID NO: 79, and the light chain CDR3 of SEQ ID NO: 81. In a further embodiment, the antigen binding moiety that is specific for MCSP comprises a heavy chain variable region sequence that is at least about 80%, 85%, 90%, 95%, 96%, 97%, 98%, 99% or 100% identical to SEQ ID NO: 75 and a light chain variable region sequence that is at least about 80%, 85%, 90%, 95%, 96%, 97%, 98%, 99% or 100% identical to SEQ ID NO: 83, or variants thereof that retain functionality. In particular embodiments the T cell activating bispecific antigen binding molecule comprises at least one, typically two or more antigen binding moieties that can compete with monoclonal antibody M4-3 ML2 (see SEQ ID NOs 239 and 247, and European patent application no. EP 11178393.2, incorporated herein by reference in its entirety) for binding to an epitope of MCSP. In one embodiment, the antigen binding moiety that is specific for MCSP binds to the same epitope of MCSP as monoclonal antibody M4-3 ML2. In one embodiment, the antigen binding moiety that is specific for MCSP comprises the heavy chain CDR1 of SEQ ID NO: 233, the heavy chain CDR2 of SEQ ID NO: 235, the heavy chain CDR3 of SEQ ID NO: 237, the light chain CDR1 of SEQ ID NO: 241, the light chain CDR2 of SEQ ID NO: 243, and the light chain CDR3 of SEQ ID NO: 245. In a further embodiment, the antigen binding moiety that is specific for MCSP comprises a heavy chain variable region sequence that is at least about 80%, 85%, 90%, 95%, 96%, 97%, 98%, 99% or 100%, particularly about 98%, 99% or 100%, identical to SEQ ID NO: 239 and a light chain variable region sequence that is at least about 80%, 85%, 90%, 95%, 96%, 97%, 98%, 99% or 100%, particularly about 98%, 99% or 100%, identical to SEQ ID NO: 247, or variants thereof that retain functionality. In one embodiment, the antigen binding moiety that is specific for MCSP comprises the heavy and light chain variable region sequences of an affinity matured version of monoclonal antibody M4-3 ML2. In one embodiment, the antigen binding moiety that is specific for MCSP comprises the heavy chain variable region sequence of SEQ ID NO: 239 with one, two, three, four, five, six or seven, particularly two, three, four or five, amino acid substitutions; and the light chain variable region sequence of SEQ ID NO: 247 with one, two, three, four, five, six or seven, particularly two, three, four or five, amino acid substitutions. Any amino acid residue within the variable region sequences may be substituted by a different amino acid, including amino acid residues within the CDR regions, provided that binding to MCSP, particularly human MCSP, is preserved. Preferred variants are those having a binding affinity for MCSP at least equal (or stronger) to the binding affinity of the antigen binding moiety comprising the unsubstituted variable region sequences.

In one embodiment the T cell activating bispecific antigen binding molecule comprises the polypeptide sequence of SEQ ID NO: 1, the polypeptide sequence of SEQ ID NO: 3 and the polypeptide sequence of SEQ ID NO: 5, or variants thereof that retain functionality. In a further embodiment the T cell activating bispecific antigen binding molecule comprises the polypeptide sequence of SEQ ID NO: 7, the polypeptide sequence of SEQ ID NO: 9 and the polypeptide sequence of SEQ ID NO: 11, or variants thereof that retain functionality. In yet another embodiment the T cell activating bispecific antigen binding molecule comprises the polypeptide sequence of SEQ ID NO: 13, the polypeptide sequence of SEQ ID NO: 15 and the polypeptide sequence of SEQ ID NO: 5, or variants thereof that retain functionality. In yet another embodiment the T cell activating bispecific antigen binding molecule comprises the polypeptide sequence of SEQ ID NO: 17, the polypeptide sequence of SEQ ID NO: 19 and the polypeptide sequence of SEQ ID NO: 5, or variants thereof that retain functionality. In another embodiment the T cell activating bispecific antigen binding molecule comprises the polypeptide sequence of SEQ ID NO: 21, the polypeptide sequence of SEQ ID NO: 23 and the polypeptide sequence of SEQ ID NO: 5, or variants thereof that retain functionality. In still another embodiment the T cell activating bispecific antigen binding molecule comprises the polypeptide sequence of SEQ ID NO: 25, the polypeptide sequence of SEQ ID NO: 27 and the polypeptide sequence of SEQ ID NO: 5, or variants thereof that retain functionality. In another embodiment the T cell activating bispecific antigen binding molecule comprises the polypeptide sequence of SEQ ID NO: 29, the polypeptide sequence of SEQ ID NO: 31, the polypeptide sequence of SEQ ID NO: 33, and the polypeptide sequence of SEQ ID NO: 5, or variants thereof that retain functionality. In another embodiment the T cell activating bispecific antigen binding molecule comprises the polypeptide sequence of SEQ ID NO: 29, the polypeptide sequence of SEQ ID NO: 3, the polypeptide sequence of SEQ ID NO: 33, and the polypeptide sequence of SEQ ID NO: 5, or variants thereof that retain functionality. In another embodiment the T cell activating bispecific antigen binding molecule comprises the polypeptide sequence of SEQ ID NO: 35, the polypeptide sequence of SEQ ID NO: 3, the polypeptide sequence of SEQ ID NO: 37, and the polypeptide sequence of SEQ ID NO: 5, or variants thereof that retain functionality. In another embodiment the T cell activating bispecific antigen binding molecule comprises the polypeptide sequence of SEQ ID NO: 39, the polypeptide sequence of SEQ ID NO: 3, the polypeptide sequence of SEQ ID NO: 41, and the polypeptide sequence of SEQ ID NO: 5, or variants thereof that retain functionality. In yet another embodiment the T cell activating bispecific antigen binding molecule comprises the polypeptide sequence of SEQ ID NO: 29, the polypeptide sequence of SEQ ID NO: 3, the polypeptide sequence of SEQ ID NO: 5 and the polypeptide sequence of SEQ ID NO: 179, or variants thereof that retain functionality. In one embodiment the T cell activating bispecific antigen binding molecule comprises the polypeptide sequence of SEQ ID NO: 5, the polypeptide sequence of SEQ ID NO: 29, the polypeptide sequence of SEQ ID NO: 33 and the polypeptide sequence of SEQ ID NO: 181, or variants thereof that retain functionality. In one embodiment the T cell activating bispecific antigen binding molecule comprises the polypeptide sequence of SEQ ID NO: 5, the polypeptide sequence of SEQ ID NO: 23, the polypeptide sequence of SEQ ID NO: 183 and the polypeptide sequence of SEQ ID NO: 185, or variants thereof that retain functionality. In one embodiment the T cell activating bispecific antigen binding molecule comprises the polypeptide sequence of SEQ ID NO: 5, the polypeptide sequence of SEQ ID NO: 23, the polypeptide sequence of SEQ ID NO: 183 and the polypeptide sequence of SEQ ID NO: 187, or variants thereof that retain functionality. In one embodiment the T cell activating bispecific antigen binding molecule comprises the polypeptide sequence of SEQ ID NO: 33, the polypeptide sequence of SEQ ID NO: 189, the polypeptide sequence of SEQ ID NO: 191 and the polypeptide sequence of SEQ ID NO: 193, or variants thereof that retain functionality. In one embodiment the T cell activating bispecific antigen binding molecule comprises the polypeptide sequence of SEQ ID NO: 183, the polypeptide sequence of SEQ ID NO: 189, the polypeptide sequence of SEQ ID NO: 193 and the polypeptide sequence of SEQ ID NO: 195, or variants thereof that retain functionality. In one embodiment the T cell activating bispecific antigen binding molecule comprises the polypeptide sequence of SEQ ID NO: 189, the polypeptide sequence of SEQ ID NO: 193, the polypeptide sequence of SEQ ID NO: 199 and the polypeptide sequence of SEQ ID NO: 201, or variants thereof that retain functionality. In one embodiment the T cell activating bispecific antigen binding molecule comprises the polypeptide sequence of SEQ ID NO: 5, the polypeptide sequence of SEQ ID NO: 23, the polypeptide sequence of SEQ ID NO: 215 and the polypeptide sequence of SEQ ID NO: 217, or variants thereof that retain functionality. In one embodiment the T cell activating bispecific antigen binding molecule comprises the polypeptide sequence of SEQ ID NO: 5, the polypeptide sequence of SEQ ID NO: 23, the polypeptide sequence of SEQ ID NO: 215 and the polypeptide sequence of SEQ ID NO: 219, or variants thereof that retain functionality.

In a specific embodiment the T cell activating bispecific antigen binding molecule comprises a polypeptide sequence encoded by a polynucleotide sequence that is at least about 80%, 85%, 90%, 95%, 96%, 97%, 98%, 99% or 100% identical to a sequence selected from the group of SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 234, SEQ ID NO: 236, SEQ ID NO: 238, SEQ ID NO: 240, SEQ ID NO: 242, SEQ ID NO: 244, SEQ ID NO: 246, SEQ ID NO: 248, SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 180, SEQ ID NO: 182, SEQ ID NO: 184, SEQ ID NO: 186, SEQ ID NO: 188, SEQ ID NO: 190, SEQ ID NO: 192, SEQ ID NO: 194, SEQ ID NO: 196, SEQ ID NO: 200, SEQ ID NO: 202, SEQ ID NO: 216, SEQ ID NO: 218 and SEQ ID NO: 220.

In one embodiment the T cell activating bispecific antigen binding molecule comprises at least one antigen binding moiety that is specific for Epidermal Growth Factor Receptor (EGFR). In another embodiment the T cell activating bispecific antigen binding molecule comprises at least one, typically two or more antigen binding moieties that can compete with monoclonal antibody GA201 for binding to an epitope of EGFR. See PCT publication WO 2006/082515, incorporated herein by reference in its entirety. In one embodiment, the antigen binding moiety that is specific for EGFR comprises the heavy chain CDR1 of SEQ ID NO: 85, the heavy chain CDR2 of SEQ ID NO: 87, the heavy chain CDR3 of SEQ ID NO: 89, the light chain CDR1 of SEQ ID NO: 93, the light chain CDR2 of SEQ ID NO: 95, and the light chain CDR3 of SEQ ID NO: 97. In a further embodiment, the antigen binding moiety that is specific for EGFR comprises a heavy chain variable region sequence that is at least about 80%, 85%, 90%, 95%, 96%, 97%, 98%, 99% or 100% identical to SEQ ID NO: 91 and a light chain variable region sequence that is at least about 80%, 85%, 90%, 95%, 96%, 97%, 98%, 99% or 100% identical to SEQ ID NO: 99, or variants thereof that retain functionality.

In yet another embodiment the T cell activating bispecific antigen binding molecule comprises the polypeptide sequence of SEQ ID NO: 43, the polypeptide sequence of SEQ ID NO: 45 and the polypeptide sequence of SEQ ID NO: 47, or variants thereof that retain functionality. In a further embodiment the T cell activating bispecific antigen binding molecule comprises the polypeptide sequence of SEQ ID NO: 49, the polypeptide sequence of SEQ ID NO: 51 and the polypeptide sequence of SEQ ID NO: 11, or variants thereof that retain functionality. In yet another embodiment the T cell activating bispecific antigen binding molecule comprises the polypeptide sequence of SEQ ID NO: 53, the polypeptide sequence of SEQ ID NO: 45 and the polypeptide sequence of SEQ ID NO: 47, or variants thereof that retain functionality.

In a specific embodiment the T cell activating bispecific antigen binding molecule comprises a polypeptide sequence encoded by a polynucleotide sequence that is at least about 80%, 85%, 90%, 95%, 96%, 97%, 98%, 99% or 100% identical to a sequence selected from the group of SEQ ID NO: 86, SEQ ID NO: 88, SEQ ID NO: 90, SEQ ID NO: 92, SEQ ID NO: 94, SEQ ID NO: 96, SEQ ID NO: 98, SEQ ID NO: 100, SEQ ID NO: 44, SEQ ID NO: 46, SEQ ID NO: 48, SEQ ID NO: 50, SEQ ID NO: 52, SEQ ID NO: 54 and SEQ ID NO: 12.

In one embodiment the T cell activating bispecific antigen binding molecule comprises at least one antigen binding moiety that is specific for Fibroblast Activation Protein (FAP). In another embodiment the T cell activating bispecific antigen binding molecule comprises at least one, typically two or more antigen binding moieties that can compete with monoclonal antibody 3F2 for binding to an epitope of FAP. See PCT publication WO 2012/020006, incorporated herein by reference in its entirety. In one embodiment, the antigen binding moiety that is specific for FAP comprises the heavy chain CDR1 of SEQ ID NO: 101, the heavy chain CDR2 of SEQ ID NO: 103, the heavy chain CDR3 of SEQ ID NO: 105, the light chain CDR1 of SEQ ID NO: 109, the light chain CDR2 of SEQ ID NO: 111, and the light chain CDR3 of SEQ ID NO: 113. In a further embodiment, the antigen binding moiety that is specific for FAP comprises a heavy chain variable region sequence that is at least about 80%, 85%, 90%, 95%, 96%, 97%, 98%, 99% or 100% identical to SEQ ID NO: 107 and a light chain variable region sequence that is at least about 80%, 85%, 90%, 95%, 96%, 97%, 98%, 99% or 100% identical to SEQ ID NO: 115, or variants thereof that retain functionality.

In yet another embodiment the T cell activating bispecific antigen binding molecule comprises the polypeptide sequence of SEQ ID NO: 55, the polypeptide sequence of SEQ ID NO: 51 and the polypeptide sequence of SEQ ID NO: 11, or variants thereof that retain functionality. In a further embodiment the T cell activating bispecific antigen binding molecule comprises the polypeptide sequence of SEQ ID NO: 57, the polypeptide sequence of SEQ ID NO: 59 and the polypeptide sequence of SEQ ID NO: 61, or variants thereof that retain functionality.

In a specific embodiment the T cell activating bispecific antigen binding molecule comprises a polypeptide sequence encoded by a polynucleotide sequence that is at least about 80%, 85%, 90%, 95%, 96%, 97%, 98%, 99% or 100% identical to a sequence selected from the group of SEQ ID NO: 102, SEQ ID NO: 104, SEQ ID NO: 106, SEQ ID NO: 108, SEQ ID NO: 110, SEQ ID NO: 112, SEQ ID NO: 114, SEQ ID NO: 116, SEQ ID NO: 56, SEQ ID NO: 58, SEQ ID NO: 60, SEQ ID NO: 62, SEQ ID NO: 52 and SEQ ID NO: 12.

In particular embodiments the T cell activating bispecific antigen binding molecule comprises at least one antigen binding moiety that is specific for Carcinoembryonic Antigen (CEA). In one embodiment the T cell activating bispecific antigen binding molecule comprises at least one, typically two or more antigen binding moieties that can compete with monoclonal antibody BW431/26 (described in European patent no. EP 160 897, and Bosslet et al., Int J Cancer 36, 75-84 (1985)) for binding to an epitope of CEA. In one embodiment the T cell activating bispecific antigen binding molecule comprises at least one, typically two or more antigen binding moieties that can compete with monoclonal antibody CH1A1A (see SEQ ID NOs 123 and 131) for binding to an epitope of CEA. See PCT patent publication number WO 2011/023787, incorporated herein by reference in its entirety. In one embodiment, the antigen binding moiety that is specific for CEA binds to the same epitope of CEA as monoclonal antibody CH1A1A. In one embodiment, the antigen binding moiety that is specific for CEA comprises the heavy chain CDR1 of SEQ ID NO: 117, the heavy chain CDR2 of SEQ ID NO: 119, the heavy chain CDR3 of SEQ ID NO: 121, the light chain CDR1 of SEQ ID NO: 125, the light chain CDR2 of SEQ ID NO: 127, and the light chain CDR3 of SEQ ID NO: 129. In a further embodiment, the antigen binding moiety that is specific for CEA comprises a heavy chain variable region sequence that is at least about 80%, 85%, 90%, 95%, 96%, 97%, 98%, 99% or 100%, particularly about 98%, 99% or 100%, identical to SEQ ID NO: 123 and a light chain variable region sequence that is at least about 80%, 85%, 90%, 95%, 96%, 97%, 98%, 99% or 100%, particularly about 98%, 99% or 100%, identical to SEQ ID NO: 131, or variants thereof that retain functionality. In one embodiment, the antigen binding moiety that is specific for CEA comprises the heavy and light chain variable region sequences of an affinity matured version of monoclonal antibody CH1A1A. In one embodiment, the antigen binding moiety that is specific for CEA comprises the heavy chain variable region sequence of SEQ ID NO: 123 with one, two, three, four, five, six or seven, particularly two, three, four or five, amino acid substitutions; and the light chain variable region sequence of SEQ ID NO: 131 with one, two, three, four, five, six or seven, particularly two, three, four or five, amino acid substitutions. Any amino acid residue within the variable region sequences may be substituted by a different amino acid, including amino acid residues within the CDR regions, provided that binding to CEA, particularly human CEA, is preserved. Preferred variants are those having a binding affinity for CEA at least equal (or stronger) to the binding affinity of the antigen binding moiety comprising the unsubstituted variable region sequences.

In one embodiment the T cell activating bispecific antigen binding molecule comprises the polypeptide sequence of SEQ ID NO: 63, the polypeptide sequence of SEQ ID NO: 65, the polypeptide sequence of SEQ ID NO: 67 and the polypeptide sequence of SEQ ID NO: 33, or variants thereof that retain functionality. In one embodiment the T cell activating bispecific antigen binding molecule comprises the polypeptide sequence of SEQ ID NO: 65, the polypeptide sequence of SEQ ID NO: 67, the polypeptide sequence of SEQ ID NO: 183 and the polypeptide sequence of SEQ ID NO: 197, or variants thereof that retain functionality. In one embodiment the T cell activating bispecific antigen binding molecule comprises the polypeptide sequence of SEQ ID NO: 183, the polypeptide sequence of SEQ ID NO: 203, the polypeptide sequence of SEQ ID NO: 205 and the polypeptide sequence of SEQ ID NO: 207, or variants thereof that retain functionality. In one embodiment the T cell activating bispecific antigen binding molecule comprises the polypeptide sequence of SEQ ID NO: 183, the polypeptide sequence of SEQ ID NO: 209, the polypeptide sequence of SEQ ID NO: 211 and the polypeptide sequence of SEQ ID NO: 213, or variants thereof that retain functionality.

In a specific embodiment the T cell activating bispecific antigen binding molecule comprises a polypeptide sequence encoded by a polynucleotide sequence that is at least about 80%, 85%, 90%, 95%, 96%, 97%, 98%, 99% or 100% identical to a sequence selected from the group of SEQ ID NO: 118, SEQ ID NO: 120, SEQ ID NO: 122, SEQ ID NO: 124, SEQ ID NO: 126, SEQ ID NO: 128, SEQ ID NO: 130, SEQ ID NO: 132, SEQ ID NO: 64, SEQ ID NO: 66, SEQ ID NO: 68, SEQ ID NO: 34, SEQ ID NO: 184, SEQ ID NO: 198, SEQ ID NO: 204, SEQ ID NO: 206, SEQ ID NO: 208, SEQ ID NO: 210, SEQ ID NO: 212 and SEQ ID NO: 214.

In one embodiment the T cell activating bispecific antigen binding molecule comprises at least one antigen binding moiety that is specific for CD33. In one embodiment, the antigen binding moiety that is specific for CD33 comprises the heavy chain CDR1 of SEQ ID NO: 133, the heavy chain CDR2 of SEQ ID NO: 135, the heavy chain CDR3 of SEQ ID NO: 137, the light chain CDR1 of SEQ ID NO: 141, the light chain CDR2 of SEQ ID NO: 143, and the light chain CDR3 of SEQ ID NO: 145. In a further embodiment, the antigen binding moiety that is specific for CD33 comprises a heavy chain variable region sequence that is at least about 80%, 85%, 90%, 95%, 96%, 97%, 98%, 99% or 100% identical to SEQ ID NO: 139 and a light chain variable region sequence that is at least about 80%, 85%, 90%, 95%, 96%, 97%, 98%, 99% or 100% identical to SEQ ID NO: 147, or variants thereof that retain functionality.

In one embodiment the T cell activating bispecific antigen binding molecule comprises the polypeptide sequence of SEQ ID NO: 33, the polypeptide sequence of SEQ ID NO: 213, the polypeptide sequence of SEQ ID NO: 221 and the polypeptide sequence of SEQ ID NO: 223, or variants thereof that retain functionality. In one embodiment the T cell activating bispecific antigen binding molecule comprises the polypeptide sequence of SEQ ID NO: 33, the polypeptide sequence of SEQ ID NO: 221, the polypeptide sequence of SEQ ID NO: 223 and the polypeptide sequence of SEQ ID NO: 225, or variants thereof that retain functionality.

In a specific embodiment the T cell activating bispecific antigen binding molecule comprises a polypeptide sequence encoded by a polynucleotide sequence that is at least about 80%, 85%, 90%, 95%, 96%, 97%, 98%, 99% or 100% identical to a sequence selected from the group of SEQ ID NO: 134, SEQ ID NO: 136, SEQ ID NO: 138, SEQ ID NO: 140, SEQ ID NO: 142, SEQ ID NO: 144, SEQ ID NO: 146, SEQ ID NO: 148, SEQ ID NO: 34, SEQ ID NO: 214, SEQ ID NO: 222, SEQ ID NO: 224 and SEQ ID NO: 226.

### Polynucleotides

The invention further provides isolated polynucleotides encoding a T cell activating bispecific antigen binding molecule as described herein or a fragment thereof.

Polynucleotides of the invention include those that are at least about 80%, 85%, 90%, 95%, 96%, 97%, 98%, 99%, or 100% identical to the sequences set forth in SEQ ID NOs 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262 and 264, including functional fragments or variants thereof.

The polynucleotides encoding T cell activating bispecific antigen binding molecules of the invention may be expressed as a single polynucleotide that encodes the entire T cell activating bispecific antigen binding molecule or as multiple (e.g., two or more) polynucleotides that are co-expressed. Polypeptides encoded by polynucleotides that are co-expressed may associate through, e.g., disulfide bonds or other means to form a functional T cell activating bispecific antigen binding molecule. For example, the light chain portion of an antigen binding moiety may be encoded by a separate polynucleotide from the portion of the T cell activating bispecific antigen binding molecule comprising the heavy chain portion of the antigen binding moiety, an Fc domain subunit and optionally (part of) another antigen binding moiety. When co-expressed, the heavy chain polypeptides will associate with the light chain polypeptides to form the antigen binding moiety. In another example, the portion of the T cell activating bispecific antigen binding molecule comprising one of the two Fc domain subunits and optionally (part of) one or more antigen binding moieties could be encoded by a separate polynucleotide from the portion of the T cell activating bispecific antigen binding molecule comprising the the other of the two Fc domain subunits and optionally (part of) an antigen binding moiety. When co-expressed, the Fc domain subunits will associate to form the Fc domain.

In certain embodiments, an isolated polynucleotide of the invention encodes a fragment of a T cell activating bispecific antigen binding molecule comprising a first and a second antigen binding moiety, and an Fc domain consisting of two subunits, wherein the first antigen binding moiety is a single chain Fab molecule. In one embodiment, an isolated polynucleotide of the invention encodes the first antigen binding moiety and a subunit of the Fc domain. In a more specific embodiment the isolated polynucleotide encodes a polypeptide wherein a single chain Fab molecule shares a carboxy-terminal peptide bond with an Fc domain subunit. In another embodiment, an isolated polynucleotide of the invention encodes the heavy chain of the second antigen binding moiety and a subunit of the Fc domain. In a more specific embodiment the isolated polynucleotide encodes a polypeptide wherein a Fab heavy chain shares a carboxy terminal peptide bond with an Fc domain subunit. In yet another embodiment, an isolated polynucleotide of the invention encodes the first antigen binding moiety, the heavy chain of the second antigen binding moiety and a subunit of the Fc domain. In a more specific embodiment, the isolated polynucleotide encodes a polypeptide wherein a single chain Fab molecule shares a carboxy-terminal peptide bond with a Fab heavy chain, which in turn shares a carboxy-terminal peptide bond with an Fc domain subunit.

In certain embodiments, an isolated polynucleotide of the invention encodes a fragment of a T cell activating bispecific antigen binding molecule comprising a first and a second antigen binding moiety, and an Fc domain consisting of two subunits, wherein the first antigen binding moiety is a crossover Fab molecule. In one embodiment, an isolated polynucleotide of the invention encodes the heavy chain of the first antigen binding moiety and a subunit of the Fc domain. In a more specific embodiment the isolated polynucleotide encodes a polypeptide wherein Fab light chain variable region shares a carboxy terminal peptide bond with a Fab heavy chain constant region, which in turn shares a carboxy-terminal peptide bond with an Fc domain subunit. In another specific embodiment the isolated polynucleotide encodes a polypeptide wherein Fab heavy chain variable region shares a carboxy terminal peptide bond with a Fab light chain constant region, which in turn shares a carboxy-terminal peptide bond with an Fc domain subunit. In another embodiment, an isolated polynucleotide of the invention encodes the heavy chain of the second antigen binding moiety and a subunit of the Fc domain. In a more specific embodiment the isolated polynucleotide encodes a polypeptide wherein a Fab heavy chain shares a carboxy terminal peptide bond with an Fc domain subunit. In yet another embodiment, an isolated polynucleotide of the invention encodes the heavy chain of the first antigen binding moiety, the heavy chain of the second antigen binding moiety and a subunit of the Fc domain. In a more specific embodiment, the isolated polynucleotide encodes a polypeptide wherein a Fab light chain variable region shares a carboxy-terminal peptide bond with a Fab heavy chain constant region, which in turn shares a carboxy-terminal peptide bond with a Fab heavy chain, which in turn shares a carboxy-terminal peptide bond with an Fc domain subunit. In another specific embodiment, the isolated polynucleotide encodes a polypeptide wherein a Fab heavy chain variable region shares a carboxy-terminal peptide bond with a Fab light chain constant region, which in turn shares a carboxy-terminal peptide bond with a Fab heavy chain, which in turn shares a carboxy-terminal peptide bond with an Fc domain subunit. In yet another specific embodiment the isolated polynucleotide encodes a polypeptide wherein a Fab heavy chain shares a carboxy-terminal peptide bond with a Fab light chain variable region, which in turn shares a carboxy-terminal peptide bond with a Fab heavy chain constant region, which in turn shares a carboxy-terminal peptide bond with an Fc domain subunit. In still another specific embodiment the isolated polynucleotide encodes a polypeptide wherein a Fab heavy chain shares a carboxy-terminal peptide bond with a Fab heavy chain variable region, which in turn shares a carboxy-terminal peptide bond with a Fab light chain constant region, which in turn shares a carboxy-terminal peptide bond with an Fc domain subunit.

In further embodiments, an isolated polynucleotide of the invention encodes the heavy chain of a third antigen binding moiety and a subunit of the Fc domain. In a more specific embodiment the isolated polynucleotide encodes a polypeptide wherein a Fab heavy chain shares a carboxy terminal peptide bond with an Fc domain subunit.

In further embodiments, an isolated polynucleotide of the invention encodes the light chain of an antigen binding moiety. In some embodiments, the isolated polynucleotide encodes a polypeptide wherein a Fab light chain variable region shares a carboxy-terminal peptide bond with a Fab heavy chain constant region. In other embodiments, the isolated polynucleotide encodes a polypeptide wherein a Fab heavy chain variable region shares a carboxy-terminal peptide bond with a Fab light chain constant region. In still other embodiments, an isolated polynucleotide of the invention encodes the light chain of the first antigen binding moiety and the light chain of the second antigen binding moiety. In a more specific embodiment, the isolated polynucleotide encodes a polypeptide wherein a Fab heavy chain variable region shares a carboxy-terminal peptide bond with a Fab light chain constant region, which in turn shares a carboxy-terminal peptide bond with a Fab light chain. In another specific embodiment the isolated polynucleotide encodes a polypeptide wherein a Fab light chain shares a carboxy-terminal peptide bond with a Fab heavy chain variable region, which in turn shares a carboxy-terminal peptide bond with a Fab light chain constant region. In yet another specific embodiment, the isolated polynucleotide encodes a polypeptide wherein a Fab light chain variable region shares a carboxy-terminal peptide bond with a Fab heavy chain constant region, which in turn shares a carboxy-terminal peptide bond with a Fab light chain. In yet another specific embodiment the isolated polynucleotide encodes a polypeptide wherein a Fab light chain shares a carboxy-terminal peptide bond with a Fab light chain variable region, which in turn shares a carboxy-terminal peptide bond with a Fab heavy chain constant region.

In another embodiment, the present invention is directed to an isolated polynucleotide encoding a T cell activating bispecific antigen binding molecule of the invention or a fragment thereof, wherein the polynucleotide comprises a sequence that encodes a variable region sequence as shown in SEQ ID NOs 75, 83, 91, 99, 107, 115, 123, 131, 139, 147, 169, 177, 239, 247, 255 and 263. In another embodiment, the present invention is directed to an isolated polynucleotide encoding a T cell activating bispecific antigen binding molecule or fragment thereof, wherein the polynucleotide comprises a sequence that encodes a polypeptide sequence as shown in SEQ ID NOs 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 179, 181, 183, 185, 187, 189, 191, 193, 195, 197, 199, 201, 203, 205, 207, 209, 211, 213, 215, 217, 219, 221, 223, 225, 227, 229 and 231. In another embodiment, the invention is further directed to an isolated polynucleotide encoding a T cell activating bispecific antigen binding molecule of the invention or a fragment thereof, wherein the polynucleotide comprises a sequence that is at least about 80%, 85%, 90%, 95%, 96%, 97%, 98%, or 99% identical to a nucleotide sequence shown in SEQ ID NOs 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262 or 264. In another embodiment, the invention is directed to an isolated polynucleotide encoding a T cell activating bispecific antigen binding molecule of the invention or a fragment thereof, wherein the polynucleotide comprises a nucleic acid sequence shown in SEQ ID NOs 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262 or 264. In another embodiment, the invention is directed to an isolated polynucleotide encoding a T cell activating bispecific antigen binding molecule of the invention or a fragment thereof, wherein the polynucleotide comprises a sequence that encodes a variable region sequence that is at least about 80%, 85%, 90%, 95%, 96%, 97%, 98%, or 99% identical to an amino acid sequence in SEQ ID NOs 75, 83, 91, 99, 107, 115, 123, 131, 139, 147, 169, 177, 239, 247, 255 or 263. In another embodiment, the invention is directed to an isolated polynucleotide encoding a T cell activating bispecific antigen binding molecule or fragment thereof, wherein the polynucleotide comprises a sequence that encodes a polypeptide sequence that is at least 80%, 85%, 90%, 95%, 96%, 97%, 98%, or 99% identical to an amino acid sequence in SEQ ID NOs 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 179, 181, 183, 185, 187, 189, 191, 193, 195, 197, 199, 201, 203, 205, 207, 209, 211, 213, 215, 217, 219, 221, 223, 225, 227, 229 or 231. The invention encompasses an isolated polynucleotide encoding a T cell activating bispecific antigen binding molecule of the invention or a fragment thereof, wherein the polynucleotide comprises a sequence that encodes the variable region sequence of SEQ ID NOs 75, 83, 91, 99, 107, 115, 123, 131, 139, 147, 169, 177, 239, 247, 255 or 263 with conservative amino acid substitutions. The invention also encompasses an isolated polynucleotide encoding a T cell activating bispecific antigen binding molecule of the invention or fragment thereof, wherein the polynucleotide comprises a sequence that encodes the polypeptide sequence of SEQ ID NOs 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 179, 181, 183, 185, 187, 189, 191, 193, 195, 197, 199, 201, 203, 205, 207, 209, 211, 213, 215, 217, 219, 221, 223, 225, 227, 229 or 231 with conservative amino acid substitutions.

In certain embodiments the polynucleotide or nucleic acid is DNA. In other embodiments, a polynucleotide of the present invention is RNA, for example, in the form of messenger RNA (mRNA). RNA of the present invention may be single stranded or double stranded.

### Recombinant Methods

T cell activating bispecific antigen binding molecules of the invention may be obtained, for example, by solid-state peptide synthesis (e.g. Merrifield solid phase synthesis) or recombinant production. For recombinant production one or more polynucleotide encoding the T cell activating bispecific antigen binding molecule (fragment), e.g., as described above, is isolated and inserted into one or more vectors for further cloning and/or expression in a host cell. Such polynucleotide may be readily isolated and sequenced using conventional procedures. In one embodiment a vector, preferably an expression vector, comprising one or more of the polynucleotides of the invention is provided. Methods which are well known to those skilled in the art can be used to construct expression vectors containing the coding sequence of a T cell activating bispecific antigen binding molecule (fragment) along with appropriate transcriptional/translational control signals. These methods include *in vitro* recombinant DNA techniques, synthetic techniques and *in vivo* recombination/genetic recombination. See, for example, the techniques described in Maniatis et al., MOLECULAR CLONING: A LABORATORY MANUAL, Cold Spring Harbor Laboratory, N.Y. (1989); and Ausubel et al., CURRENT PROTOCOLS IN MOLECULAR BIOLOGY, Greene Publishing Associates and Wiley Interscience, N.Y (1989). The expression vector can be part of a plasmid, virus, or may be a nucleic acid fragment. The expression vector includes an expression cassette into which the polynucleotide encoding the T cell activating bispecific antigen binding molecule (fragment) (i.e. the coding region) is cloned in operable association with a promoter and/or other transcription or translation control elements. As used herein, a "coding region" is a portion of nucleic acid which consists of codons translated into amino acids. Although a "stop codon" (TAG, TGA, or TAA) is not translated into an amino acid, it may be considered to be part of a coding region, if present, but any flanking sequences, for example promoters, ribosome binding sites, transcriptional terminators, introns, 5' and 3' untranslated regions, and the like, are not part of a coding region. Two or more coding regions can be present in a single polynucleotide construct, e.g. on a single vector, or in separate polynucleotide constructs, e.g. on separate (different) vectors. Furthermore, any vector may contain a single coding region, or may comprise two or more coding regions, e.g. a vector of the present invention may encode one or more polypeptides, which are post- or cotranslationally separated into the final proteins via proteolytic cleavage. In addition, a vector, polynucleotide, or nucleic acid of the invention may encode heterologous coding regions, either fused or unfused to a polynucleotide encoding the T cell activating bispecific antigen binding molecule (fragment) of the invention, or variant or derivative thereof. Heterologous coding regions include without limitation specialized elements or motifs, such as a secretory signal peptide or a heterologous functional domain. An operable association is when a coding region for a gene product, e.g. a polypeptide, is associated with one or more regulatory sequences in such a way as to place expression of the gene product under the influence or control of the regulatory sequence(s). Two DNA fragments (such as a polypeptide coding region and a promoter associated therewith) are "operably associated" if induction of promoter function results in the transcription of mRNA encoding the desired gene product and if the nature of the linkage between the two DNA fragments does not interfere with the ability of the expression regulatory sequences to direct the expression of the gene product or interfere with the ability of the DNA template to be transcribed. Thus, a promoter region would be operably associated with a nucleic acid encoding a polypeptide if the promoter was capable of effecting transcription of that nucleic acid. The promoter may be a cell-specific promoter that directs substantial transcription of the DNA only in predetermined cells. Other transcription control elements, besides a promoter, for example enhancers, operators, repressors, and transcription termination signals, can be operably associated with the polynucleotide to direct cell-specific transcription. Suitable promoters and other transcription control regions are disclosed herein. A variety of transcription control regions are known to those skilled in the art. These include, without limitation, transcription control regions, which function in vertebrate cells, such as, but not limited to, promoter and enhancer segments from cytomegaloviruses (e.g. the immediate early promoter, in conjunction with intron-A), simian virus 40 (e.g. the early promoter), and retroviruses (such as, e.g. Rous sarcoma virus). Other transcription control regions include those derived from vertebrate genes such as actin, heat shock protein, bovine growth hormone and rabbit â-globin, as well as other sequences capable of controlling gene expression in eukaryotic cells. Additional suitable transcription control regions include tissue-specific promoters and enhancers as well as inducible promoters (e.g. promoters inducible tetracyclins). Similarly, a variety of translation control elements are known to those of ordinary skill in the art. These include, but are not limited to ribosome binding sites, translation initiation and termination codons, and elements derived from viral systems (particularly an internal ribosome entry site, or IRES, also referred to as a CITE sequence). The expression cassette may also include other features such as an origin of replication, and/or chromosome integration elements such as retroviral long terminal repeats (LTRs), or adeno-associated viral (AAV) inverted terminal repeats (ITRs).

Polynucleotide and nucleic acid coding regions of the present invention may be associated with additional coding regions which encode secretory or signal peptides, which direct the secretion of a polypeptide encoded by a polynucleotide of the present invention. For example, if secretion of the T cell activating bispecific antigen binding molecule is desired, DNA encoding a signal sequence may be placed upstream of the nucleic acid encoding a T cell activating bispecific antigen binding molecule of the invention or a fragment thereof. According to the signal hypothesis, proteins secreted by mammalian cells have a signal peptide or secretory leader sequence which is cleaved from the mature protein once export of the growing protein chain across the rough endoplasmic reticulum has been initiated. Those of ordinary skill in the art are aware that polypeptides secreted by vertebrate cells generally have a signal peptide fused to the N-terminus of the polypeptide, which is cleaved from the translated polypeptide to produce a secreted or "mature" form of the polypeptide. In certain embodiments, the native signal peptide, *e.g.* an immunoglobulin heavy chain or light chain signal peptide is used, or a functional derivative of that sequence that retains the ability to direct the secretion of the polypeptide that is operably associated with it. Alternatively, a heterologous mammalian signal peptide, or a functional derivative thereof, may be used. For example, the wild-type leader sequence may be substituted with the leader sequence of human tissue plasminogen activator (TPA) or mouse β-glucuronidase. Exemplary amino acid and polynucleotide sequences of secretory signal peptides are given in SEQ ID NOs 154-162.

DNA encoding a short protein sequence that could be used to facilitate later purification (e.g. a histidine tag) or assist in labeling the T cell activating bispecific antigen binding molecule may be included within or at the ends of the T cell activating bispecific antigen binding molecule (fragment) encoding polynucleotide.

In a further embodiment, a host cell comprising one or more polynucleotides of the invention is provided. In certain embodiments a host cell comprising one or more vectors of the invention is provided. The polynucleotides and vectors may incorporate any of the features, singly or in combination, described herein in relation to polynucleotides and vectors, respectively. In one such embodiment a host cell comprises (e.g. has been transformed or transfected with) a vector comprising a polynucleotide that encodes (part of) a T cell activating bispecific antigen binding molecule of the invention. As used herein, the term "host cell" refers to any kind of cellular system which can be engineered to generate the T cell activating bispecific antigen binding molecules of the invention or fragments thereof. Host cells suitable for replicating and for supporting expression of T cell activating bispecific antigen binding molecules are well known in the art. Such cells may be transfected or transduced as appropriate with the particular expression vector and large quantities of vector containing cells can be grown for seeding large scale fermenters to obtain sufficient quantities of the T cell activating bispecific antigen binding molecule for clinical applications. Suitable host cells include prokaryotic microorganisms, such as E. coli, or various eukaryotic cells, such as Chinese hamster ovary cells (CHO), insect cells, or the like. For example, polypeptides may be produced in bacteria in particular when glycosylation is not needed. After expression, the polypeptide may be isolated from the bacterial cell paste in a soluble fraction and can be further purified. In addition to prokaryotes, eukaryotic microbes such as filamentous fungi or yeast are suitable cloning or expression hosts for polypeptide-encoding vectors, including fungi and yeast strains whose glycosylation pathways have been "humanized", resulting in the production of a polypeptide with a partially or fully human glycosylation pattern. See Gerngross, Nat Biotech 22, 1409-1414 (2004), and Li et al., Nat Biotech 24, 210-215 (2006). Suitable host cells for the expression of (glycosylated) polypeptides are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells. Plant cell cultures can also be utilized as hosts. See e.g. US Patent Nos. 5,959,177, 6,040,498, 6,420,548, 7,125,978, and 6,417,429 (describing PLANTIBODIES™ technology for producing antibodies in transgenic plants). Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293T cells as described, e.g., in Graham et al., J Gen Virol 36, 59 (1977)), baby hamster kidney cells (BHK), mouse sertoli cells (TM4 cells as described, e.g., in Mather, Biol Reprod 23, 243-251 (1980)), monkey kidney cells (CV1), African green monkey kidney cells (VERO-76), human cervical carcinoma cells (HELA), canine kidney cells (MDCK), buffalo rat liver cells (BRL 3A), human lung cells (W138), human liver cells (Hep G2), mouse mammary tumor cells (MMT 060562), TRI cells (as described, e.g., in Mather et al., Annals N.Y. Acad Sci 383, 44-68 (1982)), MRC 5 cells, and FS4 cells. Other useful mammalian host cell lines include Chinese hamster ovary (CHO) cells, including dhfr⁻ CHO cells (Urlaub et al., Proc Natl Acad Sci USA 77, 4216 (1980)); and myeloma cell lines such as YO, NS0, P3X63 and Sp2/0. For a review of certain mammalian host cell lines suitable for protein production, see, e.g., Yazaki and Wu, Methods in Molecular Biology, Vol. 248 (B.K.C. Lo, ed., Humana Press, Totowa, NJ), pp. 255-268 (2003). Host cells include cultured cells, e.g., mammalian cultured cells, yeast cells, insect cells, bacterial cells and plant cells, to name only a few, but also cells comprised within a transgenic animal, transgenic plant or cultured plant or animal tissue. In one embodiment, the host cell is a eukaryotic cell, preferably a mammalian cell, such as a Chinese Hamster Ovary (CHO) cell, a human embryonic kidney (HEK) cell or a lymphoid cell (e.g., Y0, NS0, Sp20 cell).

Standard technologies are known in the art to express foreign genes in these systems. Cells expressing a polypeptide comprising either the heavy or the light chain of an antigen binding domain such as an antibody, may be engineered so as to also express the other of the antibody chains such that the expressed product is an antibody that has both a heavy and a light chain.

In one embodiment, a method of producing a T cell activating bispecific antigen binding molecule according to the invention is provided, wherein the method comprises culturing a host cell comprising a polynucleotide encoding the T cell activating bispecific antigen binding molecule, as provided herein, under conditions suitable for expression of the T cell activating bispecific antigen binding molecule, and recovering the T cell activating bispecific antigen binding molecule from the host cell (or host cell culture medium).

The components of the T cell activating bispecific antigen binding molecule are genetically fused to each other. T cell activating bispecific antigen binding molecule can be designed such that its components are fused directly to each other or indirectly through a linker sequence. The composition and length of the linker may be determined in accordance with methods well known in the art and may be tested for efficacy. Examples of linker sequences between different components of T cell activating bispecific antigen binding molecules are found in the sequences provided herein. Additional sequences may also be included to incorporate a cleavage site to separate the individual components of the fusion if desired, for example an endopeptidase recognition sequence.

In certain embodiments the one or more antigen binding moieties of the T cell activating bispecific antigen binding molecules comprise at least an antibody variable region capable of binding an antigenic determinant. Variable regions can form part of and be derived from naturally or non-naturally occurring antibodies and fragments thereof. Methods to produce polyclonal antibodies and monoclonal antibodies are well known in the art (see e.g. Harlow and Lane, "Antibodies, a laboratory manual", Cold Spring Harbor Laboratory, 1988). Non-naturally occurring antibodies can be constructed using solid phase-peptide synthesis, can be produced recombinantly (e.g. as described in U.S. patent No. 4,186,567) or can be obtained, for example, by screening combinatorial libraries comprising variable heavy chains and variable light chains (see e.g. U.S. Patent. No. 5,969,108 to McCafferty).

Any animal species of antibody, antibody fragment, antigen binding domain or variable region can be used in the T cell activating bispecific antigen binding molecules of the invention. Non-limiting antibodies, antibody fragments, antigen binding domains or variable regions useful in the present invention can be of murine, primate, or human origin. If the T cell activating bispecific antigen binding molecule is intended for human use, a chimeric form of antibody may be used wherein the constant regions of the antibody are from a human. A humanized or fully human form of the antibody can also be prepared in accordance with methods well known in the art (see e. g. U.S. Patent No. 5,565,332 to Winter). Humanization may be achieved by various methods including, but not limited to (a) grafting the non-human (e.g., donor antibody) CDRs onto human (e.g. recipient antibody) framework and constant regions with or without retention of critical framework residues (e.g. those that are important for retaining good antigen binding affinity or antibody functions), (b) grafting only the non-human specificity-determining regions (SDRs or a-CDRs; the residues critical for the antibody-antigen interaction) onto human framework and constant regions, or (c) transplanting the entire non-human variable domains, but "cloaking" them with a human-like section by replacement of surface residues. Humanized antibodies and methods of making them are reviewed, e.g., in Almagro and Fransson, Front Biosci 13, 1619-1633 (2008), and are further described, e.g., in Riechmann et al., Nature 332, 323-329 (1988); Queen et al., Proc Natl Acad Sci USA 86, 10029-10033 (1989); US Patent Nos. 5,821,337, 7,527,791, 6,982,321, and 7,087,409; Jones et al., Nature 321, 522-525 (1986); Morrison et al., Proc Natl Acad Sci 81, 6851-6855 (1984); Morrison and Oi, Adv Immunol 44, 65-92 (1988); Verhoeyen et al., Science 239, 1534-1536 (1988); Padlan, Molec Immun 31(3), 169-217 (1994); Kashmiri et al., Methods 36, 25-34 (2005) (describing SDR (a-CDR) grafting); Padlan, Mol Immunol 28, 489-498 (1991) (describing "resurfacing"); Dall'Acqua et al., Methods 36, 43-60 (2005) (describing "FR shuffling"); and Osbourn et al., Methods 36, 61-68 (2005) and Klimka et al., Br J Cancer 83, 252-260 (2000) (describing the "guided selection" approach to FR shuffling). Human antibodies and human variable regions can be produced using various techniques known in the art. Human antibodies are described generally in van Dijk and van de Winkel, Curr Opin Pharmacol 5, 368-74 (2001) and Lonberg, Curr Opin Immunol 20, 450-459 (2008). Human variable regions can form part of and be derived from human monoclonal antibodies made by the hybridoma method (see e.g. Monoclonal Antibody Production Techniques and Applications, pp. 51-63 (Marcel Dekker, Inc., New York, 1987)). Human antibodies and human variable regions may also be prepared by administering an immunogen to a transgenic animal that has been modified to produce intact human antibodies or intact antibodies with human variable regions in response to antigenic challenge (see e.g. Lonberg, Nat Biotech 23, 1117-1125 (2005). Human antibodies and human variable regions may also be generated by isolating Fv clone variable region sequences selected from human-derived phage display libraries (see e.g., Hoogenboom et al. in Methods in Molecular Biology 178, 1-37 (O'Brien et al., ed., Human Press, Totowa, NJ, 2001); and McCafferty et al., Nature 348, 552-554; Clackson et al., Nature 352, 624-628 (1991)). Phage typically display antibody fragments, either as single-chain Fv (scFv) fragments or as Fab fragments.

In certain embodiments, the antigen binding moieties useful in the present invention are engineered to have enhanced binding affinity according to, for example, the methods disclosed in U.S. Pat. Appl. Publ. No. 2004/0132066, the entire contents of which are hereby incorporated by reference. The ability of the T cell activating bispecific antigen binding molecule of the invention to bind to a specific antigenic determinant can be measured either through an enzymelinked immunosorbent assay (ELISA) or other techniques familiar to one of skill in the art, e.g. surface plasmon resonance technique (analyzed on a BIACORE T100 system) (Liljeblad, et al., Glyco J 17, 323-329 (2000)), and traditional binding assays (Heeley, Endocr Res 28, 217-229 (2002)). Competition assays may be used to identify an antibody, antibody fragment, antigen binding domain or variable domain that competes with a reference antibody for binding to a particular antigen, e.g. an antibody that competes with the V9 antibody for binding to CD3. In certain embodiments, such a competing antibody binds to the same epitope (e.g. a linear or a conformational epitope) that is bound by the reference antibody. Detailed exemplary methods for mapping an epitope to which an antibody binds are provided in Morris (1996) "Epitope Mapping Protocols," in Methods in Molecular Biology vol. 66 (Humana Press, Totowa, NJ). In an exemplary competition assay, immobilized antigen (e.g. CD3) is incubated in a solution comprising a first labeled antibody that binds to the antigen (e.g. V9 antibody) and a second unlabeled antibody that is being tested for its ability to compete with the first antibody for binding to the antigen. The second antibody may be present in a hybridoma supernatant. As a control, immobilized antigen is incubated in a solution comprising the first labeled antibody but not the second unlabeled antibody. After incubation under conditions permissive for binding of the first antibody to the antigen, excess unbound antibody is removed, and the amount of label associated with immobilized antigen is measured. If the amount of label associated with immobilized antigen is substantially reduced in the test sample relative to the control sample, then that indicates that the second antibody is competing with the first antibody for binding to the antigen. See Harlow and Lane (1988) Antibodies: A Laboratory Manual ch.14 (Cold Spring Harbor Laboratory, Cold Spring Harbor, NY).

T cell activating bispecific antigen binding molecules prepared as described herein may be purified by art-known techniques such as high performance liquid chromatography, ion exchange chromatography, gel electrophoresis, affinity chromatography, size exclusion chromatography, and the like. The actual conditions used to purify a particular protein will depend, in part, on factors such as net charge, hydrophobicity, hydrophilicity etc., and will be apparent to those having skill in the art. For affinity chromatography purification an antibody, ligand, receptor or antigen can be used to which the T cell activating bispecific antigen binding molecule binds. For example, for affinity chromatography purification of T cell activating bispecific antigen binding molecules of the invention, a matrix with protein A or protein G may be used. Sequential Protein A or G affinity chromatography and size exclusion chromatography can be used to isolate a T cell activating bispecific antigen binding molecule essentially as described in the Examples. The purity of the T cell activating bispecific antigen binding molecule can be determined by any of a variety of well known analytical methods including gel electrophoresis, high pressure liquid chromatography, and the like. For example, the heavy chain fusion proteins expressed as described in the Examples were shown to be intact and properly assembled as demonstrated by reducing SDS-PAGE (see e.g. Figure 2). Three bands were resolved at approximately Mr 25,000, Mr 50,000 and Mr 75,000, corresponding to the predicted molecular weights of the T cell activating bispecific antigen binding molecule light chain, heavy chain and heavy chain/light chain fusion protein.

### Assays

T cell activating bispecific antigen binding molecules provided herein may be identified, screened for, or characterized for their physical/chemical properties and/or biological activities by various assays known in the art.

### Affinity assays

The affinity of the T cell activating bispecific antigen binding molecule for an Fc receptor or a target antigen can be determined in accordance with the methods set forth in the Examples by surface plasmon resonance (SPR), using standard instrumentation such as a BIAcore instrument (GE Healthcare), and receptors or target proteins such as may be obtained by recombinant expression. Alternatively, binding of T cell activating bispecific antigen binding molecules for different receptors or target antigens may be evaluated using cell lines expressing the particular receptor or target antigen, for example by flow cytometry (FACS). A specific illustrative and exemplary embodiment for measuring binding affinity is described in the following and in the Examples below.

According to one embodiment, K_{D} is measured by surface plasmon resonance using a BIACORE® T100 machine (GE Healthcare) at 25 °C.

To analyze the interaction between the Fc-portion and Fc receptors, His-tagged recombinant Fc-receptor is captured by an anti-Penta His antibody (Qiagen) immobilized on CM5 chips and the bispecific constructs are used as analytes. Briefly, carboxymethylated dextran biosensor chips (CM5, GE Healthcare) are activated with N-ethyl-N'-(3-dimethylaminopropyl)-carbodiimide hydrochloride (EDC) and N-hydroxysuccinimide (NHS) according to the supplier's instructions. Anti Penta-His antibody is diluted with 10 mM sodium acetate, pH 5.0, to 40 µg/ml before injection at a flow rate of 5 µl/min to achieve approximately 6500 response units (RU) of coupled protein. Following the injection of the ligand, 1 M ethanolamine is injected to block unreacted groups. Subsequently the Fc-receptor is captured for 60 s at 4 or 10 nM. For kinetic measurements, four-fold serial dilutions of the bispecific construct (range between 500 nM and 4000 nM) are injected in HBS-EP (GE Healthcare, 10 mM HEPES, 150 mM NaCl, 3 mM EDTA, 0.05 % Surfactant P20, pH 7.4) at 25 °C at a flow rate of 30 µl/min for 120 s.

To determine the affinity to the target antigen, bispecific constructs are captured by an anti human Fab specific antibody (GE Healthcare) that is immobilized on an activated CM5-sensor chip surface as described for the anti Penta-His antibody. The final amount of coupled protein is is approximately 12000 RU. The bispecific constructs are captured for 90 s at 300 nM. The target antigens are passed through the flow cells for 180 s at a concentration range from 250 to 1000 nM with a flowrate of 30 µl/min. The dissociation is monitored for 180 s.

Bulk refractive index differences are corrected for by subtracting the response obtained on reference flow cell. The steady state response was used to derive the dissociation constant K_{D} by non-linear curve fitting of the Langmuir binding isotherm. Association rates (kₒₙ) and dissociation rates (k_{off}) are calculated using a simple one-to-one Langmuir binding model (BIACORE® T100 Evaluation Software version 1.1.1) by simultaneously fitting the association and dissociation sensorgrams. The equilibrium dissociation constant (K_{D}) is calculated as the ratio k_{off}/kₒₙ. See, e.g., Chen et al., J Mol Biol 293, 865-881 (1999).

### Activity assays

Biological activity of the T cell activating bispecific antigen binding molecules of the invention can be measured by various assays as described in the Examples. Biological activities may for example include the induction of proliferation of T cells, the induction of signaling in T cells, the induction of expression of activation markers in T cells, the induction of cytokine secretion by T cells, the induction of lysis of target cells such as tumor cells, and the induction of tumor regression and/or the improvement of survival.

### Compositions, Formulations, and Routes of Administration

In a further aspect, the invention provides pharmaceutical compositions comprising any of the T cell activating bispecific antigen binding molecules provided herein, e.g., for use in any of the below therapeutic methods. In one embodiment, a pharmaceutical composition comprises any of the T cell activating bispecific antigen binding molecules provided herein and a pharmaceutically acceptable carrier. In another embodiment, a pharmaceutical composition comprises any of the T cell activating bispecific antigen binding molecules provided herein and at least one additional therapeutic agent, e.g., as described below.

Further provided is a method of producing a T cell activating bispecific antigen binding molecule of the invention in a form suitable for administration in vivo, the method comprising (a) obtaining a T cell activating bispecific antigen binding molecule according to the invention, and (b) formulating the T cell activating bispecific antigen binding molecule with at least one pharmaceutically acceptable carrier, whereby a preparation of T cell activating bispecific antigen binding molecule is formulated for administration in vivo.

Pharmaceutical compositions of the present invention comprise a therapeutically effective amount of one or more T cell activating bispecific antigen binding molecule dissolved or dispersed in a pharmaceutically acceptable carrier. The phrases "pharmaceutical or pharmacologically acceptable" refers to molecular entities and compositions that are generally non-toxic to recipients at the dosages and concentrations employed, i.e. do not produce an adverse, allergic or other untoward reaction when administered to an animal, such as, for example, a human, as appropriate. The preparation of a pharmaceutical composition that contains at least one T cell activating bispecific antigen binding molecule and optionally an additional active ingredient will be known to those of skill in the art in light of the present disclosure, as exemplified by Remington's Pharmaceutical Sciences, 18th Ed. Mack Printing Company, 1990, incorporated herein by reference. Moreover, for animal (e.g., human) administration, it will be understood that preparations should meet sterility, pyrogenicity, general safety and purity standards as required by FDA Office of Biological Standards or corresponding authorities in other countries. Preferred compositions are lyophilized formulations or aqueous solutions. As used herein, "pharmaceutically acceptable carrier" includes any and all solvents, buffers, dispersion media, coatings, surfactants, antioxidants, preservatives (e.g. antibacterial agents, antifungal agents), isotonic agents, absorption delaying agents, salts, preservatives, antioxidants, proteins, drugs, drug stabilizers, polymers, gels, binders, excipients, disintegration agents, lubricants, sweetening agents, flavoring agents, dyes, such like materials and combinations thereof, as would be known to one of ordinary skill in the art (see, for example, Remington's Pharmaceutical Sciences, 18th Ed. Mack Printing Company, 1990, pp. 1289-1329, incorporated herein by reference). Except insofar as any conventional carrier is incompatible with the active ingredient, its use in the therapeutic or pharmaceutical compositions is contemplated.

The composition may comprise different types of carriers depending on whether it is to be administered in solid, liquid or aerosol form, and whether it need to be sterile for such routes of administration as injection. T cell activating bispecific antigen binding molecules of the present invention (and any additional therapeutic agent) can be administered intravenously, intradermally, intraarterially, intraperitoneally, intralesionally, intracranially, intraarticularly, intraprostatically, intrasplenically, intrarenally, intrapleurally, intratracheally, intranasally, intravitreally, intravaginally, intrarectally, intratumorally, intramuscularly, intraperitoneally, subcutaneously, subconjunctivally, intravesicularlly, mucosally, intrapericardially, intraumbilically, intraocularally, orally, topically, locally, by inhalation (e.g. aerosol inhalation), injection, infusion, continuous infusion, localized perfusion bathing target cells directly, via a catheter, via a lavage, in cremes, in lipid compositions (e.g. liposomes), or by other method or any combination of the forgoing as would be known to one of ordinary skill in the art (see, for example, Remington's Pharmaceutical Sciences, 18th Ed. Mack Printing Company, 1990, incorporated herein by reference). Parenteral administration, in particular intravenous injection, is most commonly used for administering polypeptide molecules such as the T cell activating bispecific antigen binding molecules of the invention.

Parenteral compositions include those designed for administration by injection, e.g. subcutaneous, intradermal, intralesional, intravenous, intraarterial intramuscular, intrathecal or intraperitoneal injection. For injection, the T cell activating bispecific antigen binding molecules of the invention may be formulated in aqueous solutions, preferably in physiologically compatible buffers such as Hanks' solution, Ringer's solution, or physiological saline buffer. The solution may contain formulatory agents such as suspending, stabilizing and/or dispersing agents. Alternatively, the T cell activating bispecific antigen binding molecules may be in powder form for constitution with a suitable vehicle, e.g., sterile pyrogen-free water, before use. Sterile injectable solutions are prepared by incorporating the T cell activating bispecific antigen binding molecules of the invention in the required amount in the appropriate solvent with various of the other ingredients enumerated below, as required. Sterility may be readily accomplished, e.g., by filtration through sterile filtration membranes. Generally, dispersions are prepared by incorporating the various sterilized active ingredients into a sterile vehicle which contains the basic dispersion medium and/or the other ingredients. In the case of sterile powders for the preparation of sterile injectable solutions, suspensions or emulsion, the preferred methods of preparation are vacuum-drying or freeze-drying techniques which yield a powder of the active ingredient plus any additional desired ingredient from a previously sterile-filtered liquid medium thereof. The liquid medium should be suitably buffered if necessary and the liquid diluent first rendered isotonic prior to injection with sufficient saline or glucose. The composition must be stable under the conditions of manufacture and storage, and preserved against the contaminating action of microorganisms, such as bacteria and fungi. It will be appreciated that endotoxin contamination should be kept minimally at a safe level, for example, less that 0.5 ng/mg protein. Suitable pharmaceutically acceptable carriers include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Aqueous injection suspensions may contain compounds which increase the viscosity of the suspension, such as sodium carboxymethyl cellulose, sorbitol, dextran, or the like. Optionally, the suspension may also contain suitable stabilizers or agents which increase the solubility of the compounds to allow for the preparation of highly concentrated solutions. Additionally, suspensions of the active compounds may be prepared as appropriate oily injection suspensions. Suitable lipophilic solvents or vehicles include fatty oils such as sesame oil, or synthetic fatty acid esters, such as ethyl cleats or triglycerides, or liposomes.

Active ingredients may be entrapped in microcapsules prepared, for example, by coacervation techniques or by interfacial polymerization, for example, hydroxymethylcellulose or gelatinmicrocapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences (18th Ed. Mack Printing Company, 1990). Sustained-release preparations may be prepared. Suitable examples of sustained-release preparations include semipermeable matrices of solid hydrophobic polymers containing the polypeptide, which matrices are in the form of shaped articles, e.g. films, or microcapsules. In particular embodiments, prolonged absorption of an injectable composition can be brought about by the use in the compositions of agents delaying absorption, such as, for example, aluminum monostearate, gelatin or combinations thereof.

In addition to the compositions described previously, the T cell activating bispecific antigen binding molecules may also be formulated as a depot preparation. Such long acting formulations may be administered by implantation (for example subcutaneously or intramuscularly) or by intramuscular injection. Thus, for example, the T cell activating bispecific antigen binding molecules may be formulated with suitable polymeric or hydrophobic materials (for example as an emulsion in an acceptable oil) or ion exchange resins, or as sparingly soluble derivatives, for example, as a sparingly soluble salt.

Pharmaceutical compositions comprising the T cell activating bispecific antigen binding molecules of the invention may be manufactured by means of conventional mixing, dissolving, emulsifying, encapsulating, entrapping or lyophilizing processes. Pharmaceutical compositions may be formulated in conventional manner using one or more physiologically acceptable carriers, diluents, excipients or auxiliaries which facilitate processing of the proteins into preparations that can be used pharmaceutically. Proper formulation is dependent upon the route of administration chosen.

The T cell activating bispecific antigen binding molecules may be formulated into a composition in a free acid or base, neutral or salt form. Pharmaceutically acceptable salts are salts that substantially retain the biological activity of the free acid or base. These include the acid addition salts, e.g., those formed with the free amino groups of a proteinaceous composition, or which are formed with inorganic acids such as for example, hydrochloric or phosphoric acids, or such organic acids as acetic, oxalic, tartaric or mandelic acid. Salts formed with the free carboxyl groups can also be derived from inorganic bases such as for example, sodium, potassium, ammonium, calcium or ferric hydroxides; or such organic bases as isopropylamine, trimethylamine, histidine or procaine. Pharmaceutical salts tend to be more soluble in aqueous and other protic solvents than are the corresponding free base forms.

### Therapeutic Methods and Compositions

Any of the T cell activating bispecific antigen binding molecules provided herein may be used in therapeutic methods. T cell activating bispecific antigen binding molecules of the invention can be used as immunotherapeutic agents, for example in the treatment of cancers.

For use in therapeutic methods, T cell activating bispecific antigen binding molecules of the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

In one aspect, T cell activating bispecific antigen binding molecules of the invention for use as a medicament are provided. In further aspects, T cell activating bispecific antigen binding molecules of the invention for use in treating a disease are provided. In certain embodiments, T cell activating bispecific antigen binding molecules of the invention for use in a method of treatment are provided. In one embodiment, the invention provides a T cell activating bispecific antigen binding molecule as described herein for use in the treatment of a disease in an individual in need thereof. In certain embodiments, the invention provides a T cell activating bispecific antigen binding molecule for use in a method of treating an individual having a disease comprising administering to the individual a therapeutically effective amount of the T cell activating bispecific antigen binding molecule. In certain embodiments the disease to be treated is a proliferative disorder. In a particular embodiment the disease is cancer. In certain embodiments the method further comprises administering to the individual a therapeutically effective amount of at least one additional therapeutic agent, e.g., an anti-cancer agent if the disease to be treated is cancer. In further embodiments, the invention provides a T cell activating bispecific antigen binding molecule as described herein for use in inducing lysis of a target cell, particularly a tumor cell. In certain embodiments, the invention provides a T cell activating bispecific antigen binding molecule for use in a method of inducing lysis of a target cell, particularly a tumor cell, in an individual comprising administering to the individual an effective amount of the T cell activating bispecific antigen binding molecule to induce lysis of a target cell. An "individual" according to any of the above embodiments is a mammal, preferably a human.

In a further aspect, the invention provides for the use of a T cell activating bispecific antigen binding molecule of the invention in the manufacture or preparation of a medicament. In one embodiment the medicament is for the treatment of a disease in an individual in need thereof. In a further embodiment, the medicament is for use in a method of treating a disease comprising administering to an individual having the disease a therapeutically effective amount of the medicament. In certain embodiments the disease to be treated is a proliferative disorder. In a particular embodiment the disease is cancer. In one embodiment, the method further comprises administering to the individual a therapeutically effective amount of at least one additional therapeutic agent, e.g., an anti-cancer agent if the disease to be treated is cancer. In a further embodiment, the medicament is for inducing lysis of a target cell, particularly a tumor cell. In still a further embodiment, the medicament is for use in a method of inducing lysis of a target cell, particularly a tumor cell, in an individual comprising administering to the individual an effective amount of the medicament to induce lysis of a target cell. An "individual" according to any of the above embodiments may be a mammal, preferably a human.

In a further aspect, the invention provides a method for treating a disease. In one embodiment, the method comprises administering to an individual having such disease a therapeutically effective amount of a T cell activating bispecific antigen binding molecule of the invention. In one embodiment a composition is administered to said invididual, comprising the T cell activating bispecific antigen binding molecule of the invention in a pharmaceutically acceptable form. In certain embodiments the disease to be treated is a proliferative disorder. In a particular embodiment the disease is cancer. In certain embodiments the method further comprises administering to the individual a therapeutically effective amount of at least one additional therapeutic agent, e.g., an anti-cancer agent if the disease to be treated is cancer. An "individual" according to any of the above embodiments may be a mammal, preferably a human.

In a further aspect, the invention provides a method for inducing lysis of a target cell, particularly a tumor cell. In one embodiment the method comprises contacting a target cell with a T cell activating bispecific antigen binding molecule of the invention in the presence of a T cell, particularly a cytotoxic T cell. In a further aspect, a method for inducing lysis of a target cell, particularly a tumor cell, in an individual is provided. In one such embodiment, the method comprises administering to the individual an effective amount of a T cell activating bispecific antigen binding molecule to induce lysis of a target cell. In one embodiment, an "individual" is a human.

In certain embodiments the disease to be treated is a proliferative disorder, particularly cancer. Non-limiting examples of cancers include bladder cancer, brain cancer, head and neck cancer, pancreatic cancer, lung cancer, breast cancer, ovarian cancer, uterine cancer, cervical cancer, endometrial cancer, esophageal cancer, colon cancer, colorectal cancer, rectal cancer, gastric cancer, prostate cancer, blood cancer, skin cancer, squamous cell carcinoma, bone cancer, and kidney cancer. Other cell proliferation disorders that can be treated using a T cell activating bispecific antigen binding molecule of the present invention include, but are not limited to neoplasms located in the: abdomen, bone, breast, digestive system, liver, pancreas, peritoneum, endocrine glands (adrenal, parathyroid, pituitary, testicles, ovary, thymus, thyroid), eye, head and neck, nervous system (central and peripheral), lymphatic system, pelvic, skin, soft tissue, spleen, thoracic region, and urogenital system. Also included are pre-cancerous conditions or lesions and cancer metastases. In certain embodiments the cancer is chosen from the group consisting of renal cell cancer, skin cancer, lung cancer, colorectal cancer, breast cancer, brain cancer, head and neck cancer. A skilled artisan readily recognizes that in many cases the T cell activating bispecific antigen binding molecule may not provide a cure but may only provide partial benefit. In some embodiments, a physiological change having some benefit is also considered therapeutically beneficial. Thus, in some embodiments, an amount of T cell activating bispecific antigen binding molecule that provides a physiological change is considered an "effective amount" or a "therapeutically effective amount". The subject, patient, or individual in need of treatment is typically a mammal, more specifically a human.

In some embodiments, an effective amount of a T cell activating bispecific antigen binding molecule of the invention is administered to a cell. In other embodiments, a therapeutically effective amount of a T cell activating bispecific antigen binding molecule of the invention is administered to an individual for the treatment of disease.

For the prevention or treatment of disease, the appropriate dosage of a T cell activating bispecific antigen binding molecule of the invention (when used alone or in combination with one or more other additional therapeutic agents) will depend on the type of disease to be treated, the route of administration, the body weight of the patient, the type of T cell activating bispecific antigen binding molecule, the severity and course of the disease, whether the T cell activating bispecific antigen binding molecule is administered for preventive or therapeutic purposes, previous or concurrent therapeutic interventions, the patient's clinical history and response to the T cell activating bispecific antigen binding molecule, and the discretion of the attending physician. The practitioner responsible for administration will, in any event, determine the concentration of active ingredient(s) in a composition and appropriate dose(s) for the individual subject. Various dosing schedules including but not limited to single or multiple administrations over various time-points, bolus administration, and pulse infusion are contemplated herein.

The T cell activating bispecific antigen binding molecule is suitably administered to the patient at one time or over a series of treatments. Depending on the type and severity of the disease, about 1 µg/kg to 15 mg/kg (e.g. 0.1 mg/kg - 10 mg/kg) of T cell activating bispecific antigen binding molecule can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the T cell activating bispecific antigen binding molecule would be in the range from about 0.005 mg/kg to about 10 mg/kg. In other non-limiting examples, a dose may also comprise from about 1 microgram/kg body weight, about 5 microgram/kg body weight, about 10 microgram/kg body weight, about 50 microgram/kg body weight, about 100 microgram/kg body weight, about 200 microgram/kg body weight, about 350 microgram/kg body weight, about 500 microgram/kg body weight, about 1 milligram/kg body weight, about 5 milligram/kg body weight, about 10 milligram/kg body weight, about 50 milligram/kg body weight, about 100 milligram/kg body weight, about 200 milligram/kg body weight, about 350 milligram/kg body weight, about 500 milligram/kg body weight, to about 1000 mg/kg body weight or more per administration, and any range derivable therein. In non-limiting examples of a derivable range from the numbers listed herein, a range of about 5 mg/kg body weight to about 100 mg/kg body weight, about 5 microgram/kg body weight to about 500 milligram/kg body weight, etc., can be administered, based on the numbers described above. Thus, one or more doses of about 0.5 mg/kg, 2.0 mg/kg, 5.0 mg/kg or 10 mg/kg (or any combination thereof) may be administered to the patient. Such doses may be administered intermittently, e.g. every week or every three weeks (e.g. such that the patient receives from about two to about twenty, or e.g. about six doses of the T cell activating bispecific antigen binding molecule). An initial higher loading dose, followed by one or more lower doses may be administered. However, other dosage regimens may be useful. The progress of this therapy is easily monitored by conventional techniques and assays.

The T cell activating bispecific antigen binding molecules of the invention will generally be used in an amount effective to achieve the intended purpose. For use to treat or prevent a disease condition, the T cell activating bispecific antigen binding molecules of the invention, or pharmaceutical compositions thereof, are administered or applied in a therapeutically effective amount. Determination of a therapeutically effective amount is well within the capabilities of those skilled in the art, especially in light of the detailed disclosure provided herein.

For systemic administration, a therapeutically effective dose can be estimated initially from *in vitro* assays, such as cell culture assays. A dose can then be formulated in animal models to achieve a circulating concentration range that includes the IC₅₀ as determined in cell culture. Such information can be used to more accurately determine useful doses in humans.

Initial dosages can also be estimated from *in vivo* data, *e.g.,* animal models, using techniques that are well known in the art. One having ordinary skill in the art could readily optimize administration to humans based on animal data.

Dosage amount and interval may be adjusted individually to provide plasma levels of the T cell activating bispecific antigen binding molecules which are sufficient to maintain therapeutic effect. Usual patient dosages for administration by injection range from about 0.1 to 50 mg/kg/day, typically from about 0.5 to 1 mg/kg/day. Therapeutically effective plasma levels may be achieved by administering multiple doses each day. Levels in plasma may be measured, for example, by HPLC.

In cases of local administration or selective uptake, the effective local concentration of the T cell activating bispecific antigen binding molecules may not be related to plasma concentration. One having skill in the art will be able to optimize therapeutically effective local dosages without undue experimentation.

A therapeutically effective dose of the T cell activating bispecific antigen binding molecules described herein will generally provide therapeutic benefit without causing substantial toxicity. Toxicity and therapeutic efficacy of a T cell activating bispecific antigen binding molecule can be determined by standard pharmaceutical procedures in cell culture or experimental animals. Cell culture assays and animal studies can be used to determine the LD₅₀ (the dose lethal to 50% of a population) and the ED₅₀ (the dose therapeutically effective in 50% of a population). The dose ratio between toxic and therapeutic effects is the therapeutic index, which can be expressed as the ratio LD₅₀/ED₅₀. T cell activating bispecific antigen binding molecules that exhibit large therapeutic indices are preferred. In one embodiment, the T cell activating bispecific antigen binding molecule according to the present invention exhibits a high therapeutic index. The data obtained from cell culture assays and animal studies can be used in formulating a range of dosages suitable for use in humans. The dosage lies preferably within a range of circulating concentrations that include the ED₅₀ with little or no toxicity. The dosage may vary within this range depending upon a variety of factors, e.g., the dosage form employed, the route of administration utilized, the condition of the subject, and the like. The exact formulation, route of administration and dosage can be chosen by the individual physician in view of the patient's condition (see, e.g., Fingl et al., 1975, in: The Pharmacological Basis of Therapeutics, Ch. 1, p. 1, incorporated herein by reference in its entirety).

The attending physician for patients treated with T cell activating bispecific antigen binding molecules of the invention would know how and when to terminate, interrupt, or adjust administration due to toxicity, organ dysfunction, and the like. Conversely, the attending physician would also know to adjust treatment to higher levels if the clinical response were not adequate (precluding toxicity). The magnitude of an administered dose in the management of the disorder of interest will vary with the severity of the condition to be treated, with the route of administration, and the like. The severity of the condition may, for example, be evaluated, in part, by standard prognostic evaluation methods. Further, the dose and perhaps dose frequency will also vary according to the age, body weight, and response of the individual patient.

### Other Agents and Treatments

The T cell activating bispecific antigen binding molecules of the invention may be administered in combination with one or more other agents in therapy. For instance, a T cell activating bispecific antigen binding molecule of the invention may be co-administered with at least one additional therapeutic agent. The term "therapeutic agent" encompasses any agent administered to treat a symptom or disease in an individual in need of such treatment. Such additional therapeutic agent may comprise any active ingredients suitable for the particular indication being treated, preferably those with complementary activities that do not adversely affect each other. In certain embodiments, an additional therapeutic agent is an immunomodulatory agent, a cytostatic agent, an inhibitor of cell adhesion, a cytotoxic agent, an activator of cell apoptosis, or an agent that increases the sensitivity of cells to apoptotic inducers. In a particular embodiment, the additional therapeutic agent is an anti-cancer agent, for example a microtubule disruptor, an antimetabolite, a topoisomerase inhibitor, a DNA intercalator, an alkylating agent, a hormonal therapy, a kinase inhibitor, a receptor antagonist, an activator of tumor cell apoptosis, or an antiangiogenic agent.

Such other agents are suitably present in combination in amounts that are effective for the purpose intended. The effective amount of such other agents depends on the amount of T cell activating bispecific antigen binding molecule used, the type of disorder or treatment, and other factors discussed above. The T cell activating bispecific antigen binding molecules are generally used in the same dosages and with administration routes as described herein, or about from 1 to 99% of the dosages described herein, or in any dosage and by any route that is empirically/clinically determined to be appropriate.

Such combination therapies noted above encompass combined administration (where two or more therapeutic agents are included in the same or separate compositions), and separate administration, in which case, administration of the T cell activating bispecific antigen binding molecule of the invention can occur prior to, simultaneously, and/or following, administration of the additional therapeutic agent and/or adjuvant. T cell activating bispecific antigen binding molecules of the invention can also be used in combination with radiation therapy.

### Articles of Manufacture

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is a T cell activating bispecific antigen binding molecule of the invention. The label or package insert indicates that the composition is used for treating the condition of choice. Moreover, the article of manufacture may comprise (a) a first container with a composition contained therein, wherein the composition comprises a T cell activating bispecific antigen binding molecule of the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

### Examples

The following are examples of methods and compositions of the invention. It is understood that various other embodiments may be practiced, given the general description provided above.

### General methods

### Recombinant DNA Techniques

Standard methods were used to manipulate DNA as described in Sambrook et al., Molecular cloning: A laboratory manual; Cold Spring Harbor Laboratory Press, Cold Spring Harbor, New York, 1989. The molecular biological reagents were used according to the manufacturers' instructions. General information regarding the nucleotide sequences of human immunoglobulins light and heavy chains is given in: Kabat, E.A. et al., (1991) Sequences of Proteins of Immunological Interest, 5th ed., NIH Publication No. 91-3242.

### DNA Sequencing

DNA sequences were determined by double strand sequencing.

### Gene Synthesis

Desired gene segments where required were either generated by PCR using appropriate templates or were synthesized by Geneart AG (Regensburg, Germany) from synthetic oligonucleotides and PCR products by automated gene synthesis. In cases where no exact gene sequence was available, oligonucleotide primers were designed based on sequences from closest homologues and the genes were isolated by RT-PCR from RNA originating from the appropriate tissue. The gene segments flanked by singular restriction endonuclease cleavage sites were cloned into standard cloning / sequencing vectors. The plasmid DNA was purified from transformed bacteria and concentration determined by UV spectroscopy. The DNA sequence of the subcloned gene fragments was confirmed by DNA sequencing. Gene segments were designed with suitable restriction sites to allow sub-cloning into the respective expression vectors. All constructs were designed with a 5'-end DNA sequence coding for a leader peptide which targets proteins for secretion in eukaryotic cells. SEQ ID NOs 154-162 give exemplary leader peptides and polynucleotide sequences encoding them, respectively.

### Isolation of primary human pan T cells from PBMCs

Peripheral blood mononuclear cells (PBMCs) were prepared by Histopaque density centrifugation from enriched lymphocyte preparations (buffy coats) obtained from local blood banks or from fresh blood from healthy human donors. Briefly, blood was diluted with sterile PBS and carefully layered over a Histopaque gradient (Sigma, H8889). After centrifugation for 30 minutes at 450 x g at room temperature (brake switched off), part of the plasma above the PBMC containing interphase was discarded. The PBMCs were transferred into new 50 ml Falcon tubes and tubes were filled up with PBS to a total volume of 50 ml. The mixture was centrifuged at room temperature for 10 minutes at 400 x g (brake switched on). The supernatant was discarded and the PBMC pellet washed twice with sterile PBS (centrifugation steps at 4°C for 10 minutes at 350 x g). The resulting PBMC population was counted automatically (ViCell) and stored in RPMI1640 medium, containing 10% FCS and 1% L-alanyl-L-glutamine (Biochrom, K0302) at 37°C, 5% CO2 in the incubator until assay start.

T cell enrichment from PBMCs was performed using the Pan T Cell Isolation Kit II (Miltenyi Biotec #130-091-156), according to the manufacturer's instructions. Briefly, the cell pellets were diluted in 40 µl cold buffer per 10 million cells (PBS with 0.5% BSA, 2 mM EDTA, sterile filtered) and incubated with 10 µl Biotin-Antibody Cocktail per 10 million cells for 10 min at 4°C. 30 µl cold buffer and 20 µl Anti-Biotin magnetic beads per 10 million cells were added, and the mixture incubated for another 15 min at 4°C. Cells were washed by adding 10-20x the current volume and a subsequent centrifugation step at 300 x g for 10 min. Up to 100 million cells were resuspended in 500 µl buffer. Magnetic separation of unlabeled human pan T cells was performed using LS columns (Miltenyi Biotec #130-042-401) according to the manufacturer's instructions. The resulting T cell population was counted automatically (ViCell) and stored in AIM-V medium at 37°C, 5% CO₂ in the incubator until assay start (not longer than 24 h).

### Isolation of primary human naive T cells from PBMCs

Peripheral blood mononuclar cells (PBMCs) were prepared by Histopaque density centrifugation from enriched lymphocyte preparations (buffy coats) obtained from local blood banks or from fresh blood from healthy human donors. T-cell enrichment from PBMCs was performed using the Naive CD8⁺ T cell isolation Kit from Miltenyi Biotec (#130-093-244), according to the manufacturer's instructions, but skipping the last isolation step of CD8⁺ T cells (also see description for the isolation of primary human pan T cells).

### Isolation of murine pan T cells from splenocytes

Spleens were isolated from C57BL/6 mice, transferred into a GentleMACS C-tube (Miltenyi Biotech #130-093-237) containing MACS buffer (PBS + 0.5% BSA + 2 mM EDTA) and dissociated with the GentleMACS Dissociator to obtain single-cell suspensions according to the manufacturer's instructions. The cell suspension was passed through a pre-separation filter to remove remaining undissociated tissue particles. After centrifugation at 400 x g for 4 min at 4°C, ACK Lysis Buffer was added to lyse red blood cells (incubation for 5 min at room temperature). The remaining cells were washed with MACS buffer twice, counted and used for the isolation of murine pan T cells. The negative (magnetic) selection was performed using the Pan T Cell Isolation Kit from Miltenyi Biotec (#130-090-861), following the manufacturer's instructions. The resulting T cell population was automatically counted (ViCell) and immediately used for further assays.

### Isolation of primary cynomolgus PBMCs from heparinized blood

Peripheral blood mononuclar cells (PBMCs) were prepared by density centrifugation from fresh blood from healthy cynomolgus donors, as follows: Heparinized blood was diluted 1:3 with sterile PBS, and Lymphoprep medium (Axon Lab #1114545) was diluted to 90% with sterile PBS. Two volumes of the diluted blood were layered over one volume of the diluted density gradient and the PBMC fraction was separated by centrifugation for 30 min at 520 x g, without brake, at room temperature. The PBMC band was transferred into a fresh 50 ml Falcon tube and washed with sterile PBS by centrifugation for 10 min at 400 x g at 4°C. One low-speed centrifugation was performed to remove the platelets (15 min at 150 x g, 4°C), and the resulting PBMC population was automatically counted (ViCell) and immediately used for further assays.

### Target cells

For the assessment of MCSP-targeting bispecific antigen binding molecules, the following tumor cell lines were used: the human melanoma cell line WM266-4 (ATCC #CRL-1676), derived from a metastatic site of a malignant melanoma and expressing high levels of human MCSP; and the human melanoma cell line MV-3 (a kind gift from The Radboud University Nijmegen Medical Centre), expressing medium levels of human MCSP.

For the assessment of CEA-targeting bispecific antigen binding molecules, the following tumor cell lines were used: the human gastric cancer cell line MKN45 (DSMZ #ACC 409), expressing very high levels of human CEA; the human female Caucasian colon adenocarcinoma cell line LS-174T (ECACC #87060401), expressing medium to low levels of human CEA; the human epithelioid pancreatic carcinoma cell line Panc-1 (ATCC #CRL-1469), expressing (very) low levels of human CEA; and a murine colon carcinoma cell line MC38-huCEA, that was engineered in-house to stably express human CEA.

In addition, a human T cell leukaemia cell line, Jurkat (ATCC #TIB-152), was used to assess binding of different bispecific constructs to human CD3 on cells.

### Example 1

### Preparation, purification and characterization of bispecific antigen binding molecules

The heavy and light chain variable region sequences were subcloned in frame with either the constant heavy chain or the constant light chain pre-inserted into the respective recipient mammalian expression vector. The antibody expression was driven by an MPSV promoter and a synthetic polyA signal sequence is located at the 3' end of the CDS. In addition each vector contained an EBV OriP sequence.

The molecules were produced by co-transfecting HEK293 EBNA cells with the mammalian expression vectors. Exponentially growing HEK293 EBNA cells were transfected using the calcium phosphate method. Alternatively, HEK293 EBNA cells growing in suspension were transfected using polyethylenimine (PEI). For preparation of "1+1 IgG scFab, one armed / one armed inverted" constructs, cells were transfected with the corresponding expression vectors in a 1:1:1 ratio ("vector heavy chain" : "vector light chain" : "vector heavy chain-scFab"). For preparation of "2+1 IgG scFab" constructs, cells were transfected with the corresponding expression vectors in a 1:2:1 ratio ("vector heavy chain" : "vector light chain" : "vector heavy chain-scFab"). For preparation of "1+1 IgG Crossfab" constructs, cells were transfected with the corresponding expression vectors in a 1:1:1:1 ratio ("vector second heavy chain" : "vector first light chain" : "vector light chain Crossfab" : "vector first heavy chain-heavy chain Crossfab"). For preparation of "2+1 IgG Crossfab" constructs cells were transfected with the corresponding expression vectors in a 1:2:1:1 ratio ("vector second heavy chain" : "vector light chain" : "vector first heavy chain-heavy chain Crossfab)" : "vector light chain Crossfab". For preparation of the "2+1 IgG Crossfab, linked light chain" construct, cells were transfected with the corresponding expression vectors in a 1:1:1:1 ratio ("vector heavy chain" : "vector light chain" : "vector heavy chain (CrossFab-Fab-Fc)" : "vector linked light chain"). For preparation of the "1+1 CrossMab" construct, cells were transfected with the corresponding expression vectors in a 1:1:1:1 ratio ("vector first heavy chain" : "vector second heavy chain" : "vector first light chain" : "vector second light chain"). For preparation of the "1+1 IgG Crossfab light chain fusion " construct, cells were transfected with the corresponding expression vectors in a 1:1:1:1 ratio ("vector first heavy chain" : "vector second heavy chain" : "vector light chain Crossfab" : "vector second light chain").

For transfection using calcium phosphate cells were grown as adherent monolayer cultures in T-flasks using DMEM culture medium supplemented with 10 % (v/v) FCS, and transfected when they were between 50 and 80 % confluent. For the transfection of a T150 flask, 15 million cells were seeded 24 hours before transfection in 25 ml DMEM culture medium supplemented with FCS (at 10% v/v final), and cells were placed at 37°C in an incubator with a 5% CO₂ atmosphere overnight. For each T150 flask to be transfected, a solution of DNA, CaCl₂ and water was prepared by mixing 94 µg total plasmid vector DNA divided in the corresponding ratio, water to a final volume of 469 µl and 469 µl of a 1 M CaCl₂ solution. To this solution, 938 µl of a 50 mM HEPES, 280 mM NaCl, 1.5 mM Na₂HPO₄ solution at pH 7.05 were added, mixed immediately for 10 s and left to stand at room temperature for 20 s. The suspension was diluted with 10 ml of DMEM supplemented with 2 % (v/v) FCS, and added to the T150 in place of the existing medium. Subsequently, additional 13 ml of transfection medium were added. The cells were incubated at 37°C, 5% CO₂ for about 17 to 20 hours, then medium was replaced with 25 ml DMEM, 10 % FCS. The conditioned culture medium was harvested approximately 7 days postmedia exchange by centrifugation for 15 min at 210 x g, sterile filtered (0.22 · m filter), supplemented with sodium azide to a final concentration of 0.01 % (w/v), and kept at 4°C.

For transfection using polyethylenimine (PEI) HEK293 EBNA cells were cultivated in suspension in serum free CD CHO culture medium. For the production in 500 ml shake flasks, 400 million HEK293 EBNA cells were seeded 24 hours before transfection. For transfection cells were centrifuged for 5 min at 210 x g, and supernatant was replaced by 20 ml pre-warmed CD CHO medium. Expression vectors were mixed in 20 ml CD CHO medium to a final amount of 200 µg DNA. After addition of 540 µl PEI, the mixture was vortexed for 15 s and subsequently incubated for 10 min at room temperature. Afterwards cells were mixed with the DNA/PEI solution, transferred to a 500 ml shake flask and incubated for 3 hours at 37°C in an incubator with a 5% CO₂ atmosphere. After the incubation time 160 ml F17 medium was added and cells were cultivated for 24 hours. One day after transfection 1 mM valproic acid and 7% Feed 1 (Lonza) were added. After a cultivation of 7 days, supernatant was collected for purification by centrifugation for 15 min at 210 x g, the solution was sterile filtered (0.22 µm filter), supplemented with sodium azide to a final concentration of 0.01 % w/v, and kept at 4°C. The secreted proteins were purified from cell culture supernatants by Protein A affinity chromatography, followed by a size exclusion chromatography step.

For affinity chromatography supernatant was loaded on a HiTrap ProteinA HP column (CV = 5 ml, GE Healthcare) equilibrated with 25 ml 20 mM sodium phosphate, 20 mM sodium citrate, pH 7.5 or 40 ml 20 mM sodium phosphate, 20 mM sodium citrate, 0.5 M sodium chloride, pH 7.5. Unbound protein was removed by washing with at least ten column volumes 20 mM sodium phosphate, 20 mM sodium citrate, 0.5 M sodium chloride pH 7.5, followed by an additional wash step using six column volumes 10 mM sodium phosphate, 20 mM sodium citrate, 0.5 M sodium chloride pH 5.45. Subsequently, the column was washed with 20 ml 10 mM MES, 100 mM sodium chloride, pH 5.0, and target protein was eluted in six column volumes 20 mM sodium citrate, 100 mM sodium chloride, 100 mM glycine, pH 3.0. Alternatively, target protein was eluted using a gradient over 20 column volumes from 20 mM sodium citrate, 0.5 M sodium chloride, pH 7.5 to 20 mM sodium citrate, 0.5 M sodium chloride, pH 2.5. The protein solution was neutralized by adding 1/10 of 0.5 M sodium phosphate, pH 8. The target protein was concentrated and filtrated prior to loading on a HiLoad Superdex 200 column (GE Healthcare) equilibrated with 25 mM potassium phosphate, 125 mM sodium chloride, 100 mM glycine solution of pH 6.7. For the purification of 1+1 IgG Crossfab the column was equilibrated with 20 mM histidine, 140 mM sodium chloride solution of pH 6.0.

The protein concentration of purified protein samples was determined by measuring the optical density (OD) at 280 nm, using the molar extinction coefficient calculated on the basis of the amino acid sequence. Purity and molecular weight of the bispecific constructs were analyzed by SDS-PAGE in the presence and absence of a reducing agent (5 mM 1,4-dithiotreitol) and staining with Coomassie (SimpleBlue™ SafeStain from Invitrogen) using the NuPAGE® PreCast gel system (Invitrogen, USA) was used according to the manufacturer's instructions (4-12% Tris-Acetate gels or 4-12% Bis-Tris). Alternatively, purity and molecular weight of molecules were analyzed by CE-SDS analyses in the presence and absence of a reducing agent, using the Caliper LabChip GXII system (Caliper Lifescience) according to the manufacturer's instructions. The aggregate content of the protein samples was analyzed using a Superdex 200 10/300GL analytical size-exclusion chromatography column (GE Healthcare) in 2 mM MOPS, 150 mM NaCl, 0.02% (w/v) NaN3, pH 7.3 running buffer at 25°C. Alternatively, the aggregate content of antibody samples was analyzed using a TSKgel G3000 SW XL analytical size-exclusion column (Tosoh) in 25 mM K₂HPO₄, 125 mM NaCl, 200 mM L-arginine monohydrocloride, 0.02% (w/v) NaN3, pH 6.7 running buffer at 25°C.

Figures 2-14 show the results of the SDS PAGE and analytical size exclusion chromatography and Table 2A shows the yields, aggregate content after Protein A, and final monomer content of the preparations of the different bispecific constructs.

Figure 47 shows the result of the CE-SDS analyses of the anti-CD3/anti-MCSP bispecific "2+1 IgG Crossfab, linked light chain" construct (see SEQ ID NOs 3, 5, 29 and 179). 2 µg sample was used for analyses. Figure 48 shows the result of the analytical size exclusion chromatography of the final product (20 µg sample injected).

Figure 54 shows the results of the CE-SDS and SDS PAGE analyses of various constructs, and Table 2A shows the yields, aggregate content after Protein A and final monomer content of the preparations of the different bispecific constructs.

**TABLE 2A. Yields, aggregate content after Protein A and final monomer content.**

| **Construct** | **Yield [mg/l]** | **Aggregate content after Protein A [%]** | **HMW [%]** | **LMW [%]** | **Monomer [%]** |
|---|---|---|---|---|---|
| **MCSP** | | | | | |
| 2+1 IgG Crossfab; VH/VL exchange (LC007/V9) | 12.8 | 2.2 | 0 | 0 | 100 |
| (SEQ ID NOs 3, 5, 29, 33) | | | | | |
| 2+1 IgG Crossfab; VH/VL exchange (LC007/FN18) (SEQ ID NOs 3, 5, 35, 37) | 3.2 | 5.7 | 0.4 | 0 | 99.6 |
| 2+1 IgG scFab, P329G LALA (SEQ ID NOs 5, 21, 23) | 11.9 | 23 | 0.3 | 0 | 99.7 |
| 2+1 IgG scFab, LALA (SEQ ID NOs 5, 17, 19) | 9 | 23 | 0 | 0 | 100 |
| 2+1 IgG scFab, P329G LALA N297D (SEQ ID NOs 5, 25, 27) | 12.9 | 32.7 | 0 | 0 | 100 |
| 2+1 IgG scFab, wt (SEQ ID NOs 5, 13, 15) | 15.5 | 31.8 | 0 | 0 | 100 |
| 1+1 IgG scFab (SEQ ID NOs 5, 21, 213) | 7 | 24.5 | 0 | 0 | 100 |
| 1+1 IgG scFab "one armed" (SEQ ID NOs 1, 3, 5) | 7.6 | 43.7 | 2.3 | 0 | 97.7 |
| 1+1 IgG scFab "one armed inverted" (SEQ ID NOs 7, 9, 11) | 1 | 27 | 7.1 | 9.1 | 83.8 |
| 1+1 IgG Crossfab; VH/VL exchange (LC007/V9) (SEQ ID NOs 5, 29, 31, 33) | 9.8 | 0 | 0 | 0 | 100 |
| 2+1 IgG Crossfab, linked light chain; VL/VH exchange (LC007/V9) (SEQ ID NOs 3, 5, 29, 179) | 0.54 | 40 | 1.4 | 0 | 98.6 |
| 1+1 IgG Crossfab; VL/VH exchange (LC007/V9) (SEQ ID NOs 5, 29, 33, 181) | 6.61 | 8.5 | 0 | 0 | 100 |
| 1+1 CrossMab; CL/CH1 exchange (LC00/V9) (SEQ ID NOs 5, 23, 183, 185) | 6.91 | 10.5 | 1.3 | 1.7 | 97 |
| 2+1 IgG Crossfab, inverted; CL/CH1 exchange (LC007/V9) (SEQ ID NOs 5, 23, 183, 187) | 9.45 | 6.1 | 0.8 | 0 | 99.2 |
| 2+1 IgG Crossfab; VL/VH exchange (M4-3 ML2/V9) (SEQ ID NOs 33, 189, 191, 193) | 36.6 | 0 | 9.5 | 35.3 | 55.2 |
| 2+1 IgG Crossfab; CL/CH1 exchange (M4-3 ML2/V9) (SEQ ID NOs 183, 189, 193, 195) | 2.62 | 12 | 2.8 | 0 | 97.2 |
| 2+1 IgG Crossfab; CL/CH1 exchange (M4-3 ML2/H2C) (SEQ ID NOs 189, 193, 199, 201) | 29.75 | 0 | 0 | 0 | 100 |
| 2+1 IgG Crossfab; CL/CH1 exchange (LC007/anti-CD3) (SEQ ID NOs 5, 23, 215, 217) | 1.2 | 0 | 1.25 | 1.65 | 97.1 |
| 2+1 IgG Crossfab, inverted; CL/CH1 exchange (LC007/anti-CD3) | 7.82 | 0.5 | 0 | 0 | 100 |
| (SEQ ID NOs 5, 23, 215, 219) | | | | | |

| **EGFR** | | | | | |
|---|---|---|---|---|---|
| 2+1 IgG scFab (SEQ ID NOs 45, 47, 53) | 5.2 | 53 | 0 | 30 | 70 |
| 1+1 IgG scFab (SEQ ID NOs 47, 53, 213) | 3.4 | 66.6 | 0 | 1.6 | 98.4 |
| 1+1 IgG scFab "one armed" (SEQ ID NOs 43, 45, 47) | 9.05 | 60.8 | 0 | 0 | 100 |
| 1+1 IgG scFab "one armed inverted" (SEQ ID NOs 11, 49, 51) | 3.87 | 58.8 | 0 | 0 | 100 |

| **FAP** | | | | | |
|---|---|---|---|---|---|
| 2+1 IgG scFab (SEQ ID NOs 57, 59, 61) | 12.57 | 53 | 0 | 0 | 100 |
| 1+1 IgG scFab (SEQ ID NOs 57, 61, 213) | 17.95 | 41 | 0.4 | 0 | 99.6 |
| 1+1 IgG scFab "one armed inverted" (SEQ ID NOs 11, 51, 55) | 2.44 | 69 | 0.6 | 0 | 99.4 |

| **CEA** | | | | | |
|---|---|---|---|---|---|
| 2+1 IgG Crossfab, inverted; VL/VH exchange (CH1A1A/V9) (SEQ ID NOs 33, 63, 65, 67) | 0.34 | 13 | 4.4 | 0 | 95.6 |
| 2+1 IgG Crossfab, inverted; CL/CH1 exchange (CH1A1A/V9) (SEQ ID NOs 65, 67, 183, 197) | 12.7 | 43 | 0 | 0 | 100 |
| 2+1 IgG Crossfab, inverted; CL/CH1 exchange (431/26/V9) (SEQ ID NOs 183, 203, 205, 207) | 7.1 | 20 | 0 | 0 | 100 |
| 1+1 IgG-Crossfab light chain fusion (CH1A1A/V9) (SEQ ID NOs 183, 209, 211, 213) | 7.85 | 27 | 4.3 | 3.2 | 92.5 |

As controls, bispecific antigen binding molecules were generated in the prior art tandem scFv format ("(scFv)2") and by fusing a tandem scFv to an Fc domain ("(scFv)2-Fc"). The molecules were produced in HEK293-EBNA cells and purified by Protein A affinity chromatography followed by a size exclusion chromatographic step in an analogous manner as described above for the bispecific antigen binding molecules of the invention. Due to high aggregate formation, some of the samples had to be further purified by applying eluted and concentrated samples from the HiLoad Superdex 200 column (GE Healthcare) to a Superdex 10/300 GL column (GE Healthcare) equilibrated with 20 mM histidine, 140 mM sodium chloride, pH 6.7 in order to obtain protein with high monomer content. Subsequently, protein concentration, purity and molecular weight, and aggregate content were determined as described above.

Yields, aggregate content after the first purification step, and final monomer content for the control molecules is shown in Table 2B. Comparison of the aggregate content after the first purification step (Protein A) indicates the superior stability of the IgG Crossfab and IgG scFab constructs compared to the "(scFv)₂-Fc" and the disulfide bridge-stabilized "(dsscFv)₂-Fc" molecules.

**TABLE 2B. Yields, aggregate content after Protein A and final monomer content.**

| **Construct** | **Yield [mg/l]** | **Aggregates after ProteinA [%]** | **Final** | | |
|---|---|---|---|---|---|
| | | | **HMW [%]** | **LMW [%]** | **Monomer [%]** |
| (scFv)₂-Fc (antiMCSP/anti huCD3) | 76.5 | 40 | 0.5 | 0 | 99.5 |
| (dsscFv)₂-Fc (antiMCSP/anti huCD3) | 2.65 | 48 | 7.3 | 8.0 | 84.7 |

Thermal stability of the proteins was monitored by Dynamic Light Scattering (DLS). 30 · g of filtered protein sample with a protein concentration of 1 mg/ml was applied in duplicate to a Dynapro plate reader (Wyatt Technology Corporation; USA). The temperature was ramped from 25 to75°C at 0.05°C/min, with the radius and total scattering intensity being collected. The results are shown in Figure 15 and Table 2C. For the "(scFv)2-Fc" (antiMCSP/anti huCD3) molecule two aggregation points were observed, at 49°C and 68°C. The "(dsscFv)2-Fc" construct has an increased aggregation temperature (57°C) as a result of the introduced disulfide bridge (Figure 15A, Table 2C). Both, the "2+1 IgG scFab" and the "2+1 IgG Crossfab" constructs are aggregating at temperatures higher than 60°C, demonstrating their superior thermal stability as compared to the "(scFv)2-Fc" and "(dsscFv)2-Fc" formats (Figure 15B, Table 2C).

**TABLE 2C. Thermal stability determined by dynamic light scattering.**

| **Construct** | **T_{agg} [°C]** |
|---|---|
| 2+1 IgG scFab (LC007/V9) | 68 |
| 2+1 IgG Crossfab (LC007/V9) | 65 |
| Fc-(scFv)2 (LC007/V9) | 49/68 |
| Fc-(dsscFv)2 (LC007/V9) | 57 |

### Example 2

### Surface Plasmon resonance analysis of Fc receptor and target antigen binding

### Method

All surface plasmon resonance (SPR) experiments are performed on a Biacore T100 at 25°C with HBS-EP as running buffer (0.01 M HEPES pH 7.4, 0.15 M NaCl, 3 mM EDTA, 0.005% Surfactant P20, Biacore, Freiburg/Germany).

### Analysis of FcR binding of different Fc-variants

The assay setup is shown in Figure 16A. For analyzing interaction of different Fc-variants with human FcγRIIIa-V158 and murine FcyRIV direct coupling of around 6,500 resonance units (RU) of the anti-Penta His antibody (Qiagen) is performed on a CM5 chip at pH 5.0 using the standard amine coupling kit (Biacore, Freiburg/Germany). HuFcγRIIIa-V158-K6H6 and muFcyRIV-aviHis-biotin are captured for 60 s at 4 and 10 nM respectively.

Constructs with different Fc-mutations are passed through the flow cells for 120 s at a concentration of 1000 nM with a flow rate of 30 µl/min. The dissociation is monitored for 220 s. Bulk refractive index differences are corrected for by subtracting the response obtained in a reference flow cell. Here, the Fc-variants are flown over a surface with immobilized anti-Penta His antibody but on which HBS-EP has been injected rather than HuFcγRIIIa-V158-K6H6 or muFcyRIV-aviHis-biotin. Affinity for human FcγRIIIa-V158 and murine FcγRIV was determined for wild-type Fc using a concentration range from 500 - 4000 nM.

The steady state response was used to derive the dissociation constant K_{D} by non-linear curve fitting of the Langmuir binding isotherm. Kinetic constants were derived using the Biacore T100 Evaluation Software (vAA, Biacore AB, Uppsala/Sweden), to fit rate equations for 1:1 Langmuir binding by numerical integration.

### Result

The interaction of Fc variants with human FcγRIIIa and murine FcγRIV was monitored by surface plasmon resonance. Binding to captured huFcγRIIIa-V158-K6H6 and muFcyRIV-aviHis-biotin is significantly reduced for all analyzed Fc mutants as compared to the construct with a wild-type (wt) Fc domain.

The Fc mutants with the lowest binding to the human Fcγ-receptor were P329G L234A L235A (LALA) and P329G LALA N297D. The LALA mutation alone was not enough to abrogate binding to huFcγRIIIa-V158-K6H6. The Fc variant carrying only the LALA mutation had a residual binding affinity to human FcyRIIIa of 2.100 nM, while the wt Fc bound the human FcγRIIIa receptor with an affinity of 600 nM (Table 3). Both K_{D} values were derived by 1:1 binding model, using a single concentration.

Affinity to human FcγRIIIa-V158 and murine FcyRIV could only be analyzed for wt Fc. K_{D} values are listed in Table 3. Binding to the murine FcyRIV was almost completely eliminated for all analyzed Fc mutants.

**TABLE 3. Affinity of Fc-variants to the human FcγRIIIa-V158 and murine FcyRIV.**

| **K_{D} in nM T = 25°C** | **human FcγRIIIa-V158** | | **murine FcγRIV** | |
|---|---|---|---|---|
| | **kinetic** | **steady state** | **kinetic** | **steady state** |
| Fc-wt (SEQ ID NOs 5, 13, 15) | 600* (1200) | 3470 | 576 | 1500 |
| Fc-LALA (SEQ ID NOs 5, 17, 19) | 2130* | n.d. | n.d. | |
| Fc-P329G LALA (SEQ ID NOs 5, 21, 23) | n.d. | | n.d. | |
| Fc-P329G LALA N297D (SEQ ID NOs 5, 25, 27) | n.d. | | n.d. | |

| | | | | |
|---|---|---|---|---|
| *determined using one concentration (1000 nM) | | | | |

### Analysis of simultaneous binding to tumor antigen and CD3

Analysis of simultaneous binding of the T-cell bispecific constructs to the tumor antigen and the human CD3ε was performed by direct coupling of 1650 resonance units (RU) of biotinylated D3 domain of MCSP on a sensor chip SA using the standard coupling procedure. Human EGFR was immobilized using standard amino coupling procedure. 8000 RU were immobilized on a CM5 sensor chip at pH 5.5. The assay setup is shown in Figure 16B.

Different T-cell bispecific constructs were captured for 60 s at 200 nM. Human CD3γ(G₄S)₅CD3ε-AcTev-Fc(knob)-Avi/Fc(hole) was subsequently passed at a concentration of 2000 nM and a flow rate of 40 µl/min for 60 s. Bulk refractive index differences were corrected for by subtracting the response obtained on a reference flow cell where the recombinant CD3ε was flown over a surface with immobilized D3 domain of MCSP or EGFR without captured T-cell bispecific constructs.

### Result

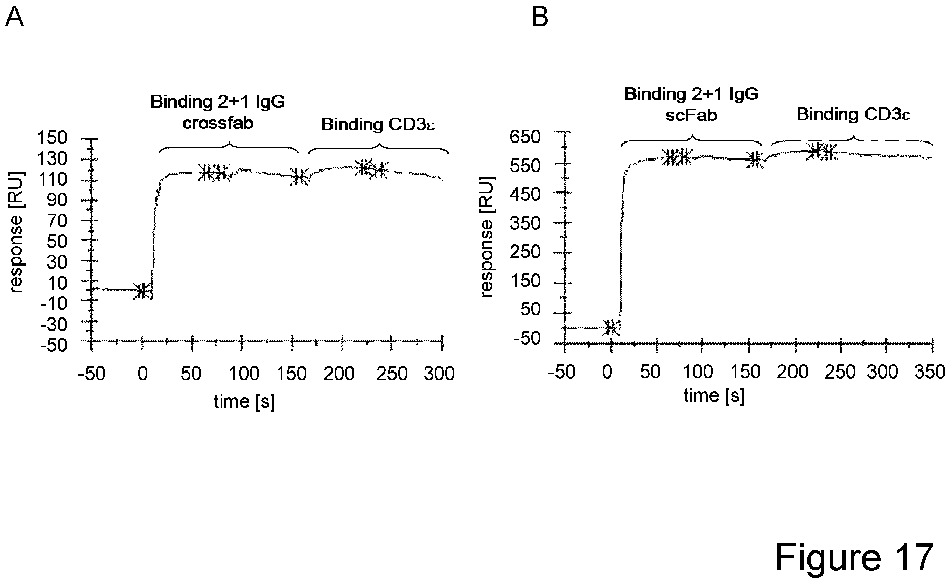

Simultaneous binding to both tumor antigen and human CD3ε was analyzed by surface plasmon resonance (Figure 17, Figure 18). All constructs were able to bind the tumor antigen and the CD3 simultaneously. For most of the constructs the binding level (RU) after injection of human CD3ε was higher than the binding level achieved after injection of the construct alone reflecting that both tumor antigen and the human CD3ε were bound to the construct.

### Example 3

### Binding of bispecific constructs to the respective target antigen on cells

Binding of the different bispecific constructs to CD3 on Jurkat cells (ATCC #TIB-152), and the respective tumor antigen on target cells, was determined by FACS. Briefly, cells were harvested, counted and checked for viability. 0.15 - 0.2 million cells per well (in PBS containing 0.1% BSA; 90 µl) were plated in a round-bottom 96-well plate and incubated with the indicated concentration of the bispecific constructs and corresponding IgG controls (10 µl) for 30 min at 4°C. For a better comparison, all constructs and IgG controls were normalized to same molarity. After the incubation, cells were centrifuged (5 min, 350 x g), washed with 150 µl PBS containing 0.1% BSA, resuspended and incubated for further 30 min at 4°C with 12 µl/well of a FITC-or PE-conjugated secondary antibody. Bound constructs were detected using a FACSCantoII (Software FACS Diva). The "(scFv)2" molecule was detected using a FITC-conjugated anti-His antibody (Lucerna, #RHIS-45F-Z). For all other molecules, a FITC- or PE-conjugated AffiniPure F(ab')2 Fragment goat anti-human IgG Fcγ Fragment Specific (Jackson Immuno Research Lab # 109-096-098 / working solution 1:20, or #109-116-170 / working solution 1:80, respectively) was used. Cells were washed by addition of 120 µl/well PBS containing 0.1% BSA and centrifugation at 350 x g for 5 min. A second washing step was performed with 150 µl/well PBS containing 0.1% BSA. Unless otherwise indicated, cells were fixed with 100 µl/well fixation buffer (BD #554655) for 15 min at 4°C in the dark, centrifuged for 6 min at 400 x g and kept in 200 µl/well PBS containing 0.1% BSA until the samples were measured with FACS CantoII. EC50 values were calculated using the GraphPad Prism software. In a first experiment, different bispecific constructs targeting human MCSP and human CD3 were analyzed by flow cytometry for binding to human CD3 expressed on Jurkat, human T cell leukaemia cells, or to human MCSP on Colo-38 human melanoma cells.

Results are presented in Figure 19-21, which show the mean fluorescence intensity of cells that were incubated with the bispecific molecule, control IgG, the secondary antibody only, or left untreated.

As shown in Figure 19, for both antigen binding moieties of the "(scFv)2" molecule, i.e. CD3 (Figure 191A) and MCSP (Figure 19B), a clear binding signal is observed compared to the control samples.

The "2+1 IgG scFab" molecule (SEQ ID NOs 5, 17, 19) shows good binding to huMCSP on Colo-38 cells (Figure 20A). The CD3 moiety binds CD3 slightly better than the reference anti-human CD3 IgG (Figure 20B).

As depicted in Figure 21A, the two "1+1" constructs show comparable binding signals to human CD3 on cells. The reference anti-human CD3 IgG gives a slightly weaker signal. In addition, both constructs tested ("1+1 IgG scFab, one-armed" (SEQ ID NOs 1, 3, 5) and "1+1 IgG scFab, one-armed inverted" (SEQ ID NOs 7, 9, 11)) show comparable binding to human MCSP on cells (Figure 21B). The binding signal obtained with the reference anti-human MCSP IgG is slightly weaker.

In another experiment, the purified "2+1 IgG scFab" bispecific construct (SEQ ID NOs 5, 17, 19) and the corresponding anti human MCSP IgG were analyzed by flow cytometry for dosedependent binding to human MCSP on Colo-38 human melanoma cells, to determine whether the bispecific construct binds to MCSP via one or both of its "arms". As depicted in Figure 22, the "2+1 IgG scFab" construct shows the same binding pattern as the MCSP IgG.

In yet another experiment, the binding of CD3/CEA "2+1 IgG Crossfab, inverted" bispecific constructs with either a VL/VH (see SEQ ID NOs 33, 63, 65, 67) or a CL/CH1 exchange (see SEQ ID NOs 66, 67, 183, 197) in the Crossfab fragment to human CD3, expressed by Jurkat cells, or to human CEA, expressed by LS-174T cells, was assessed. As a control, the equivalent maximum concentration of the corresponding IgGs and the background staining due to the labeled 2ndary antibody (goat anti-human FITC-conjugated AffiniPure F(ab')2 Fragment, Fcγ Fragment-specific, Jackson Immuno Research Lab # 109-096-098) were assessed as well. As illustrated in Figure 55, both constructs show good binding to human CEA, as well as to human CD3 on cells. The calculated EC50 values were 4.6 and 3.9 nM (CD3), and 9.3 and 6.7 nM (CEA) for the "2+1 IgG Crossfab, inverted (VL/VH)" and the "2+1 IgG Crossfab, inverted (CL/CH1)" constructs, respectively.

In another experiment, the binding of CD3/MCSP "2+1 IgG Crossfab" (see SEQ ID NOs 3, 5, 29, 33) and "2+1 IgG Crossfab, inverted" (see SEQ ID NOs 5, 23, 183, 187) constructs to human CD3, expressed by Jurkat cells, or to human MCSP, expressed by WM266-4 cells, was assessed. Figure 56 shows that, while binding of both constructs to MCSP on cells was comparably good, the binding of the "inverted" construct to CD3 was reduced compared to the other construct. The calculated EC50 values were 6.1 and 1.66 nM (CD3), and 0.57 and 0.95 nM (MCSP) for the "2+1 IgG Crossfab, inverted" and the "2+1 IgG Crossfab" constructs, respectively.

In a further experiment, binding of the "1+1 IgG Crossfab light chain (LC) fusion" construct (SEQ ID NOs 183, 209, 211, 213) to human CD3, expressed by Jurkat cells, and to human CEA, expressed by LS-174T cells was determined. As a control, the equivalent maximum concentration of the corresponding anti-CD3 and anti-CEA IgGs and the background staining due to the labeled 2ndary antibody (goat anti-human FITC-conjugated AffiniPure F(ab')2 Fragment, Fcγ Fragment-specific, Jackson Immuno Research Lab #109-096-098) were assessed as well. As depicted in Figure 57, the binding of the "1+1 IgG Crossfab LC fusion" to CEA appears to be greatly reduced, whereas the binding to CD3 was at least comparable to the reference IgG.

In a final experiment, binding of the "2+1 IgG Crossfab" (SEQ ID NOs 5, 23, 215, 217) and the "2+1 IgG Crossfab, inverted" (SEQ ID NOs 5, 23, 215, 219) constructs to human CD3, expressed by Jurkat cells, and to human MCSP, expressed by WM266-4 tumor cells was determined. As depicted in Figure 58 the binding to human CD3 was reduced for the "2+1 IgG Crossfab, inverted" compared to the other construct, but the binding to human MCSP was comparably good. The calculated EC50 values were 10.3 and 32.0 nM (CD3), and 3.1 and 3.4 nM (MCSP) for the "2+1 IgG Crossfab" and the "2+1 IgG Crossfab, inverted" construct, respectively.

### Example 4

### FACS analysis of surface activation markers on primary human T cells upon engagement of bispecific constructs

The purified huMCSP-huCD3-targeting bispecific "2+1 IgG scFab" (SEQ ID NOs 5, 17, 19) and "(scFv)₂" molecules were tested by flow cytometry for their potential to up-regulate the early surface activation marker CD69, or the late activation marker CD25 on CD8⁺ T cells in the presence of human MCSP-expressing tumor cells.

Briefly, MCSP-positive Colo-38 cells were harvested with Cell Dissociation buffer, counted and checked for viability. Cells were adjusted to 0.3 x 10⁶ (viable) cells per ml in AIM-V medium, 100 µl of this cell suspension per well were pipetted into a round-bottom 96-well plate (as indicated). 50 µl of the (diluted) bispecific construct were added to the cell-containing wells to obtain a final concentration of 1 nM. Human PBMC effector cells were isolated from fresh blood of a healthy donor and adjusted to 6 x 10⁶ (viable) cells per ml in AIM-V medium. 50 µl of this cell suspension was added per well of the assay plate (see above) to obtain a final E:T ratio of 10:1. To analyze whether the bispecific constructs are able to activate T cells exclusively in the presence of target cells expressing the tumor antigen huMCSP, wells were included that contained 1 nM of the respective bispecific molecules, as well as PBMCs, but no target cells. After incubation for 15 h (CD69), or 24 h (CD25) at 37°C, 5% CO₂, cells were centrifuged (5 min, 350 x g) and washed twice with 150 µl/well PBS containing 0.1% BSA. Surface staining for CD8 (mouse IgG1,κ; clone HIT8a; BD #555635), CD69 (mouse IgG1; clone L78; BD #340560) and CD25 (mouse IgG1,κ; clone M-A251; BD #555434) was performed at 4°C for 30 min, according to the supplier's suggestions. Cells were washed twice with 150 µl/well PBS containing 0.1% BSA and fixed for 15 min at 4°C, using 100 µl/well fixation buffer (BD #554655). After centrifugation, the samples were resuspended in 200 µl/well PBS with 0.1% BSA and analyzed using a FACS CantoII machine (Software FACS Diva).

Figure 23 depicts the expression level of the early activation marker CD69 (A), or the late activation marker CD25 (B) on CD8⁺ T cells after 15 hours or 24 hours incubation, respectively. Both constructs induce up-regulation of both activation markers exclusively in the presence of target cells. The "(scFv)2" molecule seems to be slightly more active in this assay than the "2+1 IgG scFab" construct.

The purified huMCSP-huCD3-targeting bispecific "2+1 IgG scFab" and "(scFv)₂" molecules were further tested by flow cytometry for their potential to up-regulate the late activation marker CD25 on CD8⁺ T cells or CD4⁺ T cells in the presence of human MCSP-expressing tumor cells. Experimental procedures were as described above, using human pan T effector cells at an E:T ratio of 5:1 and an incubation time of five days.

Figure 24 shows that both constructs induce up-regulation of CD25 exclusively in the presence of target cells on both, CD8⁺ (A) as well as CD4⁺ (B) T cells. The "2+1 IgG scFab" construct seems to induce less up-regulation of CD25 in this assay, compared to the "(scFv)2" molecule. In general, the up-regulation of CD25 is more pronounced on CD8⁺ than on CD4⁺ T cells.

In another experiment, purified "2+1 IgG Crossfab" targeting cynomolgus CD3 and human MCSP (SEQ ID NOs 3, 5, 35, 37) was analyzed for its potential to up-regulate the surface activation marker CD25 on CD8⁺ T cells in the presence of tumor target cells. Briefly, human MCSP-expressing MV-3 tumor target cells were harvested with Cell Dissociation Buffer, washed and resuspendend in DMEM containing 2% FCS and 1% GlutaMax. 30 000 cells per well were plated in a round-bottom 96-well plate and the respective antibody dilution was added at the indicated concentrations (Figure 25). The bispecific construct and the different IgG controls were adjusted to the same molarity. Cynomolgus PBMC effector cells, isolated from blood of two healthy animals, were added to obtain a final E:T ratio of 3:1. After an incubation for 43 h at 37°C, 5% CO₂, the cells were centrifuged at 350 x g for 5 min and washed twice with PBS, containing 0.1% BSA. Surface staining for CD8 (Miltenyi Biotech #130-080-601) and CD25 (BD #557138) was performed according to the supplier's suggestions. Cells were washed twice with 150 µl/well PBS containing 0.1% BSA and fixed for 15 min at 4°C, using 100 µl/well fixation buffer (BD #554655). After centrifugation, the samples were resuspended in 200 µl/well PBS with 0.1% BSA and analyzed using a FACS CantoII machine (Software FACS Diva).

As depicted in Figure 25, the bispecific construct induces concentration-dependent up-regulation of CD25 on CD8⁺ T cells only in the presence of target cells. The anti cyno CD3 IgG (clone FN-18) is also able to induce up-regulation of CD25 on CD8⁺ T cells, without being crosslinked (see data obtained with cyno Nestor). There is no hyperactivation of cyno T cells with the maximal concentration of the bispecific construct (in the absence of target cells).

In another experiment, the CD3-MCSP "2+1 IgG Crossfab, linked light chain" (see SEQ ID NOs 3, 5, 29, 179) was compared to the CD3-MCSP "2+1 IgG Crossfab" (see SEQ ID NOs 3, 5, 29, 33) for its potential to up-regulate the early activation marker CD69 or the late activation marker CD25 on CD8⁺ T cells in the presence of tumor target cells. Primary human PBMCs (isolated as described above) were incubated with the indicated concentrations of bispecific constructs for at least 22 h in the presence or absence of MCSP-positive Colo38 target cells. Briefly, 0.3 million primary human PBMCs were plated per well of a flat-bottom 96-well plate, containing the MCSP-positive target cells (or medium). The final effector to target cell (E:T) ratio was 10:1. The cells were incubated with the indicated concentration of the bispecific constructs and controls for the indicated incubation times at 37°C, 5% CO₂. The effector cells were stained for CD8, and CD69 or CD25 and analyzed by FACS CantoII.

Figure 53 shows the result of this experiment. There were no significant differences detected for CD69 (A) or CD25 up-regulation (B) between the two 2+1 IgG Crossfab molecules (with or without the linked light chain).

In yet another experiment, the CD3/MCSP "2+1 IgG Crossfab" (see SEQ ID NOs 3, 5, 29, 33) and "1+1 IgG Crossfab" (see SEQ ID NOs 5, 29, 33, 181) constructs were compared to the "1+1 CrossMab" construct (see SEQ ID NOs 5, 23, 183, 185) for their potential to up-regulate CD69 or CD25 on CD4⁺ or CD8⁺ T cells in the presence of tumor target cells. The assay was performed as described above, in the presence of absence of human MCSP expressing MV-3 tumor cells, with an incubation time of 24 h.

As shown in Figure 59, the "1+1 IgG Crossfab" and "2+1 IgG Crossfab" constructs induced more pronounced upregulation of activation markers than the "1+1 CrossMab" molecule.

In a final experiment, the CD3/MCSP "2+1 IgG Crossfab" (see SEQ ID NOs 5, 23, 215, 217) and "2+1 IgG Crossfab, inverted" (see SEQ ID NOs 5, 23, 215, 219) constructs were assessed for their potential to up-regulate CD25 on CD4⁺ or CD8⁺ T cells from two different cynomolgus monkeys in the presence of tumor target cells. The assay was performed as described above, in the presence of absence of human MCSP expressing MV-3 tumor cells, with an E:T ratio of 3:1 and an incubation time of about 41 h.

As shown in Figure 60, both constructs were able to up-regulate CD25 on CD4⁺ and CD8⁺ T cells in a concentration-dependent manner, without significant difference between the two formats. Control samples without antibody and without target cells gave a comparable signal to the samples with antibody but no targets (not shown).

### Example 5

### Interferon-y secretion upon activation of human pan T cells with CD3 bispecific constructs

Purified "2+1 IgG scFab" targeting human MCSP and human CD3 (SEQ ID NOs 5, 17, 19) was analyzed for its potential to induce T cell activation in the presence of human MCSP-positive U-87MG cells, measured by the release of human interferon (IFN)-γ into the supernatant. As controls, anti-human MCSP and anti-human CD3 IgGs were used, adjusted to the same molarity. Briefly, huMCSP-expressing U-87MG glioblastoma astrocytoma target cells (ECACC 89081402) were harvested with Cell Dissociation Buffer, washed and resuspendend in AIM-V medium (Invitrogen #12055-091). 20 000 cells per well were plated in a round-bottom 96-wellplate and the respective antibody dilution was added to obtain a final concentration of 1 nM. Human pan T effector cells, isolated from Buffy Coat, were added to obtain a final E:T ratio of 5:1. After an overnight incubation of 18.5 h at 37°C, 5% CO₂, the assay plate was centrifuged for 5 min at 350 x g and the supernatant was transferred into a fresh 96-well plate. Human IFN-γ levels in the supernatant were measured by ELISA, according to the manufacturer's instructions (BD OptEIA human IFN-γ ELISA Kit II from Becton Dickinson, #550612).

As depicted in Figure 26, the reference IgGs show no to weak induction of IFN-γ secretion, whereas the "2+1 IgG scFab" construct is able to activate human T cells to secrete IFN-γ.

### Example 6

### Re-directed T cell cytotoxicity mediated by cross-linked bispecific constructs targeting CD3 on T cells and MCSP or EGFR on tumor cells (LDH release assay)

In a first series of experiments, bispecific constructs targeting CD3 and MCSP were analyzed for their potential to induce T cell-mediated apoptosis in tumor target cells upon crosslinkage of the construct via binding of the antigen binding moieties to their respective target antigens on cells (Figures 27-38).

In one experiment purified "2+1 IgG scFab" (SEQ ID NOs 5, 21, 23) and "2+1 IgG Crossfab" (SEQ ID NOs 3, 5, 29, 33) constructs targeting human CD3 and human MCSP, and the corresponding "(scFv)₂" molecule, were compared. Briefly, huMCSP-expressing MDA-MB-435 human melanoma target cells were harvested with Cell Dissociation Buffer, washed and resuspendend in AIM-V medium (Invitrogen # 12055-091). 30 000 cells per well were plated in a round-bottom 96-well plate and the respective dilution of the construct was added at the indicated concentration. All constructs and corresponding control IgGs were adjusted to the same molarity. Human pan T effector cells were added to obtain a final E:T ratio of 5:1. As a positive control for the activation of human pan T cells, 1 µg/ml PHA-M (Sigma #L8902; mixture of isolectins isolated from Phaseolus vulgaris) was used. For normalization, maximal lysis of the target cells (= 100%) was determined by incubation of the target cells with a final concentration of 1% Triton X-100. Minimal lysis (= 0%) refers to target cells co-incubated with effector cells, but without any construct or antibody. After an overnight incubation of 20 h at 37°C, 5% CO₂, LDH release of apoptotic/necrotic target cells into the supernatant was measured with the LDH detection kit (Roche Applied Science, #11 644 793 001), according to the manufacturer's instructions.

As depicted in Figure 27, both "2+1" constructs induce apoptosis in target cells comparable to the "(scFv)₂" molecule.

Further, purified "2+1 IgG Crossfab" (SEQ ID NOs 3, 5, 29, 33) and "2+1 IgG scFab" constructs differing in their Fc domain, as well as the "(scFv)2" molecule, were compared. The different mutations in the Fc domain (L234A+L235A (LALA), P329G and/or N297D, as indicated) reduce or abolish the (NK) effector cell function induced by constructs containing a wild-type (wt) Fc domain. Experimental procedures were as described above.

Figure 28 shows that all constructs induce apoptosis in target cells comparable to the "(scFv)2" molecule.

Figure 29 shows the result of a comparison of the purified "2+1 IgG scFab" (SEQ ID NOs 5, 17, 19) and the "(scFv)2" molecule for their potential to induce T cell-mediated apoptosis in tumor target cells. Experimental procedures were as decribed above, using huMCSP-expressing Colo-38 human melanoma target cells at an E:T ratio of 5:1, and an overnight incubation of 18.5 h. As depicted in the figure, the "2+1 IgG scFab" construct shows comparable cytotoxic activity to the "(scFv)₂" molecule.

Similarly, Figure 30 shows the result of a comparison of the purified "2+1 IgG scFab" construct (SEQ ID NOs 5, 17, 19)and the "(scFv)2" molecule, using huMCSP-expressing Colo-38 human melanoma target cells at an E:T ratio of 5:1 and an incubation time of 18 h. As depicted in the figure, the "2+1 IgG scFab" construct shows comparable cytotoxic activity to the (scFv)2 molecule.

Figure 31 shows the result of a comparison of the purified "2+1 IgG scFab" construct (SEQ ID NOs 5, 17, 19) and the "(scFv)2" molecule, using huMCSP-expressing MDA-MB-435 human melanoma target cells at an E:T ratio of 5:1 and an overnight incubation of 23.5 h. As depicted in the figure, the construct induces apoptosis in target cells comparably to the "(scFv)2" molecule. The "2+1 IgG scFab" construct shows reduced efficacy at the highest concentrations.

Furthermore, different bispecific constructs that are monovalent for both targets, human CD3 and human MCSP, as well as the corresponding "(scFv)2" molecule were analyzed for their potential to induce T cell-mediated apoptosis. Figure 32 shows the results for the "1+1 IgG scFab, one-armed" (SEQ ID NOs 1, 3, 5) and "1+1 IgG scFab, one-armed inverted" (SEQ ID NOs 7, 9, 11) constructs, using huMCSP-expressing Colo-38 human melanoma target cells at an E:T ratio of 5:1, and an incubation time of 19 h. As depicted in the figure, both "1+1" constructs are less active than the "(scFv)2" molecule, with the "1+1 IgG scFab, one-armed" molecule being superior to the "1+1 IgG scFab, one-armed inverted" molecule in this assay.

Figure 33 shows the results for the "1+1 IgG scFab" construct (SEQ ID NOs 5, 21, 213), using huMCSP-expressing Colo-38 human melanoma target cells at an E:T ratio of 5:1, and an incubation time of 20 h. As depicted in the figure, the "1+1 IgG scFab" construct is less cytotoxic than the "(scFv)2" molecule.

In a further experiment the purified "2+1 IgG Crossfab" (SEQ ID NOs 3, 5, 29, 33), the "1+1 IgG Crossfab" (SEQ ID NOs 5, 29, 31, 33) and the "(scFv)2" molecule were analyzed for their potential to induce T cell-mediated apoptosis in tumor target cells upon crosslinkage of the construct via binding of both target antigens, CD3 and MCSP, on cells. huMCSP-expressing MDA-MB-435 human melanoma cells were used as target cells, the E:T ratio was 5:1, and the incubation time 20 h. The results are shown in Figure 34. The "2+1 IgG Crossfab" construct induces apoptosis in target cells comparably to the "(scFv)2" molecule. The comparison of the mono- and bivalent "IgG Crossfab" formats clearly shows that the bivalent one is much more potent.

In yet another experiment, the purified "2+1 IgG Crossfab" (SEQ ID NOs 3, 5, 29, 33) construct was analyzed for its potential to induce T cell-mediated apoptosis in different (tumor) target cells. Briefly, MCSP-positive Colo-38 tumor target cells, mesenchymal stem cells (derived from bone marrow, Lonza #PT-2501 or adipose tissue, Invitrogen #R7788-115) or pericytes (from placenta; PromoCell #C-12980), as indicated, were harvested with Cell Dissociation Buffer, washed and resuspendend in AIM-V medium (Invitrogen #12055-091). 30 000 cells per well were plated in a round-bottom 96-well plate and the respective antibody dilution was added at the indicated concentrations. Human PBMC effector cells isolated from fresh blood of a healthy donor were added to obtain a final E:T ratio of 25:1. After an incubation of 4 h at 37°C, 5% CO₂, LDH release of apoptotic/necrotic target cells into the supernatant was measured with the LDH detection kit (Roche Applied Science, #11 644 793 001), according to the manufacturer's instructions.

As depicted in Figure 35, significant T-cell mediated cytotoxicity could be observed only with Colo-38 cells. This result is in line with Colo-38 cells expressing significant levels of MCSP, whereas mesenchymal stem cells and pericytes express MCSP only very weakly.

The purified "2+1 IgG scFab" (SEQ ID NOs 5, 17, 19) construct and the "(scFv)₂" molecule were also compared to a glycoengineered anti-human MCSP IgG antibody, having a reduced proportion of fucosylated N-glycans in its Fc domain (MCSP GlycoMab). For this experiment huMCSP-expressing Colo-38 human melanoma target cells and human PBMC effector cells were used, either at a fixed E:T ratio of 25:1 (Figure 36A), or at different E:T ratios from 20:1 to 1:10 (Figure 36B). The different molecules were used at the concentrations indicated in Figure 36A, or at a fixed concentration of 1667 pM (Figure 36B). Read-out was done after 21 h incubation. As depicted in Figure 36 A and B, both bispecific constructs show a higher potency than the MSCP GlycoMab.

In another experiment, purified "2+1 IgG Crossfab" targeting cynomolgus CD3 and human MCSP (SEQ ID NOs 3, 5, 35, 37) was analyzed. Briefly, human MCSP-expressing MV-3 tumor target cells were harvested with Cell Dissociation Buffer, washed and resuspendend in DMEM containing 2% FCS and 1% GlutaMax. 30 000 cells per well were plated in a round-bottom 96-well plate and the respective dilution of construct or reference IgG was added at the concentrations indicated. The bispecific construct and the different IgG controls were adjusted to the same molarity. Cynomolgus PBMC effector cells, isolated from blood of healthy cynomolgus, were added to obtain a final E:T ratio of 3:1. After incubation for 24 h or 43 h at 37°C, 5% CO₂, LDH release of apoptotic/necrotic target cells into the supernatant was measured with the LDH detection kit (Roche Applied Science, #11 644 793 001), according to the manufacturer's instructions.

As depicted in Figure 37, the bispecific construct induces concentration-dependent LDH release from target cells. The effect is stronger after 43 h than after 24 h. The anti-cynoCD3 IgG (clone FN-18) is also able to induce LDH release of target cells without being crosslinked.

Figure 38 shows the result of a comparison of the purified "2+1 IgG Crossfab" (SEQ ID NOs 3, 5, 29, 33) and the "(scFv)2" construct, using MCSP-expressing human melanoma cell line (MV-3) as target cells and human PBMCs as effector cells with an E:T ratio of 10:1 and an incubation time of 26 h. As depicted in the figure, the "2+1 IgG Crossfab" construct is more potent in terms of EC50 than the "(scFv)2" molecule.

In a second series of experiments, bispecific constructs targeting CD3 and EGFR were analyzed for their potential to induce T cell-mediated apoptosis in tumor target cells upon crosslinkage of the construct via binding of the antigen binding moieties to their respective target antigens on cells (Figures 39-41).

In one experiment purified "2+1 IgG scFab" (SEQ ID NOs 45, 47, 53) and "1+1 IgG scFab" (SEQ ID NOs 47, 53, 213) constructs targeting CD3 and EGFR, and the corresponding "(scFv)2" molecule, were compared. Briefly, human EGFR-expressing LS-174T tumor target cells were harvested with trypsin, washed and resuspendend in AIM-V medium (Invitrogen # 12055-091). 30 000 cells per well were plated in a round-bottom 96-well-plate and the respective antibody dilution was added at the indicated concentrations. All constructs and controls were adjusted to the same molarity. Human pan T effector cells were added to obtain a final E:T ratio of 5:1. As a positive control for the activation of human pan T cells, 1 µg/ml PHA-M (Sigma #L8902) was used. For normalization, maximal lysis of the target cells (= 100%) was determined by incubation of the target cells with a final concentration of 1% Triton X-100. Minimal lysis (= 0%) refers to target cells co-incubated with effector cells, but without any construct or antibody. After an overnight incubation of 18 h at 37°C, 5% CO₂, LDH release of apoptotic/necrotic target cells into the supernatant was measured with the LDH detection kit (Roche Applied Science, #11 644 793 001), according to the manufacturer's instructions.

As depicted in Figure 39, the "2+1 IgG scFab" construct shows comparable cytotoxic activity to the "(scFv)₂" molecule, whereas the "1+1 IgG scFab" construct is less active.

In another experiment the purified "1+1 IgG scFab, one-armed" (SEQ ID NOs 43, 45, 47), "1+1 IgG scFab, one-armed inverted" (SEQ ID NOs 11, 49, 51), "1+1 IgG scFab" (SEQ ID NOs 47, 53, 213), and the "(scFv)₂" molecule were compared. Experimental conditions were as described above, except for the incubation time which was 21 h.

As depicted in Figure 40, the "1+1 IgG scFab" construct shows a slightly lower cytotoxic activity than the "(scFv)2" molecule in this assay. Both "1+1 IgG scFab, one-armed (inverted)" constructs are clearly less active than the "(scFv)2" molecule.

In yet a further experiment the purified "1+1 IgG scFab, one-armed" (SEQ ID NO 43, 45, 47) and "1+1 IgG scFab, one-armed inverted" (SEQ ID NOs 11, 49, 51) constructs and the "(scFv)2" molecule were compared. The incubation time in this experiment was 16 h, and the result is depicted in Figure 41. Incubated with human pan T cells, both "1+1 IgG scFab, one-armed (inverted)" constructs are less active than the "(scFv)₂" molecule, but show concentration-dependent release of LDH from target cells (Figure 41A). Upon co-cultivation of the LS-174T tumor cells with naive T cells isolated from PBMCs, the constructs had only a basal activity - the most active among them being the "(scFv)2" molecule (Figure 41B).

In a further experiment, purified "1+1 IgG scFab, one-armed inverted" (SEQ ID NOs 11, 51, 55), "1+1 IgG scFab" (57, 61, 213), and "2+1 IgG scFab" (57, 59, 61) targeting CD3 and Fibroblast Activation Protein (FAP), and the corresponding "(scFv)2" molecule were analyzed for their potential to induce T cell-mediated apoptosis in human FAP-expressing fibroblasts GM05389 cells upon crosslinkage of the construct via binding of both targeting moieties to their respective target antigens on the cells. Briefly, human GM05389 target cells were harvested with trypsin on the day before, washed and resuspendend in AIM-V medium (Invitrogen #12055-091). 30 000 cells per well were plated in a round-bottom 96-well plate and incubated overnight at 37°C, 5% CO2 to allow the cells to recover and adhere. The next day, the cells were centrifuged, the supernatant was discarded and fresh medium, as well as the respective dilution of the constructs or reference IgGs was added at the indicated concentrations. All constructs and controls were adjusted to the same molarity. Human pan T effector cells were added to obtain a final E:T ratio of 5:1. As a positive control for the activation of human pan T cells, 5 µg/ml PHA-M (Sigma #L8902) was used. For normalization, maximal lysis of the target cells (= 100%) was determined by incubation of the target cells with a final concentration of 1% Triton X-100. Minimal lysis (= 0%) refers to target cells co-incubated with effector cells, but without any construct or antibody. After an additional overnight incubation of 18 h at 37°C, 5% CO₂, LDH release of apoptotic/necrotic target cells into the supernatant was measured with the LDH detection kit (Roche Applied Science, #11 644 793 001), according to the manufacturer's instructions.

As depicted in Figure 42, the "2+1 IgG scFab" construct shows comparable cytotoxic activity to the "(scFv)₂" molecule in terms of EC50 values. The "1+1 IgG scFab, one-armed inverted" construct is less active than the other constructs tested in this assay.

In another set of experiments, the CD3/MCSP "2+1 IgG Crossfab, linked light chain" (see SEQ ID NOs 3, 5, 29, 179) was compared to the CD3/MCSP "2+1 IgG Crossfab" (see SEQ ID NOs 3, 5, 29, 33). Briefly, target cells (human Colo-38, human MV-3 or WM266-4 melanoma cells) were harvested with Cell Dissociation Buffer on the day of the assay (or with trypsin one day before the assay was started), washed and resuspended in the appropriate cell culture medium (RPMI1640, including 2% FCS and 1% Glutamax). 20 000 - 30 000 cells per well were plated in a flat-bottom 96-well plate and the respective antibody dilution was added as indicated (triplicates). PBMCs as effector cells were added to obtain a final effector-to-target cell (E:T) ratio of 10:1. All constructs and controls were adjusted to the same molarity, incubation time was 22 h. Detection of LDH release and normalization was done as described above.

Figure 49 to 52 show the result of four assays performed with MV-3 melanoma cells (Figure 49), Colo-38 cells (Figure 50 and 51) or WM266-4 cells (Figure 52). As shown in Figure 49, the construct with the linked light chain was less potent compared to the one without the linked light chain in the assay with MV-3 cells as target cells. As shown in Figure 50 and 51, the construct with the linked light chain was more potent compared to the one without the linked light chain in the assays with high MCSP expressing Colo-38 cells as target cells. Finally, as shown in Figure 52, there was no significant difference between the two constructs when high MCSP-expressing WM266-4 cells were used as target cells.

In another experiment, two CEA-targeting "2+1 IgG Crossfab, inverted" constructs were compared, wherein in the Crossfab fragment either the V regions (VL/VH, see SEQ ID NOs 33, 63, 65, 67) or the C regions (CL/CH1, see SEQ ID NOs 65, 67, 183, 197) were exchanged. The assay was performed as described above, using human PBMCs as effector cells and human CEA-expressing target cells. Target cells (MKN-45 or LS-174T tumor cells) were harvested with trypsin-EDTA (LuBiosciences #25300-096), washed and resuspendend in RPMI1640 (Invitrogen #42404042), including 1% Glutamax (LuBiosciences #35050087) and 2% FCS. 30 000 cells per well were plated in a round-bottom 96-well plate and the bispecific constructs were added at the indicated concentrations. All constructs and controls were adjusted to the same molarity. Human PBMC effector cells were added to obtain a final E:T ratio of 10:1, incubation time was 28 h. EC50 values were calculated using the GraphPad Prism 5 software.

As shown in Figure 61, the construct with the CL/CH1 exchange shows slightly better activity on both target cell lines than the construct with the VL/VH exchange. Calculated EC50 values were 115 and 243 pM on MKN-45 cells, and 673 and 955 pM on LS-174T cells, for the CL/CH1-exchange construct and the VL/VH-exchange construct, respectively.

Similarly, two MCSP-targeting "2+1 IgG Crossfab" constructs were compared, wherein in the Crossfab fragment either the V regions (VL/VH, see SEQ ID NOs 33, 189, 191, 193) or the C regions (CL/CH1, see SEQ ID NOs 183, 189, 193, 195) were exchanged. The assay was performed as described above, using human PBMCs as effector cells and human MCSP-expressing target cells. Target cells (WM266-4) were harvested with Cell Dissociation Buffer (LuBiosciences #13151014), washed and resuspendend in RPMI1640 (Invitrogen #42404042), including 1% Glutamax (LuBiosciences #35050087) and 2% FCS. 30 000 cells per well were plated in a round-bottom 96-well plate and the constructs were added at the indicated concentrations. All constructs and controls were adjusted to the same molarity. Human PBMC effector cells were added to obtain a final E:T ratio of 10:1, incubation time was 26 h. EC50 values were calculated using the GraphPad Prism 5 software.

As depicted in Figure 62, the two constructs show comparable activity, the construct with the CL/CH1 exchange having a slightly lower EC50 value (12.9 pM for the CL/CH1-exchange construct, compared to 16.8 pM for the VL/VH-exchange construct).

Figure 63 shows the result of a similar assay, performed with human MCSP-expressing MV-3 target cells. Again, both constructs show comparable activity, the construct with the CL/CH1 exchange having a slightly lower EC50 value (approximately 11.7 pM for the CL/CH1-exchange construct, compared to approximately 82.2 pM for the VL/VH-exchange construct). Exact EC50 values could not be calculated, since the killing curves did not reach a plateau at high concentrations of the compounds.

In a further experiment, the CD3/MCSP "2+1 IgG Crossfab" (see SEQ ID NOs 3, 5, 29, 33) and "1+1 IgG Crossfab" (see SEQ ID NOs 5, 29, 33, 181) constructs were compared to the CD3/MCSP "1+1 CrossMab" (see SEQ ID NOs 5, 23, 183, 185). The assay was performed as described above, using human PBMCs as effector cells and WM266-4 or MV-3 target cells (E:T ratio = 10:1) and an incubation time of 21 h.

As shown in Figure 64, the "2+1 IgG Crossfab" construct is the most potent molecule in this assay, followed by the "1+1 IgG Crossfab" and the "1+1 CrossMab". This ranking is even more pronounced with MV-3 cells, expressing medium levels of MCSP, compared to high MCSP expressing WM266-4 cells. The calculated EC50 values on MV-3 cells were 9.2, 40.9 and 88.4 pM, on WM266-4 cells 33.1, 28.4 and 53.9 pM, for the "2+1 IgG Crossfab", the "1+1 IgG Crossfab" and the "1+1 CrossMab", respectively.

In a further experiment, different concentrations of the "1+1 IgG Crossfab LC fusion" construct (SEQ ID NOs 183, 209, 211, 213) were tested, using MKN-45 or LS-174T tumor target cells and human PBMC effector cells at an E:T ratio of 10:1 and an incubation time of 28 hours. As shown in Figure 65, the "1+1 IgG Crossfab LC fusion" construct induced apoptosis in MKN-45 target cells with a calculated EC50 of 213 pM, whereas the calculated EC50 is 1.56 nM with LS-174T cells, showing the influence of the different tumor antigen expression levels on the potency of the bispecific constructs within a certain period of time.

In yet another experiment, the "1+1 IgG Crossfab LC fusion" construct (SEQ ID NOs 183, 209, 211, 213) was compared to a untargeted "2+1 IgG Crossfab" molecule. MC38-huCEA tumor cells and human PBMCs (E:T ratio = 10:1) and an incubation time of 24 hours were used. As shown in Figure 66, the "1+1 IgG Crossfab LC fusion" construct induced apoptosis of target cells in a concentration-dependent manner, with a calculated EC50 value of approximately 3.2 nM. In contrast, the untargeted "2+1 IgG Crossfab" showed antigen-independent T cell-mediated killing of target cells only at the highest concentration.

In a final experiment, the "2+1 IgG Crossfab (V9)" (SEQ ID NOs 3, 5, 29, 33), the "2+1 IgG Crossfab, inverted (V9)" (SEQ ID NOs 5, 23, 183, 187), the "2+1 IgG Crossfab (anti-CD3)" (SEQ ID NOs 5, 23, 215, 217), the "2+1 IgG Crossfab, inverted (anti-CD3)" (SEQ ID NOs 5, 23, 215, 219) were compared, using human MCSP-positive MV-3 or WM266-4 tumor cells and human PBMCs (E:T ratio = 10:1), and an incubation time of about 24 hours. As depicted in Figure 67, the T cell-mediated killing of the "2+1 IgG Crossfab, inverted" constructs seems to be slightly stronger or at least equal to the one induced by the "2+1 IgG Crossfabt" constructs for both CD3 binders. The calculated EC50 values were as follows:

| **EC50 [pM]** | 2+1 IgG Crossfab (V9) | 2+1 IgG Crossfab inverted (V9) | 2+1 IgG Crossfab (anti-CD3) | 2+1 IgG Crossfab, inverted (anti-CD3) |
|---|---|---|---|---|
| MV-3 | 10.0 | 4.1 | 11.0 | 3.0 |
| WM266-4 | 12.4 | 3.7 | 11.3 | 7.1 |

### Example 7

### CD107a/b assay

Purified "2+1 IgG scFab" construct (SEQ ID NOs 5, 17, 19) and the "(scFv)2" molecule, both targeting human MCSP and human CD3, were tested by flow cytometry for their potential to up-regulate CD107a and intracellular perforin levels in the presence or absence of human MCSP-expressing tumor cells.

Briefly, on day one, 30 000 Colo-38 tumor target cells per well were plated in a round-bottom 96-well plate and incubated overnight at 37°C, 5% CO₂ to let them adhere. Primary human pan T cells were isolated on day 1 or day 2 from Buffy Coat, as described.

On day two, 0.15 million effector cells per well were added to obtain a final E:T ratio of 5:1. FITC-conjugated CD107a/b antibodies, as well as the different bispecific constructs and controls are added. The different bispecific molecules and antibodies were adjusted to same molarities to obtain a final concentration of 9.43 nM. Following a 1 h incubation step at 37°C, 5% CO₂, monensin was added to inhibit secretion, but also to neutralize the pH within endosomes and lysosomes. After an additional incubation time of 5 h, cells were stained at 4°C for 30 min for surface CD8 expression. Cells were washed with staining buffer (PBS / 0.1% BSA), fixed and permeabilized for 20 min using the BD Cytofix/Cytoperm Plus Kit with BD Golgi Stop (BD Biosciences #554715). Cells were washed twice using 1 x BD Perm/Wash buffer, and intracellular staining for perforin was performed at 4°C for 30 min. After a final washing step with 1 x BD Perm/Wash buffer, cells were resuspended in PBS / 0.1% BSA and analyzed on FACS CantoII (all antibodies were purchased from BD Biosciences or BioLegend).

Gates were set either on all CD107a/b positive, perforin-positive or double-positive cells, as indicated (Figure 43). The "2+1 IgG scFab" construct was able to activate T cells and up-regulate CD107a/b and intracellular perforin levels only in the presence of target cells (Figure 43A), whereas the "(scFv)2" molecule shows (weak) induction of activation of T cells also in the absence of target cells (Figure 43B). The bivalent reference anti-CD3 IgG results in a lower level of activation compared to the "(scFv)2" molecule or the other bispecific construct.

### Example 8

### Proliferation assay

The purified "2+1 IgG scFab" (SEQ ID NOs 5, 17, 19) and "(scFv)2" molecules, both targeting human CD3 and human MCSP, were tested by flow cytometry for their potential to induce proliferation of CD8⁺ or CD4⁺ T cells in the presence and absence of human MCSP-expressing tumor cells.

Briefly, freshly isolated human pan T cells were adjusted to 1 million cells per ml in warm PBS and stained with 1 µM CFSE at room temperature for 10 minutes. The staining volume was doubled by addition of RPMI1640 medium, containing 10% FCS and 1% GlutaMax. After incubation at room temperature for further 20 min, the cells were washed three times with prewarmed medium to remove remaining CFSE. MCSP-positive Colo-38 cells were harvested with Cell Dissociation buffer, counted and checked for viability. Cells were adjusted to 0.2 x 10⁶ (viable) cells per ml in AIM-V medium, 100 µl of this cell suspension were pipetted per well into a round-bottom 96-well plate (as indicated). 50 µl of the (diluted) bispecific constructs were added to the cell-containing wells to obtain a final concentration of 1 nM. CFSE-stained human pan T effector cells were adjusted to 2 x 10⁶ (viable) cells per ml in AIM-V medium. 50 µl of this cell suspension was added per well of the assay plate (see above) to obtain a final E:T ratio of 5:1. To analyze whether the bispecific constructs are able to activate T cells only in the presence of target cells, expressing the tumor antigen huMCSP, wells were included that contained 1 nM of the respective bispecific molecules as well as PBMCs, but no target cells. After incubation for five days at 37°C, 5% CO₂, cells were centrifuged (5 min, 350 x g) and washed twice with 150 µl/well PBS, including 0.1% BSA. Surface staining for CD8 (mouse IgG1,κ; clone HIT8a; BD #555635), CD4 (mouse IgG1,κ; clone RPA-T4 ; BD #560649), or CD25 (mouse IgG1,κ; clone M-A251; BD #555434) was performed at 4°C for 30 min, according to the supplier's suggestions. Cells were washed twice with 150 µl/well PBS containing 0.1% BSA, resuspended in 200 µl/well PBS with 0.1% BSA, and analyzed using a FACS CantoII machine (Software FACS Diva). The relative proliferation level was determined by setting a gate around the non-proliferating cells and using the cell number of this gate relative to the overall measured cell number as the reference.

Figure 44 shows that all constructs induce proliferation of CD8⁺ T cells (A) or CD4⁺ T cells (B) only in the presence of target cells, comparably to the "(scFv)2" molecule. In general, activated CD8⁺ T cells proliferate more than activated CD4⁺ T cells in this assay.

### Example 9

### Cytokine release assay

The purified "2+1 IgG scFab" construct (SEQ ID NOs 5, 17, 19) and the "(scFv)2"molecule, both targeting human MCSP and human CD3, were analyzed for their ability to induce T cell-mediated *de novo* secretion of cytokines in the presence or absence of tumor target cells.

Briefly, human PBMCs were isolated from Buffy Coats and 0.3 million cells were plated per well into a round-bottom 96-well plate. Colo-38 tumor target cells, expressing human MCSP, were added to obtain a final E:T-ratio of 10:1. Bispecific constructs and IgG controls were added at 1 nM final concentration and the cells were incubated for 24 h at 37°C, 5% CO₂. The next day, the cells were centrifuged for 5 min at 350 x g and the supernatant was transferred into a new deep-well 96-well-plate for the subsequent analysis. The CBA analysis was performed according to manufacturer's instructions for FACS CantoII, using the Human Th1/Th2 Cytokine Kit II (BD #551809).

Figure 45 shows levels of the different cytokine measured in the supernatant. In the presence of target cells the main cytokine secreted upon T cell activation is IFN-γ. The "(scFv)2" molecule induces a slightly higher level of IFN-γ than the "2+1 IgG scFab" construct. The same tendency might be found for human TNF, but the overall levels of this cytokine were much lower compared to IFN-γ. There was no significant secretion of Th2 cytokines (IL-10 and IL-4) upon activation of T cells in the presence (or absence) of target cells. In the absence of Colo-38 target cells, only very weak induction of TNF secretion was observed, which was highest in samples treated with the "(scFv)₂" molecule.

In a second experiment, the following purified bispecific constructs targeting human MCSP and human CD3 were analyzed: the "2+1 IgG Crossfab" construct (SEQ ID NOs 3, 5, 29, 33), the "(scFv)₂" molecule, as well as different "2+1 IgG scFab" molecules comprising either a wild-type or a mutated (LALA, P329G and/or N297D, as indicated) Fc domain. Briefly, 280 µl whole blood from a healthy donor were plated per well of a deep-well 96-well plate. 30 000 Colo-38 tumor target cells, expressing human MCSP, as well as the different bispecific constructs and IgG controls were added at 1 nM final concentration. The cells were incubated for 24 h at 37°C, 5% CO₂ and then centrifuged for 5 min at 350 x g. The supernatant was transferred into a new deep-well 96-well-plate for the subsequent analysis. The CBA analysis was performed according to manufacturer's instructions for FACS CantoII, using the combination of the following CBA Flex Sets: human granzyme B (BD #560304), human IFN-γ Flex Set (BD #558269), human TNF Flex Set (BD #558273), human IL-10 Flex Set (BD #558274), human IL-6 Flex Set (BD #558276), human IL-4 Flex Set (BD #558272), human IL-2 Flex Set (BD #558270).

Figure 46 shows the levels of the different cytokine measured in the supernatant. The main cytokine secreted in the presence of Colo-38 tumor cells was IL-6, followed by IFN-γ. In addition, also the levels of granzyme B strongly increased upon activation of T cells in the presence of target cells. In general, the "(scFv)2" molecule induced higher levels of cytokine secretion in the presence of target cells (Figure 46, A and B). There was no significant secretion of Th2 cytokines (IL-10 and IL-4) upon activation of T cells in the presence (or absence) of target cells.

In this assay, there was a weak secretion of IFN-γ, induced by different "2+1 IgG scFab" constructs, even in the absence of target cells (Figure 46, C and D). Under these conditions, no significant differences could be observed between "2+1 IgG scFab" constructs with a wild-type or a mutated Fc domain.

Furthermore, the invention relates to the following items:
1. A T cell activating bispecific antigen binding molecule comprising a first and a second antigen binding moiety, one of which is a Fab molecule capable of specific binding to an activating T cell antigen and the other one of which is a Fab molecule capable of specific binding to a target cell antigen, and an Fc domain composed of a first and a second subunit capable of stable association;
   wherein the first antigen binding moiety is
   (a) a single chain Fab molecule wherein the Fab light chain and the Fab heavy chain are connected by a peptide linker, or
   (b) a crossover Fab molecule wherein either the variable or the constant regions of the Fab light chain and the Fab heavy chain are exchanged.
2. The T cell activating bispecific antigen binding molecule of item 1, comprising not more than one antigen binding moiety capable of specific binding to an activating T cell antigen.
3. The T cell activating bispecific antigen binding molecule of item 1 or 2, wherein the first and the second antigen binding moiety are fused to each other, optionally via a peptide linker.
4. The T cell activating bispecific antigen binding molecule of any one of items 1 to 3, wherein the second antigen binding moiety is fused at the C-terminus of the Fab heavy chain to the N-terminus of the Fab heavy chain of the first antigen binding moiety.
5. The T cell activating bispecific antigen binding molecule of any one of items 1 to 3, wherein the first antigen binding moiety is fused at the C-terminus of the Fab heavy chain to the N-terminus of the Fab heavy chain of the second antigen binding moiety.
6. The T cell activating bispecific antigen binding molecule of item 4 or 5, wherein the first antigen binding moiety is a crossover Fab molecule and the Fab light chain of the first antigen binding moiety and the Fab light chain of the second antigen binding moiety are fused to each other, optionally via a peptide linker.
7. The T cell activating bispecific antigen binding molecule of any one of items 1 to 3, wherein the second antigen binding moiety of the T cell activating bispecific antigen binding molecule is fused at the C-terminus of the Fab light chain to the N-terminus of the Fab light chain of the first antigen binding moiety.
8. The T cell activating bispecific antigen binding molecule of any one of items 1 to 3, 5 or 7, wherein the second antigen binding moiety of the T cell activating bispecific antigen binding molecule is fused at the C-terminus of the Fab heavy chain to the N-terminus of the first or the second subunit of the Fc domain.
9. The T cell activating bispecific antigen binding molecule of any one of items 1 to 4, wherein the first antigen binding moiety is fused at the C-terminus of the Fab heavy chain to the N-terminus of the first or second subunit of the Fc domain.
10. The T cell activating bispecific antigen binding molecule of item 1 or 2, wherein the first and the second antigen binding moiety are each fused at the C-terminus of the Fab heavy chain to the N-terminus of one of the subunits of the Fc domain.
11. The T cell activating bispecific antigen binding molecule of any one of items 1 to 10, comprising a third antigen binding moiety which is a Fab molecule capable of specific binding to a target cell antigen.
12. The T cell activating bispecific antigen binding molecule of item 11, wherein the third antigen binding moiety is fused at the C-terminus of the Fab heavy chain to the N-terminus of the first or second subunit of the Fc domain.
13. The T cell activating bispecific antigen binding molecule of item 11 or 12, wherein the second and the third antigen binding moiety are each fused at the C-terminus of the Fab heavy chain to the N-terminus of one of the subunits of the Fc domain, and the first antigen binding moiety is fused at the C-terminus of the Fab heavy chain to the N-terminus of the Fab heavy chain of the second antigen binding moiety.
14. The T cell activating bispecific antigen binding molecule of item 11 or 12, wherein the first and the third antigen binding moiety are each fused at the C-terminus of the Fab heavy chain to the N-terminus of one of the subunits of the Fc domain, and the second antigen binding moiety is fused at the C-terminus of the Fab heavy chain to the N-terminus of the Fab heavy chain of the first antigen binding moiety.
15. The T cell activating bispecific antigen binding molecule of item 13, wherein the second and the third antigen binding moiety and the Fc domain are part of an immunoglobulin molecule, particularly an IgG class immunoglobulin.
16. The T cell activating bispecific antigen binding molecule of any one of the preceding items, wherein the Fc domain is an IgG, specifically an IgG₁ or IgG₄, Fc domain.
17. The T cell activating bispecific antigen binding molecule of any one of the preceding items, wherein the Fc domain is a human Fc domain.
18. The T cell activating bispecific antigen binding molecule of any one of the preceding items, wherein the Fc domain comprises a modification promoting the association of the first and the second subunit of the Fc domain.
19. The T cell activating bispecific antigen binding molecule of item 18, wherein in the CH3 domain of the first subunit of the Fc domain an amino acid residue is replaced with an amino acid residue having a larger side chain volume, thereby generating a protuberance within the CH3 domain of the first subunit which is positionable in a cavity within the CH3 domain of the second subunit, and in the CH3 domain of the second subunit of the Fc domain an amino acid residue is replaced with an amino acid residue having a smaller side chain volume, thereby generating a cavity within the CH3 domain of the second subunit within which the protuberance within the CH3 domain of the first subunit is positionable.
20. The T cell activating bispecific antigen binding molecule of any one of the preceding items, wherein the Fc domain exhibits reduced binding affinity to an Fc receptor and/or reduced effector function, as compared to a native IgG1 Fc domain.
21. The T cell activating bispecific antigen binding molecule of any one of the preceding items, wherein the Fc domain comprises one or more amino acid substitution that reduces binding to an Fc receptor and/or effector function.
22. The T cell activating bispecific antigen binding molecule of item 21, wherein said one or more amino acid substitution is at one or more position selected from the group of L234, L235, and P329.
23. The T cell activating bispecific antigen binding molecule of item 22, wherein each subunit of the Fc domain comprises three amino acid substitutions that reduce binding to an activating Fc receptor and/or effector function wherein said amino acid substitutions are L234A, L235A and P329G.
24. The T cell activating bispecific antigen binding molecule of any one of items 20 to 23, wherein the Fc receptor is an Fcγ receptor.
25. The T cell activating bispecific antigen binding molecule of any one of items 20 to 23, wherein the effector function is antibody-dependent cell-mediated cytotoxicity (ADCC).
26. The T cell activating bispecific antigen binding molecule of any one of the preceding items, wherein the activating T cell antigen is CD3.
27. The T cell activating bispecific antigen binding molecule of any one of the preceding items, wherein the target cell antigen is selected from the group consisting of: Melanoma-associated Chondroitin Sulfate Proteoglycan (MCSP), Epidermal Growth Factor Receptor (EGFR), CD19, CD20, CD33, Carcinoembryonic Antigen (CEA) and Fibroblast Activation Protein (FAP).
28. An isolated polynucleotide encoding the T cell activating bispecific antigen binding molecule of any one of items 1 to 27 or a fragment thereof.
29. A polypeptide encoded by the isolated polynucleotide of item 28.
30. A vector, particularly an expression vector, comprising the isolated polynucleotide of item 28.
31. A host cell comprising the isolated polynucleotide of item 28 or the expression vector of item 30.
32. A method of producing the T cell activating bispecific antigen binding molecule of any one of items 1 to 27, comprising the steps of a) culturing the host cell of item 31 under conditions suitable for the expression of the T cell activating bispecific antigen binding molecule and b) recovering the T cell activating bispecific antigen binding molecule.
33. A T cell activating bispecific antigen binding molecule produced by the method of item 32.
34. A pharmaceutical composition comprising the T cell activating bispecific antigen binding molecule of any one of items 1 to 27 and a pharmaceutically acceptable carrier.
35. The T cell activating bispecific antigen binding molecule of any one of items 1 to 27 or the pharmaceutical composition of item 34 for use as a medicament.
36. The T cell activating bispecific antigen binding molecule of any one of items 1 to 27 or the pharmaceutical composition of item 34 for use in the treatment of a disease in an individual in need thereof.
37. The T cell activating bispecific antigen binding molecule or the pharmaceutical composition of item 36, wherein the disease is cancer.
38. Use of the T cell activating bispecific antigen binding molecule of any one of items 1 to 27 for the manufacture of a medicament for the treatment of a disease in an individual in need thereof.
39. A method of treating a disease in an individual, comprising administering to said individual a therapeutically effective amount of a composition comprising the T cell activating bispecific antigen binding molecule of any one of items 1 to 27 in a pharmaceutically acceptable form.
40. The use of item 38 or the method of item 39, wherein said disease is cancer.
41. A method for inducing lysis of a target cell, comprising contacting a target cell with the T cell activating bispecific antigen binding molecule of any one of items 1-27 in the presence of a T cell.
42. The invention as described hereinbefore.

Although the foregoing invention has been described in some detail by way of illustration and example for purposes of clarity of understanding, the descriptions and examples should not be construed as limiting the scope of the invention. The disclosures of all patent and scientific literature cited herein are expressly incorporated in their entirety by reference.

## Claims

1. A T cell activating bispecific antigen binding molecule comprising
(i) a first antigen binding moiety, which is a Fab molecule capable of specific binding to an activating T cell antigen,
(ii) a second and a third antigen binding moiety, which are each a Fab molecule capable of specific binding to a target cell antigen, and
(iii) an Fc domain composed of a first and a second subunit capable of stable association; wherein (a) the second antigen binding moiety is fused at the C-terminus of the Fab heavy chain to the N-terminus of the Fab heavy chain of the first antigen binding moiety, and the first antigen binding moiety is fused at the C-terminus of the Fab heavy chain to the N-terminus of the first or the second subunit of the Fc domain, or (b) the first antigen binding moiety is fused at the C-terminus of the Fab heavy chain to the N-terminus of the Fab heavy chain of the second antigen binding moiety, and the second antigen binding moiety is fused at the C-terminus of the Fab heavy chain to the N-terminus of the first or the second subunit of the Fc domain; and wherein the T cell activating bispecific antigen binding molecule comprises not more than one antigen binding moiety capable of specific binding to an activating T cell antigen.

2. The T cell activating bispecific antigen binding molecule of claim 1, wherein the activating T cell antigen is CD3.

3. The T cell activating bispecific antigen binding molecule of claim 1 or 2, wherein the first and the second antigen binding moiety are fused to each other via a peptide linker.

4. The T cell activating bispecific antigen binding molecule of any one of the preceding claims, wherein the third antigen binding moiety is fused at the C-terminus of the Fab heavy chain to the N-terminus of the first or the second subunit of the Fc domain.

5. The T cell activating bispecific antigen binding molecule of any one of the preceding claims, wherein the Fc domain is an IgG, specifically an IgG₁ or IgG₄, Fc domain.

6. The T cell activating bispecific antigen binding molecule of any one of the preceding claims, wherein the Fc domain is a human IgGi Fc domain.

7. The T cell activating bispecific antigen binding molecule of any one of the preceding claims, wherein the Fc domain comprises a modification promoting the association of the first and the second subunit of the Fc domain.

8. The T cell activating bispecific antigen binding molecule of claim 7, wherein (a) in the CH3 domain of the first subunit of the Fc domain an amino acid residue is replaced with an amino acid residue having a larger side chain volume, thereby generating a protuberance within the CH3 domain of the first subunit which is positionable in a cavity within the CH3 domain of the second subunit, and in the CH3 domain of the second subunit of the Fc domain an amino acid residue is replaced with an amino acid residue having a smaller side chain volume, thereby generating a cavity within the CH3 domain of the second subunit within which the protuberance within the CH3 domain of the first subunit is positionable; or (b) at the interface of the two Fc domain subunits one or more amino acid residues is/are replaced by charged amino acid residues so that homodimer formation becomes electrostatically unfavorable but heterodimerization electrostatically favorable.

9. The T cell activating bispecific antigen binding molecule of any one of the preceding claims, wherein in the CH3 domain of the first subunit of the Fc domain the threonine residue at position 366 is replaced with a tryptophan residue (T366W), and in the CH3 domain of the second subunit of the Fc domain the tyrosine residue at position 407 is replaced with a valine residue (Y407V) and optionally in the second subunit of the Fc domain additionally the threonine residue at position 366 is replaced with a serine residue (T366S) and the leucine residue at position 368 is replaced with an alanine residue (L368A);
and wherein optionally in the first subunit of the Fc domain additionally the serine residue at position 354 is replaced with a cysteine residue (S354C), and in the second subunit of the Fc domain additionally the tyrosine residue at position 349 is replaced by a cysteine residue (Y349C) (EU numbering).

10. The T cell activating bispecific antigen binding molecule of any one of the preceding claims, wherein the Fc domain comprises one or more amino acid substitution that reduces binding to an Fc receptor, particularly an Fcγ receptor, and/or effector function, particularly antibody-dependent cell-mediated cytotoxicity (ADCC).

11. The T cell activating bispecific antigen binding molecule of claim 10, wherein said one or more amino acid substitution is at one or more position selected from the group of E233, L234, L235, N297, P331 and P329 (EU numbering).

12. The T cell activating bispecific antigen binding molecule of any one of the preceding claims, wherein the Fc domain comprises the amino acid substitutions L234A, L235A and P329G (EU numbering).

13. An isolated polynucleotide or plurality of polynucleotides encoding the T cell activating bispecific antigen binding molecule of any one of claims 1 to 12.

14. A host cell comprising the isolated polynucleotide(s) of claim 13.

15. A method of producing the T cell activating bispecific antigen binding molecule of any one of claims 1 to 12, comprising the steps of a) culturing the host cell of claim 14 under conditions suitable for the expression of the T cell activating bispecific antigen binding molecule and b) recovering the T cell activating bispecific antigen binding molecule.

16. A pharmaceutical composition comprising the T cell activating bispecific antigen binding molecule of any one of claims 1 to 12 and a pharmaceutically acceptable carrier.

17. The T cell activating bispecific antigen binding molecule of any one of claims 1 to 12 for use as a medicament.

18. The T cell activating bispecific antigen binding molecule of any one of claims 1 to 12 for use in the treatment of cancer.
